(19) Europäisches Patentamt
European Patent Office
Office européen des brevets

(11) EP 1 790 662 A1

(12) EUROPEAN PATENT APPLICATION

(43) Date of publication:
**30.05.2007 Bulletin 2007/22**

(21) Application number: **07001331.3**

(22) Date of filing: **09.04.2002**

(51) Int Cl.:
***C07K 14/47*** *(2006.01)*
***C12N 15/12*** *(2006.01)*
***C07K 16/00*** *(2006.01)*
***A61K 38/00*** *(2006.01)*
***A61K 39/00*** *(2006.01)*

(84) Designated Contracting States:
**AT BE CH CY DE DK ES FI FR GB GR IE IT LI LU MC NL PT SE TR**

(30) Priority:
**10.04.2001 US 282739 P**
**25.04.2001 US 286630 P**
**22.06.2001 US 300373 P**

(62) Document number(s) of the earlier application(s) in accordance with Art. 76 EPC:
**02762038.4 / 1 434 592**

(71) Applicant: **Agensys, Inc.**
**Santa Monica, CA 90404 (US)**

(72) Inventors:
- **Challita-Eid, Pia M.**
  **Encino, CA 91436 (US)**
- **Raitano, Arthur B.**
  **Los Angeles, CA 90064 (US)**
- **Faris, Mary**
  **Los Angeles, CA 90077 (US)**
- **Hubert, Rene S.**
  **Los Angeles, CA 90026 (US)**
- **Mitchell, Steve Chappell**
  **Gurnee, IL 60031 (US)**
- **Afar, Daniel E. H.**
  **Brisbane, CA 94005 (US)**
- **Saffran, Douglas**
  **Encinitas, CA 92024 (US)**
- **Morrison, Karen**
  **Santa Monica, CA 90403 (US)**
- **Morrison, Robert Kendall**
  **Santa Monica, CA 90403 (US)**
- **Ge, Wangmeo**
  **Culver City, CA 90230 (US)**
- **Jakobovits, Aya**
  **Beverly Hills, CA 90210 (US)**

(74) Representative: **Roques, Sarah Elizabeth**
**J.A. Kemp & Co.**
**14 South Square**
**Gray's Inn**
**London WC1R 5JJ (GB)**

Remarks:
This application was filed on 22 - 01 - 2007 as a divisional application to the application mentioned under INID code 62.

# (54) Nucleic acid and corresponding protein entitled 121P2A3 useful in treatment and detection of cancer

(57) A novel gene (designated 121P2A3) and its encoded protein, and variants thereof, are described wherein 121P2A3 exhibits tissue specific in normal adult tissue, and is aberrantly in the cancers listed in Table 1. Consequently, 121 P2A3 provides a diagnostic, prognostic, prophylactic target for cancer. The 121P2A3 gene or fragment thereof, or its encoded protein, or variants thereof, or fragment thereof, can be used to elicit a humoral or cellular immune response; antibodies or T cells reactive with 121P2A3 can be used in active or passive immunization.

EP 1 790 662 A1

SEQUENCE LISTING

<110> Agensys, Inc.

<120> NUCLEIC ACID AND CORRESPONDING PROTEIN
ENTITLED 121P2A3 USEFUL IN TREATMENT AND DETECTION OF CANCER

<130> N89988A SER

<150> US 60/282,739
<151> 2001-04-10

<150> US 60/286,630
<151> 2001-04-25

<150> US 60/300,373
<151> 2001-06-22

<160> 82

<170> FastSEQ for Windows Version 4.0

<210> 1
<211> 259
<212> DNA
<213> Homo Sapiens

<400> 1
```
gatcattaca ttgcccagct ttaagaatgc caaaaataac taaaatactg tcaatcaaat 60
gagagggcta catgggttta ttaaagttta ttttaacaat tttagctaag cagaatgtgc 120
taatgtaatt caagttacag ttactgccag ataacataag agaaaacatt gtgtgtggcc 180
acttaagatt atgcctcaaa cagatactgt ttcgtgcgca gaacagagtt ggggaacaca 240
gctggggatt ttcttgatc                                              259
```

<210> 2
<211> 2473
<212> DNA
<213> Homo Sapiens

<220>
<221> CDS
<222> (175)...(1566)

<400> 2
```
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

tct tcc aga agt acc aaa gat tta att aaa agt aag tgg gga tcg aag   225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa   273
Pro Ser Asn Ser Lys Ser Glu Thr Thr Leu Glu Lys Leu Lys Gly Glu
        20                  25                  30
```

```
att gca cac tta aag aca tca gtg gat gaa atc aca agt ggg aaa gga    321
Ile Ala His Leu Lys Thr Ser Val Asp Glu Ile Thr Ser Gly Lys Gly
    35                  40                  45

aag ctg act gat aaa gag aga cac aga ctt ttg gag aaa att cga gtc    369
Lys Leu Thr Asp Lys Glu Arg His Arg Leu Leu Glu Lys Ile Arg Val
 50                  55                  60                  65

ctt gag gct gag aag gag aag aat gct tat caa ctc aca gag aag gac    417
Leu Glu Ala Glu Lys Glu Lys Asn Ala Tyr Gln Leu Thr Glu Lys Asp
                 70                  75                  80

aaa gaa ata cag cga ctg aga gac caa ctg aag gcc aga tat agt act    465
Lys Glu Ile Gln Arg Leu Arg Asp Gln Leu Lys Ala Arg Tyr Ser Thr
             85                  90                  95

acc gca ttg ctt gaa cag ctg gaa gag aca acg aga gaa gga gaa agg    513
Thr Ala Leu Leu Glu Gln Leu Glu Glu Thr Thr Arg Glu Gly Glu Arg
        100                 105                 110

agg gag cag gtg ttg aaa gcc tta tct gaa gag aaa gac gta ttg aaa    561
Arg Glu Gln Val Leu Lys Ala Leu Ser Glu Glu Lys Asp Val Leu Lys
    115                 120                 125

caa cag ttg tct gct gca acc tca cga att gct gaa ctt gaa agc aaa    609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac    657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct    705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc    753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg    801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca    849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg    897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag    945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa    993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca    1041
```

```
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa     1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag     1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca     1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa     1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac     1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga     1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat     1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa     1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc     1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc     1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag         1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
```

```
tttttgtaaa ccaaaaactt ttaaatttct tcaggttttc taacatgctt accactgggc 2466
tactgta                                                           2473

<210> 3
<211> 464
<212> PRT
<213> Homo Sapiens

<400> 3
Met Ser Ser Arg Ser Thr Lys Asp Leu Ile Lys Ser Lys Trp Gly Ser
1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
```

```
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460


<210> 4
<211> 2324
<212> DNA
<213> Homo Sapiens

<220>
<221> CDS
<222> (533)...(1417)

<400> 4
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agagatgtct 180
tccagaagta ccaaagattt aattaaaaaa aattcgagtc cttgaggctg agaaggagaa 240
gaatgcttat caactcacag agaaggacaa agaaatacag cgactgagag accaactgaa 300
ggccagatat agtactaccg cattgcttga acagctggaa gagacaacga gagaaggaga 360
aaggagggag caggtgttga aagccttatc tgaagagaaa gacgtattga aacaacagtt 420
gtctgctgca acctcacgaa ttgctgaact tgaaagcaaa accaatacac tccgtttatc 480
acagactgtg gctccaaact gcttcaactc atcaataaat aatattcatg aa atg gaa 538
                                                          Met Glu
                                                            1

ata cag ctg aaa gat gct ctg gag aaa aat cag cag tgg ctc gtg tat   586
Ile Gln Leu Lys Asp Ala Leu Glu Lys Asn Gln Gln Trp Leu Val Tyr
        5                   10                  15

gat cag cag cgg gaa gtc tat gta aaa gga ctt tta gca aag atc ttt   634
Asp Gln Gln Arg Glu Val Tyr Val Lys Gly Leu Leu Ala Lys Ile Phe
    20                  25                  30

gag ttg gaa aag aaa acg gaa aca gct gct cat tca ctc cca cag cag   682
Glu Leu Glu Lys Lys Thr Glu Thr Ala Ala His Ser Leu Pro Gln Gln
 35                  40                  45                  50

aca aaa aag cct gaa tca gaa ggt tat ctt caa gaa gag aag cag aaa   730
Thr Lys Lys Pro Glu Ser Glu Gly Tyr Leu Gln Glu Glu Lys Gln Lys
                55                  60                  65

tgt tac aac gat ctc ttg gca agt gca aaa aaa gat ctt gag gtt gaa   778
Cys Tyr Asn Asp Leu Leu Ala Ser Ala Lys Lys Asp Leu Glu Val Glu
            70                  75                  80

cga caa acc ata act cag ctg agt ttt gaa ctg agt gaa ttt cga aga   826
Arg Gln Thr Ile Thr Gln Leu Ser Phe Glu Leu Ser Glu Phe Arg Arg
        85                  90                  95

aaa tat gaa gaa acc caa aaa gaa gtt cac aat tta aat cag ctg ttg   874
Lys Tyr Glu Glu Thr Gln Lys Glu Val His Asn Leu Asn Gln Leu Leu
    100                 105                 110

tat tca caa aga agg gca gat gtg caa cat ctg gaa gat gat agg cat   922
Tyr Ser Gln Arg Arg Ala Asp Val Gln His Leu Glu Asp Asp Arg His
115                 120                 125                 130
```

```
aaa aca gag aag ata caa aaa ctc agg gaa gag aat gat att gct agg    970
Lys Thr Glu Lys Ile Gln Lys Leu Arg Glu Glu Asn Asp Ile Ala Arg
                135                 140                 145

gga aaa ctt gaa gaa gag aag aag aga tcc gaa gag ctc tta tct cag    1018
Gly Lys Leu Glu Glu Glu Lys Lys Arg Ser Glu Glu Leu Leu Ser Gln
            150                 155                 160

gtc cag ttt ctt tac aca tct ctg cta aag cag caa gaa gaa caa aca    1066
Val Gln Phe Leu Tyr Thr Ser Leu Leu Lys Gln Gln Glu Glu Gln Thr
        165                 170                 175

agg gta gct ctg ttg gaa caa cag atg cag gca tgt act tta gac ttt    1114
Arg Val Ala Leu Leu Glu Gln Gln Met Gln Ala Cys Thr Leu Asp Phe
    180                 185                 190

gaa aat gaa aaa ctc gac cgt caa cat gtg cag cat caa ttg cat gta    1162
Glu Asn Glu Lys Leu Asp Arg Gln His Val Gln His Gln Leu His Val
195                 200                 205                 210

att ctt aag gag ctc cga aaa gca aga aat caa ata aca cag ttg gaa    1210
Ile Leu Lys Glu Leu Arg Lys Ala Arg Asn Gln Ile Thr Gln Leu Glu
                215                 220                 225

tcc ttg aaa cag ctt cat gag ttt gcc atc aca gag cca tta gtc act    1258
Ser Leu Lys Gln Leu His Glu Phe Ala Ile Thr Glu Pro Leu Val Thr
            230                 235                 240

ttc caa gga gag act gaa aac aga gaa aaa gtt gcc gcc tca cca aaa    1306
Phe Gln Gly Glu Thr Glu Asn Arg Glu Lys Val Ala Ala Ser Pro Lys
        245                 250                 255

agt ccc act gct gca ctc aat gaa agc ctg gtg gaa tgt ccc aag tgc    1354
Ser Pro Thr Ala Ala Leu Asn Glu Ser Leu Val Glu Cys Pro Lys Cys
    260                 265                 270

aat ata cag tat cca gcc act gag cat cgc gat ctg ctt gtc cat gtg    1402
Asn Ile Gln Tyr Pro Ala Thr Glu His Arg Asp Leu Leu Val His Val
275                 280                 285                 290

gaa tac tgt tca aag tagcaaaata agtatttgtt ttgatattaa aagattcaat    1457
Glu Tyr Cys Ser Lys
                295

actgtatttt ctgttagctt gtgggcattt tgaattatat atttcacatt ttgcataaaa 1517
ctgcctatct acctttgaca ctccagcatg ctagtgaatc atgtatcttt taggctgctg 1577
tgcatttctc ttggcagtga tacctccctg acatggttca tcatcaggct gcaatgacag 1637
aatgtggtga gcagcgtcta ctgagatact aacattttgc actgtcaaaa tacttggtga 1697
ggaaaagata gctcaggtta ttgctaatgg gttaatgcac cagcaagcaa aatattttat 1757
gttttggggg ttttgaaaaa tcaaagataa ttaaccaagg atcttaactg tgttcgcatt 1817
ttttatccaa gcacttagaa aacctacaat cctaattttg atgtccattg ttaagaggtg 1877
gtgatagata ctattttttt tttcatattg tatagcggtt attagaaaag ttggggattt 1937
tcttgatctt tattgctgct taccattgaa acttaaccca gctgtgttcc ccaactctgt 1997
tctgcgcacg aaacagtatc tgtttgaggc ataatcttaa gtggccacac acaatgtttt 2057
ctcttatgtt atctggcagt aactgtaact tgaattacat tagcacattc tgcttagcta 2117
aaattgttaa aataaacttt aataaaccca tgtagccctc tcatttgatt gacagtattt 2177
tagttatttt tggcattctt aaagctgggc aatgtaatga tcagatcttt gtttgtctga 2237
acaggtattt ttatacatgc tttttgtaaa ccaaaaactt ttaaatttct tcaggttttc 2297
taacatgctt accactgggc tactgta                                     2324
```

<210> 5
<211> 295
<212> PRT
<213> Homo Sapiens

<400> 5
Met Glu Ile Gln Leu Lys Asp Ala Leu Glu Lys Asn Gln Gln Trp Leu
 1               5                   10                  15
Val Tyr Asp Gln Gln Arg Glu Val Tyr Val Lys Gly Leu Leu Ala Lys
            20                  25                  30
Ile Phe Glu Leu Glu Lys Lys Thr Glu Thr Ala Ala His Ser Leu Pro
        35                  40                  45
Gln Gln Thr Lys Lys Pro Glu Ser Glu Gly Tyr Leu Gln Glu Glu Lys
    50                  55                  60
Gln Lys Cys Tyr Asn Asp Leu Leu Ala Ser Ala Lys Lys Asp Leu Glu
65                  70                  75                  80
Val Glu Arg Gln Thr Ile Thr Gln Leu Ser Phe Glu Leu Ser Glu Phe
                85                  90                  95
Arg Arg Lys Tyr Glu Glu Thr Gln Lys Glu Val His Asn Leu Asn Gln
            100                 105                 110
Leu Leu Tyr Ser Gln Arg Arg Ala Asp Val Gln His Leu Glu Asp Asp
        115                 120                 125
Arg His Lys Thr Glu Lys Ile Gln Lys Leu Arg Glu Glu Asn Asp Ile
    130                 135                 140
Ala Arg Gly Lys Leu Glu Glu Glu Lys Lys Arg Ser Glu Glu Leu Leu
145                 150                 155                 160
Ser Gln Val Gln Phe Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
Asp Phe Glu Asn Glu Lys Leu Asp Arg Gln His Val Gln His Gln Leu
        195                 200                 205
His Val Ile Leu Lys Glu Leu Arg Lys Ala Arg Asn Gln Ile Thr Gln
    210                 215                 220
Leu Glu Ser Leu Lys Gln Leu His Glu Phe Ala Ile Thr Glu Pro Leu
225                 230                 235                 240
Val Thr Phe Gln Gly Glu Thr Glu Asn Arg Glu Lys Val Ala Ala Ser
                245                 250                 255
Pro Lys Ser Pro Thr Ala Ala Leu Asn Glu Ser Leu Val Glu Cys Pro
            260                 265                 270
Lys Cys Asn Ile Gln Tyr Pro Ala Thr Glu His Arg Asp Leu Leu Val
        275                 280                 285
His Val Glu Tyr Cys Ser Lys
    290                 295

<210> 6
<211> 2473
<212> DNA
<213> Homo Sapiens

<220>
<221> CDS
<222> (175)...(1566)

<400> 6
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

```
tct tcc aga agt acc aaa gat tta att aaa agt aag tgg gga tcg aag     225
Ser Ser Arg Ser Thr Lys Asp Leu Ile Lys Ser Lys Trp Gly Ser Lys
                5                  10                  15

cct agt aac tcc aaa tcc gaa act aca tta gaa aaa tta aag gga gaa     273
Pro Ser Asn Ser Lys Ser Glu Thr Thr Leu Glu Lys Leu Lys Gly Glu
            20                  25                  30

att gca cac tta aag aca tca gtg gat gaa atc aca agt ggg aaa gga     321
Ile Ala His Leu Lys Thr Ser Val Asp Glu Ile Thr Ser Gly Lys Gly
        35                  40                  45

aag ctg act gat aaa gag aga cag aga ctt ttg gag aaa att cga gtc     369
Lys Leu Thr Asp Lys Glu Arg Gln Arg Leu Leu Glu Lys Ile Arg Val
    50                  55                  60                  65

ctt gag gct gag aag gag aag aat gct tat caa ctc aca gag aag gac     417
Leu Glu Ala Glu Lys Glu Lys Asn Ala Tyr Gln Leu Thr Glu Lys Asp
                    70                  75                  80

aaa gaa ata cag cga ctg aga gac caa ctg aag gcc aga tat agt act     465
Lys Glu Ile Gln Arg Leu Arg Asp Gln Leu Lys Ala Arg Tyr Ser Thr
                85                  90                  95

acc gca ttg ctt gaa cag ctg gaa gag aca acg aga gaa gga gaa agg     513
Thr Ala Leu Leu Glu Gln Leu Glu Glu Thr Thr Arg Glu Gly Glu Arg
            100                 105                 110

agg gag cag gtg ttg aaa gcc tta tct gaa gag aaa gac gta ttg aaa     561
Arg Glu Gln Val Leu Lys Ala Leu Ser Glu Glu Lys Asp Val Leu Lys
        115                 120                 125

caa cag ttg tct gct gca acc tca cga att gct gaa ctt gaa agc aaa     609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac     657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct     705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc     753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg     801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca     849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg     897
Glu Gly Tyr Leu Gln Glu Glu Lys Gln Lys Cys Tyr Asn Asp Leu Leu
                230                 235                 240
```

```
gca agt gca aaa aaa gat ctt gag gtt gaa cga caa acc ata act cag     945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa     993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca    1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa    1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag    1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca    1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa    1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac    1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc    1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag        1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
```

ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta 2473

<210> 7
<211> 464
<212> PRT
<213> Homo Sapiens

<400> 7
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg Gln Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Phe Leu Tyr
325 330 335
Thr Ser Leu Leu Lys Gln Gln Glu Glu Gln Thr Arg Val Ala Leu Leu

```
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460


<210> 8
<211> 2473
<212> DNA
<213> Homo Sapiens

<220>
<221> CDS
<222> (175)...(1566)

<400> 8
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

tct tcc aga agt acc aaa gat tta att aaa agt aag tgg gga tcg aag   225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa   273
Pro Ser Asn Ser Lys Ser Glu Thr Thr Leu Glu Lys Leu Lys Gly Glu
        20                  25                  30

att gca cac tta aag aca tca gtg gat gaa atc aca agt ggg aaa gga   321
Ile Ala His Leu Lys Thr Ser Val Asp Glu Ile Thr Ser Gly Lys Gly
    35                  40                  45

aag ctg act gat aaa gag aga cac aga ctt ttg gag aaa att cga gtc   369
Lys Leu Thr Asp Lys Glu Arg His Arg Leu Leu Glu Lys Ile Arg Val
 50                  55                  60                  65

ctt gag gct gag aag gag aag aat gct tat caa ctc aca gag aag gac   417
Leu Glu Ala Glu Lys Glu Lys Asn Ala Tyr Gln Leu Thr Glu Lys Asp
                70                  75                  80

aaa gaa ata cag cga ctg aga gac caa ctg aag gcc aga tat agt act   465
Lys Glu Ile Gln Arg Leu Arg Asp Gln Leu Lys Ala Arg Tyr Ser Thr
            85                  90                  95

acc aca ttg ctt gaa cag ctg gaa gag aca acg aga gaa gga gaa agg   513
Thr Thr Leu Leu Glu Gln Leu Glu Glu Thr Thr Arg Glu Gly Glu Arg
        100                 105                 110
```

```
agg gag cag gtg ttg aaa gcc tta tct gaa gag aaa gac gta ttg aaa      561
Arg Glu Gln Val Leu Lys Ala Leu Ser Glu Glu Lys Asp Val Leu Lys
    115                 120                 125

caa cag ttg tct gct gca acc tca cga att gct gaa ctt gaa agc aaa      609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac      657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct      705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc      753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg      801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca      849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg      897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag      945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa      993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca     1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa     1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag     1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca     1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa     1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac     1281
```

Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc    1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag        1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta                                                           2473

<210> 9
<211> 464
<212> PRT
<213> Homo Sapiens

<400> 9
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser

```
                85                  90                  95
Thr Thr Thr Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 10
<211> 2473
<212> DNA
<213> Homo Sapiens

<220>
<221> CDS
<222> (175)...(1566)

<400> 10

```
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
```

```
ctcaagaccg ttgtctcttc gatcgcttct ttggacttgg cgaccatttc agag atg    177
                                                            Met
                                                              1

tct tcc aga agt acc aaa gat tta att aaa agt aag tgg gga tcg aag    225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa    273
Pro Ser Asn Ser Lys Ser Glu Thr Thr Leu Glu Lys Leu Lys Gly Glu
        20                  25                  30

att gca cac tta aag aca tca gtg gat gaa atc aca agt ggg aaa gga    321
Ile Ala His Leu Lys Thr Ser Val Asp Glu Ile Thr Ser Gly Lys Gly
    35                  40                  45

aag ctg act gat aaa gag aga cac aga ctt ttg gag aaa att cga gtc    369
Lys Leu Thr Asp Lys Glu Arg His Arg Leu Leu Glu Lys Ile Arg Val
 50                  55                  60                  65

ctt gag gct gag aag gag aag aat gct tat caa ctc aca gag aag gac    417
Leu Glu Ala Glu Lys Glu Lys Asn Ala Tyr Gln Leu Thr Glu Lys Asp
                 70                  75                  80

aaa gaa ata cag cga ctg aga gac caa ctg aag gcc aga tat agt act    465
Lys Glu Ile Gln Arg Leu Arg Asp Gln Leu Lys Ala Arg Tyr Ser Thr
            85                  90                  95

acc gca ttg ctt gaa cag ctg gaa gag aca acg aga gaa gga gaa cgg    513
Thr Ala Leu Leu Glu Gln Leu Glu Glu Thr Thr Arg Glu Gly Glu Arg
        100                 105                 110

agg gag cag gtg ttg aaa gcc tta tct gaa gag aaa gac gta ttg aaa    561
Arg Glu Gln Val Leu Lys Ala Leu Ser Glu Glu Lys Asp Val Leu Lys
    115                 120                 125

caa cag ttg tct gct gca acc tca cga att gct gaa ctt gaa agc aaa    609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac    657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct    705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc    753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg    801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca    849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg    897
```

```
Glu Gly Tyr Leu Gln Glu Glu Lys Gln Lys Cys Tyr Asn Asp Leu Leu
            230                 235                 240

gca agt gca aaa aaa gat ctt gag gtt gaa cga caa acc ata act cag     945
Ala Ser Ala Lys Lys Asp Leu Glu Val Glu Arg Gln Thr Ile Thr Gln
        245                 250                 255

ctg agt ttt gaa ctg agt gaa ttt cga aga aaa tat gaa gaa acc caa     993
Leu Ser Phe Glu Leu Ser Glu Phe Arg Arg Lys Tyr Glu Glu Thr Gln
    260                 265                 270

aaa gaa gtt cac aat tta aat cag ctg ttg tat tca caa aga agg gca    1041
Lys Glu Val His Asn Leu Asn Gln Leu Leu Tyr Ser Gln Arg Arg Ala
275                 280                 285

gat gtg caa cat ctg gaa gat gat agg cat aaa aca gag aag ata caa    1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag    1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca    1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa    1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac    1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc    1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag        1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
```

```
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta                                                           2473
```

<210> 11
<211> 464
<212> PRT
<213> Homo Sapiens

<400> 11

```
Met Ser Ser Arg Ser Thr Lys Asp Leu Ile Lys Ser Lys Trp Gly Ser
 1              5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
```

```
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460


<210> 12
<211> 2473
<212> DNA
<213> Homo Sapiens

<220>
<221> CDS
<222> (175)...(1566)

<400> 12
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

tct tcc aga agt acc aaa gat tta att aaa agt aag tgg gga tcg aag   225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa   273
Pro Ser Asn Ser Lys Ser Glu Thr Thr Leu Glu Lys Leu Lys Gly Glu
        20                  25                  30

att gca cac tta aag aca tca gtg gat gaa atc aca agt ggg aaa gga   321
Ile Ala His Leu Lys Thr Ser Val Asp Glu Ile Thr Ser Gly Lys Gly
    35                  40                  45

aag ctg act gat aaa gag aga cac aga ctt ttg gag aaa att cga gtc   369
Lys Leu Thr Asp Lys Glu Arg His Arg Leu Leu Glu Lys Ile Arg Val
 50                  55                  60                  65

ctt gag gct gag aag gag aag aat gct tat caa ctc aca gag aag gac   417
Leu Glu Ala Glu Lys Glu Lys Asn Ala Tyr Gln Leu Thr Glu Lys Asp
                70                  75                  80

aaa gaa ata cag cga ctg aga gac caa ctg aag gcc aga tat agt act   465
Lys Glu Ile Gln Arg Leu Arg Asp Gln Leu Lys Ala Arg Tyr Ser Thr
            85                  90                  95

acc gca ttg ctt gaa cag ctg gaa gag aca acg aga gaa gga gaa agg   513
```

```
Thr Ala Leu Leu Glu Gln Leu Glu Glu Thr Thr Arg Glu Gly Glu Arg
        100                 105                 110

agg gag cag gtg ttg aaa gcc tta tct gaa gag aaa gac gta ttg aaa      561
Arg Glu Gln Val Leu Lys Ala Leu Ser Glu Glu Lys Asp Val Leu Lys
    115                 120                 125

caa cag ttg tct gct gca acc tca cga att gct gaa ctt gaa agc aaa      609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac      657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct      705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc      753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg      801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca      849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg      897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag      945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa      993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca      1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa      1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag      1137
Lys Leu Arg Glu Glu Asn Asp Ile Ala Arg Gly Lys Leu Glu Glu Glu
                310                 315                 320

aag aag aga tcc gaa gag ctc tta tct cag gtc cag tct ctt tac aca      1185
Lys Lys Arg Ser Glu Glu Leu Leu Ser Gln Val Gln Ser Leu Tyr Thr
            325                 330                 335

tct ctg cta aag cag caa gaa gaa caa aca agg gta gct ctg ttg gaa      1233
Ser Leu Leu Lys Gln Gln Glu Glu Gln Thr Arg Val Ala Leu Leu Glu
```

```
        340                 345                 350

caa cag atg cag gca tgt act tta gac ttt gaa aat gaa aaa ctc gac    1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc    1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag        1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta                                                           2473

<210> 13
<211> 464
<212> PRT
<213> Homo Sapiens

<400> 13
Met Ser Ser Arg Ser Thr Lys Asp Leu Ile Lys Ser Lys Trp Gly Ser
1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
```

```
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Ser Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 14
<211> 2473
<212> DNA
<213> Homo Sapiens

<220>
<221> CDS
<222> (175)...(1566)

<400> 14
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg 177
Met
1

tct tcc aga agt acc aaa gat tta att aaa agt aag tgg gga tcg aag 225
Ser Ser Arg Ser Thr Lys Asp Leu Ile Lys Ser Lys Trp Gly Ser Lys
5 10 15

cct agt aac tcc aaa tcc gaa act aca tta gaa aaa tta aag gga gaa 273
Pro Ser Asn Ser Lys Ser Glu Thr Thr Leu Glu Lys Leu Lys Gly Glu
20 25 30

att gca cac tta aag aca tca gtg gat gaa atc aca agt ggg aaa gga 321
Ile Ala His Leu Lys Thr Ser Val Asp Glu Ile Thr Ser Gly Lys Gly
35 40 45

aag ctg act gat aaa gag aga cac aga ctt ttg gag aaa att cga gtc 369
Lys Leu Thr Asp Lys Glu Arg His Arg Leu Leu Glu Lys Ile Arg Val
50 55 60 65

ctt gag gct gag aag gag aag aat gct tat caa ctc aca gag aag gac 417
Leu Glu Ala Glu Lys Glu Lys Asn Ala Tyr Gln Leu Thr Glu Lys Asp
70 75 80

aaa gaa ata cag cga ctg aga gac caa ctg aag gcc aga tat agt act 465
Lys Glu Ile Gln Arg Leu Arg Asp Gln Leu Lys Ala Arg Tyr Ser Thr
85 90 95

acc gca ttg ctt gaa cag ctg gaa gag aca acg aga gaa gga gaa agg 513
Thr Ala Leu Leu Glu Gln Leu Glu Glu Thr Thr Arg Glu Gly Glu Arg
100 105 110

agg gag cag gtg ttg aaa gcc tta tct gaa gag aaa gac gta ttg aaa 561
Arg Glu Gln Val Leu Lys Ala Leu Ser Glu Glu Lys Asp Val Leu Lys
115 120 125

caa cag ttg tct gct gca acc tca cga att gct gaa ctt gaa agc aaa 609
Gln Gln Leu Ser Ala Ala Thr Ser Arg Ile Ala Glu Leu Glu Ser Lys
130 135 140 145

acc aat aca ctc cgt tta tca cag act gtg gct cca aac tgc ttc aac 657
Thr Asn Thr Leu Arg Leu Ser Gln Thr Val Ala Pro Asn Cys Phe Asn
150 155 160

tca tca ata aat aat att cat gaa atg gaa ata cag ctg aaa gat gct 705
Ser Ser Ile Asn Asn Ile His Glu Met Glu Ile Gln Leu Lys Asp Ala
165 170 175

ctg gag aaa aat cag cag tgg ctc gtg tat gat cag cag cgg gaa gtc 753
Leu Glu Lys Asn Gln Gln Trp Leu Val Tyr Asp Gln Gln Arg Glu Val
180 185 190

tat gta aaa gga ctt tta gca aag atc ttt gag ttg gaa aag aaa acg 801
Tyr Val Lys Gly Leu Leu Ala Lys Ile Phe Glu Leu Glu Lys Lys Thr
195 200 205

gaa aca gct gct cat tca ctc cca cag cag aca aaa aag cct gaa tca 849
Glu Thr Ala Ala His Ser Leu Pro Gln Gln Thr Lys Lys Pro Glu Ser

```
210                 215                 220                 225

gaa ggt tat ctt caa gaa gag aag cag aaa tgt tac aac gat ctc ttg     897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag     945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa     993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca     1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa     1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag     1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca     1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa     1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac     1281
Gln Gln Met Gln Ala Cys Thr Leu Asp Phe Glu Asn Glu Lys Leu Asp
    355                 360                 365

cgt caa cat gtg cag cat caa ttg ctt gta att ctt aag gag ctc cga     1329
Arg Gln His Val Gln His Gln Leu Leu Val Ile Leu Lys Glu Leu Arg
370                 375                 380                 385

aaa gca aga aat caa ata aca cag ttg gaa tcc ttg aaa cag ctt cat     1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa     1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc     1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc     1521
Asn Glu Ser Leu Val Glu Cys Pro Lys Cys Asn Ile Gln Tyr Pro Ala
    435                 440                 445

act gag cat cgc gat ctg ctt gtc cat gtg gaa tac tgt tca aag         1566
Thr Glu His Arg Asp Leu Leu Val His Val Glu Tyr Cys Ser Lys
450                 455                 460
```

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta 2473

<210> 15
<211> 464
<212> PRT
<213> Homo Sapiens

<400> 15
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile

```
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu Leu Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 16
<211> 2473
<212> DNA
<213> Homo Sapiens

<220>
<221> CDS
<222> (175)...(1566)

<400> 16

```
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

tct tcc aga agt acc aaa gat tta att aaa agt aag tgg gga tcg aag   225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa   273
Pro Ser Asn Ser Lys Ser Glu Thr Thr Leu Glu Lys Leu Lys Gly Glu
        20                  25                  30

att gca cac tta aag aca tca gtg gat gaa atc aca agt ggg aaa gga   321
Ile Ala His Leu Lys Thr Ser Val Asp Glu Ile Thr Ser Gly Lys Gly
    35                  40                  45

aag ctg act gat aaa gag aga cac aga ctt ttg gag aaa att cga gtc   369
Lys Leu Thr Asp Lys Glu Arg His Arg Leu Leu Glu Lys Ile Arg Val
50                  55                  60                  65

ctt gag gct gag aag gag aag aat gct tat caa ctc aca gag aag gac   417
Leu Glu Ala Glu Lys Glu Lys Asn Ala Tyr Gln Leu Thr Glu Lys Asp
                70                  75                  80

aaa gaa ata cag cga ctg aga gac caa ctg aag gcc aga tat agt act   465
Lys Glu Ile Gln Arg Leu Arg Asp Gln Leu Lys Ala Arg Tyr Ser Thr
```

```
                85                  90                  95

acc gca ttg ctt gaa cag ctg gaa gag aca acg aga gaa gga gaa agg     513
Thr Ala Leu Leu Glu Gln Leu Glu Glu Thr Thr Arg Glu Gly Glu Arg
        100                 105                 110

agg gag cag gtg ttg aaa gcc tta tct gaa gag aaa gac gta ttg aaa     561
Arg Glu Gln Val Leu Lys Ala Leu Ser Glu Glu Lys Asp Val Leu Lys
    115                 120                 125

caa cag ttg tct gct gca acç tca cga att gct gaa ctt gaa agc aaa     609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac     657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct     705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc     753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg     801
Tyr Val Lys Gly Leu Leu Ala Lys Ile Phe Glu Leu Glu Lys Lys Thr
    195                 200                 205

gaa aca gct gct cat tca ctc cca cag cag aca aaa aag cct gaa tca     849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg     897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag     945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa     993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca     1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa     1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag     1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca     1185
Lys Lys Arg Ser Glu Glu Leu Leu Ser Gln Val Gln Phe Leu Tyr Thr
            325                 330                 335
```

```
tct ctg cta aag cag caa gaa gaa caa aca agg gta gct ctg ttg gaa    1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac    1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gga agc ctg gtg gaa tgt ccc aag tgc aat ata cag tat cca gcc    1521
Asn Gly Ser Leu Val Glu Cys Pro Lys Cys Asn Ile Gln Tyr Pro Ala
    435                 440                 445

act gag cat cgc gat ctg ctt gtc cat gtg gaa tac tgt tca aag        1566
Thr Glu His Arg Asp Leu Leu Val His Val Glu Tyr Cys Ser Lys
450                 455                 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gttttggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta                                                           2473
```

<210> 17
<211> 464
<212> PRT
<213> Homo Sapiens

<400> 17

```
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
```

```
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Gly Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 18
<211> 2473
<212> DNA
<213> Homo Sapiens

<220>

```
<221> CDS
<222> (175)...(1566)

<400> 18
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agag atg   177
                                                            Met
                                                             1

tct tcc aga agt acc aaa gat tta att aaa agt aag tgg gga tcg aag   225
Ser Ser Arg Ser Thr Lys Asp Leu Ile Lys Ser Lys Trp Gly Ser Lys
            5                  10                  15

cct agt aac tcc aaa tcc gaa act aca tta gaa aaa tta aag gga gaa   273
Pro Ser Asn Ser Lys Ser Glu Thr Thr Leu Glu Lys Leu Lys Gly Glu
         20                  25                  30

att gca cac tta aag aca tca gtg gat gaa atc aca agt ggg aaa gga   321
Ile Ala His Leu Lys Thr Ser Val Asp Glu Ile Thr Ser Gly Lys Gly
     35                  40                  45

aag ctg act gat aaa gag aga cac aga ctt ttg gag aaa att cga gtc   369
Lys Leu Thr Asp Lys Glu Arg His Arg Leu Leu Glu Lys Ile Arg Val
 50                  55                  60                  65

ctt gag gct gag aag gag aag aat gct tat caa ctc aca gag aag gac   417
Leu Glu Ala Glu Lys Glu Lys Asn Ala Tyr Gln Leu Thr Glu Lys Asp
                 70                  75                  80

aaa gaa ata cag cga ctg aga gac caa ctg aag gcc aga tat agt act   465
Lys Glu Ile Gln Arg Leu Arg Asp Gln Leu Lys Ala Arg Tyr Ser Thr
             85                  90                  95

acc gca ttg ctt gaa cag ctg gaa gag aca acg aga gaa gga gaa agg   513
Thr Ala Leu Leu Glu Gln Leu Glu Glu Thr Thr Arg Glu Gly Glu Arg
        100                 105                 110

agg gag cag gtg ttg aaa gcc tta tct gaa gag aaa gac gta ttg aaa   561
Arg Glu Gln Val Leu Lys Ala Leu Ser Glu Glu Lys Asp Val Leu Lys
    115                 120                 125

caa cag ttg tct gct gca acc tca cga att gct gaa ctt gaa agc aaa   609
Gln Gln Leu Ser Ala Ala Thr Ser Arg Ile Ala Glu Leu Glu Ser Lys
130                 135                 140                 145

acc aat aca ctc cgt tta tca cag act gtg gct cca aac tgc ttc aac   657
Thr Asn Thr Leu Arg Leu Ser Gln Thr Val Ala Pro Asn Cys Phe Asn
                150                 155                 160

tca tca ata aat aat att cat gaa atg gaa ata cag ctg aaa gat gct   705
Ser Ser Ile Asn Asn Ile His Glu Met Glu Ile Gln Leu Lys Asp Ala
            165                 170                 175

ctg gag aaa aat cag cag tgg ctc gtg tat gat cag cag cgg gaa gtc   753
Leu Glu Lys Asn Gln Gln Trp Leu Val Tyr Asp Gln Gln Arg Glu Val
        180                 185                 190

tat gta aaa gga ctt tta gca aag atc ttt gag ttg gaa aag aaa acg   801
Tyr Val Lys Gly Leu Leu Ala Lys Ile Phe Glu Leu Glu Lys Lys Thr
    195                 200                 205
```

```
gaa aca gct gct cat tca ctc cca cag cag aca aaa aag cct gaa tca    849
Glu Thr Ala Ala His Ser Leu Pro Gln Gln Thr Lys Lys Pro Glu Ser
210                 215                 220                 225

gaa ggt tat ctt caa gaa gag aag cag aaa tgt tac aac gat ctc ttg    897
Glu Gly Tyr Leu Gln Glu Glu Lys Gln Lys Cys Tyr Asn Asp Leu Leu
                230                 235                 240

gca agt gca aaa aaa gat ctt gag gtt gaa cga caa acc ata act cag    945
Ala Ser Ala Lys Lys Asp Leu Glu Val Glu Arg Gln Thr Ile Thr Gln
            245                 250                 255

ctg agt ttt gaa ctg agt gaa ttt cga aga aaa tat gaa gaa acc caa    993
Leu Ser Phe Glu Leu Ser Glu Phe Arg Arg Lys Tyr Glu Glu Thr Gln
        260                 265                 270

aaa gaa gtt cac aat tta aat cag ctg ttg tat tca caa aga agg gca    1041
Lys Glu Val His Asn Leu Asn Gln Leu Leu Tyr Ser Gln Arg Arg Ala
    275                 280                 285

gat gtg caa cat ctg gaa gat gat agg cat aaa aca gag aag ata caa    1089
Asp Val Gln His Leu Glu Asp Asp Arg His Lys Thr Glu Lys Ile Gln
290                 295                 300                 305

aaa ctc agg gaa gag aat gat att gct agg gga aaa ctt gaa gaa gag    1137
Lys Leu Arg Glu Glu Asn Asp Ile Ala Arg Gly Lys Leu Glu Glu Glu
                310                 315                 320

aag aag aga tcc gaa gag ctc tta tct cag gtc cag ttt ctt tac aca    1185
Lys Lys Arg Ser Glu Glu Leu Leu Ser Gln Val Gln Phe Leu Tyr Thr
            325                 330                 335

tct ctg cta aag cag caa gaa gaa caa aca agg gta gct ctg ttg gaa    1233
Ser Leu Leu Lys Gln Gln Glu Glu Gln Thr Arg Val Ala Leu Leu Glu
        340                 345                 350

caa cag atg cag gca tgt act tta gac ttt gaa aat gaa aaa ctc gac    1281
Gln Gln Met Gln Ala Cys Thr Leu Asp Phe Glu Asn Glu Lys Leu Asp
    355                 360                 365

cgt caa cat gtg cag cat caa ttg cat gta att ctt aag gag ctc cga    1329
Arg Gln His Val Gln His Gln Leu His Val Ile Leu Lys Glu Leu Arg
370                 375                 380                 385

aaa gca aga aat caa ata aca cag ttg gaa tcc ttg aaa cag ctt cat    1377
Lys Ala Arg Asn Gln Ile Thr Gln Leu Glu Ser Leu Lys Gln Leu His
                390                 395                 400

gag ttt gcc atc aca gag cca tta gtc act ttc caa gga gag act gaa    1425
Glu Phe Ala Ile Thr Glu Pro Leu Val Thr Phe Gln Gly Glu Thr Glu
            405                 410                 415

aac aga gaa aaa gtt gcc gcc tca cca aaa agt ccc act gct gca ctc    1473
Asn Arg Glu Lys Val Ala Ala Ser Pro Lys Ser Pro Thr Ala Ala Leu
        420                 425                 430

aat gaa agc ctg gtg gaa tgt ccc aag tgc aat ata cag tat cca gcc    1521
Asn Glu Ser Leu Val Glu Cys Pro Lys Cys Asn Ile Gln Tyr Pro Ala
    435                 440                 445
```

act gag cat cgc gat ctg ctt gtc cat gtg gaa tac tgt tca aag 1566
Thr Glu His Arg Asp Leu Leu Val His Val Glu Tyr Cys Ser Lys
450 455 460

tagcaaaata agtatttgtt ttgatattaa aagattcaat actgtatttt ctgttagctt 1626
gtgggcattt tgaattatat atttcacatt ttgcataaaa ctgcctatct acctttgaca 1686
ctccagcatg ctagtgaatc atgtatcttt taggctgctg tgcatttctc ttggcagtga 1746
tacctccctg acatggttca tcatcaggct gcaatgacag aatgtggtga gcagcgtcta 1806
ctgagatact aacattttgc actgtcaaaa tacttggtga ggaaaagata gctcaggtta 1866
ttgctaatgg gttaatgcac cagcaagcaa aatattttat gtttcggggg ttttgaaaaa 1926
tcaaagataa ttaaccaagg atcttaactg tgttcgcatt ttttatccaa gcacttagaa 1986
aacctacaat cctaattttg atgtccattg ttaagaggtg gtgatagata ctattttttt 2046
tttcatattg tatagcggtt attagaaaag ttggggattt tcttgatctt tattgctgct 2106
taccattgaa acttaaccca gctgtgttcc ccaactctgt tctgcgcacg aaacagtatc 2166
tgtttgaggc ataatcttaa gtggccacac acaatgtttt ctcttatgtt atctggcagt 2226
aactgtaact tgaattacat tagcacattc tgcttagcta aaattgttaa aataaacttt 2286
aataaaccca tgtagccctc tcatttgatt gacagtattt tagttatttt tggcattctt 2346
aaagctgggc aatgtaatga tcagatcttt gtttgtctga acaggtattt ttatacatgc 2406
tttttgtaaa ccaaaaactt ttaaatttct tcaggttttc taacatgctt accactgggc 2466
tactgta 2473

<210> 19
<211> 464
<212> PRT
<213> Homo Sapiens

<400> 19
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270

Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Phe Leu Tyr
325 330 335
Thr Ser Leu Leu Lys Gln Gln Glu Glu Gln Thr Arg Val Ala Leu Leu
340 345 350
Glu Gln Gln Met Gln Ala Cys Thr Leu Asp Phe Glu Asn Glu Lys Leu
355 360 365
Asp Arg Gln His Val Gln His Gln Leu His Val Ile Leu Lys Glu Leu
370 375 380
Arg Lys Ala Arg Asn Gln Ile Thr Gln Leu Glu Ser Leu Lys Gln Leu
385 390 395 400
His Glu Phe Ala Ile Thr Glu Pro Leu Val Thr Phe Gln Gly Glu Thr
405 410 415
Glu Asn Arg Glu Lys Val Ala Ala Ser Pro Lys Ser Pro Thr Ala Ala
420 425 430
Leu Asn Glu Ser Leu Val Glu Cys Pro Lys Cys Asn Ile Gln Tyr Pro
435 440 445
Ala Thr Glu His Arg Asp Leu Leu Val His Val Glu Tyr Cys Ser Lys
450 455 460

<210> 20
<211> 464
<212> PRT
<213> Homo Sapiens

<400> 20
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu

```
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460


<210> 21
<211> 295
<212> PRT
<213> Homo Sapiens

<400> 21
Met Glu Ile Gln Leu Lys Asp Ala Leu Glu Lys Asn Gln Gln Trp Leu
1               5                   10                  15
Val Tyr Asp Gln Gln Arg Glu Val Tyr Val Lys Gly Leu Leu Ala Lys
            20                  25                  30
Ile Phe Glu Leu Glu Lys Lys Thr Glu Thr Ala Ala His Ser Leu Pro
        35                  40                  45
Gln Gln Thr Lys Lys Pro Glu Ser Glu Gly Tyr Leu Gln Glu Glu Lys
    50                  55                  60
Gln Lys Cys Tyr Asn Asp Leu Leu Ala Ser Ala Lys Lys Asp Leu Glu
65                  70                  75                  80
Val Glu Arg Gln Thr Ile Thr Gln Leu Ser Phe Glu Leu Ser Glu Phe
                85                  90                  95
Arg Arg Lys Tyr Glu Glu Thr Gln Lys Glu Val His Asn Leu Asn Gln
            100                 105                 110
Leu Leu Tyr Ser Gln Arg Arg Ala Asp Val Gln His Leu Glu Asp Asp
        115                 120                 125
Arg His Lys Thr Glu Lys Ile Gln Lys Leu Arg Glu Glu Asn Asp Ile
    130                 135                 140
Ala Arg Gly Lys Leu Glu Glu Glu Lys Lys Arg Ser Glu Glu Leu Leu
145                 150                 155                 160
Ser Gln Val Gln Phe Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
```

Asp Phe Glu Asn Glu Lys Leu Asp Arg Gln His Val Gln His Gln Leu
195 200 205
His Val Ile Leu Lys Glu Leu Arg Lys Ala Arg Asn Gln Ile Thr Gln
210 215 220
Leu Glu Ser Leu Lys Gln Leu His Glu Phe Ala Ile Thr Glu Pro Leu
225 230 235 240
Val Thr Phe Gln Gly Glu Thr Glu Asn Arg Glu Lys Val Ala Ala Ser
245 250 255
Pro Lys Ser Pro Thr Ala Ala Leu Asn Glu Ser Leu Val Glu Cys Pro
260 265 270
Lys Cys Asn Ile Gln Tyr Pro Ala Thr Glu His Arg Asp Leu Leu Val
275 280 285
His Val Glu Tyr Cys Ser Lys
290 295

<210> 22
<211> 464
<212> PRT
<213> Homo Sapiens

<400> 22
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg Gln Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu

```
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 23
<211> 464
<212> PRT
<213> Homo Sapiens

<400> 23

```
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Thr Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
```

Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Phe Leu Tyr
325 330 335
Thr Ser Leu Leu Lys Gln Gln Glu Glu Gln Thr Arg Val Ala Leu Leu
340 345 350
Glu Gln Gln Met Gln Ala Cys Thr Leu Asp Phe Glu Asn Glu Lys Leu
355 360 365
Asp Arg Gln His Val Gln His Gln Leu His Val Ile Leu Lys Glu Leu
370 375 380
Arg Lys Ala Arg Asn Gln Ile Thr Gln Leu Glu Ser Leu Lys Gln Leu
385 390 395 400
His Glu Phe Ala Ile Thr Glu Pro Leu Val Thr Phe Gln Gly Glu Thr
405 410 415
Glu Asn Arg Glu Lys Val Ala Ala Ser Pro Lys Ser Pro Thr Ala Ala
420 425 430
Leu Asn Glu Ser Leu Val Glu Cys Pro Lys Cys Asn Ile Gln Tyr Pro
435 440 445
Ala Thr Glu His Arg Asp Leu Leu Val His Val Glu Tyr Cys Ser Lys
450 455 460

<210> 24
<211> 464
<212> PRT
<213> Homo Sapiens

<400> 24
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu

225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Ser Leu Tyr
325 330 335
Thr Ser Leu Leu Lys Gln Gln Glu Glu Gln Thr Arg Val Ala Leu Leu
340 345 350
Glu Gln Gln Met Gln Ala Cys Thr Leu Asp Phe Glu Asn Glu Lys Leu
355 360 365
Asp Arg Gln His Val Gln His Gln Leu His Val Ile Leu Lys Glu Leu
370 375 380
Arg Lys Ala Arg Asn Gln Ile Thr Gln Leu Glu Ser Leu Lys Gln Leu
385 390 395 400
His Glu Phe Ala Ile Thr Glu Pro Leu Val Thr Phe Gln Gly Glu Thr
405 410 415
Glu Asn Arg Glu Lys Val Ala Ala Ser Pro Lys Ser Pro Thr Ala Ala
420 425 430
Leu Asn Glu Ser Leu Val Glu Cys Pro Lys Cys Asn Ile Gln Tyr Pro
435 440 445
Ala Thr Glu His Arg Asp Leu Leu Val His Val Glu Tyr Cys Ser Lys
450 455 460

<210> 25
<211> 464
<212> PRT
<213> Homo Sapiens

<400> 25
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190

```
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu Leu Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 26
<211> 464
<212> PRT
<213> Homo Sapiens

<400> 26

```
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
```

```
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Gly Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 27
<211> 2203
<212> DNA
<213> Homo Sapiens

<400> 27

```
gaaattgcac acttaaagac atcagtggat gaaatcacaa gtgggaaagg aaagctgact 60
gataaagaga gacacagact tttggagaaa attcgagtcc ttgaggctga gaaggagaag 120
aatgcttatc aactcacaga gaaggacaaa gaaatacagc gactgagaga ccaactgaag 180
gccagatata gtactaccgc attgcttgaa cagctggaag agacaacgag agaaggagaa 240
aggagggagc aggtgttgaa agccttatct gaagagaaag acgtattgaa acaacagttg 300
tctgctgcaa cctcacgaat tgctgaactt gaaagcaaaa ccaatacact ccgtttatca 360
cagactgtgg ctccaaactg cttcaactca tcaataaata atattcatga aatggaaata 420
cagctgaaag atgctctgga gaaaaatcag cagtggctcg tgtatgatca gcagcgggaa 480
gtctatgtaa aggacttttt agcaaagatc tttgagttgg aaaagaaaac ggaaacagct 540
gctcattcac tcccacagca gacaaaaaag cctgaatcag aaggttatct tcaagaagag 600
aagcagaaat gttacaacga tctcttggca agtgcaaaaa aagatcttga ggttgaacga 660
caaaccataa ctcagctgag ttttgaactg agtgaatttc gaagaaaata tgaagaaacc 720
caaaaagaag ttcacaattt aaatcagctg ttgtattcac aaagaagggc agatgtgcaa 780
catctggaag atgataggca taaaacagag aagatacaaa aactcaggga agagaatgat 840
```

attgctaggg gaaaacttga agaagagaag aagagatccg aagagctctt atctcaggtc 900
cagtttcttt acacatctct gctaaagcag caagaagaac aaacaagggt agctctgttg 960
gaacaacaga tgcaggcatg tactttagac tttgaaaatg aaaaactcga ccgtcaacat 1020
gtgcagcatc aattgcatgt aattcttaag gagctccgaa aagcaagaaa tcaaataaca 1080
cagttggaat ccttgaaaca gcttcatgag tttgccatca cagagccatt agtcactttc 1140
caaggagaga ctgaaaacag agaaaaagtt gccgcctcac caaaaagtcc cactgctgca 1200
ctcaatgaaa gcctggtgga atgtcccaag tgcaatatac agtatccagc cactgagcat 1260
cgcgatctgc ttgtccatgt ggaatactgt tcaaagtagc aaaataagta tttgttttga 1320
tattaaaaga ttcaatactg tattttctgt tagcttgtgg gcattttgaa ttatatattt 1380
cacattttgc ataaaactgc ctatctacct ttgacactcc agcatgctag tgaatcatgt 1440
atcttttagg ctgctgtgca tttctcttgg cagtgatacc tccctgacat ggttcatcat 1500
caggctgcaa tgacagaatg tggtgagcag cgtctactga gatactaaca ttttgcactg 1560
tcaaaatact tggtgaggaa aagatagctc aggttattgc taatgggtta atgcaccagc 1620
aagcaaaata ttttatgttt tggggtttt gaaaaatcaa agataattaa ccaaggatct 1680
taactgtgtt cgcatttttt atccaagcac ttagaaaacc tacaatccta attttgatgt 1740
ccattgttaa gaggtggtga tagatactat tttttttttc atattgtata gcggttatta 1800
gaaaagttgg ggattttctt gatctttatt gctgcttacc attgaaactt aacccagctg 1860
tgttccccaa ctctgttctg cgcacgaaac agtatctgtt tgaggcataa tcttaagtgg 1920
ccacacacaa tgttttctct tatgttatct ggcagtaact gtaacttgaa ttacattagc 1980
acattctgct tagctaaaat tgttaaaata aactttaata aacccatgta gccctctcat 2040
ttgattgaca gtattttagt tatttttggc attcttaaag ctgggcaatg taatgatcag 2100
atctttgttt gtctgaacag gtatttttat acatgctttt tgtaaaccaa aaacttttaa 2160
atttcttcag gttttctaac atgcttacca ctgggctact gta 2203

<210> 28
<211> 2200
<212> DNA
<213> Homo Sapiens

<400> 28
gaaattgcac acttaaagac atcagtggat gaaatcacaa gtgggaaagg aaagctgact 60
gataaagaga gacagagact tttggagaaa attcgagtcc ttgaggctga gaaggagaag 120
aatgcttatc aactcacaga gaaggacaaa gaaatacagc gactgagaga ccaactgaag 180
gccagatata gtactaccgc attgcttgaa cagctggaag agacaacgag agaaggagaa 240
aggagggagc aggtgttgaa agccttatct gaagagaaag acgtattgaa acaacagttg 300
tctgctgcaa cctcacgaat tgctgaactt gaaagcaaaa ccaatacact ccgtttatca 360
cagactgtgg ctccaaactg cttcaactca tcaataaata atattcatga aatggaaata 420
cagctgaaag atgctctgga gaaaaatcag cagtggctcg tgtatgatca gcagcgggaa 480
gtctatgtaa aaggactttt agcaaagatc tttgagttgg aaaagaaaac ggaaacagct 540
gctcattcac tcccacagca gacaaaaaag cctgaatcag aaggttatct tcaagaagag 600
aagcagaaat gttacaacga tctcttggca agtgcaaaaa aagatcttga ggttgaacga 660
caaaccataa ctcagctgag ttttgaactg agtgaatttc gaagaaaata tgaagaaacc 720
caaaaagaag ttcacaattt aaatcagctg ttgtattcac aaagaagggc agatgtgcaa 780
catctggaag atgataggca taaaacagag aagatacaaa aactcaggga agagaatgat 840
attgctaggg gaaaacttga agaagagaag aagagatccg aagagctctt atctcaggtc 900
cagtctcttt acacatctct gctaaagcag caagaagaac aaacaagggt agctctgttg 960
gaacaacaga tgcaggcatg tactttagac tttgaaaatg aaaaactcga ccgtcaacat 1020
gtgcagcatc aattgcatgt aattcttaag gagctccgaa aagcaagaaa aaataacaca 1080
gttggaatcc ttgaaacagc ttcatgagtt tgccatcaca gagccattag tcactttcca 1140
aggagagact gaaaacagag aaaaagttgc cgcctcacca aaaagtccca ctgctgcact 1200
caatggaagc ctggtggaat gtcccaagtg caatatacag tatccagcca ctgagcatcg 1260
cgatctgctt gtccatgtgg aatactgttc aaagtagcaa aataagtatt tgttttgata 1320
ttaaaagatt caatactgta ttttctgtta gcttgtgggc attttgaatt atatatttca 1380
cattttgcat aaaactgcct atctaccttt gacactccag catgctagtg aatcatgtat 1440
cttttaggct gctgtgcatt tctcttggca gtgatacctc cctgacatgg ttcatcatca 1500
ggctgcaatg acagaatgtg gtgagcagcg tctactgaga tactaacatt ttgcactgtc 1560
aaaatacttg gtgaggaaaa gatagctcag gttattgcta atgggttaat gcaccagcaa 1620
gcaaaatatt ttatgtttcg gggttttga aaaatcaaag ataattaacc aaggatctta 1680
actgtgttcg catttttat ccaagcactt agaaaaccta caatcctaat tttgatgtcc 1740
attgttaaga ggtggtgata gatactattt tttttcata ttgtatagcg gttattagaa 1800
aagttgggga ttttcttgat ctttattgct gcttaccatt gaaacttaac ccagctgtgt 1860

tccccaactc tgttctgcgc acgaaacagt atctgtttga ggcataatct taagtggcca 1920
cacacaatgt tttctcttat gttatctggc agtaactgta acttgaatta cattagcaca 1980
ttctgcttag ctaaaattgt taaaataaac tttaataaac ccatgtagcc ctctcatttg 2040
attgacagta ttttagttat ttttggcatt cttaaagctg ggcaatgtaa tgatcagatc 2100
tttgtttgtc tgaacaggta tttttataca tgctttttgt aaaccaaaaa cttttaaatt 2160
tcttcaggtt ttctaacatg cttaccactg ggctactgta 2200

<210> 29
<211> 2472
<212> DNA
<213> Homo Sapiens

<400> 29
ggaccgccag ggagggcagg tcagtgggca gatcgcgtcc gcgggattca atctctgccc 60
gctctgataa cagtcctttt ccctggcgct cacttcgtgc ctggcacccg gctgggcgcc 120
tcaagaccgt tgtctcttcg atcgcttctt tggacttggc gaccatttca gagatgtctt 180
ccagaagtac caaagattta attaaaagta agtggggatc gaagcctagt aactccaaat 240
ccgaaactac attagaaaaa ttaaagggag aaattgcaca cttaaagaca tcagtggatg 300
aaatcacaag tgggaaagga aagctgactg ataaagagag acacagactt ttggagaaaa 360
ttcgagtcct tgaggctgag aaggagaaga atgcttatca actcacagag aaggacaaag 420
aaatacagcg actgagagac caactgaagg ccagatatag tactaccgca ttgcttgaac 480
agctggaaga gacaacgaga gaaggagaaa ggagggagca ggtgttgaaa gccttatctg 540
aagagaaaga cgtattgaaa caacagttgt ctgctgcaac ctcacgaatt gctgaacttg 600
aaagcaaaac caatacactc cgtttatcac agactgtggc tccaaactgc ttcaactcat 660
caataaataa tattcatgaa atggaaatac agctgaaaga tgctctggag aaaaatcagc 720
agtggctcgt gtatgatcag cagcgggaag tctatgtaaa aggactttta gcaaagatct 780
ttgagttgga aaagaaaacg gaaacagctg ctcattcact cccacagcag acaaaaaagc 840
ctgaatcaga aggttatctt caagaagaga agcagaaatg ttacaacgat ctcttggcaa 900
gtgcaaaaaa agatcttgag gttgaacgac aaaccataac tcagctgagt tttgaactga 960
gtgaatttcg aagaaaatat gaagaaaccc aaaaagaagt tcacaattta aatcagctgt 1020
tgtattcaca aagaagggca gatgtgcaac atctggaaga tgataggcat aaaacagaga 1080
agatacaaaa actcagggaa gagaatgata ttgctagggg aaaacttgaa gaagagaaga 1140
agagatccga agagctctta tctcaggtcc agtttcttta cacatctctg ctaaagcagc 1200
aagaagaaca aacaagggta gctctgttgg aacaacagat gcaggcatgt actttagact 1260
ttgaaaatga aaaactcgac cgtcaacatg tgcagcatca attgcatgta attcttaagg 1320
agctccgaaa agcaagaaat caaataacac agttggaatc cttgaaacag cttcatgagt 1380
ttgccatcac agagccatta gtcactttcc aaggagagac tgaaaacaga gaaaaagttg 1440
ccgcctcacc aaaaagtccc actgctgcac tcaatgaaag cctggtggaa tgtcccaagt 1500
gcaatataca gtatccagcc actgagcatc gcgatctgct tgtccatgtg gaatactgtt 1560
caaagtagca aaataagtat ttgttttgat attaaaagat tcaatactgt attttctgtt 1620
agcttgtggg cattttgaat tatatatttc acatttgca taaaactgcc tatctacctt 1680
tgacactcca gcatgctagt gaatcatgta tcttttaggc tgctgtgcat ttctcttggc 1740
agtgatacct ccctgacatg gttcatcatc aggctgcaat gacagaatgt ggtgagcagc 1800
gtctactgag atactaacat tttgcactgt caaaatactt ggtgaggaaa agatagctca 1860
ggttattgct aatgggttaa tgcaccagca agcaaaatat tttatgtttt gggggttttg 1920
aaaaatcaaa gataattaac caaggatctt aactgtgttc gcattttta tccaagcact 1980
tagaaaacct acaatcctaa ttttgatgtc cattgttaag aggtggtgat agatactatt 2040
tttttttca tattgtatag cggttattag aaaagttggg gatttcttg atctttattg 2100
ctgcttacca ttgaaactta acccagctgt gttccccaac tctgttctgc gcacgaaaca 2160
gtatctgttt gaggcataat cttaagtggc cacacacaat gttttctctt atgttatctg 2220
gcagtaactg taacttgaat tacattagca cattctgctt agctaaaatt gttaaaataa 2280
actttaataa acccatgtag ccctctcatt tgattgacag tattttagtt atttttggca 2340
ttcttaaagc tgggcaatgt aatgatcaga tctttgtttg tctgaacagg tatttttata 2400
catgcttttt gtaaaccaaa aacttttaaa tttcttcagg ttttctaaca tgcttaccac 2460
tgggctactg ta 2472

<210> 30
<211> 2472
<212> DNA
<213> Homo Sapiens

<400> 30
ggaccgccag ggagggcagg tcagtgggca gatcgcgtcc gcgggattca atctctgccc 60
gctctgataa cagtcctttt ccctggcgct cacttcgtgc ctggcacccg gctgggcgcc 120
tcaagaccgt tgtctcttcg atcgcttctt tggacttggc gaccatttca gagatgtctt 180
ccagaagtac caaagattta attaaaagta agtggggatc gaagcctagt aactccaaat 240
ccgaaactac attagaaaaa ttaaagggag aaattgcaca cttaaagaca tcagtggatg 300
aaatcacaag tgggaaagga aagctgactg ataaagagag acacagactt ttggagaaaa 360
ttcgagtcct tgaggctgag aaggagaaga atgcttatca actcacagag aaggacaaag 420
aaatacagcg actgagagac caactgaagg ccagatatag tactaccgca ttgcttgaac 480
agctggaaga gacaacgaga gaaggagaaa ggagggagca ggtgttgaaa gccttatctg 540
aagagaaaga cgtattgaaa caacagttgt ctgctgcaac ctcacgaatt gctgaacttg 600
aaagcaaaac caatacactc cgtttatcac agactgtggc tccaaactgc ttcaactcat 660
caataaataa tattcatgaa atggaaatac agctgaaaga tgctctggag aaaaatcagc 720
agtggctcgt gtatgatcag cagcgggaag tctatgtaaa aggactttta gcaaagatct 780
ttgagttgga aaagaaaacg gaaacagctg ctcattcact cccacagcag acaaaaaagc 840
ctgaatcaga aggttatctt caagaagaga agcagaaatg ttacaacgat ctcttggcaa 900
gtgcaaaaaa agatcttgag gttgaacgac aaaccataac tcagctgagt tttgaactga 960
gtgaatttcg aagaaaatat gaagaaaccc aaaaagaagt tcacaattta aatcagctgt 1020
tgtattcaca aagaagggca gatgtgcaac atctggaaga tgataggcat aaaacagaga 1080
agatacaaaa actcagggaa gagaatgata ttgctagggg aaaacttgaa gaagagaaga 1140
agagatccga agagctctta tctcaggtcc agtttcttta cacatctctg ctaaagcagc 1200
aagaagaaca aacaagggta gctctgttgg aacaacagat gcaggcatgt actttagact 1260
ttgaaaatga aaaactcgac cgtcaacatg tgcagcatca attgcttgta attcttaagg 1320
agctccgaaa agcaagaaat caaataacac agttggaatc cttgaaacag cttcatgagt 1380
ttgccatcac agagccatta gtcactttcc aaggagagac tgaaaacaga gaaaaagttg 1440
ccgcctcacc aaaaagtccc actgctgcac tcaatgaaag cctggtggaa tgtcccaagt 1500
gcaatataca gtatccagcc actgagcatc gcgatctgct tgtccatgtg gaatactgtt 1560
caaagtagca aaataagtat ttgttttgat attaaaagat tcaatactgt attttctgtt 1620
agcttgtggg cattttgaat tatatatttc acattttgca taaaactgcc tatctacctt 1680
tgacactcca gcatgctagt gaatcatgta tcttttaggc tgctgtgcat ttctcttggc 1740
agtgatacct ccctgacatg gttcatcatc aggctgcaat gacagaatgt ggtgagcagc 1800
gtctactgag actactaaca ttttgcactg tcaaaatact tggtgaggaa aagatagctc 1860
aggttattgc taatgggtta atgcaccagc aagcaaaata ttttatgttt tgggggtttg 1920
aaaaatcaaa gataattaac caaggatctt aactgtgttc gcattttta tccaagcact 1980
tagaaaacct acaatcctaa ttttgatgtc cattgttaag aggtggtgat agatactatt 2040
ttttttttca tattgtatag cggttattag aaaagttggg gattttcttg atctttattg 2100
ctgcttacca ttgaaactta acccagctgt gttccccaac tctgttctgc gcacgaaaca 2160
gtatctgttt gaggcataat cttaagtggc cacacacaat gttttctctt atgttatctg 2220
gcagtaactg taacttgaat tacattagca cattctgctt agctaaaatt gttaaaataa 2280
actttaataa acccatgtag ccctctcatt tgattgacag tattttagtt atttttggca 2340
ttcttaaagc tgggcaatgt aatgatcaga tctttgtttg tctgaacagg tatttttata 2400
catgcttttt gtaaaccaaa aacttttaaa tttcttcagg ttttctaaca tgcttaccac 2460
tgggctactg ta 2472

<210> 31
<211> 223
<212> PRT
<213> Homo Sapiens

<400> 31
Met Glu Ile Gln Leu Lys Asp Ala Leu Glu Lys Asn Gln Gln Trp Leu
1 5 10 15
Val Tyr Asp Gln Gln Arg Glu Val Tyr Val Lys Gly Leu Leu Ala Lys
20 25 30
Ile Phe Glu Leu Glu Lys Lys Thr Glu Thr Ala Ala His Ser Leu Pro
35 40 45
Gln Gln Thr Lys Lys Pro Glu Ser Glu Gly Tyr Leu Gln Glu Glu Lys
50 55 60
Gln Lys Cys Tyr Asn Asp Leu Leu Ala Ser Ala Lys Lys Asp Leu Glu
65 70 75 80
Val Glu Arg Gln Thr Ile Thr Gln Leu Ser Phe Glu Leu Ser Glu Phe

```
                85                  90                  95
Arg Arg Lys Tyr Glu Glu Thr Gln Lys Glu Val His Asn Leu Asn Gln
            100                 105                 110
Leu Leu Tyr Ser Gln Arg Arg Ala Asp Val Gln His Leu Glu Asp Asp
        115                 120                 125
Arg His Lys Thr Glu Lys Ile Gln Lys Leu Arg Glu Glu Asn Asp Ile
    130                 135                 140
Ala Arg Gly Lys Leu Glu Glu Glu Lys Lys Arg Ser Glu Glu Leu Leu
145                 150                 155                 160
Ser Gln Val Gln Phe Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
Asp Phe Glu Asn Glu Lys Leu Asp Arg Gln His Val Gln His Gln Leu
        195                 200                 205
His Val Ile Leu Lys Glu Leu Arg Lys Ala Arg Asn Gln Ile Thr
    210                 215                 220
```

<210> 32
<211> 223
<212> PRT
<213> Homo Sapiens

<400> 32

```
Met Glu Ile Gln Leu Lys Asp Ala Leu Glu Lys Asn Gln Gln Trp Leu
 1               5                  10                  15
Val Tyr Asp Gln Gln Arg Glu Val Tyr Val Lys Gly Leu Leu Ala Lys
            20                  25                  30
Ile Phe Glu Leu Glu Lys Lys Thr Glu Thr Ala Ala His Ser Leu Pro
        35                  40                  45
Gln Gln Thr Lys Lys Pro Glu Ser Glu Gly Tyr Leu Gln Glu Glu Lys
    50                  55                  60
Gln Lys Cys Tyr Asn Asp Leu Leu Ala Ser Ala Lys Lys Asp Leu Glu
65                  70                  75                  80
Val Glu Arg Gln Thr Ile Thr Gln Leu Ser Phe Glu Leu Ser Glu Phe
                85                  90                  95
Arg Arg Lys Tyr Glu Glu Thr Gln Lys Glu Val His Asn Leu Asn Gln
            100                 105                 110
Leu Leu Tyr Ser Gln Arg Arg Ala Asp Val Gln His Leu Glu Asp Asp
        115                 120                 125
Arg His Lys Thr Glu Lys Ile Gln Lys Leu Arg Glu Glu Asn Asp Ile
    130                 135                 140
Ala Arg Gly Lys Leu Glu Glu Glu Lys Lys Arg Ser Glu Glu Leu Leu
145                 150                 155                 160
Ser Gln Val Gln Ser Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
Asp Phe Glu Asn Glu Lys Leu Asp Arg Gln His Val Gln His Gln Leu
        195                 200                 205
His Val Ile Leu Lys Glu Leu Arg Lys Ala Arg Lys Asn Asn Thr
    210                 215                 220
```

<210> 33
<211> 464
<212> PRT
<213> Homo Sapiens

<400> 33

```
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 34

<211> 464
<212> PRT
<213> Homo Sapiens

<400> 34
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Thr Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Phe Leu Tyr
325 330 335
Thr Ser Leu Leu Lys Gln Gln Glu Glu Gln Thr Arg Val Ala Leu Leu
340 345 350
Glu Gln Gln Met Gln Ala Cys Thr Leu Asp Phe Glu Asn Glu Lys Leu
355 360 365
Asp Arg Gln His Val Gln His Gln Leu His Val Ile Leu Lys Glu Leu
370 375 380
Arg Lys Ala Arg Asn Gln Ile Thr Gln Leu Glu Ser Leu Lys Gln Leu
385 390 395 400
His Glu Phe Ala Ile Thr Glu Pro Leu Val Thr Phe Gln Gly Glu Thr
405 410 415
Glu Asn Arg Glu Lys Val Ala Ala Ser Pro Lys Ser Pro Thr Ala Ala
420 425 430
Leu Asn Glu Ser Leu Val Glu Cys Pro Lys Cys Asn Ile Gln Tyr Pro
435 440 445

Ala Thr Glu His Arg Asp Leu Leu Val His Val Glu Tyr Cys Ser Lys
    450                 455                 460

<210> 35
<211> 464
<212> PRT
<213> Homo Sapiens

<400> 35
Met Ser Ser Arg Ser Thr Lys Asp Leu Ile Lys Ser Lys Trp Gly Ser
1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr

```
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 36
<211> 462
<212> PRT
<213> Mus musculus

<400> 36

```
Met Ser Ser Arg Ser Pro Lys Asp Leu Ile Lys Ser Lys Trp Gly Ser
 1               5                  10                  15
Arg Pro Ser Ser Ser Lys Ser Asp Thr Ala Leu Glu Lys Phe Lys Gly
            20                  25                  30
Glu Ile Ala Ala Phe Lys Thr Ser Leu Asp Glu Ile Thr Ser Gly Lys
        35                  40                  45
Gly Lys Met Ala Glu Lys Gly Arg Ser Arg Leu Leu Glu Lys Ile Gln
    50                  55                  60
Val Leu Glu Ala Glu Arg Glu Lys Asn Val Tyr Tyr Leu Leu Glu Lys
65                  70                  75                  80
Asp Lys Glu Ile Gln Arg Leu Lys Asp His Leu Arg Ser Arg Tyr Ser
                85                  90                  95
Ser Ser Ser Leu Phe Glu Gln Leu Glu Glu Lys Thr Lys Glu Cys Glu
            100                 105                 110
Lys Lys Gln Gln Leu Leu Glu Ser Leu Ser Lys Glu Thr Asp Val Leu
        115                 120                 125
Lys Asn Gln Leu Ser Ala Thr Thr Lys Arg Leu Ser Glu Leu Glu Ser
    130                 135                 140
Lys Ala Ser Thr Leu His Leu Ser Gln Ser Met Pro Ala Asn Cys Phe
145                 150                 155                 160
Asn Ser Ser Met Asn Ser Ile His Glu Lys Glu Met Gln Leu Lys Asp
                165                 170                 175
Ala Leu Glu Lys Asn Gln Gln Trp Leu Val Tyr Asp Gln Gln Arg Glu
            180                 185                 190
Ala Tyr Val Lys Gly Leu Leu Ala Lys Ile Phe Glu Leu Glu Lys Arg
        195                 200                 205
Thr Glu Thr Ala Ala Ala Ser Leu Thr Gln Gln Met Lys Lys Ile Glu
    210                 215                 220
Ser Glu Gly Tyr Leu Gln Val Glu Lys Gln Lys Tyr Asp His Leu Leu
225                 230                 235                 240
Glu Asn Ala Lys Lys Asp Leu Glu Val Glu Arg Gln Ala Val Thr Gln
                245                 250                 255
Leu Arg Leu Glu Leu Asp Glu Phe Arg Arg Lys Tyr Glu Glu Ala Arg
            260                 265                 270
Lys Glu Val Glu Asp Leu Asn Gln Leu Leu Ser Ser Gln Arg Lys Ala
        275                 280                 285
Asp Ile Gln His Leu Glu Glu Asp Lys Gln Lys Thr Glu Arg Ile Gln
    290                 295                 300
Lys Leu Arg Glu Glu Ser Ser Ile Phe Lys Gly Lys Leu Glu Glu Glu
305                 310                 315                 320
Arg Lys Arg Ser Glu Glu Leu Leu Ser Gln Val Arg Ile Leu Tyr Asp
                325                 330                 335
Ser Leu Leu Lys His Gln Glu Glu Gln Ala Arg Val Ala Leu Leu Glu
            340                 345                 350
Gln Gln Met Gln Ala Cys Thr Leu Asp Phe Glu Asn Glu Lys Leu Asp
        355                 360                 365
```

Arg Gln Asn Met Gln His Gln Leu Tyr Val Ile Leu Lys Glu Leu Arg
370 375 380
Lys Ala Lys Ser Gln Ile Thr Gln Leu Glu Ser Leu Lys Gln Leu His
385 390 395 400
Gly Phe Thr Ile Thr Glu Gln Pro Phe Pro Leu Gln Arg Glu Pro Glu
405 410 415
Ser Arg Val Lys Ala Thr Ser Pro Lys Ser Pro Ser Ala Ala Leu Asn
420 425 430
Asp Ser Leu Val Glu Cys Pro Lys Cys Ser Val Gln Tyr Pro Ala Thr
435 440 445
Glu His Arg Asp Leu Leu Val His Val Glu Tyr Cys Met Lys
450 455 460

<210> 37
<211> 310
<212> PRT
<213> Homo Sapiens

<400> 37
Glu Ile Gln Arg Leu Arg Asp Gln Leu Lys Ala Arg Tyr Ser Thr Thr
1 5 10 15
Ala Leu Leu Glu Gln Leu Glu Glu Thr Thr Arg Glu Gly Glu Arg Arg
20 25 30
Glu Gln Val Leu Lys Ala Leu Ser Glu Glu Lys Asp Val Leu Lys Gln
35 40 45
Gln Leu Ser Ala Ala Thr Ser Arg Ile Ala Glu Leu Glu Ser Lys Thr
50 55 60
Asn Thr Leu Arg Leu Ser Gln Thr Val Ala Pro Asn Cys Phe Asn Ser
65 70 75 80
Ser Ile Asn Asn Ile His Glu Met Glu Ile Gln Leu Lys Asp Ala Leu
85 90 95
Glu Lys Asn Gln Gln Trp Leu Val Tyr Asp Gln Gln Arg Glu Val Tyr
100 105 110
Val Lys Gly Leu Leu Ala Lys Ile Phe Glu Leu Glu Lys Lys Thr Glu
115 120 125
Thr Ala Ala His Ser Leu Pro Gln Gln Thr Lys Lys Pro Glu Ser Glu
130 135 140
Gly Tyr Leu Gln Glu Glu Lys Gln Lys Cys Tyr Asn Asp Leu Leu Ala
145 150 155 160
Ser Ala Lys Lys Asp Leu Glu Val Glu Arg Gln Thr Ile Thr Gln Leu
165 170 175
Ser Phe Glu Leu Ser Glu Phe Arg Arg Lys Tyr Glu Glu Thr Gln Lys
180 185 190
Glu Val His Asn Leu Asn Gln Leu Leu Tyr Ser Gln Arg Arg Ala Asp
195 200 205
Val Gln His Leu Glu Asp Asp Arg His Lys Thr Glu Lys Ile Gln Lys
210 215 220
Leu Arg Glu Glu Asn Asp Ile Ala Arg Gly Lys Leu Glu Glu Glu Lys
225 230 235 240
Lys Arg Ser Glu Glu Leu Leu Ser Gln Val Gln Phe Leu Tyr Thr Ser
245 250 255
Leu Leu Lys Gln Gln Glu Glu Gln Thr Arg Val Ala Leu Leu Glu Gln
260 265 270
Gln Met Gln Ala Cys Thr Leu Asp Phe Glu Asn Glu Lys Leu Asp Arg
275 280 285
Gln His Val Gln His Gln Leu His Val Ile Leu Lys Glu Leu Arg Lys
290 295 300
Ala Arg Asn Gln Ile Thr
305 310

<210> 38
<211> 313
<212> PRT
<213> Homo Sapiens

<400> 38
Glu Phe Asn Arg Leu Ala Ser Lys Val His Lys Asn Glu Gln Arg Thr
1 5 10 15
Ser Ile Leu Gln Thr Leu Cys Glu Gln Leu Arg Lys Glu Asn Glu Ala
20 25 30
Leu Lys Ala Lys Leu Asp Lys Gly Leu Glu Gln Arg Asp Gln Ala Ala
35 40 45
Glu Arg Leu Arg Glu Glu Asn Leu Glu Leu Lys Lys Leu Leu Met Ser
50 55 60
Asn Gly Asn Lys Glu Gly Ala Ser Gly Arg Pro Gly Ser Pro Lys Met
65 70 75 80
Glu Gly Thr Gly Lys Lys Ala Val Ala Gly Gln Gln Gln Ala Ser Val
85 90 95
Thr Ala Gly Lys Val Pro Glu Val Val Ala Leu Gly Ala Ala Glu Lys
100 105 110
Lys Val Lys Met Leu Glu Gln Gln Arg Ser Glu Leu Leu Glu Val Asn
115 120 125
Lys Gln Trp Asp Gln His Phe Arg Ser Met Lys Gln Gln Tyr Glu Gln
130 135 140
Lys Ile Thr Glu Leu Arg Gln Lys Leu Ala Asp Leu Gln Lys Gln Val
145 150 155 160
Thr Asp Leu Glu Ala Glu Arg Glu Gln Lys Gln Arg Asp Phe Asp Arg
165 170 175
Lys Leu Leu Leu Ala Lys Ser Lys Ile Glu Met Glu Glu Thr Asp Lys
180 185 190
Glu Gln Leu Thr Ala Glu Ala Lys Glu Leu Arg Gln Lys Val Lys Tyr
195 200 205
Leu Gln Asp Gln Leu Ser Pro Leu Thr Arg Gln Arg Glu Tyr Gln Glu
210 215 220
Lys Glu Ile Gln Arg Leu Asn Lys Ala Leu Glu Glu Ala Leu Ser Ile
225 230 235 240
Gln Thr Pro Pro Ser Ser Pro Pro Thr Ala Phe Gly Ser Pro Glu Gly
245 250 255
Ala Gly Ala Leu Leu Arg Lys Gln Glu Leu Val Thr Gln Asn Glu Leu
260 265 270
Leu Lys Gln Gln Val Lys Ile Phe Glu Glu Asp Phe Gln Arg Glu Arg
275 280 285
Ser Asp Arg Glu Arg Met Asn Glu Glu Lys Glu Glu Leu Lys Lys Gln
290 295 300
Val Glu Lys Leu Gln Ala Gln Val Thr
305 310

<210> 39
<211> 464
<212> PRT
<213> Homo Sapiens

<400> 39
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45

Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460

<210> 40
<211> 462
<212> PRT
<213> Mus musculus

<400> 40
Met Ser Ser Arg Ser Pro Lys Asp Leu Ile Lys Ser Lys Trp Gly Ser

```
1               5                   10                  15
Arg Pro Ser Ser Ser Lys Ser Asp Thr Ala Leu Glu Lys Phe Lys Gly
            20                  25                  30
Glu Ile Ala Ala Phe Lys Thr Ser Leu Asp Glu Ile Thr Ser Gly Lys
        35                  40                  45
Gly Lys Met Ala Glu Lys Gly Arg Ser Arg Leu Leu Glu Lys Ile Gln
    50                  55                  60
Val Leu Glu Ala Glu Arg Glu Lys Asn Val Tyr Tyr Leu Leu Glu Lys
65                  70                  75                  80
Asp Lys Glu Ile Gln Arg Leu Lys Asp His Leu Arg Ser Arg Tyr Ser
                85                  90                  95
Ser Ser Ser Leu Phe Glu Gln Leu Glu Glu Lys Thr Lys Glu Cys Glu
            100                 105                 110
Lys Lys Gln Gln Leu Leu Glu Ser Leu Ser Lys Glu Thr Asp Val Leu
        115                 120                 125
Lys Asn Gln Leu Ser Ala Thr Thr Lys Arg Leu Ser Glu Leu Glu Ser
    130                 135                 140
Lys Ala Ser Thr Leu His Leu Ser Gln Ser Met Pro Ala Asn Cys Phe
145                 150                 155                 160
Asn Ser Ser Met Asn Ser Ile His Glu Lys Glu Met Gln Leu Lys Asp
                165                 170                 175
Ala Leu Glu Lys Asn Gln Gln Trp Leu Val Tyr Asp Gln Gln Arg Glu
            180                 185                 190
Ala Tyr Val Lys Gly Leu Leu Ala Lys Ile Phe Glu Leu Glu Lys Arg
        195                 200                 205
Thr Glu Thr Ala Ala Ala Ser Leu Thr Gln Gln Met Lys Lys Ile Glu
    210                 215                 220
Ser Glu Gly Tyr Leu Gln Val Glu Lys Gln Lys Tyr Asp His Leu Leu
225                 230                 235                 240
Glu Asn Ala Lys Lys Asp Leu Glu Val Glu Arg Gln Ala Val Thr Gln
                245                 250                 255
Leu Arg Leu Glu Leu Asp Glu Phe Arg Arg Lys Tyr Glu Glu Ala Arg
            260                 265                 270
Lys Glu Val Glu Asp Leu Asn Gln Leu Leu Ser Ser Gln Arg Lys Ala
        275                 280                 285
Asp Ile Gln His Leu Glu Glu Asp Lys Gln Lys Thr Glu Arg Ile Gln
    290                 295                 300
Lys Leu Arg Glu Glu Ser Ser Ile Phe Lys Gly Lys Leu Glu Glu Glu
305                 310                 315                 320
Arg Lys Arg Ser Glu Glu Leu Leu Ser Gln Val Arg Ile Leu Tyr Asp
                325                 330                 335
Ser Leu Leu Lys His Gln Glu Glu Gln Ala Arg Val Ala Leu Leu Glu
            340                 345                 350
Gln Gln Met Gln Ala Cys Thr Leu Asp Phe Glu Asn Glu Lys Leu Asp
        355                 360                 365
Arg Gln Asn Met Gln His Gln Leu Tyr Val Ile Leu Lys Glu Leu Arg
    370                 375                 380
Lys Ala Lys Ser Gln Ile Thr Gln Leu Glu Ser Leu Lys Gln Leu His
385                 390                 395                 400
Gly Phe Thr Ile Thr Glu Gln Pro Phe Pro Leu Gln Arg Glu Pro Glu
                405                 410                 415
Ser Arg Val Lys Ala Thr Ser Pro Lys Ser Pro Ser Ala Ala Leu Asn
            420                 425                 430
Asp Ser Leu Val Glu Cys Pro Lys Cys Ser Val Gln Tyr Pro Ala Thr
        435                 440                 445
Glu His Arg Asp Leu Leu Val His Val Glu Tyr Cys Met Lys
    450                 455                 460
```

<210> 41
<211> 398

<212> PRT
<213> Homo Sapiens

<400> 41
```
Met Ser Ser Arg Ser Thr Lys Asp Leu Ile Lys Ser Lys Trp Gly Ser
 1               5                   10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
Ala Leu Glu Lys Asn Gln Gln Trp Leu Val Tyr Asp Gln Gln Arg Glu
            180                 185                 190
Val Tyr Val Lys Gly Leu Leu Ala Lys Ile Phe Glu Leu Glu Lys Lys
        195                 200                 205
Thr Glu Thr Ala Ala His Ser Leu Pro Gln Gln Thr Lys Lys Pro Glu
    210                 215                 220
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys
385                 390                 395
```

<210> 42
<211> 373
<212> PRT
<213> Homo Sapiens

<400> 42
Met Met Asn Gln Leu Glu Glu Asp Leu Val Ser Ala Arg Arg Arg Ser
1 5 10 15
Asp Leu Tyr Glu Ser Glu Leu Arg Glu Ser Arg Leu Ala Ala Glu Glu
20 25 30
Phe Lys Arg Lys Ala Asn Glu Cys Gln His Lys Leu Met Lys Ala Lys
35 40 45
Asp Gln Gly Lys Pro Glu Val Gly Glu Tyr Ser Lys Leu Glu Lys Ile
50 55 60
Asn Ala Glu Gln Gln Leu Lys Ile Gln Glu Leu Gln Glu Lys Leu Glu
65 70 75 80
Lys Ala Val Lys Ala Ser Thr Glu Ala Thr Glu Leu Leu Gln Asn Ile
85 90 95
Arg Gln Ala Lys Glu Arg Ala Glu Arg Glu Leu Glu Lys Leu His Asn
100 105 110
Arg Glu Asp Ser Ser Glu Gly Ile Lys Lys Lys Leu Val Glu Ala Glu
115 120 125
Glu Arg Arg His Ser Leu Glu Asn Lys Val Lys Arg Leu Glu Thr Met
130 135 140
Glu Arg Arg Glu Asn Arg Leu Lys Asp Asp Ile Gln Thr Lys Ser Glu
145 150 155 160
Gln Ile Gln Gln Met Ala Asp Lys Ile Leu Glu Leu Glu Glu Lys His
165 170 175
Arg Glu Ala Gln Val Ser Ala Gln His Leu Glu Val His Leu Lys Gln
180 185 190
Lys Glu Gln His Tyr Glu Glu Lys Ile Lys Val Leu Asp Asn Gln Ile
195 200 205
Lys Lys Asp Leu Ala Asp Lys Glu Ser Leu Glu Asn Met Met Gln Arg
210 215 220
His Glu Glu Glu Ala His Glu Lys Gly Lys Ile Leu Ser Glu Gln Lys
225 230 235 240
Ala Met Ile Asn Ala Met Asp Ser Lys Ile Arg Ser Leu Glu Gln Arg
245 250 255
Ile Val Glu Leu Ser Glu Ala Asn Lys Leu Ala Ala Asn Ser Ser Leu
260 265 270
Phe Thr Gln Arg Asn Met Lys Ala Gln Glu Glu Met Ile Ser Glu Leu
275 280 285
Arg Gln Gln Lys Phe Tyr Leu Glu Thr Gln Ala Gly Lys Leu Glu Ala
290 295 300
Gln Asn Arg Lys Leu Glu Glu Gln Leu Glu Lys Ile Ser His Gln Asp
305 310 315 320
His Ser Asp Lys Ser Arg Leu Leu Glu Leu Glu Thr Arg Leu Arg Glu
325 330 335
Val Ser Leu Glu His Glu Glu Gln Lys Leu Glu Leu Lys Arg Gln Leu
340 345 350
Thr Glu Leu Gln Leu Ser Leu Gln Glu Arg Glu Ser Gln Leu Thr Ala
355 360 365
Leu Gln Ala Ala Arg
370

<210> 43
<211> 14
<212> PRT
<213> Clostridium toxin

<400> 43
Gln Tyr Ile Lys Ala Asn Ser Lys Phe Ile Gly Ile Thr Glu
1 5 10

<210> 44
<211> 21
<212> PRT
<213> Plasmodium falciparum

<400> 44
Asp Ile Glu Lys Lys Ile Ala Lys Met Glu Lys Ala Ser Ser Val Phe
1 5 10 15
Asn Val Val Asn Ser
20

<210> 45
<211> 16
<212> PRT
<213> Streptococcus aureus

<400> 45
Gly Ala Val Asp Ser Ile Leu Gly Gly Val Ala Thr Tyr Gly Ala Ala
1 5 10 15

<210> 46
<211> 13
<212> PRT
<213> Artificial Sequence

<220>
<221> source
<223> Artificially Synthesized Peptide

<221> VARIANT
<222> 3
<223> Xaa = cyclohexylalanine, phenylalanine, or tyrosine

<221> VARIANT
<222> 1
<223> Xaa = D-alanine or L-alanine

<221> VARIANT
<222> 13
<223> Xaa = D-alanine or L-alanine

<400> 46
Xaa Lys Xaa Val Ala Ala Trp Thr Leu Lys Ala Ala Xaa
1 5 10

<210> 47
<211> 14
<212> DNA
<213> Artificial Sequence

<220>
<221> source
<223> Primer

<400> 47
ttttgatcaa gctt 14

<210> 48
<211> 42
<212> DNA
<213> Artificial Sequence

<220>
<221> source
<223> Primer

<400> 48
ctaatacgac tcactatagg gctcgagcgg ccgcccgggc ag 42

<210> 49
<211> 12
<212> DNA
<213> Artificial Sequence

<220>
<221> source
<223> Primer

<400> 49
gatcctgccc gg 12

<210> 50
<211> 40
<212> DNA
<213> Artificial Sequence

<220>
<221> source
<223> Primer

<400> 50
gtaatacgac tcactatagg gcagcgtggt cgcggccgag 40

<210> 51
<211> 10
<212> DNA
<213> Artificial Sequence

<220>
<221> source
<223> Primer

<400> 51
gatcctcggc 10

<210> 52
<211> 22
<212> DNA
<213> Artificial Sequence

<220>
<221> source
<223> PCR Primer

<400> 52
ctaatacgac tcactatagg gc 22

<210> 53

<211> 22
<212> DNA
<213> Artificial Sequence

<220>
<221> source
<223> Nested Primer

<400> 53
tcgagcggcc gcccgggcag ga 22

<210> 54
<211> 20
<212> DNA
<213> Artificial Sequence

<220>
<221> source
<223> Nested Primer

<400> 54
agcgtggtcg cggccgagga 20

<210> 55
<211> 25
<212> DNA
<213> Artificial Sequence

<220>
<221> source
<223> Primer

<400> 55
atatcgccgc gctcgtcgtc gacaa 25

<210> 56
<211> 26
<212> DNA
<213> Artificial Sequence

<220>
<221> source
<223> Primer

<400> 56
agccacacgc agctcattgt agaagg 26

<210> 57
<211> 24
<212> DNA
<213> Artificial Sequence

<220>
<221> source
<223> RT-PCR Primer

<400> 57
tgtcaatcaa atgagagggc taca 24

<210> 58
<211> 25

<212> DNA
<213> Artificial Sequence

<220>
<221> source
<223> RT-PCR Primer

<400> 58
ctgtttgagg cataatctta agtgg 25

<210> 59
<211> 24
<212> DNA
<213> Artificial Sequence

<220>
<221> source
<223> Epitope Tag

<400> 59
gattacaagg atgacgacga taag 24

<210> 60

<400> 60
000

<210> 61
<211> 464
<212> PRT
<213> Homo Sapiens

<400> 61
Met Ser Ser Arg Ser Thr Lys Asp Leu Ile Lys Ser Lys Trp Gly Ser
1 5 10 15
Lys Pro Ser Asn Ser Lys Ser Glu Thr Thr Leu Glu Lys Leu Lys Gly
20 25 30
Glu Ile Ala His Leu Lys Thr Ser Val Asp Glu Ile Thr Ser Gly Lys
35 40 45
Gly Lys Leu Thr Asp Lys Glu Arg His Arg Leu Leu Glu Lys Ile Arg
50 55 60
Val Leu Glu Ala Glu Lys Glu Lys Asn Ala Tyr Gln Leu Thr Glu Lys
65 70 75 80
Asp Lys Glu Ile Gln Arg Leu Arg Asp Gln Leu Lys Ala Arg Tyr Ser
85 90 95
Thr Thr Ala Leu Leu Glu Gln Leu Glu Glu Thr Thr Arg Glu Gly Glu
100 105 110
Arg Arg Glu Gln Val Leu Lys Ala Leu Ser Glu Glu Lys Asp Val Leu
115 120 125
Lys Gln Gln Leu Ser Ala Ala Thr Ser Arg Ile Ala Glu Leu Glu Ser
130 135 140
Lys Thr Asn Thr Leu Arg Leu Ser Gln Thr Val Ala Pro Asn Cys Phe
145 150 155 160
Asn Ser Ser Ile Asn Asn Ile His Glu Met Glu Ile Gln Leu Lys Asp
165 170 175
Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220

```
Ser Glu Gly Tyr Leu Gln Glu Glu Lys Gln Lys Cys Tyr Asn Asp Leu
225                 230                 235                 240
Leu Ala Ser Ala Lys Lys Asp Leu Glu Val Glu Arg Gln Thr Ile Thr
                245                 250                 255
Gln Leu Ser Phe Glu Leu Ser Glu Phe Arg Arg Lys Tyr Glu Glu Thr
            260                 265                 270
Gln Lys Glu Val His Asn Leu Asn Gln Leu Leu Tyr Ser Gln Arg Arg
        275                 280                 285
Ala Asp Val Gln His Leu Glu Asp Asp Arg His Lys Thr Glu Lys Ile
    290                 295                 300
Gln Lys Leu Arg Glu Glu Asn Asp Ile Ala Arg Gly Lys Leu Glu Glu
305                 310                 315                 320
Glu Lys Lys Arg Ser Glu Glu Leu Leu Ser Gln Val Gln Phe Leu Tyr
                325                 330                 335
Thr Ser Leu Leu Lys Gln Gln Glu Glu Gln Thr Arg Val Ala Leu Leu
            340                 345                 350
Glu Gln Gln Met Gln Ala Cys Thr Leu Asp Phe Glu Asn Glu Lys Leu
        355                 360                 365
Asp Arg Gln His Val Gln His Gln Leu His Val Ile Leu Lys Glu Leu
    370                 375                 380
Arg Lys Ala Arg Asn Gln Ile Thr Gln Leu Glu Ser Leu Lys Gln Leu
385                 390                 395                 400
His Glu Phe Ala Ile Thr Glu Pro Leu Val Thr Phe Gln Gly Glu Thr
                405                 410                 415
Glu Asn Arg Glu Lys Val Ala Ala Ser Pro Lys Ser Pro Thr Ala Ala
            420                 425                 430
Leu Asn Glu Ser Leu Val Glu Cys Pro Lys Cys Asn Ile Gln Tyr Pro
        435                 440                 445
Ala Thr Glu His Arg Asp Leu Leu Val His Val Glu Tyr Cys Ser Lys
    450                 455                 460
```

<210> 62
<211> 17
<212> PRT
<213> Homo Sapiens

<400> 62

```
Gly Lys Leu Thr Asp Lys Glu Arg Gln Arg Leu Leu Glu Lys Ile Arg
 1               5                  10                  15
Val
```

<210> 63
<211> 21
<212> PRT
<213> Homo Sapiens

<400> 63

```
Ser Gly Lys Gly Lys Leu Thr Asp Lys Glu Arg Gln Arg Leu Leu Glu
 1               5                  10                  15
Lys Ile Arg Val Leu
            20
```

<210> 64
<211> 29
<212> PRT
<213> Homo Sapiens

<400> 64
Glu Ile Thr Ser Gly Lys Gly Lys Leu Thr Asp Lys Glu Arg Gln Arg
1 5 10 15
Leu Leu Glu Lys Ile Arg Val Leu Glu Ala Glu Lys Glu
20 25

<210> 65
<211> 17
<212> PRT
<213> Homo Sapiens

<400> 65
Leu Lys Ala Arg Tyr Ser Thr Thr Thr Leu Leu Glu Gln Leu Glu Glu
1 5 10 15
Thr

<210> 66
<211> 19
<212> PRT
<213> Homo Sapiens

<400> 66
Gln Leu Lys Ala Arg Tyr Ser Thr Thr Thr Leu Leu Glu Gln Leu Glu
1 5 10 15
Glu Thr Thr

<210> 67
<211> 28
<212> PRT
<213> Homo Sapiens

<400> 67
Gln Arg Leu Arg Asp Gln Leu Lys Ala Arg Tyr Ser Thr Thr Thr Leu
1 5 10 15
Leu Glu Gln Leu Glu Glu Thr Thr Arg Glu Gly Glu
20 25

<210> 68
<211> 17
<212> PRT
<213> Homo Sapiens

<400> 68
Glu Glu Leu Leu Ser Gln Val Gln Ser Leu Tyr Thr Ser Leu Leu Lys
1 5 10 15
Gln

<210> 69
<211> 19
<212> PRT
<213> Homo Sapiens

<400> 69

```
Ser Glu Glu Leu Leu Ser Gln Val Gln Ser Leu Tyr Thr Ser Leu Leu
 1              5                   10                  15
Lys Gln Gln
```

<210> 70
<211> 29
<212> PRT
<213> Homo Sapiens

<400> 70

```
Glu Glu Lys Lys Arg Ser Glu Glu Leu Leu Ser Gln Val Gln Ser Leu
 1              5                   10                  15
Tyr Thr Ser Leu Leu Lys Gln Gln Glu Glu Gln Thr Arg
            20                  25
```

<210> 71
<211> 17
<212> PRT
<213> Homo Sapiens

<400> 71

```
Arg Gln His Val Gln His Gln Leu Leu Val Ile Leu Lys Glu Leu Arg
 1              5                   10                  15
Lys
```

<210> 72
<211> 19
<212> PRT
<213> Homo Sapiens

<400> 72

```
Asp Arg Gln His Val Gln His Gln Leu Leu Val Ile Leu Lys Glu Leu
 1              5                   10                  15
Arg Lys Ala
```

<210> 73
<211> 29
<212> PRT
<213> Homo Sapiens

<400> 73

```
Glu Asn Glu Lys Leu Asp Arg Gln His Val Gln His Gln Leu Leu Val
 1              5                   10                  15
Ile Leu Lys Glu Leu Arg Lys Ala Arg Asn Gln Ile Thr
            20                  25
```

<210> 74
<211> 17
<212> PRT
<213> Homo Sapiens

<400> 74

```
Lys Ser Pro Thr Ala Ala Leu Asn Gly Ser Leu Val Glu Cys Pro Lys
```

1 5 10 15
Cys

<210> 75
<211> 18
<212> PRT
<213> Homo Sapiens

<400> 75
Pro Lys Ser Pro Thr Ala Ala Leu Asn Gly Ser Leu Val Glu Cys Pro
1 5 10 15
Lys Cys

<210> 76
<211> 29
<212> PRT
<213> Homo Sapiens

<400> 76
Lys Val Ala Ala Ser Pro Lys Ser Pro Thr Ala Ala Leu Asn Gly Ser
1 5 10 15
Leu Val Glu Cys Pro Lys Cys Asn Ile Gln Tyr Pro Ala
20 25

<210> 77
<211> 2324
<212> DNA
<213> Homo Sapiens

<400> 77
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agagatgtct 180
tccagaagta ccaaagattt aattaaaaaa aattcgagtc cttgaggctg agaaggagaa 240
gaatgcttat caactcacag agaaggacaa agaaatacag cgactgagag accaactgaa 300
ggccagatat agtactaccg cattgcttga acagctggaa gagacaacga gagaaggaga 360
aaggagggag caggtgttga aagccttatc tgaagagaaa gacgtattga aacaacagtt 420
gtctgctgca acctcacgaa ttgctgaact tgaaagcaaa accaatacac tccgtttatc 480
acagactgtg gctccaaact gcttcaactc atcaataaat aatattcatg aaatggaaat 540
acagctgaaa gatgctctgg agaaaaatca gcagtggctc gtgtatgatc agcagcggga 600
agtctatgta aaaggacttt tagcaaagat ctttgagttg gaaaagaaaa cggaaacagc 660
tgctcattca ctcccacagc agacaaaaaa gcctgaatca gaaggttatc ttcaagaaga 720
gaagcagaaa tgttacaacg atctcttggc aagtgcaaaa aaagatcttg aggttgaacg 780
acaaaccata actcagctga gttttgaact gagtgaattt cgaagaaaat atgaagaaac 840
ccaaaaagaa gttcacaatt taaatcagct gttgtattca caaagaaggg cagatgtgca 900
acatctggaa gatgataggc ataaaacaga gaagatacaa aaactcaggg aagagaatga 960
tattgctagg ggaaaacttg aagaagagaa gaagagatcc gaagagctct tatctcaggt 1020
ccagtttctt tacacatctc tgctaaagca gcaagaagaa caaacaaggg tagctctgtt 1080
ggaacaacag atgcaggcat gtactttaga ctttgaaaat gaaaaactcg accgtcaaca 1140
tgtgcagcat caattgcatg taattcttaa ggagctccga aaagcaagaa atcaaataac 1200
acagttggaa tccttgaaac agcttcatga gtttgccatc acagagccat tagtcacttt 1260
ccaaggagag actgaaaaca gagaaaaagt tgccgcctca ccaaaaagtc ccactgctgc 1320
actcaatgaa agcctggtgg aatgtcccaa gtgcaatata cagtatccag ccactgagca 1380
tcgcgatctg cttgtccatg tggaatactg ttcaaagtag caaaataagt atttgttttg 1440
atattaaaag attcaatact gtattttctg ttagcttgtg ggcattttga attatatatt 1500
tcacattttg cataaaactg cctatctacc tttgacactc cagcatgcta gtgaatcatg 1560

```
tatcttttag gctgctgtgc atttctcttg gcagtgatac ctccctgaca tggttcatca 1620
tcaggctgca atgacagaat gtggtgagca gcgtctactg agatactaac attttgcact 1680
gtcaaaatac ttggtgagga aaagatagct caggttattg ctaatgggtt aatgcaccag 1740
caagcaaaat attttatgtt ttggggtttt tgaaaaatca aagataatta accaaggatc 1800
ttaactgtgt tcgcattttt tatccaagca cttagaaaac ctacaatcct aattttgatg 1860
tccattgtta agaggtggtg atagatacta tttttttttt catattgtat agcggttatt 1920
agaaaagttg gggattttct tgatctttat tgctgcttac cattgaaact taacccagct 1980
gtgttcccca actctgttct gcgcacgaaa cagtatctgt ttgaggcata atcttaagtg 2040
gccacacaca atgttttctc ttatgttatc tggcagtaac tgtaacttga attacattag 2100
cacattctgc ttagctaaaa ttgttaaaat aaactttaat aaacccatgt agccctctca 2160
tttgattgac agtattttag ttatttttgg cattcttaaa gctgggcaat gtaatgatca 2220
gatctttgtt tgtctgaaca ggtattttta tacatgcttt ttgtaaacca aaaactttta 2280
aatttcttca ggttttctaa catgcttacc actgggctac tgta                  2324
```

<210> 78
<211> 2473
<212> DNA
<213> Homo Sapiens

<400> 78

```
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agagatgtct 180
tccagaagta ccaaagattt aattaaaagt aagtggggat cgaagcctag taactccaaa 240
tccgaaacta cattagaaaa attaaaggga gaaattgcac acttaaagac atcagtggat 300
gaaatcacaa gtgggaaagg aaagctgact gataaagaga gacacagact tttggagaaa 360
attcgagtcc ttgaggctga gaaggagaag aatgcttatc aactcacaga gaaggacaaa 420
gaaatacagc gactgagaga ccaactgaag gccagatata gtactaccgc attgcttgaa 480
cagctggaag agacaacgag agaaggagaa aggagggagc aggtgttgaa agccttatct 540
gaagagaaag acgtattgaa acaacagttg tctgctgcaa cctcacgaat tgctgaactt 600
gaaagcaaaa ccaatacact ccgtttatca cagactgtgg ctccaaactg cttcaactca 660
tcaataaata atattcatga aatggaaata cagctgaaag atgctctgga gaaaaatcag 720
cagtggctcg tgtatgatca gcagcgggaa gtctatgtaa aaggactttt agcaaagatc 780
tttgagttgg aaaagaaaac ggaaacagct gctcattcac tcccacagca gacaaaaaag 840
cctgaatcag aaggttatct tcaagaagag aagcagaaat gttacaacga tctcttggca 900
agtgcaaaaa aagatcttga ggttgaacga caaaccataa ctcagctgag ttttgaactg 960
agtgaatttc gaagaaaata tgaagaaacc caaaaagaag ttcacaattt aaatcagctg 1020
ttgtattcac aaagaagggc agatgtgcaa catctggaag atgataggca taaaacagag 1080
aagatacaaa aactcaggga agagaatgat attgctaggg gaaaacttga agaagagaag 1140
aagagatccg aagagctctt atctcaggtc cagtttcttt acacatctct gctaaagcag 1200
caagaagaac aaacaagggt agctctgttg gaacaacaga tgcaggcatg tactttagac 1260
tttgaaaatg aaaaactcga ccgtcaacat gtgcagcatc aattgcatgt aattcttaag 1320
gagctccgaa aagcaagaaa tcaaataaca cagttggaat ccttgaaaca gcttcatgag 1380
tttgccatca cagagccatt agtcactttc caaggagaga ctgaaaacag agaaaaagtt 1440
gccgcctcac caaaaagtcc cactgctgca ctcaatgaaa gcctggtgga atgtcccaag 1500
tgcaatatac agtatccagc cactgagcat cgcgatctgc ttgtccatgt ggaatactgt 1560
tcaaagtagc aaaataagta tttgttttga tattaaaaga ttcaatactg tattttctgt 1620
tagcttgtgg gcattttgaa ttatatattt cacattttgc ataaaactgc ctatctacct 1680
ttgacactcc agcatgctag tgaatcatgt atcttttagg ctgctgtgca tttctcttgg 1740
cagtgatacc tccctgacat ggttcatcat caggctgcaa tgacagaatg tggtgagcag 1800
cgtctactga gatactaaca ttttgcactg tcaaaatact tggtgaggaa aagatagctc 1860
aggttattgc taatgggtta atgcaccagc aagcaaaata ttttatgttt tggggttttt 1920
gaaaaatcaa agataattaa ccaaggatct taactgtgtt cgcatttttt atccaagcac 1980
ttagaaaacc tacaatccta attttgatgt ccattgttaa gaggtggtga tagatactat 2040
tttttttttc atattgtata gcggttatta gaaaagttgg ggattttctt gatctttatt 2100
gctgcttacc attgaaactt aacccagctg tgttccccaa ctctgttctg cgcacgaaac 2160
agtatctgtt tgaggcataa tcttaagtgg ccacacacaa tgttttctct tatgttatct 2220
ggcagtaact gtaacttgaa ttacattagc acattctgct tagctaaaat tgttaaaata 2280
aactttaata aacccatgta gccctctcat ttgattgaca gtattttagt tatttttggc 2340
attcttaaag ctgggcaatg taatgatcag atctttgttt gtctgaacag gtatttttat 2400
acatgctttt tgtaaaccaa aaacttttaa atttcttcag gttttctaac atgcttacca 2460
```

ctgggctact gta 2473

<210> 79
<211> 2324
<212> DNA
<213> Homo Sapiens

<400> 79
gggaccgcca gggagggcag gtcagtgggc agatcgcgtc cgcgggattc aatctctgcc 60
cgctctgata acagtccttt tccctggcgc tcacttcgtg cctggcaccc ggctgggcgc 120
ctcaagaccg ttgtctcttc gatcgcttct ttggacttgg cgaccatttc agagatgtct 180
tccagaagta ccaaagattt aattaaaaaa aattcgagtc cttgaggctg agaaggagaa 240
gaatgcttat caactcacag agaaggacaa agaaatacag cgactgagag accaactgaa 300
ggccagatat agtactaccg cattgcttga acagctggaa gagacaacga gagaaggaga 360
aaggagggag caggtgttga aagccttatc tgaagagaaa gacgtattga aacaacagtt 420
gtctgctgca acctcacgaa ttgctgaact tgaaagcaaa accaatacac tccgtttatc 480
acagactgtg gctccaaact gcttcaactc atcaataaat aatattcatg aaatggaaat 540
acagctgaaa gatgctctgg agaaaaatca gcagtggctc gtgtatgatc agcagcggga 600
agtctatgta aaaggacttt tagcaaagat ctttgagttg gaaaagaaaa cggaaacagc 660
tgctcattca ctcccacagc agacaaaaaa gcctgaatca gaaggttatc ttcaagaaga 720
gaagcagaaa tgttacaacg atctcttggc aagtgcaaaa aaagatcttg aggttgaacg 780
acaaaccata actcagctga gttttgaact gagtgaattt cgaagaaaat atgaagaaac 840
ccaaaaagaa gttcacaatt taaatcagct gttgtattca caaagaaggg cagatgtgca 900
acatctggaa gatgataggc ataaaacaga gaagatacaa aaactcaggg aagagaatga 960
tattgctagg ggaaaacttg aagaagagaa gaagagatcc gaagagctct tatctcaggt 1020
ccagtttctt tacacatctc tgctaaagca gcaagaagaa caaacaaggg tagctctgtt 1080
ggaacaacag atgcaggcat gtactttaga ctttgaaaat gaaaaactcg accgtcaaca 1140
tgtgcagcat caattgcatg taattcttaa ggagctccga aaagcaagaa atcaaataac 1200
acagttggaa tccttgaaac agcttcatga gtttgccatc acagagccat tagtcacttt 1260
ccaaggagag actgaaaaca gagaaaaagt tgccgcctca ccaaaaagtc ccactgctgc 1320
actcaatgaa agcctggtgg aatgtcccaa gtgcaatata cagtatccag ccactgagca 1380
tcgcgatctg cttgtccatg tggaatactg ttcaaagtag caaaataagt atttgttttg 1440
atattaaaag attcaatact gtattttctg ttagcttgtg ggcattttga attatatatt 1500
tcacattttg cataaaactg cctatctacc tttgacactc cagcatgcta gtgaatcatg 1560
tatcttttag gctgctgtgc atttctcttg gcagtgatac ctccctgaca tggttcatca 1620
tcaggctgca atgacagaat gtggtgagca gcgtctactg agatactaac attttgcact 1680
gtcaaaatac ttggtgagga aaagatagct caggttattg ctaatgggtt aatgcaccag 1740
caagcaaaat attttatgtt ttggggggttt tgaaaaatca aagataatta accaaggatc 1800
ttaactgtgt tcgcattttt tatccaagca cttagaaaac ctacaatcct aattttgatg 1860
tccattgtta agaggtggtg atagatacta ttttttttt catattgtat agcggttatt 1920
agaaaagttg ggatttttct tgatctttat tgctgcttac cattgaaact taacccagct 1980
gtgttcccca actctgttct gcgcacgaaa cagtatctgt ttgaggcata atcttaagtg 2040
gccacacaca atgttttctc ttatgttatc tggcagtaac tgtaacttga attacattag 2100
cacattctgc ttagctaaaa ttgttaaaat aaactttaat aaacccatgt agccctctca 2160
tttgattgac agtattttag ttatttttgg cattcttaaa gctgggcaat gtaatgatca 2220
gatctttgtt tgtctgaaca ggtattttta tacatgcttt ttgtaaacca aaaactttta 2280
aatttcttca ggttttctaa catgcttacc actgggctac tgta 2324

<210> 80
<211> 295
<212> PRT
<213> Homo Sapiens

<400> 80
Met Glu Ile Gln Leu Lys Asp Ala Leu Glu Lys Asn Gln Gln Trp Leu
1 5 10 15
Val Tyr Asp Gln Gln Arg Glu Val Tyr Val Lys Gly Leu Leu Ala Lys
20 25 30
Ile Phe Glu Leu Glu Lys Lys Thr Glu Thr Ala Ala His Ser Leu Pro
35 40 45
Gln Gln Thr Lys Lys Pro Glu Ser Glu Gly Tyr Leu Gln Glu Glu Lys

```
    50                  55                  60
Gln Lys Cys Tyr Asn Asp Leu Leu Ala Ser Ala Lys Lys Asp Leu Glu
65                  70                  75                  80
Val Glu Arg Gln Thr Ile Thr Gln Leu Ser Phe Glu Leu Ser Glu Phe
                85                  90                  95
Arg Arg Lys Tyr Glu Glu Thr Gln Lys Glu Val His Asn Leu Asn Gln
            100                 105                 110
Leu Leu Tyr Ser Gln Arg Arg Ala Asp Val Gln His Leu Glu Asp Asp
        115                 120                 125
Arg His Lys Thr Glu Lys Ile Gln Lys Leu Arg Glu Glu Asn Asp Ile
    130                 135                 140
Ala Arg Gly Lys Leu Glu Glu Glu Lys Lys Arg Ser Glu Glu Leu Leu
145                 150                 155                 160
Ser Gln Val Gln Phe Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
Asp Phe Glu Asn Glu Lys Leu Asp Arg Gln His Val Gln His Gln Leu
        195                 200                 205
His Val Ile Leu Lys Glu Leu Arg Lys Ala Arg Asn Gln Ile Thr Gln
    210                 215                 220
Leu Glu Ser Leu Lys Gln Leu His Glu Phe Ala Ile Thr Glu Pro Leu
225                 230                 235                 240
Val Thr Phe Gln Gly Glu Thr Glu Asn Arg Glu Lys Val Ala Ala Ser
                245                 250                 255
Pro Lys Ser Pro Thr Ala Ala Leu Asn Glu Ser Leu Val Glu Cys Pro
            260                 265                 270
Lys Cys Asn Ile Gln Tyr Pro Ala Thr Glu His Arg Asp Leu Leu Val
        275                 280                 285
His Val Glu Tyr Cys Ser Lys
    290                 295
```

```
<210> 81
<211> 464
<212> PRT
<213> Homo Sapiens

<400> 81
Met Ser Ser Arg Ser Thr Lys Asp Leu Ile Lys Ser Lys Trp Gly Ser
 1               5                  10                  15
Lys Pro Ser Asn Ser Lys Ser Glu Thr Thr Leu Glu Lys Leu Lys Gly
            20                  25                  30
Glu Ile Ala His Leu Lys Thr Ser Val Asp Glu Ile Thr Ser Gly Lys
        35                  40                  45
Gly Lys Leu Thr Asp Lys Glu Arg His Arg Leu Leu Glu Lys Ile Arg
    50                  55                  60
Val Leu Glu Ala Glu Lys Glu Lys Asn Ala Tyr Gln Leu Thr Glu Lys
65                  70                  75                  80
Asp Lys Glu Ile Gln Arg Leu Arg Asp Gln Leu Lys Ala Arg Tyr Ser
                85                  90                  95
Thr Thr Ala Leu Leu Glu Gln Leu Glu Glu Thr Thr Arg Glu Gly Glu
            100                 105                 110
Arg Arg Glu Gln Val Leu Lys Ala Leu Ser Glu Glu Lys Asp Val Leu
        115                 120                 125
Lys Gln Gln Leu Ser Ala Ala Thr Ser Arg Ile Ala Glu Leu Glu Ser
    130                 135                 140
Lys Thr Asn Thr Leu Arg Leu Ser Gln Thr Val Ala Pro Asn Cys Phe
145                 150                 155                 160
Asn Ser Ser Ile Asn Asn Ile His Glu Met Glu Ile Gln Leu Lys Asp
                165                 170                 175
```

Ala Leu Glu Lys Asn Gln Gln Trp Leu Val Tyr Asp Gln Gln Arg Glu
180 185 190
Val Tyr Val Lys Gly Leu Leu Ala Lys Ile Phe Glu Leu Glu Lys Lys
195 200 205
Thr Glu Thr Ala Ala His Ser Leu Pro Gln Gln Thr Lys Lys Pro Glu
210 215 220
Ser Glu Gly Tyr Leu Gln Glu Glu Lys Gln Lys Cys Tyr Asn Asp Leu
225 230 235 240
Leu Ala Ser Ala Lys Lys Asp Leu Glu Val Glu Arg Gln Thr Ile Thr
245 250 255
Gln Leu Ser Phe Glu Leu Ser Glu Phe Arg Arg Lys Tyr Glu Glu Thr
260 265 270
Gln Lys Glu Val His Asn Leu Asn Gln Leu Leu Tyr Ser Gln Arg Arg
275 280 285
Ala Asp Val Gln His Leu Glu Asp Asp Arg His Lys Thr Glu Lys Ile
290 295 300
Gln Lys Leu Arg Glu Glu Asn Asp Ile Ala Arg Gly Lys Leu Glu Glu
305 310 315 320
Glu Lys Lys Arg Ser Glu Glu Leu Leu Ser Gln Val Gln Phe Leu Tyr
325 330 335
Thr Ser Leu Leu Lys Gln Gln Glu Glu Gln Thr Arg Val Ala Leu Leu
340 345 350
Glu Gln Gln Met Gln Ala Cys Thr Leu Asp Phe Glu Asn Glu Lys Leu
355 360 365
Asp Arg Gln His Val Gln His Gln Leu His Val Ile Leu Lys Glu Leu
370 375 380
Arg Lys Ala Arg Asn Gln Ile Thr Gln Leu Glu Ser Leu Lys Gln Leu
385 390 395 400
His Glu Phe Ala Ile Thr Glu Pro Leu Val Thr Phe Gln Gly Glu Thr
405 410 415
Glu Asn Arg Glu Lys Val Ala Ala Ser Pro Lys Ser Pro Thr Ala Ala
420 425 430
Leu Asn Glu Ser Leu Val Glu Cys Pro Lys Cys Asn Ile Gln Tyr Pro
435 440 445
Ala Thr Glu His Arg Asp Leu Leu Val His Val Glu Tyr Cys Ser Lys
450 455 460

<210> 82
<211> 295
<212> PRT
<213> Homo Sapiens

<400> 82
Met Glu Ile Gln Leu Lys Asp Ala Leu Glu Lys Asn Gln Gln Trp Leu
1 5 10 15
Val Tyr Asp Gln Gln Arg Glu Val Tyr Val Lys Gly Leu Leu Ala Lys
20 25 30
Ile Phe Glu Leu Glu Lys Lys Thr Glu Thr Ala Ala His Ser Leu Pro
35 40 45
Gln Gln Thr Lys Lys Pro Glu Ser Glu Gly Tyr Leu Gln Glu Glu Lys
50 55 60
Gln Lys Cys Tyr Asn Asp Leu Leu Ala Ser Ala Lys Lys Asp Leu Glu
65 70 75 80
Val Glu Arg Gln Thr Ile Thr Gln Leu Ser Phe Glu Leu Ser Glu Phe
85 90 95
Arg Arg Lys Tyr Glu Glu Thr Gln Lys Glu Val His Asn Leu Asn Gln
100 105 110
Leu Leu Tyr Ser Gln Arg Arg Ala Asp Val Gln His Leu Glu Asp Asp
115 120 125
Arg His Lys Thr Glu Lys Ile Gln Lys Leu Arg Glu Glu Asn Asp Ile

```
    130                 135                 140
Ala Arg Gly Lys Leu Glu Glu Glu Lys Lys Arg Ser Glu Glu Leu Leu
145                 150                 155                 160
Ser Gln Val Gln Phe Leu Tyr Thr Ser Leu Leu Lys Gln Gln Glu Glu
                165                 170                 175
Gln Thr Arg Val Ala Leu Leu Glu Gln Gln Met Gln Ala Cys Thr Leu
            180                 185                 190
Asp Phe Glu Asn Glu Lys Leu Asp Arg Gln His Val Gln His Gln Leu
        195                 200                 205
His Val Ile Leu Lys Glu Leu Arg Lys Ala Arg Asn Gln Ile Thr Gln
    210                 215                 220
Leu Glu Ser Leu Lys Gln Leu His Glu Phe Ala Ile Thr Glu Pro Leu
225                 230                 235                 240
Val Thr Phe Gln Gly Glu Thr Glu Asn Arg Glu Lys Val Ala Ala Ser
                245                 250                 255
Pro Lys Ser Pro Thr Ala Ala Leu Asn Glu Ser Leu Val Glu Cys Pro
            260                 265                 270
Lys Cys Asn Ile Gln Tyr Pro Ala Thr Glu His Arg Asp Leu Leu Val
        275                 280                 285
His Val Glu Tyr Cys Ser Lys
    290                 295
```

## Description

### CROSS-REFERENCE TO RELATED APPLICATIONS

This application claims priority benefit of United States Provisional Patent Application Serial No. 60/282,739 filed April 10, 2001, United States Provisional Application Serial No. 60/286,630, filed April 25, 2001, and United States Provisional Patent Application Serial No. 60/300,373, filed June 22, 2001. The contents of these applications are hereby incorporated by reference herein in their entirety.

### STATEMENT OF RIGHTS TO INVENTIONS MADE UNDER FEDERALLY SPONSORED RESEARCH

Not applicable.

### FIELD OF THE INVENTION

The invention described herein relates to a gene and its encoded protein, termed 121P2A3, expressed in certain cancers, and to diagnostic and therapeutic methods and compositions useful in the management of cancers that express 121P2A3.

### BACKGROUND OF THE INVENTION

Cancer is the second leading cause of human death next to coronary disease. Worldwide, millions of people die from cancer every year. In the United States alone, as reported by the American Cancer Society, cancer causes the death of well over a half-million people annually, with over 1.2 million new cases diagnosed per year. While deaths from heart disease have been declining significantly, those resulting from cancer generally are on the rise. In the early part of the next century, cancer is predicted to become the leading cause of death.

Worldwide, several cancers stand out as the leading killers. In particular, carcinomas of the lung, prostate, breast, colon, pancreas, and ovary represent the primary causes of cancer death. These and virtually all other carcinomas share a common lethal feature. With very few exceptions, metastatic disease from a carcinoma is fatal. Moreover, even for those cancer patients who initially survive their primary cancers, common experience has shown that their lives are dramatically altered. Many cancer patients experience strong anxieties driven by the awareness of the potential for recurrence or treatment failure. Many cancer patients experience physical debilitations following treatment. Furthermore, many cancer patients experience a recurrence.

Worldwide, prostate cancer is the fourth most prevalent cancer in men. In North America and Northern Europe, it is by far the most common cancer in males and is the second leading cause of cancer death in men. In the United States alone, well over 30,000 men die annually of this disease - second only to lung cancer. Despite the magnitude of these figures, there is still no effective treatment for metastatic prostate cancer. Surgical prostatectomy, radiation therapy, hormone ablation therapy, surgical castration and chemotherapy continue to be the main treatment modalities. Unfortunately, these treatments are ineffective for many and are often associated with undesirable consequences.

On the diagnostic front, the lack of a prostate tumor marker that can accurately detect early-stage, localized tumors remains a significant limitation in the diagnosis and management of this disease. Although the serum prostate specific antigen (PSA) assay has been a very useful tool, however its specificity and general utility is widely regarded as lacking in several important respects.

Progress in identifying additional specific markers for prostate cancer has been improved by the generation of prostate cancer xenografts that can recapitulate different stages of the disease in mice. The LAPC (Los Angeles Prostate Cancer) xenografts are prostate cancer xenografts that have survived passage in severe combined immune deficient (SCID) mice and have exhibited the capacity to mimic the transition from androgen dependence to androgen independence (Klein et al., 1997, Nat. Med. 3:402). More recently identified prostate cancer markers include PCTA-1 (Su et al., 1996, Proc. Natl. Acad. Sci. USA 93: 7252), prostate-specific membrane (PSM) antigen (Pinto et al., Clin Cancer Res 1996 Sep 2 (9): 1445-51), STEAP (Hubert, et al., Proc Natl Acad Sci U S A. 1999 Dec 7; 96(25): 14523-8) and prostate stem cell antigen (PSCA) (Reiter et al., 1998, Proc. Natl. Acad. Sci. USA 95: 1735).

While previously identified markers such as PSA, PSM, PCTA and PSCA have facilitated efforts to diagnose and treat prostate cancer, there is need for the identification of additional markers and therapeutic targets for prostate and related cancers in order to further improve diagnosis and therapy.

Renal cell carcinoma (RCC) accounts for approximately 3 percent of adult malignancies. Once adenomas reach a diameter of 2 to 3 cm, malignant potential exists. In the adult, the two principal malignant renal tumors are renal cell adenocarcinoma and transitional cell carcinoma of the renal pelvis or ureter. The incidence of renal cell adenocarcinoma is estimated at more than 29,000 cases in the United States, and more than 11,600 patients died of this disease in 1998. Transitional cell carcinoma is less frequent, with an incidence of approximately 500 cases per year in the United States.

Surgery has been the primary therapy for renal cell adenocarcinoma for many decades. Until recently, metastatic disease has been refractory to any systemic therapy. With recent developments in systemic therapies, particularly immunotherapies, metastatic renal cell carcinoma may be approached aggressively in appropriate patients with a possibility of durable responses. Nevertheless, there is a remaining need for effective therapies for these patients.

Of all new cases of cancer in the United States, bladder cancer represents approximately 5 percent in men (fifth most common neoplasm) and 3 percent in women (eighth most common neoplasm). The incidence is increasing slowly, concurrent with an increasing older population. In 1998, there was an estimated 54,500 cases, including 39,500 in men and 15,000 in women. The age-adjusted incidence in the United States is 32 per 100,000 for men and 8 per 100,000 in women. The historic male/female ratio of 3:1 may be decreasing related to smoking patterns in women. There were an estimated 11,000 deaths from bladder cancer in 1998 (7,800 in men and 3,900 in women). Bladder cancer incidence and mortality strongly increase with age and will be an increasing problem as the population becomes more elderly.

Most bladder cancers recur in the bladder. Bladder cancer is managed with a combination of transurethral resection of the bladder (TUR) and intravesical chemotherapy or immunotherapy. The multifocal and recurrent nature of bladder cancer points out the limitations of TUR. Most muscle-invasive cancers are not cured by TUR alone. Radical cystectomy and urinary diversion is the most effective means to eliminate the cancer but carry an undeniable impact on urinary and sexual function. There continues to be a significant need for treatment modalities that are beneficial for bladder cancer patients.

An estimated 130,200 cases of colorectal cancer occurred in 2000 in the United States, including 93,800 cases of colon cancer and 36,400 of rectal cancer. Colorectal cancers are the third most common cancers in men and women. Incidence rates declined significantly during 1992-1996 (-2.1% per year). Research suggests that these declines have been due to increased screening and polyp removal, preventing progression of polyps to invasive cancers. There were an estimated 56,300 deaths (47,700 from colon cancer, 8,600 from rectal cancer) in 2000, accounting for about 11% of all U.S. cancer deaths.

At present, surgery is the most common form of therapy for colorectal cancer, and for cancers that have not spread, it is frequently curative. Chemotherapy, or chemotherapy plus radiation, is given before or after surgery to most patients whose cancer has deeply perforated the bowel wall or has spread to the lymph nodes. A permanent colostomy (creation of an abdominal opening for elimination of body wastes) is occasionally needed for colon cancer and is infrequently required for rectal cancer. There continues to be a need for effective diagnostic and treatment modalities for colorectal cancer.

There were an estimated 164,100 new cases of lung and bronchial cancer in 2000, accounting for 14% of all U.S. cancer diagnoses. The incidence rate of lung and bronchial cancer is declining significantly in men, from a high of 86.5 per 100,000 in 1984 to 70.0 in 1996. In the 1990s, the rate of increase among women began to slow. In 1996, the incidence rate in women was 42.3 per 100,000.

Lung and bronchial cancer caused an estimated 156,900 deaths in 2000, accounting for 28% of all cancer deaths. During 1992-1996, mortality from lung cancer declined significantly among men (-1.7% per year) while rates for women were still significantly increasing (0.9% per year). Since 1987, more women have died each year of lung cancer than breast cancer, which, for over 40 years, was the major cause of cancer death in women. Decreasing lung cancer incidence and mortality rates most likely resulted from decreased smoking rates over the previous 30 years; however, decreasing smoking patterns among women lag behind those of men. Of concern, although the declines in adult tobacco use have slowed, tobacco use in youth is increasing again.

Treatment options for lung and bronchial cancer are determined by the type and stage of the cancer and include surgery, radiation therapy, and chemotherapy. For many localized cancers, surgery is usually the treatment of choice. Because the disease has usually spread by the time it is discovered, radiation therapy and chemotherapy are often needed in combination with surgery. Chemotherapy alone or combined with radiation is the treatment of choice for small cell lung cancer; on this regimen, a large percentage of patients experience remission, which in some cases is long lasting. There is however, an ongoing need for effective treatment and diagnostic approaches for lung and bronchial cancers.

An estimated 182,800 new invasive cases of breast cancer were expected to occur among women in the United States during 2000. Additionally, about 1,400 new cases of breast cancer were expected to be diagnosed in men in 2000. After increasing about 4% per year in the 1980s, breast cancer incidence rates in women have leveled off in the 1990s to about 110.6 cases per 100,000.

In the U.S. alone, there were an estimated 41,200 deaths (40,800 women, 400 men) in 2000 due to breast cancer. Breast cancer ranks second among cancer deaths in women. According to the most recent data, mortality rates declined significantly during 1992-1996 with the largest decreases in younger women, both white and black. These decreases were probably the result of earlier detection and improved treatment.

Taking into account the medical circumstances and the patient's preferences, treatment of breast cancer may involve lumpectomy (local removal of the tumor) and removal of the lymph nodes under the arm; mastectomy (surgical removal of the breast) and removal of the lymph nodes under the arm; radiation therapy; chemotherapy; or hormone therapy. Often, two or more methods are used in combination. Numerous studies have shown that, for early stage disease, long-term survival rates after lumpectomy plus radiotherapy are similar to survival rates after modified radical mastectomy. Significant advances in reconstruction techniques provide several options for breast reconstruction after mastectomy. Recently, such reconstruction has been done at the same time as the mastectomy.

Local excision of ductal carcinoma *in situ* (DCIS) with adequate amounts of surrounding normal breast tissue may prevent the local recurrence of the DCIS. Radiation to the breast and/or tamoxifen may reduce the chance of DCIS occurring in the remaining breast tissue. This is important because DCIS, if left untreated, may develop into invasive breast cancer. Nevertheless, there are serious side effects or sequelae to these treatments. There is, therefore, a need for efficacious breast cancer treatments.

There were an estimated 23,100 new cases of ovarian cancer in the United States in 2000. It accounts for 4% of all cancers among women and ranks second among gynecologic cancers. During 1992-1996, ovarian cancer incidence rates were significantly declining. Consequent to ovarian cancer, there were an estimated 14,000 deaths in 2000. Ovarian cancer causes more deaths than any other cancer of the female reproductive system.

Surgery, radiation therapy, and chemotherapy are treatment options for ovarian cancer. Surgery usually includes the removal of one or both ovaries, the fallopian tubes (salpingo-oophorectomy), and the uterus (hysterectomy). In some very early tumors, only the involved ovary will be removed, especially in young women who wish to have children. In advanced disease, an attempt is made to remove all intra-abdominal disease to enhance the effect of chemotherapy. There continues to be an important need for effective treatment options for ovarian cancer.

There were an estimated 28,300 new cases of pancreatic cancer in the United States in 2000. Over the past 20 years, rates of pancreatic cancer have declined in men. Rates among women have remained approximately constant but may be beginning to decline. Pancreatic cancer caused an estimated 28,200 deaths in 2000 in the United States. Over the past 20 years, there has been a slight but significant decrease in mortality rates among men (about -0.9% per year) while rates have increased slightly among women.

Surgery, radiation therapy, and chemotherapy are treatment options for pancreatic cancer. These treatment options can extend survival and/or relieve symptoms in many patients but are not likely to produce a cure for most. There is a significant need for additional therapeutic and diagnostic options for pancreatic cancer.

## SUMMARY OF THE INVENTION

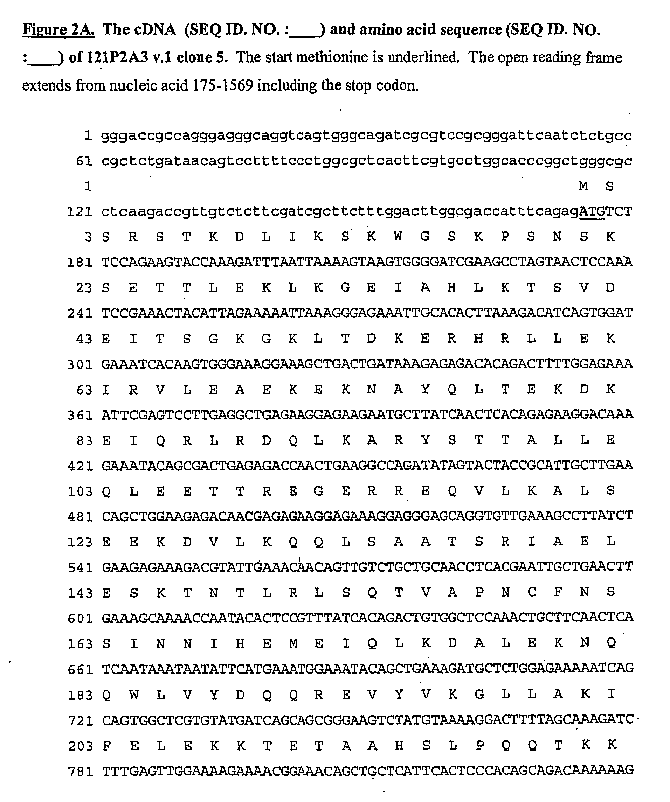

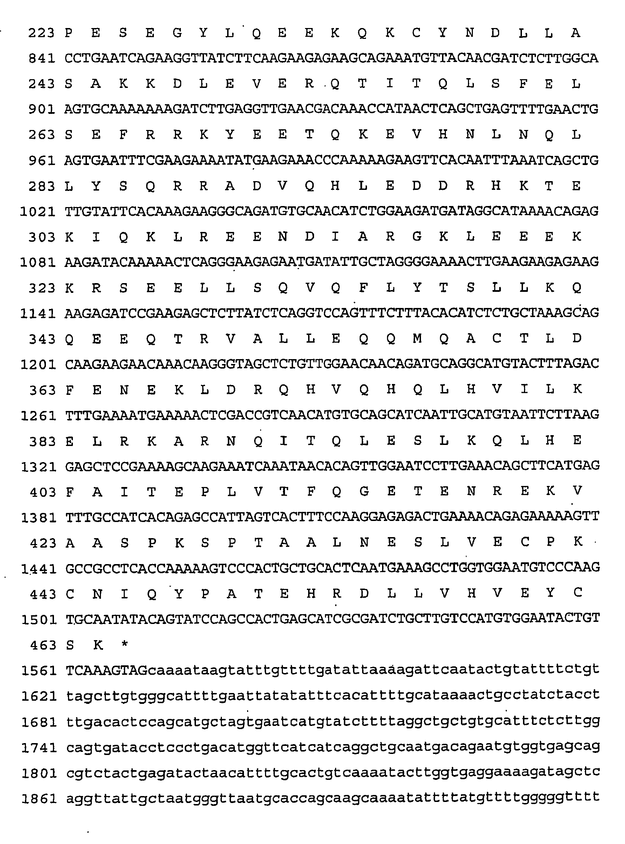

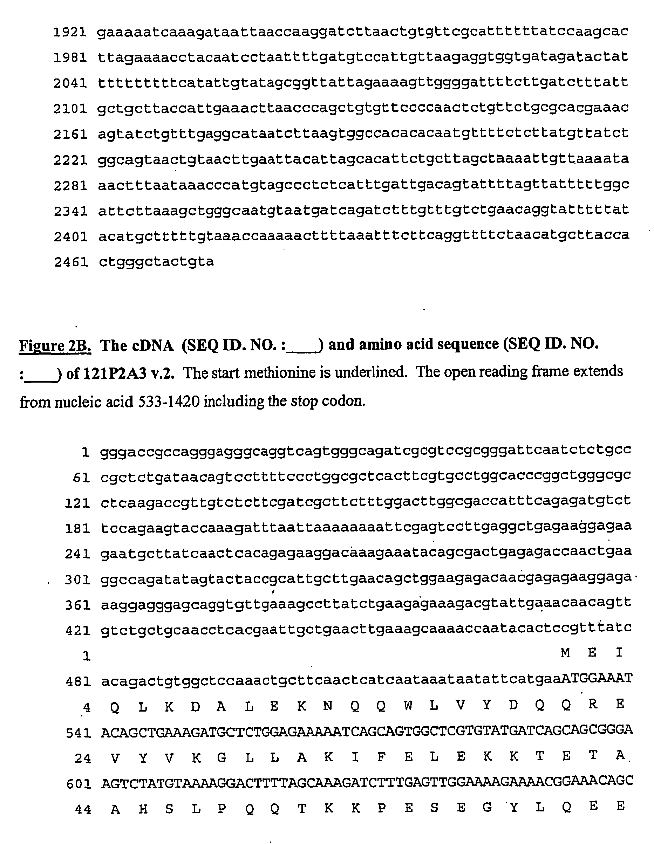

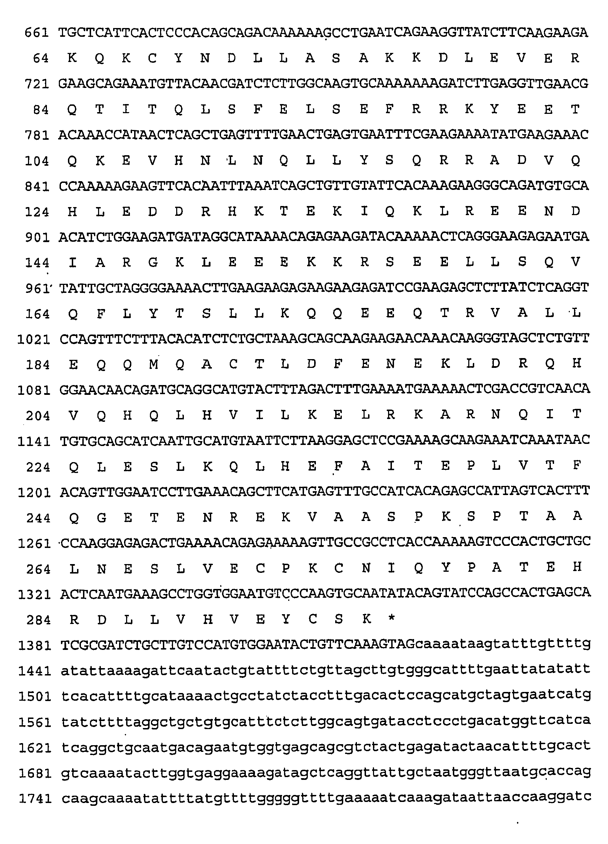

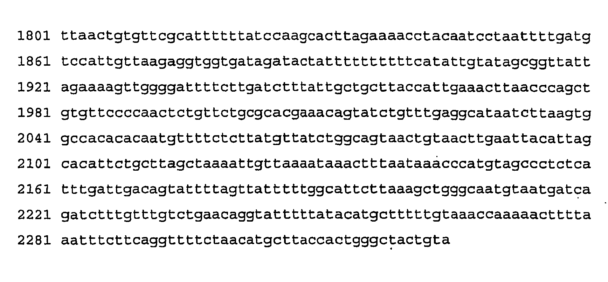

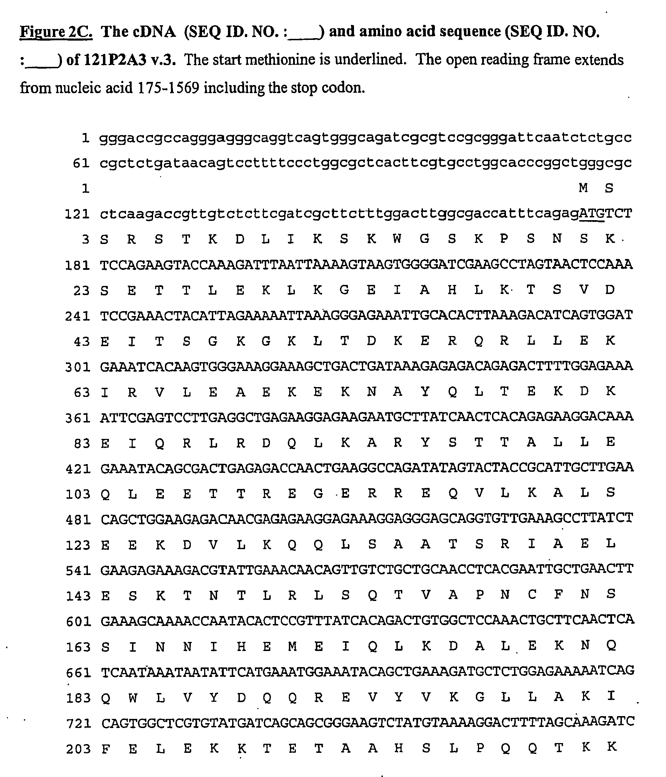

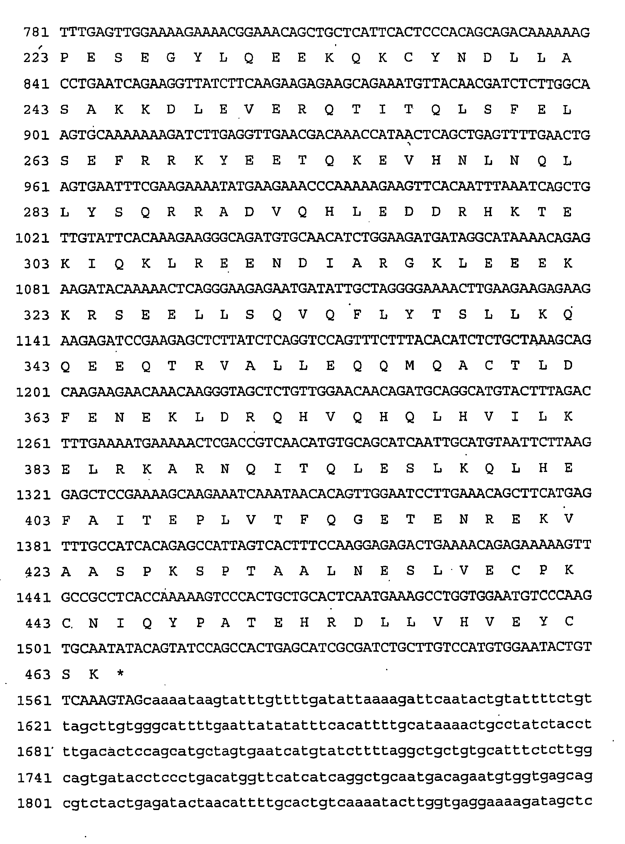

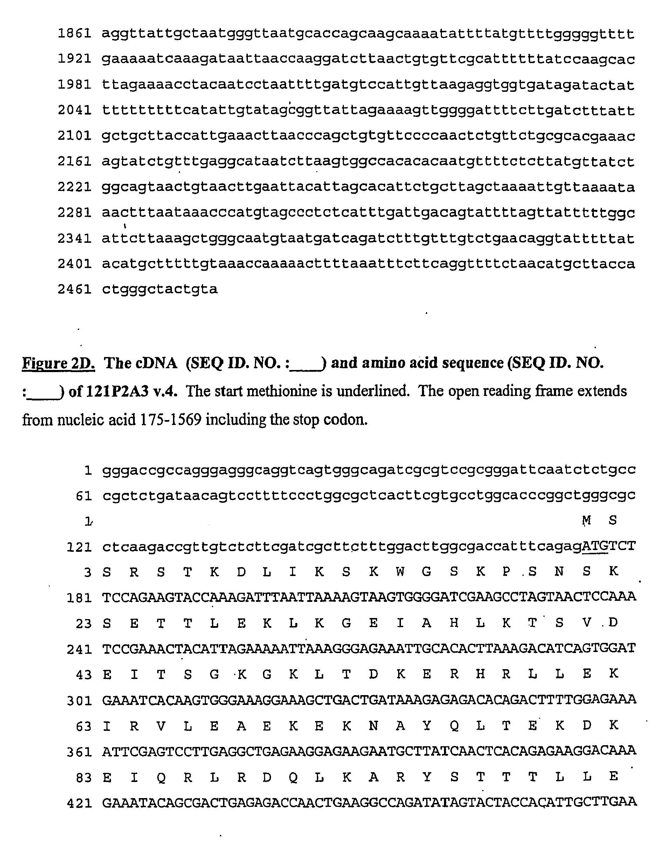

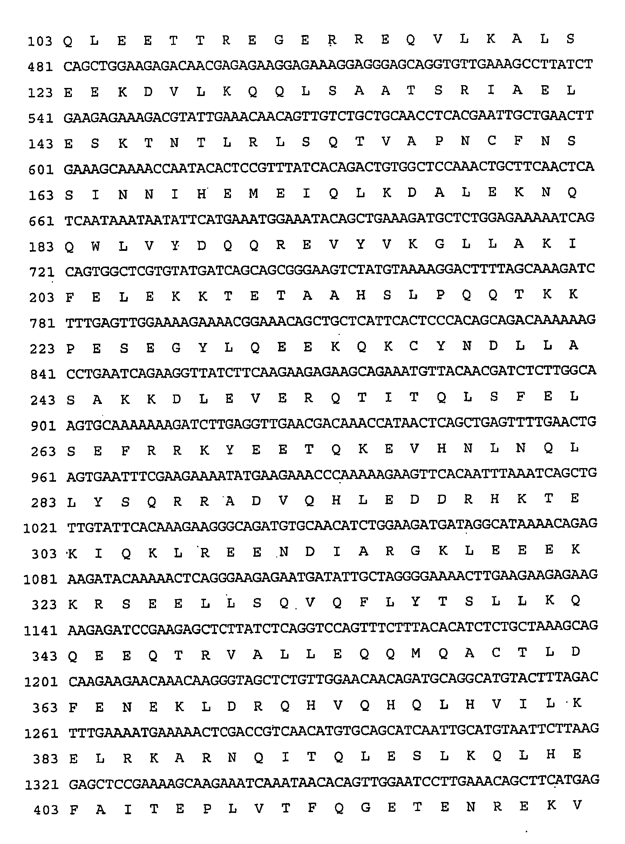

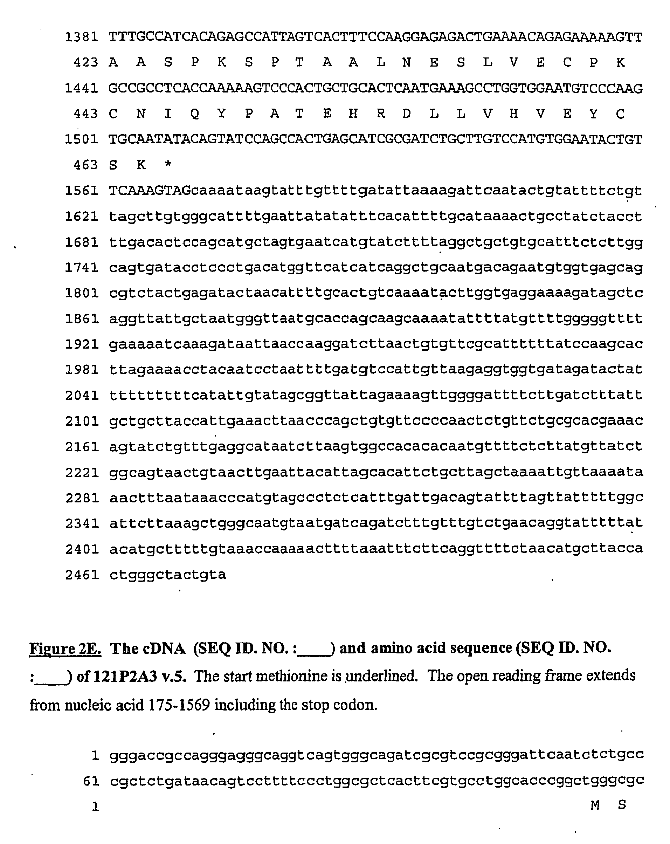

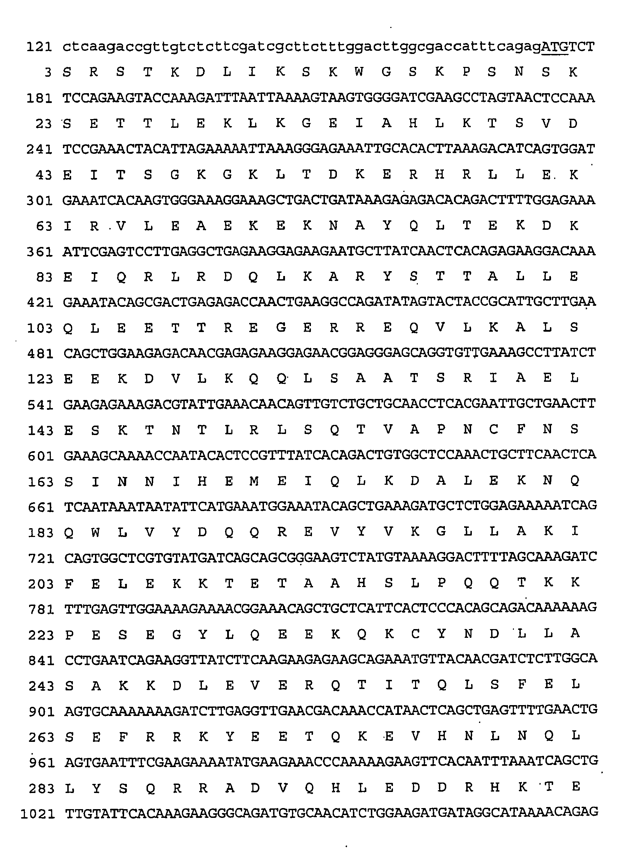

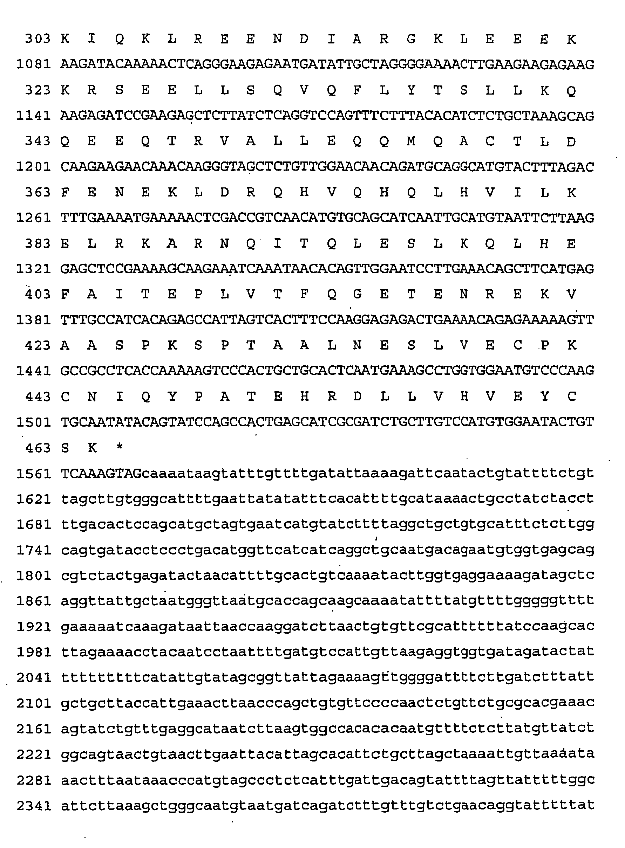

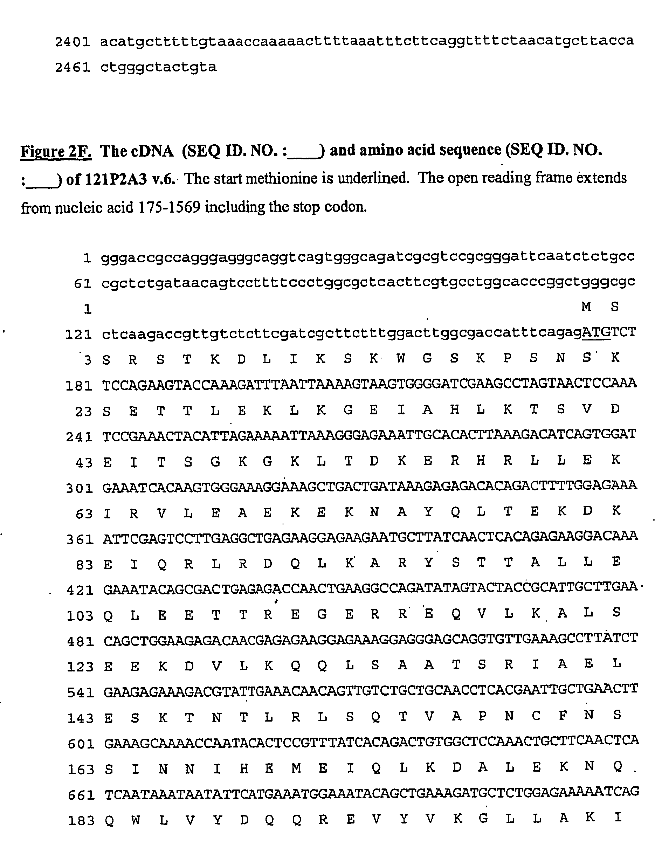

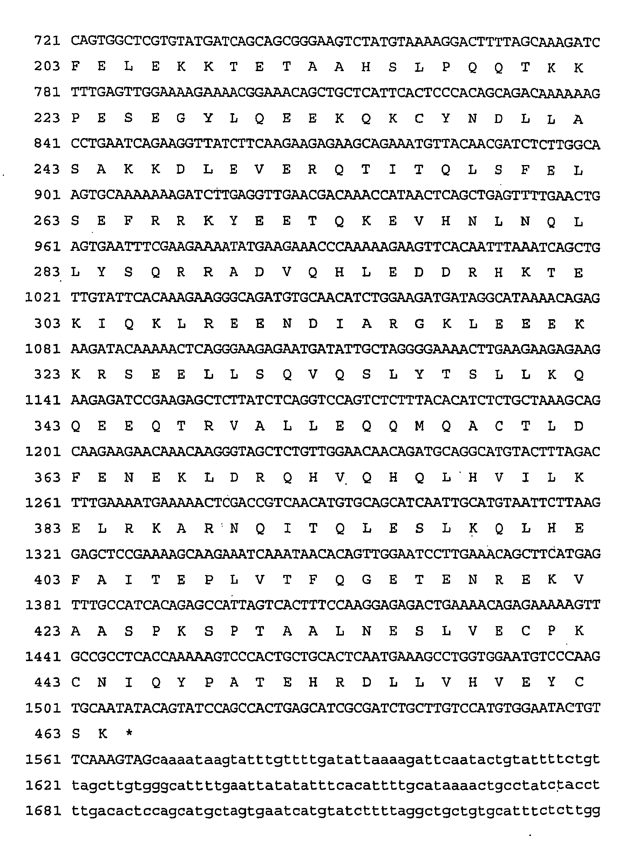

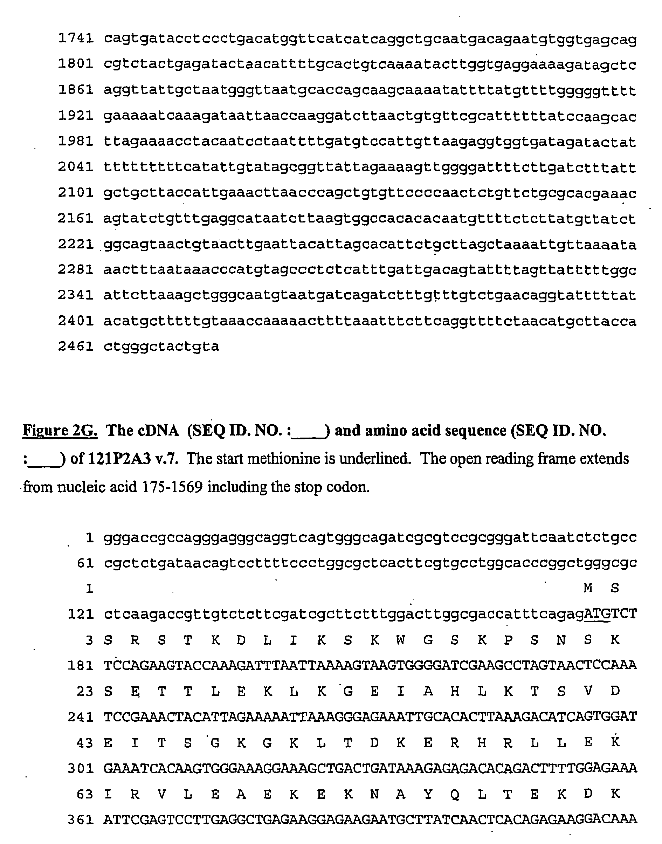

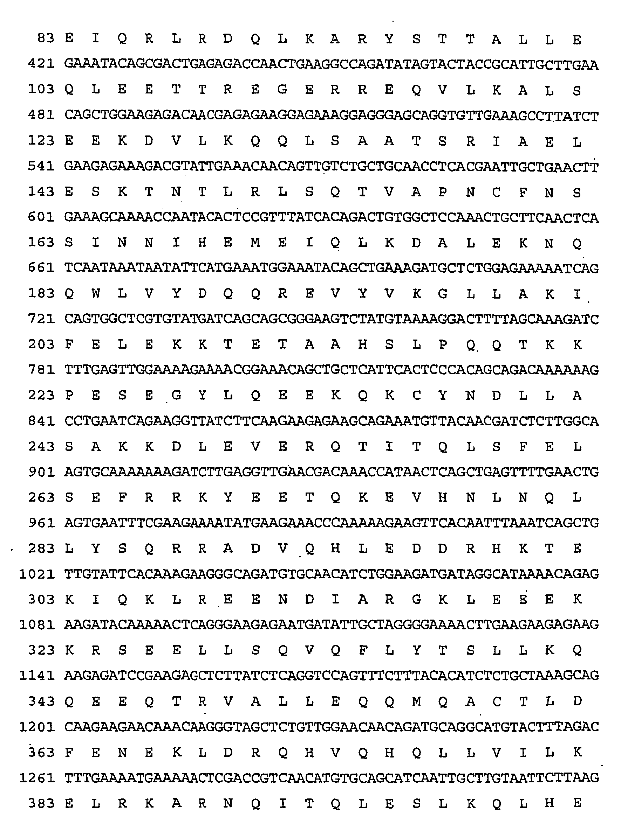

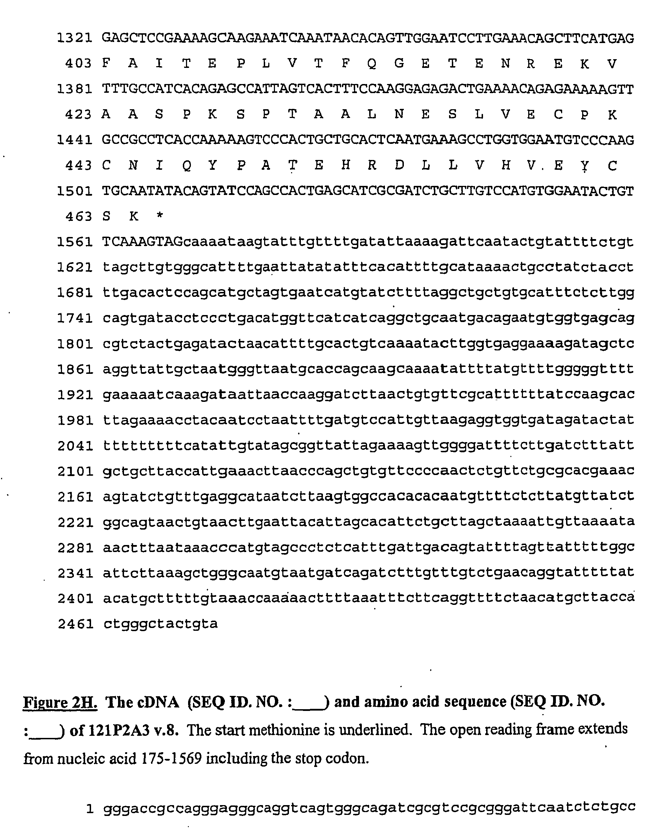

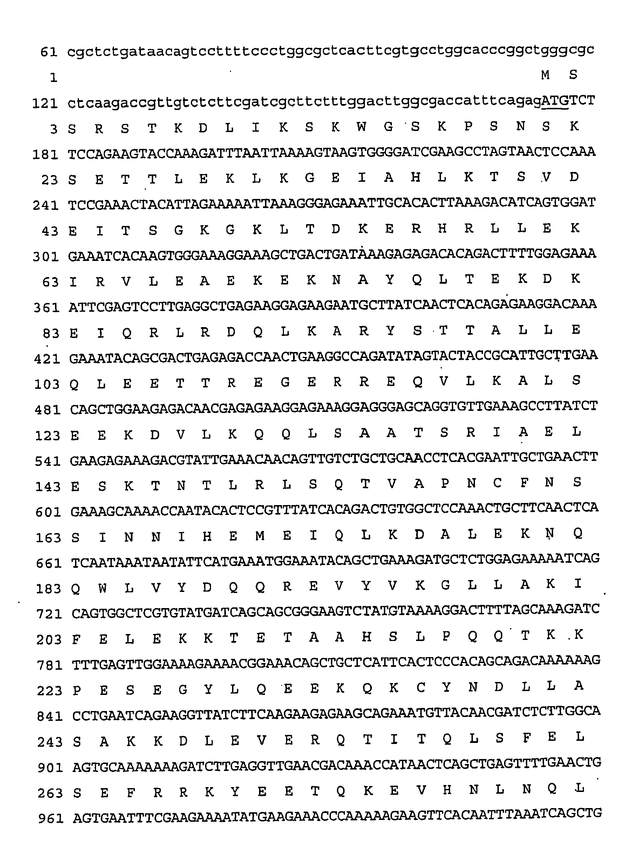

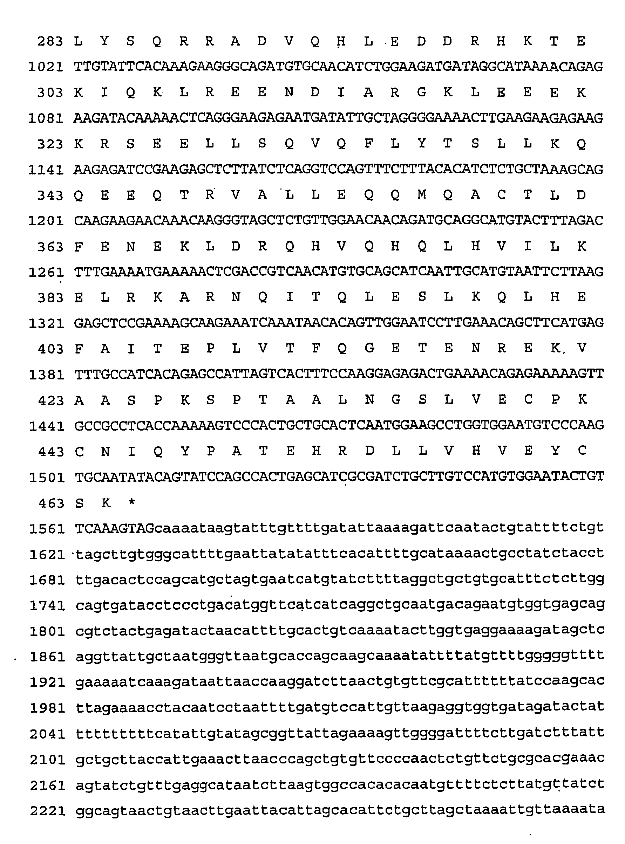

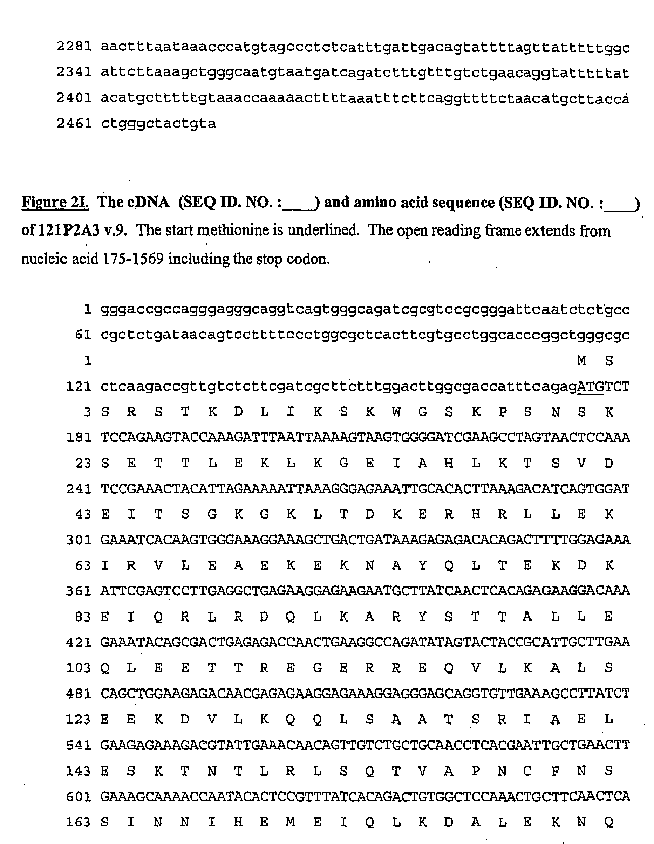

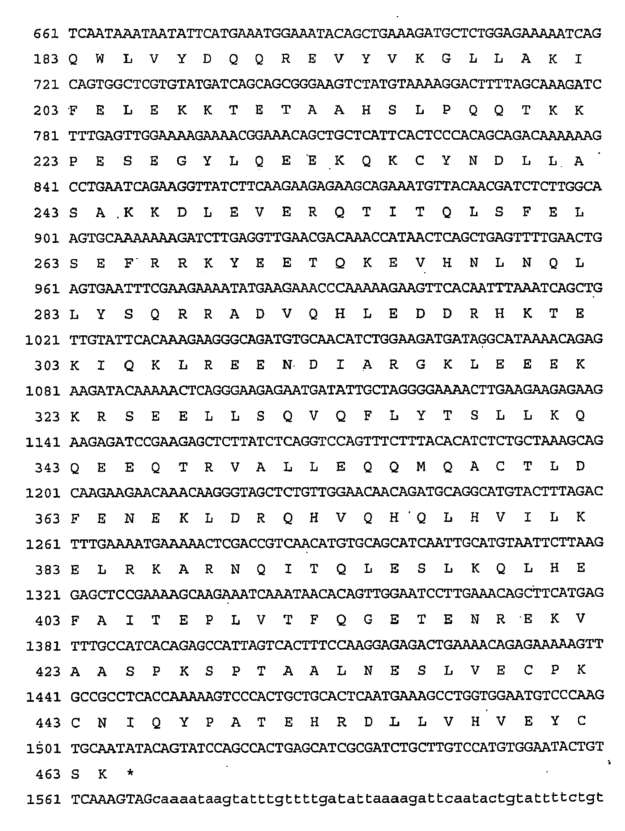

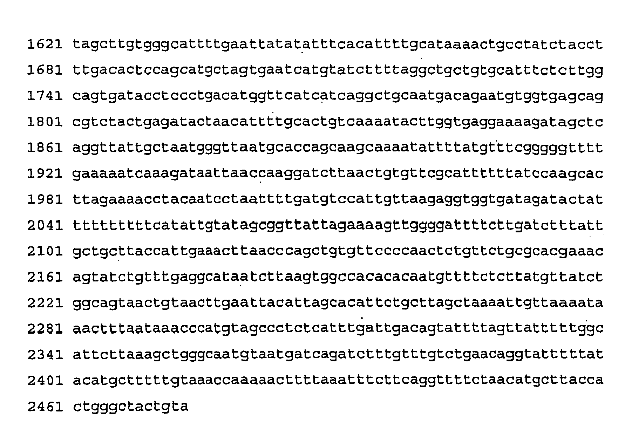

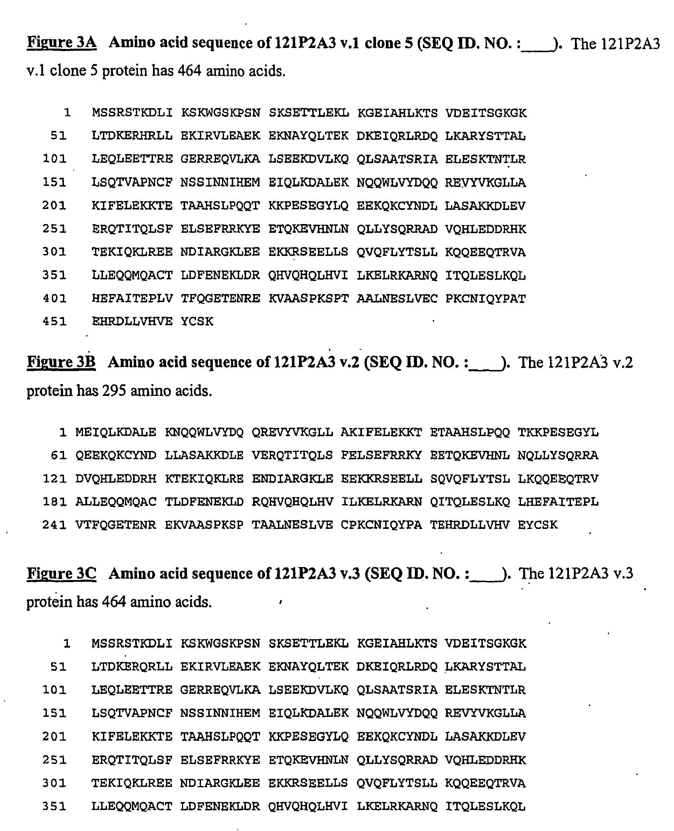

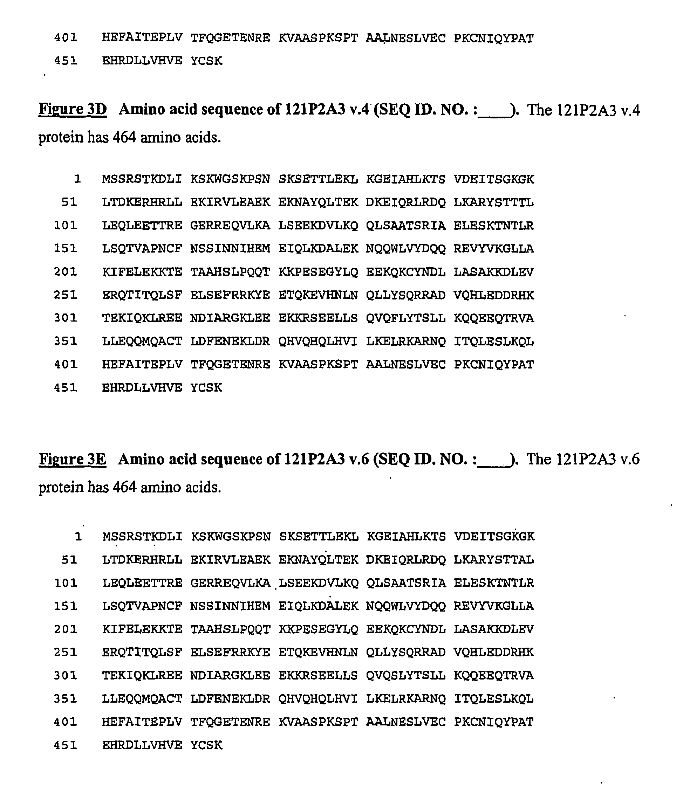

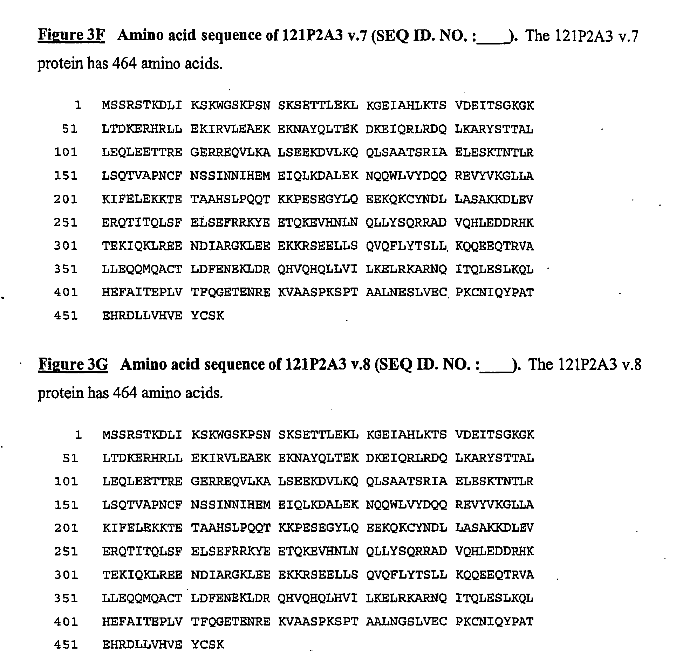

The present invention relates to a gene, designated 121P2A3, that has now been found to be over-expressed in the cancer(s) listed in Table I. Northern blot expression analysis of 121P2A3 gene expression in normal tissues shows a restricted expression pattern in adult tissues. The nucleotide (Figure 2) and amino acid (Figure 2, and Figure 3) sequences of 121P2A3 are provided. The tissue-related profile of 121P2A3 in normal adult tissues, combined with the over-expression observed in the tissues listed in Table I, shows that 121P2A3 is aberrantly over-expressed in at least some cancers, and thus serves as a useful diagnostic, prophylactic, prognostic, and/or therapeutic target for cancers of the tissue(s) such as those listed in Table I.

The invention provides polynucleotides corresponding or complementary to all or part of the 121P2A3 genes, mRNAs, and/or coding sequences, preferably in isolated form, including polynucleotides encoding 121P2A3-related proteins and fragments of 4, 5, 6, 7, 8, 9, 10, 11, 12, 13, 14, 15, 16, 17, 18, 19, 20, 21, 22, 23, 24, 25, or more than 25 contiguous amino acids; at least 30, 35, 40, 45, 50, 55, 60, 65, 70, 80, 85, 90, 95, 100 or more than 100 contiguous amino acids of a 121P2A3-related protein, as well as the peptides/proteins themselves; DNA, RNA, DNA/RNA hybrids, and related molecules, polynucleotides or oligonucleotides complementary or having at least a 90% homology to the 121P2A3 genes or mRNA sequences or parts thereof, and polynucleotides or oligonucleotides that hybridize to the 121P2A3 genes, mRNAs, or to 121P2A3-encoding polynucleotides. Also provided are means for isolating cDNAs and the genes encoding 121P2A3. Recombinant DNA molecules containing 121P2A3 polynucleotides, cells transformed or transduced with such molecules, and host-vector systems for the expression of 121P2A3 gene products are also provided. The invention further provides antibodies that bind to 121P2A3 proteins and polypeptide fragments thereof, including polyclonal and monoclonal antibodies, murine and other mammalian antibodies, chimeric antibodies, humanized and fully human antibodies, and antibodies labeled with a detectable marker or therapeutic agent. In certain embodiments there is a *proviso* that the entire nucleic acid sequence of Figure 2 is not encoded and/or the entire amino acid sequence of Figure 2 is not prepared. In certain embodiments, the entire nucleic acid sequence of Figure 2 is encoded and/or the entire amino acid sequence of Figure 2 is prepared, either of which are in respective human unit dose forms.

The invention further provides methods for detecting the presence and status of 121P2A3 polynucleotides and proteins in various biological samples, as well as methods for identifying cells that express 121P2A3. A typical embodiment of this invention provides methods for monitoring 121P2A3 gene products in a tissue or hematology sample having or suspected of having some form of growth dysregulation such as cancer.

The invention further provides various immunogenic or therapeutic compositions and strategies for treating cancers that express 121P2A3 such as cancers of tissues listed in Table I, including therapies aimed at inhibiting the transcription, translation, processing or function of 121P2A3 as well as cancer vaccines. In one aspect, the invention provides compositions, and methods comprising them, for treating a cancer that expresses 121P2A3 in a human subject wherein the composition comprises a carrier suitable for human use and a human unit dose of one or more than one agent that inhibits the production or function of 121P2A3. Preferably, the carrier is a uniquely human carrier. In another aspect of the invention, the agent is a moiety that is immunoreactive with 121P2A3 protein. Non-limiting examples of such moieties include, but are not limited to, antibodies (such as single chain, monoclonal, polyclonal, humanized, chimeric, or human antibodies), functional equivalents thereof (whether naturally occurring or synthetic), and combinations thereof. The antibodies can be conjugated to a diagnostic or therapeutic moiety. In another aspect, the agent is a small molecule as defined herein.

In another aspect, the agent comprises one or more than one peptide which comprises a cytotoxic T lymphocyte (CTL) epitope that binds an HLA class I molecule in a human to elicit a CTL response to 121P2A3 and/or one or more than one peptide which comprises a helper T lymphocyte (HTL) epitope which binds an HLA class II molecule in a human to elicit an HTL response. The peptides of the invention may be on the same or on one or more separate polypeptide molecules. In a further aspect of the invention, the agent comprises one or more than one nucleic acid molecule that expresses one or more than one of the CTL or HTL response stimulating peptides as described above. In yet another aspect of the invention, the one or more than one nucleic acid molecule may express a moiety that is immunologically reactive with 121P2A3 as described above. The one or more than one nucleic acid molecule may also be, or encodes, a molecule that inhibits production of 121P2A3. Non-limiting examples of such molecules include, but are not limited to, those complementary to a nucleotide sequence essential for production of 121P2A3 (e.g. antisense sequences or molecules that form a triple helix with a nucleotide double helix essential for 121P2A3 production) or a ribozyme effective to lyse 121P2A3 mRNA.

Note: To determine the starting position of any peptide set forth in Tables V-XVIII and XXII to LI (collectively HLA Peptide Tables) respective to its parental protein, e.g., variant 1, variant 2, etc., reference is made to three factors: the particular variant, the length of the peptide in an HLA Peptide Table, and the Search Peptides in Table LII. Generally, a unique Search Peptide is used to obtain HLA peptides of a partiular for a particular variant. The position of each Search Peptide relative to its respective parent molecule is listed in Table LII. Accordingly if a Search Peptide begins at position "X", one must add the value "X - 1" to each position in Tables V-XVIII and XXII to LI to obtain the actual position of the HLA peptides in their parental molecule. For example if a particular Search Peptide begins at position 150 of is parental molecule, one must add 150-1, i.e., 149 to each HLA peptide amino acid position to calculate the position of that amino acid in the parent molecule.

## BRIEF DESCRIPTION OF THE FIGURES

**Figure 1.** The 121P2A3 SSH sequence of 259 nucleotides.

**Figure 2A.** The cDNA (SEQ ID. NO.:__________________) and amino acid sequence (SEQ ID. NO.:__________________) of 121P2A v.1 clone 5. The start methionine is underlined. The open reading frame extends from nucleic acid 175-1569 including the stop codon.

**Figure 2B.** The cDNA (SEQ ID. NO.:__________________) and amino acid sequence (SEQ ID. NO.:__________________) of 121P2A v.2. The start methionine is underlined. The open reading frame extends from nucleic acid 533-1420 including the stop codon.

**Figure 2C.** The cDNA (SEQ ID. NO.:__________________) and amino acid sequence (SEQ ID. NO.:__________________) of 121P2A: v.3. The start methionine is underlined. The open reading frame extends from nucleic acid 175-1569 including the stop codon.

**Figure 2D.** The cDNA (SEQ ID. NO.:__________________) and amino acid sequence (SEQ ID. NO.:__________________) of 121P2A3 v.4. The start methionine is underlined. The open reading frame extends from nucleic acid 175-1569 including the stop codon.

**Figure 2E.** The cDNA (SEQ ID. NO.:__________________) and amino acid sequence (SEQ ID. NO.:__________________) of 121P2A3 v.5. The start methionine is underlined. The open reading frame extends from nucleic acid 175-1569 including the stop codon.

**Figure 2F.** The cDNA (SEQ ID. NO.:__________________) and amino acid sequence (SEQ ID. NO.:__________________) of 121P2A3 v.6. The start methionine is underlined. The open reading frame extends from nucleic acid 175-1569 including the stop codon.

**Figure 2G.** The cDNA (SEQ ID. NO.:__________________) and amino acid sequence (SEQ ID. NO.:

__________________) of 121P2A3 v.7. The start methionine is underlined. The open reading frame extends from nucleic acid 175-1569 including the stop codon.

**Figure 2H.** The cDNA (SEQ ID. NO.:__________________) and amino acid sequence (SEQ ED. NO.: __________________) of 121P2A3 v.8. The start methionine is underlined. The open reading frame extends from nucleic acid 175-1569 including the stop codon.

**Figure 2I.** The cDNA (SEQ ID. NO.:__________________) and amino acid sequence (SEQ ID. NO.: __________________) of 121P2A3 v.9. The start methionine is underlined. The open reading frame extends from nucleic acid 175-1569 including the stop codon.

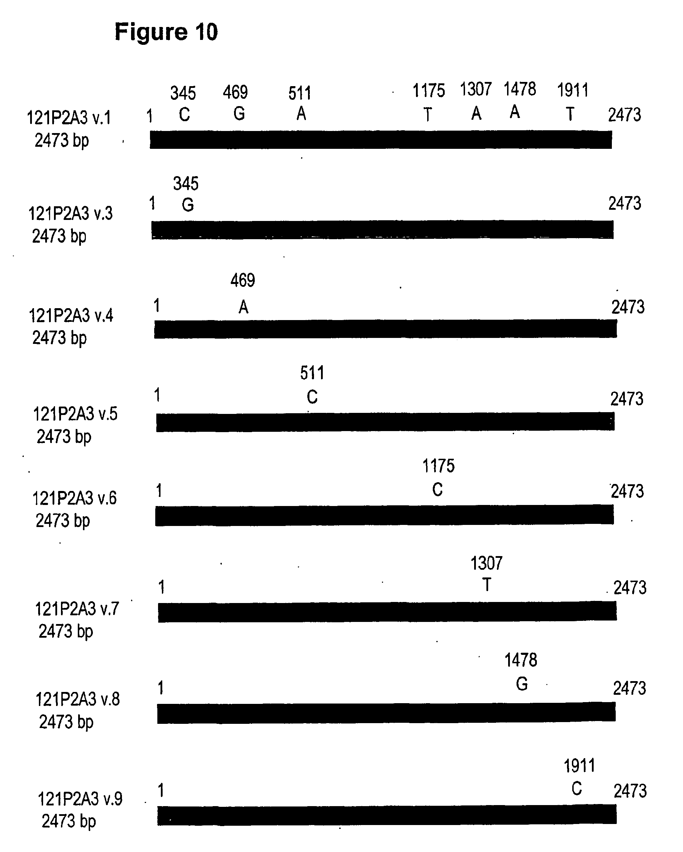

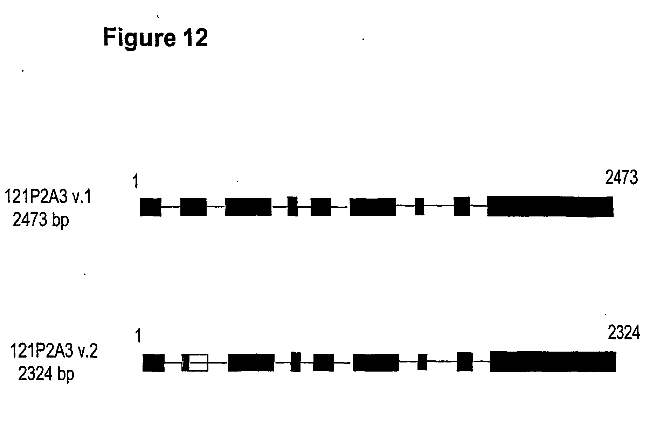

As used herein, a reference to 121P2A3 includes all variants thereof, including those shown in Figure 10 and Figure 12, unless a variant is specified.

**Figure 3A** Amino acid sequence of 121P2A3.v.1 clone 5 (SEQ ID. NO.:__________________). The 121P2A3 v.1 clone 5 protein has 464 amino acids.

**Figure 3B** Amino acid sequence of 121P2A3 v.2 (SEQ ID. NO.:__________________). The 121P2A3 v.2 protein has 295 amino acids.

**Figure 3C** Amino acid sequence of 121P2A3 v.3 (SEQ ID. NO.:__________________). The 121P2A3 v.3 protein has 464 amino acids.

**Figure 3D** Amino acid sequence of 121P2A3 v.4 (SEQ ID. NO.:__________________). The 121P2A3 v.4 protein has 464 amino acids.

**Figure 3E** Amino acid sequence of 121P2A3 v.6 (SEQ ID. NO.:__________________). The 121P2A3 v.6 protein has 464 amino acids.

**Figure 3F** Amino acid sequence of 121P2A3 v.7 (SEQ ID. NO.:__________________). The 121P2A3 v.7 protein has 464 amino acids.

**Figure 3G** Amino acid sequence of 121P2A3 v.8 (SEQ ID. **NO.:**__________________**).** The 121P2A3 v.8 protein has 464 amino acids.

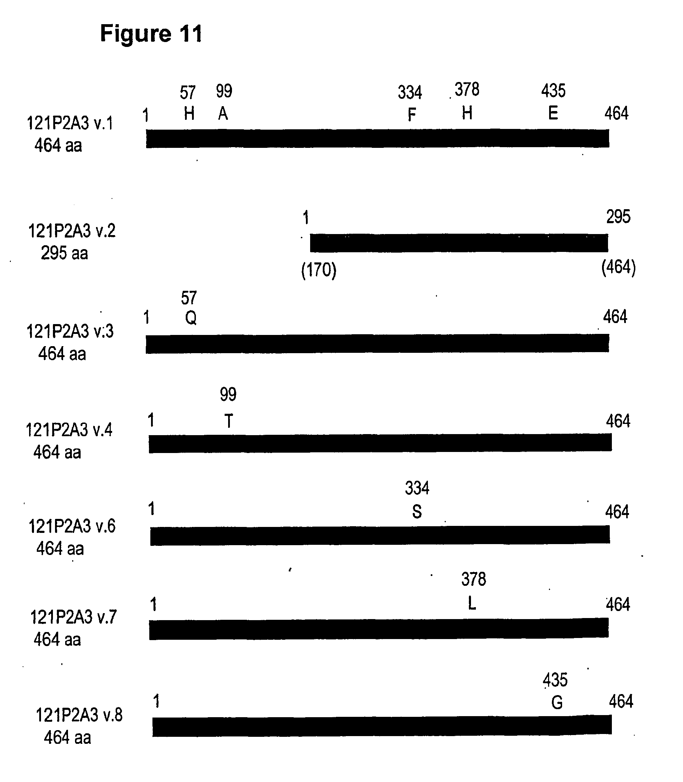

As used herein, a reference to 121P2A3 includes all variants thereof, including those shown in Figure 11, unless a variant is specified.

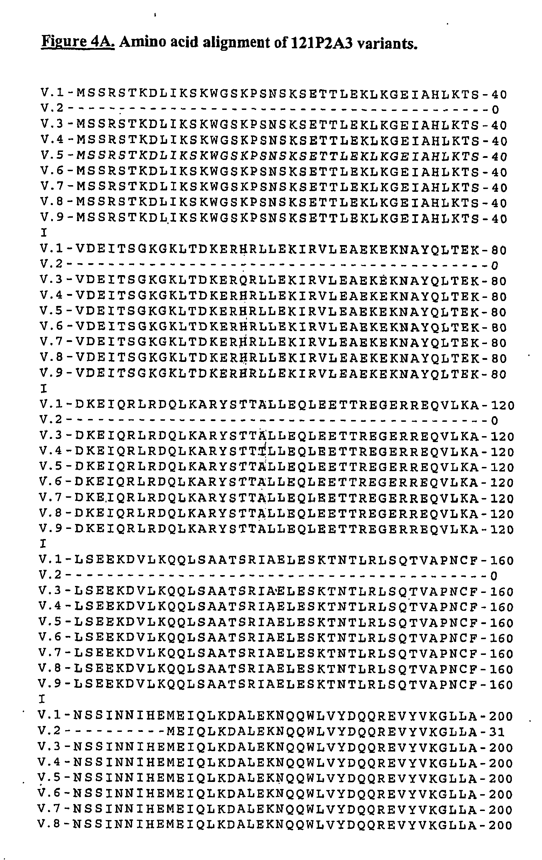

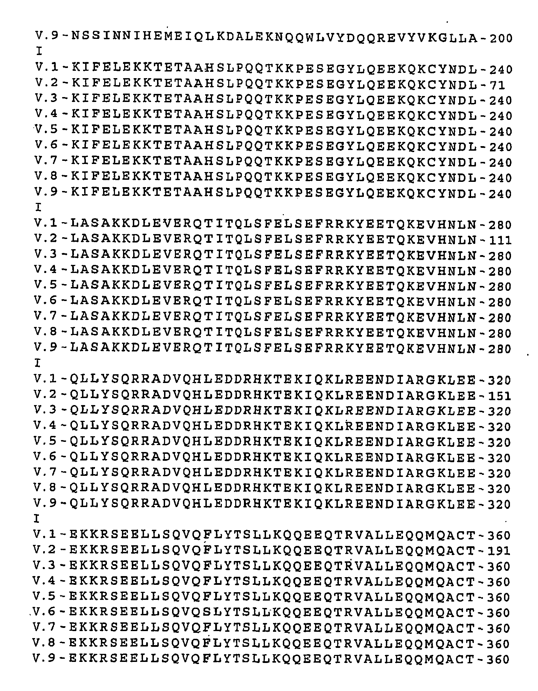

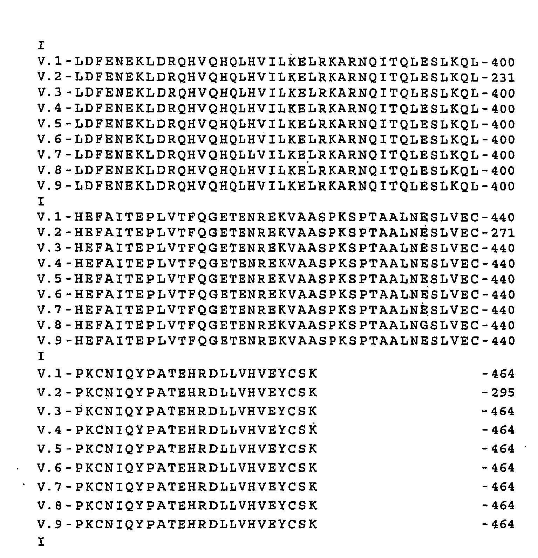

**Figure 4A.** Amino acid alignment of 121P2A3 variants.

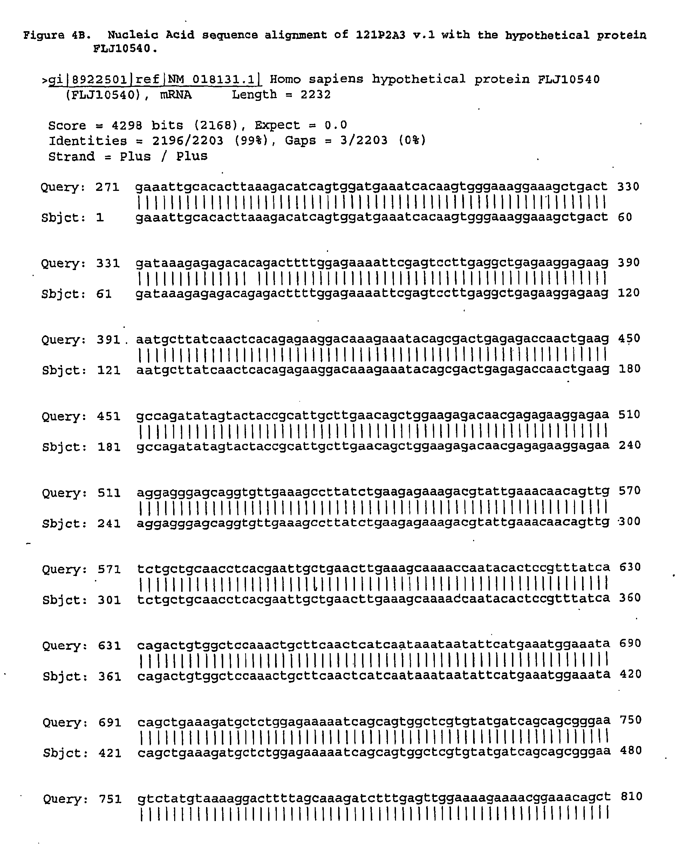

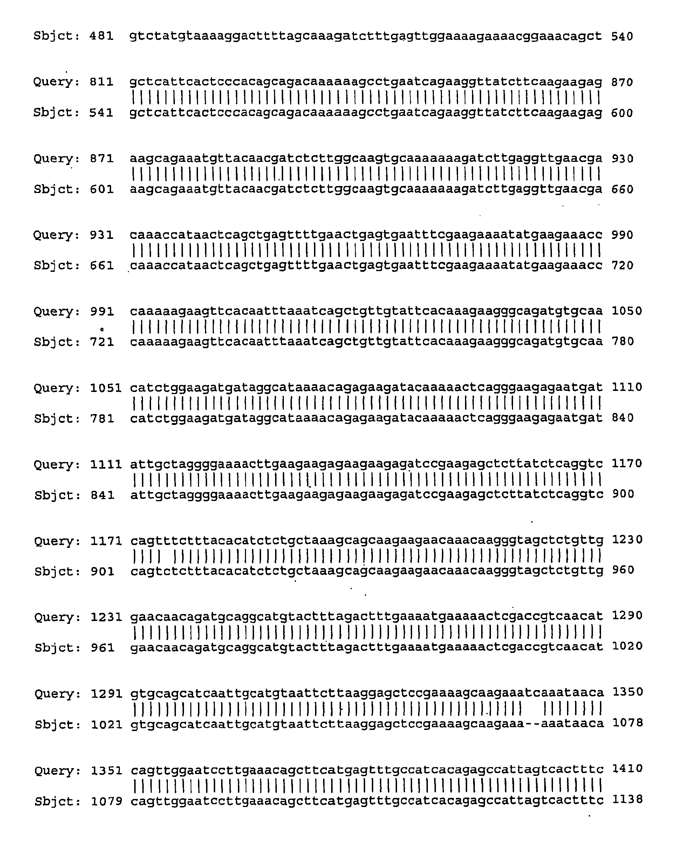

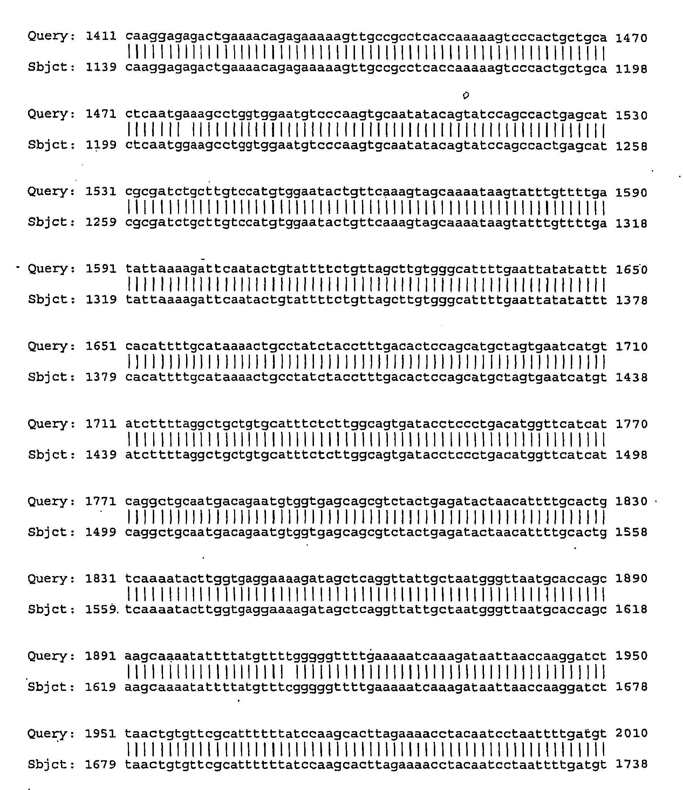

**Figure 4B.** Nucleic Acid sequence alignment of 121P2A3 v.1 with the hypothetical protein FLJ10540.

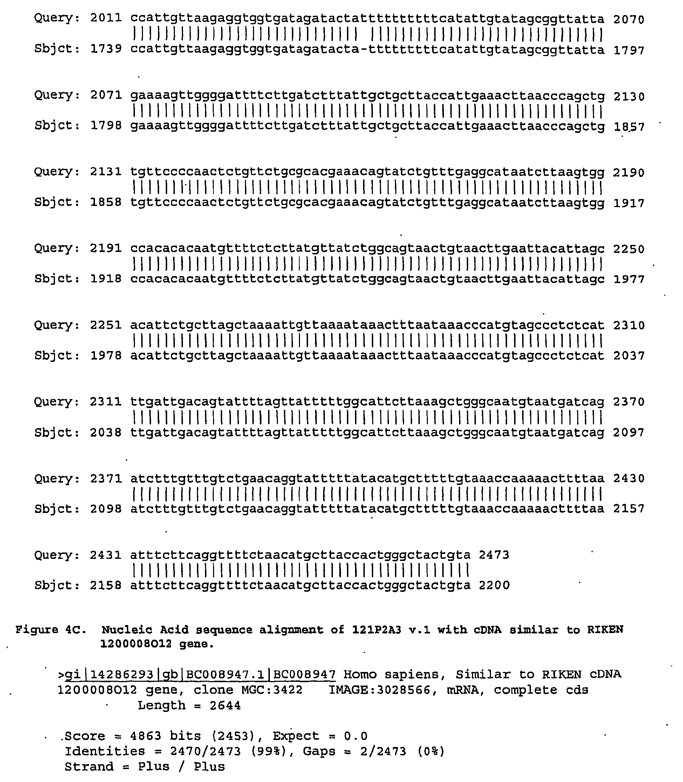

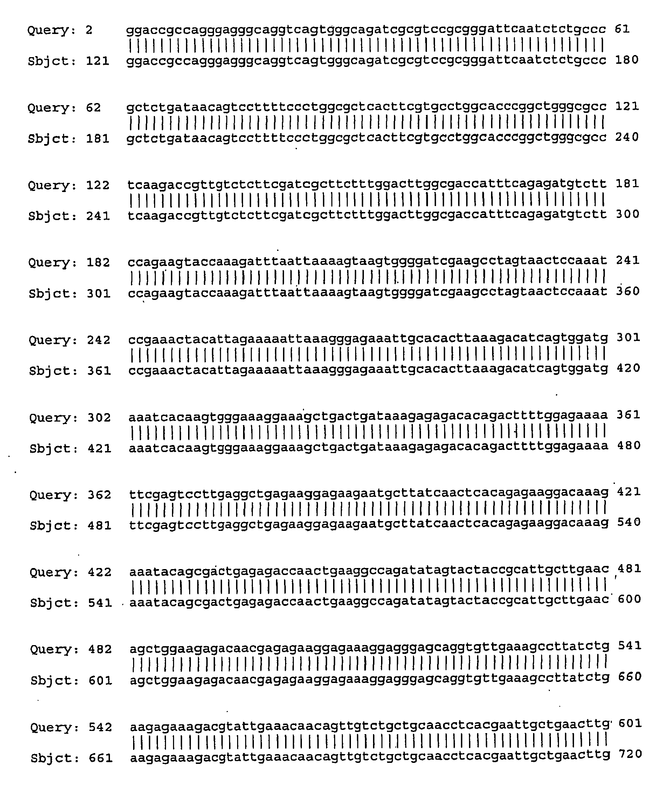

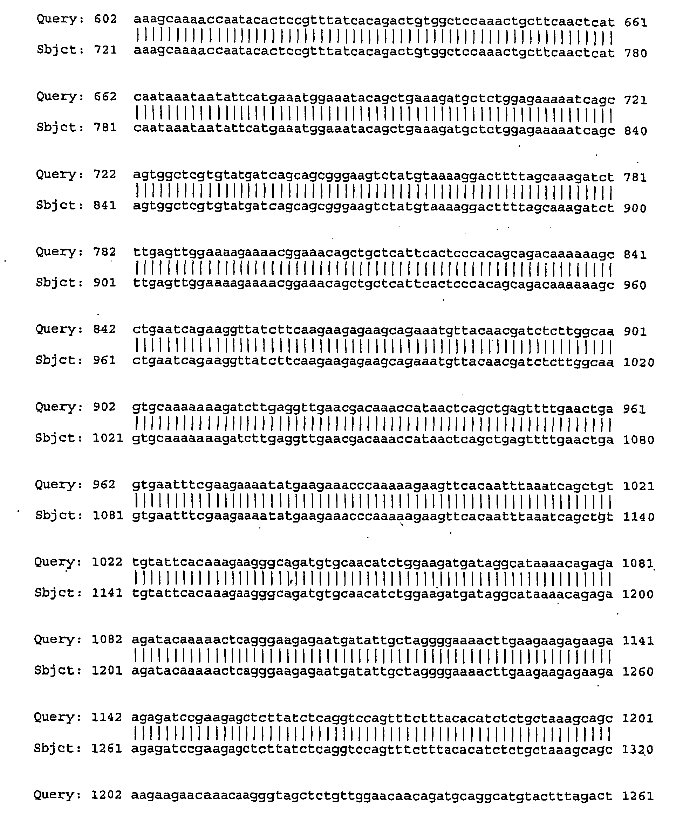

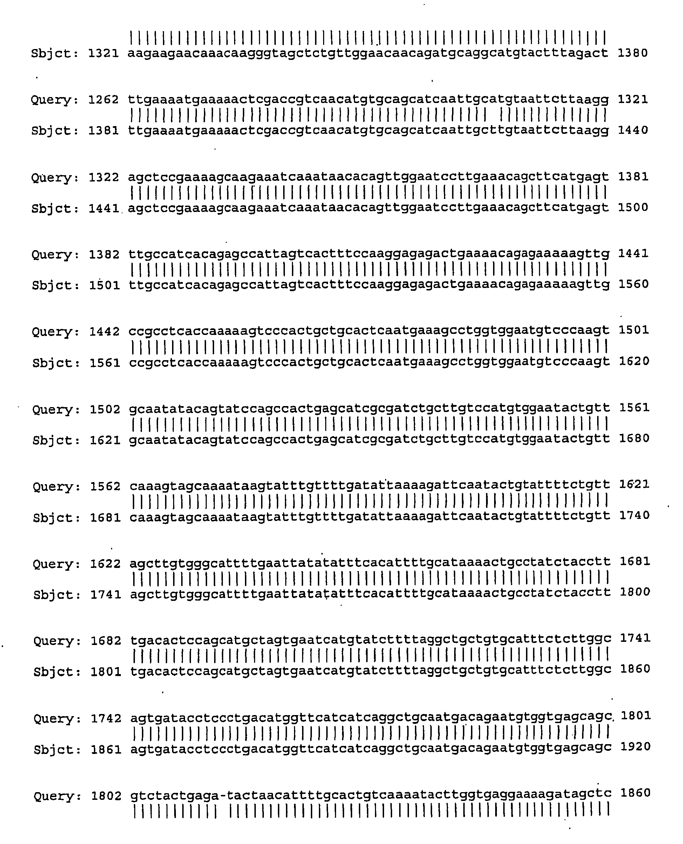

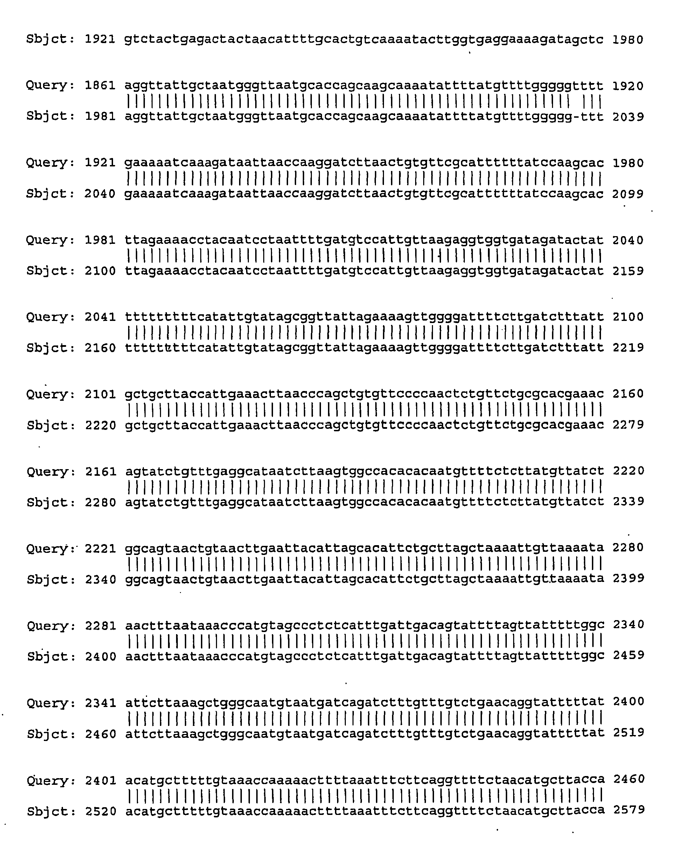

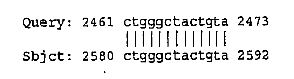

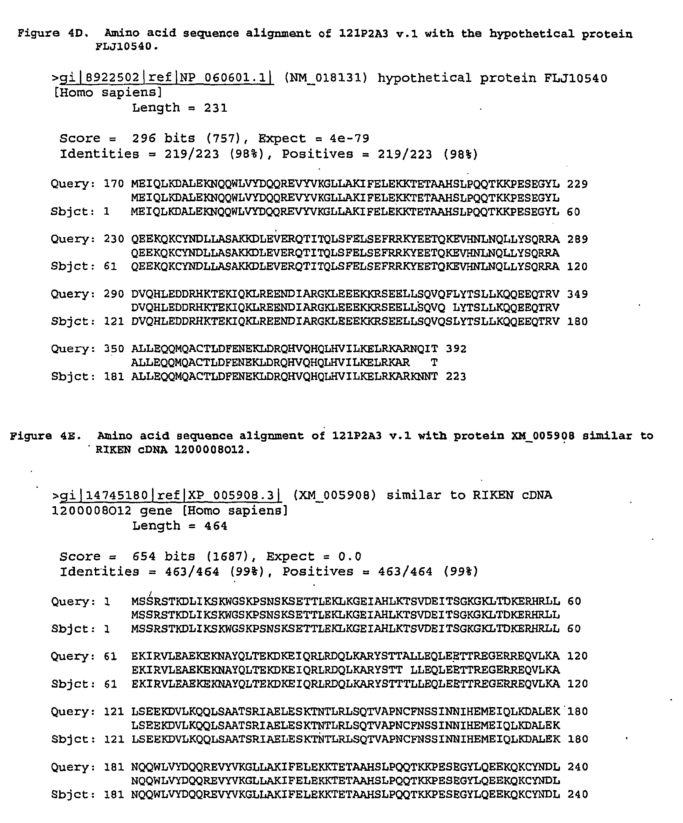

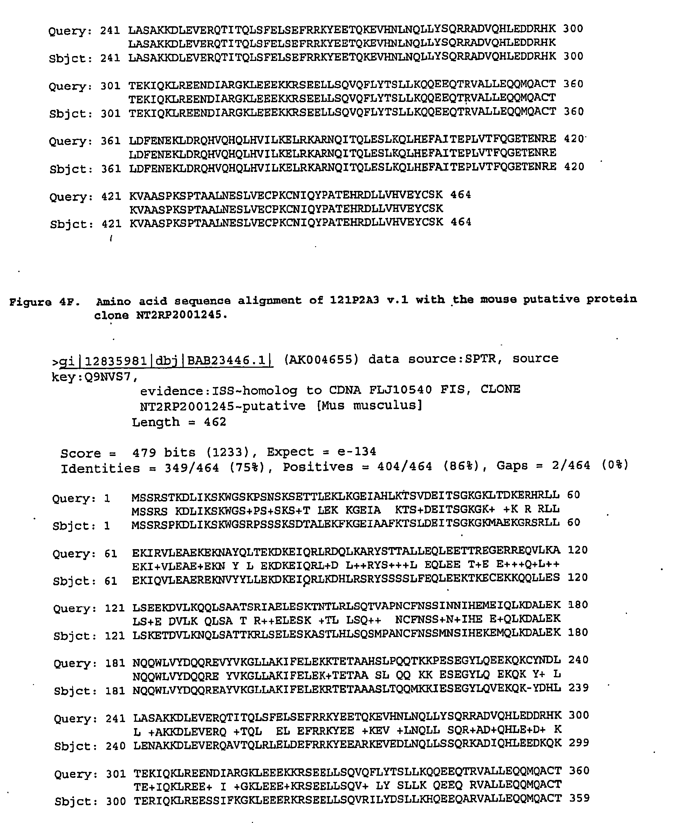

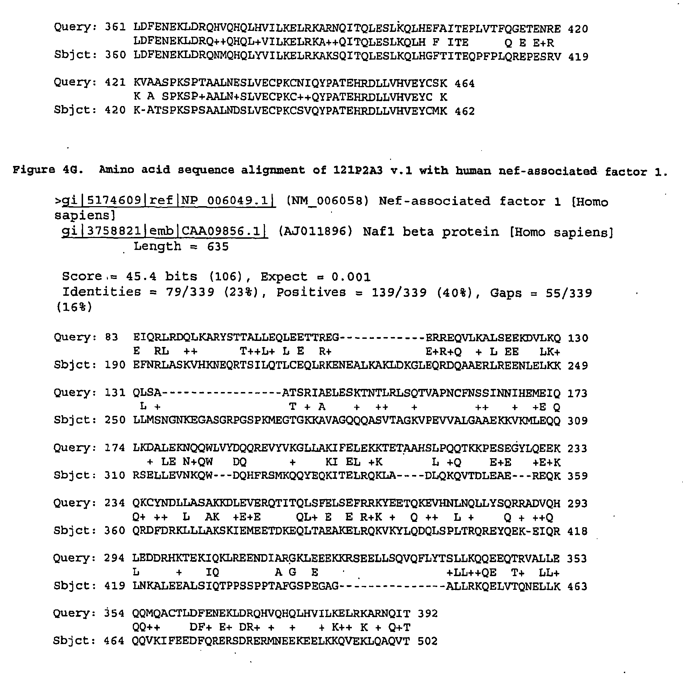

**Figure 4C.** Nucleic Acid sequence alignment of 121P2A3 v.1 with cDNA similar to RIKEN 1200008012 gene.

**Figure 4D.** Amino acid sequence alignment of 121P2A3 v.1 with the hypothetical human protein FLJ10540.

**Figure 4E.** Amino acid sequence alignment of 121P2A3 v.1 with protein XM_005908 similar to RIKEN cDNA 1200008012.

**Figure 4F.** Amino acid sequence alignment of 121P2A3 v.1 with the mouse putative protein clone NT2RP2001245.

**Figure 4G.** Amino acid sequence alignment of 121P2A3 v.1 with human nef-associated factor 1.

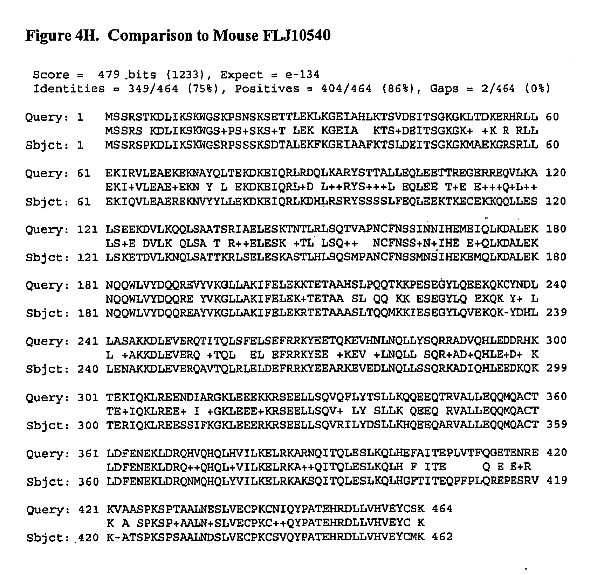

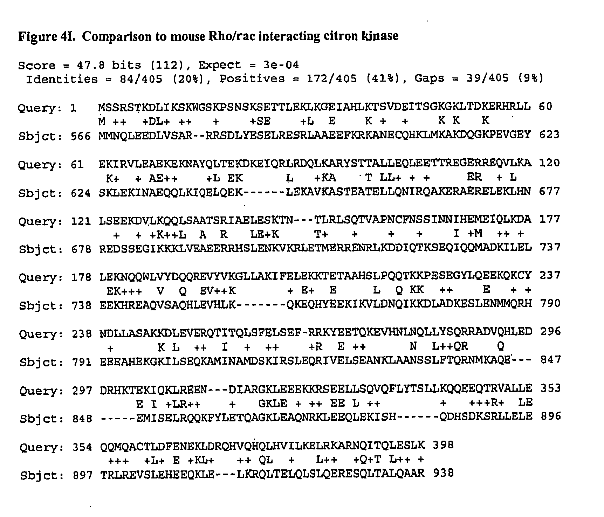

**Figure 4H.** Amino acid sequence alignment of 121P2A3 v.1 with mouse FLJ10540 protein.

**Figure 4I.** Amino acid sequence alignment of 121P2A3 v.1 with mouse Rho/rac interacting citron kinase.

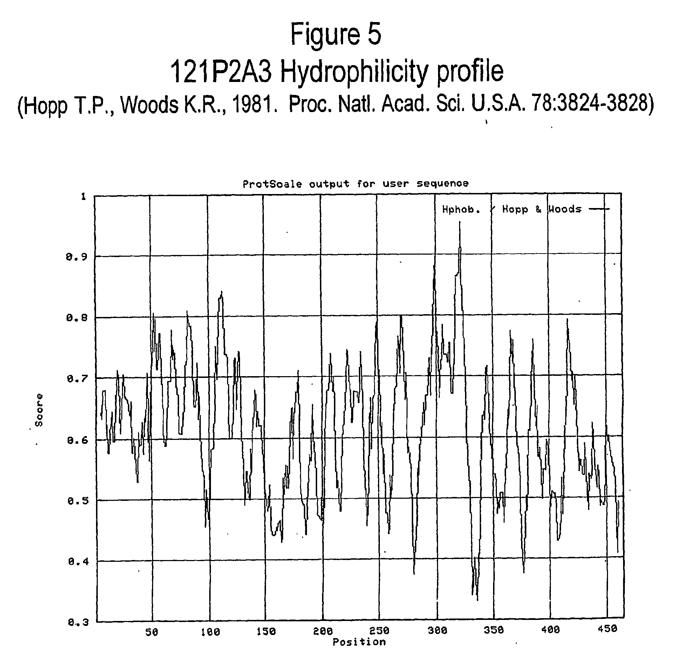

**Figure 5.** Hydrophilicity amino acid profile of 121P2A3 variant 1, determined by computer algorithm sequence analysis using the method ofHopp and Woods (Hopp T.P., Woods K.R, 1981. Proc. Natl. Acad. Sci. U.S.A. 78: 3824-3828) accessed on the Protscale website (www.expasy.ch/cgi-bin/protscale.pl) through the ExPasy molecular biology server.

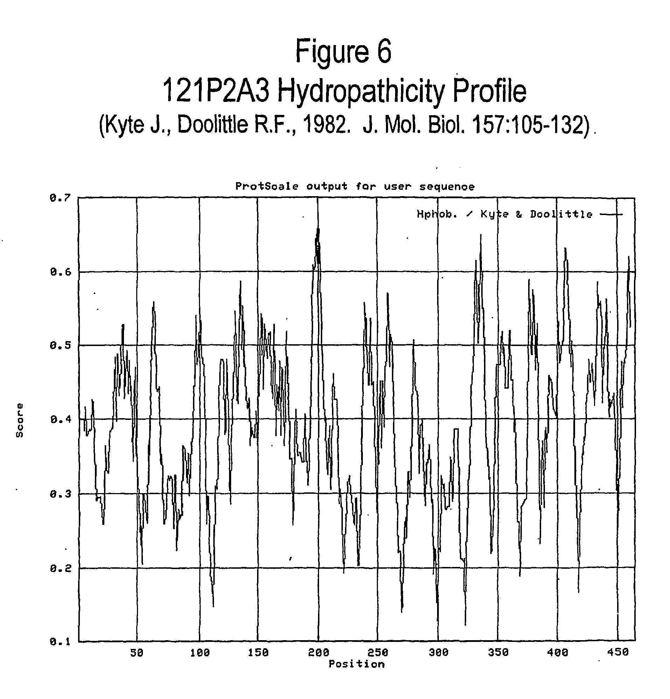

**Figure 6.** Hydropathicity amino acid profile of 121P2A3 variant 1, determined by computer algorithm sequence analysis using the method of Kyte and Doolittle (Kyte J., Doolittle R.F., 1982. J. Mol. Biol. 157:105-132) accessed on the ProtScale website (www.expasy.ch/cgi-bin/protscale.pl) through the ExPasy molecular biology server.

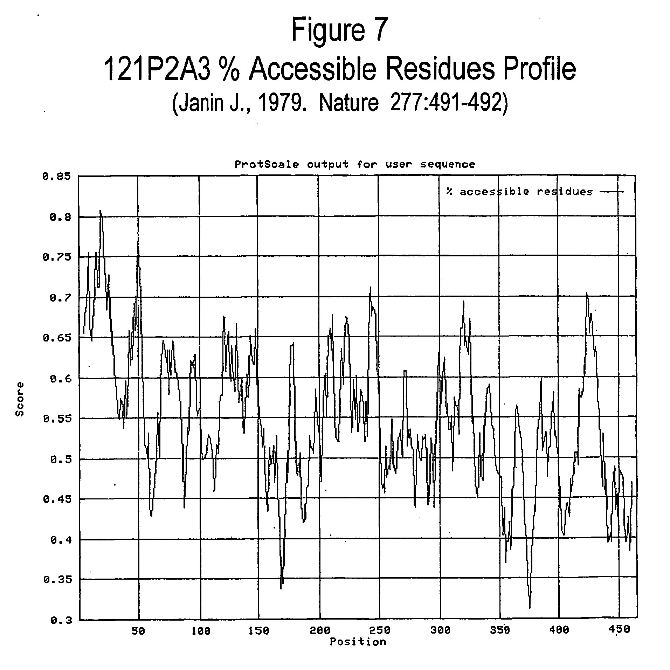

**Figure 7.** Percent accessible residues amino acid profile of 121P2A3 variant 1, determined by computer algorithm sequence analysis using the method of Janin (Janin J., 1979 Nature 277:491-492) accessed on the ProtScale website (www.expasy.ch/cgi-bin/protscale.pl) through the ExPasy molecular biology server.

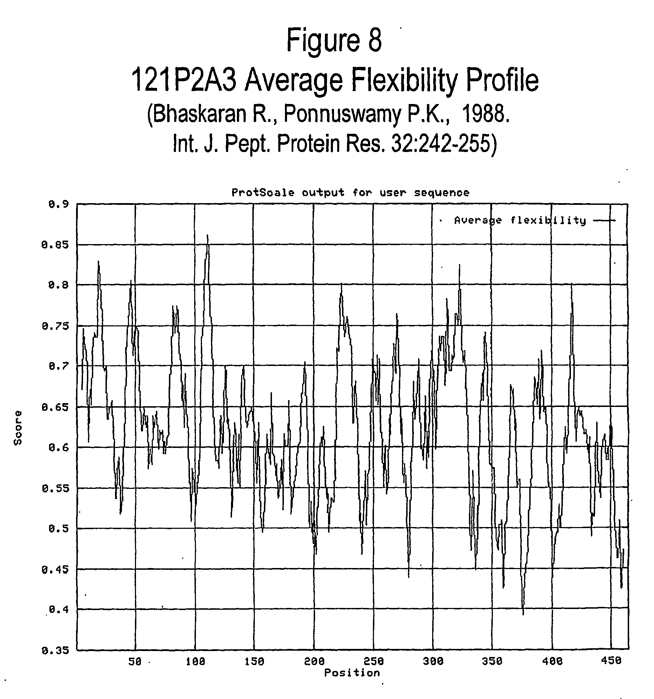

**Figure 8.** Average flexibility amino acid profile of 121P2A3 variant 1, determined by computer algorithm sequence analysis using the method of Bhaskaran and Ponnuswamy (Bhaskaran R, and Ponnuswamy P.K., 1988. Int. J. Pept. Protein Res. 32:242-255) accessed on the ProtScale website (www.expasy.ch/cgi-bin/protscale.pl) through the ExPasy molecular biology server.

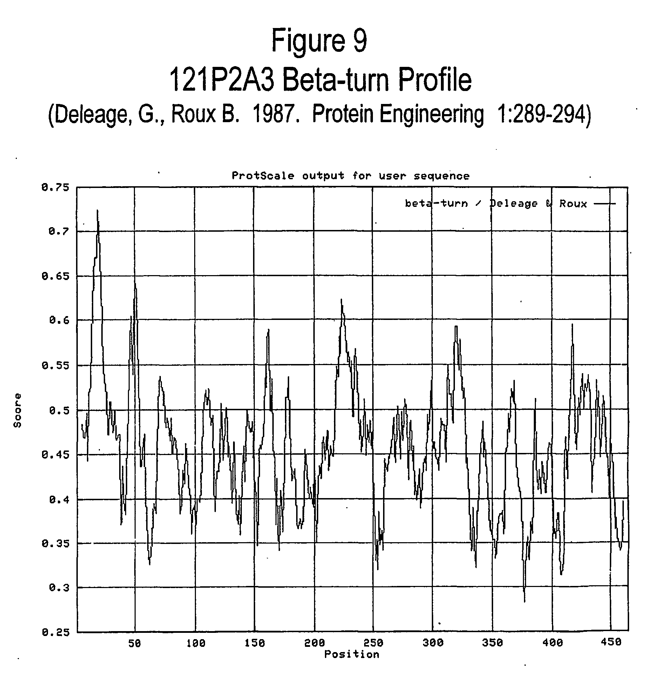

**Figure 9.** Beta-turn amino acid profile of 121P2A3 variant 1, determined by computer algorithm sequence analysis using the method of Deleage and Roux (Deleage, G., Roux B. 1987 Protein Engineering 1:289-294) accessed on the ProtScale website (www.expasy.ch/cgi-bin/protscale.pl) through the ExPasy molecular biology server.

**Figure 10.** Schematic alignment of SNP variants of 121P2A3. Variants 121P2A3 v.3 through v.9 are variants with single nucleotide differences. Though these SNP variants are shown separately, they could also occur in any combinations and in any one of the transcript variants that contains the base pairs. Numbers correspond to those of 121P2A3 v.1. The black boxes show the same sequence as 121P2A3 v.1. SNPs are indicated above the box.

**Figure 11.** Schematic alignment of protein variants of 121P2A3. Protein variants correspond to nucleotide variants.

Nucleotide variants 121P2A3 v.5 and v.9 in Figure 10 code for the same protein as 121P2A3 v.1. Black boxes represent the same sequence as 121P2A3 v.1. Single amino acid differences were indicated above the boxes. Numbers in "()" underneath the box correspond to 121P2A3 v.1.

**Figure 12.** Exon compositions of transcript variants of 121P2A3. Variant 121P2A3 v.2 is a splice variant whose exon 2 is 149 bp shorter than exon 2 of 121P2A3 v.1. Empty (white) box shows the portion of exon 2 in 121P2A3 v.1 but not in 121P2A3 v.2. Black boxes show the same sequence as 121P2A3 v.1. Numbers correspond to those of 121P2A3 v.1. Length of introns are not proportional.

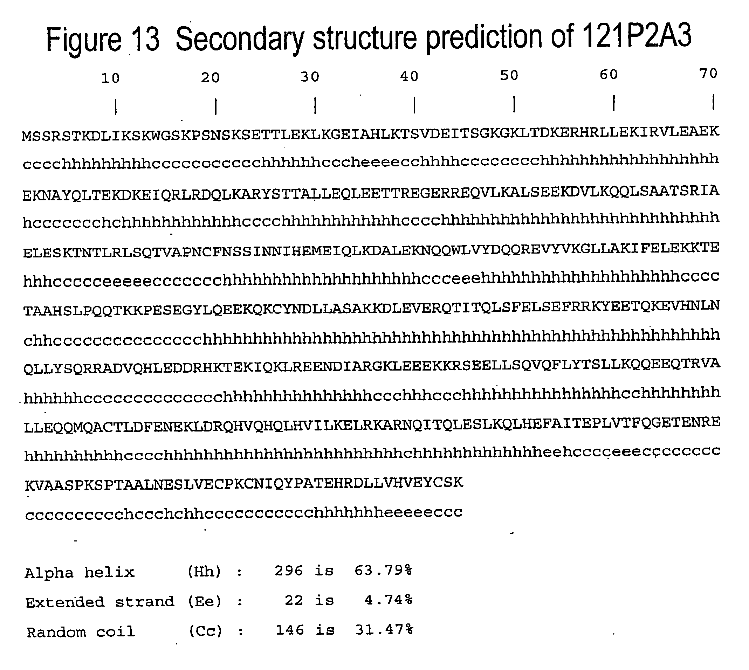

**Figure 13.** Secondary structure prediction for 121P2A3 protein. The secondary structure of 121P2A3 protein was predicted using the HNN - Hierarchical Neural Network method (Guermeur, 1997, URL pbil.ibcp.fr/cgi-bin/npsa_automat.pl?page=npsa_nn.html), accessed from the ExPasy molecular biology server (URL www.expasy.ch/tools/). This method predicts the presence and location of alpha helices, extended strands, and random coils from the primary protein sequence. The percent of the protein in a given secondary structure is also listed.

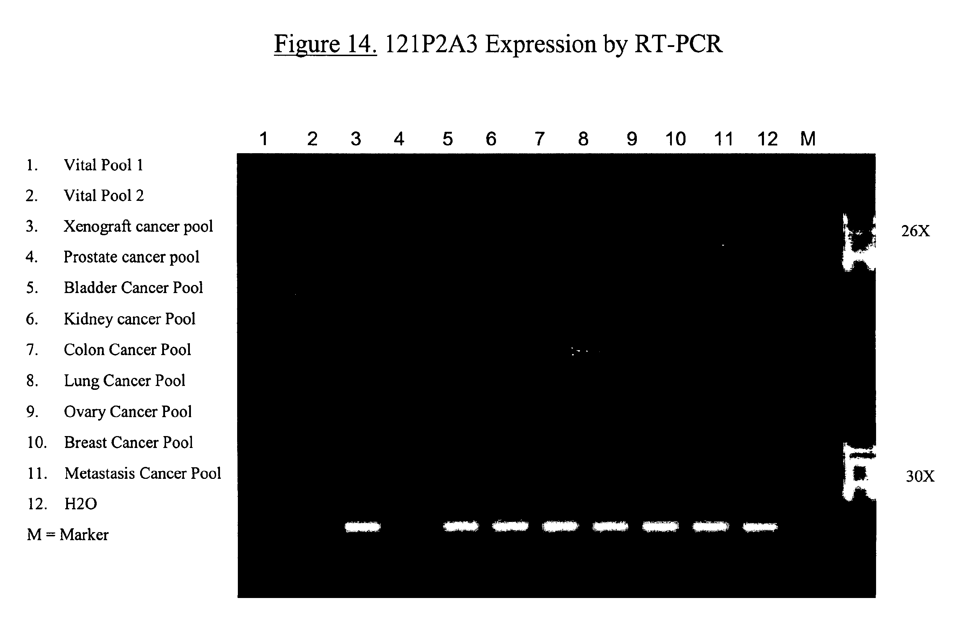

**Figure 14.** Expression of 121P2A3 by RT-PCR. First strand cDNA was prepared from vital pool 1 (liver, lung and kidney), vital pool 2 (pancreas, colon and stomach), LAPC xenograft pool (LAPC-4AD, LAPC-4AI, LAPC-9AD and LAPC-9AI), prostate cancer pool, bladder cancer pool, kidney cancer pool, colon cancer pool, lung cancer pool, ovary cancer pool, breast cancer pool, and cancer metastasis pool. Normalization was performed by PCR using primers to actin and GAPDH. Semi-quantitative PCR, using primers to 121P2A3, was performed at 26 and 30 cycles of amplification. Results show strong expression of 121P2A3 in LAPC xenograft pool, bladder cancer pool, kidney cancer pool, colon cancer pool, lung cancer pool, ovary cancer pool, breast cancer pool, and cancer metastasis pool. Expression of 121P2A3 was also detected in prostate cancer pool. Very low expression was detected in vital pool 1 and 2.

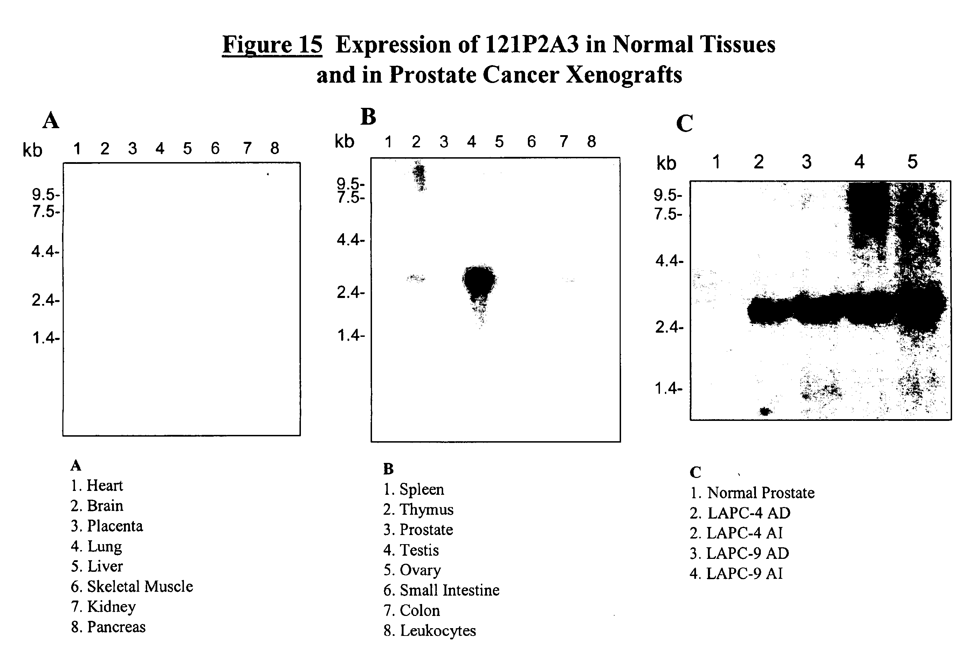

**Figure 15.** Expression of 121P2A3 in normal tissues. Two multiple tissue northern blots (A and B; Clontech) both with 2 ug of mRNA/lane, and a LAPC xenograft blot both with 10 ug of total RNA/lane (C) were probed with the 121P2A3 SSH sequence. Size standards in kilobases (kb) are indicated on the side. Results show expression of an approximately 2.7 kb121P2A3 transcript in testis. Lower level expression was also detected in thymus and colon, but not in the other normal tissues tested. 121P2A3 expression was strongly demonstrated in all 4 LAPC prostate xenograft tissues but not in normal prostate.

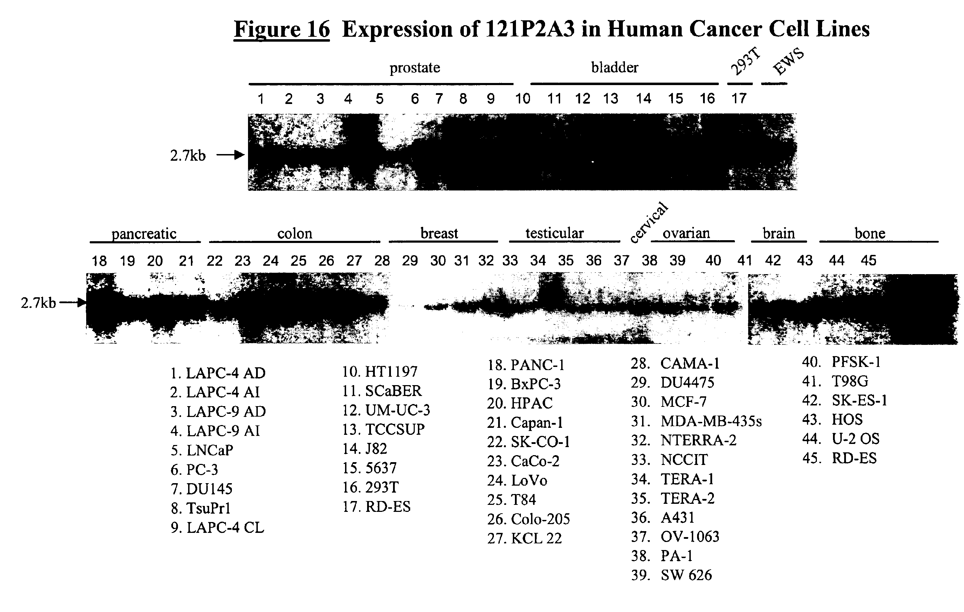

**Figure 16.** Expression of 121P2A3 in human cancer cell lines. RNA was extracted from a number of human cancer cell lines. Northern blots with 10 ug of total RNA/lane were probed with the 121P2A3 SSH fragment. Size standards in kilobases (kb) are indicated on the side. Results show expression of 121P2A3 in all the cell lines tested.

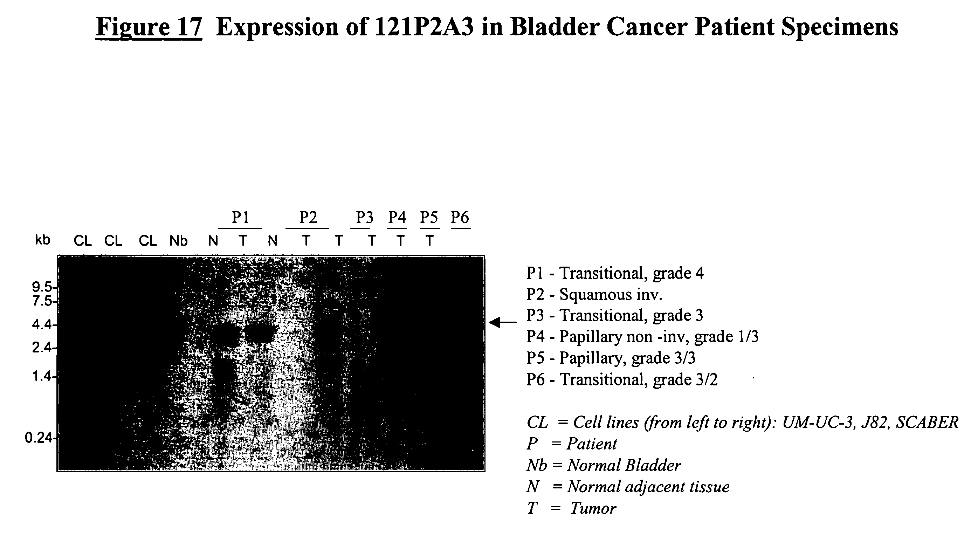

**Figure 17.** Expression of 121P2A3 in bladder cancer patient tissues. RNA was extracted from normal bladder (Nb), bladder cancer cell lines (CL; UM-UC-3, J82, SCaBER), bladder cancer patient tumors (T) and normal adjacent tissue (N). Northern blots with 10 ug of total RNA were probed with the 121P2A3 SSH sequence. Size standards in kilobases are indicated on the side. Results show expression of 121P2A3 in patient bladder cancer tissues, and in all bladder cancer cell lines tested, but not in normal bladder.

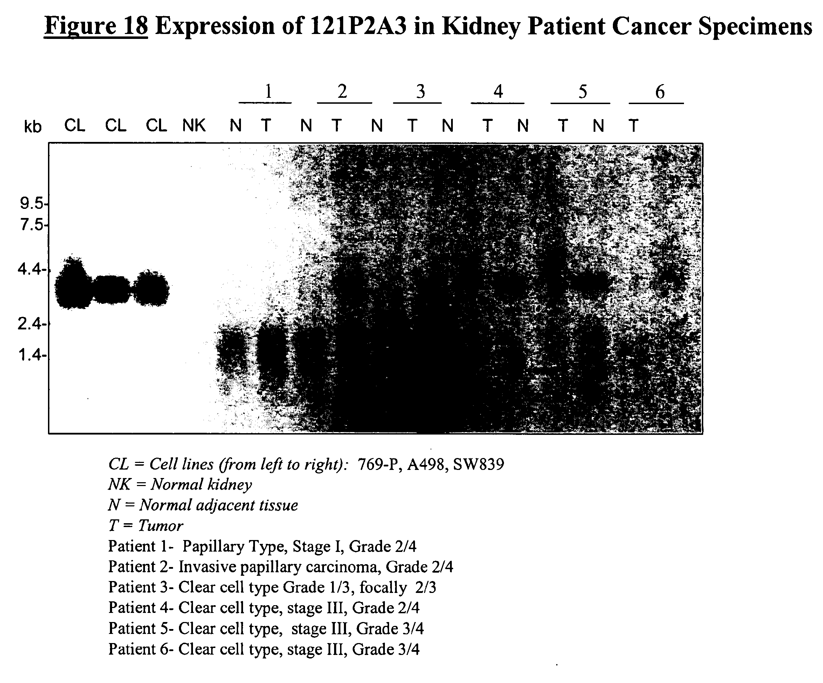

**Figure 18.** Expression of 121P2A3 in kidney cancer patient tissues. RNA was extracted from kidney cancer cell lines (CL: 769-P, A498, SW839), normal kidney (NK), kidney cancer patient tumors (T) and their normal adjacent tissues (N). Northern blots with 10 ug of total RNA were probed with the 121P2A3 SSH sequence. Size standards in kilobases are on the side. Results show expression of 121P2A3 in patient kidney tumor tissues and in all kidney cancer cell lines tested, but not in normal kidney.

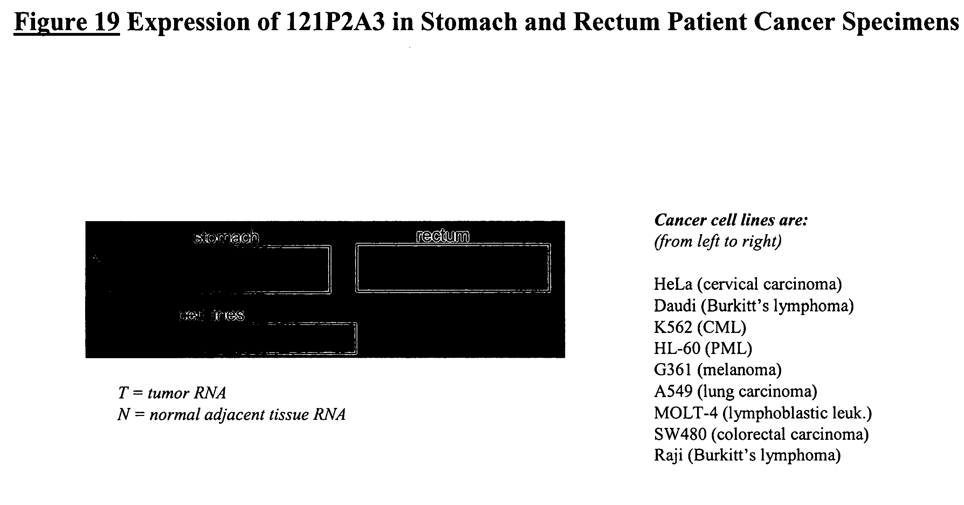

**Figure 19.** Expression of 121P2A3 in stomach and rectum human cancer specimens. Expression of 121P2A3 was assayed in a panel of human stomach and rectum cancers (T) and their respective matched normal tissues (N) on RNA dot blots, and in human cancer cell lines. 121P2A3 expression was seen in both stomach and rectum cancers. The expression detected in normal adjacent tissues (isolated from diseased tissues) but not in normal tissues (isolated from healthy donors) may indicate that these tissues are not fully normal and that 121P2A3 may be expressed in early stage tumors. 121P2A3 was also found to be highly expressed in the following cancer cell lines; HeLa, Daudi, K562, HL-60, G361, A549, MOLT-4, SW480, and Raji.

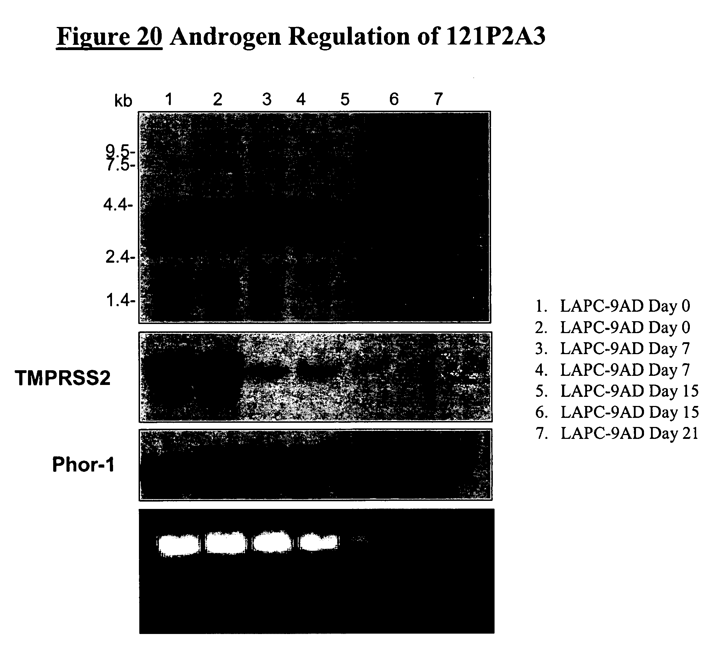

**Figure 20.** Androgen regulation of 121P2A3. Male mice were injected with LAPC-9AD tumor cells. When tumor reached a palpable size (0.3-0.5cm in diameter), mice were castrated and tumors harvested at different time points following castration. RNA was isolated from the xenograft tissues. Northern blots with 10 ug of total RNA/lane were probed with the 121P2A3 SSH fragment. Size standards in kilobases (kb) are indicated on the side. Results show expression of 121P2A3 is downregulated within 7 days of castration. The experimental samples were confirmed by testing for the expression of the androgen-regulated prostate cancer gene TMPRSS2 and the androgen-independent gene PHOR-1 (B). This experiment shows that, as expected, TMPRSS2 expression level goes down 7 days after castration, whereas the expression of PHOR-1 does not change. A picture of the ethidium-bromide staining of the RNA gel is also presented confirming the quality of the RNA.

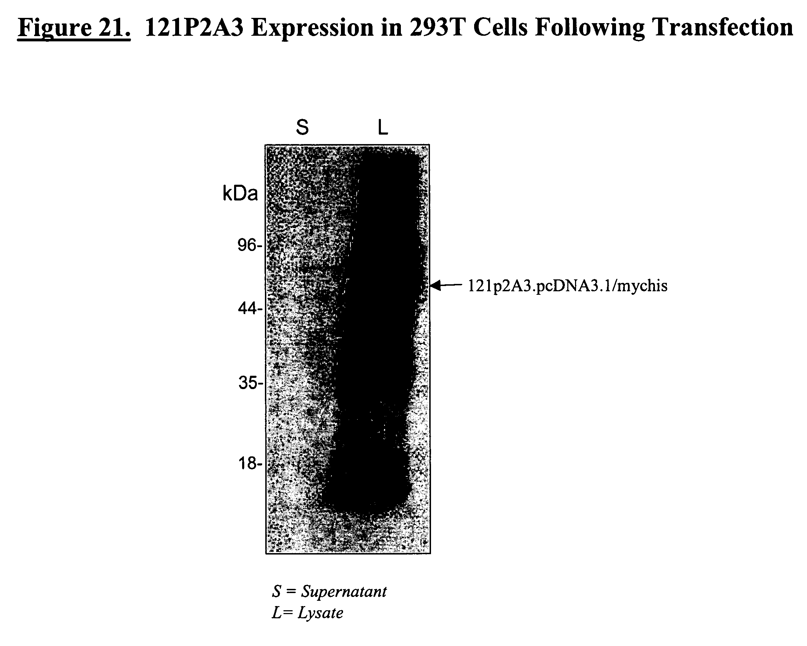

**Figure 21.** 121P2A3 expression in 293T cells following transfection. 293T cells were transfected with 121P2A3 .pcDNA3.1/mychis. Forty hours later, cell lysates (L) and supernatant (S) were collected. Samples were run on an SDS-PAGE acrylamide gel, blotted and stained with anti-his antibody. The blot was developed using the ECL chemiluminescence kit and visualized by autoradiography. Results show expression of the expected 54kDa molecular weight 121P2A3 from the 121P2A3 .pcDNA3.1/mychis mammalian expression construct in the lysates of 121P2A3.pcDNA3.1/mychis transfected cells, but not in the supernatant.

## DETAILED DESCRIPTION OF THE INVENTION

**Outline of Sections**

I.) Definitions
II.) 121P2A3 Polynucleotides
II.A.) Uses of 121P2A3 Polynucleotides
II.A.1.) Monitoring of Genetic Abnormalities
II.A.2.) Antisense Embodiments
II.A.3.) Primers and Primer Pairs

II.A.4.) Isolation of 121P2A3-Encoding Nucleic Acid Molecules

II.A.5.) Recombinant Nucleic Acid Molecules and Host-Vector Systems
III.) 121P2A3-related Proteins

III.A.) Motif-bearing Protein Embodiments
III.B.) Expression of 121P2A3-related Proteins
III.C.) Modifications of 121P2A3-related Proteins
III.D.) Uses of 121P2A3-related Proteins

IV.) 121P2A3 Antibodies
V.) 121P2A3 Cellular Immune Responses
VI.) 121P2A3 Transgenic Animals
VII.) Methods for the Detection of 121P2A3
VIII.) Methods for Monitoring the Status of 121P2A3-related Genes and Their Products
IX.) Identification of Molecules That Interact With 121P2A3
X.) Therapeutic Methods and Compositions

X.A.) Anti-Cancer Vaccines

X.B.) 121P2A3 as a Target for Antibody-Based Therapy
X.C.) 121P2A3 as a Target for Cellular Immune Responses
X.C.1. Minigene Vaccines

X.C.2. Combinations of CTL Peptides with Helper Peptides
X.C.3. Combinations of CTL Peptides with T Cell Priming Agents

X.C.4. Vaccine Compositions Comprising DC Pulsed with CTL and/or HTL Peptides

X.D.) Adoptive Immunotherapy

X.E.) Administration of Vaccines for Therapeutic or Prophylactic Purposes
XI.) Diagnostic and Prognostic Embodiments of 121P2A3.
XII.) Inhibition of 121P2A3 Protein Function

XII.A.) Inhibition of 121P2A3 With Intracellular Antibodies
XII.B.) Inhibition of 121P2A3 with Recombinant Proteins
XII.C.) Inhibition of 121P2A3 Transcription or Translation

XII.D.) General Considerations for Therapeutic Strategies

XIII.) KITS

**I.) Definitions:**

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

The terms "advanced prostate cancer", "locally advanced prostate cancer", "advanced disease" and "locally advanced disease" mean prostate cancers that have extended through the prostate capsule, and are meant to include stage C disease under the American Urological Association (AUA) system, stage C1 - C2 disease under the Whitmore-Jewett system, and stage T3 - T4 and N+ disease under the TNM (tumor, node, metastasis) system. In general, surgery is not recommended for patients with locally advanced disease, and these patients have substantially less favorable outcomes compared to patients having clinically localized (organ-confined) prostate cancer. Locally advanced disease is clinically identified by palpable evidence of induration beyond the lateral border of the prostate, or asymmetry or induration above the prostate base. Locally advanced prostate cancer is presently diagnosed pathologically following radical prostatectomy if the tumor invades or penetrates the prostatic capsule, extends into the surgical margin, or invades the seminal vesicles.

"Altering the native glycosylation pattern" is intended for purposes herein to mean deleting one or more carbohydrate moieties found in native sequence 121P2A3 (either by removing the underlying glycosylation site or by deleting the glycosylation by chemical and/or enzymatic means), and/or adding one or more glycosylation sites that are not present in the native sequence 121P2A3. In addition, the phrase includes qualitative changes in the glycosylation of the native proteins, involving a change in the nature and proportions of the various carbohydrate moieties present.

The term "analog" refers to a molecule which is structurally similar or shares similar or corresponding attributes with another molecule (e.g. a 121P2A3-related protein). For example an analog of a 121P2A3 protein can be specifically bound by an antibody or T cell that specifically binds to 121P2A3.

The term "antibody" is used in the broadest sense. Therefore an "antibody" can be naturally occurring or man-made such as monoclonal antibodies produced by conventional hybridoma technology. Anti-121P2A3 antibodies comprise monoclonal and polyclonal antibodies as well as fragments containing the antigen-binding domain and/or one or more complementarity determining regions of these antibodies.

An "antibody fragment" is defined as at least a portion of the variable region of the immunoglobulin molecule that binds to its target, i.e., the antigen-binding region. In one embodiment it specifically covers single anti-121P2A3 antibodies and clones thereof (including agonist, antagonist and neutralizing antibodies) and anti-121P2A3 antibody compositions with polyepitopic specificity.

The term "codon optimized sequences" refers to nucleotide sequences that have been optimized for a particular host species by replacing any codons having a usage frequency of less than about 20%. Nucleotide sequences that have been optimized for expression in a given host species by elimination of spurious polyadenylation sequences, elimination of exon/intron splicing signals, elimination of iransposon-like repeats and/or optimization of GC content in addition to codon optimization are referred to herein as an "expression enhanced sequences."

The term "cytotoxic agent" refers to a substance that inhibits or prevents the expression activity of cells, function of cells and/or causes destruction of cells. The term is intended to include radioactive isotopes chemotherapeutic agents, and toxins such as small molecule toxins or enzymatically active toxins of bacterial, fungal, plant or animal origin, including fragments and/or variants thereof. Examples of cytotoxic agents include, but are not limited to maytansinoids, yttrium, bismuth, ricin, ricin A-chain, doxorubicin, daunorubicin, taxol, ethidium bromide, mitomycin, etoposide, tenoposide, vincristine, vinblastine, colchicine, dihydroxy anthracin dione, actinomycin, diphtheria toxin, Pseudomonas exotoxin (PE) A, PE40, abrin, abrin A chain, modeccin A chain, alpha-sarcin, gelonin, mitogellin, retstrictocin, phenomycin, enomycin, curicin, crotin, calicheamicin, sapaonaria officinalis inhibitor, and glucocorticoid and other chemotherapeutic agents, as well as radioisotopes such as $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu. Antibodies may also be conjugated to an anti-cancer pro-drug activating enzyme capable of converting the pro-drug to its active form.

The term "homolog" refers to a molecule which exhibits homology to another molecule, by for example, having sequences of chemical residues that are the same or similar at corresponding positions.

"Human Leukocyte Antigen" or "HLA" is a human class I or class II Major Histocompatibility Complex (MHC) protein (*see, e.g.,* Stites, et al., IMMUNOLOGY, 8TH ED., Lange Publishing, Los Altos, CA (1994).

The terms "hybridize", "hybridizing", "hybridizes" and the like, used in the context of polynucleotides, are meant to refer to conventional hybridization conditions, preferably such as hybridization in 50% formamide/6XSSC/0.1% SDS/ 100 μg/ml ssDNA, in which temperatures for hybridization are above 37 degrees C and temperatures for washing in 0.1XSSC/0.1% SDS are above 55 degrees C.

The phrases "isolated" or "biologically pure" refer to material which is substantially or essentially free from components which normally accompany the material as it is found in its native state. Thus, isolated peptides in accordance with the invention preferably do not contain materials normally associated with the peptides in their *in situ* environment. For example, a polynucleotide is said to be "isolated" when it is substantially separated from contaminant polynucleotides that correspond or are complementary to genes other than the 121P2A3 genes or that encode polypeptides other than 121P2A3 gene product or fragments thereof. A skilled artisan can readily employ nucleic acid isolation procedures to obtain an isolated 121P2A3 polynucleotide. A protein is said to be "isolated," for example, when physical, mechanical or chemical methods are employed to remove the 121P2A3 proteins from cellular constituents that are normally associated with the protein. A skilled artisan can readily employ standard purification methods to obtain an isolated 121P2A3 protein. Alternatively, an isolated protein can be prepared by chemical means.

The term "mammal" refers to any organism classified as a mammal, including mice, rats, rabbits, dogs, cats, cows, horses and humans. In one embodiment of the invention, the mammal is a mouse. In another embodiment of the invention, the mammal is a human.

The terms "metastatic prostate cancer" and "metastatic disease" mean prostate cancers that have spread to regional lymph nodes or to distant sites, and are meant to include stage D disease under the AUA system and stage TxNxM+ under the TNM system. As is the case with locally advanced prostate cancer, surgery is generally not indicated for patients with metastatic disease, and hormonal (androgen ablation) therapy is a preferred treatment modality. Patients with metastatic prostate cancer eventually develop an androgen-refractory state within 12 to 18 months of treatment initiation. Approximately half of these androgen-refractory patients die within 6 months after developing that status. The most common site for prostate cancer metastasis is bone. Prostate cancer bone metastases are often osteoblastic rather than osteolytic (i.e., resulting in net bone formation). Bone metastases are found most frequently in the spine, followed by the femur, pelvis, rib cage, skull and humerus. Other common sites for metastasis include lymph nodes, lung, liver and brain. Metastatic prostate cancer is typically diagnosed by open or laparoscopic pelvic lymphadenectomy, whole body radionuclide scans, skeletal radiography, and/or bone lesion biopsy.

The term "monoclonal antibody" refers to an antibody obtained from a population of substantially homogeneous antibodies, i.e., the antibodies comprising the population are identical except for possible naturally occurring mutations that are present in minor amounts.

A "motif", as in biological motif of a 121P2A3-related protein, refers to any pattern of amino acids forming part of the primary sequence of a protein, that is associated with a particular function (e.g. protein-protein interaction, protein-DNA interaction, etc) or modification (e.g. that is phosphorylated, glycosylated or amidated), or localization (e.g. secretory sequence, nuclear localization sequence, etc.) or a sequence that is correlated with being immunogenic, either humorally or cellularly. A motif can be either contiguous or capable of being aligned to certain positions that are generally correlated with a certain function or property. In the context of HLA motifs, "motif" refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs for HLA binding are typically different for each protein encoded by each human HLA allele and differ in the pattern of the primary and secondary anchor residues.

A "pharmaceutical excipient" comprises a material such as an adjuvant, a carrier, pH-adjusting and buffering agents, tonicity adjusting agents, wetting agents, preservative, and the like.

"Pharmaceutically acceptable" refers to a non-toxic, inert, and/or composition that is physiologically compatible with humans or other mammals.

The term "polynucleotide" means a polymeric form of nucleotides of at least 10 bases or base pairs in length, either ribonucleotides or deoxynucleotides or a modified form of either type of nucleotide, and is meant to include single and double stranded forms of DNA and/or RNA. In the art, this term if often used interchangeably with "oligonucleotide". A polynucleotide can comprise a nucleotide sequence disclosed herein wherein thymidine (T), as shown for example in Figure 2, can also be uracil (U); this definition pertains to the differences between the chemical structures of DNA and RNA, in particular the observation that one of the four major bases in RNA is uracil (U) instead of thymidine (T).

The term "polypeptide" means a polymer of at least about 4, 5, 6, 7, or 8 amino acids. Throughout the specification, standard three letter or single letter designations for amino acids are used. In the art, this term is often used interchangeably with "peptide" or "protein".

An HLA "primary anchor residue" is an amino acid at a specific position along a peptide sequence which is understood to provide a contact point between the immunogenic peptide and the HLA molecule. One to three, usually two, primary anchor residues within a peptide of defined length generally defines a "motif" for an immunogenic peptide. These residues are understood to fit in close contact with peptide binding groove of an HLA molecule, with their side chains buried in specific pockets of the binding groove. In one embodiment, for example, the primary anchor residues for an HLA class I molecule are located at position 2 (from the amino terminal position) and at the carboxyl terminal position of a 8, 9, 10, 11, or 12 residue peptide epitope in accordance with the invention. In another embodiment, for example, the primary anchor residues of a peptide that will bind an HLA class II molecule are spaced relative to each other, rather than to the termini of a peptide, where the peptide is generally of at least 9 amino acids in length. The primary anchor positions for each motif and supermotif are set forth in Table IV. For example, analog peptides can be created by altering the presence or absence of particular residues in the primary and/or secondary anchor positions shown in Table IV. Such analogs are used to modulate the binding affinity and/or population coverage of a peptide comprising a particular HLA motif or supermotif.

A "recombinant" DNA or RNA molecule is a DNA or RNA molecule that has been subjected to molecular manipulation *in vitro.*

Non-limiting examples of small molecules include compounds that bind or interact with 121P2A3, ligands including hormones, neuropeptides, chemokines, odorants, phospholipids, and functional equivalents thereof that bind and preferably inhibit 121P2A3 protein function. Such non-limiting small molecules preferably have a molecular weight of less than about 10 kDa, more preferably below about 9, about 8, about 7, about 6, about 5 or about 4 kDa. In certain embodiments, small molecules physically associate with, or bind, 121P2A3 protein; are not found in naturally occurring metabolic pathways; and/or are more soluble in aqueous than non-aqueous solutions

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured nucleic acid sequences to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, are identified by, but not limited to, those that: (1) employ low ionic strength and high temperature for washing, for example 0.015 M sodium chloride/0.0015 M sodium citrate/0.1% sodium dodecyl sulfate at 50˚C; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42 ˚C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42 ˚C, with washes at 42˚C in 0.2 x SSC (sodium chloride/sodium citrate) and 50% formamide at 55 ˚C, followed by a high-stringency wash consisting of 0.1 x SSC containing EDTA at 55 ˚C. "Moderately stringent conditions" are described by, but not limited to, those in Sambrook et al., Molecular Cloning: A Laboratory Manual, New York: Cold Spring Harbor Press, 1989, and include the use of washing solution and hybridization conditions (e.g., temperature, ionic strength and %SDS) less stringent than those described above. An example of moderately stringent conditions is overnight incubation at 37˚C in a solution comprising: 20% formamide, 5 x SSC (150 mM NaCl, 15 mM trisodium citrate), 50 mM sodium phosphate (pH 7.6), 5 x Denhardfs solution, 10% dextran sulfate, and 20 mg/mL denatured sheared salmon sperm DNA, followed by washing the filters in 1 x SSC at about 37-50˚C. The skilled artisan will recognize how to adjust the temperature, ionic strength, etc. as necessary to accommodate factors such as probe length and the like.

An HLA "supermotif' is a peptide binding specificity shared by HLA molecules encoded by two or more HLA alleles.

As used herein "to treat" or "therapeutic" and grammatically related terms, refer to any improvement of any consequence of disease, such as prolonged survival, less morbidity, and/or a lessening of side effects which are the byproducts of an alternative therapeutic modality; full eradication of disease is not required.

A "transgenic animal" (e.g., a mouse or rat) is an animal having cells that contain a transgene, which transgene was introduced into the animal or an ancestor of the animal at a prenatal, e.g., an embryonic stage. A "transgene" is a DNA that is integrated into the genome of a cell from which a transgenic animal develops.

As used herein, an HLA or cellular immune response "vaccine" is a composition that contains or encodes one or more peptides of the invention. There are numerous embodiments of such vaccines, such as a cocktail of one or more individual peptides; one or more peptides of the invention comprised by a polyepitopic peptide; or nucleic acids that encode such individual peptides or polypeptides, *e.g.,* a minigene that encodes a polyepitopic peptide. The "one or more peptides" can include any whole unit integer from 1-150 or more, e.g., at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 105, 110, 115, 120, 125, 130, 135, 140, 145, or 150 or more peptides of the invention. The peptides or polypeptides can optionally be modified, such as by lipidation, addition of targeting or other sequences. HLA class I peptides of the invention can be admixed with, or linked to, HLA class II peptides, to facilitate activation of both cytotoxic T lymphocytes and helper T lymphocytes. HLA vaccines can also comprise peptide-pulsed antigen presenting cells, *e.g.*, dendritic cells.

The term "variant" refers to a molecule that exhibits a variation from a described type or norm, such as a protein that has one or more different amino acid residues in the corresponding position(s) of a specifically described protein (e.g. the 121P2A3 protein shown in Figure 2 or Figure 3. An analog is an example of a variant protein. Splice isoforms and single nucleotides polymorphisms (SNPs) are further examples of variants.

The "121P2A3-related proteins" of the invention include those specifically identified herein, as well as allelic variants, conservative substitution variants, analogs and homologs that can be isolated/generated and characterized without undue experimentation following the methods outlined herein or readily available in the art. Fusion proteins that combine parts of different 121P2A3 proteins or fragments thereof, as well as fusion proteins of a 121P2A3 protein and a heterologous polypeptide are also included. Such 121P2A3 proteins are collectively referred to as the 121P2A3-related proteins, the proteins of the invention, or 121P2A3. The term "121P2A3-related protein" refers to a polypeptide fragment or a 121P2A3 protein sequence of 4, 5, 6, 7, 8, 9, 10, 11,12, 13, 14, 15, 16, 17, 18, 19, 20, 21, 22, 23, 24, 25, 30, 35, 40, 45, 50, 55, 60, 65, 70, 75, 80, 90, 95, 100, or more amino acids.

## II.) 121P2A3 Polynucleotides

One aspect of the invention provides polynucleotides corresponding or complementary to all or part of a 121P2A3 gene, mRNA, and/or coding sequence, preferably in isolated form, including polynucleotides encoding a 12lP2A3-related protein and fragments thereof, DNA, RNA, DNA/RNA hybrid, and related molecules, polynucleotides or oligonucleotides complementary to a 121P2A3 gene or mRNA sequence or a part thereof, and polynucleotides or oligonucleotides that hybridize to a 121P2A3 gene, mRNA, or to a 121P2A3 encoding polynucleotide (collectively, "121P2A3 polynucleotides"). In all instances when referred to in this section, T can also be U in Figure 2.

Embodiments of a 121P2A3 polynucleotide include: a 121P2A3 polynucleotide having the sequence shown in Figure 2, the nucleotide sequence of 121P2A3 as shown in Figure 2 wherein T is U; at least 10 contiguous nucleotides of a polynucleotide having the sequence as shown in Figure 2; or, at least 10 contiguous nucleotides of a polynucleotide having the sequence as shown in Figure 2 where T is U. For example, embodiments of 121P2A3 nucleotides comprise, without limitation:

(I) a polynucleotide comprising, consisting essentially of, or consisting of a sequence as shown in Figure 2 (SEQ ID NO: ______________), wherein T can also be U;

(II) a polynucleotide comprising, consisting essentially of, or consisting of the sequence as shown in Figure 2A (SEQ ID NO: ______________), from nucleotide residue number 175 through nucleotide residue number 1569, including the stop codon, wherein T can also be U;

(III) a polynucleotide comprising, consisting essentially of, or consisting of the sequence as shown in Figure 2B (SEQ ID NO: ______________), from nucleotide residue number 533 through nucleotide residue number 1420, including the stop codon, wherein T can also be U;

(IV) a polynucleotide comprising, consisting essentially of, or consisting of the sequence as shown in Figure 2C (SEQ ID NO: ______________), from nucleotide residue number 175 through nucleotide residue number 1569, including the a stop codon, wherein T can also be U;

(V) a polynucleotide comprising, consisting essentially of, or consisting of the sequence as shown in Figure 2D (SEQ ID NO: ______________), from nucleotide residue number 175 through nucleotide residue number 1569, including the stop codon, wherein T can also be U;

(VI) a polynucleotide comprising, consisting essentially of, or consisting of the sequence as shown in Figure 2E (SEQ ID NO: ______________), from nucleotide residue number 175 through nucleotide residue number 1569, including the stop codon, wherein T can also be U;

(VII) a polynucleotide comprising, consisting essentially of, or consisting of the sequence as shown in Figure 2F (SEQ ID NO: ______________), from nucleotide residue number 175 through nucleotide residue number 1569, including the stop codon, wherein T can also be U;

(VIII) a polynucleotide comprising, consisting essentially of, or consisting of the sequence as shown in Figure 2G (SEQ ID NO: __________________), from nucleotide residue number 175 through nucleotide residue number 1569, including the stop codon, wherein T can also be U;

(IX) a polynucleotide comprising, consisting essentially of, or consisting of the sequence as shown in Figure 2H (SEQ ID NO: __________________), from nucleotide residue number 175 through nucleotide residue number 1569, including the stop codon, wherein T can also be U;

(X) a polynucleotide comprising, consisting essentially of, or consisting of the sequence as shown in Figure 2I (SEQ ID NO: __________________), from nucleotide residue number 175 through nucleotide residue number 1569, including the stop codon, wherein T can also be U;

(XI) a polynucleotide that encodes a 121P2A3-related protein that is at least 90% homologous to an entire amino acid sequence shown in Figure 2A-I (SEQ ID NO: __________________);

(XII) a polynucleotide that encodes a 121P2A3-related protein that is at least 90% identical to an entire amino acid sequence shown in Figure 2A-I (SEQ ID NO: __________________);

(XIII) a polynucleotide that encodes at least one peptide set forth in Tables V-XVIII and XXII-LI;

(XIV) a polynucleotide that encodes a peptide region of at least 5 amino acids of a peptide of Figure 3A, 3C, 3D, 3E, 3F, or 3G in any whole number increment up to 464, or of Figure 3B in any whole number increment up to 295, that includes an amino acid position having a value greater than 0.5 in the Hydrophilicity profile of Figure 5;

(XV) a polynucleotide that encodes a peptide region of at least 5 amino acids of a peptide of Figure 3A, 3C, 3D, 3E, 3F, or 3G in any whole number increment up to 464, or of Figure 3B in any whole number increment up to 295, that includes an amino acid position having a value less than 0.5 in the Hydropathicity profile of Figure 6;

(XVI) a polynucleotide that encodes a peptide region of at least 5 amino acids of a peptide of Figure 3A, 3C, 3D, 3E, 3F, or 3G in any whole number increment up to 464, or of Figure 3B in any whole number increment up to 295, that includes an amino acid position having a value greater than 0.5 in the Percent Accessible Residues profile of Figure 7;

(XVII) a polynucleotide that encodes a peptide region of at least 5 amino acids of a peptide of Figure 3A, 3C, 3D, 3E, 3F, or 3G in any whole number increment up to 464, or of Figure 3B in any whole number increment up to 295, that includes an amino acid position having a value greater than 0.5 in the Average Flexibility profile of Figure 8;

(XVIII) a polynucleotide that encodes a peptide region of at least 5 amino acids of a peptide of Figure 3A, 3C, 3D, 3E, 3F, or 3G in any whole number increment up to 464, or of Figure 3B in any whole number increment up to 295, that includes an amino acid position having a value greater than 0.5 in the Beta-turn profile of Figure 9;

(XIX) a polynucleotide that is fully complementary to a polynucleotide of any one of (I)-(XVIII).

(XX) a polynucleotide that encodes a 121P2A3-related protein whose sequence is encoded by the cDNAs contained in the plasmid deposited on March 1, 2001 with the American Type Culture Collection (ATCC) as Accession No. PTA-3138; and

(XXI) a peptide that is encoded by any of (I)-(XX);

(XXII) a polynucleotide of any of (I)-(XX) or peptide of (XXI) together with a pharmaceutical excipient and/or in a human unit dose form.

As used herein, a range is understood to specifically disclose all whole unit positions thereof.

Typical embodiments of the invention disclosed herein include 121P2A3 polynucleotides that encode specific portions of 121P2A3 mRNA sequences (and those which are complementary to such sequences) such as those that encode the proteins and/or fragments thereof, for example:

(a) 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 105, 110, 115, 120, 125, 130, 135, 140,145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, or 464 contiguous amino acids of 121P2A3.

For example, representative embodiments of the invention disclosed herein include: polynucleotides and their encoded peptides themselves encoding about amino acid 1 to about amino acid 10 of the 121P2A3 protein shown in Figure 2 or Figure 3, polynucleotides encoding about amino acid 10 to about amino acid 20 of the 121P2A3 protein shown in Figure 2 or Figure 3, polynucleotides encoding about amino acid 20 to about amino acid 30 of the 121P2A3 protein shown in Figure 2 or Figure 3, polynucleotides encoding about amino acid 30 to about amino acid 40 of the 121P2A3 protein shown in Figure 2 or Figure 3, polynucleotides encoding about amino acid 40 to about amino acid 50 of the 121P2A3 protein shown in Figure 2 or Figure 3, polynucleotides encoding about amino acid 50 to about amino acid 60 of the 121P2A3 protein shown in Figure 2 or Figure 3, polynucleotides encoding about amino acid 60 to about amino acid 70 of the 121P2A3 protein or variants shown in Figure 2 or Figure 3, polynucleotides encoding about amino acid 70 to about amino acid 80 of the 121P2A3 protein or variants shown in Figure 2 or Figure 3, polynucleotides encoding about amino acid 80 to about amino acid 90 of the 121P2A3 protein or variants shown in Figure 2 or Figure 3, polynucleotides encoding about amino acid 90 to about amino acid 100 of the 121P2A3 protein or variants shown in Figure 2 or Figure 3, or encoding regions from about amino acid 100 to amino acids later in the sequence, in increments of about 10 amino acids, ending at the carboxyl terminal amino acid set forth in Figure 2 or Figure 3. Accordingly polynucleotides encoding portions of the amino acid sequence (of about 10 amino acids), of amino acids 1 through the carboxyl terminal amino acid of the 121P2A3 protein are embodiments of the invention. Wherein it is understood that each particular amino acid position discloses that position plus or minus five amino acid residues.

Polynucleotides encoding relatively long portions of a 121P2A3 protein are also within the scope of the invention. For example, polynucleotides encoding from about amino acid 1 (or 20 or 30 or 40 etc.) to about amino acid 20, (or 30, or 40 or 50 etc.) of the 121P2A3 protein "or variant" shown in Figure 2 or Figure 3 can be generated by a variety of techniques well known in the art. These polynucleotide fragments can include any portion of the 121P2A3 sequence as shown in Figure 2.

Additional illustrative embodiments of the invention disclosed herein include 121P2A3 polynucleotide fragments encoding one or more of the biological motifs contained within a 121P2A3 protein "or variant" sequence, including one or more of the motif-bearing subsequences of a 121P2A3 protein "or variant" set forth in Tables V-XVIII, Table XXI, and Tables XXII-LI. In another embodiment, typical polynucleotide fragments of the invention encode one or more of the regions of 121P2A3 protein or variant that exhibit homology to a known molecule. In another embodiment of the invention, typical polynucleotide fragments can encode one or more of the 121P2A3 protein or variant N-glycosylation sites, cAMP and cGMP-dependent protein kinase phosphorylation sites, casein kinase II phosphorylation sites or N-myristoylation site and amidation sites.

Note that to determine the starting position of any peptide set forth in Tables V-XVIII and Tables XXII-LI (collectively HLA Peptide Tables) respective to its parental protein, e.g., variant 1, variant 2, etc., reference is made to three factors: the particular variant, the length of the peptide in an HLA Peptide Table, and the Search Peptides listed in Table LLII. Generally, a unique Search Peptide is used to obtain HLA peptides for a particular variant. The position of each Search Peptide relative to its respective parent molecule is listed in Table LLII. Accordingly if a Search Peptide begins at position "X", one must add the value "X - 1" to each position in Tables V-XVIII and Tables XXII-LLI to obtain the actual position of the HLA peptides in their parental molecule. For example if a particular Search Peptide begins at position 150 of its parental molecule, one must add 150 -1, i.e., 149 to each HLA peptide amino acid position to calculate the position of that amino acid in the parent molecule.

One embodiment of the invention comprises an HLA peptide, that occurs at least twice in Tables V-XVIII and XXII to LI collectively, or an oligonucleotide that encodes the HLA peptide. Another embodiment of the invention comprises an HLA peptide that occurs at least once in Tables V-XVIII and at least once in tables XXII to LI, or an oligonucleotide that encodes the HLA peptide.

Another embodiment of the invention is antibody epitopes which comprise a peptide regions, or an oligonucleotide encoding the peptide region, that has one two, three, four, or five of the following characteristics:

i) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Hydrophilicity profile of Figure 5;
ii) a peptide region of at least 5 amino acids of a particular peptide of Figure 3, in any whole number increment up to the full length of that protein in Figure 3, that includes an amino acid position having a value equal to or less than 0.5, 0.4, 0.3, 0.2, 0.1, or having a value equal to 0.0, in the Hydropathicity profile of Figure 6;
iii) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Percent Accessible Residues profile of Figure 7;
iv) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Average Flexibility profile of Figure 8; or
v) a peptide region of at least 5 amino acids of a particular peptide of Figure 3, in any whole number increment up to the full length of that protein in Figure 3, that includes an amino acid position having a value equal to or greater than 0.5, 0.6, 0.7, 0.8, 0.9, or having a value equal to 1.0, in the Beta-turn profile of Figure 9.

## II.A.) Uses of 121P2A3 Polynucleotides

### II.A.1.) Monitoring of Genetic Abnormalities

The polynucleotides of the preceding paragraphs have a number of different specific uses. The human 121P2A3 gene maps to the chromosomal location set forth in the Example entitled "Chromosomal Mapping of 121P2A3." For example, because the 121P2A3 gene maps to this chromosome, polynucleotides that encode different regions of the 121P2A3 proteins are used to characterize cytogenetic abnormalities of this chromosomal locale, such as abnormalities that are identified as being associated with various cancers. In certain genes, a variety of chromosomal abnormalities including rearrangements have been identified as frequent cytogenetic abnormalities in a number of different cancers (see e.g. Krajinovic et al., Mutat. Res. 382(3-4): 81-83 (1998); Johansson et al., Blood 86(10): 3905-3914 (1995) and Finger et al., P.N.A.S. 85(23): 9158-9162 (1988)). Thus, polynucleotides encoding specific regions of the 121P2A3 proteins provide new tools that can be used to delineate, with greater precision than previously possible, cytogenetic abnormalities in the chromosomal region that encodes 121P2A3 that may contribute to the malignant phenotype. In this context, these polynucleotides satisfy a need in the art for expanding the sensitivity of chromosomal screening in order to identify more subtle and less common chromosomal abnormalities (see e.g. Evans et al., Am. J. Obstet. Gynecol 171 (4): 1055-1057(1994)).

Furthermore, as 121P2A3 was shown to be highly expressed in bladder and other cancers, 121P2A3 polynucleotides are used in methods assessing the status of 121P2A3 gene products in normal versus cancerous tissues. Typically, polynucleotides that encode specific regions of the 121P2A3 proteins are used to assess the presence of perturbations (such as deletions, insertions, point mutations, or alterations resulting in a loss of an antigen etc.) in specific regions of the 121P2A3 gene, such as regions containing one or more motifs. Exemplary assays include both RT-PCR assays as well as single-strand conformation polymorphism (SSCP) analysis (see, e.g., Marrogi et al., J. Cutan. Pathol. 26(8): 369-378 (1999), both of which utilize polynucleotides encoding specific regions of a protein to examine these regions within the protein.

### II.A.2.) Antisense Embodiments

Other specifically contemplated nucleic acid related embodiments of the invention disclosed herein are genomic DNA, cDNAs, ribozymes, and antisense molecules, as well as nucleic acid molecules based on an alternative backbone, or including alternative bases, whether derived from natural sources or synthesized, and include molecules capable of inhibiting the RNA or protein expression of 121P2A3. For example, antisense molecules can be RNAs or other molecules, including peptide nucleic acids (PNAs) or non-nucleic acid molecules such as phosphorothioate derivatives, that specifically bind DNA or RNA in a base pair-dependent manner. A skilled artisan can readily obtain these classes of nucleic acid molecules using the 121P2A3 polynucleotides and polynucleotide sequences disclosed herein.

Antisense technology entails the administration of exogenous oligonucleotides that bind to a target polynucleotide located within the cells. The term "antisense" refers to the fact that such oligonucleotides are complementary to their intracellular targets, e.g., 121P2A3. See for example, Jack Cohen, Oligodeoxynucleotides, Antisense Inhibitors of Gene Expression, CRC Press, 1989; and Synthesis 1:1-5 (1988). The 121P2A3 antisense oligonucleotides of the present invention include derivatives such as S-oligonucleotides (phosphorothioate derivatives or S-oligos, see, Jack Cohen, supra), which exhibit enhanced cancer cell growth inhibitory action. S-oligos (nucleoside phosphorothioates) are isoelectronic analogs of an oligonucleotide (O-oligo) in which a nonbridging oxygen atom of the phosphate group is replaced by a sulfur atom. The S-oligos of the present invention can be prepared by treatment of the corresponding O-oligos with 3H-1,2-benzodithiol-3-one-1,1-dioxide, which is a sulfur transfer reagent See, *e.g.,* Iyer, R. P. et al., J. Org. Chem. 55: 4693-4698 (1990); and Iyer, R. P. et al., J. Am. Chem. Soc. 112:1253-1254 (1990). Additional 121P2A3 antisense oligonucleotides of the present invention include morpholino antisense oligonucleotides known in the art (see, e.g., Partridge et al., 1996, Antisense & Nucleic Acid Drug Development 6: 169-175).

The 121P2A3 antisense oligonucleotides of the present invention typically can be RNA or DNA that is complementary to and stably hybridizes with the first 100 5' codons or last 100 3' codons of a 121P2A3 genomic sequence or the corresponding mRNA. Absolute complementarity is not required, although high degrees of complementarity are preferred. Use of an oligonucleotide complementary to this region allows for the selective hybridization to 121P2A3 mRNA and not to mRNA specifying other regulatory subunits of protein kinase. In one embodiment, 121P2A3 antisense oligonucleotides of the present invention are 15 to 30-mer fragments of the antisense DNA molecule that have a sequence that hybridizes to 121P2A3 mRNA. Optionally, 121P2A3 antisense oligonucleotide is a 30-mer oligonucleotide that is complementary to a region in the first 10 5' codons or last 10 3' codons of 121P2A3. Alternatively, the antisense molecules are modified to employ ribozymes in the inhibition of 121P2A3 expression, see, e.g., L. A. Couture & D. T. Stinchcomb; Trends Genet 12: 510-515 (1996).

### II.A.3.) Primers and Primer Pairs

Further specific embodiments of this nucleotides of the invention include primers and primer pairs, which allow the specific amplification of polynucleotides of the invention or of any specific parts thereof, and probes that selectively or specifically hybridize to nucleic acid molecules of the invention or to any part thereof. Probes can be labeled with a detectable marker, such as, for example, a radioisotope, fluorescent compound, bioluminescent compound, a chemiluminescent compound, metal chelator or enzyme. Such probes and primers are used to detect the presence of a 121P2A3 polynucleotide in a sample and as a means for detecting a cell expressing a 121P2A3 protein.

Examples of such probes include polypeptides comprising all or part of the human 121P2A3 cDNA sequence shown in Figure 2. Examples of primer pairs capable of specifically amplifying 121P2A3 mRNAs are also described in the Examples. As will be understood by the skilled artisan, a great many different primers and probes can be prepared based on the sequences provided herein and used effectively to amplify and/or detect a 121P2A3 mRNA.

The 121P2A3 polynucleotides of the invention are useful for a variety of purposes, including but not limited to their use as probes and primers for the amplification and/or detection of the 121P2A3 gene(s), mRNA(s), or fragments thereof; as reagents for the diagnosis and/or prognosis of prostate cancer and other cancers; as coding sequences capable of directing the expression of 121P2A3 polypeptides; as tools for modulating or inhibiting the expression of the 121P2A3 gene(s) and/or translation of the 121P2A3 transcript(s); and as therapeutic agents.

The present invention includes the use of any probe as described herein to identify and isolate a 121P2A3 or 121P2A3 related nucleic acid sequence from a naturally occurring source, such as humans or other mammals, as well as the isolated nucleic acid sequence *per se,* which would comprise all or most of the sequences found in the probe used.

### II.A.4.) Isolation of 121P2A3-Encoding Nucleic Acid Molecules

The 121P2A3 cDNA sequences described herein enable the isolation of other polynucleotides encoding 121P2A3 gene product(s), as well as the isolation of polynucleotides encoding 121P2A3 gene product homologs, alternatively spliced isoforms, allelic variants, and mutant forms of a 121P2A3 gene product as well as polynucleotides that encode analogs of 121P2A3-related proteins. Various molecular cloning methods that can be employed to isolate full length cDNAs encoding a 121P2A3 gene are well known (see, for example, Sambrook, J. et al., Molecular Cloning: A Laboratory Manual, 2d edition, Cold Spring Harbor Press, New York, 1989; Current Protocols in Molecular Biology. Ausubel et al., Eds., Wiley and Sons, 1995). For example, lambda phage cloning methodologies can be conveniently employed, using commercially available cloning systems (e.g., Lambda ZAP Express, Stratagene). Phage clones containing 121P2A3 gene cDNAs can be identified by probing with a labeled 121P2A3 cDNA or a fragment thereof. For example, in one embodiment, a 121P2A3 cDNA (e.g., Figure 2) or a portion thereof can be synthesized and used as a probe to retrieve overlapping and full-length cDNAs corresponding to a 121P2A3 gene. A 121P2A3 gene itself can be isolated by screening genomic DNA libraries, bacterial artificial chromosome libraries (BACs), yeast artificial chromosome libraries (YACs), and the like, with 121P2A3 DNA probes or primers.

### II.A.5.) Recombinant Nucleic Acid Molecules and Host-Vector Systems

The invention also provides recombinant DNA or RNA molecules containing a 121P2A3 polynucleotide, a fragment, analog or homologue thereof, including but not limited to phages, plasmids, phagemids, cosmids, YACs, BACs, as well as various viral and non-viral vectors well known in the art, and cells transformed or transfected with such recombinant DNA or RNA molecules. Methods for generating such molecules are well known (see, for example, Sambrook *et al.,* 1989, supra).

The invention further provides a host-vector system comprising a recombinant DNA molecule containing a 121P2A3 polynucleotide, fragment, analog or homologue thereof within a suitable prokaryotic or eukaryotic host cell. Examples of suitable eukaryotic host cells include a yeast cell, a plant cell, or an animal cell, such as a mammalian cell or an insect cell (e.g., a baculovirus-infectible cell such as an Sf9 or HighFive cell). Examples of suitable mammalian cells include various prostate cancer cell lines such as DU145 and TsuPr1, other transfectable or transducible prostate cancer cell lines, primary cells (PrEC), as well as a number of mammalian cells routinely used for the expression of recombinant proteins (e.g., COS, CHO, 293, 293T cells). More particularly, a polynucleotide comprising the coding sequence of 121P2A3 or a fragment, analog or homolog thereof can be used to generate 121P2A3 proteins or fragments thereof using any number of host-vector systems routinely used and widely known in the art.

A wide range of host-vector systems suitable for the expression of 121P2A3 proteins or fragments thereof are available, see for example, Sambrook *et al.,* 1989, supra; Current Protocols in Molecular Biology, 1995, supra). Preferred vectors for mammalian expression include but are not limited to pcDNA 3.1 myc-His-tag (Invitrogen) and the retroviral vector pSRαtkneo (Muller et al., 1991, MCB 11:1785). Using these expression vectors, 121P2A3 can be expressed in several prostate cancer and non-prostate cell lines, including for example 293, 293T, rat-1, NIH 3T3 and TsuPrl. The host-vector systems of the invention are useful for the production of a 121P2A3 protein or fragment thereof. Such host-vector systems can be employed to study the functional properties of 121P2A3 and 121P2A3 mutations or analogs.

Recombinant human 121P2A3 protein or an analog or homolog or fragment thereof can be produced by mammalian cells transfected with a construct encoding a 121P2A3-related nucleotide. For example, 293T cells can be transfected with an expression plasmid encoding 121P2A3 or fragment, analog or homolog thereof, a 121P2A3-related protein is expressed in the 293T cells, and the recombinant 121P2A3 protein is isolated using standard purification methods (e.g., affinity purification using anti-121P2A3 antibodies). In another embodiment, a 121P2A3 coding sequence is subcloned into the retroviral vector pSRαMSVtkneo and used to infect various mammalian cell lines, such as NIH 3T3, TsuPr1, 293 and rat-1 in order to establish 121P2A3 expressing cell lines. Various other expression systems well known in the art can also be employed. Expression constructs encoding a leader peptide joined in frame to a 121P2A3 coding sequence can be used for the generation of a secreted form of recombinant 121P2A3 protein.

As discussed herein, redundancy in the genetic code permits variation in 121P2A3 gene sequences. In particular, it is known in the art that specific host species often have specific codon preferences, and thus one can adapt the disclosed sequence as preferred for a desired host. For example, preferred analog codon sequences typically have rare codons (i.e., codons having a usage frequency of less than about 20% in known sequences of the desired host) replaced with higher frequency codons. Codon preferences for a specific species are calculated, for example, by utilizing codon usage tables available on the INTERNET such as at URL www.dna.affrc.go.jp/~nakamura/codon.html.

Additional sequence modifications are known to enhance protein expression in a cellular host. These include elimination of sequences encoding spurious polyadenylation signals, exon/intron splice site signals, transposon-like repeats, and/or other such well-characterized sequences that are deleterious to gene expression. The GC content of the sequence is adjusted to levels average for a given cellular host, as calculated by reference to known genes expressed in the host cell. Where possible, the sequence is modified to avoid predicted hairpin secondary mRNA structures. Other useful modifications include the addition of a translational initiation consensus sequence at the start of the open reading frame, as described in Kozak, Mol. Cell Biol., 9:5073-5080 (1989). Skilled artisans understand that the general rule that eukaryotic ribosomes initiate translation exclusively at the 5' proximal AUG codon is abrogated only under rare conditions (see, e.g., Kozak PNAS 92(7): 2662-2666, (1995) and Kozak NAR 15(20): 8125-8148 (1987)).

## III.) 121P2A3-related Proteins

Another aspect of the present invention provides 121P2A3-related proteins. Specific embodiments of 121P2A3 proteins comprise a polypeptide having all or part of the amino acid sequence of human 121P2A3 as shown in Figure 2 or Figure 3. Alternatively, embodiments of 121P2A3 proteins comprise variant, homolog or analog polypeptides that have alterations in the amino acid sequence of 121P2A3 shown in Figure 2 or Figure 3.

In general, naturally occurring allelic variants of human 121P2A3 share a high degree of structural identity and homology (e.g., 90% or more homology). Typically, allelic variants of a 121P2A3 protein contain conservative amino acid substitutions within the 121P2A3 sequences described herein or contain a substitution of an amino acid from a corresponding position in a homologue of 121P2A3. One class of 121P2A3 allelic variants are proteins that share a high degree of homology with at least a small region of a particular 121P2A3 amino acid sequence, but further contain a radical departure from the sequence, such as a non-conservative substitution, truncation, insertion or frame shift. In comparisons of protein sequences, the terms, similarity, identity, and homology each have a distinct meaning as appreciated in the field of genetics. Moreover, orthology and paralogy can be important concepts describing the relationship of members of a given protein family in one organism to the members of the same family in other organisms.

Amino acid abbreviations are provided in Table II. Conservative amino acid substitutions can frequently be made in a protein without altering either the conformation or the function of the protein. Proteins of the invention can comprise 1, 2, 3, 4, 5, 6, 7, 8, 9, 10, 11, 12, 13, 14, 15 conservative substitutions. Such changes include substituting any of isoleucine (I), valine (V), and leucine (L) for any other of these hydrophobic amino acids; aspartic acid (D) for glutamic acid (E) and vice versa; glutamine (Q) for asparagine (N) and vice versa; and serine (S) for threonine (T) and vice versa. Other substitutions can also be considered conservative, depending on the environment of the particular amino acid and its role in the three-dimensional structure of the protein. For example, glycine (G) and alanine (A) can frequently be interchangeable, as can alanine (A) and valine (V). Methionine (M), which is relatively hydrophobic, can frequently be interchanged with leucine and isoleucine, and sometimes with valine. Lysine (K) and arginine (R) are frequently interchangeable in locations in which the significant feature of the amino acid residue is its charge and the differing pK's of these two amino acid residues are not significant. Still other changes can be considered "conservative" in particular environments (see, e.g. Table III herein; pages 13-15 "Biochemistry" 2nd ED. Lubert Stryer ed (Stanford University); Henikoff et al., PNAS 1992 Vol 89 10915-10919; Lei et al., J Biol Chem 1995 May 19; 270(20):11882-6).

Embodiments of the invention disclosed herein include a wide variety of art-accepted variants or analogs of 121P2A3 proteins such as polypeptides having amino acid insertions, deletions and substitutions. 121P2A3 variants can be made using methods known in the art such as site-directed mutagenesis, alanine scanning, and PCR mutagenesis. Site-directed mutagenesis (Carter et al., Nucl. Acids Res., 13:4331 (1986); Zoller et al., Nucl. Acids Res., 10:6487 (1987)), cassette mutagenesis (Wells et al., Gene, 34:315 (1985)), restriction selection mutagenesis (Wells et al., Philos. Trans. R. Soc. London SerA, 317:415 (1986)) or other known techniques can be performed on the cloned DNA to produce the 121P2A3 variant DNA.

Scanning amino acid analysis can also be employed to identify one or more amino acids along a contiguous sequence that is involved in a specific biological activity such as a protein-protein interaction. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. Further, it is frequently found in both buried and exposed positions ( Creighton, The Proteins, (W.H. Freeman & Co., N.Y.); Chothia, J. Mol. Biol., 150:1 (1976)). If alanine substitution does not yield adequate amounts of variant, an isosteric amino acid can be used.

As defined herein, 121P2A3 variants, analogs or homologs, have the distinguishing attribute of having at least one epitope that is "cross reactive" with a 121P2A3 protein having an amino acid sequence of Figure 3. As used in this sentence, "cross reactive" means that an antibody or T cell that specifically binds to a 121P2A3 variant also specifically binds to a 121P2A3 protein having an amino acid sequence set forth in Figure 3. A polypeptide ceases to be a variant of a protein shown in Figure 3, when it no longer contains any epitope capable of being recognized by an antibody or T cell that specifically binds to the starting 121P2A3 protein. Those skilled in the art understand that antibodies that recognize proteins bind to epitopes of varying size, and a grouping of the order of about four or five amino acids, contiguous or not, is regarded as a typical number of amino acids in a minimal epitope. See, e.g., Nair et al., J. Immunol 2000 165(12): 6949-6955; Hebbes et al., Mol Immunol (1989) 26(9):865-73; Schwartz et al., J Immunol (1985) 135(4): 2598-608.

Other classes of 121P2A3-related protein variants share 70%, 75%, 80%, 85% or 90% or more similarity with an amino acid sequence of Figure 3, or a fragment thereof. Another specific class of 121P2A3 protein variants or analogs comprise one or more of the 121P2A3 biological motifs described herein or presently known in the art. Thus, encompassed by the present invention are analogs of 121P2A3 fragments (nucleic or amino acid) that have altered functional (e.g. immunogenic) properties relative to the starting fragment. It is to be appreciated that motifs now or which become part of the art are to be applied to the nucleic or amino acid sequences of Figure 2 or Figure 3.

As discussed herein, embodiments of the claimed invention include polypeptides containing less than the full amino acid sequence of a 121P2A3 protein shown in Figure 2 or Figure 3. For example, representative embodiments of the invention comprise peptides/proteins having any 4, 5, 6, 7, 8, 9, 10, 11, 12, 13, 14, 15 or more contiguous amino acids of a 121P2A3 protein shown in Figure 2 or Figure 3.

Moreover, representative embodiments of the invention disclosed herein include polypeptides consisting of about amino acid 1 to about amino acid 10 of a 121P2A3 protein shown in Figure 2 or Figure 3, polypeptides consisting of about amino acid 10 to about amino acid 20 of a 121P2A3 protein shown in Figure 2 or Figure 3, polypeptides consisting of about amino acid 20 to about amino acid 30 of a 121P2A3 protein shown in Figure 2 or Figure 3, polypeptides consisting of about amino acid 30 to about amino acid 40 of a 121P2A3 protein shown in Figure 2 or Figure 3, polypeptides consisting of about amino acid 40 to about amino acid 50 of a 121P2A3 protein shown in Figure 2 or Figure 3, polypeptides consisting of about amino acid 50 to about amino acid 60 of a 121P2A3 protein shown in Figure 2 or Figure 3, polypeptides consisting of about amino acid 60 to about amino acid 70 of a 121P2A3 protein shown in Figure 2 or Figure 3, polypeptides consisting of about amino acid 70 to about amino acid 80 of a 121P2A3 protein shown in Figure 2 or Figure 3, polypeptides consisting of about amino acid 80 to about amino acid 90 of a 121P2A3 protein shown in Figure 2 or Figure 3, polypeptides consisting of about amino acid 90 to about amino acid 100 of a 121P2A3 protein shown in Figure 2 or Figure 3, etc. throughout the entirety of a 121P2A3 amino acid sequence. Moreover, polypeptides consisting of about amino acid 1 (or 20 or 30 or 40 etc.) to about amino acid 20, (or 130, or 140 or 150 etc.) of a 121P2A3 protein shown in Figure 2 or Figure 3 are embodiments of the invention. It is to be appreciated that the starting and stopping positions in this paragraph refer to the specified position as well as that position plus or minus 5 residues.

121P2A3-related proteins are generated using standard peptide synthesis technology or using chemical cleavage methods well known in the art. Alternatively, recombinant methods can be used to generate nucleic acid molecules that encode a 121P2A3-related protein. In one embodiment, nucleic acid molecules provide a means to generate defined fragments of a 121P2A3 protein (or variants, homologs or analogs thereof).

**III.A.) Motif-bearing Protein Embodiments**

Additional illustrative embodiments of the invention disclosed herein include 121P2A3 polypeptides comprising the amino acid residues of one or more of the biological motifs contained within a 121P2A3 polypeptide sequence set forth in Figure 2 or Figure 3. Various motifs are known in the art, and a protein can be evaluated for the presence of such motifs by a number of publicly available Internet sites (see, e.g., URL addresses: URLs pfam.wustl.edul; searchlauncher.bcmtmc.edu/seq-search/struc-predict.htrnl; psort.ims.u-tokyo.ac.jp/; www.cbs.dtu.dk/; www.ebi.ac.uk/interpro/scan.html; www.expasy.ch/tools/scnpsitl.html; Epimatrix™ and Epimer™, Brown University, www.brown.edu/Research/IB-HIV_Lab/epimatrix/epimatrix.html; and BIMAS, bimas.dcrt.nih.gov/.).

Motif bearing subsequences of all 121P2A3 variant proteins are set forth and identified in Tables V-XVIII, Tables XXII-LI, and Table XXI.

Table XIX sets forth several frequently occurring motifs based on pfam searches (see URL address pfam.wustl.edu/). The columns of Table XIX list (1) motif name abbreviation, (2) percent identity found amongst the different member of the motif family, (3) motif name or description and (4) most common function; location information is included if the motif is relevant for location.

Polypeptides comprising one or more of the 121P2A3 motifs discussed above are useful in elucidating the specific characteristics of a malignant phenotype in view of the observation that the 121P2A3 motifs discussed above are associated with growth dysregulation and because 121P2A3 is overexpressed in certain cancers (See, e.g., Table I). Casein kinase II, cAMP and camp-dependent protein kinase, and Protein Kinase C, for example, are enzymes known to be associated with the development of the malignant phenotype (see e.g. Chen et al., Lab Invest., 78(2): 165-174 (1998); Gaiddon et al., Endocrinology 136(10): 4331-4338 (1995); Hall et al., Nucleic Acids Research 24(6): 1119-1126 (1996); Peterziel et al., Oncogene 18(46): 6322-6329 (1999) and O'Brian, Oncol. Rep. 5(2): 305-309 (1998)). Moreover, both glycosylation and myristoylation are protein modifications also associated with cancer and cancer progression (see e.g. Dennis et al., Biochem. Biophys. Acta 1473(1):21-34 (1999); Raju et al., Exp. Cell Res. 235(1): 145-154 (1997)). Amidation is another protein modification also associated with cancer and cancer progression (see e.g. Treston et al., J. Natl. Cancer Inst. Monogr. (13): 169-175 (1992)).

In another embodiment, proteins of the invention comprise one or more of the immunoreactive epitopes identified in accordance with art-accepted methods, such as the peptides set forth in Tables V-XVIII and XXII-LI. CTL epitopes can be determined using specific algorithms to identify peptides within a 121P2A3 protein that are capable of optimally binding to specified HLA alleles (e.g., Table IV; Epimatrix™ and Epimer™, Brown University, URL www.brown.edu/Research/TB-HIV_Lab/epimatrix/epimatrix.html; and BIMAS, URL bimas.dcrt.nih.gov/.) Moreover, processes for identifying peptides that have sufficient binding affinity for HLA molecules and which are correlated with being immunogenic epitopes, are well known in the art, and are carried out without undue experimentation. In addition, processes for identifying peptides that are immunogenic epitopes, are well known in the art, and are carried out without undue experimentation either in *vitro* or *in vivo.*

Also known in the art are principles for creating analogs of such epitopes in order to modulate immunogenicity. For example, one begins with an epitope that bears a CTL or HTL motif (see, e.g., the HLA Class I and HLA Class II motifs/supermotifs of Table IV). The epitope is analoged by substituting out an amino acid at one of the specified positions, and replacing it with another amino acid specified for that position. For example, one can substitute out a deleterious residue in favor of any other residue, such as a preferred residue as defined in Table IV; substitute a less-preferred residue with a preferred residue as defined in Table IV; or substitute an originally-occurring preferred residue with another preferred residue as defined in Table IV. Substitutions can occur at primary anchor positions or at other positions in a peptide; see, e.g., Table IV.

A variety of references reflect the art regarding the identification and generation of epitopes in a protein of interest as well as analogs thereof. See, for example, WO 97/33602 to Chesnut *et al.*; Sette, Immunogenetics 1999 50 (3-4): 201-212; Sette et al., J. Immunol. 2001 166(2): 1389-1397; Sidney et al., Hum. Immunol. 1997 58(1): 12-20; Kondo et al., Immunogenetics 1997 45(4): 249-258; Sidney et al., J. Immunol. 1996 157(8): 3480-90; and Falk et al., Nature 351: 290-6 (1991); Hunt et al., Science 255:1261-3 (1992); Parker et al., J. Immunol. 149:3580-7 (1992); Parker et al., J. Immunol. 152:163-75 (1994)); Kast et al., 1994 152(8): 3904-12; Borras-Cuesta et al., Hum. Immunol. 2000 61(3): 266-278; Alexander et al., J. Immunol. 2000 164(3); 164(3): 1625-1633; Alexander *et al.,* PMID: 7895164, UI: 95202582; O'Sullivan et al., J. Immunol. 1991 147(8): 2663-2669; Alexander et al., Immunity 1994 1(9): 751-761 and Alexander et al., Immunol. Res. 1998 18(2): 79-92.

Related embodiments of the invention include polypeptides comprising combinations of the different motifs set forth in Table XX, and/or, one or more of the predicted CTL epitopes of Tables V-XVII and XXII-XLVII, and/or, one or more of the predicted HTL epitopes of Tables XLVIII-LI, and/or, one or more of the T cell binding motifs known in the art. Preferred embodiments contain no insertions, deletions or substitutions either within the motifs or the intervening sequences of the polypeptides. In addition, embodiments which include a number of either N-terminal and/or C-terminal amino acid residues on either side of these motifs may be desirable (to, for example, include a greater portion of the polypeptide architecture in which the motif is located). Typically the number of N-terminal and/or C-terminal amino acid residues on either side of a motif is between about 1 to about 100 amino acid residues, preferably 5 to about 50 amino acid residues.

121P2A3-related proteins are embodied in many forms, preferably in isolated form A purified 121P2A3 protein molecule will be substantially free of other proteins or molecules that impair the binding of 121P2A3 to antibody, T cell or other ligand. The nature and degree of isolation and purification will depend on the intended use. Embodiments of a 121P2A3-related proteins include purified 121P2A3-related proteins and functional, soluble 121P2A3-related proteins. In one embodiment, a functional, soluble 121P2A3 protein or fragment thereof retains the ability to be bound by antibody, T cell or other ligand.

The invention also provides 121P2A3 proteins comprising biologically active fragments of a 121P2A3 amino acid sequence shown in Figure 2 or Figure 3. Such proteins exhibit properties of the starting 121P2A3 protein, such as the ability to elicit the generation of antibodies that specifically bind an epitope associated with the starting 121P2A3 protein; to be bound by such antibodies; to elicit the activation of HTL or CTL; and/or, to be recognized by HTL or CTL that also specifically bind to the starting protein.

121P2A3-related polypeptides that contain particularly interesting structures can be predicted and/or identified using various analytical techniques well known in the art, including, for example, the methods of Chou-Fasman, Garnier-Robson, Kyte-Doolittle, Eisenberg, Karplus-Schultz or Jameson-Wolf analysis, or on the basis of immunogenicity. Fragments that contain such structures are particularly useful in generating subunit-specific anti-121P2A3 antibodies, or T cells or in identifying cellular factors that bind to 121P2A3. For example, hydrophilicity profiles can be generated, and immunogenic peptide fragments identified, using the method of Hopp, T.P. and Woods, K.R., 1981, Proc. Natl. Acad. Sci. U.S.A. 78:3824-3828. Hydropathicity profiles can be generated, and immunogenic peptide fragments identified, using the method of Kyte, J. and Doolittle, R.F., 1982, J. Mol. Biol. 157:105-132. Percent (%) Accessible Residues profiles can be generated, and immunogenic peptide fragments identified, using the method of Janin J., 1979, Nature 277:491-492. Average Flexibility profiles can be generated, and immunogenic peptide fragments identified, using the method of Bhaskaran R., Ponnuswamy P.K., 1988, Int. J. Pept. Protein Res. 32:242-255. Beta-turn profiles can be generated, and immunogenic peptide fragments identified, using the method of Deleage, G., Roux B., 1987, Protein Engineering 1:289-294.

CTL epitopes can be determined using specific algorithms to identify peptides within a 121P2A3 protein that are capable of optimally binding to specified HLA alleles (e.g., by using the SYFPEITHI site at World Wide Web URL syfpeithi.bmi-heidelberg.com/; the listings in Table IV(A)-(E); Epimatrix™ and Epimer™, Brown University, URL (www.brown.edu/Research/TB-HIV_Lab/epimatrix/epimatrix.html); and BIMAS, URL bimas.dcrt.nih.gov/). Illustrating this, peptide epitopes from 121P2A3 that are presented in the context of human MHC Class I molecules, e.g., HLA-A1, A2, A3, A11, A24, B7 and B35 were predicted (see, e.g., Tables V-XVIII, XXII-LI). Specifically, the complete amino acid sequence of the 121P2A3 protein and relevant portions of other variants, i.e., for HLA Class I predictions 9 flanking redisues on either side of a point mutation, and for HLA Class II predictions 14 flanking residues on either side of a point mutation, were entered into the HLA Peptide Motif Search algorithm found in the Bioinformatics and Molecular Analysis Section (BIMAS) web site listed above; and the site SYFPEITHI at URL syfpeithi.bmi-heidelberg.com/ was used.

The HLA peptide motif search algorithm was developed by Dr. Ken Parker based on binding of specific peptide sequences in the groove of HLA Class I molecules, in particular HLA-A2 (see, e.g., Falk et al., Nature 351: 290-6 (1991) ; Hunt et al., Science 255:1261-3 (1992); Parker et al., J. Immunol. 149:3580-7 (1992); Parker et al., J. Immunol. 152: 163-75 (1994)). This algorithm allows location and ranking of 8-mer, 9-mer, and 10-mer peptides from a complete protein sequence for predicted binding to HLA-A2 as well as numerous other HLA Class I molecules. Many HLA class I binding peptides are 8-, 9-, 10 or 11-mers. For example, for Class I HLA-A2, the epitopes preferably contain a leucine (L) or methionine (M) at position 2 and a valine (V) or leucine (L) at the C-terminus (see, e.g., Parker et al., J. Immunol. 149: 3580-7 (1992)). Selected results of 121P2A3 predicted binding peptides are shown in Tables V-XVIII and XXII-LI herein. In Tables V-XVIll and XXII-LI, selected candidates, 9-mers, 10-mers, and 15-mers for each family member are shown along with their location, the amino acid sequence of each specific peptide, and an estimated binding score. The binding score corresponds to.the estimated half time of dissociation of complexes containing the peptide at 37°C at pH 6.5. Peptides with the highest binding score are predicted to be the most tightly bound to HLA Class I on the cell surface for the greatest period of time and thus represent the best immunogenic targets for T-cell recognition.

Actual binding of peptides to an HLA allele can be evaluated by stabilization of HLA expression on the antigen-processing defective cell line T2 (see, e.g., Xue et al., Prostate 30:73-8 (1997) and Peshwa et al., Prostate 36:129-38 (1998)). Immunogenicity of specific peptides can be evaluated *in vitro* by stimulation of CD8+ cytotoxic T lymphocytes (CTL) in the presence of antigen presenting cells such as dendritic cells.

It is to be appreciated that every epitope predicted by the BIMAS site, Epimer™ and Epimatrix™ sites, or specified by the HLA class I or class II motifs available in the art or which become part of the art such as set forth in Table IV (or determined using World Wide Web site URL syfpeithi.bmi-heidelberg.com/, or BIMAS, bimas.dcrt.nih.gov/) are to be "applied" to a 121P2A3 protein in accordance with the invention. As used in this context "applied" means that a 121P2A3 protein is evaluated, e.g., visually or by computer-based patterns finding methods, as appreciated by those of skill in the relevant art. Every subsequence of a 121P2A3 protein of 8, 9, 10, or 11 amino acid residues that bears an HLA Class I motif, or a subsequence of 9 or more amino acid residues that bear an HLA Class II motif are within the scope of the invention.

**III.B.) Expression of 121P2A3-related Proteins**

In an embodiment described in the examples that follow, 121P2A3 can be conveniently expressed in cells (such as 293T cells) transfected with a commercially available expression vector such as a CMV-driven expression vector encoding 121P2A3 with a C-terminal 6XHis and MYC tag (pcDNA3.1/mycHIS, Invitrogen or Tag5, GenHunter Corporation, Nashville TN). The Tag5 vector provides an IgGK secretion signal that can be used to facilitate the production of a secreted 121P2A3 protein in transfected cells. The secreted HIS-tagged 121P2A3 in the culture media can be purified, e.g., using a nickel column using standard techniques.

**III.C.) Modifications of 121P2A3-related Proteins**

Modifications of 121P2A3-related proteins such as covalent modifications are included within the scope of this invention. One type of covalent modification includes reacting targeted amino acid residues of a 121P2A3 polypeptide with an organic derivatizing agent that is capable of reacting with selected side chains or the N- or C- terminal residues of a 121P2A3 protein. Another type of covalent modification of a 121P2A3 polypeptide included within the scope of this invention comprises altering the native glycosylation pattern of a protein of the invention. Another type of covalent modification of 121P2A3 comprises linking a 121P2A3 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The 121P2A3-related proteins of the present invention can also be modified to form a chimeric molecule comprising 121P2A3 fused to another, heterologous polypeptide or amino acid sequence. Such a chimeric molecule can be synthesized chemically or recombinantly. A chimeric molecule can have a protein of the invention fused to another tumor-associated antigen or fragment thereof. Alternatively, a protein in accordance with the invention can comprise a fusion of fragments of a 121P2A3 sequence (amino or nucleic acid) such that a molecule is created that is not, through its length, directly homologous to the amino or nucleic acid sequences shown in Figure 2 or Figure 3. Such a chimeric molecule can comprise multiples of the same subsequence of 121P2A3. A chimeric molecule can comprise a fusion of a 121P2A3-related protein with a polyhistidine epitope tag, which provides an epitope to which immobilized nickel can selectively bind, with cytokines or with growth factors. The epitope tag is generally placed at the amino- or carboxyl-terminus of a121P2A3 protein. In an alternative embodiment, the chimeric molecule can comprise a fusion of a 121P2A3-related protein with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a 121P2A3 polypeptide in place of at least one variable region within an Ig molecule. In a preferred embodiment, the immunoglobulin fusion includes the hinge, CH2 and CH3, or the hinge, CH1, CH2 and CH3 regions of an IgGI molecule. For the production of immunoglobulin fusions see, e.g., U.S. Patent No. 5,428,130 issued June 27, 1995.

**III.D.) Uses of 121P2A3-related Proteins**

The proteins of the invention have a number of different specific uses. As 121P2A3 is highly expressed in prostate and other cancers, 121P2A3-related proteins are used in methods that assess the status of 121P2A3 gene products in normal versus cancerous tissues, thereby elucidating the malignant phenotype. Typically, polypeptides from specific regions of a 121P2A3 protein are used to assess the presence of perturbations (such as deletions, insertions, point mutations etc.) in those regions (such as regions containing one or more motifs). Exemplary assays utilize antibodies or T cells targeting 121 P2A3-related proteins comprising the amino acid residues of one or more of the biological motifs contained within a 121P2A3 polypeptide sequence in order to evaluate the characteristics of this region in normal versus cancerous tissues or to elicit an immune response to the epitope. Alternatively, 121P2A3-related proteins that contain the amino acid residues of one or more of the biological motifs in a 121P2A3 protein are used to screen for factors that interact with that region of 121P2A3.

121P2A3 protein fragments/subsequences are particularly useful in generating and characterizing domain-specific antibodies (e.g., antibodies recognizing an extracellular or intracellular epitope of a 121P2A3 protein), for identifying agents or cellular factors that bind to 121P2A3 or a particular structural domain thereof, and in various therapeutic and diagnostic contexts, including but not limited to diagnostic assays, cancer vaccines and methods of preparing such vaccines.

Proteins encoded by the 121P2A3 genes, or by analogs, homologs or fragments thereof, have a variety of uses, including but not limited to generating antibodies and in methods for identifying ligands and other agents and cellular constituents that bind to a 121P2A3 gene product. Antibodies raised against a 121P2A3 protein or fragment thereof are useful in diagnostic and prognostic assays, and imaging methodologies in the management of human cancers characterized by expression of 121P2A3 protein, such as those listed in Table I. Such antibodies can be expressed intracellularly and used in methods of treating patients with such cancers. 121P2A3-related nucleic acids or proteins are also used in generating HTL or CTL responses.

Various immunological assays useful for the detection of 121P2A3 proteins are used, including but not limited to various types of radioimmunoassays, enzyme-linked immunosorbent assays (ELISA), enzyme-linked immunofluorescent assays (ELIFA), immunocytochemical methods, and the like. Antibodies can be labeled and used as immunological imaging reagents capable of detecting 121P2A3-expressing cells (e.g., in radioscintigraphic imaging methods). 121P2A3 proteins are also particularly useful in generating cancer vaccines, as further described herein.

## IV.) 121P2A3 Antibodies

Another aspect of the invention provides antibodies that bind to 121P2A3-related proteins. Preferred antibodies specifically bind to a 121P2A3-related protein and do not bind (or bind weakly) to peptides or proteins that are not 121P2A3-related proteins. For example, antibodies that bind 121P2A3 can bind 121P2A3-related proteins such as the homologs or analogs thereof.

121P2A3 antibodies of the invention are particularly useful in cancer (see, e.g., Table I) diagnostic and prognostic assays, and imaging methodologies. Similarly, such antibodies are useful in the treatment, diagnosis, and/or prognosis of other cancers, to the extent 121P2A3 is also expressed or overexpressed in these other cancers. Moreover, intracellularly expressed antibodies (e.g., single chain antibodies) are therapeutically useful in treating cancers in which the expression of 121P2A3 is involved, such as advanced or metastatic prostate cancers.

The invention also provides various immunological assays useful for the detection and quantification of 121P2A3 and mutant 121P2A3-related proteins. Such assays can comprise one or more 121P2A3 antibodies capable of recognizing and binding a 121P2A3-related protein, as appropriate. These assays are performed within various immunological assay formats well known in the art, including but not limited to various types of radioimmunoassays, enzyme-linked immunosorbent assays (ELISA), enzyme-linked immunofluorescent assays (ELIFA), and the like.

Immunological non-antibody assays of the invention also comprise T cell immunogenicity assays (inhibitory or stimulatory) as well as major histocompatibility complex (MHC) binding assays.

In addition, immunological imaging methods capable of detecting prostate cancer and other cancers expressing 121P2A3 are also provided by the invention, including but not limited to radioscintigraphic imaging methods using labeled 121P2A3 antibodies. Such assays are clinically useful in the detection, monitoring, and prognosis of 121P2A3 expressing cancers such as prostate cancer.

121P2A3 antibodies are also used in methods for purifying a 121P2A3-related protein and for isolating 121P2A3 homologues and related molecules. For example, a method of purifying a 121P2A3-related protein comprises incubating a 121P2A3 antibody, which has been coupled to a solid matrix, with a lysate or other solution containing a 121P2A3-related protein under conditions that permit the 121P2A3 antibody to bind to the 121P2A3-related protein; washing the solid matrix to eliminate impurities; and eluting the 121P2A3-related protein from the coupled antibody. Other uses of 121P2A3 antibodies in accordance with the invention include generating anti-idiotypic antibodies that mimic a 121P2A3 protein.

Various methods for the preparation of antibodies are well known in the art. For example, antibodies can be prepared by immunizing a suitable mammalian host using a 121P2A3-related protein, peptide, or fragment, in isolated or immunoconjugated form (Antibodies: A Laboratory Manual, CSH Press, Eds., Harlow, and Lane (1988); Harlow, Antibodies, Cold Spring Harbor Press, NY (1989)). In addition, fusion proteins of 121P2A3 can also be used, such as a 121P2A3 GST-fusion protein. In a particular embodiment, a GST fusion protein comprising all or most of the amino acid sequence of Figure 2 or Figure 3 is produced, then used as an immunogen to generate appropriate antibodies. In another embodiment, a 121P2A3-related protein is synthesized and used as an immunogen.

In addition, naked DNA immunization techniques known in the art are used (with or without purified 121P2A3-related protein or 121P2A3 expressing cells) to generate an immune response to the encoded immunogen (for review, see Donnelly et al., 1997, Ann. Rev. Immunol. 15: 617-648).

The amino acid sequence of a 121P2A3 protein as shown in Figure 2 or Figure 3 can be analyzed to select specific regions of the 121P2A3 protein for generating antibodies. For example, hydrophobicity and hydrophilicity analyses of a 121P2A3 amino acid sequence are used to identify hydrophilic regions in the 121P2A3 structure. Regions of a 121P2A3 protein that show immunogenic structure, as well as other regions and domains, can readily be identified using various other methods known in the art, such as Chou-Fasman, Garnier-Robson, Kyte-Doolittle, Eisenberg, Karplus-Schultz or Jameson-Wolf analysis. Hydrophilicity profiles can be generated using the method of Hopp, T.P. and Woods, K.R., 1981, Proc. Natl. Acad. Sci. U.S.A. 78:3824-3828. Hydropathicity profiles can be generated using the method of Kyte, J. and Doolittle, R.F., 1982, J. Mol. Biol. 157:105-132. Percent (%) Accessible Residues profiles can be generated using the method of Janin J., 1979, Nature 277:491-492. Average Flexibility profiles can be generated using the method of Bhaskaran R., Ponnuswamy P.K., 1988, Int. J. Pept. Protein Res. 32:242-255. Beta-turn profiles can be generated using the method of Deleage, G., Roux B., 1987, Protein Engineering 1:289-294. Thus, each region identified by any of these programs or methods is within the scope of the present invention. Methods for the generation of 121P2A3 antibodies are further illustrated by way of the examples provided herein. Methods for preparing a protein or polypeptide for use as an immunogen are well known in the art Also well known in the art are methods for preparing immunogenic conjugates of a protein with a carrier, such as BSA, KLH or other carrier protein. In some circumstances, direct conjugation using, for example, carbodiimide reagents are used; in other instances linking reagents such as those supplied by Pierce Chemical Co., Rockford, IL, are effective. Administration of a 121P2A3 immunogen is often conducted by injection over a suitable time period and with use of a suitable adjuvant, as is understood in the art. During the immunization schedule, titers of antibodies can be taken to determine adequacy of antibody formation.

121P2A3 monoclonal antibodies can be produced by various means well known in the art. For example, immortalized cell lines that secrete a desired monoclonal antibody are prepared using the standard hybridoma technology of Kohler and Milstein or modifications that immortalize antibody-producing B cells, as is generally known. Immortalized cell lines that secrete the desired antibodies are screened by immunoassay in which the antigen is a 121P2A3-related protein. When the appropriate immortalized cell culture is identified, the cells can be expanded and antibodies produced either from *in vitro* cultures or from ascites fluid.

The antibodies or fragments of the invention can also be produced, by recombinant means. Regions that bind specifically to the desired regions of a 121P2A3 protein can also be produced in the context of chimeric or, complementarity determining region (CDR) grafted antibodies of multiple species origin. Humanized or human 121P2A3 antibodies can also be produced, and are preferred for use in therapeutic contexts. Methods for humanizing murine and other non-human antibodies, by substituting one or more of the non-human antibody CDRs for corresponding human antibody sequences, are well known (see for example, Jones et al., 1986, Nature 321: 522-525; Riechmann et al., 1988, Nature 332: 323-327; Verhoeyen et al., 1988, Science 239: 1534-1536). See also, Carter et al., 1993, Proc. Natl. Acad. Sci. USA 89: 4285 and Sims et al., 1993, J. Immunol. 151: 2296.

Methods for producing fully human monoclonal antibodies include phage display and transgenic methods (for review, see Vaughan et al., 1998, Nature Biotechnology 16: 535-539). Fully human 121P2A3 monoclonal antibodies can be generated using cloning technologies employing large human Ig gene combinatorial libraries (i.e., phage display) (Griffiths and Hoogenboom, Building an in vitro immune system human antibodies from phage display libraries. In: Protein Engineering of Antibody Molecules for Prophylactic and Therapeutic Applications in Man, Clark, M. (Ed.), Nottingham Academic, pp 45-64 (1993); Burton and Barbas, Human Antibodies from combinatorial libraries. Id., pp 65-82). Fully human 121P2A3 monoclonal antibodies can also be produced using transgenic mice engineered to contain human immunoglobulin gene loci as described in PCT Patent Application WO98/24893, Kucherlapati and Jakobovits et al., published December 3,1997 (see also, Jakobovits, 1998, Exp. Opin. Invest. Drugs 7(4): 607-614; U.S. patents 6,162,963 issued 19 December 2000; 6,150,584 issued 12 November 2000; and, 6,114598 issued 5 September 2000). This method avoids the *in vitro* manipulation required with phage display technology and efficiently produces high affinity authentic human antibodies.

Reactivity of 121P2A3 antibodies with a 121P2A3-related protein can be established by a number of well known means, including Western blot, immunoprecipitation, ELISA, and FACS analyses using, as appropriate, 121P2A3-related proteins, 121P2A3-expressing cells or extracts thereof. A 121P2A3 antibody or fragment thereof can be labeled with a detectable marker or conjugated to a second molecule. Suitable detectable markers include, but are not limited to, a radioisotope, a fluorescent compound, a bioluminescent compound, chemiluminescent compound, a metal chelator or an enzyme. Further, bi-specific antibodies specific for two or more 121P2A3 epitopes are generated using methods generally known in the art. Homodimeric antibodies can also be generated by cross-linking techniques known in the art (e.g., Wolff et al., Cancer Res. 53: 2560-2565).

## V.) 121P2A3 Cellular Immune Responses

The mechanism by which T cells recognize antigens has been delineated. Efficacious peptide epitope vaccine compositions of the invention induce a therapeutic or prophylactic immune responses in very broad segments of the world-wide population. For an understanding of the value and efficacy of compositions of the invention that induce cellular immune responses, a brief review of immunology-related technology is provided.

A complex of an HLA molecule and a peptidic antigen acts as the ligand recognized by HLA-restricted T cells (Buus, S. et al., Cell 47:1071, 1986; Babbitt, B. P. et al., Nature 317:359, 1985; Townsend, A. and Bodmer, H., Annu. Rev. Immunol.,7:601, 1989; Germain, R. N., Annu. Rev. Immunol. 11:403, 1993). Through the study of single amino acid substituted antigen analogs and the sequencing of endogenously bound, naturally processed peptides, critical residues that correspond to motifs required for specific binding to HLA antigen molecules have been identified and are set forth in Table IV (*see* also, *e.g.,* Southwood, et al., J. Immunol. 160:3363, 1998; Rammensee, et al., Immunogenetics 41:178, 1995; Rammensee *et al.,* SYFPEITHI, access via World Wide Web at URL syfpeithi.bmi-heidelberg.com/; Sette, A. and Sidney, J. Curr. Opin. Immunol. 10:478, 1998; Engelhard, V. H., Curr. Opin. Immunol. 6:13, 1994; Sette, A. and Grey, H. M., Curr. Opin. Immunol. 4:79, 1992; Sinigaglia, F. and Hammer, J. Curr. Biol. 6:52, 1994; Ruppert et al., Cell 74:929-937, 1993; Kondo et al., J. Immunol. 155:4307-4312, 1995; Sidney et al., J. Immunol. 157:3480-3490,1996; Sidney et al., Human Immunol. 45:79-93, 1996; Sette, A. and Sidney, J. Immunogenetics 1999 Nov; 50(3-4):201-12*,* Review).

Furthermore, x-ray crystallographic analyses of HLA-peptide complexes have revealed pockets within the peptide binding cleft/groove of HLA molecules which accommodate, in an allele-specific mode, residues borne by peptide ligands; these residues in turn determine the HLA binding capacity of the peptides in which they are present (*See, e.g.,* Madden, D.R. Annu. Rev. Immunol. 13:587, 1995; Smith, et al., Immunity 4:203, 1996; Fremont et al., Immunity 8:305, 1998; Stem et al., Structure 2:245, 1994; Jones, E.Y. Curr. Opin. Immunol. 9:75, 1997; Brown, J. H. et al., Nature 364: 33, 1993; Guo, H. C. et al., Proc. Natl. Acad. Sci. USA 90:8053, 1993; Guo, H. C. et al., Nature 360:364, 1992; Silver, M. L. et al., Nature 360:367, 1992; Matsumura, M. et al., Science 257:927, 1992; Madden et al., Cell 70:1035, 1992; Fremont, D. H, et al., Science 257:919, 1992; Saper, M. A. , Bjorkman, P. J. and Wiley, D. C., J. Mol. Biol. 219:277, 1991.)

Accordingly, the definition of class I and class II allele-specific HLA binding motifs, or class I or class II supermotifs allows identification of regions within a protein that are correlated with binding to particular HLA antigen(s).

Thus, by a process of HLA motif identification, candidates for epitope-based vaccines have been identified; such candidates can be further evaluated by HLA-peptide binding assays to determine binding affinity and/or the time period of association of the epitope and its corresponding HLA molecule. Additional confirmatory work can be performed to select, amongst these vaccine candidates, epitopes with preferred characteristics in terms of population coverage, and/or immunogenicity.

Various strategies can be utilized to evaluate cellular immunogenicity, including:

1) Evaluation of primary T cell cultures from normal individuals (*see, e.g.,* Wentworth, P. A. et al., Mol. Immunol. 32:603, 1995; Celis, E. et al., Proc. Natl. Acad. Sci. USA 91:2105, 1994; Tsai, V. et al., J. Immunol. 158:1796, 1997; Kawashima, I. et al., Human Immunol. 59:1, 1998). This procedure involves the stimulation of peripheral blood lymphocytes (PBL) from normal subjects with a test peptide in the presence of antigen presenting cells *in vitro* over a period of several weeks. T cells specific for the peptide become activated during this time and are detected using, *e.g.,* a lymphokine- or $^{51}$Cr-release assay involving peptide sensitized target cells.

2) Immunization of HLA transgenic mice (*see, e.g.,* Wentworth, P. A. et al., J. Immunol. 26:97, 1996; Wentworth, P. A. et al., Int. Immunol. 8:651, 1996; Alexander, J. et al., J. Immunol. 159:4753, 1997). For example, in such methods peptides in incomplete Freund's adjuvant are administered subcutaneously to HLA transgenic mice. Several weeks following immunization, splenocytes are removed and cultured *in vitro* in the presence of test peptide for approximately one week. Peptide-specific T cells are detected using, *e.g*., a $^{51}$Cr-release assay involving peptide sensitized target cells and target cells expressing endogenously generated antigen.

3) Demonstration of recall T cell responses from immune individuals who have been either effectively vaccinated and/or from chronically ill patients (*see, e.g.,* Rehermann, B. et al., J. Exp. Med. 181:1047, 1995; Doolan, D. L. et al., Immunity 7:97, 1997; Bertoni, R. et al., J. Clin. Invest. 100:503, 1997; Threlkeld, S. C. et al., J. Immunol. 159: 1648, 1997; Diepolder, H. M. et al., J. Virol. 71:6011, 1997). Accordingly, recall responses are detected by culturing PBL from subjects that have been exposed to the antigen due to disease and thus have generated an immune response "naturally", or from patients who were vaccinated against the antigen. PBL from subjects are cultured *in vitro* for 1-2 weeks in the presence of test peptide plus antigen presenting cells (APC) to allow activation of "memory" T cells, as compared to "naive" T cells. At the end of the culture period, T cell activity is detected using assays including $^{51}$Cr release involving peptide-sensitized targets, T cell proliferation, or lymphokine release.

**VI.) 121P2A3 Transgenic Animals**

Nucleic acids that encode a 121P2A3-related protein can also be used to generate either transgenic animals or "knock out" animals that, in turn, are useful in the development and screening of therapeutically useful reagents. In accordance with established techniques, cDNA encoding 121P2A3 can be used to clone genomic DNA that encodes 121P2A3. The cloned genomic sequences can then be used to generate transgenic animals containing cells that express DNA that encode 121P2A3. Methods for generating transgenic animals, particularly animals such as mice or rats, have become conventional in the art and are described, for example, in U.S. Patent Nos. 4,736,866 issued 12 April 1988, and 4,870,009 issued 26 September 1989. Typically, particular cells would be targeted for 121P2A3 transgene incorporation with tissue-specific enhancers.

Transgenic animals that include a copy of a transgene encoding 121P2A3 can be used to examine the effect of increased expression of DNA that encodes 121P2A3. Such animals can be used as tester animals for reagents thought to confer protection from, for example, pathological conditions associated with its overexpression. In accordance with this aspect of the invention, an animal is treated with a reagent and a reduced incidence of a pathological condition, compared to untreated animals that bear the transgene, would indicate a potential therapeutic intervention for the pathological condition.

Alternatively, non-human homologues of 121P2A3 can be used to construct a 121P2A3 "knock out" animal that has a defective or altered gene encoding 121P2A3 as a result of homologous recombination between the endogenous gene encoding 121P2A3 and altered genomic DNA encoding 121P2A3 introduced into an embryonic cell of the animal. For example, cDNA that encodes 121P2A3 can be used to clone genomic DNA encoding 121P2A3 in accordance with established techniques. A portion of the genomic DNA encoding 121P2A3 can be deleted or replaced with another gene, such as a gene encoding a selectable marker that can be used to monitor integration. Typically, several kilobases of unaltered flanking DNA (both at the 5' and 3' ends) are included in the vector (see, e.g., Thomas and Capecchi, Cell, 51:503 (1987) for a description of homologous recombination vectors). The vector is introduced into an embryonic stem cell line (e.g., by electroporation) and cells in which the introduced DNA has homologously recombined with the endogenous DNA are selected (see, e.g., Li et al., Cell, 69:915 (1992)). The selected cells are then injected into a blastocyst of an animal (e.g., a mouse or rat) to form aggregation chimeras (see, e.g., Bradley, in Teratocarcinomas and Embryonic Stem Cells: A Practical Approach, E. J. Robertson, ed. (IRL, Oxford, 1987), pp. 113-152). A chimeric embryo can then be implanted into a suitable pseudopregnant female foster animal, and the embryo brought to term to create a "knock out" animal. Progeny harboring the homologously recombined DNA in their germ cells can be identified by standard techniques and used to breed animals in which all cells of the animal contain the homologously recombined DNA. Knock out animals can be characterized, for example, for their ability to defend against certain pathological conditions or for their development of pathological conditions due to absence of a 121P2A3 polypeptide.

## VII.) Methods for the Detection of 121P2A3

Another aspect of the present invention relates to methods for detecting 121P2A3 polynucleotides and 121P2A3-related proteins, as well as methods for identifying a cell that expresses 121P2A3. The expression profile of 121P2A3 makes it a diagnostic marker for metastasized disease. Accordingly, the status of 121P2A3 gene products provides information useful for predicting a variety of factors including susceptibility to advanced stage disease, rate of progression, and/or tumor aggressiveness. As discussed in detail herein, the status of 121P2A3 gene products in patient samples can be analyzed by a variety protocols that are well known in the art including immunohistochemical analysis, the variety of Northern blotting techniques including *in situ* hybridization, RT-PCR analysis (for example on laser capture micro-dissected samples), Western blot analysis and tissue array analysis.

More particularly, the invention provides assays for the detection of 121P2A3 polynucleotides in a biological sample, such as serum, bone, prostate, and other tissues, urine, semen, cell preparations, and the like. Detectable 121P2A3 polynucleotides include, for example, a 121P2A3 gene or fragment thereof, 121P2A3 mRNA, alternative splice variant 121P2A3 mRNAs, and recombinant DNA or RNA molecules that contain a 121P2A3 polynucleotide. A number of methods for amplifying and/or detecting the presence of 121P2A3 polynucleotides are well known in the art and can be employed in the practice of this aspect of the invention.

In one embodiment, a method for detecting a 121P2A3 mRNA in a biological sample comprises producing cDNA from the sample by reverse transcription using at least one primer; amplifying the cDNA so produced using a 121P2A3 polynucleotides as sense and antisense primers to amplify 121P2A3 cDNAs therein; and detecting the presence of the amplified 121P2A3 cDNA. Optionally, the sequence of the amplified 121P2A3 cDNA can be determined.

In another embodiment, a method of detecting a 121P2A3 gene in a biological sample comprises first isolating genomic DNA from the sample; amplifying the isolated genomic DNA using 121P2A3 polynucleotides as sense and antisense primers; and detecting the presence of the amplified 121P2A3 gene. Any number of appropriate sense and antisense probe combinations can be designed from a 121P2A3 nucleotide sequence (see, e.g., Figure 2) and used for this purpose.

The invention also provides assays for detecting the presence of a 121P2A3 protein in a tissue or other biological sample such as serum, semen, bone, prostate, urine, cell preparations, and the like. Methods for detecting a 121P2A3-related protein are also well known and include, for example, immunoprecipitation, immunohistochemical analysis, Western blot analysis, molecular binding assays, ELISA, ELIFA and the like. For example, a method of detecting the presence of a 121P2A3-related protein in a biological sample comprises first contacting the sample with a 121P2A3 antibody, a 121P2A3-reactive fragment thereof, or a recombinant protein containing an antigen binding region of a 121P2A3 antibody; and then detecting the binding of 121P2A3-related protein in the sample.

Methods for identifying a cell that expresses 121P2A3 are also within the scope of the invention. In one embodiment, an assay for identifying a cell that expresses a 121P2A3 gene comprises detecting the presence of 121P2A3 mRNA in the cell. Methods for the detection of particular mRNAs in cells are well known and include, for example, hybridization assays using complementary DNA probes (such as *in situ* hybridization using labeled 121P2A3 riboprobes, Northern blot and related techniques) and various nucleic acid amplification assays (such as RT-PCR using complementary primers specific for 121P2A3, and other amplification type detection methods, such as, for example, branched DNA, SISBA, TMA and the like). Alternatively, an assay for identifying a cell that expresses a 121P2A3 gene comprises detecting the presence of 121P2A3-related protein in the cell or secreted by the cell. Various methods for the detection of proteins are well known in the art and are employed for the detection of 121P2A3-related proteins and cells that express 121P2A3-related proteins.

121P2A3 expression analysis is also useful as a tool for identifying and evaluating agents that modulate 121P2A3 gene expression. For example, 121P2A3 expression is significantly upregulated in prostate cancer, and is expressed in cancers of the tissues listed in Table I. Identification of a molecule or biological agent that inhibits 121P2A3 expression or over-expression in cancer cells is of therapeutic value. For example, such an agent can be identified by using a screen that quantifies 121P2A3 expression by RT-PCR, nucleic acid hybridization or antibody binding.

**VIII.) Methods for Monitoring the Status of 121P2A3-related Genes and Their Products**

Oncogenesis is known to be a multistep process where cellular growth becomes progressively dysregulated and cells progress from a normal physiological state to precancerous and then cancerous states (see, e.g., Alers et al., Lab Invest. 77(5): 437-438 (1997) and Isaacs et al., Cancer Surv. 23: 19-32 (1995)). In this context, examining a biological sample for evidence of dysregulated cell growth (such as aberrant 121P2A3 expression in cancers) allows for early detection of such aberrant physiology, before a pathologic state such as cancer has progressed to a stage that therapeutic options are more limited and or the prognosis is worse. In such examinations, the status of 121P2A3 in a biological sample of interest can be compared, for example, to the status of 121P2A3 in a corresponding normal sample (e.g. a sample from that individual or alternatively another individual that is not affected by a pathology). An alteration in the status of 121P2A3 in the biological sample (as compared to the normal sample) provides evidence of dysregulated cellular growth. In addition to using a biological sample that is not affected by a pathology as a normal sample, one can also use a predetermined normative value such as a predetermined normal level of mRNA expression (see, e.g., Grever et al., J. Comp. Neurol. 1996 Dec 9; 376(2): 306-14 and U.S. Patent No. 5,837,501) to compare 121P2A3 status in a sample.

The term "status" in this context is used according to its art accepted meaning and refers to the condition or state of a gene and its products. Typically, skilled artisans use a number of parameters to evaluate the condition or state of a gene and its products. These include, but are not limited to the location of expressed gene products (including the location of 121P2A3 expressing cells) as well as the level, and biological activity of expressed gene products (such as 121P2A3 mRNA, polynucleotides and polypeptides). Typically, an alteration in the status of 121P2A3 comprises a change in the location of 121P2A3 and/or 121P2A3 expressing cells and/or an increase in 121P2A3 mRNA and/or protein expression.

121P2A3 status in a sample can be analyzed by a number of means well known in the art, including without limitation, immunohistochemical analysis, *in situ* hybridization, RT-PCR analysis on laser capture micro-dissected samples, Western blot analysis, and tissue array analysis. Typical protocols for evaluating the status of a 121P2A3 gene and gene products are found, for example in Ausubel *et al.* eds., 1995, Current Protocols In Molecular Biology, Units 2 (Northern Blotting), 4 (Southern Blotting), 15 (Immunoblotting) and 18 (PCR Analysis). Thus, the status of 121P2A3 in a biological sample is evaluated by various methods utilized by skilled artisans including, but not limited to genomic Southern analysis (to examine, for example perturbations in a 121P2A3 gene), Northern analysis and/or PCR analysis of 121P2A3 mRNA (to examine, for example alterations in the polynucleotide sequences or expression levels of 121P2A3 mRNAs), and, Western and/or immunohistochemical analysis (to examine, for example alterations in polypeptide sequences, alterations in polypeptide localization within a sample, alterations in expression levels of 121P2A3 proteins and/or associations of 121P2A3 proteins with polypeptide binding partners). Detectable 121P2A3 polynucleotides include, for example, a 121P2A3 gene or fragment thereof, 121P2A3 mRNA, alternative splice variants, 121P2A3 mRNAs, and recombinant DNA or RNA molecules containing a 121P2A3 polynucleotide.

The expression profile of 121P2A3 makes it a diagnostic marker for local and/or metastasized disease, and provides information on the growth or oncogenic potential of a biological sample. In particular, the status of 121P2A3 provides information useful for predicting susceptibility to particular disease stages, progression, and/or tumor aggressiveness. The invention provides methods and assays for determining 121P2A3 status and diagnosing cancers that express 121P2A3, such as cancers of the tissues listed in Table I. For example, because 121P2A3 mRNA is so highly expressed in prostate and other cancers relative to normal prostate tissue, assays that evaluate the levels of 121P2A3 mRNA transcripts or proteins in a biological sample can be used to diagnose a disease associated with 121P2A3 dysregulation, and can provide prognostic information useful in defining appropriate therapeutic options.

The expression status of 121P2A3 provides information including the presence, stage and location of dysplastic, precancerous and cancerous cells, predicting susceptibility to various stages of disease, and/or for gauging tumor aggressiveness. Moreover, the expression profile makes it useful as an imaging reagent for metastasized disease. Consequently, an aspect of the invention is directed to the various molecular prognostic and diagnostic methods for examining the status of 121P2A3 in biological samples such as those from individuals suffering from, or suspected of suffering from a pathology characterized by dysregulated cellular growth, such as cancer.

As described above, the status of 121P2A3 in a biological sample can be examined by a number of well-known procedures in the art. For example, the status of 121P2A3 in a biological sample taken from a specific location in the body can be examined by evaluating the sample for the presence or absence of 121P2A3 expressing cells (e.g. those that express 121P2A3 mRNAs or proteins). This examination can provide evidence of dysregulated cellular growth, for example, when 121P2A3-expressing cells are found in a biological sample that does not normally contain such cells (such as a lymph node), because such alterations in the status of 121P2A3 in a biological sample are often associated with dysregulated cellular growth. Specifically, one indicator of dysregulated cellular growth is the metastases of cancer cells from an organ of origin (such as the prostate) to a different area of the body (such as a lymph node). In this context, evidence of dysregulated cellular growth is important for example because occult lymph node metastases can be detected in a substantial proportion of patients with prostate cancer, and such metastases are associated with known predictors of disease progression (see, e.g., Murphy et al., Prostate 42(4): 315-317 (2000);Su et al., Semin. Surg. Oncol. 18(1): 17-28 (2000) and Freeman et al., J Urol 1995 Aug 154(2 Pt 1):474-8).

In one aspect, the invention provides methods for monitoring 121P2A3 gene products by determining the status of 121P2A3 gene products expressed by cells from an individual suspected of having a disease associated with dysregulated cell growth (such as hyperplasia or cancer) and then comparing the status so determined to the status of 121P2A3 gene products in a corresponding normal sample. The presence of aberrant 121P2A3 gene products in the test sample relative to the normal sample provides an indication of the presence of dysregulated cell growth within the cells of the individual.

In another aspect, the invention provides assays useful in determining the presence of cancer in an individual, comprising detecting a significant increase in 121P2A3 mRNA or protein expression in a test cell or tissue sample relative to expression levels in the corresponding normal cell or tissue. The presence of 121P2A3 mRNA can, for example, be evaluated in tissues including but not limited to those listed in Table I. The presence of significant 121P2A3 expression in any of these tissues is useful to indicate the emergence, presence and/or severity of a cancer, since the corresponding normal tissues do not express 121P2A3 mRNA or express it at lower levels.

In a related embodiment, 121P2A3 status is determined at the protein level rather than at the nucleic acid level. For example, such a method comprises determining the level of 121P2A3 protein expressed by cells in a test tissue sample and comparing the level so determined to the level of 121P2A3 expressed in a corresponding normal sample. In one embodiment, the presence of 121P2A3 protein is evaluated, for example, using immunohistochemical methods. 121P2A3 antibodies or binding partners capable of detecting 121P2A3 protein expression are used in a variety of assay formats well known in the art for this purpose.

In a further embodiment, one can evaluate the status of 121P2A3 nucleotide and amino acid sequences in a biological sample in order to identity perturbations in the structure of these molecules. These perturbations can include insertions, deletions, substitutions and the like. Such evaluations are useful because perturbations in the nucleotide and amino acid sequences are observed in a large number of proteins associated with a growth dysregulated phenotype (see, e.g., Marrogi et al., 1999, J. Cutan. Pathol. 26(8):369-378). For example, a mutation in the sequence of 121P2A3 may be indicative of the presence or promotion of a tumor. Such assays therefore have diagnostic and predictive value where a mutation in 121P2A3 indicates a potential loss of function or increase in tumor growth.

A wide variety of assays for observing perturbations in nucleotide and amino acid sequences are well known in the art. For example, the size and structure of nucleic acid or amino acid sequences of 121P2A3 gene products are observed by the Northern, Southern, Western, PCR and DNA sequencing protocols discussed herein. In addition, other methods for observing perturbations in nucleotide and amino acid sequences such as single strand conformation polymorphism analysis are well known in the art (see, e.g., U.S. Patent Nos. 5,382,510 issued 7 September 1999, and 5,952,170 issued 17 January 1995).

Additionally, one can examine the methylation status of a 121P2A3 gene in a biological sample. Aberrant demethylation and/or hypermethylation of CpG islands in gene 5' regulatory regions frequently occurs in immortalized and transformed cells, and can result in altered expression of various genes. For example, promoter hypermethylation of the pi-class glutathione S-transferase (a protein expressed in normal prostate but not expressed in >90% of prostate carcinomas) appears to permanently silence transcription of this gene and is the most frequently detected genomic alteration in prostate carcinomas (De Marzo et al., Am. J. Pathol. 155(6): 1985-1992 (1999)). In addition, this alteration is present in at least 70% of cases of high-grade prostatic intraepithelial neoplasia (PIN) (Brooks et al., Cancer Epidemiol. Biomarkers Prev., 1998, 7:531-536). In another example, expression of the LAGE-I tumor specific gene (which is not expressed in normal prostate but is expressed in 25-50% of prostate cancers) is induced by deoxy-azacytidine in lymphoblastoid cells, suggesting that tumoral expression is due to demethylation (Lethe et al., Int. J. Cancer 76(6): 903-908 (1998)). A variety of assays for examining methylation status of a gene are well known in the art. For example, one can utilize, in Southern hybridization approaches, methylation-sensitive restriction enzymes that cannot cleave sequences that contain methylated CpG sites to assess the methylation status of CpG islands. In addition, MSP (methylation specific PCR) can rapidly profile the methylation status of all the CpG sites present in a CpG island of a given gene. This procedure involves initial modification of DNA by sodium bisulfite (which will convert all unmethylated cytosines to uracil) followed by amplification using primers specific for methylated versus unmethylated DNA. Protocols involving methylation interference can also be found for example in Current Protocols In Molecular Biology, Unit 12, Frederick M. Ausubel et al. eds., 1995.

Gene amplification is an additional method for assessing the status of 121P2A3. Gene amplification is measured in a sample directly, for example, by conventional Southern blotting or Northern blotting to quantitate the transcription of mRNA (Thomas, 1980, Proc. Natl. Acad. Sci. USA, 77:5201-5205), dot blotting (DNA analysis), or *in situ* hybridization, using an appropriately labeled probe, based on the sequences provided herein. Alternatively, antibodies are employed that recognize specific duplexes, including DNA duplexes, RNA duplexes, and DNA-RNA hybrid duplexes or DNA-protein duplexes. The antibodies in turn are labeled and the assay carried out where the duplex is bound to a surface, so that upon the formation of duplex on the surface, the presence of antibody bound to the duplex can be detected.

Biopsied tissue or peripheral blood can be conveniently assayed for the presence of cancer cells using for example, Northern, dot blot or RT-PCR analysis to detect 121P2A3 expression. The presence of RT-PCR amplifiable 121P2A3 mRNA provides an indication of the presence of cancer. RT-PCR assays are well known in the art. RT-PCR detection assays for tumor cells in peripheral blood are currently being evaluated for use in the diagnosis and management of a number of human solid tumors. In the prostate cancer field, these include RT-PCR assays for the detection of cells expressing PSA and PSM (Verkaik et al., 1997, Urol. Res. 25:373-384; Ghossein et al., 1995, J. Clin. Oncol. 13: 1195-2000; Heston et al., 1995, Clin. Chem. 41:1687-1688).

A further aspect of the invention is an assessment of the susceptibility that an individual has for developing cancer. In one embodiment, a method for predicting susceptibility to cancer comprises detecting 121P2A3 mRNA or 121P2A3 protein in a tissue sample, its presence indicating susceptibility to cancer, wherein the degree of 121P2A3 mRNA expression correlates to the degree of susceptibility. In a specific embodiment, the presence of 121P2A3 in prostate or other tissue is examined, with the presence of 121P2A3 in the sample providing an indication of prostate cancer susceptibility (or the emergence or existence of a prostate tumor). Similarly, one can evaluate the integrity 121P2A3 nucleotide and amino acid sequences in a biological sample, in order to identify perturbations in the structure of these molecules such as insertions, deletions, substitutions and the like. The presence of one or more perturbations in 121P2A3 gene products in the sample is an indication of cancer susceptibility (or the emergence or existence of a tumor).

The invention also comprises methods for gauging tumor aggressiveness. In one embodiment, a method for gauging aggressiveness of a tumor comprises determining the level of 121P2A3 mRNA or 121P2A3 protein expressed by tumor cells, comparing the level so determined to the level of 121P2A3 mRNA or 121P2A3 protein expressed in a corresponding normal tissue taken from the same individual or a normal tissue reference sample, wherein the degree of 121P2A3 mRNA or 121P2A3 protein expression in the tumor sample relative to the normal sample indicates the degree of aggressiveness. In a specific embodiment, aggressiveness of a tumor is evaluated by determining the extent to which 121P2A3 is expressed in the tumor cells, with higher expression levels indicating more aggressive tumors. Another embodiment is the evaluation of the integrity of 121P2A3 nucleotide and amino acid sequences in a biological sample, in order to identify perturbations in the structure of these molecules such as insertions, deletions, substitutions and the like. The presence of one or more perturbations indicates more aggressive tumors.

Another embodiment of the invention is directed to methods for observing the progression of a malignancy in an individual over time. In one embodiment, methods for observing the progression of a malignancy in an individual over time comprise determining the level of 121P2A3 mRNA or 121P2A3 protein expressed by cells in a sample of the tumor, comparing the level so determined to the level of 121P2A3 mRNA or 121P2A3 protein expressed in an equivalent tissue sample taken from the same individual at a different time, wherein the degree of 121P2A3 mRNA or 121P2A3 protein expression in the tumor sample over time provides information on the progression of the cancer. In a specific embodiment, the progression of a cancer is evaluated by determining 121P2A3 expression in the tumor cells over time, where increased expression over time indicates a progression of the cancer. Also, one can evaluate the integrity 121P2A3 nucleotide and amino acid sequences in a biological sample in order to identify perturbations in the structure of these molecules such as insertions, deletions, substitutions and the like, where the presence of one or more perturbations indicates a progression of the cancer.

The above diagnostic approaches can be combined with any one of a wide variety of prognostic and diagnostic protocols known in the art. For example, another embodiment of the invention is directed to methods for observing a coincidence between the expression of 121P2A3 gene and 121P2A3 gene products (or perturbations in 121P2A3 gene and 121P2A3 gene products) and a factor that is associated with malignancy, as a means for diagnosing and prognosticating the status of a tissue sample. A wide variety of factors associated with malignancy can be utilized, such as the expression of genes associated with malignancy (e.g. PSA, PSCA and PSM expression for prostate cancer etc.) as well as gross cytological observations (see, e.g., Bocking et al., 1984, Anal. Quant. Cytol. 6(2):74-88; Epstein, 1995, Hum. Pathol. 26(2):223-9; Thorson et al., 1998, Mod. Pathol. 11(6):543-51; Baisden et al., 1999, Am. J. Surg. Pathol. 23(8): 918-24). Methods for observing a coincidence between the expression of 121P2A3 gene and 121P2A3 gene products (or perturbations in 121P2A3 gene and 121P2A3 gene products) and another factor that is associated with malignancy are useful, for example, because the presence of a set of specific factors that coincide with disease provides information crucial for diagnosing and prognosticating the status of a tissue sample.

In one embodiment, methods for observing a coincidence between the expression of 121P2A3 gene and 121P2A3 gene products (or perturbations in 121P2A3 gene and 121P2A3 gene products) and another factor associated with malignancy entails detecting the overexpression of 121P2A3 mRNA or protein in a tissue sample, detecting the overexpression of PSA mRNA or protein in a tissue sample (or PSCA or PSM expression), and observing a coincidence of 121P2A3 mRNA or protein and PSA mRNA or protein overexpression (or PSCA or PSM expression). In a specific embodiment, the expression of 121P2A3 and PSA mRNA in prostate tissue is examined, where the coincidence of 121P2A3 and PSA mRNA overexpression in the sample indicates the existence of prostate cancer, prostate cancer susceptibility or the emergence or status of a prostate tumor.

Methods for detecting and quantifying the expression of 121P2A3 mRNA or protein are described herein, and standard nucleic acid and protein detection and quantification technologies are well known in the art. Standard methods for the detection and quantification of 121P2A3 mRNA include *in situ* hybridization using labeled 121P2A3 riboprobes, Northern blot and related techniques using 121P2A3 polynucleotide probes, RT-PCR analysis using primers specific for 121P2A3, and other amplification type detection methods, such as, for example, branched DNA, SISBA, TMA and the like. In a specific embodiment, semi-quantitative RT-PCR is used to detect and quantify 121P2A3 mRNA expression. Any number of primers capable of amplifying 121P2A3 can be used for this purpose, including but not limited to the various primer sets specifically described herein. In a specific embodiment, polyclonal or monoclonal antibodies specifically reactive with the wild-type 121P2A3 protein can be used in an immunohistochemical assay ofbiopsied tissue.

## IX.) Identification of Molecules That Interact With 121P2A3

The 121P2A3 protein and nucleic acid sequences disclosed herein allow a skilled artisan to identify proteins, small molecules and other agents that interact with 121P2A3, as well as pathways activated by 121P2A3 via any one of a variety of art accepted protocols. For example, one can utilize one of the so-called interaction trap systems (also referred to as the "two-hybrid assay"). In such systems, molecules interact and reconstitute a transcription factor which directs expression of a reporter gene, whereupon the expression of the reporter gene is assayed. Other systems identify protein-protein interactions in *vivo* through reconstitution of a eukaryotic transcriptional activator, see, e.g., U.S. Patent Nos. 5,955,280 issued 21 September 1999, 5,925,523 issued 20 July 1999, 5,846,722 issued 8 December 1998 and 6,004,746 issued 21 December 1999. Algorithms are also available in the art for genome-based predictions of protein function (see, e.g., Marcotte, et al., Nature 402: 4 November 1999, 83-86).

Alternatively one can screen peptide libraries to identify molecules that interact with 121P2A3 protein sequences. In such methods, peptides that bind to 121 P2A3 are identified by screening libraries that encode a random or controlled collection of amino acids. Peptides encoded by the libraries are expressed as fusion proteins of bacteriophage coat proteins, the bacteriophage particles are then screened against the 121P2A3 protein(s).

Accordingly, peptides having a wide variety of uses, such as therapeutic, prognostic or diagnostic reagents, are thus identified without any prior information on the structure of the expected ligand or receptor molecule. Typical peptide libraries and screening methods that can be used to identify molecules that interact with 121R2A3 protein sequences are disclosed for example in U.S. Patent Nos. 5,723,286 issued 3 March 1998 and 5,733,731 issued 31 March 1998.

Alternatively, cell lines that express 121P2A3 are used to identify protein-protein interactions mediated by 121P2A3. Such interactions can be examined using immunoprecipitation techniques (see, e.g., Hamilton B.J., et al. Biochem Biophys. Res. Commun. 1999, 261:646-51). 121P2A3 protein can be immunoprecipitated from 121P2A3-expressing cell lines using anti-121P2A3 antibodies. Alternatively, antibodies against His-tag can be used in a cell line engineered to express fusions of 121P2A3 and a His-tag (vectors mentioned above). The immunoprecipitated complex can be examined for protein association by procedures such as Western blotting, $^{35}$S-methionine labeling of proteins, protein microsequencing, silver staining and two-dimensional gel electrophoresis.

Small molecules and ligands that interact with 121P2A3 can be identified through related embodiments of such screening assays. For example, small molecules can be identified that interfere with protein function, including molecules that interfere with 121P2A3's ability to mediate phosphorylation and de-phosphorylation, interaction with DNA or RNA molecules as an indication of regulation of cell cycles, second messenger signaling or tumorigenesis. Similarly, small molecules that modulate 121P2A3-related ion channel, protein pump, or cell communication functions are identified and used to treat patients that have a cancer that expresses 121P2A3 (see, e.g., Hille, B., Ionic Channels of Excitable Membranes 2nd Ed., Sinauer Assoc., Sunderland, MA, 1992). Moreover, ligands that regulate 121P2A3 function can be identified based on their ability to bind 121P2A3 and activate a reporter construct. Typical methods are discussed for example in U.S. Patent No. 5,928,868 issued 27 July 1999, and include methods for forming hybrid ligands in which at least one ligand is a small molecule. In an illustrative embodiment, cells engineered to express a fusion protein of 121P2A3 and a DNA-binding protein are used to co-express a fusion protein of a hybrid ligand/small molecule and a cDNA library transcriptional activator protein. The cells further contain a reporter gene, the expression of which is conditioned on the proximity of the first and second fusion proteins to each other, an event that occurs only if the hybrid ligand binds to target sites on both hybrid proteins. Those cells that express the reporter gene are selected and the unknown small molecule or the unknown ligand is identified. This method provides a means of identifying modulators which activate or inhibit 121P2A3.

An embodiment of this invention comprises a method of screening for a molecule that interacts with a 121P2A3 amino acid sequence shown in Figure 2 or Figure 3, comprising the steps of contacting a population of molecules with a 121P2A3 amino acid sequence, allowing the population of molecules and the 121P2A3 amino acid sequence to interact under conditions that facilitate an interaction, determining the presence of a molecule that interacts with the 121P2A3 amino acid sequence, and then separating molecules that do not interact with the 121P2A3 amino acid sequence from molecules that do. In a specific embodiment, the method further comprises purifying, characterizing and identifying a molecule that interacts with the 121P2A3 amino acid sequence. The identified molecule can be used to modulate a function performed by 121P2A3. In a preferred embodiment, the 121P2A3 amino acid sequence is contacted with a library of peptides.

## X.) Therapeutic Methods and Compositions

The identification of 121P2A3 as a protein that is normally expressed in a restricted set of tissues, but which is also expressed in prostate and other cancers, opens a number of therapeutic approaches to the treatment of such cancers. As contemplated herein, 121P2A3 functions as a transcription factor involved in activating tumor-promoting genes or repressing genes that block tumorigenesis.

Accordingly, therapeutic approaches that inhibit the activity of a 121P2A3 protein are useful for patients suffering from a cancer that expresses 121P2A3. These therapeutic approaches generally fall into two classes. One class comprises various methods for inhibiting the binding or association of a 121P2A3 protein with its binding partner or with other proteins. Another class comprises a variety of methods for inhibiting the transcription of a 121P2A3 gene or translation of 121P2A3 mRNA.

### X.A.) Anti-Cancer Vaccines

The invention provides cancer vaccines comprising a 121P2A3-related protein or 121P2A3-related nucleic acid. In view of the expression of 121P2A3, cancer vaccines prevent and/or treat 121P2A3-expressing cancers with minimal or no effects on non-target tissues. The use of a tumor antigen in a vaccine that generates humoral and/or cell-mediated immune responses as anti-cancer therapy is well known in the art and has been employed in prostate cancer using human PSMA and rodent PAP immunogens (Hodge et al., 1995, Int. J. Cancer 63:231-237; Fong et al., 1997, J. Immunol. 159:3113-3117).

Such methods can be readily practiced by employing a 121P2A3-related protein, or a 121P2A3-encoding nucleic acid molecule and recombinant vectors capable of expressing and presenting the 121P2A3 immunogen (which typically comprises a number of antibody or T cell epitopes). Skilled artisans understand that a wide variety of vaccine systems for delivery of immunoreactive epitopes are known in the art (see, e.g., Heryln et al., Ann Med 1999 Feb 31(1): 66-78; Maruyama et al., Cancer Immunol Immunother 2000 Jun 49(3):123-32) Briefly, such methods of generating an immune response (e.g. humoral and/or cell-mediated) in a mammal, comprise the steps of: exposing the mammal's immune system to an immunoreactive epitope (e.g. an epitope present in a 121P2A3 protein shown in Figure 3 or analog or homolog thereof) so that the mammal generates an immune response that is specific for that epitope (e.g. generates antibodies that specifically recognize that epitope). In a preferred method, a 121P2A3 immunogen contains a biological motif, see e.g., Tables V-XVIII and XXII-LI, or a peptide of a size range from 121P2A3 indicated in Figure 5, Figure 6, Figure 7, Figure 8, and Figure 9.

The entire 121P2A3 protein, immunogenic regions or epitopes thereof can be combined and delivered by various means. Such vaccine compositions can include, for example, lipopeptides (*e.g.,* Vitiello, A. et al., J. Clin. Invest. 95:341, 1995), peptide compositions encapsulated in poly(DL-lactide-co-glycolide) ("PLG") microspheres (*see, e.g.,* Eldridge, et al., Molec. Immunol. 28:287-294, 1991: Alonso et al., Vaccine 12:299-306, 1994; Jones et al., Vaccine 13: 675-681, 1995), peptide compositions contained in immune stimulating complexes (ISCOMS) *(see, e.g.,* Takahashi et al., Nature 344:873-875, 1990; Hu et al., Clin Exp Immunol. 113:235-243, 1998), multiple antigen peptide systems (MAPs) (*see e.g.,* Tam, J. P., Proc. Natl. Acad. Sci. U.S.A. 85:5409-5413, 1988; Tam, J.P., J. Immunol. Methods 196: 17-32, 1996), peptides formulated as multivalent peptides; peptides for use in ballistic delivery systems, typically crystallized peptides, viral delivery vectors (Perkus, M. E. et al., In: Concepts in vaccine development, Kaufmann, S. H. E., ed., p. 379, 1996; Chakrabarti, S. et al., Nature 320:535, 1986; Hu, S. L. et al., Nature 320:537, 1986; Kieny, M.-P. et al., AIDS Bio/Technology 4:790, 1986; Top, F. H. et al., J. Infect. Dis. 124:148, 1971; Chanda, P. K. et al., Virology 175: 535, 1990), particles of viral or synthetic origin (e.g., Kofler, N. et al., J. Immunol. Methods. 192:25,1996; Eldridge, J. H. et al., Sem. Hematol. 30:16, 1993; Falo, L. D., Jr. et al., Nature Med. 7:649, 1995), adjuvants (Warren, H. S., Vogel, F. R., and Chedid, L. A. Annu. Rev. Immunol. 4:369, 1986; Gupta, R. K. et al., Vaccine 11:293, 1993), liposomes (Reddy, R. et al., J. Immunol. 148:1585, 1992; Rock, K. L., Immunol. Today 17:131, 1996), or, naked or particle absorbed cDNA (Ulmer, J. B. et al., Science 259:1745, 1993; Robinson, H. L., Hunt, L. A., and Webster, R. G., Vaccine 11:957, 1993; Shiver, J. W. et al., In: Concepts in vaccine development, Kaufmann, S. H. E., ed., p. 423, 1996; Cease, K. B., and Berzofsky, J. A., Annu. Rev. Immunol. 12:923, 1994 and Eldridge, J. H. et al., Sem. Hematol. 30:16, 1993). Toxin-targeted delivery technologies, also known as receptor mediated targeting, such as those of Avant Immunotherapeutics, Inc. (Needham, Massachusetts) may also be used.

In patients with 121P2A3-associated cancer, the vaccine compositions of the invention can also be used in conjunction with other treatments used for cancer, *e.g.*, surgery, chemotherapy, drug therapies, radiation therapies, etc. including use in combination with immune adjuvants such as IL-2, IL-12, GM-CSF, and the like.

Cellular Vaccines:

CTL epitopes can be determined using specific algorithms to identify peptides within 121P2A3 protein that bind corresponding HLA alleles (see e.g., Table IV; Epimer™ and Epimatrix™, Brown University (LJRL www.brown.edu/Research/TB-HIV_Lab/epimatrix/epimatrix.html); and, BIMAS, (URL bimas.dcrt.nih.gov/; SYFPEITHI at URL syfpeithi.bmi-heidelberg.com/). In a preferred embodiment, a 121P2A3 immunogen contains one or more amino acid sequences identified using techniques well known in the art, such as the sequences shown in Tables V-XVIII and XXII-LI or a peptide of 8, 9, 10 or 11 amino acids specified by an HLA Class I motif/supermotif (e.g., Table IV (A), Table IV (D), or Table IV (E)) and/or a peptide of at least 9 amino acids that comprises an HLA Class II motif/supermotif (e.g., Table IV (B) or Table IV (C)). As is appreciated in the art, the HLA Class I binding groove is essentially closed ended so that peptides of only a particular size range can fit into the groove and be bound, generally HLA Class I epitopes are 8, 9, 10, or 11 amino acids long. In contrast, the HLA Class II binding groove is essentially open ended; therefore a peptide of about 9 or more amino acids can be bound by an HLA Class II molecule. Due to the binding groove differences between HLA Class I and II, HLA Class I motifs are length specific, i.e., position two of a Class I motif is the second amino acid in an amino to carboxyl direction of the peptide. The amino acid positions in a Class II motif are relative only to each other, not the overall peptide, i.e., additional amino acids can be attached to the amino and/or carboxyl termini of a motif-bearing sequence. HLA Class II epitopes are often 9, 10, 11, 12, 13, 14, 15, 16, 17, 18, 19, 20, 21, 22, 23, 24, or 25 amino acids long, or longer than 25 amino acids.

Antibody-based Vaccines

A wide variety of methods for generating an immune response in a mammal are known in the art (for example as the first step in the generation of hybridomas). Methods of generating an immune response in a mammal comprise exposing the mammal's immune system to an immunogenic epitope on a protein (e.g. a 121P2A3 protein) so that an immune response is generated. A typical embodiment consists of a method for generating an immune response to 121P2A3 in a host, by contacting the host with a sufficient amount of at least one 121P2A3 B cell or cytotoxic T-cell epitope or analog thereof; and at least one periodic interval thereafter re-contacting the host with the 121P2A3 B cell or cytotoxic T-cell epitope or analog thereof. A specific embodiment consists of a method of generating an immune response against a 121P2A3-related protein or a man-made multiepitopic peptide comprising: administering 121P2A3 immunogen (e.g. a 121P2A3 protein or a peptide fragment thereof, a 121P2A3 fusion protein or analog etc.) in a vaccine preparation to a human or another mammal. Typically, such vaccine preparations further contain a suitable adjuvant (see, e.g., U.S. Patent No. 6,146,635) or a universal helper epitope such as a PADRE™ peptide (Epimmune Inc., San Diego, CA; see, e.g., Alexander et al., J. Immunol. 2000 164(3); 164(3): 1625-1633; Alexander et al., Immunity 1994 1(9): 751-761 and Alexander et al., Immunol. Res. 1998 18(2): 79-92). An alternative method comprises generating an immune response in an individual against a 121P2A3 immunogen by: administering in *vivo* to muscle or skin of the individual's body a DNA molecule that comprises a DNA sequence that encodes a 121P2A3 immunogen, the DNA sequence operatively linked to regulatory sequences which control the expression of the DNA sequence; wherein the DNA molecule is taken up by cells, the DNA sequence is expressed in the cells and an immune response is generated against the immunogen (see, e.g., U.S. Patent No. 5,962,428). Optionally a genetic vaccine facilitator such as anionic lipids; saponins; lectins; estrogenic compounds; hydroxylated lower alkyls; dimethyl sulfoxide; and urea is also administered. In addition, an antiidiotypic antibody can be administered that mimics 121P2A3, in order to generate a response to the target antigen.

Nucleic Acid Vaccines:

Vaccine compositions of the invention include nucleic acid-mediated modalities. DNA or RNA that encode protein(s) of the invention can be administered to a patient. Genetic immunization methods can be employed to generate prophylactic or therapeutic humoral and cellular immune responses directed against cancer cells expressing 121P2A3. Constructs comprising DNA encoding a 121P2A3-related protein/immunogen and appropriate regulatory sequences can be injected directly into muscle or skin of an individual, such that the cells of the muscle or skin take-up the construct and express the encoded 121P2A3 protein/immunogen. Alternatively, a vaccine comprises a 121P2A3-related protein. Expression of the 121P2A3-related protein immunogen results in the generation of prophylactic or therapeutic humoral and cellular immunity against cells that bear a 121P2A3 protein. Various prophylactic and therapeutic genetic immunization techniques known in the art can be used (for review, see information and references published at Internet address www.genweb.com). Nucleic acid-based delivery is described, for instance, in Wolff et. al., Science 247:1465 (1990) as well as U.S. Patent Nos. 5,580,859; 5,589,466; 5,804,566; 5,739,118; 5,736,524; 5,679,647; WO 98/04720. Examples of DNA-based delivery technologies include "naked DNA", facilitated (bupivicaine, polymers, peptide-mediated) delivery, cationic lipid complexes, and particle-mediated ("gene gun") or pressure-mediated delivery *(see, e.g.,* U.S. Patent No. 5,922,687).

For therapeutic or prophylactic immunization purposes, proteins of the invention can be expressed via viral or bacterial vectors. Various viral gene delivery systems that can be used in the practice of the invention include, but are not limited to, vaccinia, fowlpox, canarypox, adenovirus, influenza, poliovirus, adeno-associated virus, lentivirus, and sindbis virus (see, e.g., Restifo,1996, Curr. Opin. Immunol. 8:658-663; Tsang et al. J. Natl. Cancer Inst. 87:982-990 (1995)). Non-viral delivery systems can also be employed by introducing naked DNA encoding a 121P2A3-related protein into the patient (e.g., intramuscularly or intradermally) to induce an anti-tumor response.

Vaccinia virus is used, for example, as a vector to express nucleotide sequences that encode the peptides of the invention. Upon introduction into a host, the recombinant vaccinia virus expresses the protein immunogenic peptide, and thereby elicits a host immune response. Vaccinia vectors and methods useful in immunization protocols are described in, *e.g.,* U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the peptides of the invention, *e.g.* adeno and adeno-associated virus vectors, retroviral vectors, *Salmonella typhi* vectors, detoxified anthrax toxin vectors, and the like, will be apparent to those skilled in the art from the description herein.

Thus, gene delivery systems are used to deliver a 121P2A3-related nucleic acid molecule. In one embodiment, the full-length human 121P2A3 cDNA is employed. In another embodiment, 121P2A3 nucleic acid molecules encoding specific cytotoxic T lymphocyte (CTL) and/or antibody epitopes are employed.

Ex Vivo Vaccines

Various *ex vivo* strategies can also be employed to generate an immune response. One approach involves the use of antigen presenting cells (APCs) such as dendritic cells (DC) to present 121P2A3 antigen to a patient's immune system Dendritic cells express MHC class I and II molecules, B7 co-stimulator, and IL-12, and are thus highly specialized antigen presenting cells. In prostate cancer, autologous dendritic cells pulsed with peptides of the prostate-specific membrane antigen (PSMA) are being used in a Phase I clinical trial to stimulate prostate cancer patients' immune systems (Tjoa et al., 1996, Prostate 28:65-69; Murphy et al., 1996, Prostate 29:371-380). Thus, dendritic cells can be used to present 121P2A3 peptides to T cells in the context of MHC class I or II molecules. In one embodiment, autologous dendritic cells are pulsed with 121P2A3 peptides capable of binding to MHC class I and/or class II molecules. In another embodiment, dendritic cells are pulsed with the complete 121P2A3 protein. Yet another embodiment involves engineering the overexpression of a 121P2A3 gene in dendritic cells using various implementing vectors known in the art, such as adenovirus (Arthur et al., 1997, Cancer Gene Ther. 4:17-25), retrovirus (Henderson et al., 1996, Cancer Res. 56: 3763-3770), lentivirus, adeno-associated virus, DNA transfection (Ribas et al., 1997, Cancer Res. 57:2865-2869), or tumor-derived RNA transfection (Ashley et al., 1997, J. Exp. Med. 186:1177-1182). Cells that express 121P2A3 can also be engineered to express immune modulators, such as GM-CSF, and used as immunizing agents.

**X.B.) 121P2A3 as a Target for Antibody-based Therapy**

121P2A3 is an attractive target for antibody-based therapeutic strategies. A number of antibody strategies are known in the art for targeting both extracellular and intracellular molecules (see, e.g., complement and ADCC mediated killing as well as the use of intrabodies). Because 121P2A3 is expressed by cancer cells of various lineages relative to corresponding normal cells, systemic administration of 121P2A3-immunoreactive compositions are prepared that exhibit excellent sensitivity without toxic, non-specific and/or non-target effects caused by binding of the immunoreactive composition to non-target organs and tissues. Antibodies specifically reactive with domains of 121P2A3 are useful to treat 121P2A3-expressing cancers systemically, either as conjugates with a toxin or therapeutic agent, or as naked antibodies capable of inhibiting cell proliferation or function.

121P2A3 antibodies can be introduced into a patient such that the antibody binds to 121P2A3 and modulates a function, such as an interaction with a binding partner, and consequently mediates destruction of the tumor cells and/or inhibits the growth of the tumor cells. Mechanisms by which such antibodies exert a therapeutic effect can include complement-mediated cytolysis, antibody-dependent cellular cytotoxicity, modulation of the physiological function of 121P2A3, inhibition of ligand binding or signal transduction pathways, modulation of tumor cell differentiation, alteration of tumor angiogenesis factor profiles, and/or apoptosis.

Those skilled in the art understand that antibodies can be used to specifically target and bind immunogenic molecules such as an immunogenic region of a 121P2A3 sequence shown in Figure 2 or Figure 3. In addition, skilled artisans understand that it is routine to conjugate antibodies to cytotoxic agents (see, e.g., Slevers et al. Blood 93:11 3678-3684 (June 1, 1999)). When cytotoxic and/or therapeutic agents are delivered directly to cells, such as by conjugating them to antibodies specific for a molecule expressed by that cell (e.g. 121P2A3), the cytotoxic agent will exert its known biological effect (i.e. cytotoxicity) on those cells.

A wide variety of compositions and methods for using antibody-cytotoxic agent conjugates to kill cells are known in the art. In the context of cancers, typical methods entail administering to an animal having a tumor a biologically effective amount of a conjugate comprising a selected cytotoxic and/or therapeutic agent linked to a targeting agent (e.g. an anti-121P2A3 antibody) that binds to a marker (e.g. 121P2A3) expressed, accessible to binding or localized on the cell surfaces. A typical embodiment is a method of delivering a cytotoxic and/or therapeutic agent to a cell expressing 121P2A3, comprising conjugating the cytotoxic agent to an antibody that immunospecifically binds to a 121P2A3 epitope, and, exposing the cell to the antibody-agent conjugate. Another illustrative embodiment is a method of treating an individual suspected of suffering from metastasized cancer, comprising a step of administering parenterally to said individual a pharmaceutical composition comprising a therapeutically effective amount of an antibody conjugated to a cytotoxic and/or therapeutic agent.

Cancer immunotherapy using anti-121P2A3 antibodies can be done in accordance with various approaches that have been successfully employed in the treatment of other types of cancer, including but not limited to colon cancer (Arlen et al., 1998, Crit. Rev. Immunol. 18:133-138), multiple myeloma (Ozaki et al., 1997, Blood 90:3179-3186, Tsunenari et al., 1997, Blood 90:2437-2444), gastric cancer (Kasprzyk et al., 1992, Cancer Res. 52:2771-2776), B-cell lymphoma (Funakoshi et al., 1996, J. Immunother. Emphasis Tumor Immunol. 19:93-101), leukemia (Zhong et al., 1996, Leuk. Res. 20:581-589), colorectal cancer (Moun et al., 1994, Cancer Res. 54:6160-6166; Velders et al., 1995, Cancer Res. 55:4398-4403), and breast cancer , (Shepard et al., 1991, J. Clin. Immunol. 11:117-127). Some therapeutic approaches involve conjugation of naked antibody to a toxin or radioisotope, such as the conjugation of $Y^{91}$ or $I^{131}$ to anti-CD20 antibodies (e.g., Zevalin™, IDEC Pharmaceuticals Corp. or Bexxar™, Coulter Pharmaceuticals), while others involve coadministration of antibodies and other therapeutic agents, such as Herceptin™ (trastuzumab) with paclitaxel (Genentech, Inc.). The antibodies can be conjugated to a therapeutic agent. To treat prostate cancer, for example, 121P2A3 antibodies can be administered in conjunction with radiation, chemotherapy or hormone ablation. Also, antibodies can be conjugated to a toxin such as calicheamicin (e.g., Mylotarg™, Wyeth-Ayerst, Madison, NJ, a recombinant humanized $IgG_4$ kappa antibody conjugated to antitumor antibiotic calicheamicin) or a maytansinoid (e.g., taxane-based Tumor-Activated Prodrug, TAP, platform, ImmunoGen, Cambridge, MA, also see e.g., US Patent 5,416,064).

Although 121P2A3 antibody therapy is useful for all stages of cancer, antibody therapy can be particularly appropriate in advanced or metastatic cancers. Treatment with the antibody therapy of the invention is indicated for patients who have received one or more rounds of chemotherapy. Alternatively, antibody therapy of the invention is combined with a chemotherapeutic or radiation regimen for patients who have not received chemotherapeutic treatment. Additionally, antibody therapy can enable the use of reduced dosages of concomitant chemotherapy, particularly for patients who do not tolerate the toxicity of the chemotherapeutic agent very well. Fan et al. (Cancer Res. 53:4637-4642, 1993), Prewett et al. (International J. of Onco. 9:217-224, 1996), and Hancock et al. (Cancer Res. 51:4575-4580, 1991) describe the use of various antibodies together with chemotherapeutic agents.

Although 121P2A3 antibody therapy is useful for all stages of cancer, antibody therapy can be particularly appropriate in advanced or metastatic cancers. Treatment with the antibody therapy of the invention is indicated for patients who have received one or more rounds of chemotherapy. Alternatively, antibody therapy of the invention is combined with a chemotherapeutic or radiation regimen for patients who have not received chemotherapeutic treatment. Additionally, antibody therapy can enable the use of reduced dosages of concomitant chemotherapy, particularly for patients who do not tolerate the toxicity of the chemotherapeutic agent very well.

Cancer patients can be evaluated for the presence and level of 121P2A3 expression, preferably using immunohistochemical assessments of tumor tissue, quantitative 121P2A3 imaging, or other techniques that reliably indicate the presence and degree of 121P2A3 expression. Immunohistochemical analysis of tumor biopsies or surgical specimens is preferred for this purpose. Methods for immunohistochemical analysis of tumor tissues are well known in the art.

Anti-121P2A3 monoclonal antibodies that treat prostate and other cancers include those that initiate a potent immune response against the tumor or those that are directly cytotoxic. In this regard, anti-121P2A3 monoclonal antibodies (mAbs) can elicit tumor cell lysis by either complement-mediated or antibody-dependent cell cytotoxicity (ADCC) mechanisms, both of which require an intact Fc portion of the immunoglobulin molecule for interaction with effector cell Fc receptor sites on complement proteins. In addition, anti-121P2A3 mAbs that exert a direct biological effect on tumor growth are useful to treat cancers that express 121P2A3. Mechanisms by which directly cytotoxic mAbs act include: inhibition of cell growth, modulation of cellular differentiation, modulation of tumor angiogenesis factor profiles, and the induction of apoptosis. The mechanism(s) by which a particular anti-121P2A3 mAb exerts an anti-tumor effect is evaluated using any number of *in vitro* assays that evaluate cell death such as ADCC, ADMMC, complement-mediated cell lysis, and so forth, as is generally known in the art.

In some patients, the use of murine or other non-human monoclonal antibodies, or human/mouse chimeric mAbs can induce moderate to strong immune responses against the non-human antibody. This can result in clearance of the antibody from circulation and reduced efficacy. In the most severe cases, such an immune response can lead to the extensive formation of immune complexes which, potentially, can cause renal failure. Accordingly, preferred monoclonal antibodies used in the therapeutic methods of the invention are those that are either fully human or humanized and that bind specifically to the target 121P2A3 antigen with high affinity but exhibit low or no antigenicity in the patient.

Therapeutic methods of the invention contemplate the administration of single anti-121P2A3 mAbs as well as combinations, or cocktails, of different mAbs. Such mAb cocktails can have certain advantages inasmuch as they contain mAbs that target different epitopes, exploit different effector mechanisms or combine directly cytotoxic mAbs with mAbs that rely on immune effector functionality. Such mAbs in combination can exhibit synergistic therapeutic effects. In addition, anti-121P2A3 mAbs can be administered concomitantly with other therapeutic modalities, including but not limited to various chemotherapeutic agents, androgen-blockers, immune modulators (e.g., IL-2, GM-CSF), surgery or radiation. The anti-121P2A3 mAbs are administered in their "naked" or unconjugated form, or can have a therapeutic agent(s) conjugated to them.

Anti-121P2A3 antibody formulations are administered via any route capable of delivering the antibodies to a tumor cell. Routes of administration include, but are not limited to, intravenous, intraperitoneal, intramuscular, intratumor, intradermal, and the like. Treatment generally involves repeated administration of the anti-121P2A3 antibody preparation, via an acceptable route of administration such as intravenous injection (IV), typically at a dose in the range of about 0.1, .2, .3, .4, .5, .6, .7, .8, .9., 1, 2, 3, 4, 5, 6, 7, 8, 9, 10, 15, 20, or 25 mg/kg body weight. In general, doses in the range of 10-1000 mg mAb per week are effective and well tolerated.

Based on clinical experience with the Herceptin™ mAb in the treatment of metastatic breast cancer, an initial loading dose of approximately 4 mg/kg patient body weight IV, followed by weekly doses of about 2 mg/kg IV of the anti-121P2A3 mAb preparation represents an acceptable dosing regimen. Preferably, the initial loading dose is administered as a 90 minute or longer infusion. The periodic maintenance dose is administered as a 30 minute or longer infusion, provided the initial dose was well tolerated. As appreciated by those of skill in the art, various factors can influence the ideal dose regimen in a particular case. Such factors include, for example, the binding affinity and half life of the Ab or mAbs used, the degree of 121P2A3 expression in the patient, the extent of circulating shed 121P2A3 antigen, the desired steady-state antibody concentration level, frequency of treatment, and the influence of chemotherapeutic or other agents used in combination with the treatment method of the invention, as well as the health status of a particular patient.

Optionally, patients should be evaluated for the levels of 121P2A3 in a given sample (e.g. the levels of circulating 121P2A3 antigen and/or 121P2A3 expressing cells) in order to assist in the determination of the most effective dosing regimen, etc. Such evaluations are also used for monitoring purposes throughout therapy, and are useful to gauge therapeutic success in combination with the evaluation of other parameters (for example, urine cytology and/or ImmunoCyt levels in bladder cancer therapy, or by analogy, serum PSA levels in prostate cancer therapy).

Anti-idiotypic anti-121P2A3 antibodies can also be used in anti-cancer therapy as a vaccine for inducing an immune response to cells expressing a 121P2A3-related protein. In particular, the generation of anti-idiotypic antibodies is well known in the art; this methodology can readily be adapted to generate anti-idiotypic anti-121P2A3 antibodies that mimic an epitope on a 121P2A3-related protein (see, for example, Wagner et al., 1997, Hybridoma 16: 33-40; Foon et al., 1995, J. Clin. Invest. 96:334-342; Herlyn et al., 1996, Cancer Immunol. Immunother. 43:65-76). Such an anti-idiotypic antibody can be used in cancer vaccine strategies.

**X.C.) 121P2A3 as a Target for Cellular Immune Responses**

Vaccines and methods of preparing vaccines that contain an immunogenically effective amount of one or more HLA-binding peptides as described herein are further embodiments of the invention. Furthermore, vaccines in accordance with the invention encompass compositions of one or more of the claimed peptides. A peptide can be present in a vaccine individually. Alternatively, the peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism or tumor-related peptide targeted for an immune response. The composition can be a naturally occurring region of an antigen or can be prepared, *e.g.*, recombinantly or by chemical synthesis.

Carriers that can be used with vaccines of the invention are well known in the art, and include, *e.g.*, thyroglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (*i.e.*, acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, as disclosed herein, CTL responses can be primed by conjugating peptides of the invention to lipids, such as tripalmitoyl-S-glycerylcysteinlyseryl- serine ($P_3CSS$). Moreover, an adjuvant such as a synthetic cytosine-phosphorothiolated-guanine-containing (CpG) oligonucleotides has been found to increase CTL responses 10- to 100-fold. (see, e.g. Davila and Celis, J. Immunol. 165:539-547 (2000))

Upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs and/or HTLs specific for the desired antigen. Consequently, the host becomes at least partially immune to later development of cells that express or overexpress 121P2A3 antigen, or derives at least some therapeutic benefit when the antigen was tumor-associated.

In some embodiments, it may be desirable to combine the class I peptide components with components that induce or facilitate neutralizing antibody and or helper T cell responses directed to the target antigen. A preferred embodiment of such a composition comprises class I and class II epitopes in accordance with the invention. An alternative embodiment of such a composition comprises a class I and/or class II epitope in accordance with the invention, along with a cross reactive HTL epitope such as PADRE™ (Epimmune, San Diego, CA) molecule (described *e.g.*, in U.S. Patent Number 5,736,142).

A vaccine of the invention can also include antigen-presenting cells (APC), such as dendritic cells (DC), as a vehicle to present peptides of the invention. Vaccine compositions can be created *in vitro,* following dendritic cell mobilization and harvesting, whereby loading of dendritic cells occurs *in vitro.* For example, dendritic cells are transfected, *e.g.,* with a minigene in accordance with the invention, or are pulsed with peptides. The dendritic cell can then be administered to a patient to elicit immune responses *in vivo.* Vaccine compositions, either DNA- or peptide-based, can also be administered *in vivo* in combination with dendritic cell mobilization whereby loading of dendritic cells occurs *in vivo.*

Preferably, the following principles are utilized when selecting an array of epitopes for inclusion in a polyepitopic composition for use in a vaccine, or for selecting discrete epitopes to be included in a vaccine and/or to be encoded by nucleic acids such as a minigene. It is preferred that each of the following principles be balanced in order to make the selection. The multiple epitopes to be incorporated in a given vaccine composition may be, but need not be, contiguous in sequence in the native antigen from which the epitopes are derived.

1.) Epitopes are selected which, upon administration, mimic immune responses that have been observed to be correlated with tumor clearance. For HLA Class I this includes 3-4 epitopes that come from at least one tumor associated antigen (TAA). For HLA Class II a similar rationale is employed; again 3-4 epitopes are selected from at least one TAA (*see, e.g.,* Rosenberg et al., Science 278:1447-1450). Epitopes from one TAA may be used in combination with epitopes from one or more additional TAAs to produce a vaccine that targets tumors with varying expression patterns of frequently-expressed TAAs.

2.) Epitopes are selected that have the requisite binding affinity established to be correlated with immunogenicity: for HLA Class I an $IC_{50}$ of 500 nM or less, often 200 nM or less; and for Class II an $IC_{50}$ of 1000 nM or less.

3.) Sufficient supermotif bearing-peptides, or a sufficient array of allele-specific motif-bearing peptides, are selected to give broad population coverage. For example, it is preferable to have at least 80% population coverage. A Monte Carlo analysis, a statistical evaluation known in the art, can be employed to assess the breadth, or redundancy of, population coverage.

4.) When selecting epitopes from cancer-related antigens it is often useful to select analogs because the patient may have developed tolerance to the native epitope.

5.) Of particular relevance are epitopes referred to as "nested epitopes." Nested epitopes occur where at least two epitopes overlap in a given peptide sequence. A nested peptide sequence can comprise B cell, HLA class I and/or HLA class II epitopes. When providing nested epitopes, a general objective is to provide the greatest number of epitopes per sequence. Thus, an aspect is to avoid providing a peptide that is any longer than the amino terminus of the amino terminal epitope and the carboxyl terminus of the carboxyl terminal epitope in the peptide. When providing a multi-epitopic sequence, such as a sequence comprising nested epitopes, it is generally important to screen the sequence in order to insure that it does not have pathological or other deleterious biological properties.
6.) If a polyepitopic protein is created, or when creating a minigene, an objective is to generate the smallest peptide that encompasses the epitopes of interest. This principle is similar, if not the same as that employed when selecting a peptide comprising nested epitopes. However, with an artificial polyepitopic peptide, the size minimization objective is balanced against the need to integrate any spacer sequences between epitopes in the polyepitopic protein. Spacer amino acid residues can, for example, be introduced to avoid junctional epitopes (an epitope recognized by the immune system, not present in the target antigen, and only created by the man-made juxtaposition of epitopes), or to facilitate cleavage between epitopes and thereby enhance epitope presentation. Junctional epitopes are generally to be avoided because the recipient may generate an immune response to that non-native epitope. Of particular concern is a junctional epitope that is a "dominant epitope." A dominant epitope may lead to such a zealous response that immune responses to other epitopes are diminished or suppressed.
7.) Where the sequences of multiple variants of the same target protein are present, potential peptide epitopes can also be selected on the basis of their conservancy. For example, a criterion for conservancy may define that the entire sequence of an HLA class I binding peptide or the entire 9-mer core of a class II binding peptide be conserved in a designated percentage of the sequences evaluated for a specific protein antigen.

**X.C.1. Minigene Vaccines**

A number of different approaches are available which allow simultaneous delivery of multiple epitopes. Nucleic acids encoding the peptides of the invention are a particularly useful embodiment of the invention. Epitopes for inclusion in a minigene are preferably selected according to the guidelines set forth in the previous section. A preferred means of administering nucleic acids encoding the peptides of the invention uses minigene constructs encoding a peptide comprising one or multiple epitopes of the invention.

The use of multi-epitope minigenes is described below and in, Ishioka et al., J. Immunol. 162:3915-3925, 1999; An, L. and Whitton, J. L., J. Virol. 71:2292, 1997; Thomson, S. A. et al., J. Immunol. 157:822, 1996; Whitton, J. L. et al., J. Virol. 67:348, 1993; Hanke, R. et al., Vaccine 16:426, 1998. For example, a multi-epitope DNA plasmid encoding supermotif- and/or motif-bearing epitopes derived 121P2A3, the PADRE® universal helper T cell epitope or multiple HTL epitopes from 121P2A3, (see e.g., Tables V-XVIII and XXII to LI), and an endoplasmic reticulum-translocating signal sequence can be engineered. A vaccine may also comprise epitopes that are derived from other TAAs.

The immunogenicity of a multi-epitopic minigene can be confirmed in transgenic mice to evaluate the magnitude of CTL induction responses against the epitopes tested. Further, the immunogenicity of DNA-encoded epitopes *in vivo* can be correlated with the *in vitro* responses of specific CTL lines against target cells transfected with the DNA plasmid. Thus, these experiments can show that the minigene serves to both: 1.) generate a CTL response and 2.) that the induced CTLs recognized cells expressing the encoded epitopes.

For example, to create a DNA sequence encoding the selected epitopes (minigene) for expression in human cells, the amino acid sequences of the epitopes may be reverse translated. A human codon usage table can be used to guide the codon choice for each amino acid. These epitope-encoding DNA sequences may be directly adjoined, so that when translated, a continuous polypeptide sequence is created. To optimize expression and/or immunogenicity, additional elements can be incorporated into the minigene design. Examples of amino acid sequences that can be reverse translated and included in the minigene sequence include: HLA class I epitopes, HLA class II epitopes, antibody epitopes, a ubiquitination signal sequence, and/or an endoplasmic reticulum targeting signal. In addition, HLA presentation of CTL and HTL epitopes may be improved by including synthetic (*e.g.* poly-alanine) or naturally-occurring flanking sequences adjacent to the CTL or HTL epitopes; these larger peptides comprising the epitope(s) are within the scope of the invention.

The minigene sequence may be converted to DNA by assembling oligonucleotides that encode the plus and minus strands of the minigene. Overlapping oligonucleotides (30-100 bases long) may be synthesized, phosphorylated, purified and annealed under appropriate conditions using well known techniques. The ends of the oligonucleotides can be joined, for example, using T4 DNA ligase. This synthetic minigene, encoding the epitope polypeptide, can then be cloned into a desired expression vector.

Standard regulatory sequences well known to those of skill in the art are preferably included in the vector to ensure expression in the target cells. Several vector elements are desirable: a promoter with a downstream cloning site for minigene insertion; a polyadenylation signal for efficient transcription termination; an *E. coli* origin of replication; and an *E. coli* selectable marker (*e.g.* ampicillin or kanamycin resistance). Numerous promoters can be used for this purpose, *e.g.*, the human cytomegalovirus (hCMV) promoter. See, *e.g.*, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences and sequences for replication in mammalian cells may also be considered for increasing minigene expression.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

In addition, immunostimulatory sequences (ISSs or CpGs) appear to play a role in the immunogenicity of DNA vaccines. These sequences may be included in the vector, outside the minigene coding sequence, if desired to enhance immunogenicity.

In some embodiments, a bi-cistronic expression vector which allows production of both the minigene-encoded epitopes and a second protein (included to enhance or decrease immunogenicity) can be used. Examples of proteins or polypeptides that could beneficially enhance the immune response if co-expressed include cytokines (*e.g*., IL-2, IL-12, GM-CSF), cytokine-inducing molecules (*e.g.*, LeIF), costimulatory molecules, or for HTL responses, pan-DR binding proteins (PADRE™, Epimmune, San Diego, CA). Helper (HTL) epitopes can be joined to intracellular targeting signals and expressed separately from expressed CTL epitopes; this allows direction of the HTL epitopes to a cell compartment different than that of the CTL epitopes. If required, this could facilitate more efficient entry of HTL epitopes into the HLA class II pathway, thereby improving HTL induction. In contrast to HTL or CTL induction, specifically decreasing the immune response by co-expression of immunosuppressive molecules (*e.g.* TGF-β) may be beneficial in certain diseases.

Therapeutic quantities of plasmid DNA can be produced for example, by fermentation in E. *coli,* followed by purification. Aliquots from the working cell bank are used to inoculate growth medium, and grown to saturation in shaker flasks or a bioreactor according to well-known techniques. Plasmid DNA can be purified using standard bioseparation technologies such as solid phase anion-exchange resins supplied by QIAGEN, Inc. (Valencia, California). If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids, glycolipids, and fusogenic liposomes can also be used in the formulation (see, *e.g.*, as described by WO 93/24640; Mannino & Gould-Fogerite, BioTechniques 6(7): 682 (1988); U.S. Pat No. 5,279,833; WO 91/06309; and Felgner, et al., Proc. Nat'l Acad. Sci. USA 84:7413 (1987). In addition, peptides and compounds referred to collectively as protective, interactive, non-condensing compounds (PINC) could also be complexed to purified plasmid DNA to influence variables such as stability, intramuscular dispersion, or trafficking to specific organs or cell types.

Target cell sensitization can be used as a functional assay for expression and HLA class I presentation of minigene-encoded CTL epitopes. For example, the plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 ($^{51}Cr$) labeled and used as target cells for epitope-specific CTL lines; cytolysis, detected by $^{51}Cr$ release, indicates both production of, and HLA presentation of, minigene-encoded CTL epitopes. Expression of HTL epitopes may be evaluated in an analogous manner using assays to assess HTL activity.

*In vivo* immunogenicity is a second approach for functional testing of minigene DNA formulations. Transgenic mice expressing appropriate human HLA proteins are immunized with the DNA product The dose and route of administration are formulation dependent (*e.g.,* IM for DNA in PBS, intraperitoneal (i.p.) for lipid-complexed DNA). Twenty-one days after immunization, splenocytes are harvested and restimulated for one week in the presence of peptides encoding each epitope being tested. Thereafter, for CTL effector cells, assays are conducted for cytolysis of peptide-loaded, $^{51}Cr$-labeled target cells using standard techniques. Lysis of target cells that were sensitized by HLA loaded with peptide epitopes, corresponding to minigene-encoded epitopes, demonstrates DNA vaccine function for in *vivo* induction of CTLs. Immunogenicity of HTL epitopes is confirmed in transgenic mice in an analogous manner.

Alternatively, the nucleic acids can be administered using ballistic delivery as described, for instance, in U.S. Patent No. 5,204,253. Using this technique, particles comprised solely of DNA are administered. In a further alternative embodiment, DNA can be adhered to particles, such as gold particles.

Minigenes can also be delivered using other bacterial or viral delivery systems well known in the art, *e.g.,* an expression construct encoding epitopes of the invention can be incorporated into a viral vector such as vaccinia.

### X.C.2. Combinations of CTL Peptides with Helper Peptides

Vaccine compositions comprising CTL peptides of the invention can be modified, e.g., analoged, to provide desired attributes, such as improved serum half life, broadened population coverage or enhanced immunogenicity.

For instance, the ability of a peptide to induce CTL activity can be enhanced by linking the peptide to a sequence which contains at least one epitope that is capable of inducing a T helper cell response. Although a CTL peptide can be directly linked to a T helper peptide, often CTL epitope/HTL epitope conjugates are linked by a spacer molecule. The spacer is typically comprised of relatively small, neutral molecules, such as amino acids or amino acid mimetics, which are substantially uncharged under physiological conditions. The spacers are typically selected from, *e.g.*, Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues and sometimes 10 or more residues. The CTL peptide epitope can be linked to the T helper peptide epitope either directly or via a spacer either at the amino or carboxy terminus of the CTL peptide. The amino terminus of either the immunogenic peptide or the T helper peptide may be acylated.

In certain embodiments, the T helper peptide is one that is recognized by T helper cells present in a majority of a genetically diverse population. This can be accomplished by selecting peptides that bind to many, most, or all of the HLA class II molecules. Examples of such amino acid bind many HLA Class II molecules include sequences from antigens such as tetanus toxoid at positions 830-843 (QYIKANSKFIGITE; SEQ ID NO: __________________), *Plasmodium falciparum* circumsporozoite (CS) protein at positions 378-398 (DIEKICIAKMEKASSVFNWNS; SEQ ID NO: __________________), and *Streptococcus* 18kD protein at positions 116-131 (GAVDSILGGVATYGAA; SEQ ID NO: __________________). Other examples include peptides bearing a DR 1-4-7 supermotif, or either of the DR3 motifs.

Alternatively, it is possible to prepare synthetic peptides capable of stimulating T helper lymphocytes, in a loosely HLA-restricted fashion, using amino acid sequences not found in nature (*see, e.g.,* PCT publication WO 95/07707). These synthetic compounds called Pan-DR-binding epitopes (e.g., PADRE™, Epimmune, Inc., San Diego, CA) are designed to most preferably bind most HLA-DR (human HLA class II) molecules. For instance, a pan-DR-binding epitope peptide having the formula: aKXVAAWTLKAAa (SEQ ID NO: __________________), where "X" is either cyclohexylalanine, phenylalanine, or tyrosine, and a is either D-alanine or L-alanine, has been found to bind to most HLA-DR alleles, and to stimulate the response of T helper lymphocytes from most individuals, regardless of their HLA type. An alternative of a pan-DR binding epitope comprises all "L" natural amino acids and can be provided in the form of nucleic acids that encode the epitope.

HTL peptide epitopes can also be modified to alter their biological properties. For example, they can be modified to include D-amino acids to increase their resistance to proteases and thus extend their serum half life, or they can be conjugated to other molecules such as lipids, proteins, carbohydrates, and the like to increase their biological activity. For example, a T helper peptide can be conjugated to one or more palmitic acid chains at either the amino or carboxyl termini.

### X.C.3. Combinations of CTL Peptides with T Cell Priming Agents

In some embodiments it may be desirable to include in the pharmaceutical compositions of the invention at least one component which primes B lymphocytes or T lymphocytes. Lipids have been identified as agents capable of priming CTL *in vivo.* For example, palmitic acid residues can be attached to the ε-and α-amino groups of a lysine residue and then linked, *e.g.*, via one or more linking residues such as Gly, Gly-Gly-, Ser, Ser-Ser, or the like, to an immunogenic peptide. The lipidated peptide can then be administered either directly in a micelle or particle, incorporated into a liposome, or emulsified in an adjuvant, *e.g.,* incomplete Freund's adjuvant. In a preferred embodiment, a particularly effective immunogenic composition comprises palmitic acid attached to ε- and α- amino groups of Lys, which is attached via linkage, *e.g.,* Ser-Ser, to the amino terminus of the immunogenic peptide.

As another example of lipid priming of CTL responses, *E. coli* lipoproteins, such as tripalmitoyl-S-glycerylcysteinlyseryl- serine ($P_3CSS$) can be used to prime virus specific CTL when covalently attached to an appropriate peptide *(see, e.g.,* Deres, et al., Nature 342:561, 1989). Peptides of the invention can be coupled to $P_3CSS$, for example, and the lipopeptide administered to an individual to specifically prime an immune response to the target antigen. Moreover, because the induction of neutralizing antibodies can also be primed with $P_3CSS$-conjugated epitopes, two such compositions can be combined to more effectively elicit both humoral and cell-mediated responses.

### X.C.4. Vaccine Compositions Comprising DC Pulsed with CTL and/or HTL Peptides

An embodiment of a vaccine composition in accordance with the invention comprises *ex vivo* administration of a cocktail of epitope-bearing peptides to PBMC, or isolated DC therefrom, from the patient's blood. A pharmaceutical to facilitate harvesting of DC ean be used, such as Progenipoietin™ (Pharmacia-Monsanto, St. Louis, MO) or GM-CSF/IL-4. After pulsing the DC with peptides and prior to reinfusion into patients, the DC are washed to remove unbound peptides. In this embodiment, a vaccine comprises peptide-pulsed DCs which present the pulsed peptide epitopes complexed with HLA molecules on their surfaces.

The DC can be pulsed *ex vivo* with a cocktail of peptides, some of which stimulate CTL responses to 121P2A3. Optionally, a helper T cell (HTL) peptide, such as a natural or artificial loosely restricted HLA Class II peptide, can be included to facilitate the CTL response. Thus, a vaccine in accordance with the invention is used to treat a cancer which expresses or overexpresses 121P2A3.

## X.D. Adoptive Immunotherapy

Antigenic 121P2A3-related peptides are used to elicit a CTL and/or HTL response *ex vivo*, as well. The resulting CTL or HTL cells, can be used to treat tumors in patients that do not respond to other conventional forms of therapy, or will not respond to a therapeutic vaccine peptide or nucleic acid in accordance with the invention. *Ex vivo* CTL or HTL responses to a particular antigen are induced by incubating in tissue culture the patient's, or genetically compatible, CTL or HTL precursor cells together with a source of antigen-presenting cells (APC), such as dendritic cells, and the appropriate immunogenic peptide. After an appropriate incubation time (typically about 7-28 days), in which the precursor cells are activated and expanded into effector cells, the cells are infused back into the patient, where they will destroy (CTL) or facilitate destruction (HTL) of their specific target cell (e.g., a tumor cell). Transfected dendritic cells may also be used as antigen presenting cells.

## X.E. Administration of Vaccines for Therapeutic or Prophylactic Purposes

Pharmaceutical and vaccine compositions of the invention are typically used to treat and/or prevent a cancer that expresses or overexpresses 121P2A3. In therapeutic applications, peptide and/or nucleic acid compositions are administered to a patient in an amount sufficient to elicit an effective B cell, CTL and/or HTL response to the antigen and to cure or at least partially arrest or slow symptoms and/or complications. An amount adequate to accomplish this is defined as "therapeutically effective dose." Amounts effective for this use will depend on, *e.g.,* the particular composition administered, the manner of administration, the stage and severity of the disease being treated, the weight and general state of health of the patient, and the judgment of the prescribing physician.

For pharmaceutical compositions, the immunogenic peptides of the invention, or DNA encoding them, are generally administered to an individual already bearing a tumor that expresses 121P2A3. The peptides or DNA encoding them can be administered individually or as fusions of one or more peptide sequences. Patients can be treated with the immunogenic peptides separately or in conjunction with other treatments, such as surgery, as appropriate.

For therapeutic use, administration should generally begin at the first diagnosis of 121P2A3-associated cancer. This is followed by boosting doses until at least symptoms are substantially abated and for a period thereafter. The embodiment of the vaccine composition (*i.e.,* including, but not limited to embodiments such as peptide cocktails, polyepitopic polypeptides, minigenes, or TAA-specific CTLs or pulsed dendritic cells) delivered to the patient may vary according to the stage of the disease or the patient's health status. For example, in a patient with a tumor that expresses 121P2A3, a vaccine comprising 121P2A3-specific CTL may be more efficacious in killing tumor cells in patient with advanced disease than alternative embodiments.

It is generally important to provide an amount of the peptide epitope delivered by a mode of administration sufficient to effectively stimulate a cytotoxic T cell response; compositions which stimulate helper T cell responses can also be given in accordance with this embodiment of the invention.

The dosage for an initial therapeutic immunization generally occurs in a unit dosage range where the lower value is about 1, 5, 50, 500, or 1,000 $\mu$g and the higher value is about 10,000; 20,000; 30,000; or 50,000 $\mu$g. Dosage values for a human typically range from about 500 $\mu$g to about 50,000 $\mu$g per 70 kilogram patient. Boosting dosages of between about 1.0 $\mu$g to about 50,000 $\mu$g of peptide pursuant to a boosting regimen over weeks to months may be administered depending upon the patient's response and condition as determined by measuring the specific activity of CTL and HTL obtained from the patient's blood. Administration should continue until at least clinical symptoms or laboratory tests indicate that the neoplasia, has been eliminated or reduced and for a period thereafter. The dosages, routes of administration, and dose schedules are adjusted in accordance with methodologies known in the art.

In certain embodiments, the peptides and compositions of the present invention are employed in serious disease states, that is, life-threatening or potentially life threatening situations. In such cases, as a result of the minimal amounts of extraneous substances and the relative nontoxic nature of the peptides in preferred compositions of the invention, it is possible and may be felt desirable by the treating physician to administer substantial excesses of these peptide compositions relative to these stated dosage amounts.

The vaccine compositions of the invention can also be used purely as prophylactic agents. Generally the dosage for an initial prophylactic immunization generally occurs in a unit dosage range where the lower value is about 1, 5, 50, 500, or 1000 μg and the higher value is about 10,000; 20,000; 30,000; or 50,000 μg. Dosage values for a human typically range from about 500 μg to about 50,000 μg per 70 kilogram patient This is followed by boosting dosages of between about 1.0 μg to about 50,000 μg of peptide administered at defined intervals from about four weeks to six months after the initial administration of vaccine. The immunogenicity of the vaccine can be assessed by measuring the specific activity of CTL and HTL obtained from a sample of the patient's blood.

The pharmaceutical compositions for therapeutic treatment are intended for parenteral, topical, oral, nasal, intrathecal, or local (*e.g.* as a cream or topical ointment) administration. Preferably, the pharmaceutical compositions are administered parentally, *e.g.*, intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier.

A variety of aqueous carriers may be used, *e.g.*, water, buffered water, 0.8% saline, 0.3% glycine, hyaluronic acid and the like. These compositions may be sterilized by conventional, well-known sterilization techniques, or may be sterile filtered. The resulting aqueous solutions may be packaged for use as is, or lyophilized, the lyophilized preparation being combined with a sterile solution prior to administration.

The compositions may contain pharmaceutically acceptable auxiliary substances as required to approximate physiological conditions, such as pH-adjusting and buffering agents, tonicity adjusting agents, wetting agents, preservatives, and the like, for example, sodium acetate, sodium lactate, sodium chloride, potassium chloride, calcium chloride, sorbitan monolaurate, triethanolamine oleate, *etc.*

The concentration of peptides of the invention in the pharmaceutical formulations can vary widely, *i.e.,* from less than about 0.1%, usually at or at least about 2% to as much as 20% to 50% or more by weight, and will be selected primarily by fluid volumes, viscosities, etc., in accordance with the particular mode of administration selected.

A human unit dose form of a composition is typically included in a pharmaceutical composition that comprises a human unit dose of an acceptable carrier, in one embodiment an aqueous carrier; and is administered in a volume/quantity that is known by those of skill in the art to be used for administration of such compositions to humans (*see, e.g.,* Remington's Pharmaceutical Sciences, 17th Edition, A. Gennaro, Editor, Mack Publishing Co., Easton, Pennsylvania, 1985). For example a peptide dose for initial immunization can be from about 1 to about 50,000 μg, generally 100-5,000 μg, for a 70 kg patient. For example, for nucleic acids an initial immunization may be performed using an expression vector in the form of naked nucleic acid administered IM (or SC or ID) in the amounts of 0.5-5 mg at multiple sites. The nucleic acid (0.1 to 1000 μg) can also be administered using a gene gun. Following an incubation period of 3-4 weeks, a booster dose is then administered. The booster can be recombinant fowlpox virus administered at a dose of 5-$10^7$ to 5x$10^9$ pfu.

For antibodies, a treatment generally involves repeated administration of the anti-121P2A3 antibody preparation, via an acceptable route of administration such as intravenous injection (IV), typically at a dose in the range of about 0.1 to about 10 mg/kg body weight. In general, doses in the range of 10-500 mg mAb per week are effective and well tolerated. Moreover, an initial loading dose of approximately 4 mg/kg patient body weight IV, followed by weekly doses of about 2 mg/kg IV of the anti- 121P2A3 mAb preparation represents an acceptable dosing regimen. As appreciated by those of skill in the art, various factors can influence the ideal dose in a particular case. Such factors include, for example, half life of a composition, the binding affinity of an Ab, the immunogenicity of a substance, the degree of 121P2A3 expression in the patient, the extent of circulating shed 121P2A3 antigen, the desired steady-state concentration level, frequency of treatment, and the influence of chemotherapeutic or other agents used in combination with the treatment method of the invention, as well as the health status of a particular patient. Non-limiting preferred human unit doses are, for example, 500μg - 1mg, 1mg - 50mg, 50mg - -100mg, 100mg - 200mg, 200mg - 300mg, 400mg - 500mg, 500mg - 600mg, 600mg - 700mg, 700mg - 800mg, 800mg - 900mg, 900mg - 1g, or 1mg - 700mg. In certain embodiments, the dose is in a range of 2-5 mg/kg body weight, e.g., with follow on weekly doses of 1-3 mg/kg; 0.5mg, 1, 2, 3, 4, 5, 6, 7, 8, 9, 10mg/kg body weight followed, e.g., in two, three or four weeks by weekly doses; 0.5 - 10mg/kg body weight, e.g., followed in two, three or four weeks by weekly doses; 225, 250, 275, 300, 325, 350, 375, 400mg $m^2$ of body area weekly; 1-600mg $m^2$ of body area weekly; 225-400mg $m^2$ of body area weekly; these does can be followed by weekly doses for 2, 3, 4, 5, 6, 7, 8, 9, 19, 11, 12 or more weeks.

In one embodiment, human unit dose forms of polynucleotides comprise a suitable dosage range or effective amount that provides any therapeutic effect. As appreciated by one of ordinary skill in the art a therapeutic effect depends on a number of factors, including the sequence of the polynucleotide, molecular weight of the polynucleotide and route of administration. Dosages are generally selected by the physician or other health care professional in accordance with a variety of parameters known in the art, such as severity of symptoms, history of the patient and the like. Generally, for a polynucleotide of about 20 bases, a dosage range may be selected from, for example, an independently selected lower limit such as about 0.1, 0.25, 0.5, 1, 2, 5, 10, 20, 30, 40, 50, 60, 70, 80, 90, 100, 200, 300, 400 or 500 mg/kg up to an independently selected upper limit, greater than the lower limit, of about 60, 80, 100, 200, 300, 400, 500, 750, 1000, 1500, 2000, 3000, 4000, 5000, 6000, 7000, 8000, 9000 or 10,000 mg/kg. For example, a dose may be about any of the following: 0.1 to 100 mg/kg, 0.1 to 50 mg/kg, 0.1 to 25 mg/kg, 0.1 to 10 mg/kg, 1 to 500 mg/kg, 100 to 400 mg/kg, 200 to 300 mg/kg, 1 to 100 mg/kg, 100 to 200 mg/kg, 300 to 400 mg/kg, 400 to 500 mg/kg, 500 to 1000 mg/kg, 500 to 5000 mg/kg, or 500 to 10,000 mg/kg. Generally, parenteral routes of administration may require higher doses of polynucleotide compared to more direct application to the nucleotide to diseased tissue, as do polynucleotides of increasing length.

In one embodiment, human unit dose forms of T-cells comprise a suitable dosage range or effective amount that provides any therapeutic effect. As appreciated by one of ordinary skill in the art, a therapeutic effect depends on a number of factors. Dosages are generally selected by the physician or other health care professional in accordance with a variety of parameters known in the art, such as severity of symptoms, history of the patient and the like. A dose may be about $10^4$ cells to about $10^6$ cells, about $10^6$ cells to about $10^8$ cells, about $10^8$ to about $10^{11}$ cells, or about $10^8$ to about 5 x $10^{10}$ cells. A dose may also about $10^6$ cells/$m^2$ to about $10^{10}$ cells/$m^2$, or about $10^6$ cells/$m^2$ to about $10^8$ cells/$m^2$.

Proteins(s) of the invention, and/or nucleic acids encoding the protein(s), can also be administered via liposomes, which may also serve to: 1) target the proteins(s) to a particular tissue, such as lymphoid tissue; 2) to target selectively to diseases cells; or, 3) to increase the half-life of the peptide composition. Liposomes include emulsions, foams, micelles, insoluble monolayers, liquid crystals, phospholipid dispersions, lamellar layers and the like. In these preparations, the peptide to be delivered is incorporated as part of a liposome, alone or in conjunction with a molecule which binds to a receptor prevalent among lymphoid cells, such as monoclonal antibodies which bind to the CD45 antigen, or with other therapeutic or immunogenic compositions. Thus, liposomes either filled or decorated with a desired peptide of the invention can be directed to the site of lymphoid cells, where the liposomes then deliver the peptide compositions. Liposomes for use in accordance with the invention are formed from standard vesicle-forming lipids, which generally include neutral and negatively charged phospholipids and a sterol, such as cholesterol. The selection of lipids is generally guided by consideration of, *e.g.,* liposome size, acid lability and stability of the liposomes in the blood stream. A variety of methods are available for preparing liposomes, as described in, *e.g.,* Szoka, et al., Ann. Rev. Biophys. Bioeng. 9:467 (1980), and U.S. Patent Nos. 4,235,871, 4,501,728, 4,837,028, and 5,019,369.

For targeting cells of the immune system, a ligand to be incorporated into the liposome can include, *e.g.*, antibodies or fragments thereof specific for cell surface determinants of the desired immune system cells. A liposome suspension containing a peptide may be administered intravenously, locally, topically, *etc.* in a dose which varies according to, *inter alia,* the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, immunogenic peptides are preferably supplied in finely divided form along with a surfactant and propellant. Typical percentages of peptides are about 0.01%-20% by weight, preferably about 1%-10%. The surfactant must, of course, be nontoxic, and preferably soluble in the propellant. Representative of such agents are the esters or partial esters of fatty acids containing from about 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute about 0.1%-20% by weight of the composition, preferably about 0.25-5%. The balance of the composition is ordinarily propellant. A carrier can also be included, as desired, as with, e.g., lecithin for intranasal delivery.

## XI.) Diagnostic and Prognostic Embodiments of 121P2A3.

As disclosed herein, 121P2A3 polynucleotides, polypeptides, reactive cytotoxic T cells (CTL), reactive helper T cells (HTL) and anti-polypeptide antibodies are used in well known diagnostic, prognostic and therapeutic assays that examine conditions associated with dysregulated cell growth such as cancer, in particular the cancers listed in Table I (see, e.g., both its specific pattern of tissue expression as well as its overexpression in certain cancers as described for example in the Example entitled "Expression analysis of 121P2A3 in normal tissues, and patient specimens").

121P2A3 can be analogized to a prostate associated antigen PSA, the archetypal marker that has been used by medical practitioners for years to identify and monitor the presence of prostate cancer (see, e.g., Merrill et al., J. Urol. 163(2): 503-5120 (2000); Polascik et al., J. Urol. Aug; 162(2):293-306 (1999) and Fortier et al., J. Nat. Cancer Inst. 91 (19): 1635-1640(1999)). A variety of other diagnostic markers are also used in similar contexts including p53 and K-ras (see, e.g., Tulchinsky et al., Int J Mol Med 1999 Jul 4(1):99-102 and Minimoto et al., Cancer Detect Prev 2000;24(1): 1-12). Therefore, this disclosure of 121P2A3 polynucleotides and polypeptides (as well as 121P2A3 polynucleotide probes and anti-121P2A3 antibodies used to identify the presence of these molecules) and their properties allows skilled artisans to utilize these molecules in methods that are analogous to those used, for example, in a variety of diagnostic assays directed to examining conditions associated with cancer.

Typical embodiments of diagnostic methods which utilize the 121P2A3 polynucleotides, polypeptides, reactive T cells and antibodies are analogous to those methods from well-established diagnostic assays which employ, e.g., PSA polynucleotides, polypeptides, reactive T cells and antibodies. For example, just as PSA polynucleotides are used as probes (for example in Northern analysis, see, e.g., Sharief et al., Biochem. Mol. Biol. Int. 33(3):567-74(1994)) and primers (for example in PCR analysis, see, e.g., Okegawa et al., J. Urol. 163(4): 1189-1190 (2000)) to observe the presence and/or the level of PSA mRNAs in methods of monitoring PSA overexpression or the metastasis of prostate cancers, the 121P2A3 polynucleotides described herein can be utilized in the same way to detect 121P2A3 overexpression or the metastasis of prostate and other cancers expressing this gene. Alternatively, just as PSA polypeptides are used to generate antibodies specific for PSA which can then be used to observe the presence and/or the level of PSA proteins in methods to monitor PSA protein overexpression (see, e.g., Stephan et al., Urology 55(4):560-3 (2000)) or the metastasis of prostate cells (see, e.g., Alanen et al., Pathol. Res. Pract. 192(3):233-7 (1996)), the 121P2A3 polypeptides described herein can be utilized to generate antibodies for use in detecting 121P2A3 overexpression or the metastasis of prostate cells and cells of other cancers expressing this gene.

Specifically, because metastases involves the movement of cancer cells from an organ of origin (such as the lung or prostate gland etc.) to a different area of the body (such as a lymph node), assays which examine a biological sample for the presence of cells expressing 121P2A3 polynucleotides and/or polypeptides can be used to provide evidence of metastasis. For example, when a biological sample from tissue that does not normally contain 121P2A3-expressing cells (lymph node) is found to contain 121P2A3-expressing cells such as the 121P2A3 expression seen in LAPC4 and LAPC9, xenografts isolated from lymph node and bone metastasis, respectively, this finding is indicative of metastasis.

Alternatively 121P2A3 polynucleotides and/or polypeptides can be used to provide evidence of cancer, for example, when cells in a biological sample that do not normally express 121P2A3 or express 121P2A3 at a different level are found to express 121P2A3 or have an increased expression of 121P2A3 (see, e.g., the 121P2A3 expression in the cancers listed in Table I and in patient samples etc. shown in the accompanying Figures). In such assays, artisans may further wish to generate supplementary evidence of metastasis by testing the biological sample for the presence of a second tissue restricted marker (in addition to 121P2A3) such as PSA, PSCA etc. (see, e.g., Alanen et al., Pathol. Res. Pract. 192(3): 233-237 (1996)).

Just as PSA polynucleotide fragments and polynucleotide variants are employed by skilled artisans for use in methods of monitoring PSA, 121P2A3 polynucleotide fragments and polynucleotide variants are used in an analogous manner. In particular, typical PSA polynucleotides used in methods of monitoring PSA are probes or primers which consist of fragments of the PSA cDNA sequence. Illustrating this, primers used to PCR amplify a PSA polynucleotide must include less than the whole PSA sequence to function in the polymerase chain reaction. In the context of such PCR reactions, skilled artisans generally create a variety of different polynucleotide fragments that can be used as primers in order to amplify different portions of a polynucleotide of interest or to optimize amplification reactions (see, e.g., Caetano-Anolles, G. Biotechniques 25(3): 472-476, 478-480 (1998); Robertson et al., Methods Mol. Biol. 98:121-154 (1998)). An additional illustration of the use of such fragments is provided in the Example entitled "Expression analysis of 121P2A3 in normal tissues, and patient specimens," where a 121P2A3 polynucleotide fragment is used as a probe to show the expression of 121P2A3 RNAs in cancer cells. In addition, variant polynucleotide sequences are typically used as primers and probes for the corresponding mRNAs in PCR and Northern analyses (see, e.g., Sawai et al., Fetal Diagn. Ther. 1996 Nov-Dec 11(6):407-13 and Current Protocols In Molecular Biology, Volume 2, Unit 2, Frederick M. Ausubel et al. eds., 1995)). Polynucleotide fragments and variants are useful in this context where they are capable of binding to a target polynucleotide sequence (e.g., a 121P2A3 polynucleotide shown in Figure 2 or variant thereof) under conditions of high stringency.

Furthermore, PSA polypeptides which contain an epitope that can be recognized by an antibody or T cell that specifically binds to that epitope are used in methods of monitoring PSA. 121P2A3 polypeptide fragments and polypeptide analogs or variants can also be used in an analogous manner. This practice of using polypeptide fragments or polypeptide variants to generate antibodies (such as anti-PSA antibodies or T cells) is typical in the art with a wide variety of systems such as fusion proteins being used by practitioners (see, e.g., Current Protocols In Molecular Biology, Volume 2, Unit 16, Frederick M. Ausubel et al. eds., 1995). In this context, each epitope(s) functions to provide the architecture with which an antibody or T cell is reactive. Typically, skilled artisans create a variety of different polypeptide fragments that can be used in order to generate immune responses specific for different portions of a polypeptide of interest (see, e.g., U.S. Patent No. 5,840,501 and U.S. Patent No. 5,939,533). For example it may be preferable to utilize a polypeptide comprising one of the 121P2A3 biological motifs discussed herein or a motif-bearing subsequence which is readily identified by one of skill in the art based on motifs available in the art. Polypeptide fragments, variants or analogs are typically useful in this context as long as they comprise an epitope capable of generating an antibody or T cell specific for a target polypeptide sequence (e.g. a 121P2A3 polypeptide shown in Figure 3).

As shown herein, the 121P2A3 polynucleotides and polypeptides (as well as the 121P2A3 polynucleotide probes and anti-121P2A3 antibodies or T cells used to identify the presence of these molecules) exhibit specific properties that make them useful in diagnosing cancers such as those listed in Table I. Diagnostic assays that measure the presence of 121P2A3 gene products, in order to evaluate the presence or onset of a disease condition described herein, such as prostate cancer, are used to identify patients for preventive measures or further monitoring, as has been done so successfully with PSA. Moreover, these materials satisfy a need in the art for molecules having similar or complementary characteristics to PSA in situations where, for example, a definite diagnosis of metastasis of prostatic origin cannot be made on the basis of a test for PSA alone (see, e.g., Alanen et al., Pathol. Res. Pract. 192(3): 233-237 (1996)), and consequently, materials such as 121P2A3 polynucleotides and polypeptides (as well as the 121P2A3 polynucleotide probes and anti-121P2A3 antibodies used to identify the presence of these molecules) need to be employed to confirm a metastases of prostatic origin.

Finally, in addition to their use in diagnostic assays, the 121P2A3 polynucleotides disclosed herein have a number of other utilities such as their use in the identification of oncogenetic associated chromosomal abnormalities in the chromosomal region to which the 121P2A3 gene maps (see the Example entitled "Chromosomal Mapping of 121P2A3" below). Moreover, in addition to their use in diagnostic assays, the 121P2A3-related proteins and polynucleotides disclosed herein have other utilities such as their use in the forensic analysis of tissues of unknown origin (see, e.g., Takahama K Forensic Sci Int 1996 Jun 28;80(1-2): 63-9).

Additionally, 121P2A3-related proteins or polynucleotides of the invention can be used to treat a pathologic condition characterized by the over-expression of 121P2A3. For example, the amino acid or nucleic acid sequence of Figure 2 or Figure 3, or fragments of either, can be used to generate an immune response to a 121P2A3 antigen. Antibodies or other molecules that react with 121P2A3 can be used to modulate the function of this molecule, and thereby provide a therapeutic benefit.

## XII.) Inhibition of 121P2A3 Protein Function

The invention includes various methods and compositions for inhibiting the binding of 121P2A3 to its binding partner or its association with other protein(s) as well as methods for inhibiting 121P2A3 function.

### XII.A.) Inhibition of 121P2A3 With Intracellular Antibodies

In one approach, a recombinant vector that encodes single chain antibodies that specifically bind to 121P2A3 are introduced into 121P2A3 expressing cells via gene transfer technologies. Accordingly, the encoded single chain anti-121P2A3 antibody is expressed intracellularly, binds to 121P2A3 protein, and thereby inhibits its function. Methods for engineering such intracellular single chain antibodies are well known. Such intracellular antibodies, also known as "intrabodies", are specifically targeted to a particular compartment within the cell, providing control over where the inhibitory activity of the treatment is focused. This technology has been successfully applied in the art (for review, see Richardson and Marasco, 1995, TIBTECH vol. 13). Intrabodies have been shown to virtually eliminate the expression of otherwise abundant cell surface receptors (see, e.g., Richardson et al., 1995, Proc. Natl. Acad. Sci. USA 92: 3137-3141; Beerli et al., 1994, J. Biol. Chem. 289: 23931-23936; Deshane et al., 1994, Gene Ther. 1:332-337).

Single chain antibodies comprise the variable domains of the heavy and light chain joined by a flexible linker polypeptide, and are expressed as a single polypeptide. Optionally, single chain antibodies are expressed as a single chain variable region fragment joined to the light chain constant region. Well-known intracellular trafficking signals are engineered into recombinant polynucleotide vectors encoding such single chain antibodies in order to precisely target the intrabody to the desired intracellular compartment. For example, intrabodies targeted to the endoplasmic reticulum (ER) are engineered to incorporate a leader peptide and, optionally, a C-terminal ER retention signal, such as the KDEL amino acid motif. Intrabodies intended to exert activity in the nucleus are engineered to include a nuclear localization signal. Lipid moieties are joined to intrabodies in order to tether the intrabody to the cytosolic side of the plasma membrane. Intrabodies can also be targeted to exert function in the cytosol. For example, cytosolic intrabodies are used to sequester factors within the cytosol, thereby preventing them from being transported to their natural cellular destination.

In one embodiment, intrabodies are used to capture 121P2A3 in the nucleus, thereby preventing its activity within the nucleus. Nuclear targeting signals are engineered into such 121P2A3 intrabodies in order to achieve the desired targeting. Such 121P2A3 intrabodies are designed to bind specifically to a particular 121P2A3 domain. In another embodiment, cytosolic intrabodies that specifically bind to a 121P2A3 protein are used to prevent 121P2A3 from gaining access to the nucleus, thereby preventing it from exerting any biological activity within the nucleus (e.g., preventing 121P2A3 from forming transcription complexes with other factors).

In order to specifically direct the expression of such intrabodies to particular cells, the transcription of the intrabody is placed under the regulatory control of an appropriate tumor-specific promoter and/or enhancer. In order to target intrabody expression specifically to prostate, for example, the PSA promoter and/or promoter/enhancer can be utilized (See, for example, U.S. Patent No. 5,919,652 issued 6 July 1999).

**XII.B.) Inhibition of 121P2A3 with Recombinant Proteins**

In another approach, recombinant molecules bind to 121P2A3 and thereby inhibit 121P2A3 function. For example, these recombinant molecules prevent or inhibit 121P2A3 from accessing/binding to its binding partner(s) or associating with other protein(s). Such recombinant molecules can, for example, contain the reactive part(s) of a 121P2A3 specific antibody molecule. In a particular embodiment, the 121P2A3 binding domain of a 121P2A3 binding partner is engineered into a dimeric fusion protein, whereby the fusion protein comprises two 121P2A3 ligand binding domains linked to the Fc portion of a human IgG, such as human IgG1. Such IgG portion can contain, for example, the $C_H2$ and $C_H3$ domains and the hinge region, but not the $C_H1$ domain. Such dimeric fusion proteins are administered in soluble form to patients suffering from a cancer associated with the expression of 121P2A3, whereby the dimeric fusion protein specifically binds to 121P2A3 and blocks 121P2A3 interaction with a binding partner. Such dimeric fusion proteins are further combined into multimeric proteins using known antibody linking technologies.

**XII.C.) Inhibition of 121P2A3 Transcription or Translation**

The present invention also comprises various methods and compositions for inhibiting the transcription of the 121P2A3 gene. Similarly, the invention also provides methods and compositions for inhibiting the translation of 121P2A3 mRNA into protein.

In one approach, a method of inhibiting the transcription of the 121P2A3 gene comprises contacting the 121P2A3 gene with a 121P2A3 antisense polynucleotide. In another approach, a method of inhibiting 121P2A3 mRNA translation comprises contacting a 121P2A3 mRNA with an antisense polynucleotide. In another approach, a 121P2A3 specific ribozyme is used to cleave a 121P2A3 message, thereby inhibiting translation. Such antisense and ribozyme based methods can also be directed to the regulatory regions of the 121P2A3 gene, such as 121P2A3 promoter and/or enhancer elements. Similarly, proteins capable of inhibiting a 121P2A3 gene transcription factor are used to inhibit 121P2A3 mRNA transcription. The various polynucleotides and compositions useful in the aforementioned methods have been described above. The use of antisense and ribozyme molecules to inhibit transcription and translation is well known in the art.

Other factors that inhibit the transcription of 121P2A3 by interfering with 121P2A3 transcriptional activation are also useful to treat cancers expressing 121P2A3. Similarly, factors that interfere with 121P2A3 processing are useful to treat cancers that express 121P2A3. Cancer treatment methods utilizing such factors are also within the scope of the invention.

**XII.D.) General Considerations for Therapeutic Strategies**

Gene transfer and gene therapy technologies can be used to deliver therapeutic polynucleotide molecules to tumor cells synthesizing 121P2A3 (i.e., antisense, ribozyme, polynucleotides encoding intrabodies and other 121P2A3 inhibitory molecules). A number of gene therapy approaches are known in the art. Recombinant vectors encoding 121P2A3 antisense polynucleotides, ribozymes, factors capable of interfering with 121P2A3 transcription, and so forth, can be delivered to target tumor cells using such gene therapy approaches.

The above therapeutic approaches can be combined with any one of a wide variety of surgical, chemotherapy or radiation therapy regimens. The therapeutic approaches of the invention can enable the use of reduced dosages of chemotherapy (or other therapies) and/or less frequent administration, an advantage for all patients and particularly for those that do not tolerate the toxicity of the chemotherapeutic agent well.

The anti-tumor activity of a particular composition (e.g., antisense, ribozyme, intrabody), or a combination of such compositions, can be evaluated using various *in vitro* and *in vivo* assay systems. *In vitro* assays that evaluate therapeutic activity include cell growth assays, soft agar assays and other assays indicative of tumor promoting activity, binding assays capable of determining the extent to which a therapeutic composition will inhibit the binding of 121P2A3 to a binding partner, etc.

*In vivo,* the effect of a 121P2A3 therapeutic composition can be evaluated in a suitable animal model. For example, xenogenic prostate cancer models can be used, wherein human prostate cancer explants or passaged xenograft tissues are introduced into immune compromised animals, such as nude or SCID mice (Klein et al., 1997, Nature Medicine 3: 402-408). For example, PCT Patent Application WO98/16628 and U.S. Patent 6,107,540 describe various xenograft models of human prostate cancer capable of recapitulating the development of primary tumors, micrometastasis, and the formation of osteoblastic metastases characteristic of late stage disease. Efficacy can be predicted using assays that measure inhibition of tumor formation, tumor regression or metastasis, and the like.

*In vivo* assays that evaluate the promotion of apoptosis are useful in evaluating therapeutic compositions. In one embodiment, xenografts from tumor bearing mice treated with the therapeutic composition can be examined for the presence of apoptotic foci and compared to untreated control xenograft-bearing mice. The extent to which apoptotic foci are found in the tumors of the treated mice provides an indication of the therapeutic efficacy of the composition.

The therapeutic compositions used in the practice of the foregoing methods can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980).

Therapeutic formulations can be solubilized and administered via any route capable of delivering the therapeutic composition to the tumor site. Potentially effective routes of administration include, but are not limited to, intravenous, parenteral, intraperitoneal, intramuscular, intratumor, intradermal, intraorgan, orthotopic, and the like. A preferred formulation for intravenous injection comprises the therapeutic composition in a solution of preserved bacteriostatic water, sterile unpreserved water, and/or diluted in polyvinylchloride or polyethylene bags containing 0.9% sterile Sodium Chloride for Injection, USP. Therapeutic protein preparations can be lyophilized and stored as sterile powders, preferably under vacuum, and then reconstituted in bacteriostatic water (containing for example, benzyl alcohol preservative) or in sterile water prior to injection.

Dosages and administration protocols for the treatment of cancers using the foregoing methods will vary with the method and the target cancer, and will generally depend on a number of other factors appreciated in the art.

## XIII.) Kits

For use in the diagnostic and therapeutic applications described herein, kits are also within the scope of the invention. Such kits can comprise a carrier, package or container that is compartmentalized to receive one or more containers such as vials, tubes; and the like, each of the container(s) comprising one of the separate elements to be used in the method. For example, the container(s) can comprise a probe that is or can be detectably labeled. Such probe can be an antibody or polynucleotide specific for a 121P2A3-related protein or a 121P2A3 gene or message, respectively. Where the method utilizes nucleic acid hybridization to detect the target nucleic acid, the kit can also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label. The kit can include all or part of the amino acid sequence of Figure 2 or Figure 3 or analogs thereof, or a nucleic acid molecules that encodes such amino acid sequences.

The kit of the invention will typically comprise the container described above and one or more other containers comprising materials desirable from a commercial and user standpoint, including buffers, diluents, filters, needles, syringes, and package inserts with instructions for use.

A label can be present on the container to indicate that the composition is used for a specific therapy or non-therapeutic application, and can also indicate directions for either in *vivo* or in *vitro* use, such as those described above. Directions and or other information can also be included on an insert which is included with the kit.

## EXAMPLES:

Various aspects of the invention are further described and illustrated by way of the several examples that follow, none of which are intended to limit the scope of the invention.

## Example 1: SSH-Generated Isolation of a cDNA Fragment of the 121P2A3 Gene

To isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an AI phenotype and start growing again in the castrated males. Tumors were harvested at different time points after castration to identify genes that are turned on or off during the transition to androgen independence.

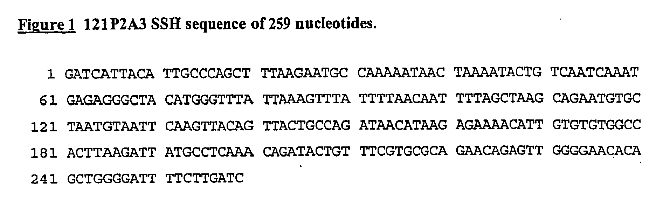

The gene 121P2A3 was derived from an LAPC-9 AD minus LAPC-9 AD (28 days post-castration) subtraction. The SSH DNA sequence of 259 bp is listed in Figure 1. A cDNA (121P2A3 clone 5) of 2473 bp was isolated from a LAPC-9AD cDNA library, revealing an ORF of 464 amino acids (Figures 2 and 3). Variants of 121P2A3 v.1 were also identified, and these are listed in Figures 2 and 3.

The 121P2A3 protein shows homology to a novel hypothetical protein FLJ10540 isolated from the human teratocarcinoma cell line NT2 (Figure 4B and 4D). The two proteins are 98% identical over a 223 amino acid region starting from position 170 of 121P2A3 v.1. The 121P2A3 protein also shows homology to the XM_005908 (similar to RIKEN cDNA 1200008012) gene. The gene XM_005908 was isolated from rhabdomyosarcoma cDNA library, validating the expression of 121P2A3 in human cancers. 121P2A3 v.1 and XM_005908 proteins are 99% identical over 464 amino acids (Figure 4E).

Amino acid sequence analysis of 121P2A3 reveals 75% identity over 464 amino acid region to a mouse putative protein (Figure 4F). 121P2A3 v.1 also shows homology to the human nef-associated factor-1 (naf-1). The two proteins are 23% identical over a 339 amino acid region (Figure 4G).

Additional homology analysis is presented in Example 44.

Materials and Methods

LAPC Xenografts and Human Tissues:

LAPC xenografts were obtained from Dr. Charles Sawyers (UCLA) and generated as described (Klein et al, 1997, Nature Med. 3: 402-408; Craft et al., 1999, Cancer Res. 59: 5030-5036). Androgen dependent and independent LAPC-9 AD and AI xenografts were grown in male SCID mice and were passaged as small tissue chunks in recipient males. LAPC-9 AI xenografts were derived from LAPC-9 AD tumors, respectively. To generate the AI xenografts, male mice bearing AD tumors were castrated and maintained for 2-3 months. After the tumors re-grew, the tumors were harvested and passaged in castrated males or in female SCID mice.

Cell Lines:

Human cell lines (e.g., HeLa) were obtained from the ATCC and were maintained in DMEM with 5% fetal calf serum.

Human Tissues:

The patient cancer and normal tissues were purchased from different sources such as the NDRI (Philadelphia, PA). mRNA for some normal tissues were purchased from Clontech, Palo Alto, CA.

RNA Isolation:

Tissues were homogenized in Trizol reagent (Life Technologies, Gibco BRL) using 10 ml/ g tissue isolate total RNA. Poly A RNA was purified from total RNA using Qiagen's Oligotex mRNA Mini and Midi kits. Total and mRNA were quantified by spectrophotometric analysis (O.D. 260/280 nm) and analyzed by gel electrophoresis

Oligonucleotides:

The following HPLC purified oligonucleotides were used.

DPNCDN (cDNA synthesis primer):

5'TTTTGATCAAGCTT$_{30}$3' (SEQ ID NO:__________________)

Adaptor 1:

5'CTAATACGACTCACTATAGGGCTCGAGCGGCCGCCCGGGCAG3' (SEQ ID NO: ____)

3'GGCCCGTCCTAG5' (SEQ ID NO: ____)

Adaptor 2:

5'GTAATACGACTCACTATAGGGCAGCGTGGTCGCGGCCGAG3' (SEQ ID NO: ____)

3'CGGCTCCTAG5' (SEQ ID NO: ____)

PCR primer 1:

5'CTAATACGACTCACTATAGGGC3' (SEQ ID NO:__________________)

Nested primer (NP)1:
5'TCGAGCGGCCGCCCGGGCAGGA3' (SEQ ID NO: ________________)
Nested primer (NP)2:
5'AGCGTGGTCGCGGCCGAGGA3' (SEQ ID NO: ________________)

Suppression Subtractive Hybridization:

Suppression Subtractive Hybridization (SSH) was used to identify cDNAs corresponding to genes that are differentially expressed in prostate cancer. The SSH reaction utilized cDNA from two LAPC-9 AD xenografts. Specifically, to isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an AI phenotype and start growing again in the castrated males. Tumors were harvested at different time points after castration to identify genes that are turned on or off during the transition to androgen independence.

The gene 121P2A3 was derived from an LAPC-9 AD tumor (grown in intact male mouse) minus an LAPC-9 AD tumor (28 days post-castration) subtraction. The SSH DNA sequence 121P2A3 (Figure 1) was identified.

The cDNA derived from an LAPC-9 AD tumor (28 days post-castration) was used as the source of the "driver" cDNA, while the cDNA from the LAPC-9 AD tumor (grown in intact male mouse) was used as the source of the "tester" cDNA. Double stranded cDNAs corresponding to tester and driver cDNAs were synthesized from 2 μg of poly(A)$^+$ RNA isolated from the relevant xenograft tissue, as described above, using CLONTECH's PCR-Select cDNA Subtraction Kit and 1 ng of oligonucleotide DPNCDN as primer. First-and second-strand synthesis were carried out as described in the Kit's user manual protocol (CLONTECH Protocol No. PT1117-1, Catalog No. K1804-1). The resulting cDNA was digested with Dpn II for 3 hrs at 37˚C. Digested cDNA was extracted with phenol/chloroform (1:1) and ethanol precipitated.

Driver cDNA was generated by combining in a 1:1 ratio Dpn II digested cDNA from the relevant xenograft source (see above) with a mix of digested cDNAs derived from the human cell lines HeLa, 293, A431, Colo205, and mouse liver.

Tester cDNA was generated by diluting 1 μl of Dpn II digested cDNA from the relevant xenograft source (see above) (400 ng) in 5 μl of water. The diluted cDNA (2 μl, 160 ng) was then ligated to 2 μl of Adaptor 1 and Adaptor 2 (10 μM), in separate ligation reactions, in a total volume of 10 μl at 16˚C overnight, using 400 u of T4 DNA ligase (CLONTECH). Ligation was terminated with 1 μl of 0.2 M EDTA and heating at 72˚C for 5 min.

The first hybridization was performed by adding 1.5 μl (600 ng) of driver cDNA to each of two tubes containing 1.5 μl (20 ng) Adaptor 1- and Adaptor 2- ligated tester cDNA. In a final volume of 4 μl, the samples were overlaid with mineral oil, denatured in an MJ Research thermal cycler at 98˚C for 1.5 minutes, and then were allowed to hybridize for 8 hrs at 68˚C. The two hybridizations were then mixed together with an additional 1 μl of fresh denatured driver cDNA and were allowed to hybridize overnight at 68˚C. The second hybridization was then diluted in 200 μl of 20 mM Hepes, pH 8.3, 50 mM NaCl, 0.2 mM EDTA, heated at 70˚C for 7 min. and stored at -20˚C.

PCR Amplification Cloning and Sequencing of Gene Fragments Generated from SSH:

To amplify gene fragments resulting from SSH reactions, two PCR amplifications were performed. In the primary PCR reaction 1 μl of the diluted final hybridization mix was added to 1 μl of PCR primer 1(10 μM), 0.5 μl dNTP mix (10 μM), 2.5 μl 10 x reaction buffer (CLONTECH) and 0.5 μl 50 x Advantage cDNA polymerase Mix (CLONTECH) in a final volume of 25 μl. PCR 1 was conducted using the following conditions: 75˚C for 5 min., 94˚C for 25 sec., then 27 cycles of 94˚C for 10 sec, 66˚C for 30 sec, 72˚C for 1.5 min. Five separate primary PCR reactions were performed for each experiment. The products were pooled and diluted 1:10 with water. For the secondary PCR reaction, 1 μl from the pooled and diluted primary PCR reaction was added to the same reaction mix as used for PCR 1, except that primers NP1 and NP2 (10 μM) were used instead of PCR primer 1. PCR 2 was performed using 10-12 cycles of 94˚C for 10 sec, 68˚C for 30 sec, and 72˚C for 1.5 minutes. The PCR products were analyzed using 2% agarose gel electrophoresis.

The PCR products were inserted into pCR2.1 using the T/A vector cloning kit (Invitrogen). Transformed *E. coli* were subjected to blue/white and ampicillin selection. White colonies were picked and arrayed into 96 well plates and were grown in liquid culture overnight. To identify inserts, PCR amplification was performed on 1 ml of bacterial culture using the conditions of PCR and NP1 and NP2 as primers. PCR products were analyzed using 2% agarose gel electrophoresis.

Bacterial clones were stored in 20% glycerol in a 96 well format. Plasmid DNA was prepared, sequenced, and subjected to nucleic acid homology searches of the GenBank, dBest, and NCI-CGAP databases.

RT-PCR Expression Analysis:

First strand cDNAs can be generated from 1 μg of mRNA with oligo (dT)12-18 priming using the Gibco-BRL Superscript Preamplification system. The manufacturer's protocol was used which included an incubation for 50 min at 42°C with reverse transcriptase followed by RNAse H treatment at 37°C for 20 min. After completing the reaction, the volume can be increased to 200 μl with water prior to normalization. First strand cDNAs from 16 different normal human tissues can be obtained from Clontech.

Normalization of the first strand cDNAs from multiple tissues was'performed by using the primers 5'atatcgccgcgctcgtcgtcgacaa3' (SEQ ID NO:__________________) and 5'agccacacgcagctcattgtagaagg 3' (SEQ ID NO: __________________) to amplify β-actin. First strand cDNA (5 μl) were amplified in a total volume of 50 μl containing 0.4 μM primers, 0.2 μM each dNTPs, 1XPCR buffer (Clontech, 10 mM Tris-HCL, 1.5 mM $MgCl_2$, 50 mM KCl, pH8.3) and 1X Klentaq DNA polymerase (Clontech). Five μl of the PCR reaction can be removed at 18, 20, and 22 cycles and used for agarose gel electrophoresis. PCR was performed using an MJ Research thermal cycler under the following conditions: initial denaturation can be at 94°C for 15 sec, followed by a 18, 20, and 22 cycles of 94°C for 15, 65°C for 2 min, 72°C for 5 sec. A final extension at 72°C was carried out for 2 min. After agarose gel electrophoresis, the band intensities of the 283 b.p. β-actin bands from multiple tissues were compared by visual inspection. Dilution factors for the first strand cDNAs were calculated to result in equal β-actin band intensities in all tissues after 22 cycles of PCR. Three rounds of normalization can be required to achieve equal band intensities in all tissues after 22 cycles of PCR.

To determine expression levels of the 121P2A3 gene, 5 μl of normalized first strand cDNA were analyzed by PCR using 26, and 30 cycles of amplification. Semi-quantitative expression analysis can be achieved by comparing the PCR products at cycle numbers that give light band intensities. The primers used for RT-PCR were designed using the 121P2A3 SSH sequence and are listed below:

**121P2A3.1**
5'- TGTCAATCAAATGAGAGGGCTACA - 3' (SEQ ID NO: __________________)

**121P2A3.2**
5'- CTGTTTGAGGCATAATCTTAAGTGG - 3' (SEQ ID NO:__________________)

A typical RT-PCR expression study is shown in Figure 14. First strand cDNA was prepared from vital pool 1 (liver, lung and kidney), vital pool 2 (pancreas, colon and stomach), LAPC xenograft pool (LAPC-4AD, LAPC-4AI, LAPC-9AD and LAPC-9AI), prostate cancer pool, bladder cancer pool, kidney cancer pool, colon cancer pool lung cancer pool, ovary cancer pool, breast cancer pool, and cancer metastasis pool. Normalization was performed t PCR using primers to actin and GAPDH. Semi-quantitative PCR, using primers to 121P2A3, was performed at 26 and 30 cycles of amplification. Results show strong expression of 121P2A3 in LAPC xenograft pool, bladder cancer pool, kidney cancer pool, colon cancer pool, lung cancer pool, ovary cancer pool, breast cancer pool, and cancer metastasis pool. Expression of 121P2A3 was also detected in prostate cancer pool. Very low expression was detect in vital pool 1 and 2.

**Example 2: Full Length Cloning of 121P2A3**

To isolate genes that are involved in the progression of androgen dependent (AD) prostate cancer to androgen independent (AI) cancer, an experiment was conducted with the LAPC-9 AD xenograft in male SCID mice. Mice that harbored LAPC-9 AD xenografts were castrated when the tumors reached a size of 1 cm in diameter. The tumors regressed in size and temporarily stopped producing the androgen dependent protein PSA. Seven to fourteen days post-castration, PSA levels were detectable again in the blood of the mice. Eventually the tumors develop an AI phenotype and start growing again in the castrated males. Tumors were harvested at different time points after castration to identify genes that are turned on or off during the transition to androgen independence.

The gene 121P2A3 was derived from an LAPC-9 AD (no castration) minus LAPC-9AD (28 days post-castration) subtraction. The SSH DNA sequence (Figure 1) was designated 121P2A3. cDNA clone 121P2A3-clone 5 (Figure 4) was identified by screening an LAPC-9AD cDNA library (Lambda ZAP Express, Stratagene) using the 121P2A3 SSH DNA as a probe.

121P2A3 clone 5 cDNA was deposited under the terms of the Budapest Treaty on 1 March 2001, with the American Type Culture Collection (ATCC; 10801 University Blvd., Manassas, VA 20110-2209 USA) as plasmid 121P2A3-5, and has been assigned Accession No. PTA-3138.

**Example 3: Chromosomal Mapping of the 121P2A3 Gene**

The chromosomal localization of 121P2A3 was determined using the NCBI Human Genome web site (URL www.ncbi.nlm.nih.gov/genome/seq/page.ci?F=HsBlast,html&&ORG=Hs). The mapping program placed 121P2A3 on chromosome 10q23.32, a genomic region found to be rearranged in certain cancers.

**Example 4: Expression analysis of 121P2A3 in normal tissues, cancer cell lines and patient samples**

Analysis by RT-PCR demonstrates that 121P2A3 expression in multiple human cancer tissues (Figure 14). First strand cDNA was prepared from vital pool 1 (liver, lung and kidney), vital pool 2 (pancreas, colon and stomach), LAPC xenograft pool (LAPC-4AD, LAPC-4AI, LAPC-9AD and LAPC-9AI), prostate cancer pool, bladder cancer pool, kidney cancer pool, colon cancer pool, lung cancer pool, ovary cancer pool, breast cancer pool, and cancer metastasis pool. Normalization was performed by PCR using primers to actin and GAPDH. Semi-quantitative PCR, using primers to 121P2A3, was performed at 26 and 30 cycles of amplification. Results show strong expression of 121P2A3 in LAPC xenograft pool, bladder cancer pool, kidney cancer pool, colon cancer pool, lung cancer pool, ovary cancer pool, breast cancer pool, and cancer metastasis pool. Expression of 121P2A3 was also detected in prostate cancer pool. Very low expression was detected in vital pool 1 and 2.

Extensive northern blot analysis of 121P2A3 in 16 human normal tissues and in xenograft tissues confirms the expression observed by RT-PCR (Figure 15). Two multiple tissue northern blots (A and B; Clontech) both with 2 ug of mRNA/lane, and a LAPC xenograft blot with 10 ug of total RNA/lane (C) were probed with the 121P2A3 SSH sequence. Size standards in kilobases (kb) are indicated on the side. Results show expression of an approximately 2.7 kb121P2A3 transcript in testis. Lower level expression was also detected in thymus and colon but not in the other normal tissues tested. 121P2A3 expression is also shown in prostate cancer xenografts but not in normal prostate.

121P2A3 expression was detected in all cell lines tested (Figure 16). RNA was extracted from a number of human cancer cell lines. Northern blots with 10 ug of total RNA/lane were probed with the 121P2A3 SSH fragment. Results show expression in prostate (LAPC 4AD, LAPC 4AI, LAPC 9AD, LAPC 9AI, LNCaP, PC-3, DU145, Tsu-Pr1 and LAPC-4 CL), bladder (HT1197, SCaBER, UM-UC-3, TCCSUP, J82, 5637), 293T cell line, Ewing's sarcoma (RD-ES), pancreas (PANC-1, Bx PC-3, HPAC, Capan-1) colon (SK-CO-1, Caco-2, LoVo, T84, Colo205), breast (CAMA-1, DU4475, MCF-7, MDA-MB-435s), testicular (NTERRA-2, NCCIT, TERA-1, TERA-2), cervical (A431), ovarian (OV-1063, PA-1, SW 626), brain (PFSK-1, T98G) and bone (SK-ES-1, HOS, U-2 OS, RD-ES) cancer cell lines. These results suggest that 121P2A3 is a testis specific gene that is upregulated in multiple cancers.

Expression of 121P2A3 in patient bladder cancer specimens is shown in Figure 17. RNA was extracted from normal bladder (Nb), bladder cancer cell lines (CL; UM-UC-3, J82, SCaBER), bladder cancer patient tumors (T) and normal adjacent tissue (N). Northern blots with 10 ug of total RNA were probed with the 121P2A3 SSH sequence. Size standards in kilobases are indicated on the side. Results show expression of 121P2A3 in patient bladder cancer tissues, and in all bladder cancer cell lines tested, but not in normal bladder.

Figure 18 shows that 121P2A3 was expressed in kidney cancer patient specimens. RNA was extracted from kidney cancer cell lines (CL: 769-P, A498, SW839), normal kidney (NK), kidney cancer patient tumors (T) and their normal adjacent tissues (N). Northern blots with 10 ug of total RNA were probed with the 121P2A3 SSH sequence. Size standards in kilobases are on the side. Results show expression of 121P2A3 in patient kidney tumor tissues and in all kidney cancer cell lines tested, but not in normal kidney.

121P2A3 is also expressed in stomach, and rectum patient cancer samples (Figure 19). The expression detected in normal adjacent tissues (isolated from diseased tissues) but not in normal tissues (isolated from healthy donors) indicates that these tissues are not fully normal and that 121P2A3 can be expressed in early stage tumors. 121P2A3 was also found to be highly expressed in the nine human cancer cell lines tested, the cervical carcinoma HeLa, the CML line K562, the PML line HL-60, the melanoma line G361, the lung carcinoma line A549, the lymphoblastic leukemia line MOLT-4, the colorectal carcinoma SW480, and Burkitt's lymphoma lines Daudi and Raji.

In order to assay for androgen regulation of 121P2A3 expression, LAPC-9AD tumor cells were injected in male mice (Figure 20). When tumor reached a palpable size (0.3-0.5cm in diameter), mice were castrated and tumors harvested at different time points following castration. RNA was isolated from the xenograft tissues. Northern blots with 10 ug of total RNA/lane were probed with the 121P2A3 SSH fragment. Size standards in kilobases (kb) are indicated on the side. Results show expression of 121P2A3 is downregulated within 7 days of castration. The experimental samples were confirmed by testing for the expression of the androgen-regulated prostate cancer gene TMPRSS2, and the androgen-independent gene PHOR-1 (B). This experiment shows that, as expected, TMPRSS2 expression level goes down 7 days after castration, whereas the expression of PHOR-1 does not change. A picture of the ethidium-bromide staining of the RNA gel is also presented confirming the quality of the RNA.

121P2A3 expression is reminiscent of a cancer-testis gene. Its restricted normal tissue expression and the upregulation detected in human cancers indicate that 121P2A3 is therapeutic and prophylactic target and a diagnostic and prognostic marker for human cancers.

**Example 5: Transcript Variants of 121P2A3**

Transcript variants are variants of mature mRNA from the same gene which arise by alternative transcription or alternative splicing. Alternative transcripts are transcripts from the same gene but start transcription at different points. Splice variants are mRNA variants spliced differently from the same transcript. In eukaryotes, when a multi-exon gene is transcribed from genomic DNA, the initial RNA is spliced to produce functional mRNA, which has only exons and is used for translation into an amino acid sequence. Accordingly, a given gene can have zero to many alternative transcripts and each transcript can have zero to many splice variants. Each transcript variant has a unique exon makeup, and can have different coding and/or non-coding (5' or 3' end) portions, from the original transcript. Transcript variants can code for similar or different proteins with the same or a similar function or can encode proteins with different functions, and can be expressed in the same tissue at the same time, or in different tissues at the same time, or in the same tissue at different times, or in different tissues at different times. Proteins encoded by transcript variants can have similar or different cellular or extracellular localizations, e.g., secreted versus intracellular.

Transcript variants are identified by a variety of art-accepted methods. For example, alternative transcripts and splice variants are identified by full-length cloning experiment, or by use of full-length transcript and EST sequences. First, all human ESTs were grouped into clusters which show direct or indirect identity with each other. Second, ESTs in the same cluster were further grouped into sub-clusters and assembled into a consensus sequence. The original gene sequence is compared to the consensus sequence(s) or other full-length sequences. Each consensus sequence is a potential splice variant for that gene (see, e.g., URL www.doubletwist.com/products/c11_agentsOverview.jhtml). Even when a variant is identified that is not a full-length clone, that portion of the variant is very useful for antigen generation and for further cloning of the full-length splice variant, using techniques known in the art.

Moreover, computer programs are available in the art that identify transcript variants based on genomic sequences. Genomic-based transcript variant identification programs include FgenesH (A. Salamov and V. Solovyev, "Ab initio gene finding in Drosophila genomic DNA," Genome Research. 2000 April;10(4):516-22); Grail (URL compbio.ornl.gov/Grail-bin/EmptyGrailForm) and GenScan (URL genes.mit.edu/GENSCAN.html). For a general discussion of splice variant identification protocols see., e.g., Southan, C., A genomic perspective on human proteases, FEBS Lett. 2001 Jun 8; 498(2-3):214-8; de Souza, S.J., et al., Identification of human chromosome 22 transcribed sequences with ORF expressed sequence tags, Proc. Natl Acad Sci U S A. 2000 Nov 7; 97(23):12690-3.

To further confirm the parameters of a transcript variant, a variety of techniques are available in the art, such as full-length cloning, proteomic validation, PCR-based validation, and 5' RACE validation, etc. (see e.g., Proteomic Validation: Brennan, S.O., et al., Albumin banks peninsula: a new termination variant characterized by electrospray mass spectrometry, Biochem Biophys Acta. 1999 Aug 17; 1433(1-2):321-6; Ferranti P, et al., Differential splicing of pre-messenger RNA produces multiple forms of mature caprine alpha(sl)-casein, Eur J Biochem. 1997 Oct 1;249(1):1-7. For PCR-based Validation: Wellmann S, et al., Specific reverse transcription-PCR quantification of vascular endothelial growth factor (VEGF) splice variants by LightCycler technology, Clin Chem. 2001 Apr;47(4):654-60; Jia, H.P., et al., Discovery of new human beta-defensins using a genomics-based approach, Gene. 2001 Jan 24; 263(1-2):211-8. For PCR-based and 5' RACE Validation: Brigle, K.E., et al., Organization of the murine reduced folate carrier gene and identification of variant splice forms, Biochem Biophys Acta. 1997 Aug 7; 1353(2): 191-8).

It is known in the art that genomic regions are modulated in cancers. When the genomic region to which a gene maps is modulated in a particular cancer, the alternative transcripts or splice variants of the gene are modulated as well. Disclosed herein is that 121P2A3 has a particular expression profile related to cancer. Alternative transcripts and splice variants of 121P2A3 may also be involved in cancers in the same or different tissues, thus serving as tumor-associated markers/antigens.

The exon composition of the original transcript, designated as 121P2A3 v.1, is shown in Table LIII. Using the full-length gene and EST sequences, one transcript variant was identified, designated as 121P2A3 v.2. Compared with 121P2A3 v.1, transcript variant 121P2A3 v.2 has a shorter exon 2, as shown in Figure 12. All other exons are the same corresponding exons of 121P2A3 v.1. Theoretically, each different combination of exons in spatial order, e.g. exons 2 and 3, is a potential splice variant. Figure 12 shows the schematic alignment of exons of the two transcript variants.

Table LIV shows nucleotide sequence of the transcript variant. Table LV shows the alignment of the transcript variant with nucleic acid sequence of 121P2A3 v.1. Table LVI lays out amino acid translation of the transcript variant for the identified reading frame orientation. Table LVII displays alignments of the amino acid sequence encoded by the splice variant with that of 121P2A3 v.1.

**Example 6: Single Nucleotide Polymorphisms of 121P2A3**

A Single Nucleotide Polymorphism (SNP) is a single base pair variation in a nucleotide sequence at a specific location. At any given point of the genome, there are four possible nucleotide base pairs: A/T, C/G, G/C and T/A. Genotype refers to the specific base pair sequence of one or more locations in the genome of an individual. Haplotype refers to the base pair sequence of more than one location on the same DNA molecule (or the same chromosome in higher organisms), often in the context of one gene or in the context of several tightly linked genes. SNPs that occur on a cDNA are called cSNPs. These cSNPs may change amino acids of the protein encoded by the gene and thus change the functions of the protein. Some SNPs cause inherited diseases; others contribute to quantitative variations in phenotype and reactions to environmental factors including diet and drugs among individuals. Therefore, SNPs and/or combinations of alleles (called haplotypes) have many applications, including diagnosis of inherited diseases, determination of drug reactions and dosage, identification of genes responsible for diseases, and analysis of the genetic relationship between individuals ( P. Nowotny, J. M. Kwon and A. M. Goate, " SNP analysis to dissect human traits," Curr. Opin. Neurobiol. 2001 Oct; 11(5):637-641; M. Pirmohamed and B. K. Park, "Genetic susceptibility to adverse drug reactions," Trends Pharmacol. Sci. 2001 Jun; 22(6):298-305; J. H. Riley, C. J. Allan, E. Lai and A. Roses, " The use of single nucleotide polymorphisms in the isolation of common disease genes," Pharmacogenomics. 2000 Feb; 1(1):39-47; R. Judson, J. C. Stephens and A. Windemuth, "The predictive power of haplotypes in clinical response," Pharmacogenomics. 2000 feb; 1(1):15-26).

SNPs are identified by a variety of art-accepted methods (P. Bean, "The promising voyage of SNP target discovery," Am. Clin. Lab. 2001 Oct-Nov; 20(9):18-20; K. M. Weiss, "In search of human variation," Genome Res. 1998 Jul; 8(7):691-697; M. M. She, "Enabling large-scale pharmacogenetic studies by high-throughput mutation detection and genotyping technologies," Clin. Chem. 2001 Feb; 47(2):164-172). For example, SNPs are identified by sequencing DNA fragments that show polymorphism by gel-based methods such as restriction fragment length polymorphism (RFLP) and denaturing gradient gel electrophoresis (DGGE). They can also be discovered by direct sequencing of DNA samples pooled from different individuals or by comparing sequences from different DNA samples. With the rapid accumulation of sequence data in public and private databases, one can discover SNPs by comparing sequences using computer programs (Z. Gu, L. Hillier and P. Y. Kwok, "Single nucleotide polymorphism hunting in cyberspace," Hum. Mutat. 1998; 12(4):221-225). SNPs can be verified and genotype or haplotype of an individual can be determined by a variety of methods including direct sequencing and high throughput microarrays (P. Y. Kwok, "Methods for genotyping single nucleotide polymorphisms," Annu. Rev. Genomics Hum. Genet. 2001; 2:235-258; M. Kokoris, K. Dix, K. Moynihan, J. Mathis, B. Erwin, P. Grass, B. Hines and A. Duesterhoeft, "High-throughput SNP genotyping with the Masscode system," Mol. Diagn. 2000 Dec; 5(4):329-340).

Using the methods described above, seven SNPs were identified in the original transcript, 121P2A3 v.1, at positions 345 (C/G), 469 (G/A), 511 (A/C), 1175 (T/C), 1307 (A/T), 1478 (A/G) and 1911 (T/C). The transcripts or proteins with alternative alleles were designated as variants 121P2A3 v.3, v.4, v.5, v.6, v.7, v.8 and v.9. Figure 10 and Figure 12 show the schematic alignment of the nucleotide variants. Figure 11 shows the schematic alignment of protein variants, corresponding to nucleotide variants. Nucleotide variants that code for the same amino acid sequence as variant 1 are not shown in Figure 11. These alleles of the SNPs, though shown separately here, can occur in different combinations (haplotypes) and in any one of the transcript variants (such as 121P2A3 v.2) that contains the sequence context of the SNPs. Figure 4A and Table LVIII show detailed sequence alignments of the variant proteins; variant locations are shaded.

**Example 7: Production Of Recombinant 121p2a3 In Prokaryotic Systems**

To express recombinant 121P2A3 and 121P2A3 variants in prokaryotic cells, the full or partial length 121P2A3 and 121P2A3 variant cDNA sequences are cloned into any one of a variety of expression vectors known in the art. One or more of the following regions of 121P2A3 variants are expressed: the full length sequence presented in Figures 2 and 3, or any 8, 9, 10, 11, 12, 13, 14, 15, 16, 17, 18, 19, 20, 21, 22, 23, 24, 25, 26, 27, 28, 29, 30 or more contiguous amino acids from 121P2A3, variants, or analogs thereof.

A. *In vitro* transcription and translation constructs:

pCRII: To generate 121P2A3 sense and anti-sense RNA probes for RNA *in situ* investigations, pCRII constructs (Invitrogen, Carlsbad CA) are generated encoding either all or fragments of the 121P2A3 cDNA. The pCRII vector has Sp6 and T7 promoters flanking the insert to drive the transcription of 121P2A3 RNA for use as probes in RNA *in situ* hybridization experiments. These probes are used to analyze the cell and tissue expression of 121P2A3 at the RNA level. Transcribed 121P2A3 RNA representing the cDNA amino acid coding region of the 121P2A3 gene is used in *in vitro* translation systems such as the TnT™ Coupled Reticulolysate System (Promega, Corp., Madison, WI) to synthesize 121P2A3 protein.

B. Bacterial Constructs:

pGEX Constructs: To generate recombinant 121P2A3 proteins in bacteria that are fused to the Glutathione S-transferase (GST) protein, all or parts of the 121P2A3 cDNA protein coding sequence are cloned into the pGEX family of GST-fusion vectors (Amersham Pharmacia Biotech, Piscataway, NJ). These constructs allow controlled expression of recombinant 121P2A3 protein sequences with GST fused at the amino-terminus and a six histidine epitope (6X His) at the carboxyl-terminus. The GST and 6X His tags permit purification of the recombinant fusion protein from induced bacteria with the appropriate affinity matrix and allow recognition of the fusion protein with anti-GST and anti-His antibodies. The 6X His tag is generated by adding 6 histidine codons to the cloning primer at the 3' end, e.g., of the open reading frame (ORF). A proteolytic cleavage site, such as the Prescission™ recognition site in pGEX-6P-1, may be employed such that it permits cleavage of the GST tag from 121P2A3-related protein. The ampicillin resistance gene and pBR322 origin permits selection and maintenance of the pGEX plasmids in *E. coli.*

pMAL Constructs: To generate, in bacteria, recombinant 121P2A3 proteins that are fused to maltose-binding protein (MBP), all or parts of the 121P2A3 cDNA protein coding sequence are fused to the MBP gene by cloning into the pMAL-c2X and pMAL-p2X vectors (New England Biolabs, Beverly, MA). These constructs allow controlled expression of recombinant 121P2A3 protein sequences with MBP fused at the amino-terminus and a 6X His epitope tag at the carboxyl-terminus. The MBP and 6X His tags permit purification of the recombinant protein from induced bacteria with the appropriate affinity matrix and allow recognition of the fusion protein with anti-MBP and anti-His antibodies. The 6X His epitope tag is generated by adding 6 histidine codons to the 3' cloning primer. A Factor Xa recognition site permits cleavage of the pMAL tag from 121P2A3. The pMAL-c2X and pMAL-p2X vectors are optimized to express the recombinant protein in the cytoplasm or periplasm respectively. Periplasm expression enhances folding of proteins with disulfide bonds.

pET Constructs: To express 121P2A3 in bacterial cells, all or parts of the 121P2A3 cDNA protein coding sequence are cloned into the pET family of vectors (Novagen, Madison, WI). These vectors allow tightly controlled expression of recombinant 121P2A3 protein in bacteria with and without fusion to proteins that enhance solubility, such as NusA and thioredoxin (Trx), and epitope tags, such as 6X His and S-Tag™ that aid purification and detection of the recombinant protein. For example, constructs are made utilizing pET NusA fusion system 43.1 such that regions of the 121P2A3 protein are expressed as amino-terminal fusions to NusA.

C. Yeast Constructs:

pESC Constructs: To express 121P2A3 in the yeast species *Saccharomyces cerevisiae* for generation of recombinant protein and functional studies, all or parts of the 121P2A3 cDNA protein coding sequence are cloned into the pESC family of vectors each of which contain 1 of 4 selectable markers, HIS3, TRP1, LEU2, and URA3 (Stratagene, La Jolla, CA). These vectors allow controlled expression from the same plasmid of up to 2 different genes or cloned sequences containing either Flag™ or Myc epitope tags in the same yeast cell. This system is useful to confirm protein-protein interactions of 121P2A3. In addition, expression in yeast yields similar post-translational modifications, such as glycosylations and phosphorylations, that are found when expressed in eukaryotic cells.

pESP Constructs: To express 121P2A3 in the yeast species *Saccharomyces pombe,* all or parts of the 121P2A3 cDNA protein coding sequence are cloned into the pESP family of vectors. These vectors allow controlled high level of expression of a 121 P2A3 protein sequence that is fused at either the amino terminus or at the carboxyl terminus to GST which aids purification of the recombinant protein. A Flag™ epitope tag allows detection of the recombinant protein with anti- Flag™ antibody.

**Example 8: Production of Recombinant 121P2A3 in Eukaryotic Systems**

**A. Mammalian Constructs:**

To express recombinant 121P2A3 in eukaryotic cells, the full or partial length 121P2A3 cDNA sequences can be cloned into any one of a variety of expression vectors known in the art. One or more of the following regions of 121P2A3 are expressed in these constructs, amino acids 1 to 464 of 121P2A3 v.1, v.3, v.4, v.6, v.7 and v.8, amino acids 1 to 295 of 121P2A3 v.2, or any 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more contiguous amino acids from 121P2A3, variants, or analogs thereof. In certain embodiments a region of a specific variant of 121P2A3 is expressed that encodes an amino acid at a specific position which differs from the amino acid of any other variant found at that position. In other embodiments, a region of a variant of 121P2A3 is expressed that lies partly or entirely within a sequence that is unique to that variant.

The constructs can be transfected into any one of a wide variety of mammalian cells such as 293T cells. Transfected 293T cell lysates can be probed with the anti-121P2A3 polyclonal serum, described herein.

**pcDNA4/HisMax Constructs:** To express 121P2A3 in mammalian cells, a 121P2A3 ORF, or portions thereof, of 121P2A3 are cloned into pcDNA4/HisMax Version A (Invitrogen, Carlsbad, CA). Protein expression is driven from the cytomegalovirus (CMV) promoter and the SP16 translational enhancer. The recombinant protein has Xpress™ and six histidine (6X His) epitopes fused to the amino-teminus. The pcDNA4/HisMax vector also contains the bovine growth hormone (BGH) polyadenylation signal and transcription termination sequence to enhance mRNA stability along with the SV40 origin for episomal replication and simple vector rescue in cell lines expressing the large T antigen. The Zeocin resistance gene allows for selection of mammalian cells expressing the protein and the ampicillin resistance gene and Co1E1 origin permits selection and maintenance of the plasmid in *E. coli.*

**pcDNA3.1/MycHis Constructs:** To express 121P2A3 in mammalian cells, a 121P2A3 ORF, or portions thereof, of 121P2A3 with a consensus Kozak translation initiation site was cloned into pcDNA3.1/MycHis Version A (Invitrogen, Carlsbad, CA). Protein expression is driven from the cytomegalovirus (CMV) promoter. The recombinant protein has the myc epitope and 6X His epitope fused to the carboxyl-terminus. The pcDNA3.1/MycHis vector also contains the bovine growth hormone (BGH) polyadenylation signal and transcription termination sequence to enhance mRNA stability, along with the SV40 origin for episomal replication and simple vector rescue in cell lines expressing the large T antigen. The Neomycin resistance.gene was used, as it allows for selection of mammalian cells expressing the protein and the ampicillin resistance gene and ColE1 origin permits selection and maintenance of the plasmid in *E. coli.* Results of expression from 121P2A3.pcDNA3.1/MycHis construct are shown in Figure 21.

**pcDNA3.1/CT-GFP-TOPO Construct:** To express 121P2A3 in mammalian cells and to allow detection of the recombinant proteins using fluorescence, a 121P2A3 ORF, or portions thereof, with a consensus Kozak translation initiation site are cloned into pcDNA3.1/CT-GFP-TOPO (Invitrogen, CA). Protein expression is driven from the cytomegalovirus (CMV) promoter. The recombinant proteins have the Green Fluorescent Protein (GFP) fused to the carboxyl-terminus facilitating non-invasive, *in vivo* detection and cell biology studies. The pcDNA3.1CT-GFP-TOPO vector also contains the bovine growth hormone (BGH) polyadenylation signal and transcription termination sequence to enhance mRNA stability along with the SV40 origin for episomal replication and simple vector rescue in cell lines expressing the large T antigen. The Neomycin resistance gene allows for selection of mammalian cells that express the protein, and the ampicillin resistance gene and Co1E1 origin permits selection and maintenance of the plasmid in E. *coli.* Additional constructs with an amino-terminal GFP fusion are made in pcDNA3.1/NT-GFP-TOPO spanning the entire length of a 121P2A3 protein.

**PAPtag:** A 121P2A3 ORF, or portions thereof, is cloned into pAPtag-5 (GenHunter Corp. Nashville, TN). This construct generates an alkaline phosphatase fusion at the carboxyl-terminus of a 121P2A3 protein while fusing the IgGκ signal sequence to the amino-terminus. Constructs are also generated in which alkaline phosphatase with an amino-terminal IgGκ signal sequence is fused to the amino-terminus of a 121P2A3 protein. The resulting recombinant 121P2A3 proteins are optimized for secretion into the media of transfected mammalian cells and can be used to identify proteins such as ligands or receptors that interact with 121P2A3 proteins. Protein expression is driven from the CMV promoter and the recombinant proteins also contain myc and 6X His epitopes fused at the carboxyl-terminus that facilitates detection and purification. The Zeocin resistance gene present in the vector allows for selection of mammalian cells expressing the recombinant protein and the ampicillin resistance gene permits selection of the plasmid in *E. coli.*

**ptag5:** A 121P2A3 ORF, or portions thereof, is cloned into pTag-5. This vector is similar to pAPtag but without the alkaline phosphatase fusion. This construct generates 121P2A3 protein with an amino-terminal IgGκ signal sequence and myc and 6X His epitope tags at the carboxyl-terminus that facilitate detection and affinity purification. The resulting recombinant 121P2A3 protein is optimized for secretion into the media of transfected mammalian cells, and is used as immunogen or ligand to identify proteins such as ligands or receptors that interact with the 121P2A3 proteins. Protein expression is driven from the CMV promoter. The Zeocin resistance gene present in the vector allows for selection of mammalian cells expressing the protein, and the ampicillin resistance gene permits selection of the plasmid in *E. coli.*

**PsecFc:** A 121P2A3 ORF, or portions thereof, is also cloned into psecFc. The psecFc vector was assembled by cloning the human immunoglobulin G1 (IgG) Fc (hinge, CH2, CH3 regions) into pSecTag2 (Invitrogen, California). This construct generates an IgG1 Fc fusion at the carboxyl-terminus of the 121P2A3 proteins, while fusing the IgGK signal sequence to N-terminus. 121P2A3 fusions utilizing the murine IgG1 Fc region are also used. The resulting recombinant 121P2A3 proteins are optimized for secretion into the media of transfected mammalian cells, and can be used as immunogens or to identify proteins such as ligands or receptors that interact with 121P2A3 protein. Protein expression is driven from the CMV promoter. The hygromycin resistance gene present in the vector allows for selection of mammalian cells that express the recombinant protein, and the ampicillin resistance gene permits selection of the plasmid in *E. coli.*

**pSRα Constructs:** To generate mammalian cell lines that express 121P2A3 constitutively, 121P2A3 ORF, or portions thereof, of 121P2A3 are cloned into pSRα constructs. Amphotropic and ecotropic retroviruses are generated by transfection of pSRα constructs into the 293T-10A1 packaging line or co-transfection of pSRα and a helper plasmid (containing deleted packaging sequences) into the 293 cells, respectively. The retrovirus is used to infect a variety of mammalian cell lines, resulting in the integration of the cloned gene, 121P2A3, into the host cell-lines. Protein expression is driven from a long terminal repeat (LTR). The Neomycin resistance gene present in the vector allows for selection of mammalian cells that express the protein, and the ampicillin resistance gene and Co1E1 origin permit selection and maintenance of the plasmid in *E. coli.* The retroviral vectors can thereafter be used for infection and generation of various cell lines using, for example, PC3, NIH 3T3, TsuPr1, 293 or rat-1 cells.

Additional pSRα constructs are made that fuse an epitope tag such as the FLAG™ tag to the carboxyl-terminus of 121P2A3 sequences to allow detection using anti-Flag antibodies. For example, the FLAG™ sequence 5' gat tac aag gat gac gac gat aag 3' is added to cloning primer at the 3' end of the ORF. Additional pSRα constructs are made to produce both amino-terminal and carboxyl-terminal GFP and myc/6X His fusion proteins of the full-length 121P2A3 proteins.

<u>**Additional Viral Vectors:**</u> Additional constructs are made for viral-mediated delivery and expression of 121P2A3. High virus titer leading to high level expression of 121P2A3 is achieved in viral delivery systems such as adenoviral vectors and herpes amplicon vectors. A 121P2A3 coding sequences or fragments thereof are amplified by PCR and subcloned into the AdEasy shuttle vector (Stratagene). Recombination and virus packaging are performed according to the manufacturer's instructions to generate adenoviral vectors. Alternatively, 121P2A3 coding sequences or fragments thereof are cloned into the HSV-1 vector (Imgenex) to generate herpes viral vectors. The viral vectors are thereafter used for infection of various cell lines such as PC3, NIH 3T3, 293 or rat-1 cells.

<u>**Regulated Expression Systems:**</u> To control expression of 121P2A3 in mammalian cells, coding sequences of 121P2A3, or portions thereof, are cloned into regulated mammalian expression systems such as the T-Rex System (Invitrogen), the GeneSwitch System (Invitrogen) and the tightly-regulated Ecdysone System (Sratagene). These systems allow the study of the temporal and concentration dependent effects of recombinant 121P2A3. These vectors are thereafter used to control expression of 121P2A3 in various cell lines such as PC3, NIH 3T3, 293. or rat-1 cells.

### B. Baculovirus Expression Systems

To generate recombinant 121P2A3 proteins in a baculovirus expression system, 121P2A3 ORF, or portions thereof, are cloned into the baculovirus transfer vector pBlueBac 4.5 (Invitrogen), which provides a His-tag at the N-terminus. Specifically, pBlueBac-121P2A3 is co-transfected with helper plasmid pBac-N-Blue (Invitrogen) into SF9 (*Spodoptera frugiperda*) insect cells to generate recombinant baculovirus (see Invitrogen instruction manual for details). Baculovirus is then collected from cell supernatant and purified by plaque assay.

Recombinant 121P2A3 protein is then generated by infection of HighFive insect cells (Invitrogen) with purified baculovirus. Recombinant 121P2A3 protein can be detected using anti-121P2A3 or anti-His-tag antibody. 121P2A3 protein can be purified and used in various cell-based assays or as immunogen to generate polyclonal and monoclonal antibodies specific for 121P2A3.

## <u>Example 9 Antigenicity Profiles and Secondary Structure</u>

Figure 5, Figure 6, Figure 7, Figure 8, and Figure 9 depict graphically five amino acid profiles of 121P2A3 variants 1 and 2, each assessment available by accessing the ProtScale website (URL www.expasy.ch/cgi-bin/protscale.pl) on the ExPasy molecular biology server.

These profiles: Figure 5, Hydrophilicity, (Hopp T.P., Woods K.R, 1981. Proc. Natl. Acad. Sci. U.S.A. 78: 3824-3828); Figure 6, Hydropathicity, (Kyte J., Doolittle R.F., 1982. J. Mol. Biol. 157:105-132); Figure 7, Percentage Accessible Residues (Janin J., 1979 Nature 277:491-492); Figure 8, Average Flexibility, (Bhaskaran R., and Ponnuswamy P.K., 1988. Int. J. Pept. Protein Res. 32:242-255); Figure 9, Beta-turn (Deleage, G., Roux B. 1987 Protein Engineering 1:289-294); and optionally others available in the art, such as on the ProtScale website, were used to identify antigenic regions of the 121P2A3 protein. Each of the above amino acid profiles of 121P2A3 were generated using the following ProtScale parameters for analysis: 1) A window size of 9; 2) 100% weight of the window edges compared to the window center; and, 3) amino acid profile values normalized to lie between 0 and 1.

Hydrophilicity (Figure 5), Hydropathicity (Figure 6) and Percentage Accessible Residues (Figure 7) profiles were used to determine stretches of hydrophilic amino acids (i.e., values greater than 0.5 on the Hydrophilicity and Percentage Accessible Residues profile, and values less than 0.5 on the Hydropathicity profile). Such regions are likely to be exposed to the aqueous environment, be present on the surface of the protein, and thus available for immune recognition, such as by antibodies.

Average Flexibility (Figure 8) and Beta-turn (Figure 9) profiles determine stretches of amino acids (i.e., values greater than 0.5 on the Beta-turn profile and the Average Flexibility profile) that are not constrained in secondary structures such as beta sheets and alpha helices. Such regions are also more likely to be exposed on the protein and thus accessible to immune recognition, such as by antibodies.

Antigenic sequences of the 121P2A3 protein indicated, e.g., by the profiles set forth in Figure 5, Figure 6, Figure 7, Figure 8, and/or Figure 9 are used to prepare immunogens, either peptides or nucleic acids that encode them, to generate therapeutic and diagnostic anti-121P2A3 antibodies. The immunogen can be any 5, 6, 7, 8, 9, 10, 11, 12, 13, 14, 15, 16, 17, 18, 19, 20, 21, 22, 23, 24, 25, 30, 35, 40, 45, 50 or more than 50 contiguous amino acids, or the corresponding nucleic acids that encode them, from the 121P2A3 protein or variants listed in Figures 2 and 3. In particular, peptide immunogens of the invention can comprise, a peptide region of at least 5 amino acids of Figures 2 and 3 in any whole number increment that includes an amino acid position having a value greater than 0.5 in the Hydrophilicity profiles of Figure 5; a peptide region of at least 5 amino acids of Figures 2 and 3 in any whole number increment that includes an amino acid position having a value less than 0.5 in the Hydropathicity profile of Figures 6 ; a peptide region of at least 5 amino acids of Figures 2 and 3 in any whole number increment that includes an amino acid position having a value greater than 0.5 in the Percent Accessible Residues profiles of Figure 7; a peptide region of at least 5 amino acids of Figures 2 and 3 in any whole number increment that includes an amino acid position having a value greater than 0.5 in the Average Flexibility profiles on Figure 8; and, a peptide region of at least 5 amino acids of Figures 2 and 3 in any whole number increment that includes an amino acid position having a value greater than 0.5 in the Beta-turn profile of Figure 9. Peptide immunogens of the invention can also comprise nucleic acids that encode any of the forgoing.

All immunogens of the invention, peptide or nucleic acid, can be embodied in human unit dose form, or comprised by a composition that includes a pharmaceutical excipient compatible with human physiology.

The secondary structure of 121P2A3 protein, namely the predicted presence and location of alpha helices, extended strands, and random coils, is predicted from the primary amino acid sequence using the HNN - Hierarchical Neural Network method (Guermeur, 1997, URL pbil.ibcp.fr/cgi-bin/npsa automat.pl?page=npsa nn.html), accessed from the ExPasy molecular biology server (URL www.expasy.ch/tools/). The analysis indicates that 121P2A3 protein is composed of 63.79% alpha helix, 4.74% extended strand, and 31.47% random coil (Figure 13).

Analysis for the potential presence of transmembrane domains in the 121P2A3 variant proteins was carried out using a variety of transmembrane prediction algorithms accessed from the ExPasy molecular biology server (URL www.expasy.ch/tools/). The programs do not predict the presence of transmembrane domains in 121P2A3 protein, suggesting that that it is a soluble protein.

## Example 10: Generation of 121P2A3 Polyclonal Antibodies

Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. In addition to immunizing with a full length 121P2A3 protein variant, computer algorithms are employed in design of immunogens that, based on amino acid sequence analysis contain characteristics of being antigenic and available for recognition by the immune system of the immunized host (see the Example entitled "Antigenicity Profiles"). Such regions would be predicted to be hydrophilic, flexible, in beta-turn conformations, and be exposed on the surface of the protein (see, e.g., Figure 5, Figure 6, Figure 7, Figure 8, or Figure 9 for amino acid profiles that indicate such regions of 121P2A3 protein).

For example, recombinant bacterial fusion proteins or peptides containing hydrophilic, flexible, beta-turn regions of 121P2A3 protein are used as antigens to generate polyclonal antibodies in New Zealand White rabbits. For example, such regions include, but are not limited to, amino acids 1-38, amino acids 97-12, amino acids, 213-238, and amino acids 284-330. It is useful to conjugate the immunizing agent to a protein known to be immunogenic in the mammal being immunized. Examples of such immunogenic proteins include, but are not limited to, keyhole limpet hemocyanin (KLH), serum albumin, bovine thyroglobulin, and soybean trypsin inhibitor. In one embodiment, a peptide encoding amino acids 1-38 of 121P2A3 variant 1 is conjugated to KLH and used to immunize the rabbit. Alternatively the immunizing agent may include all or portions of the 121P2A3 variant proteins, analogs or fusion proteins thereof. For example, the 121P2A3 variant 1 amino acid sequence can be fused using recombinant DNA techniques to any one of a variety of fusion protein partners that are well known in the art, such as glutathione-S-transferase (GST) and HIS tagged fusion proteins. Such fusion proteins are purified from induced bacteria using the appropriate affinity matrix.

In one embodiment, a GST-fusion protein encoding amino acids 1-150 of 121P2A3 variant 1, is produced, purified and used as immunogen. Other recombinant bacterial fusion proteins that may be employed include maltose binding protein, LacZ, thioredoxin, NusA, or an immunoglobulin constant region (see the section entitled "Production of 121P2A3 in Prokaryotic Systems" and Current Protocols In Molecular Biology, Volume 2, Unit 16, Frederick M. Ausubul et al. eds., 1995; Linsley, P.S., Brady, W., Urnes, M., Grosmaire, L., Damle, N., and Ledbetter, L.(1991) J.Exp. Med. 174, 561-566).

In addition to bacterial derived fusion proteins, mammalian expressed protein antigens are also used. These antigens are expressed from mammalian expression vectors such as the Tag5 and Fc-fusion vectors (see the section entitled "Production of Recombinant 121P2A3 in Eukaryotic Systems"), and retain post-translational modifications such as glycosylations found in native protein. In one embodiment, amino acids 1 -464 of variant 1, is cloned into the Tag5 mammalian secretion vector. The recombinant protein is purified by metal chelate chromatography from tissue culture supernatants of 293T cells stably expressing the recombinant vector. The purified Tag5 121P2A3 protein is then used as immunogen.

During the immunization protocol, it is useful to mix or emulsify the antigen in adjuvants that enhance the immune response of the host animal. Examples of adjuvants include, but are not limited to, complete Freund's adjuvant (CFA) and MPL-TDM adjuvant (monophosphoryl Lipid A, synthetic trehalose dicorynomycolate).

In a typical protocol, rabbits are initially immunized subcutaneously with up to 200 μg, typically 100-200 μg, of fusion protein or peptide conjugated to KLH mixed in complete Freund's adjuvant (CFA). Rabbits are then injected subcutaneously every two weeks with up to 200 μg, typically 100-200 μg, of the immunogen in incomplete Freund's adjuvant (IFA). Test bleeds are taken approximately 7-10 days following each immunization and used to monitor the titer of the antiserum by ELISA.

To test reactivity and specificity of immune serum, such as the rabbit serum derived from immunization with the Tag5 -121P2A3 protein, the full-length 121P2A3 cDNA is cloned into pCDNA 3.1 myc-his expression vector (Invitrogen, see the Example entitled "Production of Recombinant 121P2A3 in Eukaryotic Systems"). After transfection of the constructs into 293T cells, cell lysates are probed with the anti-121P2A3 serum and with anti-His antibody (Santa Cruz Biotechnologies, Santa Cruz, CA) to determine specific reactivity to denatured 121P2A3 protein using the Western blot technique. Figure 21 shows expression of Myc His epitope tagged 121P2A3 variant 1 protein in 293T cells as detected by an anti-His antibody. In addition, the immune serum is tested by fluorescence microscopy, flow cytometry and immunoprecipitation against 293T and other recombinant 121P2A3-expressing cells to determine specific recognition of native protein. Western blot, immunoprecipitation, fluorescent microscopy, and flow cytometric techniques using cells that endogenously express 121P2A3 are also carried out to test reactivity and specificity.

Anti-serum from rabbits immunized with 121P2A3 variant fusion proteins, such as GST and MBP fusion proteins, are purified by depletion of antibodies reactive to the fusion partner sequence by passage over an affinity column containing the fusion partner either alone or in the context of an irrelevant fusion protein. For example, antiserum derived from a GST-121P2A3 variant 1 fusion protein encoding amino acids 1-150 is first purified by passage over a column of GST protein covalently coupled to AffiGel matrix (BioRad, Hercules, Calif.). The antiserum is then affinity purified by passage over a column composed of a MBP-fusion protein also encoding amino acids 1-150 covalently coupled to Affigel matrix. The serum is then further purified by protein G affinity chromatography to isolate the IgG fraction. Sera from other His-tagged antigens and peptide immunized rabbits as well as fusion partner depleted sera are affinity purified by passage over a column matrix composed of the original protein immunogen or free peptide.

## Example 11: Generation of 121P2A3 Monoclonal Antibodies (mAbs)

In one embodiment, therapeutic mAbs to 121P2A3 variants comprise those that react with epitopes specific for each variant protein or specific to sequences in common between the variants that would disrupt or modulate the biological function of the 121P2A3 variants, for example those that would disrupt the interaction with ligands and binding partners. Immunogens for generation of such mAbs include those designed to encode or contain the entire 121P2A3 protein variant sequence, regions of the 121P2A3 protein variants predicted to be antigenic from computer analysis of the amino acid sequence (see, e.g., Figure 5, Figure 6, Figure 7, Figure 8, or Figure 9, and the Example entitled "Antigenicity Profiles"). Immunogens include peptides, recombinant bacterial proteins, and mammalian expressed Tag 5 proteins and human and murine IgG FC fusion proteins. In addition, cells engineered to express high levels of a respective 121P2A3 variant, such as 293T-121P2A3 variant 1 or 300.19-121P2A3 variant Imurine Pre-B cells, are used to immunize mice.

To generate mAbs to a 121P2A3 variant, mice are first immunized intraperitoneally (IP) with, typically, 10-50 μg of protein immunogen or $10^7$ 121P2A3-expressing cells mixed in complete Freund's adjuvant. Mice are then subsequently immunized IP every 2-4 weeks with, typically, 10-50 μg of protein immunogen or $10^7$ cells mixed in incomplete Freund's adjuvant. Alternatively, MPL-TDM adjuvant is used in immunizations. In addition to the above protein and cell-based immunization strategies, a DNA-based immunization protocol is employed in which a mammalian expression vector encoding a 121P2A3 variant sequence is used to immunize mice by direct injection of the plasmid DNA. For example, amino acids 1-464 is cloned into the Tag5 mammalian secretion vector and the recombinant vector is used as immunogen. In another example the same amino acids are cloned into an Fc-fusion secretion vector in which the 121P2A3 variant 1 sequence is fused at the amino-terminus to an IgK leader sequence and at the carboxyl-terminus to the coding sequence of the human or murine IgG Fc region. This recombinant vector is then used as immunogen. The plasmid immunization protocols are used in combination with purified proteins expressed from the same vector and with cells expressing the respective 121P2A3 variant.

During the immunization protocol, test bleeds are taken 7-10 days following an injection to monitor titer and specificity of the immune response. Once appropriate reactivity and specificity is obtained as determined by ELISA, Western blotting, immunoprecipitation, fluorescence microscopy, and flow cytometric analyses, fusion and hybridoma generation is then carried out with established procedures well known in the art (see, e.g., Harlow and Lane, 1988).

In one embodiment for generating 121P2A3 monoclonal antibodies, a Tag5-121P2A3 variant 1 antigen encoding amino acids 1-464, is expressed and purified from stably transfected 293T cells. Balb C mice are initially immunized intraperitoneally with 25 μg of the Tag5-121P2A3 variant 1 protein mixed in complete Freund's adjuvant. Mice are subsequently immunized every two weeks with 25 μg of the antigen mixed in incomplete Freund's adjuvant for a total of three immunizations. ELISA using the Tag5 antigen determines the titer of serum from immunized mice. Reactivity and specificity of serum to full length 121P2A3 variant 1 protein is monitored by Western blotting, immunoprecipitation and flow cytometry using 293T cells transfected with an expression vector encoding the 121P2A3 variant 1 cDNA (see e.g., the Example entitled "Production of Recombinant 121P2A3 in Eukaryotic Systems" and Figure 21). Other recombinant 121P2A3 variant 1-expressing cells or cells endogenously expressing 121P2A3 variant 1 are also used, Mice showing the strongest reactivity are rested and given a final injection of Tag5 antigen in PBS and then sacrificed four days later. The spleens of the sacrificed mice are harvested and fused to SPO/2 myeloma cells using standard procedures (Harlow and Lane, 1988). Supernatants from HAT selected growth wells are screened by ELISA, Western blot, immunoprecipitation, fluorescent microscopy, and flow cytometry to identify 121P2A3 specific antibody-producing clones.

The binding affinity of a 121P2A3 monoclonal antibody is determined using standard technologies. Affinity measurements quantify the strength of antibody to epitope binding and are used to help define which 121P2A3 monoclonal antibodies preferred for diagnostic or therapeutic use, as appreciated by one of skill in the art. The BIAcore system (Uppsala, Sweden) is a preferred method for determining binding affinity. The BIAcore system uses surface plasmon resonance (SPR, Welford K. 1991, Opt. Quant. Elect. 23:1; Morton and Myszka, 1998, Methods in Enzymology 295: 268) to monitor biomolecular interactions in real time. BIAcore analysis conveniently generates association rate constants, dissociation rate constants, equilibrium dissociation constants, and affinity constants.

## Example 12: HLA Class I and Class II Binding Assays

HLA class I and class II binding assays using purified HLA molecules are performed in accordance with disclosed protocols (*e.g.,* PCT publications WO 94/20127 and WO 94/03205; Sidney et al., Current Protocols in Immunology 18.3.1 (1998); Sidney, et al., J. Immunol. 154:247 (1995); Sette, et al., Mol. Immunol. 31:813 (1994)). Briefly, purified MHC molecules (5 to 500 nM) are incubated with various unlabeled peptide inhibitors and 1-10 nM $^{125}$I-radiolabeled probe peptides as described. Following incubation, MHC-peptide complexes are separated from free peptide by gel filtration and the fraction of peptide bound is determined. Typically, in preliminary experiments, each MHC preparation is titered in the presence of fixed amounts of radiolabeled peptides to determine the concentration of HLA molecules necessary to bind 10-20% of the total radioactivity. All subsequent inhibition and direct binding assays are performed using these HLA concentrations.

Since under these conditions [label]<[HLA] and $IC_{50}$≥[HLA], the measured $IC_{50}$ values are reasonable approximations of the true $K_D$ values. Peptide inhibitors are typically tested at concentrations ranging from 120 μg/ml to 1.2 ng/ml, and are tested in two to four completely independent experiments. To allow comparison of the data obtained in different experiments, a relative binding figure is calculated for each peptide by dividing the $IC_{50}$ of a positive control for inhibition by the $IC_{50}$ for each tested peptide (typically unlabeled versions of the radiolabeled probe peptide). For database purposes, and inter-experiment comparisons, relative binding values are compiled. These values can subsequently be converted back into $IC_{50}$ nM values by dividing the $IC_{50}$ nM of the positive controls for inhibition by the relative binding of the peptide of interest. This method of data compilation is accurate and consistent for comparing peptides that have been tested on different days, or with different lots of purified MHC.

Binding assays as outlined above may be used to analyze HLA supermotif and/or HLA motif-bearing peptides (see Table IV).

## Example 13: Identification of HLA Supermotif- and Motif-Bearine CTL Candidate Epitopes

HLA vaccine compositions of the invention can include multiple epitopes. The multiple epitopes can comprise multiple HLA supermotifs or motifs to achieve broad population coverage. This example illustrates the identification and confirmation of supermotif- and motif-bearing epitopes for the inclusion in such a vaccine composition. Calculation of population coverage is performed using the strategy described below.

### Computer searches and algorithms for identification of supermotif and/or motif-bearing epitopes

The searches performed to identify the motif-bearing peptide sequences in the Example entitled "Antigenicity Profiles" and Tables V-XVIII and XXII-LI employ the protein sequence data from the gene product of 121P2A3 set forth in Figures 2 and 3; the specific peptides used to generate the tables are listed in Table LII.

Computer searches for epitopes bearing HLA Class I or Class II supermotifs or motifs are performed as follows. All translated 121P2A3 protein sequences are analyzed using a text string search software program to identify potential peptide sequences containing appropriate HLA binding motifs; such programs are readily produced in accordance with information in the art in view of known motif/supermotif disclosures. Furthermore, such calculations can be made mentally.

Identified A2-, A3-, and DR-supermotif sequences are scored using polynomial algorithms to predict their capacity to bind to specific HLA-Class I or Class II molecules. These polynomial algorithms account for the impact of different amino acids at different positions, and are essentially based on the premise that the overall affinity (or $\Delta G$) of peptide-HLA molecule interactions can be approximated as a linear polynomial function of the type:

$$\text{“}\Delta G\text{”} = a_{1i} \times a_{2i} \times a_{3i} \ldots\ldots \times a_{ni}$$

where $a_{ji}$ is a coefficient which represents the effect of the presence of a given amino acid (*j*) at a given position (*i*) along the sequence of a peptide of n amino acids. The crucial assumption of this method is that the effects at each position are essentially independent of each other (i.e., independent binding of individual side-chains). When residue *j* occurs at position *i* in the peptide, it is assumed to contribute a constant amount $j_i$ to the free energy of binding of the peptide irrespective of the sequence of the rest of the peptide.

The method of derivation of specific algorithm coefficients has been described in Gulukota et al., J. Mol. Biol. 267:1258-126, 1997; (see also Sidney et al., Human Immunol. 45:79-93, 1996; and Southwood et al., J. Immunol. 160: 3363-3373, 1998). Briefly, for all *i* positions, anchor and non-anchor alike, the geometric mean of the average relative binding (ARB) of all peptides carrying *j* is calculated relative to the remainder of the group, and used as the estimate of $j_i$. For Class II peptides, if multiple alignments are possible, only the highest scoring alignment is utilized, following an iterative procedure. To calculate an algorithm score of a given peptide in a test set, the ARB values corresponding to the sequence of the peptide are multiplied. If this product exceeds a chosen threshold, the peptide is predicted to bind. Appropriate thresholds are chosen as a function of the degree of stringency of prediction desired.

Selection of HLA-A2 supertype cross-reactive peptides

Protein sequences from 121P2A3 are scanned utilizing motif identification software, to identify 8-, 9- 10- and 11-mer sequences containing the HLA-A2-supermotif main anchor specificity. Typically, these sequences are then scored using the protocol described above and the peptides corresponding to the positive-scoring sequences are synthesized and tested for their capacity to bind purified HLA-A*0201 molecules in *vitro* (HLA-A*0201 is considered a prototype A2 supertype molecule).

These peptides are then tested for the capacity to bind to additional A2-supertype molecules (A*0202, A*0203, A*0206, and A*6802). Peptides that bind to at least three of the five A2-supertype alleles tested are typically deemed A2-supertype cross-reactive binders. Preferred peptides bind at an affinity equal to or less than 500 nM to three or more HLA-A2 supertype molecules.

Selection of HLA-A3 supermotif-bearing epitopes

The 121P2A3 protein sequence(s) scanned above is also examined for the presence of peptides with the HLA-A3-supermotif primary anchors. Peptides corresponding to the HLA A3 supermotif-bearing sequences are then synthesized and tested for binding to HLA-A*0301 and HLA-A*1101 molecules, the molecules encoded by the two most prevalent A3-supertype alleles. The peptides that bind at least one of the two alleles with binding affinities of $\leq$500 nM, often $\leq$ 200 nM, are then tested for binding cross-reactivity to the other common A3-supertype alleles (e.g., A*3101, A*3301, and A*6801) to identify those that can bind at least three of the five HLA-A3-supertype molecules tested.

Selection of HLA-B7 supermotif bearing epitopes

The 121P2A3 protein(s) scanned above is also analyzed for the presence of 8-, 9- 10-, or 11-mer peptides with the HLA-B7-supermotif. Corresponding peptides are synthesized and tested for binding to HLA-B*0702, the molecule encoded by the most common B7-supertype allele (*i.e.,* the prototype B7 supertype allele). Peptides binding B*0702 with $IC_{50}$ of $\leq$500 nM are identified using standard methods. These peptides are then tested for binding to other common B7-supertype molecules (e.g., B*3501, B*5101, B*5301, and B*5401). Peptides capable of binding to three or more of the five B7-supertype alleles tested are thereby identified.

Selection of A1 and A24 motif-bearing epitopes

To further increase population coverage, HLA-A1 and -A24 epitopes can also be incorporated into vaccine compositions. An analysis of the 121P2A3 protein can also be performed to identify HLA-A1- and A24-motif-containing sequences.

High affinity and/or cross-reactive binding epitopes that bear other motif and/or supermotifs are identified using analogous methodology.

**Example 14: Confirmation of Immunogenicity**

Cross-reactive candidate CTL A2-supermotif-bearing peptides that are identified as described herein are selected to confirm *in vitro* immunogenicity. Confirmation is performed using the following methodology:

Target Cell Lines for Cellular Screening:

The .221A2.1 cell line, produced by transferring the HLA-A2.1 gene into the HLA-A, -B, -C null mutant human B-lymphoblastoid cell line 721.221, is used as the peptide-loaded target to measure activity of HLA-A2.1-restricted CTL. This cell line is grown in RPMI-1640 medium supplemented with antibiotics, sodium pyruvate, nonessential amino acids and 10% (v/v) heat inactivated FCS. Cells that express an antigen of interest, or transfectants comprising the gene encoding the antigen of interest, can be used as target cells to confirm the ability of peptide-specific CTLs to recognize endogenous antigen.

Primary CTL Induction Cultures:

*Generation of Dendritic Cells (DC):* PBMCs are thawed in RPMI with 30 μg/ml DNAse, washed twice and resuspended in complete medium (RPMI-1640 plus 5% AB human serum, non-essential amino acids, sodium pyruvate, L-glutamine and penicillin/streptomycin). The monocytes are purified by plating 10 x $10^6$ PBMC/well in a 6-well plate. After 2 hours at 37˚C, the non-adherent cells are removed by gently shaking the plates and aspirating the supernatants. The wells are washed a total of three times with 3 ml RPMI to remove most of the non-adherent and loosely adherent cells. Three.ml of complete medium containing 50 ng/ml of GM-CSF and 1,000 U/ml of IL-4 are then added to each well. TNFα is added to the DCs on day 6 at 75 ng/ml and the cells are used for CTL induction cultures on day 7.

*Induction of CTL with DC and Peptide:* CD8+ T-cells are isolated by positive selection with Dynal immunomagnetic beads (Dynabeads® M-450) and the detacha-bead® reagent. Typically about 200-250x$10^6$ PBMC are processed to obtain 24x$10^6$ $CD8^+$ T-cells (enough for a 48-well plate culture). Briefly, the PBMCs are thawed in RPMI with 30μg/ml DNAse, washed once with PBS containing 1% human AB serum and resuspended in PBS/1% AB serum at a concentration of 20x$10^6$cells/ml. The magnetic beads are washed 3 times with PBS/AB serum, added to the cells (140μl beads/20x$10^6$ cells) and incubated for 1 hour at 4˚C with continuous mixing. The beads and cells are washed 4x with PBS/AB serum to remove the nonadherent cells and resuspended at 100x$10^6$ cells/ml (based on the original cell number) in PBS/AB serum containing 100μl/ml detacha-bead® reagent and 30 μg/ml DNAse. The mixture is incubated for 1 hour at room temperature with continuous mixing. The beads are washed again with PBS/AB/DNAse to collect the CD8+ T-cells. The DC are collected and centrifuged at 1300 rpm for 5-7 minutes, washed once with PBS with 1% BSA, counted and pulsed with 40μg/ml of peptide at a cell concentration of 1-2x$10^6$/ml in the presence of 3μg/ml $\beta_2$- microglobulin for 4 hours at 20˚C. The DC are then irradiated (4,200 rads), washed 1 time with medium and counted again.

*Setting up induction cultures:* 0.25 ml cytokine-generated DC (at 1x$10^5$ cells/ml) are co-cultured with 0.25ml of CD8+ T-cells (at 2x$10^6$ cell/ml) in each well of a 48-well plate in the presence of 10 ng/ml of IL-7. Recombinant human IL-10 is added the next day at a final concentration of 10 ng/ml and rhuman IL-2 is added 48 hours later at 10 IU/ml.

*Restimulation of the induction cultures with peptide-pulsed adherent cells:* Seven and fourteen days after the primary induction, the cells are restimulated with peptide-pulsed adherent cells. The PBMCs are thawed and washed twice with RPMI and DNAse. The cells are resuspended at 5x$10^6$ cells/ml and irradiated at ~4200 rads. The PBMCs are plated at 2x$10^6$ in 0.5 ml complete medium per well and incubated for 2 hours at 37˚C. The plates are washed twice with RPMI by tapping the plate gently to remove the nonadherent cells and the adherent cells pulsed with 10μg/ml of peptide in the presence of 3μg/ml $\beta_2$ microglobulin in 0.25ml RPMI/5%AB per well for 2 hours at 37˚C. Peptide solution from each well is aspirated and the wells are washed once with RPMI. Most of the media is aspirated from the induction cultures (CD8+ cells) and brought to 0.5 ml with fresh media. The cells are then transferred to the wells containing the peptide-pulsed adherent cells. Twenty four hours later recombinant human IL-10 is added at a final concentration of 10 ng/ml and recombinant human IL2 is added the next day and again 2-3 days later at 50IU/ml (Tsai et al., Critical Reviews in Immunology 18(1-2):65-75, 1998). Seven days later, the cultures are assayed for CTL activity in a $^{51}Cr$ release assay. In some experiments the cultures are assayed for peptide-specific recognition in the *in situ* IFNγ ELISA at the time of the second restimulation followed by assay of endogenous recognition 7 days later. After expansion, activity is measured in both assays for a side-by-side comparison.

Measurement of CTL lytic activity by $^{51}Cr$ release.

Seven days after the second restimulation, cytotoxicity is determined in a standard (5 hr) $^{51}Cr$ release assay by assaying individual wells at a single E:T. Peptide-pulsed targets are prepared by incubating the cells with 10μg/ml peptide overnight at 37˚C.

Adherent target cells are removed from culture flasks with trypsin-EDTA. Target cells are labeled with 200μCi of $^{51}Cr$ sodium chromate (Dupont, Wilmington, DE) for 1 hour at 37°C. Labeled target cells are resuspended at $10^6$ per ml and diluted 1:10 with K562 cells at a concentration of $3.3 \times 10^6$/ml (an NK-sensitive erythroblastoma cell line used to reduce non-specific lysis). Target cells (100 μl) and effectors (100μl) are plated in 96 well round-bottom plates and incubated for 5 hours at 37°C. At that time, 100 μl of supernatant are collected from each well and percent lysis is determined according to the formula:

[(cpm of the test sample- cpm of the spontaneous $^{51}Cr$ release sample)/(cpm of the maximal $^{51}Cr$ release sample- cpm of the spontaneous $^{51}Cr$ release sample)] x 100.

Maximum and spontaneous release are determined by incubating the labeled targets with 1% Triton X-100 and media alone, respectively. A positive culture is defined as one in which the specific lysis (sample-background) is 10% or higher in the case of individual wells and is 15% or more at the two highest E:T ratios when expanded cultures are assayed.

*In situ* Measurement of Human IFNγ Production as an Indicator of Peptide-specific and Endogenous Recognition

Immulon 2 plates are coated with mouse anti-human IFNγ monoclonal antibody (4 μg/ml 0.1M $NaHCO_3$, pH8.2) overnight at 4°C. The plates are washed with $Ca^{2+}$, $Mg^{2+}$-free PBS/0.05% Tween 20 and blocked with PBS/10% FCS for two hours, after which the CTLs (100 μl/well) and targets (100 μl/well) are added to each well, leaving empty wells for the standards and blanks (which received media only). The target cells, either peptide-pulsed or endogenous targets, are used at a concentration of $1 \times 10^6$ cells/ml. The plates are incubated for 48 hours at 37°C with 5% $CO_2$.

Recombinant human IFN-gamma is added to the standard wells starting at 400 pg or 1200pg/100 microliter/well and the plate incubated for two hours at 37°C. The plates are washed and 100 μl of biotinylated mouse anti-human IFN-gamma monoclonal antibody (2 microgram/ml in PBS/3%FCS/0.05% Tween 20) are added and incubated for 2 hours at room temperature. After washing again, 100 microliter HRP-streptavidin (1:4000) are added and the plates incubated for one hour at room temperature. The plates are then washed 6x with wash buffer, 100 microliter/well developing solution (TMB 1:1) are added, and the plates allowed to develop for 5-15 minutes. The reaction is stopped with 50 microliter/well 1M $H_3PO_4$ and read at OD450. A culture is considered positive if it measured at least 50 pg of IFN-gamma/well above background and is twice the background level of expression.

CTL Expansion.

Those cultures that demonstrate specific lytic activity against peptide-pulsed targets and/or tumor targets are expanded over a two week period with anti-CD3. Briefly, $5 \times 10^4$ CD8+ cells are added to a T25 flask containing the following: $1 \times 10^6$ irradiated (4,200 rad) PBMC (autologous or allogeneic) per ml, $2 \times 10^5$ irradiated (8,000 rad) EBV-transformed cells per ml, and OKT3 (anti-CD3) at 30ng per ml in RPMI-1640 containing 10% (v/v) human AB serum, non-essential amino acids, sodium pyruvate, 25μM 2-mercaptoethanol, L-glutamine and penicillin/streptomycin. Recombinant human IL2 is added 24 hours later at a final concentration of200ru/ml and every three days thereafter with fresh media at 50IU/ml. The cells are split if the cell concentration exceeds $1 \times 10^6$/ml and the cultures are assayed between days 13 and 15 at E:T ratios of 30, 10, 3 and 1:1 in the $^{51}Cr$ release assay or at $1 \times 10^6$/ml in the *in situ* IFNγ assay using the same targets as before the expansion.

Cultures are expanded in the absence of anti-$CD3^+$ as follows. Those cultures that demonstrate specific lytic activity against peptide and endogenous targets are selected and $5 \times 10^4$ $CD8^+$ cells are added to a T25 flask containing the following: $1 \times 10^6$ autologous PBMC per ml which have been peptide-pulsed with 10 μg/ml peptide for two hours at 37°C and irradiated (4,200 rad); $2 \times 10^5$ irradiated (8,000 rad) EBV-transformed cells per ml RPMI-1640 containing 10% (v/v) human AB serum, non-essential AA, sodium pyruvate, 25mM 2-ME, L-glutamine and gentamicin.

Immunogenicity of A2 supermotif-bearing peptides

A2-supermotif cross-reactive binding peptides are tested in the cellular assay for the ability to induce peptide-specific CTL in normal individuals. In this analysis, a peptide is typically considered to be an epitope if it induces peptide-specific CTLs in at least individuals, and preferably, also recognizes the endogenously expressed peptide.

Immunogenicity can also be confirmed using PBMCs isolated from patients bearing a tumor that expresses 121P2A3. Briefly, PBMCs are isolated from patients, re-stimulated with peptide-pulsed monocytes and assayed for the ability to recognize peptide-pulsed target cells as well as transfected cells endogenously expressing the antigen.

Evaluation of A*03/A11 immunogenicity

HLA-A3 supermotif-bearing cross-reactive binding peptides are also evaluated for immunogenicity using methodology analogous for that used to evaluate the immunogenicity of the HLA-A2 supermotif peptides.

Evaluation of B7 immunogenicity

Immunogenicity screening of the B7-supertype cross-reactive binding peptides identified as set forth herein are confirmed in a manner analogous to the confirmation of A2-and A3-supermotif-bearing peptides.
Peptides bearing other supermotifs/motifs, *e.g.,* HLA-A1, HLA-A24 *etc.* are also confirmed using similar methodology

**Example 15: Implementation of the Extended Supermotif to Improve the Binding Capacity of Native Epitopes by Creating Analogs**

HLA motifs and supermotifs (comprising primary and/or secondary residues) are useful in the identification and preparation of highly cross-reactive native peptides, as demonstrated herein. Moreover, the definition of HLA motifs and supermotifs also allows one to engineer highly cross-reactive epitopes by identifying residues within a native peptide sequence which can be analoged to confer upon the peptide certain characteristics, *e.g.* greater cross-reactivity within the group of HLA molecules that comprise a supertype, and/or greater binding affinity for some or all of those HLA molecules. Examples of analoging peptides to exhibit modulated binding affinity are set forth in this example.

Analoging at Primary Anchor Residues

Peptide engineering strategies are implemented to further increase the cross-reactivity of the epitopes. For example, the main anchors of A2-supermotif-bearing peptides are altered, for example, to introduce a preferred L, I, V, or M at position 2, and I or V at the C-terminus.
To analyze the cross-reactivity of the analog peptides, each engineered analog is initially tested for binding to the prototype A2 supertype allele A*0201, then, if A*0201 binding capacity is maintained, for A2-supertype cross-reactivity.
Alternatively, a peptide is confirmed as binding one or all supertype members and then analoged to modulate binding affinity to any one (or more) of the supertype members to add population coverage.
The selection of analogs for immunogenicity in a cellular screening analysis is typically further restricted by the capacity of the parent wild type (WT) peptide to bind at least weakly, *i.e.,* bind at an $IC_{50}$ of 5000nM or less, to three of more A2 supertype alleles. The rationale for this requirement is that the WT peptides must be present endogenously in sufficient quantity to be biologically relevant. Analoged peptides have been shown to have increased immunogenicity and cross-reactivity by T cells specific for the parent epitope *(see, e.g.,* Parkhurst et al., J. Immunol. 157:2539, 1996; and Pogue et al., Proc. Natl. Acad. Sci. USA 92:8166, 1995).
In the cellular screening of these peptide analogs, it is important to confirm that analog-specific CTLs are also able to recognize the wild-type peptide and, when possible, target cells that endogenously express the epitope.

Analoging of HLA-A3 and B7-supermotif-bearing peptides

Analogs of HLA-A3 supermotif-bearing epitopes are generated using strategies similar to those employed in analoging HLA-A2 supermotif-bearing peptides. For example, peptides binding to 3/5 of the A3-supertype molecules are engineered at primary anchor residues to possess a preferred residue (V, S, M, or A) at position 2.
The analog peptides are then tested for the ability to bind A*03 and A*11 (prototype A3 supertype alleles). Those peptides that demonstrate ≤ 500 nM binding capacity are then confirmed as having A3-supertype cross-reactivity.
Similarly to the A2- and A3- motif bearing peptides, peptides binding 3 or more B7-supertype alleles can be improved, where possible, to achieve increased cross-reactive binding or greater binding affinity or binding half life. B7 supermotif-bearing peptides are, for example, engineered to possess a preferred residue (V, I, L, or F) at the C-terminal primary anchor position, as demonstrated by Sidney et al. (J. Immunol. 157:3480-3490, 1996).
Analoging at primary anchor residues of other motif and/or supermotif-bearing epitopes is performed in a like manner.
The analog peptides are then be confirmed for immunogenicity, typically in a cellular screening assay. Again, it is generally important to demonstrate that analog-specific CTLs are also able to recognize the wild-type peptide and, when possible, targets that endogenously express the epitope.

Analoging at Secondary Anchor Residues

Moreover, HLA supermotifs are of value in engineering highly cross-reactive peptides and/or peptides that bind HLA molecules with increased affinity by identifying particular residues at secondary anchor positions that are associated with such properties. For example, the binding capacity of a B7 supermotif-bearing peptide with an F residue at position 1 is analyzed. The peptide is then analoged to, for example, substitute L for F at position 1. The analoged peptide is evaluated for increased binding affinity, binding half life and/or increased cross-reactivity. Such a procedure identifies analoged peptides with enhanced properties.

Engineered analogs with sufficiently improved binding capacity or cross-reactivity can also be tested for immunogenicity in HLA-B7-transgenic mice, following for example, IFA immunization or lipopeptide immunization. Analoged peptides are additionally tested for the ability to stimulate a recall response using PBMC from patients with 121P2A3-expressing tumors.

Other analoging strategies

Another form of peptide analoging, unrelated to anchor positions, involves the substitution of a cysteine with α-amino butyric acid. Due to its chemical nature, cysteine has the propensity to form disulfide bridges and sufficiently alter the peptide structurally so as to reduce binding capacity. Substitution of α-amino butyric acid for cysteine not only alleviates this problem, but has been shown to improve binding and crossbinding capabilities in some instances *(see, e.g.,* the review by Sette et al., In: Persistent Viral Infections, Eds. R. Ahmed and 1. Chen, John Wiley & Sons, England, 1999).

Thus, by the use of single amino acid substitutions, the binding properties and/or cross-reactivity of peptide ligands for HLA supertype molecules can be modulated.

**Example 16: Identification and confirmation of 121P2A3-derived sequences with HLA-DR binding motifs**

Peptide epitopes bearing an HLA class II supermotif or motif are identified and confirmed as outlined below using methodology similar to that described for HLA Class I peptides.

Selection of HLA-DR-supermotif-bearing epitopes.

To identify 121P2A3-derived, HLA class II HTL epitopes, a 121P2A3 antigen is analyzed for the presence of sequences bearing an HLA-DR-motif or supermotif. Specifically, 15-mer sequences are selected comprising a DR-supermotif, comprising a 9-mer core, and three-residue N- and C-terminal flanking regions (15 amino acids total).

Protocols for predicting peptide binding to DR molecules have been developed (Southwood et al., J. Immunol. 160:3363-3373, 1998). These protocols, specific for individual DR molecules, allow the scoring, and ranking, of 9-mer core regions. Each protocol not only scores peptide sequences for the presence of DR-supermotif primary anchors (i.e., at position 1 and position 6) within a 9-mer core, but additionally evaluates sequences for the presence of secondary anchors. Using allele-specific selection tables (see, *e.g.,* Southwood *et al., ibid.),* it has been found that these protocols efficiently select peptide sequences with a high probability of binding a particular DR molecule. Additionally, it has been found that performing these protocols in tandem, specifically those for DR1, DR4w4, and DR7, can efficiently select DR cross-reactive peptides.

The 121P2A3-derived peptides identified above are tested for their binding capacity for various common HLA-DR molecules. All peptides are initially tested for binding to the DR molecules in the primary panel: DR1, DR4w4, and DR7. Peptides binding at least two of these three DR molecules are then tested for binding to DR2w2 β1, DR2w2 β2, DR6w19, and DR9 molecules in secondary assays. Finally, peptides binding at least two of the four secondary panel DR molecules, and thus cumulatively at least four of seven different DR molecules, are screened for binding to DR4w15, DR5w11, and DR8w2 molecules in tertiary assays. Peptides binding at least seven of the ten DR molecules comprising the primary, secondary, and tertiary screening assays are considered cross-reactive DR binders. 121P2A3-derived peptides found to bind common HLA-DR alleles are of particular interest.

Selection of DR3 motif peptides

Because HLA-DR3 is an allele that is prevalent in Caucasian, Black, and Hispanic populations, DR3 binding capacity is a relevant criterion in the selection of HTL epitopes. Thus, peptides shown to be candidates may also be assayed for their DR3 binding capacity. However, in view of the binding specificity of the DR3 motif, peptides binding only to DR3 can also be considered as candidates for inclusion in a vaccine formulation.

To efficiently identify peptides that bind DR3, target 121P2A3 antigens are analyzed for sequences carrying one of the two DR3-specific binding motifs reported by Geluk et al. (J. Immunol. 152:5742-5748, 1994). The corresponding peptides are then synthesized and confirmed as having the ability to bind DR3 with an affinity of 1μM or better, i.e., less than 1 μM. Peptides are found that meet this binding criterion and qualify as HLA class II high affinity binders.

DR3 binding epitopes identified in this manner are included in vaccine compositions with DR supermotif-bearing peptide epitopes.

Similarly to the case of HLA class I motif-bearing peptides, the class II motif-bearing peptides are analoged to improve affinity or cross-reactivity. For example, aspartic acid at position 4 of the 9-mer core sequence is an optimal residue for DR3 binding, and substitution for that residue often improves DR 3 binding.

**Example 17: Immunogenicity of 121P2A3-derived HTL epitopes**

This example determines immunogenic DR supermotif- and DR3 motif-bearing epitopes among those identified using the methodology set forth herein.

Immunogenicity of HTL epitopes are confirmed in a manner analogous to the determination of immunogenicity of CTL epitopes, by assessing the ability to stimulate HTL responses and/or by using appropriate transgenic mouse models. Immunogenicity is determined by screening for: 1.) *in vitro* primary induction using normal PBMC or 2.) recall responses from patients who have 121P2A3-expressing tumors.

**Example 18: Calculation of phenotypic frequencies of HLA-supertypes in various ethnic backgrounds to determine breadth of population coverage**

This example illustrates the assessment of the breadth of population coverage of a vaccine composition comprised of multiple epitopes comprising multiple supermotifs and/or motifs.

In order to analyze population coverage, gene frequencies of HLA alleles are determined. Gene frequencies for each HLA allele are calculated from antigen or allele frequencies utilizing the binomial distribution formulae gf=1-(SQRT(1-af) (see, *e.g.,* Sidney et al., Human Immunol. 45:79-93, 1996). To obtain overall phenotypic frequencies, cumulative gene frequencies are calculated, and the cumulative antigen frequencies derived by the use of the inverse formula [af=1-(1-Cgf)$^2$].

Where frequency data is not available at the level of DNA typing, correspondence to the serologically defined antigen frequencies is assumed. To obtain total potential supertype population coverage no linkage disequilibrium is assumed, and only alleles confirmed to belong to each of the supertypes are included (minimal estimates). Estimates of total potential coverage achieved by inter-loci combinations are made by adding to the A coverage the proportion of the non-A covered population that could be expected to be covered by the B alleles considered (e.g., total=A+B*(1-A)). Confirmed members of the A3-like supertype are A3, A11, A31, A*3301, and A*6801. Although the A3-like supertype may also include A34, A66, and A*7401, these alleles were not included in overall frequency calculations. Likewise, confirmed members of the A2-like supertype family are A*0201, A*0202, A*0203, A*0204, A*0205, A*0206, A*0207, A*6802, and A*6901. Finally, the B7-like supertype-confirmed alleles are: B7, B*3501-03, B51, B*5301, B*5401, B*5501-2, B*5601, B*6701, and B*7801 (potentially also B*1401, B*3504-06, B*4201, and B*5602).

Population coverage achieved by combining the A2-, A3- and B7-supertypes is approximately 86% in five major ethnic groups. Coverage may be extended by including peptides bearing the A1 and A24 motifs. On average, A1 is present in 12% and A24 in 29% of the population across five different major ethnic groups (Caucasian, North American Black, Chinese, Japanese, and Hispanic). Together, these alleles are represented with an average frequency of 39% in these same ethnic populations. The total coverage across the major ethnicities when A1 and A24 are combined with the coverage of the A2-, A3- and B7-supertype alleles is >95%. An analogous approach can be used to estimate population coverage achieved with combinations of class II motif-bearing epitopes.

Immunogenicity studies in humans (*e.g.,* Bertoni et al., J. Clin. Invest. 100:503, 1997; Doolan et al., Immunity 7:97, 1997; and Threlkeld et al., J. Immunol. 159:1648, 1997) have shown that highly cross-reactive binding peptides are almost always recognized as epitopes. The use of highly cross-reactive binding peptides is an important selection criterion in identifying candidate epitopes for inclusion in a vaccine that is immunogenic in a diverse population.

With a sufficient number of epitopes (as disclosed herein and from the art), an average population coverage is predicted to be greater than 95% in each of five major ethnic populations. The game theory Monte Carlo simulation analysis, which is known in the art (see *e.g.,* Osborne, M.J. and Rubinstein, A. "A course in game theory" MIT Press, 1994), can be used to estimate what percentage of the individuals in a population comprised of the Caucasian, North American Black, Japanese, Chinese, and Hispanic ethnic groups would recognize the vaccine epitopes described herein. A preferred percentage is 90%. A more preferred percentage is 95%.

## Example 19: CTL Recognition Of Endogenously Processed Antigens After Priming

This example confirms that CTL induced by native or analoged peptide epitopes identified and selected as described herein recognize endogenously synthesized, *i.e.,* native antigens.

Effector cells isolated from transgenic mice that are immunized with peptide epitopes, for example HLA-A2 supermotif-bearing epitopes, are re-stimulated in *vitro* using peptide-coated stimulator cells. Six days later, effector cells are assayed for cytotoxicity and the cell lines that contain peptide-specific cytotoxic activity are further re-stimulated. An additional six days later, these cell lines are tested for cytotoxic activity on $^{51}$Cr labeled Jurkat-A2.1/K$^b$ target cells in the absence or presence of peptide, and also tested on $^{51}$Cr labeled target cells bearing the endogenously synthesized antigen, *i.e.* cells that are stably transfected with 121P2A3 expression vectors.

The results demonstrate that CTL lines obtained from animals primed with peptide epitope recognize endogenously synthesized 121P2A3 antigen. The choice of transgenic mouse model to be used for such an analysis depends upon the epitope(s) that are being evaluated. In addition to HLA-A*0201/K$^b$ transgenic mice, several other transgenic mouse models including mice with human A11, which may also be used to evaluate A3 epitopes, and B7 alleles have been characterized and others (e.g., transgenic mice for HLA-A1 and A24) are being developed. HLA-DR1 and HLA-DR3 mouse models have also been developed, which may be used to evaluate HTL epitopes.

## Example 20: Activity Of CTL-HTL Conjugated Epitopes In Transgenic Mice

This example illustrates the induction of CTLs and HTLs in transgenic mice, by use of a 121P2A3-derived CTL and HTL peptide vaccine compositions. The vaccine composition used herein comprise peptides to be administered to a patient with a 121P2A3-expressing tumor. The peptide composition can comprise multiple CTL and/or HTL epitopes. The epitopes are identified using methodology as described herein. This example also illustrates that enhanced immunogenicity can be achieved by inclusion of one or more HTL epitopes in a CTL vaccine composition; such a peptide composition can comprise an HTL epitope conjugated to a CTL epitope. The CTL epitope can be one that binds to multiple HLA family members at an affinity of 500 nM or less, or analogs of that epitope. The peptides may be lipidated, if desired.

*Immunization procedures:* Immunization of transgenic mice is performed as described (Alexander et al., J. Immunol. 159:4753-4761, 1997). For example, A2/K$^b$ mice, which are transgenic for the human HLA A2.1 allele and are used to confirm the immunogenicity of HLA-A*0201 motif- or HLA-A2 supermotif-bearing epitopes, and are primed subcutaneously (base of the tail) with a 0.1 ml of peptide in Incomplete Freund's Adjuvant, or if the peptide composition is a lipidated CTL/HTL conjugate, in DMSO/saline, or if the peptide composition is a polypeptide, in PBS or Incomplete Freund's Adjuvant. Seven days after priming, splenocytes obtained from these animals are restimulated with syngenic irradiated LPS-activated lymphoblasts coated with peptide.

*Cell lines:* Target cells for peptide-specific cytotoxicity assays are Jurkat cells transfected with the HLA-A2.1/K$^b$ chimeric gene (*e.g.,* Vitiello et al., J. Exp. Med. 173:1007, 1991)

*In vitro CTL activation:* One week after priming, spleen cells (30x10$^6$ cells/flask) are co-cultured at 37°C with syngeneic, irradiated (3000 rads), peptide coated lymphoblasts (10x10$^6$ cells/flask) in 10 ml of culture medium/T25 flask. After six days, effector cells are harvested and assayed for cytotoxic activity.

*Assay for cytotoxic activity:* Target cells (1.0 to 1.5x10$^6$) are incubated at 37°C in the presence of 200 $\mu$l of $^{51}$Cr. After 60 minutes, cells are washed three times and resuspended in R10 medium. Peptide is added where required at a concentration of 1 $\mu$g/ml. For the assay, 10$^4$ $^{51}$Cr-labeled target cells are added to different concentrations of effector cells (final volume of 200 $\mu$l) in U-bottom 96-well plates. After a six hour incubation period at 37°C, a 0.1 ml aliquot of supernatant is removed from each well and radioactivity is determined in a Micromedic automatic gamma counter. The percent specific lysis is determined by the formula: percent specific release = 100 x (experimental release - spontaneous release)/(maximum release - spontaneous release). To facilitate comparison between separate CTL assays run under the same conditions, % $^{51}$Cr release data is expressed as lytic units/10$^6$ cells. One lytic unit is arbitrarily defined as the number of effector cells required to achieve 30% lysis of 10,000 target cells in a six hour $^{51}$Cr release assay. To obtain specific lytic units/10$^6$, the lytic units/10$^6$ obtained in the absence of peptide is subtracted from the lytic units/10$^6$ obtained in the presence of peptide. For example, if 30% $^{51}$Cr release is obtained at the effector (E): target (T) ratio of 50:1 (i.e., 5x10$^5$ effector cells for 10,000 targets) in the absence of peptide and 5:1 (i.e., 5x10$^4$ effector cells for 10,000 targets) in the presence of peptide, the specific lytic units would be: $[(1/50{,}000)-(1/500{,}000)] \times 10^6 = 18$ LU.

The results are analyzed to assess the magnitude of the CTL responses of animals injected with the immunogenic CTL/HTL conjugate vaccine preparation and are compared to the magnitude of the CTL response achieved using, for example, CTL epitopes as outlined above in the Example entitled "Confirmation of Immunogenicity." Analyses similar to this may be performed to confirm the immunogenicity of peptide conjugates containing multiple CTL epitopes and/or multiple HTL epitopes. In accordance with these procedures, it is found that a CTL response is induced, and concomitantly that an HTL response is induced upon administration of such compositions.

**Example 21: Selection of CTL and HTL epitopes for inclusion in a 121P2A3-specific vaccine.**

This example illustrates a procedure for selecting peptide epitopes for vaccine compositions of the invention. The peptides in the composition can be in the form of a nucleic acid sequence, either single or one or more sequences (*i.e.*, minigene) that encodes peptide(s), or can be single and/or polyepitopic peptides.

The following principles are utilized when selecting a plurality of epitopes for inclusion in a vaccine composition. Each of the following principles is balanced in order to make the selection.

Epitopes are selected which, upon administration, mimic immune responses that are correlated with 121P2A3 clearance. The number of epitopes used depends on observations of patients who spontaneously clear 121P2A3. For example, if it has been observed that patients who spontaneously clear 121P2A3-expressing cells generate an immune response to at least three (3) epitopes from 121P2A3 antigen, then at least three epitopes should be included for HLA class I. A similar rationale is used to determine HLA class II epitopes.

Epitopes are often selected that have a binding affinity of an $IC_{50}$ of 500 nM or less for an HLA class I molecule, or for class II, an $IC_{50}$ of 1000 nM or less; or HLA Class I peptides with high binding scores from the BIMAS web site, at URL bimas.dcrt.nih.gov/.

In order to achieve broad coverage of the vaccine through out a diverse population, sufficient supermotif bearing peptides, or a sufficient array of allele-specific motif bearing peptides, are selected to give broad population coverage. In one embodiment, epitopes are selected to provide at least 80% population coverage. A Monte Carlo analysis, a statistical evaluation known in the art, can be employed to assess breadth, or redundancy, of population coverage.

When creating polyepitopic compositions, or a minigene that encodes same, it is typically desirable to generate the smallest peptide possible that encompasses the epitopes of interest. The principles employed are similar, if not the same, as those employed when selecting a peptide comprising nested epitopes. For example, a protein sequence for the vaccine composition is selected because it has maximal number of epitopes contained within the sequence, *i.e.,* it has a high concentration of epitopes. Epitopes may be nested or overlapping (*i.e.,* frame shifted relative to one another). For example, with overlapping epitopes, two 9-mer epitopes and one 10-mer epitope can be present in a 10 amino acid peptide. Each epitope can be exposed and bound by an HLA molecule upon administration of such a peptide. A multi-epitopic, peptide can be generated synthetically, recombinantly, or via cleavage from the native source. Alternatively, an analog can be made of this native sequence, whereby one or more of the epitopes comprise substitutions that alter the cross-reactivity and/or binding affinity properties of the polyepitopic peptide. Such a vaccine composition is administered for therapeutic or prophylactic purposes. This embodiment provides for the possibility that an as yet undiscovered aspect of immune system processing will apply to the native nested sequence and thereby facilitate the production of therapeutic or prophylactic immune response-inducing vaccine compositions. Additionally such an embodiment provides for the possibility of motif-bearing epitopes for an HLA makeup that is presently unknown. Furthermore, this embodiment (absent the creating of any analogs) directs the immune response to multiple peptide sequences that are actually present in 121P2A3, thus avoiding the need to evaluate any junctional epitopes. Lastly, the embodiment provides an economy of scale when producing nucleic acid vaccine compositions. Related to this embodiment, computer programs can be derived in accordance with principles in the art, which identify in a target sequence, the greatest number of epitopes per sequence length.

A vaccine composition comprised of selected peptides, when administered, is safe, efficacious, and elicits an immune response similar in magnitude to an immune response that controls or clears cells that bear or overexpress 121P2A3.

**Example 22: Construction of 'Minigene" Multi-Epitope DNA Plasmids**

This example discusses the construction of a minigene expression plasmid. Minigene plasmids may, of course, contain various configurations of B cell, CTL and/or HTL epitopes or epitope analogs as described herein.

A minigene expression plasmid typically includes multiple CTL and HTL peptide epitopes. In the present example, HLA-A2, -A3, -B7 supermotif-bearing peptide epitopes and HLA-A1 and -A24 motif-bearing peptide epitopes are used in conjunction with DR supermotif-bearing epitopes and/or DR3 epitopes. HLA class I supermotif or motif-bearing peptide epitopes derived 121P2A3, are selected such that multiple supermotifs/motifs are represented to ensure broad population coverage. Similarly, HLA class II epitopes are selected from 121P2A3 to provide broad population coverage, *i.e.* both HLA DR-1-4-7 supermotif-bearing epitopes and HLA DR-3 motif-bearing epitopes are selected for inclusion in the minigene construct. The selected CTL and HTL epitopes are then incorporated into a minigene for expression in an expression vector.

Such a construct may additionally include sequences that direct the HTL epitopes to the endoplasmic reticulum. For example, the Ii protein may be fused to one or more HTL epitopes as described in the art, wherein the CLIP sequence of the Ii protein is removed and replaced with an HLA class II epitope sequence so that HLA class II epitope is directed to the endoplasmic reticulum, where the epitope binds to an HLA class II molecules.

This example illustrates the methods to be used for construction of a minigene-bearing expression plasmid. Other expression vectors that may be used for minigene compositions are available and known to those of skill in the art.

The minigene DNA plasmid of this example contains a consensus Kozak sequence and a consensus murine kappa Ig-light chain signal sequence followed by CTL and/or HTL epitopes selected in accordance with principles disclosed herein. The sequence encodes an open reading frame fused to the Myc and His antibody epitope tag coded for by the pcDNA 3.1 Myc-His vector.

Overlapping oligonucleotides that can, for example, average about 70 nucleotides in length with 15 nucleotide overlaps, are synthesized and HPLC-purified. The oligonucleotides encode the selected peptide epitopes as well as appropriate linker nucleotides, Kozak sequence, and signal sequence. The final multiepitope minigene is assembled by extending the overlapping oligonucleotides in three sets of reactions using PCR. A Perkin/Elmer 9600 PCR machine is used and a total of 30 cycles are performed using the following conditions: 95°C for 15 sec, annealing temperature (5° below the lowest calculated Tm of each primer pair) for 30 sec, and 72°C for 1 min.

For example, a minigene is prepared as follows. For a first PCR reaction, 5 μg of each of two oligonucleotides are annealed and extended: In an example using eight oligonucleotides, i.e., four pairs of primers, oligonucleotides 1+2, 3+4, 5+6, and 7+8 are combined in 100 μl reactions containing *Pfu* polymerase buffer (1x= 10 mM KCL, 10 mM $(NH4)_2SO_4$, 20 mM Tris-chloride, pH 8.75, 2 mM $MgSO_4$, 0.1% Triton X-100, 100 μg/ml BSA), 0.25 mM each dNTP, and 2.5 U of *Pfu* polymerase. The full-length dimer products are gel-purified, and two reactions containing the product of 1+2 and 3+4, and the product of 5+6 and 7+8 are mixed, annealed, and extended for 10 cycles. Half of the two reactions are then mixed, and 5 cycles of annealing and extension carried out before flanking primers are added to amplify the full length product. The full-length product is gel-purified and cloned into pCR-blunt (Invitrogen) and individual clones are screened by sequencing.

**Example 23: The Plasmid Construct and the Degree to Which It Induces Immunogenicity.**

The degree to which a plasmid construct, for example a plasmid constructed in accordance with the previous Example, is able to induce immunogenicity is confirmed *in vitro* by determining epitope presentation by APC following transduction or transfection of the APC with an epitope-expressing nucleic acid construct. Such a study determines "antigenicity" and allows the use of human APC. The assay determines the ability of the epitope to be presented by the APC in a context that is recognized by a T cell by quantifying the density of epitope-HLA class I complexes on the cell surface. Quantitation can be performed by directly measuring the amount of peptide eluted from the APC (*see, e.g.,* Sijts et al., J. Immunol. 156:683-692, 1996; Demotz et al., Nature 342:682-684, 1989); or the number of peptide-HLA class I complexes can be estimated by measuring the amount of lysis or lymphokine release induced by diseased or transfected target cells, and then determining the concentration of peptide necessary to obtain equivalent levels of lysis or lymphokine release (*see, e.g.,* Kageyama et al., J. Immunol. 154:567-576, 1995).

Alternatively, immunogenicity is confirmed through *in vivo* injections into mice and subsequent *in vitro* assessment of CTL and HTL activity, which are analyzed using cytotoxicity and proliferation assays, respectively, as detailed *e.g.,* in Alexander et al., Immunity 1:751-761, 1994.

For example, to confirm the capacity of a DNA minigene construct containing at least one HLA-A2 supermotif peptide to induce CTLs *in vivo,* HLA-A2.1/$K^b$ transgenic mice, for example, are immunized intramuscularly with 100 μg of naked cDNA. As a means of comparing the level of CTLs induced by cDNA immunization, a control group of animals is also immunized with an actual peptide composition that comprises multiple epitopes synthesized as a single polypeptide as they would be encoded by the minigene.

Splenocytes from immunized animals are stimulated twice with each of the respective compositions (peptide epitopes encoded in the minigene or the polyepitopic peptide), then assayed for peptide-specific cytotoxic activity in a $^{51}$Cr release assay. The results indicate the magnitude of the CTL response directed against the A2-restricted epitope, thus indicating the *in vivo* immunogenicity of the minigene vaccine and polyepitopic vaccine.

It is, therefore, found that the minigene elicits immune responses directed toward the HLA-A2 supermotif peptide epitopes as does the polyepitopic peptide vaccine. A similar analysis is also performed using other HLA-A3 and HLA-B7 transgenic mouse models to assess CTL induction by HLA-A3 and HLA-B7 motif or supermotif epitopes, whereby it is also found that the minigene elicits appropriate immune responses directed toward the provided epitopes.

To confirm the capacity of a class II epitope-encoding minigene to induce HTLs *in vivo,* DR transgenic mice, or for those epitopes that cross react with the appropriate mouse MHC molecule, I-$A^b$-restricted mice, for example, are immunized intramuscularly with 100 μg of plasmid DNA. As a means of comparing the level of HTLs induced by DNA immunization, a group of control animals is also immunized with an actual peptide composition emulsified in complete Freund's adjuvant. CD4+ T cells, *i.e.* HTLs, are purified from splenocytes of immunized animals and stimulated with each of the respective compositions (peptides encoded in the minigene). The HTL response is measured using a $^3$H-thymidine incorporation proliferation assay, *(see, e.g.,* Alexander et al. Immunity 1:751-761, 1994). The results indicate the magnitude of the HTL response, thus demonstrating the *in vivo* immunogenicity of the minigene.

DNA minigenes, constructed as described in the previous Example, can also be confirmed as a vaccine in combination with a boosting agent using a prime boost protocol. The boosting agent can consist of recombinant protein (*e.g.*, Barnett et al., Aids Res. and Human Retroviruses 14, Supplement 3:S299-S309, 1998) or recombinant vaccinia, for example, expressing a minigene or DNA encoding the complete protein of interest *(see, e.g.,* Hanke et al., Vaccine 16:439-445, 1998; Sedegah et al., Proc. Natl. Acad. Sci USA 95:7648-53, 1998; Hanke and McMichael, Immunol. Letters 66:177-181, 1999; and Robinson et al., Nature Med. 5:526-34, 1999).

For example, the efficacy of the DNA minigene used in a prime boost protocol is initially evaluated in transgenic mice. In this example, A2.1/$K^b$ transgenic mice are immunized IM with 100 μg of a DNA minigene encoding the immunogenic peptides including at least one HLA-A2 supermotif-bearing peptide. After an incubation period (ranging from 3-9 weeks), the mice are boosted IP with $10^7$ pfu/mouse of a recombinant vaccinia virus expressing the same sequence encoded by the DNA minigene. Control mice are immunized with 100 μg of DNA or recombinant vaccinia without the minigene sequence, or with DNA encoding the minigene, but without the vaccinia boost. After an additional incubation period of two weeks, splenocytes from the mice are immediately assayed for peptide-specific activity in an ELISPOT assay. Additionally, splenocytes are stimulated *in vitro* with the A2-restricted peptide epitopes encoded in the minigene and recombinant vaccinia, then assayed for peptide-specific activity in an alpha, beta and/or gamma IFN ELISA.

It is found that the minigene utilized in a prime-boost protocol elicits greater immune responses toward the HLA-A2 supermotif peptides than with DNA alone. Such an analysis can also be performed using HLA-A11 or HLA-B7 transgenic mouse models to assess CTL induction by HLA-A3 or HLA-B7 motif or supermotif epitopes. The use of prime boost protocols in humans is described below in the Example entitled "Induction of CTL Responses Using a Prime Boost Protocol."

**Example 24: Peptide Compositions for Prophylactic Uses**

Vaccine compositions of the present invention can be used to prevent 121P2A3 expression in persons who are at risk for tumors that bear this antigen. For example, a polyepitopic peptide epitope composition (or a nucleic acid comprising the same) containing multiple CTL and HTL epitopes such as those selected in the above Examples, which are also selected to target greater than 80% of the population, is administered to individuals at risk for a 121P2A3-associated tumor.

For example, a peptide-based composition is provided as a single polypeptide that encompasses multiple epitopes. The vaccine is typically administered in a physiological solution that comprises an adjuvant, such as Incomplete Freunds Adjuvant. The dose of peptide for the initial immunization is from about 1 to about 50,000 μg, generally 100-5,000 μg, for a 70 kg patient. The initial administration of vaccine is followed by booster dosages at 4 weeks followed by evaluation of the magnitude of the immune response in the patient, by techniques that determine the presence of epitope-specific CTL populations in a PBMC sample. Additional booster doses are administered as required. The composition is found to be both safe and efficacious as a prophylaxis against 121P2A3-associated disease.

Alternatively, a composition typically comprising transfecting agents is used for the administration of a nucleic acid-based vaccine in accordance with methodologies known in the art and disclosed herein.

**Example 25: Polyepitopic Vaccine Compositions Derived from Native 121P2A3 Sequence**

A native 121P2A3 polyprotein sequence is analyzed, preferably using computer algorithms defined for each class I and/or class II supermotif or motif, to identify "relatively short" regions of the polyprotein that comprise multiple epitopes. The "relatively short" regions are preferably less in length than an entire native antigen. This relatively short sequence that contains multiple distinct or overlapping, "nested" epitopes can be used to generate a minigene construct. The construct is engineered to express the peptide, which corresponds to the native protein sequence. The "relatively short" peptide is generally less than 250 amino acids in length, often less than 100 amino acids in length, preferably less than 75 amino acids in length, and more preferably less than 50 amino acids in length. The protein sequence of the vaccine composition is selected because it has maximal number of epitopes contained within the sequence, *i.e.,* it has a high concentration of epitopes. As noted herein, epitope motifs may be nested or overlapping (*i.e.,* frame shifted relative to one another). For example, with overlapping epitopes, two 9-mer epitopes and one 10-mer epitope can be present in a 10 amino acid peptide. Such a vaccine composition is administered for therapeutic or prophylactic purposes.

The vaccine composition will include, for example, multiple CTL epitopes from 121P2A3 antigen and at least one HTL epitope. This polyepitopic native sequence is administered either as a peptide or as a nucleic acid sequence which encodes the peptide. Alternatively, an analog can be made of this native sequence, whereby one or more of the epitopes comprise substitutions that alter the cross-reactivity and/or binding affinity properties of the polyepitopic peptide.

The embodiment of this example provides for the possibility that an as yet undiscovered aspect of immune system processing will apply to the native nested sequence and thereby facilitate the production of therapeutic or prophylactic immune response-inducing vaccine compositions. Additionally, such an embodiment provides for the possibility of motif-bearing epitopes for an HLA makeup(s) that is presently unknown. Furthermore, this embodiment (excluding an analoged embodiment) directs the immune response to multiple peptide sequences that are actually present in native 121P2A3, thus avoiding the need to evaluate any junctional epitopes. Lastly, the embodiment provides an economy of scale when producing peptide or nucleic acid vaccine compositions.

Related to this embodiment, computer programs are available in the art which can be used to identify in a target sequence, the greatest number of epitopes per sequence length.

**Example 26: Polyepitopic Vaccine Compositions From Multiple Antigens**

The 121P2A3 peptide epitopes of the present invention are used in conjunction with epitopes from other target tumor-associated antigens, to create a vaccine composition that is useful for the prevention or treatment of cancer that expresses 121P2A3 and such other antigens. For example, a vaccine composition can be provided as a single polypeptide that incorporates multiple epitopes from 121P2A3 as well as tumor-associated antigens that are often expressed with a target cancer associated with 121P2A3 expression, or can be administered as a composition comprising a cocktail of one or more discrete epitopes. Alternatively, the vaccine can be administered as a minigene construct or as dendritic cells which have been loaded with the peptide epitopes *in vitro.*

**Example 27: Use of peptides to evaluate an immune response**

Peptides of the invention may be used to analyze an immune response for the presence of specific antibodies, CTL or HTL directed to 121P2A3. Such an analysis can be performed in a manner described by Ogg et al., Science 279:2103-2106, 1998. In this Example, peptides in accordance with the invention are used as a reagent for diagnostic or prognostic purposes, not as an immunogen.

In this example highly sensitive human leukocyte antigen tetrameric complexes ("tetramers") are used for a cross-sectional analysis of, for example, 121P2A3 HLA-A*0201-specific CTL frequencies from HLA A*0201-positive individuals at different stages of disease or following immunization comprising a 121P2A3 peptide containing an A*0201 motif. Tetrameric complexes are synthesized as described (Musey et al., N. Engl. J. Med. 337:1267, 1997). Briefly, purified HLA heavy chain (A*0201 in this example) and β2-microglobulin are synthesized by means of a prokaryotic expression system. The heavy chain is modified by deletion of the transmembrane-cytosolic tail and COOH-terminal addition of a sequence containing a BirA enzymatic biotinylation site. The heavy chain, β2-microglobulin, and peptide are refolded by dilution. The 45-kD refolded product is isolated by fast protein liquid chromatography and then biotinylated by BirA in the presence of biotin (Sigma, St. Louis, Missouri), adenosine 5' triphosphate and magnesium. Streptavidin-phycoerythrin conjugate is added in a 1:4 molar ratio, and the tetrameric product is concentrated to 1 mg/ml. The resulting product is referred to as tetramer-phycoerythrin.

For the analysis of patient blood samples, approximately one million PBMCs are centrifuged at 300g for 5 minutes and resuspended in 50 μl of cold phosphate-buffered saline. Tri-color analysis is performed with the tetramer-phycoerythrin, along with anti-CD8-Tricolor, and anti-CD38. The PBMCs are incubated with tetramer and antibodies on ice for 30 to 60 min and then washed twice before formaldehyde fixation. Gates are applied to contain >99.98% of control samples. Controls for the tetramers include both A*0201-negative individuals and A*0201 -positive non-diseased donors. The percentage of cells stained with the tetramer is then determined by flow cytometry. The results indicate the number of cells in the PBMC sample that contain epitope-restricted CTLs, thereby readily indicating the extent of immune response to the 121P2A3 epitope, and thus the status of exposure to 121P2A3, or exposure to a vaccine that elicits a protective or therapeutic response.

**Example 28: Use of Peptide Epitopes to Evaluate Recall Responses**

The peptide epitopes of the invention are used as reagents to evaluate T cell responses, such as acute or recall responses, in patients. Such an analysis may be performed on patients who have recovered from 121P2A3-associated disease or who have been vaccinated with a 121P2A3 vaccine.

For example, the class I restricted CTL response of persons who have been vaccinated may be analyzed. The vaccine may be any 121P2A3 vaccine. PBMC are collected from vaccinated individuals and HLA typed. Appropriate peptide epitopes of the invention that, optimally, bear supermotifs to provide cross-reactivity with multiple HLA supertype family members, are then used for analysis of samples derived from individuals who bear that HLA type.

PBMC from vaccinated individuals are separated on Ficoll-Histopaque density gradients (Sigma Chemical Co., St. Louis, MO), washed three times in HBSS (GIBCO Laboratories), resuspended in RPMI-1640 (GIBCO Laboratories) supplemented with L-glutamine (2mM), penicillin (50U/ml), streptomycin (50μ g/ml), and Hepes (10mM) containing 10% heat-inactivated human AB serum (complete RPMI) and plated using microculture formats. A synthetic peptide comprising an epitope of the invention is added at 10 μg/ml to each well and HBV core 128-140 epitope is added at 1 μg/ml to each

well as a source ofT cell help during the first week of stimulation.

In the microculture format, 4 x $10^5$ PBMC are stimulated with peptide in 8 replicate cultures in 96-well round bottom plate in 100 μl/well of complete RPMI. On days 3 and 10, 100 μl of complete RPMI and 20 U/ml final concentration of rIL-2 are added to each well. On day 7 the cultures are transferred into a 96-well flat-bottom plate and restimulated with peptide, rIL-2 and $10^5$ irradiated (3,000 rad) autologous feeder cells. The cultures are tested for cytotoxic activity on day 14. A positive CTL response requires two or more of the eight replicate cultures to display greater than 10% specific $^{51}$Cr release, based on comparison with non-diseased control subjects as previously described (Rehermann, et al., Nature Med. 2:1104,1108, 1996; Rehermann et al., J. Clin. Invest. 97:1655-1665, 1996; and Rehermann et al. J. Clin. Invest. 98:1432-1440, 1996).

Target cell lines are autologous and allogeneic EBV-transformed B-LCL that are either purchased from the American Society for Histocompatibility and Immunogenetics (ASHI, Boston, MA) or established from the pool of patients as described (Guilhot, et al. J. Virol. 66:2670-2678, 1992).

Cytotoxicity assays are performed in the following manner. Target cells consist of either allogeneic HLA-matched or autologous EBV-transformed B lymphoblastoid cell line that are incubated overnight with the synthetic peptide epitope of the invention at 10 μM, and labeled with 100 μCi of $^{51}$Cr (Amersham Corp., Arlington Heights, IL) for 1 hour after which they are washed four times with HBSS.

Cytolytic activity is determined in a standard 4-h, split well $^{51}$Cr release assay using U-bottomed 96 well plates containing 3,000 targets/well. Stimulated PBMC are tested at effector/target (E/T) ratios of 20-50:1 on day 14. Percent cytotoxicity is determined from the formula: 100 x [(experimental release-spontaneous release)/maximum release-spontaneous release)]. Maximum release is determined by lysis of targets by detergent (2% Triton X-100; Sigma Chemical Co., St. Louis, MO). Spontaneous release is <25% of maximum release for all experiments.

The results of such an analysis indicate the extent to which HLA-restricted CTL populations have been stimulated by previous exposure to 121P2A3 or a 121P2A3 vaccine.

Similarly, Class II restricted HTL responses may also be analyzed. Purified PBMC are cultured in a 96-well flat bottom plate at a density of 1.5x$10^5$ cells/well and are stimulated with 10 μg/ml synthetic peptide of the invention, whole 121P2A3 antigen, or PHA. Cells are routinely plated in replicates of 4-6 wells for each condition. After seven days of culture, the medium is removed and replaced with fresh medium containing 10U/ml IL-2. Two days later, 1 μCi $^3$H-thymidine is added to each well and incubation is continued for an additional 18 hours. Cellular DNA is then harvested on glass fiber mats and analyzed for $^3$H-thymidine incorporation. Antigen-specific T cell proliferation is calculated as the ratio of $^3$H-thymidine incorporation in the presence of antigen divided by the $^3$H-thymidine incorporation in the absence of antigen.

## Example 29: Induction Of Specific CTL Response In Humans

A human clinical trial for an immunogenic composition comprising CTL and HTL epitopes of the invention is set up as an IND Phase I, dose escalation study and carried out as a randomized, double-blind, placebo-controlled trial. Such a trial is designed, for example, as follows:

A total of about 27 individuals are enrolled and divided into 3 groups:

Group I: 3 subjects are injected with placebo and 6 subjects are injected with 5 μg of peptide composition;
Group II: 3 subjects are injected with placebo and 6 subjects are injected with 50 μg peptide composition;
Group III: 3 subjects are injected with placebo and 6 subjects are injected with 500 μg of peptide composition.

After 4 weeks following the first injection, all subjects receive a booster inoculation at the same dosage.

The endpoints measured in this study relate to the safety and tolerability of the peptide composition as well as its immunogenicity. Cellular immune responses to the peptide composition are an index of the intrinsic activity of this the peptide composition, and can therefore be viewed as a measure of biological efficacy. The following summarize the clinical and laboratory data that relate to safety and efficacy endpoints.

Safety: The incidence of adverse events is monitored in the placebo and drug treatment group and assessed in terms of degree and reversibility.

Evaluation of Vaccine Efficacy: For evaluation of vaccine efficacy, subjects are bled before and after injection. Peripheral blood mononuclear cells are isolated from fresh heparinized blood by Ficoll-Hypaque density gradient centrifugation, aliquoted in freezing media and stored frozen. Samples are assayed for CTL and HTL activity.

The vaccine is found to be both safe and efficacious.

**Example 30: Phase II Trials In Patients Expressing 121P2A3**

Phase II trials are performed to study the effect of administering the CTL-HTL peptide compositions to patients having cancer that expresses 121P2A3. The main objectives of the trial are to determine an effective dose and regimen for inducing CTLs in cancer patients that express 121P2A3, to establish the safety of inducing a CTL and HTL response in these patients, and to see to what extent activation of CTLs improves the clinical picture of these patients, as manifested, e.g., by the reduction and/or shrinking of lesions. Such a study is designed, for example, as follows:

The studies are performed in multiple centers. The trial design is an open-label, uncontrolled, dose escalation protocol wherein the peptide composition is administered as a single dose followed six weeks later by a single booster shot of the same dose. The dosages are 50, 500 and 5,000 micrograms per injection. Drug-associated adverse effects (severity and reversibility) are recorded.

There are three patient groupings. The first group is injected with 50 micrograms of the peptide composition and the second and third groups with 500 and 5,000 micrograms of peptide composition, respectively. The patients within each group range in age from 21-65 and represent diverse ethnic backgrounds. All of them have a tumor that expresses 121P2A3,

Clinical manifestations or antigen-specific T-cell responses are monitored to assess the effects of administering the peptide compositions. The vaccine composition is found to be both safe and efficacious in the treatment of 121P2A3-associated disease.

**Example 31: Induction of CTL Responses Using a Prime Boost Protocol**

A prime boost protocol similar in its underlying principle to that used to confirm the efficacy of a DNA vaccine in transgenic mice, such as described above in the Example entitled "The Plasmid Construct and the Degree to Which It Induces Immunogenicity," can also be used for the administration of the vaccine to humans. Such a vaccine regimen can include an initial administration of, for example, naked DNA followed by a boost using recombinant virus encoding the vaccine, or recombinant protein/polypeptide or a peptide mixture administered in an adjuvant.

For example, the initial immunization may be performed using an expression vector, such as that constructed in the Example entitled "Construction of "Minigene" Multi-Epitope DNA Plasmids" in the form of naked nucleic acid administered IM (or SC or ID) in the amounts of 0.5-5 mg at multiple sites. The nucleic acid (0.1 to 1000 $\mu$g) can also be administered using a gene gun. Following an incubation period of 3-4 weeks, a booster dose is then administered. The booster can be recombinant fowlpox virus administered at a dose of 5-$10^7$ to $5x10^9$ pfu. An alternative recombinant virus, such as an MVA, canarypox, adenovirus, or adeno-associated virus, can also be used for the booster, or the polyepitopic protein or a mixture of the peptides can be administered. For evaluation of vaccine efficacy, patient blood samples are obtained before immunization as well as at intervals following administration of the initial vaccine and booster doses of the vaccine. Peripheral blood mononuclear cells are isolated from fresh heparinized blood by Ficoll-Hypaque density gradient centrifugation, aliquoted in freezing media and stored frozen. Samples are assayed for CTL and HTL activity.

Analysis of the results indicates that a magnitude of response sufficient to achieve a therapeutic or protective immunity against 121P2A3 is generated.

**Example 32: Administration of Vaccine Compositions Using Dendritic Cells (DC)**

Vaccines comprising peptide epitopes of the invention can be administered using APCs, or "professional" APCs such as DC. In this example, peptide-pulsed DC are administered to a patient to stimulate a CTL response *in vivo.* In this method, dendritic cells are isolated, expanded, and pulsed with a vaccine comprising peptide CTL and HTL epitopes of the invention. The dendritic cells are infused back into the patient to elicit CTL and HTL responses *in vivo.* The induced CTL and HTL then destroy or facilitate destruction, respectively, of the target cells that bear the 121P2A3 protein from which the epitopes in the vaccine are derived.

For example, a cocktail of epitope-comprising peptides is administered *ex vivo* to PBMC, or isolated DC therefrom. A pharmaceutical to facilitate harvesting of DC can be used, such as Progenipoietin™ (Monsanto, St. Louis, MO) or GM-CSF/IL-4. After pulsing the DC with peptides, and prior to reinfusion into patients, the DC are washed to remove unbound peptides.

As appreciated clinically, and readily determined by one of skill based on clinical outcomes, the number of DC reinfused into the patient can vary (*see, e.g.,* Nature Med. 4:328, 1998; Nature Med. 2:52, 1996 and Prostate 32:272, 1997). Although 2-50 x $10^6$ DC per patient are typically administered, larger number of DC, such as $10^7$ or $10^8$ can also be provided. Such cell populations typically contain between 50-90% DC.

In some embodiments, peptide-loaded PBMC are injected into patients without purification of the DC. For example, PBMC generated after treatment with an agent such as Progenipoietin™ are injected into patients without purification of the DC. The total number of PBMC that are administered often ranges from $10^8$ to $10^{10}$. Generally, the cell doses injected into patients is based on the percentage of DC in the blood of each patient, as determined, for example, by immunofluorescence analysis with specific anti-DC antibodies. Thus, for example, if Progenipoietin™ mobilizes 2% DC in the peripheral blood of a given patient, and that patient is to receive 5 x $10^6$ DC, then the patient will be injected with a total of 2.5 x $10^8$ peptide-loaded PBMC. The percent DC mobilized by an agent such as Progenipoietin™ is typically estimated to be between 2-10%, but can vary as appreciated by one of skill in the art.

*Ex vivo* activation of CTL/HTL responses

Alternatively, *ex vivo* CTL or HTL responses to 121P2A3 antigens can be induced by incubating, in tissue culture, the patient's, or genetically compatible, CTL or HTL precursor cells together with a source of APC, such as DC, and immunogenic peptides. After an appropriate incubation time (typically about 7-28 days), in which the precursor cells are activated and expanded into effector cells, the cells are infused into the patient, where they will destroy (CTL) or facilitate destruction (HTL) of their specific target cells, *i.e.,* tumor cells.

**Example 33: An Alternative Method of Identifying and Confirming Motif-Bearing Peptides**

Another method of identifying and confirming motif-bearing peptides is to elute them from cells bearing defined MHC molecules. For example, EBV transformed B cell lines used for tissue typing have been extensively characterized to determine which HLA molecules they express. In certain cases these cells express only a single type of HLA molecule. These cells can be transfected with nucleic acids that express the antigen of interest, e.g. 121P2A3. Peptides produced by endogenous antigen processing of peptides produced as a result of transfection will then bind to HLA molecules within the cell and be transported and displayed on the cell's surface. Peptides are then eluted from the HLA molecules by exposure to mild acid conditions and their amino acid sequence determined, *e.g.,* by mass spectral analysis (*e.g.,* Kubo et al., J. Immunol. 152:3913, 1994). Because the majority of peptides that bind a particular HLA molecule are motif-bearing, this is an alternative modality for obtaining the motif-bearing peptides correlated with the particular HLA molecule expressed on the cell.

Alternatively, cell lines that do not express endogenous HLA molecules can be transfected with an expression construct encoding a single HLA allele. These cells can then be used as described, *i.e.,* they can then be transfected with nucleic acids that encode 121P2A3 to isolate peptides corresponding to 121P2A3 that have been presented on the cell surface. Peptides obtained from such an analysis will bear motif(s) that correspond to binding to the single HLA allele that is expressed in the cell.

As appreciated by one in the art, one can perform a similar analysis on a cell bearing more than one HLA allele and subsequently determine peptides specific for each HLA allele expressed. Moreover, one of skill would also recognize that means other than transfection, such as loading with a protein antigen, can be used to provide a source of antigen to the cell.

**Example 34: Complementary Polynucleotides**

Sequences complementary to the 121P2A3-encoding sequences, or any parts thereof, are used to detect, decrease, or inhibit expression of naturally occurring 121P2A3. Although use of oligonucleotides comprising from about 15 to 30 base pairs is described, essentially the same procedure is used with smaller or with larger sequence fragments. Appropriate oligonucleotides are designed using, e.g., OLIGO 4.06 software (National Biosciences) and the coding sequence of 121P2A3. To inhibit transcription, a complementary oligonucleotide is designed from the most unique 5' sequence and used to prevent promoter binding to the coding sequence. To inhibit translation, a complementary oligonucleotide is designed to prevent ribosomal binding to a 121P2A3-encoding transcript.

**Example 35: Purification of Naturally-occurring or Recombinant 121P2A3 Using 121P2A3-Specific Antibodies**

Naturally occurring or recombinant 121P2A3 is substantially purified by immunoaffinity chromatography using antibodies specific for 121P2A3. An immunoaffinity column is constructed by covalently coupling anti-121P2A3 antibody to an activated chromatographic resin, such as CNBr-activated SEPHAROSE (Amersham Pharmacia Biotech). After the coupling, the resin is blocked and washed according to the manufacturer's instructions.

Media containing 121P2A3 are passed over the immunoaffinity column, and the column is washed under conditions that allow the preferential absorbance of 121P2A3 (e.g., high ionic strength buffers in the presence of detergent). The column is eluted under conditions that disrupt antibody/121P2A3 binding (e.g., a buffer of pH 2 to pH 3, or a high concentration of a chaotrope, such as urea or thiocyanate ion), and GCR.P is collected.

## Example 36: Identification of Molecules Which Interact with 121P2A3

121P2A3, or biologically active fragments thereof, are labeled with 121 1 Bolton-Hunter reagent (See, e.g., Bolton et al. (1973) Biochem. J. 133:529.) Candidate molecules previously arrayed in the wells of a multi-well plate are incubated with the labeled 121P2A3, washed, and any wells with labeled 121P2A3 complex are assayed. Data obtained using different concentrations of 121P2A3 are used to calculate values for the number, affinity, and association of 121P2A3 with the candidate molecules.

## Example 37: *In Vivo* Assay for 121P2A3 Tumor Growth Promotion

The effect of the 121P2A3 protein on tumor cell growth is evaluated in *vivo* by evaluating tumor development and growth of cells expressing or lacking 121P2A3. For example, SCID mice are injected subcutaneously on each flank with 1 x $10^6$ of either bladder, kidney, breast or prostate cancer cell lines (e.g. SCABER, J82, 769P, A498) that endogenously express 121P2A3, or with 3T3 or prostate cancer cells such as LNCa cells containing tkNeo empty vector or 121P2A3. At least two strategies may be used: (1) Constitutive 121P2A3 expression under regulation of a promoter such as a constitutive promoter obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), or from heterologous mammalian promoters, *e.g.*, the actin promoter or an immunoglobulin promoter, provided such promoters are compatible with the host cell systems, and (2) Regulated expression under control of an inducible vector system, such as ecdysone, tetracycline, etc., provided such promoters are compatible with the host cell systems. Tumor volume is then monitored by caliper measurement at the appearance of palpable tumors and followed over time to determine if 121P2A3-expressing cells grow at a faster rate and whether tumors produced by 121P2A3-expressing cells demonstrate characteristics of altered aggressiveness (e.g. enhanced metastasis, vascularization, reduced responsiveness to chemotherapeutic drugs).

Additionally, mice can be implanted with 1 x $10^5$ of the same cells orthotopically to determine if 121P2A3 has an effect on local growth in the bladder, kidney or prostate, and whether 121P2A3 affects the ability of the cells to metastasize, specifically to lymph nodes, adrenal tissue, liver and bone (Mild T et al, Oncol Res. 2001;12:209; Fu X et al, Int J Cancer. 1991, 49:938; Kiguchi Ket al, Clin Exp Metastasis. 1998, 16:751).

The assay is also useful to determine the 121P2A3 inhibitory effect of candidate therapeutic compositions, such as for example, 121P2A3 intrabodies, 121P2A3 antisense molecules and ribozymes.

## Example 38: 121P2A3 Monoclonal Antibody-mediated Inhibition of Bladder, Kidney and Prostate Tumors *In Vivo*

The significant expression of 121P2A3 in cancer tissues, together with its restrictive expression in normal tissues makes 121P2A3 a good target for antibody therapy. Similarly, 121P2A3 is a target for T cell-based immunotherapy. Thus, the therapeutic efficacy of anti-121P2A3 mAbs in human bladder cancer xenograft mouse models is evaluated by using recombinant cell lines such as SCABER and J82 (see, e.g., Kaighn, M.E., et al., Invest Urol, 1979. 17(1): p. 16-23). Similarly, anti-121P2A3 mAbs are evaluated in human kidney and prostate cancer xenograft models using recombinant cell lines such as A498, LNCaP-121P2A3 and 3T3-121P2A3.

Antibody efficacy on tumor growth and metastasis formation is studied, e.g., in a mouse orthotopic bladder cancer xenograft model, kidney and prostate cancer xenograft models. The antibodies can be unconjugated, as discussed in this Example, or can be conjugated to a therapeutic modality, as appreciated in the art. Anti-121P2A3 mAbs inhibit formation of kidney, ovarian and bladder xenografts. Anti-121P2A3 mAbs also retard the growth of established orthotopic tumors and prolonged survival of tumor-bearing mice. These results indicate the utility of anti-121P2A3 mAbs in the treatment of local and advanced stages of prostate, kidney and bladder cancer. (See, e.g., Saffran, D., et al., PNAS 10: 1073-1078 or URL www.pnas.org/cgi/doi/10.1073/Rnas.051624698)

Administration of the anti-121P2A3 mAbs led to retardation of established orthotopic tumor growth and inhibition of metastasis to distant sites, resulting in a significant prolongation in the survival of tumor-bearing mice. These studies indicate that 121P2A3 is an attractive target for immunotherapy and demonstrate the therapeutic potential of anti-121P2A3 mAbs for the treatment of local and metastatic cancer. This example demonstrates that unconjugated 121P2A3 monoclonal antibodies are effective to inhibit the growth of human bladder, kidney and prostate tumor xenografts grown in SCID mice; accordingly a combination of such efficacious monoclonal antibodies is also effective.

**Tumor inhibition using multiple unconjugated 121P2A3 mAbs**

*Materials and Methods*

121P2A3 Monoclonal Antibodies:

Monoclonal antibodies are raised against 121P2A3 as described in the Example entitled "Generation of 121P2A3 Monoclonal Antibodies (mAbs)." The antibodies are characterized by ELISA, Western blot, FACS, and immunoprecipitation for their capacity to bind 121P2A3. Epitope mapping data for the anti-121P2A3 mAbs, as determined by ELISA and Western analysis, recognize epitopes on the 121P2A3 protein. Immunohistochemical analysis of prostate cancer tissues and cells with these antibodies is performed.

The monoclonal antibodies are purified from ascites or hybridoma tissue culture supernatants by Protein-G Sepharose chromatography, dialyzed against PBS, filter sterilized, and stored at -20˚C. Protein determinations are performed by a Bradford assay (Bio-Rad, Hercules, CA). A therapeutic monoclonal antibody or a cocktail comprising a mixture of individual monoclonal antibodies is prepared and used for the treatment of mice receiving subcutaneous or orthotopic injections of SCABER, J82, A498, 769P, CaOvl or PA1 tumor xenografts.

Cell Lines

The bladder and kidney carcinoma cell lines, SCABER, J82, A498, 769P, as well as the fibroblast line NIH 3T3 (American Type Culture Collection) are maintained in DMEM supplemented with L-glutamine and 10% FBS. The prostate carcinoma cell line LNCaP is grown in RPMI supplemented with L-glutamine and 10% FBS. LNCaP-121P2A3 and 3T3-121P2A3 cell populations are generated by retroviral gene transfer as described in Hubert, R.S., et al., Proc Natl Acad Sci U S A, 1999. 96(25): 14523.

Xenograft Mouse Models.

The LAPC-9 xenograft, which expresses a wild-type androgen receptor and produces prostate-specific antigen (PSA), is passaged in 6- to 8-week-old male ICR-severe combined immunodeficient (SCID) mice (Taconic Farms) by s.c. trocar implant (Craft, N., *et al.,* supra).

Subcutaneous (s.c.) tumors are generated by injection of 1 x $10^6$ cancer cells mixed at a 1:1 dilution with Matrigel (Collaborative Research) in the right flank of male SCID mice. To test antibody efficacy on tumor formation, i.p. antibody injections are started on the same day as tumor-cell injections. As a control, mice are injected with either purified mouse IgG (ICN) or PBS; or a purified monoclonal antibody that recognizes an irrelevant antigen not expressed in human cells. Tumor sizes are determined by caliper measurements, and the tumor volume is calculated as length x width x height. Mice with s.c. tumors greater than 1.5 cm in diameter are sacrificed.

Orthotopic injections are performed under anesthesia by using ketamine/xylazine. For prostate orthotopic studies, an incision is made through the abdominal muscles to expose the bladder and seminal vesicles, which then are delivered through the incision to expose the dorsal prostate. LAPC-9 and LNCaP cells (5 x 105) mixed with Matrigel are injected into each dorsal lobe in a 10 μl volume. To monitor tumor growth, mice are bled on a weekly basis for determination of PSA levels. For bladder orthotopic studies, an incision is made through the abdomen to expose the bladder, and tumor cells (5 x $10^5$) mixed with Matrigel are injected into the bladder wall in a 10-μl volume. To monitor tumor growth, mice are palpated and blood is collected on a weekly basis to measure BTA levels. For kidney orthopotic models, an incision is made through the abdominal muscles to expose the kidney. Tumor cells mixed with Matrigel are injected under the kidney capsule in a 10 μl volume (Yoshida Y et al, Anticancer Res. 1998, 18:327; Ahn et al, Tumour Biol. 2001, 22:146). Tumor growth is monitored by measuring. The mice are segregated into groups for the appropriate treatments, with anti-121P2A3 or control mAbs being injected i.p.

Anti-121P2A3 mAbs Inhibit Growth of 121P2A3-Expressing Xenograft-Cancer Tumors

The effect of anti-121P2A3 mAbs on tumor formation is tested on the growth and progression of bladder, kidney and prostate cancer xenografts using cell lines and LAPC orthotopic models. As compared with the s.c. tumor model, the orthotopic model, which requires injection of tumor cells directly in the mouse bladder, kidney and ovary, respectively, results in a local tumor growth, development of metastasis in distal sites, deterioration of mouse health, and subsequent death (Saffran, D., et al., PNAS supra; Fu, X., et al., Int J Cancer, 1992. 52(6): p. 987-90; Kubota, T., J Cell Biochem, 1994. 56(1): p. 4-8). The features make the orthotopic model more representative of human disease progression and allowed us to follow the therapeutic effect of mAbs on clinically relevant end points.

Accordingly, tumor cells are injected into the mouse bladder, kidney or prostate, and 2 days later, the mice are segregated into two groups and treated with either: a) 200-500μg, of anti-121P2A3 Ab, or b) PBS three times per week for two to five weeks.

A major advantage of the orthotopic cancer models is the ability to study the development of metastases. Formation of metastasis in mice bearing established orthotopic tumors is studied by IHC analysis on lung sections using an antibody against a tumor-specific cell-surface protein such as anti-CK20 for bladder cancer, anti-G250 for kidney cancer and STEAP-1 antibody for prostate cancer models (Lin S et al, Cancer Detect Prev. 2001;25:202; McCluggage W et al, Histopathol 2001, 38:542).

Mice bearing established orthotopic tumors are administered 1000μg injections of either anti-121P2A3 mAb or PBS over a 4-week period. Mice in both groups are allowed to establish a high tumor burden, to ensure a high frequency of metastasis formation in mouse lungs. Mice then are killed and their bladders, livers, bone and lungs are analyzed for the presence of tumor cells by IHC analysis.

These studies demonstrate a broad anti-tumor efficacy of anti-121P2A3 antibodies on initiation and progression of prostate and kidney cancer in xenograft mouse models. Anti-121P2A3 antibodies inhibit tumor formation of tumors as well as retarding the growth of already established tumors and prolong the survival of treated mice. Moreover, anti-121P2A3 mAbs demonstrate a dramatic inhibitory effect on the spread of local bladder, kidney and prostate tumor to distal sites, even in the presence of a large tumor burden. Thus, anti-121P2A3 mAbs are efficacious on major clinically relevant end points (tumor growth), prolongation of survival, and health.

## Example 39: Therapeutic and Diagnostic use of Anti-121P2A3 Antibodies in Humans.

Anti-121P2A3 monoclonal antibodies are safely and effectively used for diagnostic, prophylactic, prognostic and/or therapeutic purposes in humans. Western blot and immunohistochemical analysis of cancer tissues and cancer xenografts with anti-121P2A3 mAb show strong extensive staining in carcinoma but significantly lower or undetectable levels in normal tissues. Detection of 121P2A3 in carcinoma and in metastatic disease demonstrates the usefulness of the mAb as a diagnostic and/or prognostic indicator. Anti-121P2A3 antibodies are therefore used in diagnostic applications such as immunohistochemistry of kidney biopsy specimens to detect cancer from suspect patients.

As determined by flow cytometry, anti-121P2A3 mAb specifically binds to carcinoma cells. Thus, anti-121P2A3 antibodies are used in diagnostic whole body imaging applications, such as radioimmunoscintigraphy and radioimmunotherapy, (see, e.g., Potamianos S., et. al. Anticancer Res 20(2A):925-948 (2000)) for the detection of localized and metastatic cancers that exhibit expression of 121P2A3. Shedding or release of an extracellular domain of 121P2A3 into the extracellular milieu, such as that seen for alkaline phosphodiesterase B10 (Meerson, N. R., Hepatology 27:563-568 (1998)), allows diagnostic detection of 121P2A3 by anti-121P2A3 antibodies in serum and/or urine samples from suspect patients.

Anti-121P2A3 antibodies that specifically bind 121P2A3 are used in therapeutic applications for the treatment of cancers that express 121P2A3. Anti-121P2A3 antibodies are used as an unconjugated modality and as conjugated form in which the antibodies are attached to one of various therapeutic or imaging modalities well known in the art, such as a prodrugs, enzymes or radioisotopes. In preclinical studies, unconjugated and conjugated anti-121P2A3 antibodies are tested for efficacy of tumor prevention and growth inhibition in the SCID mouse cancer xenograft models, e.g., kidney cancer models AGS-K3 and AGS-K6, (see, e.g., the Example entitled "121P2A3 Monoclonal Antibody-mediated Inhibition of Bladder, Kidney and Ovarian Tumors *In Vivo*"). Conjugated and unconjugated anti-121P2A3 antibodies are used as a therapeutic modality in human clinical trials either alone or in combination with other treatments as described in following Examples.

## Example 40: Human Clinical Trials for the Treatment and Diagnosis of Human Carcinomas through use of Human Anti-121P2A3 Antibodies *In vivo*

Antibodies are used in accordance with the present invention which recognize an epitope on 121P2A3, and are used in the treatment of certain tumors such as those listed in Table I. Based upon a number of factors, including 121P2A3 expression levels, tumors such as those listed in Table I are presently preferred indications. In connection with each of these indications, three clinical approaches are successfully pursued.

I.) Adjunctive therapy: In adjunctive therapy, patients are treated with anti-121P2A3 antibodies in combination with a chemotherapeutic or antineoplastic agent and/or radiation therapy. Primary cancer targets, such as those listed in Table I, are treated under standard protocols by the addition anti-121P2A3 antibodies to standard first and second line therapy. Protocol designs address effectiveness as assessed by reduction in tumor mass as well as the ability to reduce usual doses of standard chemotherapy. These dosage reductions allow additional and/or prolonged therapy by reducing dose-related toxicity of the chemotherapeutic agent. Anti-121P2A3 antibodies are utilized in several adjunctive clinical trials in combination with the chemotherapeutic or antineoplastic agents adriamycin (advanced prostrate carcinoma), cisplatin (advanced head and neck and lung carcinomas), taxol (breast cancer), and doxorubicin (preclinical).

II.) Monotherapy: In connection with the use of the anti-121P2A3 antibodies in monotherapy of tumors, the antibodies are administered to patients without a chemotherapeutic or antineoplastic agent. In one embodiment, monotherapy is conducted clinically in end stage cancer patients with extensive metastatic disease. Patients show some disease stabilization. Trials demonstrate an effect in refractory patients with cancerous tumors.

III.) Imaging Agent: Through binding a radionuclide (e.g., iodine or yttrium ($I^{131}$, $Y^{90}$) to anti-121P2A3 antibodies, the radiolabeled antibodies are utilized as a diagnostic and/or imaging agent. In such a role, the labeled antibodies localize to both solid tumors, as well as, metastatic lesions of cells expressing 121P2A3. In connection with the use of the anti-121P2A3 antibodies as imaging agents, the antibodies are used as an adjunct to surgical treatment of solid tumors, as both a pre-surgical screen as well as a postoperative follow-up to determine what tumor remains and/or returns. In one embodiment, a ($^{111}In$)-121P2A3 antibody is used as an imaging agent in a Phase I human clinical trial in patients having a carcinoma that expresses 121P2A3 (by analogy see, *e.g.,* Divgi et al. J. Natl. Cancer Inst. 83:97-104 (1991)). Patients are followed with standard anterior and posterior gamma camera. The results indicate that primary lesions and metastatic lesions are identified

Dose and Route of Administration

As appreciated by those of ordinary skill in the art, dosing considerations can be determined through comparison with the analogous products that are in the clinic. Thus, anti-121P2A3 antibodies can be administered with doses in the range of 5 to 400 mg/m$^2$, with the lower doses used, e.g., in connection with safety studies. The affinity ef anti-121P2A3 antibodies relative to the affinity of a known antibody for its target is one parameter used by those of skill in the art for determining analogous dose regimens. Further, anti-121P2A3 antibodies that are fully human antibodies, as compared to the chimeric antibody, have slower clearance; accordingly, dosing in patients with such fully human anti-121P2A3 antibodies can be lower, perhaps in the range of 50 to 300 mg/m$^2$, and still remain efficacious. Dosing in mg/m$^2$, as opposed to the conventional measurement of dose in mg/kg, is a measurement based on surface area and is a convenient dosing measurement that is designed to include patients of all sizes from infants to adults.

Three distinct delivery approaches are useful for delivery of anti-121P2A3 antibodies. Conventional intravenous delivery is one standard delivery technique for many tumors. However, in connection with tumors in the peritoneal cavity, such as tumors of the ovaries, biliary duct, other ducts, and the like, intraperitoneal administration may prove favorable for obtaining high dose of antibody at the tumor and to also minimize antibody clearance. In a similar manner, certain solid tumors possess vasculature that is appropriate for regional perfusion. Regional perfusion allows for a high dose of antibody at the site of a tumor and minimizes short term clearance of the antibody.

Clinical Development Plan (CDP)

Overview: The CDP follows and develops treatments of anti-121P2A3 antibodies in connection with adjunctive therapy, monotherapy, and as an imaging agent. Trials initially demonstrate safety and thereafter confirm efficacy in repeat doses. Trails are open label comparing standard chemotherapy with standard therapy plus anti-121P2A3 antibodies. As will be appreciated, one criteria that can be utilized in connection with enrollment of patients is 121P2A3 expression levels in their tumors as determined by biopsy.

As with any protein or antibody infusion-based therapeutic, safety concerns are related primarily to (i) cytokine release syndrome, i.e., hypotension, fever, shaking, chills; (ii) the development of an immunogenic response to the material (i.e., development of human antibodies by the patient to the antibody therapeutic, or HAHA response); and, (iii) toxicity to normal cells that express 121P2A3. Standard tests and follow-up are utilized to monitor each of these safety concerns. Anti-121P2A3 antibodies are found to be safe upon human administration.

**Example 41: Human Clinical Trial Adjunctive Therapy with Human Anti-121P2A3**

**Antibody and Chemotherapeutic Agent**

A phase I human clinical trial is initiated to assess the safety of six intravenous doses of a human anti-121P2A3 antibody in connection with the treatment of a solid tumor, e.g., a cancer of a tissue listed in Table I. In the study, the safety of single doses of anti-121P2A3 antibodies when utilized as an adjunctive therapy to an antineoplastic or chemotherapeutic agent, such as cisplatin, topotecan, doxorubicin, adriamycin, taxol, or the like, is assessed. The trial design includes delivery of six single doses of an anti-121P2A3 antibody with dosage of antibody escalating from approximately about 25 mg/m$^2$ to about 275 mg/m$^2$ over the course of the treatment in accordance with the following schedule:

| | Day 0 | Day 7 | Day 14 | Day 21 | Day 28 | Day 35 |
|---|---|---|---|---|---|---|
| mAb Dose | 25 mg/$m^2$ | 75 mg/$m^2$ | 125 mg/$m^2$ | 175 mg/$m^2$ | 225 mg/$m^2$ | 275 mg/$m^2$ |
| Chemotherapy (standard dose) | + | + | + | + | + | + |

Patients are closely followed for one-week following each administration of antibody and chemotherapy. In particular, patients are assessed for the safety concerns mentioned above: (i) cytokine release syndrome, i.e., hypotension, fever, shaking, chills; (ii) the development of an immunogenic response to the material (i.e., development of human antibodies by the patient to the human antibody therapeutic, or HAHA response); and, (iii) toxicity to normal cells that express 121P2A3. Standard tests and follow-up are utilized to monitor each of these safety concerns. Patients are also assessed for clinical outcome, and particularly reduction in tumor mass as evidenced by MRI or other imaging.

The anti-121P2A3 antibodies are demonstrated to be safe and efficacious, Phase II trials confirm the efficacy and refine optimum dosing.

**Example 42: Human Clinical Trial: Monotherapy with Human Anti-121P2A3 Antibody**

Anti-121P2A3 antibodies are safe in connection with the above-discussed adjunctive trial, a Phase II human clinical trial confirms the efficacy and optimum dosing for monotherapy. Such trial is accomplished, and entails the same safety and outcome analyses, to the above-described adjunctive trial with the exception being that patients do not receive chemotherapy concurrently with the receipt of doses of anti-121P2A3 antibodies.

**Example 43: Human Clinical Trial: Diagnostic Imagine with Anti-121P2A3 Antibody**

Once again, as the adjunctive therapy discussed above is safe within the safety criteria discussed above, a human clinical trial is conducted concerning the use of anti-121P2A3 antibodies as a diagnostic imaging agent. The protocol is designed in a substantially similar manner to those described in the art, such as in Divgi et al. J. Natl. Cancer Inst. 83:97-104 (1991). The antibodies are found to be both safe and efficacious when used as a diagnostic modality.

**Example 44: Homology Comparison of 121P2A3 to Known Sequences**

Several protein variants of 121P2A3 have been identified, with 121P2A3-v.1, -v.3 to -v.6 differing by one amino acid from each other, while 121P2A3-v.2 represents a truncated version of 121P2A3-v.1 and missing the corresponding first 169 aa from its N-terminus. The 121P2A3-v.1 protein has 464 amino acids with calculated molecular weight of 54.1 kDa, and pI of 6.5. All 121P2A3 variants are predicted to be cytoplasmic proteins, with a lower possibility of nuclear localization.

121P2A3 shows homology to a human cloned gene identified as RIKEN cDNA 1200008012 gene (gi 14745180), with 99% identity and 99% homology to that gene (see Figure 4E). 121P2A3 also shows homology to a putative mouse protein of unknown function, specifically FLJ10540 (gi 12835981), with 75% identity and 86% homology (see Figure 4H), as well as the corresponding human protein (see Figure 4D and Example 1). The 121P2A3 protein shows distinct homology to the mouse rho/rac interacting citron kinase (gi 3599509), with 20% identity and 41% homology (see Figure 4I), as well as the human Naf-1 beta protein (nef associated factor gi 5174609), with 23% identity and 40% homology (see Figure 4G).

Naf-1 stands for Nef-associated factor-1, which affects gene expression in mammalian cells. In particular, it regulates the expression of CD4 proteins in T lymphocytes (Fukushi M et al. Febs 1999, 442:83). Naf-1 also mediates unspliced RNA nucleocytoplasmic transport, and nuclear import/export of HIV-1 gag (Gupta, K. et al., 2000, J. Virol, 74: 11811). By transporting unspliced RNA to the cytoplasm, naf-1 can control expression of RNA transcript splice variants. Nef is a viral protein that is involved in the control of AIDS progression. Nef binds to a variety of protein kinases and adaptor molecules, thereby regulating the activation of several signaling pathways (Briggs SD et al, J Biol Chem. 1997, 272:17899; Briggs SD et al, J Biol Chem. 2001, 276: 13847; Baur AS et al, Immunity. 1997, 6:283.). Nef has been shown to regulate cell growth, apoptosis, cell survival and transformation (Xu XN, Screaton G. Nat Immunol. 2001, 2:384; Briggs SD et al, J Biol Chem. 2001 276:13847; Kramer-Hammerle S et al, AIDS Res Hum Retroviruses. 2001, 17:597). The Rho/Rac interacting citron kinase is a serine/threonine kinase of approximately 240-kDa. The protein consists of a kinase domain followed by a Rho/Rac binding motif which plays a role in protein interactions (Di Cunto F et al, J Biol Chem 1998 273: 29706).

Motif analysis revealed the presence of a CTF/NF-1 motif in all 121P2A3 variants, located at 38 and 219 relative to 121P2A3-v.1 start methionine. Nuclear factor I (NF-I) is a transcription factor that homodimerizes and binds specific DNA sequences (Mermod N et al, Cell 1989, 58:741). The CTF/NF-I proteins activate transcription and DNA replication.

Accordingly, when 121P2A3 functions as a regulator of signal transduction, protein interactions, as a transcription factor involved in activating genes involved in tumorigenesis or in controlling cell growth and apoptosis, 121P2A3 is used for therapeutic, diagnostic, prognostic or preventative purposes.

**Example 45: Identification of Potential Signal Transduction Pathways**

Many mammalian proteins have been reported to interact with signaling molecules and to participate in regulating signaling pathways. (J Neurochem. 2001; 76:217-223). In particular, Nef has been reported to associate with various kinases and transcription factors. It has also been reported to activate the NFbB pathway (Heyninck, K. et al. 1999 J. Cell. Biol., 145, 1471). Using immunoprecipitation and Western blotting techniques, proteins are identified that associate with 121P2A3 and mediate signaling events. Several pathways known to play a role in cancer biology can be regulated by 121P2A3, including phospholipid pathways such as PI3K, AKT, etc, adhesion and migration pathways, including FAK, Rho, Rac-1, etc, as well as mitogenic/survival cascades such as ERK, p38, etc (Cell Growth Differ. 2000,11:279; J Biol Chem. 1999, 274:801; Oncogene. 2000, 19:3003, J. Cell Biol. 1997, 138:913.).

Using, e.g., Western blotting techniques the ability of 121P2A3 to regulate these pathways is examined. Cells expressing or lacking 121P2A3 are either left untreated or stimulated with cytokines, androgen and anti-integrin antibodies. Cell lysates are analyzed using anti-phospho-specific antibodies (Cell Signaling, Santa Cruz Biotechnology) in order to detect phosphorylation and regulation of ERK, p38, AKT, PI3K, PLC and other signaling molecules. When 121P2A3 plays a role in the regulation of signaling pathways, whether individually or communally, it is used as a target for diagnostic, prognostic, preventative and therapeutic purposes.

To determine that 121P2A3 directly or indirectly activates known signal transduction pathways in cells, luciferase (luc) based transcriptional reporter assays are carried out in cells expressing individual genes. These transcriptional reporters contain consensus-binding sites for known transcription factors that lie downstream of well-characterized signal transduction pathways. The reporters and examples of these associated transcription factors, signal transduction pathways, and activation stimuli are listed below.

NFkB-luc, NFIcB/Rel; Ik-kinaselSAPK; growth/apoptosis/stress
SRE-luc, SRF/TCF/ELKI; MAPK/SAPK; growth/differentiation
AP-1-luc, FOS/JUN; MAPK/SAPK/PKC; growth/apoptosis/stress
ARE-luc, androgen receptor; steroids/MAPK; growth/differentiation/apoptosis
p53-luc, p53; SAPK; growth/differentiation/apoptosis
CRE-luc, CREB/ATF2; PKA/p38; growth/apoptosis/stress

Gene-mediated effects can be assayed in cells showing mRNA expression. Luciferase reporter plasmids can be introduced by lipid-mediated transfection (TFX-50, Promega). Luciferase activity, an indicator of relative transcriptional activity, is measured by incubation of cell extracts with luciferin substrate and luminescence of the reaction is monitored in a luminometer. Moreover, the 121P2A3 protein contains several phosphorylation sites (Table XX), indicating its association with specific signaling cascades.

Signaling pathways activated by 121P2A3 are mapped and used for the identification and validation of therapeutic targets. When 121P2A3 is involved in cell signaling, it is used as target for diagnostic, prognostic, preventative and therapeutic purposes.

**Example 46: Involvement in Tumor Progression**

The 121P2A3 gene can contribute to the growth of cancer cells. The role of 121P2A3 in tumor growth is investigated in a variety of primary and transfected cell lines including prostate, colon, bladder and kidney cell lines as well as NIH 3T3 cells engineered to stably express 121P2A3. Parental cells lacking 121P2A3 and cells expressing 121P2A3 are evaluated for cell growth using a well-documented proliferation assay (Fraser SP, Grimes JA, Djamgoz MB. Prostate. 2000;44:61, Johnson DE, Ochieng J, Evans SL. Anticancer Drugs. 1996, 7:288).

To determine the role of 121P2A3 in the transformation process, its effect in colony forming assays is investigated. Parental NIH3T3 cells lacking 121P2A3 are compared to NHI-3T3 cells expressing 121P2A3, using a soft agar assay under stringent and more permissive conditions (Song Z. et al. Cancer Res. 2000;60:6730).

To determine the role of 121P2A3 in invasion and metastasis of cancer cells, a well-established assay is used, e.g., a Transwell Insert System assay (Becton Dickinson) (Cancer Res. 1999; 59:6010). Control cells, including prostate, colon, bladder and kidney cell lines lacking 121P2A3 are compared to cells expressing 121P2A3. Cells are loaded with the fluorescent dye, calcein, and plated in the top well of the Transwell insert coated with a basement membrane analog.

Invasion is determined by fluorescence of cells in the lower chamber relative to the fluorescence of the entire cell population.

121P2A3 can also play a role in cell cycle and apoptosis. Parental cells and cells expressing 121P2A3 are compared for differences in cell cycle regulation using a well-established BrdU assay (Abdel-Malek ZA. J Cell Physiol. 1988, 136:247). In short, cells are grown under both optimal (full serum) and limiting (low serum) conditions, then are labeled with BrdU and stained with anti-BrdU Ab and propidium iodide. Cells are analyzed for entry into the G1, S, and G2M phases of the cell cycle. Alternatively, the effect of stress on apoptosis is evaluated in control parental cells and cells expressing 121P2A3, including normal and tumor prostate, colon and lung cells. Engineered and parental cells are treated with various chemotherapeutic agents, such as etoposide, flutamide, etc, and protein synthesis inhibitors, such as cycloheximide. Cells are stained with annexin V-FITC and cell death is measured by FACS analysis. The modulation of cell death by 121P2A3 can play a critical role in regulating tumor progression and tumor load.

When 121P2A3 plays a role in cell growth, transformation, invasion or apoptosis, it is used as a target for diagnostic, prognostic, preventative and therapeutic purposes.

## Example 47: Involvement in Angiogenesis

Angiogenesis or new capillary blood vessel formation is necessary for tumor growth (Hanahan D, Folkman J. Cell. 1996, 86:353; Folkman J. Endocrinology. 1998 139:441). Several assays have been developed to measure angiogenesis *in vitro* and *in vivo,* such as the tissue culture assays endothelial cell tube formation and endothelial cell proliferation. Using these assays as well as *in vitro* neo-vascularization, it is determined whether 121P2A3 enhances or inhibits angiogenesis.

For example, endothelial cells engineered to express 121P2A3 are evaluated using tube formation and proliferation assays. The effect of 121P2A3 can also be evaluated in animal models *in vivo.* For example, cells either expressing or lacking 121P2A3 are implanted subcutaneously in immunocompromised mice. Endothelial cell migration and angiogenesis are evaluated 5-15 days later using immunohistochemistry techniques. When 121P2A3 affects angiogenesis, it is used as a target for diagnostic, prognostic, preventative and therapeutic purposes

## Example 48: Regulation of Transcription

The localization of 121P2A3 in the nucleus and its similarity to NAF-1 indicate that 121P2A3 plays a role in the transcriptional regulation of eukaryotic genes. Regulation of gene expression is evaluated, e.g., by studying gene expression in cells expressing or lacking 121P2A3. For this purpose, two types of experiments are performed.

In the first set of experiments, RNA from parental and 121P2A3-expressing cells are extracted and hybridized to commercially available gene arrays (Clontech) (Smid-Koopman E et al. Br J Cancer. 2000. 83:246). Resting cells as well as cells treated with FBS or androgen are compared. Differentially expressed genes are identified in accordance with procedures known in the art. The differentially expressed genes are then mapped to biological pathways (Chen K et al. Thyroid. 2001. 11:41.).

In the second set of experiments, specific transcriptional pathway activation is evaluated using commercially available (Stratagene) luciferase reporter constructs including: NFkB-luc, SRE-luc, ELK1-luc, ARE-luc, p53-luc, and CRE-luc. These transcriptional reporters contain consensus binding sites for known transcription factors that lie downstream of well-characterized signal transduction pathways, and represent a good tool to ascertain pathway activation and screen for positive and negative modulators of pathway activation.

When 121P2A3 plays a role in gene regulation, it is used as a target for diagnostic, prognostic, preventative and therapeutic purposes.

## Example 49: Involvement in Cell Adhesion

Cell adhesion plays a critical role in tissue colonization and metastasis. Based on its homology to CLIP-190, 121P2A3 can participate in cellular organization, and as a consequence cell adhesion and motility. To determine that 121P2A3 regulates cell adhesion, control cells lacking 121P2A3 are compared to cells expressing 121P2A3, using techniques previously described (see, e.g., Haier et al, Br. J. Cancer. 1999, 80:1867; Lehr and Pienta, J. Natl. Cancer Inst. 1998, 90:118). Briefly, in one embodiment, cells labeled with a fluorescent indicator, such as calcein, are incubated on tissue culture wells coated with media alone or with matrix proteins. Adherent cells are detected by fluorimetric analysis and percent adhesion is calculated. In another embodiment, cells lacking or expressing 121P2A3 are analyzed for their ability to mediate cell-cell adhesion using similar experimental techniques as described above. Both of these experimental systems are used to identify proteins, antibodies and/or small molecules that modulate cell adhesion to extracellular matrix and cell-cell interaction. Since cell adhesion plays a critical role in tumor growth, progression, and, colonization, when 121P2A3 is involved in this processes it serves as a diagnostic, preventative and therapeutic modality

**Example 50: Involvement of 121P2A3 in Protein Trafficking.**

Due to its similarity to CLIP-190, 121P2A3 can regulate intracellular trafficking. Trafficking of proteins can be studied using well-established methods (Valetti C. et al. Mol Biol Cell. 1999, 10:4107). For example, FITC-conjugated $\alpha$2-macroglobulin is incubated with 121P2A3-expressing and 121P2A3-negative cells. The location and uptake of FITC-$\alpha$2-macroglobulin is visualized using a fluorescent microscope. In another set of experiments, the co-localization of 121P2A3 with vesicular proteins is evaluated by coprecipitation and Western blotting techniques and fluorescent microscopy.

Alternatively, 121P2A3-expresing and 121P2A3-lacking cells are compared using bodipy-ceramide labeled bovine serum albumine (Huber L et al. Mol. Cell. Biol. 1995, 15:918). Briefly, cells are allowed to injest the labeled BSA and are placed intermittently at 4˚C and 18˚C to allow for trafficking to take place. Cells are examined under fluorescent microscopy at different time points for the presence of labeled BSA in specific vesicular compartments, including Golgi, endoplasmic reticulum, etc. In another embodiment, the effect of 121P2A3 on membrane transport is examined using biotin-avidin complexes. Cells either expressing or lacking 121P2A3 are transiently incubated with biotin. The cells are placed at 4˚C or transiently warmed to 37˚C for various periods of time. The cells are fractionated and examined by avidin affinity precipitation for the presence of biotin in specific cellular compartments. Using such assay systems, proteins, antibodies and small molecules are identified that modify the effect of 121P2A3 on vesicular transport. When 121P2A3 plays a role in intracellular trafficking, 121P2A3 is a target for diagnostic, prognostic, preventative and therapeutic purposes

**Example 51: Protein-Protein Association**

The Naf-1 protein homologous to 121P2A3 has been shown to interact with other proteins, thereby forming a protein complex that can regulate cell division, gene transcription, and cell transformation (Renkema GH et al, Curr Biol. 1999, 9:1407; Baur AS et al, Immunity. 1997, 6:283; Karakesisoglou I, Yang Y, Fuchs E. J Cell Biol. 2000, 149:195.). Using immunoprecipitation techniques as well as two yeast hybrid systems, proteins are identified that associate with 121P2A3. Immunoprecipitates from cells expressing 121P2A3 and cells lacking 121P2A3 are compared for specific protein-protein associations.

Studies are performed to determine whether 121P2A3 associates with effector molecules, such as adaptor proteins and SH2-containing proteins. Studies comparing 121P2A3 positive and 121P2A3 negative cells as well as studies comparing unstimulated/resting cells and cells treated with epithelial cell activators, such as cytokines, growth factors, androgen and anti-integrin Ab reveal unique interactions. In addition, protein-protein interactions are studied using two yeast hybrid methodology (Curr Opin Chem Biol. 1999, 3:64). A vector carrying a library of proteins fused to the activation domain of a transcription factor is introduced into yeast expressing a 121P2A3-DNA-binding domain fusion protein and a reporter construct. Protein-protein interaction is detected by calorimetric reporter activity. Specific association with effector molecules and transcription factors indicates the mode of action of 121P2A3, and thus identifies therapeutic, preventative and/or diagnostic targets for cancer. This and similar assays can also be used to identify and screen for small molecules that interact with 121P2A3.

When 121P2A3 associates with proteins or small molecules it is used as a target for diagnostic, prognostic, preventative and therapeutic purposes.

Throughout this application, various website data content, publications, patent applications and patents are referenced. (Websites are referenced by their Uniform Resource Locator, or URL, addresses on the World Wide Web.) The disclosures of each of these references are hereby incorporated by reference herein in their entireties.

The present invention is not to be limited in scope by the embodiments disclosed herein, which are intended as single illustrations of individual aspects of the invention, and any that are functionally equivalent are within the scope of the invention. Various modifications to the models and methods of the invention, in addition to those described herein, will become apparent to those skilled in the art from the foregoing description and teachings, and are similarly intended to fall within the scope of the invention. Such modifications or other embodiments can be practiced without departing from the true scope and spirit of the invention.

**TABLE I: Tissues that Express 121P2A3 When Malignant**

- Prostate
- Bladder
- Kidney
- Colon
- Lung
- Ovary

(continued)

- Breast
- Stomach
- Rectum
- Pancreas
- Testis
- Brain
- Bone
- Cervix

**TABLE II: Amino Acid Abbreviations**

| SINGLE LETTER | THREE LETTER | FULL NAME |
|---|---|---|
| | | |
| F | Phe | phenylalanine |
| L | Leu | leucine |
| S | Ser | serine |
| Y | Tyr | tyrosine |
| C | Cys | cysteine |
| W | Trp | tryptophan |
| P | Pro | proline |
| H | His | histidine |
| Q | Gln | glutamine |
| R | Arg | arginine |
| I | Ile | isoleucine |
| M | Met | methionine |
| T | Thr | threonine |
| N | Asn | asparagine |
| K | Lys | lysine |
| V | Val | valine |
| A | Ala | alanine |
| D | Asp | aspartic acid |
| E | Glu | glutamic acid |
| G | Gly | glycine |

**TABLE III: Amino Acid Substitution Matrix**

Adapted from the GCG Software 9.0 BLOSUM62 amino acid substitution matrix (block substitution matrix). The higher the value, the more likely a substitution is found in related, natural proteins. (See URL www.ikp.unibe.ch/manual/blosum62.html)

| A | C | D | E | F | G | H | I | K | L | M | N | P | Q | R | S | T | V | W | Y | . |
|---|---|---|---|---|---|---|---|---|---|---|---|---|---|---|---|---|---|---|---|---|
| 4 | 0 | -2 | -1 | -2 | 0 | -2 | -1 | -1 | -1 | -1 | -2 | -1 | -1 | -1 | 1 | 0 | 0 | -3 | -2 | A |
| | 9 | -3 | -4 | -2 | -3 | -3 | -1 | -3 | -1 | -1 | -3 | -3 | -3 | -3 | -1 | -1 | -1 | -2 | -2 | C |

(continued)

| A | C | D | E | F | G | H | I | K | L | M | N | P | Q | R | S | T | V | W | Y | . |
|---|---|---|---|---|---|---|---|---|---|---|---|---|---|---|---|---|---|---|---|---|
| | | 6 | 2 | -3 | -1 | -1 | -3 | -1 | -4 | -3 | 1 | -1 | 0 | -2 | 0 | -1 | -3 | -4 | -3 | D |
| | | | 5 | -3 | -2 | 0 | -3 | 1 | -3 | -2 | 0 | -1 | 2 | 0 | 0 | -1 | -2 | -3 | -2 | E |
| | | | | 6 | -3 | -1 | 0 | -3 | 0 | 0 | -3 | -4 | -3 | -3 | -2 | -2 | -1 | 1 | 3 | F |
| | | | | | 6 | -2 | -4 | -2 | -4 | -3 | 0 | -2 | -2 | -2 | 0 | -2 | -3 | -2 | -3 | G |
| | | | | | | 8 | -3 | -1 | -3 | -2 | 1 | -2 | 0 | 0 | -1 | -2 | -3 | -2 | 2 | H |
| | | | | | | | 4 | -3 | 2 | 1 | -3 | -3 | -3 | -3 | -2 | -1 | 3 | -3 | -1 | I |
| | | | | | | | | 5 | -2 | -1 | 0 | -1 | 1 | 2 | 0 | -1 | -2 | -3 | -2 | K |
| | | | | | | | | | 4 | 2 | -3 | -3 | -2 | -2 | -2 | -1 | 1 | -2 | -1 | L |
| | | | | | | | | | | 5 | -2 | -2 | 0 | -1 | -1 | -1 | 1 | -1 | -1 | M |
| | | | | | | | | | | | 6 | -2 | 0 | 0 | 1 | 0 | -3 | -4 | -2 | N |
| | | | | | | | | | | | | 7 | -1 | -2 | -1 | -1 | -2 | -4 | -3 | P |
| | | | | | | | | | | | | | 5 | 1 | 0 | -1 | -2 | -2 | -1 | Q |
| | | | | | | | | | | | | | | 5 | -1 | -1 | -3 | -3 | -2 | R |
| | | | | | | | | | | | | | | | 4 | 1 | -2 | -3 | -2 | S |
| | | | | | | | | | | | | | | | | 5 | 0 | -2 | -2 | T |
| | | | | | | | | | | | | | | | | | 4 | -3 | -1 | V |
| | | | | | | | | | | | | | | | | | | 11 | 2 | W |
| | | | | | | | | | | | | | | | | | | | 7 | Y |

**TABLE IV**

**HLA Class I/II Motifs/Supermotifs**

**TABLE IV (A): HLA Class I Supermotifs/Motifs**

| SUPERMOTIFS | POSITION | POSITION | POSITION |
|---|---|---|---|
| | 2 (Primary Anchor) | 3 (Primary Anchor) | C Terminus (Primary Anchor) |
| A1 | **TI***LVMS* | | **FWY** |
| A2 | **LIVM***ATQ* | | **IV***MATL* |
| A3 | **VSMA***TLI* | | **RK** |
| A24 | **YF***WIVLMT* | | **FI***YWLM* |
| B7 | **P** | | **VIL***FMWYA* |
| B27 | **RHK** | | **FYL***WMIVA* |
| B44 | **E***D* | | **FWYLIMVA** |
| B58 | **ATS** | | **FWY***LIVMA* |
| B62 | **QL***IVMP* | | **FWYMIVLA** |
| | | | |
| MOTIFS | | | |
| A1 | **TSM** | | **Y** |
| A1 | | **DE***AS* | **Y** |
| A2.1 | **LM***VQIAT* | | **V***LIMAT* |
| A3 | **LMVISATF***CGD* | | **KYR***HFA* |

(continued)

| SUPERMOTIFS | POSITION | POSITION | POSITION |
|---|---|---|---|
| | 2 (Primary Anchor) | 3 (Primary Anchor) | C Terminus (Primary Anchor) |
| A11 | **VTMLISAGN***CDF* | | **K***RYH* |
| A24 | **YF**W*M* | | **FLIW** |
| A*3101 | **MVT***ALIS* | | **R***K* |
| A*3301 | **MVALF***IST* | | **RK** |
| A*6801 | **AVT***MSLI* | | **RK** |
| B*0702 | **P** | | **LMF***WYAIV* |
| B*3501 | **P** | | **LMFWY***IVA* |
| B51 | **P** | | **LIVF***WYAM* |
| B*5301 | **P** | | **IMFWY***ALV* |
| B*5401 | **P** | | **ATIV***LMFWY* |
| Bolded residues are preferred, italicized residues are less preferred: A peptide is considered motif-bearing if it has primary anchors at each primary anchor position for a motif or supermotif as specified in the above table. | | | |

**TABLE IV (B): HLA Class II Supermotif**

| **1** | **6** | **9** |
|---|---|---|
| | | |
| W,F,Y,V,I,L | A,V,I,L,P,C,S,T | A,V,I,L,C,S,T,M,Y |

**TABLE IV (C): HLA Class II Motifs**

| MOTIFS | | 1° anchor 1 | 2 | 3 | 4 | 5 | 1° anchor 6 | 7 | 8 | 9 |
|---|---|---|---|---|---|---|---|---|---|---|
| DR4 | preferred | FMY*LIVW* | M | T | | I | VST*CPALIM* | MH | | MH |
| | deleterious | | | | W | | | R | | WDE |
| DR1 | preferred | MF*LIVWY* | | | PAMQ | | *VMATSPLIC* | M | | AVM |
| | deleterious | | C | CH | FD | CWD | | GDE | D | |
| DR7 | preferred | MF*LIVWY* | M | W | A | | IVMSA*CTPL* | M | | IV |
| | deleterious | | C | | G | | | GRD | N | G |
| DR3 | MOTIFS | 1° anchor 1 | 2 | 3 | 1° anchor 4 | 5 | 1° anchor 6 | | | |
| motif a preferred | | LIVMFY | | | D | | | | | |
| motif b preferred | | LIVMFAY | | | DNQEST | | KRH | | | |
| DR Supermotif | | MF*LIVWY* | | | | | VMSTA*CPLI* | | | |
| Italicized residues indicate less preferred or "tolerated" residues | | | | | | | | | | |

**TABLE IV (D): HLA Class I Supermotifs**

| SUPER-MOTIFS | POSITION: | 1 | 2 | 3 | 4 | 5 | 6 | 7 | 8 | C-terminus |
|---|---|---|---|---|---|---|---|---|---|---|
| A1 | | | 1° Anchor<br>TI*LVMS* | | | | | | | 1° Anchor<br>FWY |
| A2 | | | 1° Anchor<br>LIVM*ATQ* | | | | | | | 1° Anchor<br>LIVMAT |
| A3 | preferred | | 1° Anchor<br>VSMA*TLI* | YFW<br>(4/5) | | | YFW<br>(3/5) | YFW<br>(4/5) | P<br>(4/5) | 1 ° Anchor<br>RK |
| | deleterious | DE<br>(3/5); | | DE | | | | | | |
| A24 | | P(5/5) | 1° Anchor<br>YF*WIVLMT* | (4/5) | | | | | | 1° Anchor<br>FIY*WLM* |
| B7 | preferred | FWY<br>(5/5)<br>LIVM<br>(3/5) | 1° Anchor<br>P | FWY<br>(4/5) | | | | | FWY<br>(3/5) | 1 °Anchor<br>VILF*MWYA* |
| | deleterious | DE<br>(3/5);<br>P(5/5);<br>G(4/5);<br>A(3/5); | | | | DE<br>(3/5) | G<br>(4/5) | QN<br>(4/5) | DE<br>(4/5) | |
| B27 | | QN<br>(3/5) | 1° Anchor<br>RHK | | | | | | | 1 °Anchor<br>FYL*WMIVA* |
| B44 | | | 1° Anchor<br>E*D* | | | | | | | 1° Anchor<br>FWYLIMVA |
| B58 | | | 1° Anchor<br>ATS | | | | | | | 1° Anchor<br>FWY*LIVMA* |
| B62 | | | 1 ° Anchor<br>QL*IVMP* | | | | | | | 1° Anchor<br>FWY*MIVLA* |

Italicized residues indicate less preferred or "tolerated" residues

**TABLE IV (E): HLA Class I Motifs**

| | POSITION: | 1 | 2 | 3 | 4 | 5 | 6 | 7 | 8 | 9 | C-terminus |
|---|---|---|---|---|---|---|---|---|---|---|---|
| | | | | | | | | | | or<br>C-terminus | |
| A1<br>9-mer | preferred | GFY<br>W | 1°Anchor<br>STM | DEA | YFW | G | P<br>A | DEQN | YFW | 1 °Anchor<br>Y | |
| | deleterious | DE | | RHKLIVMP | A | | | | | | |
| A1<br>9-mer | preferred | GRHK | ASTCLIVM | 1°Anchor<br>DEAS | GSTC | | ASTC | LIVM | DE | I °Anchor<br>Y | |
| | deleterious | A | RHKDEPY<br>FW | | DE | PQN | RHK | PG | GP | | |
| A1<br>10-mer | preferred | YFW | 1°Anchor<br>STM | DEAQN | A | YFWQN | | PASTC | GDE | P | 1 °Anchor<br>Y |
| | deleterious | GP | | RHKGLIVM | DE | RHK | QNA | RHKYFW | RHK | A | |
| A1<br>10-mer | preferred | YFW | STCLIVM | 1°Anchor<br>DEAS | A | YFW | | PG | G | YFW | 1 °Anchor<br>Y |
| | deleterious | RHK | RHKDEPY FW | | | P | G | | PRHK | QN | |
| A2.1<br>9-mer | preferred | YFW | 1°Anchor<br>LM*IVQAT* | YFW | STC | YFW | | A<br>DERKH | P | 1°Anchor<br>*VLIMAT* | |
| | deleterious | DEP | | DERKH | | | RKH | | | | |
| A2.1<br>10-mer | preferred | AYFW | 1°Anchor<br>LM*IVQAT* | LVIM | G | | G | | FYWL<br>VIM | | 1°Anchor<br>V*LIMAT* |
| | deleterious | DEP | | | RKHA | P | | RKH | DERKH | RKH | |
| A3 | preferred | RHK | 1°Anchor<br>LMVISA | DE YFW | PRHKYFW | A | YFW | | P | 1 °Anchor<br>KYR*HFA* | |
| | deleterious | DEP | TFCGD | DE | | | | | | | |
| A11 | preferred | A | 1°Anchor<br>VTLMIS<br>*AGNCDF* | YFW | YFW | A | YFW | YFW | P | 1°Anchor<br>K*RYH* | |
| | deleterious | DEP | | | | | | A | G | | |
| A24<br>9-mer | preferred | YFWRHK | 1°Anchor<br>YFW*M* | | STC | | | YFW | YFW | 1°Anchor<br>FLIW | |

(continued)

| | POSITION: | 1 | 2 | 3 | 4 | 5 | 6 | 7 | 8 | 9 | C-terminus |
|---|---|---|---|---|---|---|---|---|---|---|---|
| | deleterious | DEG | | DE | G | QNP | DERHK | G | AQN | | |
| A24 10-mer | preferred | | 1°Anchor<br>YFW*M* | | P | YFWP | | P | | | 1°Anchor<br>FLIW |
| | deleterious | | | GDE | QN | RHK | DE | A | QN | DEA | |
| A3101 | preferred | RHK | 1°Anchor<br>MVT*ALIS* | YFW | P | | YFW | YFW | AP | 1°Anchor<br>R*K* | |
| | deleterious | DEP | | DE | | ADE | DE | DE | DE | | |
| A3301 | preferred | | 1°Anchor<br>MVALF*IST* | YFW | | | | AYFW | | 1°Anchor<br>RK | |
| | deleterious | GP | | DE | | | | | | | |
| Italicized residues indicate less preferred or "tolerated" residues | | | | | | | | | | | |
| A6801 | preferred | YFWSTC | 1°Anchor<br>AVT*MSLI* | | | YFWLIVM | | YFW | P | 1°Anchor<br>RK | |
| | deleterious | GP | | DEG | | RHK | | | A | | |
| B0702 | preferred | RHKFWY | 1°Anchor<br>P | RHK | | RHK | RHK | RHK | PA | 1°Anchor<br>LMF*WYAIV* | |
| | deleterious | DEQNP | | DEP | DE | DE | GDE | QN | DE | | |
| B3501 | preferred | FVIYLXVM | 1°Anchor<br>P | FWY | | | | FWY | | 1°Anchor<br>LMFWY*IV* A | |
| | deleterious | AGP | | | | G | G | | | | |
| B51 | preferred | LIVMFWY | 1°Anchor<br>P | FWY | STC | FWY | | G | FWY | 1°Anchor<br>LIVF*WYAM* | |
| | deleterious | AGPDERHKSTC | | | | DE | G | DEQN | GDE | | |
| B5301 | preferred | LIVMFWY | 1°Anchor<br>P | FWY | STC | FWY | | LIVMFWY | FWY | 1°Anchor<br>IMFWY*ALV* | |
| | deleterious | AGPQN | | | | | G | RHKQN | DE | | |

(continued)

| | POSITION: | 1 | 2 | 3 | 4 | 5 | 6 | 7 | 8 | 9 | C-terminus |
|---|---|---|---|---|---|---|---|---|---|---|---|
| B5401 | preferred | FWY | 1°Anchor<br>P | FWYL<br>IVM | | LIVM | | ALIVM | FWYAP | 1°Anchor<br>*ATNLMF*<br>WY | |
| | deleterious | GPQNDE | | GDES<br>TC | | RHKDE | DE | QNDGE | DE | | |

Italicized residues indicate less preferred or "tolerated" residues. The information in this Table is specific for 9-mers unless otherwise specified

| Table V-V1-A1-9mers: | | | 121P2A3 |
|---|---|---|---|
| Pos | 123456789 | Score | SeqID |
| 121 | LSEEKDVLK | 54.000 | |
| 405 | ITEPLVTFQ | 22.500 | |
| 449 | ATEHRDLLV | 11.250 | |
| 40 | SVDEITSGK | 10.000 | |
| 229 | LQEEKQKCY | 6.750 | |
| 413 | QGETENREK | 4.500 | |
| 67 | EAEKEKNAY | 4.500 | |
| 214 | HSLPQQTKK | 3.000 | |
| 328 | LLSQVQFLY | 2.500 | |
| 87 | LRDQLKARY | 2.500 | |
| 237 | YNDLLASAK | 2.500 | |
| 300 | KTEKIQKLR | 2.250 | |
| 259 | SFELSEFRR | 2.250 | |
| 415 | ETENREKVA | 2.250 | |
| 362 | DFENEKLDR | 2.250 | |
| 208 | KTETAAHSL | 2.250 | |
| 307 | LREENDIAR | 2.250 | |
| 22 | KSETTLEKL | 1.350 | |
| 324 | RSEELLSQV | 1.350 | |
| 186 | VYDQQREVY | 1.250 | |
| 31 | KGEIAHLKT | 1.125 | |
| 378 | HVILKELRK | 1.000 | |
| 317 | KLEEEKKRS | 0.900 | |
| 247 | DLEVERQTI | 0.900 | |
| 103 | QLEETTREG | 0.900 | |
| 351 | LLEQQMQAC | 0.900 | |
| 65 | VLEAEKEKN | 0.900 | |
| 141 | ELESKTNTL | 0.900 | |
| 293 | HLEDDRHKT | 0.900 | |
| 437 | LVECPKCNI | 0.900 | |
| 139 | IAELESKTN | 0.900 | |
| 167 | IHEMEIQLK | 0.900 | |
| 393 | QLESLKQLH | 0.900 | |
| 100 | LLEQLEETT | 0.900 | |
| 154 | TVAPNCFNS | 0.500 | |
| 261 | ELSEFRRKY | 0.500 | |
| 360 | TLDFENEKL | 0.500 | |
| 359 | CTLDFENEK | 0.500 | |
| 169 | EMEIQLKDA | 0.450 | |
| 249 | EVERQTITQ | 0.450 | |
| 222 | KPESEGYLQ | 0.450 | |
| 439 | ECPKCNIQY | 0.250 | |
| 288 | RADVQHLED | 0.250 | |
| 355 | QMQACTLDF | 0.250 | |
| 273 | QKEVHNLNQ | 0.225 | |
| 77 | LTEKDKEIQ | 0.225 | |
| 390 | QITQLESLK | 0.200 | |
| 404 | AITEPLVTF | 0.200 | |
| 64 | RVLEAEKEK | 0.200 | |
| 456 | LVHVEYCSK | 0.200 | |

| Table V-V1-A1-9mers: | | | 121P2A3 |
|---|---|---|---|
| Pos | 123456789 | Score | SeqID |
| 423 | AASPKSPTA | 0.200 | |
| 258 | LSFELSEFR | 0.150 | |
| 445 | IQYPATEHR | 0.150 | |
| 342 | QQEEQTRVA | 0.135 | |
| 262 | LSEFRRKYE | 0.135 | |
| 224 | ESEGYLQEE | 0.135 | |
| 51 | LTDKERHRL | 0.125 | |
| 210 | ETAAHSLPQ | 0.125 | |
| 310 | ENDIARGKL | 0.125 | |
| 38 | KTSVDEITS | 0.125 | |
| 179 | EKNQQWLVY | 0.125 | |
| 391 | ITQLESLKQ | 0.125 | |
| 145 | KTNTLRLSQ | 0.125 | |
| 276 | VHNLNQLLY | 0.125 | |
| 24 | ETTLEKLKG | 0.125 | |
| 290 | DVQHLEDDR | 0.100 | |
| 50 | KLTDKERHR | 0.100 | |
| 257 | QLSFELSEF | 0.100 | |
| 199 | LAKIFELEK | 0.100 | |
| 367 | KLDRQHVQH | 0.100 | |
| 381 | LKELRKARN | 0.090 | |
| 308 | REENDIARG | 0.090 | |
| 177 | ALEKNQQWL | 0.090 | |
| 343 | QEEQTRVAL | 0.090 | |
| 26 | TLEKLKGEI | 0.090 | |
| 204 | ELEKKTETA | 0.090 | |
| 329 | LSQVQFLYT | 0.075 | |
| 252 | RQTITQLSF | 0.075 | |
| 95 | YSTTALLEQ | 0.075 | |
| 5 | STKDLIKSK | 0.050 | |
| 295 | EDDRHKTEK | 0.050 | |
| 166 | NIHEMEIQL | 0.050 | |
| 334 | FLYTSLLKQ | 0.050 | |
| 453 | RDLLVHVEY | 0.050 | |
| 350 | ALLEQQMQA | 0.050 | |
| 235 | KCYNDLLAS | 0.050 | |
| 357 | QACTLDFEN | 0.050 | |
| 333 | QFLYTSLLK | 0.050 | |
| 197 | GLLAKIFEL | 0.050 | |
| 158 | NCFNSSINN | 0.050 | |
| 192 | EVYVKGLLA | 0.050 | |
| 374 | QHQLHVILK | 0.050 | |
| 3 | SRSTKDLIK | 0.050 | |
| 254 | TITQLSFEL | 0.050 | |
| 72 | KNAYQLTEK | 0.050 | |
| 403 | FAITEPLVT | 0.050 | |
| 21 | SKSETTLEK | 0.050 | |
| 325 | SEELLSQVQ | 0.045 | |
| 108 | TREGERREQ | 0.045 | |
| 269 | YEETQKEVH | 0.045 | |

| Table V-V3-A1-9mers: 121P2A3 | | | |
|---|---|---|---|
| Pos | 123456789 | Score | SeqID |
| 3 | LTDKERQRL | 0.125 | |
| 2 | KLTDKERQR | 0.100 | |
| 6 | KERQRLLEK | 0.005 | |
| 9 | QRLLEKIRV | 0.003 | |
| 5 | DKERQRLLE | 0.002 | |
| 8 | RQRLLEKIR | 0.002 | |
| 1 | GKLTDKERQ | 0.001 | |
| 4 | TDKERQRLL | 0.000 | |
| 7 | ERQRLLEKI | 0.000 | |

| Table V-V4-AI-9mers: 121P2A3 | | | |
|---|---|---|---|
| Pos | 123456789 | Score | SeqID |
| 5 | YSTTTLLEQ | 0.075 | |
| 6 | STTTLLEQL | 0.025 | |
| 8 | TTLLEQLEE | 0.013 | |
| 9 | TLLEQLEET | 0.010 | |
| 7 | TTTLLEQLE | 0.003 | |
| 2 | KARYSTTTL | 0.001 | |
| 3 | ARYSTTTLL | 0.001 | |
| 4 | RYSTTTLLE | 0.000 | |
| 1 | LKARYSTTT | 0.000 | |

| Table V-V6-A1-9mers: 121P2A3 | | | |
|---|---|---|---|
| Pos | 123456789 | Score | SeqID |
| 8 | QSLYTSLLK | 1.500 | |
| 3 | LLSQVQSLY | 0.500 | |
| 4 | LSQVQSLYT | 0.075 | |
| 9 | SLYTSLLKQ | 0.050 | |
| 6 | QVQSLYTSL | 0.010 | |
| 2 | ELLSQVQSL | 0.010 | |
| 5 | SQVQSLYTS | 0.003 | |
| 7 | VQSLYTSLL | 0.002 | |
| 1 | EELLSQVQS | 0.001 | |

| Table V-V7-A1-9mers: 121P2A3 | | | |
|---|---|---|---|
| Pos | 123456789 | Score | SeqID |
| 9 | LVILKELRK | 1.000 | |
| 8 | LLVILKELR | 0.100 | |
| 5 | QHQLLVILK | 0.050 | |
| 3 | HVQHQLLVI | 0.050 | |
| 7 | QLLVILKEL | 0.010 | |
| 4 | VQHQLLVIL | 0.003 | |
| 2 | QHVQHQLLV | 0.003 | |
| 1 | RQHVQHQLL | 0.002 | |
| 6 | HQLLVILKE | 0.001 | |

| Table V-V8-Al-10mers:121P2A3 | | | |
|---|---|---|---|
| Pos | 123456789 | Score | SeqID |
| 1 | KSPTAALNG | 0.075 | |
| 8 | NGSLVECPK | 0.050 | |
| 9 | GSLVECPKC | 0.030 | |
| 6 | ALNGSLVEC | 0.020 | |
| 4 | TAALNGSLV | 0.010 | |
| 5 | AALNGSLVE | 0.005 | |
| 3 | PTAALNGSL | 0.003 | |
| 2 | SPTAALNGS | 0.003 | |
| 7 | LNGSLVECP | 0.000 | |

| Table VI-V1-A1-10mers: 121P2A3 | | | |
|---|---|---|---|
| Pos | 1234567890 | Score | SeqID |
| 405 | ITEPLVTFQG | 112.500 | |
| 22 | KSETTLEKLK | 27.000 | |
| 224 | ESEGYLQEEK | 27.000 | |
| 449 | ATEHRDLLVH | 11.250 | |
| 77 | LTEKDKEIQR | 11.250 | |
| 141 | ELESKTNTLR | 9.000 | |
| 100 | LLEQLEETTR | 9.000 | |
| 121 | LSEEKDVLKQ | 6.750 | |
| 237 | YNDLLASAKK | 5.000 | |
| 415 | ETENREKVAA | 4.500 | |
| 433 | LNESLVECPK | 4.500 | |
| 452 | HRDLLVHVEY | 2.500 | |
| 327 | ELLSQVQFLY | 2.500 | |
| 275 | EVHNLNQLLY | 2.500 | |
| 351 | LLEQQMQACT | 1.800 | |
| 324 | RSEELLSQVQ | 1.350 | |
| 438 | VECPKCNIQY | 1.250 | |
| 192 | EVYVKGLLAK | 1.000 | |
| 171 | EIQLKDALEK | 1.000 | |
| 65 | VLEAEKEKNA | 0.900 | |
| 437 | LVECPKCNIQ | 0.900 | |
| 259 | SFELSEFRRK | 0.900 | |
| 139 | IAELESKTNT | 0.900 | |
| 308 | REENDIARGK | 0.900 | |
| 325 | SEELLSQVQF | 0.900 | |
| 247 | DLEVERQTIT | 0.900 | |
| 110 | EGERREQVLK | 0.900 | |
| 41 | VDEITSGKGK | 0.900 | |
| 258 | LSFELSEFRR | 0.750 | |
| 228 | YLQEEKQKCY | 0.500 | |
| 40 | SVDEITSGKG | 0.500 | |
| 185 | LVYDQQREVY | 0.500 | |
| 294 | LEDDRHKTEK | 0.500 | |
| 169 | EMEIQLKDAL | 0.450 | |
| 393 | QLESLKQLHE | 0.450 | |
| 104 | LEETTREGER | 0.450 | |
| 59 | LLEKIRVLEA | 0.450 | |
| 53 | DKERHRLLEK | 0.450 | |
| 120 | ALSEEKDVLK | 0.400 | |
| 39 | TSVDEITSGK | 0.300 | |
| 262 | LSEFRRKYEE | 0.270 | |
| 342 | QQEEQTRVAL | 0.270 | |
| 410 | VTFQGETENR | 0.250 | |
| 413 | QGETENREKV | 0.225 | |
| 208 | KTETAAHSLP | 0.225 | |
| 31 | KGEIAHLKTS | 0.225 | |
| 73 | NAYQLTEKDK | 0.200 | |
| 166 | NIHEMEIQLK | 0.200 | |
| 358 | ACTLDFENEK | 0.200 | |
| 212 | AAHSLPQQTK | 0.200 | |

| Table VI-V1-A1-10mers: 121P2A3 | | | |
|---|---|---|---|
| Pos | 1234567890 | Score | SeqID |
| 455 | LLVHVEYCSK | 0.200 | |
| 403 | FAITEPLVTF | 0.200 | |
| 67 | EAEKEKNAYQ | 0.180 | |
| 2 | SSRSTKDLIK | 0.150 | |
| 20 | NSKSETTLEK | 0.150 | |
| 151 | LSQTVAPNCF | 0.150 | |
| 373 | VQHQLHVILK | 0.150 | |
| 332 | VQFLYTSLLK | 0.150 | |
| 229 | LQEEKQKCYN | 0.135 | |
| 253 | QTITQLSFEL | 0.125 | |
| 51 | LTDKERHRLL | 0.125 | |
| 153 | QTVAPNCFNS | 0.125 | |
| 222 | KPESEGYLQE | 0.113 | |
| 300 | KTEKIQKLRE | 0.113 | |
| 376 | QLHVILKELR | 0.100 | |
| 444 | NIQYPATEHR | 0.100 | |
| 198 | LLAKIFELEK | 0.100 | |
| 257 | QLSFELSEFR | 0.100 | |
| 154 | TVAPNCFNSS | 0.100 | |
| 278 | NLNQLLYSQR | 0.100 | |
| 423 | AASPKSPTAA | 0.100 | |
| 86 | RLRDQLKARY | 0.100 | |
| 204 | ELEKKTETAA | 0.090 | |
| 343 | QEEQTRVALL | 0.090 | |
| 307 | LREENDIARG | 0.090 | |
| 418 | NREKVAASPK | 0.090 | |
| 293 | HLEDDRHKTE | 0.090 | |
| 249 | EVERQTITQL | 0.090 | |
| 103 | QLEETTREGE | 0.090 | |
| 26 | TLEKLKGEIA | 0.090 | |
| 269 | YEETQKEVHN | 0.090 | |
| 113 | RREQVLKALS | 0.090 | |
| 317 | KLEEEKKRSE | 0.090 | |
| 177 | ALEKNQQWLV | 0.090 | |
| 354 | QQMQACTLDF | 0.075 | |
| 367 | KLDRQHVQHQ | 0.050 | |
| 328 | LLSQVQFLYT | 0.050 | |
| 163 | SINNIHEMEI | 0.050 | |
| 349 | VALLEQQMQA | 0.050 | |
| 390 | QITQLESLKQ | 0.050 | |
| 241 | LASAKKDLEV | 0.050 | |
| 306 | KLREENDIAR | 0.050 | |
| 210 | ETAAHSLPQQ | 0.050 | |
| 377 | LHVILKELRK | 0.050 | |
| 186 | VYDQQREVYV | 0.050 | |
| 360 | TLDFENEKLD | 0.050 | |
| 226 | EGYLQEEKQK | 0.050 | |
| 288 | RADVQHLEDD | 0.050 | |
| 81 | DKEIQRLRDQ | 0.045 | |
| 400 | LHEFAITEPL | 0.045 | |

| Table VI-V3-A1-10mers: 121P2A3 | | | |
|---|---|---|---|
| Pos | 1234567890 | Score | SeqID |
| 8 | DKERQRLLEK | 0.450 | |
| 6 | LTDKERQRLL | 0.125 | |
| 5 | KLTDKERQRL | 0.010 | |
| 4 | GKLTDKERQR | 0.005 | |
| 11 | RQRLLEKIRV | 0.001 | |
| 2 | GKGKLTDKER | 0.001 | |
| 10 | ERQRLLEKIR | 0.001 | |
| 12 | QRLLEKIRVL | 0.001 | |
| 3 | KGKLTDKERQ | 0.000 | |
| 9' | KERQRLLEKI | 0.000 | |
| 7 | TDKERQRLLE | 0.000 | |
| 1 | SGKGKLTDKE | 0.000 | |

| Table VI-Al-10mers: 121P2A3 | | | |
|---|---|---|---|
| Pos | 1234567890 | Score | SeqID |
| 9 | TTLLEQLEET | 0.025 | |
| 6 | YSTTTLLEQL | 0.015 | |
| 8 | TTTLLEQLEE | 0.013 | |
| 10 | TLLEQLEETT | 0.010 | |
| 7 | STTTLLEQLE | 0.003 | |
| 5 | RYSTTTLLEQ | 0.003 | |
| 3 | KARYSTTTLL | 0.001 | |
| 1 | QLKARYSTTT | 0.001 | |
| 4 | ARYSTTTLLE | 0.000 | |
| 2 | LKARYSTTTL | 0.000 | |

| Table VI-V6-A1-10mers: 121P2A3 | | | |
|---|---|---|---|
| Pos | 1234567890 | Score | SeqID |
| 3 | ELLSQVQSLY | 0.500 | |
| 8 | VQSLYTSLLK | 0.150 | |
| 1 | SEELLSQVQS | 0.090 | |
| 9 | QSLYTSLLKQ | 0.075 | |
| 4 | LLSQVQSLYT | 0.050 | |
| 5 | LSQVQSLYTS | 0.030 | |
| 10 | SLYTSLLKQQ | 0.010 | |
| 7 | QVQSLYTSLL | 0.010 | |

(continued)

| Table VI-V6-A1-10mers: 121P2A3 | | | |
|---|---|---|---|
| Pos | 1234567890 | Score | SeqID |
| 6 | SQVQSLYTSL | 0.002 | |
| 2 | EELLSQVQSL | 0.001 | |

| Table VI-V7-A1-10mers: 121P2A3 | | | |
|---|---|---|---|
| Pos | 1234567890 | Score | SeqID |
| 9 | LLVILKELRK | 1.000 | |
| 5 | VQHQLLVILK | 0.150 | |
| 8 | QLLVILKELR | 0.100 | |
| 4 | HVQHQLLVIL | 0.020 | |
| 10 | LVILKELRKA | 0.010 | |
| 2 | RQHVQHQLLV | 0.007 | |
| 3 | QHVQHQLLVI | 0.003 | |
| 7 | HQLLVILKEL | 0.002 | |
| 1 | DRQHVQHQLL | 0.001 | |
| 6 | QHQLLVILKE | 0.000 | |

| Table VI-V8-A1-10mers: 121P2A3 | | | |
|---|---|---|---|
| Pos | 1234567890 | Score | SeqID |
| 8 | LNGSLVECPK | 0.050 | |
| 6 | AALNGSLVEC | 0.020 | |
| 2 | KSPTAALNGS | 0.015 | |
| 10 | GSLVECPKCN | 0.015 | |
| 9 | NGSLVECPKC | 0.005 | |
| 5 | TAALNGSLVE | 0.005 | |
| 1 | PKSPTAALNG | 0.003 | |
| 4 | PTAALNGSLV | 0.003 | |
| 3 | SPTAALNGSL | 0.003 | |
| 7 | ALNGSLVECP | 0.001 | |

| Table VII-V1-A2-9mers: 121P2A3 | | | |
|---|---|---|---|
| Pos | 123456789 | Score | SeqID |
| 197 | GLLAKIFEL | 1054.405 | |
| 99 | ALLEQLEET | 127.404 | |
| 341 | KQQEEQTRV | 101.193 | |
| 228 | YLQEEKQKC | 93.696 | |
| 392 | TQLESLKQL | 75.571 | |
| 350 | ALLEQQMQA | 75.365 | |
| 327 | ELLSQVQFL | 74.990 | |
| 58 | RLLEKIRVL | 61.119 | |
| 201 | KIFELEKKT | 54.404 | |
| 376 | QLHVILKEL | 49.134 | |
| 432 | ALNESLVEC | 46.848 | |
| 76 | QLTEKDKEI | 42.774 | |
| 240 | LLASAKKDL | 36.316 | |
| 185 | LVYDQQREV | 27.148 | |
| 120 | ALSEEKDVL | 17.596 | |
| 254 | TITQLSFEL | 17.037 | |
| 332 | VQFLYTSLL | 13.624 | |
| 119 | KALSEEKDV | 12.510 | |
| 166 | NIHEMEIQL | 12.043 | |
| 131 | QLSAATSRI | 10.433 | |
| 203 | FELEKKTET | 10.111 | |
| 454 | DLLVHVEYC | 8.545 | |
| 398 | KQLHEFAIT | 7.622 | |
| 177 | ALEKNQQWL | 7.520 | |
| 29 | KLKGEIAHL | 6.019 | |
| 138 | RIAELESKT | 4.201 | |
| 360 | TLDFENEKL | 4.187 | |
| 281 | QLLYSQRRA | 3.676 | |
| 147 | NTLRLSQTV | 3.574 | |
| 274 | KEVHNLNQL | 3.344 | |
| 331 | QVQFLYTSL | 2.804 | |
| 109 | REGERREQV | 2.717 | |
| 414 | GETENREKV | 2.717 | |
| 187 | YDQQREVYV | 2.444 | |
| 389 | NQITQLESL | 2.441 | |
| 140 | AELESKTNT | 2.198 | |
| 379 | VILKELRKA | 1.976 | |
| 373 | VQHQLHVIL | 1.510 | |
| 351 | LLEQQMQAC | 1.243 | |
| 306 | KLREENDIA | 1.088 | |
| 430 | TAALNESLV | 0.966 | |
| 329 | LSQVQFLYT | 0.864 | |
| 328 | LLSQVQFLY | 0.735 | |
| 33 | EIAHLKTSV | 0.717 | |
| 134 | AATSRIAEL | 0.682 | |
| 127 | VLKQQLSAA | 0.680 | |
| 66 | LEAEKEKNA | 0.673 | |
| 178 | LEKNQQWLV | 0.604 | |
| 334 | FLYTSLLKQ | 0.505 | |
| 396 | SLKQLHEFA | 0.469 | |

| Table VII-V1-A2-9mers: 121P2A3 | | | |
|---|---|---|---|
| Pos | 123456789 | Score | SeqID |
| 242 | ASAKKDLEV | 0.454 | |
| 348 | RVALLEQQM | 0.435 | |
| 248 | LEVERQTIT | 0.414 | |
| 100 | LLEQLEETT | 0.397 | |
| 90 | QLKARYSTT | 0.391 | |
| 170 | MEIQLKDAL | 0.346 | |
| 194 | YVKGLLAKI | 0.338 | |
| 96 | STTALLEQL | 0.334 | |
| 436 | SLVECPKCN | 0.306 | |
| 221 | KKPESEGYL | 0.304 | |
| 324 | RSEELLSQV | 0.274 | |
| 156 | APNCFNSSI | 0.259 | |
| 442 | KCNIQYPAT | 0.255 | |
| 352 | LEQQMQACT | 0.246 | |
| 1 | MSSRSTKDL | 0.237 | |
| 399 | QLHEFAITE | 0.232 | |
| 339 | LLKQQEEQT | 0.217 | |
| 89 | DQLKARYST | 0.210 | |
| 51 | LTDKERHRL | 0.202 | |
| 403 | FAITEPLVT | 0.195 | |
| 386 | KARNQITQL | 0.182 | |
| 44 | ITSGKGKLT | 0.176 | |
| 422 | VAASPKSPT | 0.176 | |
| 257 | QLSFELSEF | 0.171 | |
| 150 | RLSQTVAPN | 0.171 | |
| 17 | KPSNSKSET | 0.170 | |
| 353 | EQQMQACTL | 0.162 | |
| 148 | TLRLSQTVA | 0.155 | |
| 397 | LKQLHEFAI | 0.143 | |
| 275 | EVHNLNQLL | 0.140 | |
| 19 | SNSKSETTL | 0.139 | |
| 233 | KQKCYNDLL | 0.130 | |
| 447 | YPATEHRDL | 0.128 | |
| 455 | LLVHVEYCS | 0.127 | |
| 250 | VERQTITQL | 0.123 | |
| 126 | DVLKQQLSA | 0.121 | |
| 164 | INNIHEMEI | 0.116 | |
| 146 | TNTLRLSQT | 0.112 | |
| 246 | KDLEVERQT | 0.110 | |
| 83 | EIQRLRDQL | 0.108 | |
| 367 | KLDRQHVQH | 0.104 | |
| 192 | EVYVKGLLA | 0.104 | |
| 212 | AAHSLPQQT | 0.104 | |
| 141 | ELESKTNTL | 0.103 | |
| 437 | LVECPKCNI | 0.099 | |
| 404 | AITEPLVTF | 0.097 | |
| 416 | TENREKVAA | 0.097 | |
| 26 | TLEKLKGEI | 0.087 | |
| 408 | PLVTFQGET | 0.081 | |
| 92 | KARYSTTAL | 0.079 | |

| Table VII-V3-A2-9mers: 121P2A3 | | | |
|---|---|---|---|
| Pos | 123456789 | Score | SeqID |
| 3 | LTDKERQRL | 0.202 | |
| 2 | KLTDKERQR | 0.043 | |
| 9 | QRLLEKIRV | 0.036 | |
| 4 | TDKERQRLL | 0.001 | |
| 6 | KERQRLLEK | 0.000 | |
| 7 | ERQRLLEKI | 0.000 | |
| 8 | RQRLLEKIR | 0.000 | |
| 1 | GKLTDKERQ | 0.000 | |
| 5 | DKERQRLLE | 0.000 | |

| Table VII-V4-A2-9mers: 121P2A3 | | | |
|---|---|---|---|
| Pos | 123456789 | Score | SeqID |
| 9 | TLLEQLEET | 127.404 | |
| 6 | STTTLLEQL | 0.334 | |
| 2 | KARYSTTTL | 0.079 | |
| 1 | LKARYSTTT | 0.018 | |
| 3 | ARYSTTTLL | 0.009 | |
| 5 | YSTTTLLEQ | 0.001 | |
| 8 | TTLLEQLEE | 0.001 | |
| 7 | TTTLLEQLE | 0.000 | |
| 4 | RYSTTTLLE | 0.000 | |

| Table VII-V6-A2-9mers: 121P2A3 | | | |
|---|---|---|---|
| Pos | 123456789 | Score | SeqID |
| 2 | ELLSQVQSL | 13.635 | |
| 7 | VQSLYTSLL | 3.682 | |
| 6 | QVQSLYTSL | 2.804 | |
| 4 | LSQVQSLYT | 0.455 | |
| 3 | LLSQVQSLY | 0.127 | |
| 9 | SLYTSLLKQ | 0.110 | |
| 5 | SQVQSLYTS | 0.017 | |
| 1 | EELLSQVQS | 0.000 | |
| 8 | QSLYTSLLK | 0.000 | |

| Table VII-V7-A2-9mers: 121P2A3 | | | |
|---|---|---|---|
| Pos | 123456789 | Score | SeqID |
| 7 | QLLVILKEL | 181.794 | |
| 4 | VQHQLLVIL | 3.472 | |
| 1 | RQHVQHQLL | 2.166 | |
| 2 | QHVQHQLLV | 0.048 | |
| 3 | HVQHQLLVI | 0.029 | |
| 8 | LLVILKELR | 0.012 | |
| 9 | LVILKELRK | 0.002 | |
| 6 | HQLLVILKE | 0.000 | |
| 5 | QHQLLVILK | 0.000 | |

| Table VII-V8-A2-9mers: 121P2A3 | | | |
|---|---|---|---|
| Pos | 123456789 | Score | SeqID |
| 6 | ALNGSLVEC | 11.426 | |
| 4 | TAALNGSLV | 0.966 | |
| 9 | GSLVECPKC | 0.120 | |
| 1 | KSPTAALNG | 0.002 | |
| 2 | SPTAALNGS | 0.001 | |
| 3 | PTAALNGSL | 0.001 | |
| 5 | AALNGSLVE | 0.000 | |
| 7 | LNGSLVECP | 0.000 | |
| 8 | NGSLVECPK | 0.000 | |

| Table VIII-V1-A2-10mers:121P2A3 | | | |
|---|---|---|---|
| Pos | 1234567890 | Score | SeqID |
| 282 | LLYSQRRADV | 378.363 | |
| 50 | KLTDKERHRL | 306.550 | |
| 350 | ALLEQQMQAC | 173.338 | |
| 328 | LLSQVQFLYT | 132.385 | |
| 184 | WLVYDQQREV | 63.988 | |
| 99 | ALLEQLEETT | 55.393 | |
| 436 | SLVECPKCNI | 42.774 | |
| 177 | ALEKNQQWLV | 33.385 | |
| 196 | KGLLAKIFEL | 24.090 | |
| 75 | YQLTEKDKEI | 18.003 | |
| 338 | SLLKQQEEQT | 13.510 | |
| 370 | RQHVQHQLHV | 7.052 | |
| 140 | AELESKTNTL | 6.301 | |
| 239 | DLLASAKKDL | 5.928 | |
| 150 | RLSQTVAPNC | 4.968 | |
| 130 | QQLSAATSRI | 3.914 | |
| 203 | FELEKKTETA | 3.303 | |
| 330 | SQVQFLYTSL | 3.249 | |
| 450 | TEHRDLLVHV | 3.111 | |
| 382 | KELRKARNQI | 2.627 | |
| 421 | KVAASPKSPT | 2.282 | |
| 359 | CTLDFENEKL | 2.205 | |
| 396 | SLKQLHEFAI | 2.118 | |
| 331 | QVQFLYTSLL | 1.869 | |
| 176 | DALEKNQQWL | 1.857 | |
| 253 | QTITQLSFEL | 1.721 | |
| 241 | LASAKKDLEV | 1.642 | |
| 189 | QQREVYVKGL | 1.552 | |
| 326 | EELLSQVQFL | 1.458 | |
| 274 | KEVHNLNQLL | 1.454 | |
| 163 | SINNIHEMEI | 1.435 | |
| 228 | YLQEEKQKCY | 1.405 | |
| 32 | GEIAHLKTSV | 1.352 | |
| 267 | RKYEETQKEV | 1.267 | |
| 59 | LLEKIRVLEA | 1.243 | |
| 391 | ITQLESLKQL | 1.160 | |
| 155 | VAPNCFNSSI | 0.936 | |
| 235 | KCYNDLLASA | 0.835 | |
| 145 | KTNTLRLSQT | 0.833 | |
| 351 | LLEQQMQACT | 0.811 | |
| 119 | KALSEEKDVL | 0.772 | |
| 246 | KDLEVERQTI | 0.769 | |
| 95 | YSTTALLEQL | 0.723 | |
| 82 | KEIQRLRDQL | 0.712 | |
| 352 | LEQQMQACTL | 0.706 | |
| 142 | LESKTNTLRL | 0.706 | |
| 109 | REGERREQVL | 0.698 | |
| 133 | SAATSRIAEL | 0.682 | |
| 375 | HQLHVILKEL | 0.627 | |
| 341 | KQQEEQTRVA | 0.593 | |

| Table VIII-V1-A2-10mers:121P2A3 | | | |
|---|---|---|---|
| Pos | 1234567890 | Score | SeqID |
| 431 | AALNESLVEC | 0.587 | |
| 158 | NCFNSSINNI | 0.580 | |
| 342 | QQEEQTRVAL | 0.568 | |
| 65 | VLEAEKEKNA | 0.541 | |
| 334 | FLYTSLLKQQ | 0.505 | |
| 146 | TNTLRLSQTV | 0.454 | |
| 127 | VLKQQLSAAT | 0.443 | |
| 349 | VALLEQQMQA | 0.434 | |
| 98 | TALLEQLEET | 0.432 | |
| 207 | KKTETAAHSL | 0.426 | |
| 131 | QLSAATSRIA | 0.407 | |
| 263 | SEFRRKYEET | 0.394 | |
| 285 | SQRRADVQHL | 0.379 | |
| 280 | NQLLYSQRRA | 0.373 | |
| 323 | KRSEELLSQV | 0.319 | |
| 89 | DQLKARYSTT | 0.314 | |
| 447 | YPATEHRDLL | 0.314 | |
| 25 | TTLEKLKGEI | 0.286 | |
| 126 | DVLKQQLSAA | 0.277 | |
| 58 | RLLEKIRVLE | 0.226 | |
| 90 | QLKARYSTTA | 0.174 | |
| 401 | HEFAITEPLV | 0.170 | |
| 118 | LKALSEEKDV | 0.164 | |
| 340 | LKQQEEQTRV | 0.164 | |
| 414 | GETENREKVA | 0.162 | |
| 453 | RDLLVHVEYC | 0.158 | |
| 388 | RNQITQLESL | 0.157 | |
| 399 | QLHEFAITEP | 0.141 | |
| 424 | ASPKSPTAAL | 0.139 | |
| 165 | NNIHEMEIQL | 0.139 | |
| 21 | SKSETTLEKL | 0.137 | |
| 78 | TEKDKEIQRL | 0.137 | |
| 327 | ELLSQVQFLY | 0.120 | |
| 422 | VAASPKSPTA | 0.117 | |
| 392 | TQLESLKQLH | 0.115 | |
| 168 | HEMEIQLKDA | 0.115 | |
| 201 | KIFELEKKTE | 0.109 | |
| 404 | AITEPLVTFQ | 0.106 | |
| 147 | NTLRLSQTVA | 0.105 | |
| 211 | TAAHSLPQQT | 0.104 | |
| 434 | NESLVECPKC | 0.097 | |
| 191 | REVYVKGLLA | 0.097 | |
| 198 | LLAKIFELEK | 0.096 | |
| 448 | PATEHRDLLV | 0.087 | |
| 17 | KPSNSKSETT | 0.083 | |
| 92 | KARYSTTALL | 0.079 | |
| 292 | QHLEDDRHKT | 0.079 | |
| 43 | EITSGKGKLT | 0.077 | |
| 161 | NSSINNIHEM | 0.075 | |
| 356 | MQACTLDFEN | 0.074 | |

| Table VIII-V3-A2-10mers: 121P2A3 | | | |
|---|---|---|---|
| Pos | 1234567890 | Score | SeqID |
| 5 | KLTDKERQRL | 306.550 | |
| 11 | RQRLLEKIRV | 0.536 | |
| 9 | KERQRLLEKI | 0.061 | |
| 6 | LTDKERQRLL | 0.040 | |
| 12 | QRLLEKIRVL | 0.002 | |
| 2 | GKGKLTDKER | 0.000 | |
| 4 | GKLTDKERQR | 0.000 | |
| 3 | KGKLTDKERQ | 0.000 | |
| 7 | TDKERQRLLE | 0.000 | |
| 1 | SGKGKLTDKE | 0.000 | |
| 8 | DKERQRLLEK | 0.000 | |
| 10 | ERQRLLEKIR | 0.000 | |

| Table VIII-V4-A2-10mers: 121P2A3 | | | |
|---|---|---|---|
| Pos | 1234567890 | Score | SeqID |
| 10 | TLLEQLEETT | 55.393 | |
| 6 | YSTTTLLEQL | 0.723 | |
| 9 | TTLLEQLEET | 0.432 | |
| 1 | QLKARYSTTT | 0.261 | |
| 3 | KARYSTTTLL | 0.079 | |
| 2 | LKARYSTTTL | 0.050 | |
| 7 | STTTLLEQLE | 0.000 | |
| 8 | TTTLLEQLEE | 0.000 | |
| 4 | ARYSTTTLLE | 0.000 | |
| 5 | RYSTTTLLEQ | 0.000 | |

| Table VIII-V6-A2-10mers: 121P2A3 | | | |
|---|---|---|---|
| Pos | 1234567890 | Score | SeqID |
| 4 | LLSQVQSLYT | 69.676 | |
| 6 | SQVQSLYTSL | 3.249 | |
| 7 | QVQSLYTSLL | 1.869 | |
| 2 | EELLSQVQSL | 0.265 | |
| 10 | SLYTSLLKQQ | 0.110 | |
| 3 | ELLSQVQSLY | 0.021 | |
| 8 | VQSLYTSLLK | 0.003 | |

(continued)

| Table VIII-V6-A2-10mers: 121P2A3 | | | |
|---|---|---|---|
| Pos | 1234567890 | Score | SeqID |
| 5 | LSQVQSLYTS | 0.002 | |
| 9 | QSLYTSLLKQ | 0.001 | |
| 1 | SEELLSQVQS | 0.000 | |

| Table VIII-V7-A2-10mers: 121P2A3 | | | |
|---|---|---|---|
| Pos | 1234567890 | Score | SeqID |
| 2 | RQHVQHQLLV | 7.052 | |
| 7 | HQLLVILKEL | 0.627 | |
| 10 | LVILKELRKA | 0.340 | |
| 4 | HVQHQLLVIL | 0.060 | |
| 8 | QLLVILKELR | 0.027 | |
| 9 | LLVILKELRK | 0.025 | |
| 3 | QHVQHQLLVI | 0.007 | |
| 5 | VQHQLLVILK | 0.006 | |
| 1 | DRQHVQHQLL | 0.000 | |
| 6 | QHQLLVILKE | 0.000 | |

| Table VIII-V8-A2-10-mers: 121P2A3 | | | |
|---|---|---|---|
| Pos | 1234567890 | Score | SeqID |
| 6 | AALNGSLVEC | 0.587 | |
| 9 | NGSLVECPKC | 0.032 | |
| 4 | PTAALNGSLV | 0.021 | |
| 3 | SPTAALNGSL | 0.018 | |
| 7 | ALNGSLVECP | 0.017 | |
| 2 | KSPTAALNGS | 0.004 | |
| 10 | GSLVECPKCN | 0.002 | |
| 8 | LNGSLVECPK | 0.000 | |
| 5 | TAALNGSLVE | 0.000 | |
| 1 | PKSPTAALNG | 0.000 | |

| Table IX-V1-A3-9mers: 121P2A3 | | | |
|---|---|---|---|
| Pos | 123456789 | Score | SeqID |
| 117 | VLKALSEEK | 20.000 | |
| 328 | LLSQVQFLY | 18.000 | |
| 197 | GLLAKIFEL | 12.150 | |
| 378 | HVILKELRK | 6.000 | |
| 62 | KIRVLEAEK | 6.000 | |
| 359 | CTLDFENEK | 4.500 | |
| 40 | SVDEITSGK | 4.500 | |
| 29 | KLKGEIAHL | 4.050 | |
| 355 | QMQACTLDF | 4.000 | |
| 380 | ILKELRKAR | 3.000 | |
| 257 | QLSFELSEF | 3.000 | |
| 86 | RLRDQLKAR | 3.000 | |
| 64 | RVLEAEKEK | 2.250 | |
| 456 | LVHVEYCSK | 2.000 | |
| 390 | QITQLESLK | 2.000 | |
| 172 | IQLKDALEK | 1.800 | |
| 36 | HLKTSVDEI | 1.800 | |
| 188 | DQQREVYVK | 1.620 | |
| 199 | LAKIFELEK | 1.200 | |
| 50 | KLTDKERHR | 1.200 | |
| 445 | IQYPATEHR | 0.900 | |
| 350 | ALLEQQMQA | 0.900 | |
| 120 | ALSEEKDVL | 0.900 | |
| 306 | KLREENDIA | 0.900 | |
| 327 | ELLSQVQFL | 0.810 | |
| 5 | STKDLIKSK | 0.750 | |
| 376 | QLHVILKEL | 0.675 | |
| 404 | AITEPLVTF | 0.675 | |
| 84 | IQRLRDQLK | 0.600 | |
| 360 | TLDFENEKL | 0.600 | |
| 367 | KLDRQHVQH | 0.600 | |
| 177 | ALEKNQQWL | 0.600 | |
| 9 | LIKSKWGSK | 0.600 | |
| 131 | QLSAATSRI | 0.600 | |
| 261 | ELSEFRRKY | 0.540 | |
| 280 | NQLLYSQRR | 0.540 | |
| 54 | KERHRLLEK | 0.540 | |
| 300 | KTEKIQKLR | 0.450 | |
| 432 | ALNESLVEC | 0.450 | |
| 76 | QLTEKDKEI | 0.450 | |
| 99 | ALLEQLEET | 0.338 | |
| 228 | YLQEEKQKC | 0.300 | |
| 334 | FLYTSLLKQ | 0.300 | |
| 240 | LLASAKKDL | 0.300 | |
| 351 | LLEQQMQAC | 0.300 | |
| 127 | VLKQQLSAA | 0.300 | |
| 332 | VQFLYTSLL | 0.270 | |
| 455 | LLVHVEYCS | 0.270 | |
| 454 | DLLVHVEYC | 0.270 | |
| 214 | HSLPQQTKK | 0.225 | |

| Table IX-V1-A3-9mers: 121P2A3 | | | |
|---|---|---|---|
| Pos | 123456789 | Score | SeqID |
| 58 | RLLEKIRVL | 0.203 | |
| 148 | TLRLSQTVA | 0.200 | |
| 396 | SLKQLHEFA | 0.200 | |
| 393 | QLESLKQLH | 0.200 | |
| 72 | KNAYQLTEK | 0.180 | |
| 166 | NIHEMEIQL | 0.180 | |
| 247 | DLEVERQTI | 0.180 | |
| 254 | TITQLSFEL | 0.180 | |
| 130 | QQLSAATSR | 0.180 | |
| 141 | ELESKTNTL | 0.180 | |
| 383 | ELRKARNQI | 0.180 | |
| 399 | QLHEFAITE | 0.180 | |
| 26 | TLEKLKGEI | 0.180 | |
| 111 | GERREQVLK | 0.180 | |
| 233 | KQKCYNDLL | 0.162 | |
| 121 | LSEEKDVLK | 0.150 | |
| 258 | LSFELSEFR | 0.150 | |
| 194 | YVKGLLAKI | 0.135 | |
| 374 | QHQLHVILK | 0.120 | |
| 290 | DVQHLEDDR | 0.120 | |
| 252 | RQTITQLSF | 0.120 | |
| 201 | KIFELEKKT | 0.113 | |
| 90 | QLKARYSTT | 0.100 | |
| 100 | LLEQLEETT | 0.100 | |
| 293 | HLEDDRHKT | 0.100 | |
| 339 | LLKQQEEQT | 0.100 | |
| 193 | VYVKGLLAK | 0.090 | |
| 229 | LQEEKQKCY | 0.090 | |
| 278 | NLNQLLYSQ | 0.090 | |
| 198 | LLAKIFELE | 0.090 | |
| 317 | KLEEEKKRS | 0.090 | |
| 208 | KTETAAHSL | 0.090 | |
| 437 | LVECPKCNI | 0.090 | |
| 372 | HVQHQLHVI | 0.090 | |
| 434 | NESLVECPK | 0.090 | |
| 333 | QFLYTSLLK | 0.060 | |
| 21 | SKSETTLEK | 0.060 | |
| 225 | SEGYLQEEK | 0.060 | |
| 331 | QVQFLYTSL | 0.060 | |
| 204 | ELEKKTETA | 0.060 | |
| 150 | RLSQTVAPN | 0.060 | |
| 192 | EVYVKGLLA | 0.060 | |
| 419 | REKVAASPK | 0.060 | |
| 106 | ETTREGERR | 0.060 | |
| 152 | SQTVAPNCF | 0.060 | |
| 8 | DLIKSKWGS | 0.054 | |
| 137 | SRIAELESK | 0.045 | |
| 227 | GYLQEEKQK | 0.045 | |
| 200 | AKIFELEKK | 0.045 | |
| 46 | SGKGKLTDK | 0.045 | |

| Table IX-V3-A3-9mers: 121P2A3 | | | |
|---|---|---|---|
| Pos | 123456789 | Score | SeqID |
| 2 | KLTDKERQR | 1.200 | |
| 6 | KERQRLLEK | 0.540 | |
| 8 | RQRLLEKIR | 0.060 | |
| 3 | LTDKERQRL | 0.030 | |
| 9 | QRLLEKIRV | 0.001 | |
| 7 | ERQRLLEKI | 0.000 | |
| 4 | TDKERQRLL | 0.000 | |
| 1 | GKLTDKERQ | 0.000 | |
| 5 | DKERQRLLE | 0.000 | |

| Table IX-V6-A3-9mers: 121P2A3 | | | |
|---|---|---|---|
| Pos | 123456789 | Score | SeqID |
| 3 | LLSQVQSLY | 6.000 | |
| 2 | ELLSQVQSL | 0.810 | |
| 8 | QSLYTSLLK | 0.300 | |
| 9 | SLYTSLLKQ | 0.300 | |
| 6 | QVQSLYTSL | 0.060 | |
| 7 | VQSLYTSLL | 0.054 | |
| 5 | SQVQSLYTS | 0.008 | |
| 4 | LSQVQSLYT | 0.001 | |
| 1 | EELLSQVQS | 0.000 | |

| Table IX-V7-A3-9mers: 121P2A3 | | | |
|---|---|---|---|
| Pos | 123456789 | Score | SeqID |
| 9 | LVILKELRK | 6.000 | |
| 8 | LLVILKELR | 6.000 | |
| 7 | QLLVILKEL | 1.012 | |
| 3 | HVQHQLLVI | 0.180 | |
| 5 | QHQLLVILK | 0.120 | |
| 4 | VQHQLLVIL | 0.027 | |
| 1 | RQHVQHQLL | 0.018 | |
| 6 | HQLLVILKE | 0.004 | |
| 2 | QHVQHQLLV | 0.001 | |

| Table IX-V8-A3-9mers: 121P2A3 | | | |
|---|---|---|---|
| Pos | 123456789 | Score | SeqID |
| 6 | ALNGSLVEC | 0.450 | |
| 8 | NGSLVECPK | 0.030 | |
| 9 | GSLVECPKC | 0.005 | |
| 4 | TAALNGSLV | 0.002 | |
| 3 | PTAALNGSL | 0.001 | |
| 5 | AALNGSLVE | 0.001 | |
| 1 | KSPTAALNG | 0.001 | |
| 2 | SPTAALNGS | 0.001 | |
| 7 | LNGSLVECP | 0.000 | |

| Table X-V1-A3-10mers: 121P2A3 | | | |
|---|---|---|---|
| Pos | 1234567890 | Score | SeqID |
| 29 | KLKGEIAHLK | 135.000 | |
| 198 | LLAKIFELEK | 120.000 | |
| 306 | KLREENDIAR | 36.000 | |
| 120 | ALSEEKDVLK | 30.000 | |
| 455 | LLVHVEYCSK | 30.000 | |
| 192 | EVYVKGLLAK | 9.000 | |
| 327 | ELLSQVQFLY | 8.100 | |
| 332 | VQFLYTSLLK | 6.000 | |
| 166 | NIHEMEIQLK | 4.500 | |
| 376 | QLHVILKELR | 4.000 | |
| 339 | LLKQQEEQTR | 4.000 | |
| 100 | LLEQLEETTR | 4.000 | |
| 257 | QLSFELSEFR | 4.000 | |
| 86 | RLRDQLKARY | 4.000 | |
| 278 | NLNQLLYSQR | 4.000 | |
| 373 | VQHQLHVILK | 3.600 | |
| 116 | QVLKALSEEK | 3.000 | |
| 228 | YLQEEKQKCY | 3.000 | |
| 8 | DLIKSKWGSK | 2.700 | |
| 436 | SLVECPKCNI | 2.025 | |
| 185 | LVYDQQREVY | 2.000 | |
| 50 | KLTDKERHRL | 1.800 | |
| 182 | QQWLVYDQQR | 1.800 | |
| 396 | SLKQLHEFAI | 1.800 | |
| 141 | ELESKTNTLR | 1.200 | |
| 171 | EIQLKDALEK | 1.200 | |
| 59 | LLEKIRVLEA | 1.200 | |
| 282 | LLYSQRRADV | 1.000 | |
| 410 | VTFQGETENR | 1.000 | |
| 389 | NQITQLESLK | 0.900 | |
| 350 | ALLEQQMQAC | 0.675 | |
| 177 | ALEKNQQWLV | 0.600 | |
| 90 | QLKARYSTTA | 0.600 | |
| 83 | EIQRLRDQLK | 0.600 | |
| 328 | LLSQVQFLYT | 0.600 | |
| 358 | ACTLDFENEK | 0.600 | |
| 73 | NAYQLTEKDK | 0.500 | |
| 258 | LSFELSEFRR | 0.450 | |
| 197 | GLLAKIFELE | 0.405 | |
| 77 | LTEKDKEIQR | 0.400 | |
| 444 | NIQYPATEHR | 0.400 | |
| 129 | KQQLSAATSR | 0.360 | |
| 212 | AAHSLPQQTK | 0.300 | |
| 199 | LAKIFELEKK | 0.300 | |
| 379 | VILKELRKAR | 0.300 | |
| 313 | IARGKLEEEK | 0.300 | |
| 150 | RLSQTVAPNC | 0.300 | |
| 275 | EVHNLNQLLY | 0.240 | |
| 39 | TSVDEITSGK | 0.225 | |
| 99 | ALLEQLEETT | 0.225 | |

| Table X-V1-A3-10mers: 121P2A3 | | | |
|---|---|---|---|
| Pos | 1234567890 | Score | SeqID |
| 20 | NSKSETTLEK | 0.200 | |
| 219 | QTKKPESEGY | 0.200 | |
| 2 | SSRSTKDLIK | 0.200 | |
| 26 | TLEKLKGEIA | 0.200 | |
| 187 | YDQQREVYVK | 0.180 | |
| 331 | QVQFLYTSLL | 0.180 | |
| 354 | QQMQACTLDF | 0.180 | |
| 169 | EMEIQLKDAL | 0.180 | |
| 367 | KLDRQHVQHQ | 0.180 | |
| 338 | SLLKQQEEQT | 0.150 | |
| 36 | HLKTSVDEIT | 0.150 | |
| 194 | YVKGLLAKIF | 0.150 | |
| 136 | TSRIAELESK | 0.150 | |
| 45 | TSGKGKLTDK | 0.150 | |
| 22 | KSETTLEKLK | 0.150 | |
| 239 | DLLASAKKDL | 0.135 | |
| 256 | TQLSFELSEF | 0.135 | |
| 253 | QTITQLSFEL | 0.135 | |
| 189 | QQREVYVKGL | 0.121 | |
| 163 | SINNIHEMEI | 0.120 | |
| 351 | LLEQQMQACT | 0.100 | |
| 127 | VLKQQLSAAT | 0.100 | |
| 65 | VLEAEKEKNA | 0.100 | |
| 13 | KWGSKPSNSK | 0.090 | |
| 412 | FQGETENREK | 0.090 | |
| 432 | ALNESLVECP | 0.090 | |
| 454 | DLLVHVEYCS | 0.081 | |
| 4 | RSTKDLIKSK | 0.075 | |
| 334 | FLYTSLLKQQ | 0.075 | |
| 58 | RLLEKIRVLE | 0.068 | |
| 403 | FAITEPLVTF | 0.068 | |
| 433 | LNESLVECPK | 0.060 | |
| 291 | VQHLEDDRHK | 0.060 | |
| 377 | LHVILKELRK | 0.060 | |
| 361 | LDFENEKLDR | 0.060 | |
| 294 | LEDDRHKTEK | 0.060 | |
| 372 | HVQHQLHVIL | 0.060 | |
| 204 | ELEKKTETAA | 0.060 | |
| 285 | SQRRADVQHL | 0.054 | |
| 235 | KCYNDLLASA | 0.045 | |
| 359 | CTLDFENEKL | 0.045 | |
| 158 | NCFNSSINNI | 0.045 | |
| 421 | KVAASPKSPT | 0.045 | |
| 399 | QLHEFAITEP | 0.045 | |
| 224 | ESEGYLQEEK | 0.045 | |
| 237 | YNDLLASAKK | 0.040 | |
| 243 | SAKKDLEVER | 0.040 | |
| 393 | QLESLKQLHE | 0.040 | |
| 233 | KQKCYNDLLA | 0.036 | |
| 438 | VECPKCNIQY | 0.036 | |

| Table X-V3-A3-10mers: 121P2A3 | | | |
|---|---|---|---|
| Pos | 1234567890 | Score | SeqID |
| 5 | KLTDKERQRL | 1.800 | |
| 8 | DKERQRLLEK | 0.018 | |
| 11 | RQRLLEKIRV | 0.012 | |
| 9 | KERQRLLEKI | 0.008 | |
| 2 | GKGKLTDKER | 0.006 | |
| 6 | LTDKERQRLL | 0.003 | |
| 4 | GKLTDKERQR | 0.002 | |
| 10 | ERQRLLEKIR | 0.001 | |
| 12 | QRLLEKIRVL | 0.000 | |
| 3 | KGKLTDKERQ | 0.000 | |
| 7 | TDKERQRLLE | 0.000 | |
| 1 | SGKGKLTDKE | 0.000 | |

| Table X-V4-A3-10mers: 121P2A3 | | | |
|---|---|---|---|
| Pos | 1234567890 | Score | SeqID |
| 1 | QLKARYSTTT | 0.300 | |
| 10 | TLLEQLEETT | 0.225 | |
| 3 | KARYSTTTLL | 0.018 | |
| 9 | TTLLEQLEET | 0.011 | |
| 6 | YSTTTLLEQL | 0.005 | |
| 8 | TTTLLEQLEE | 0.002 | |
| 7 | STTTLLEQLE | 0.001 | |
| 2 | LKARYSTTTL | 0.001 | |
| 4 | ARYSTTTLLE | 0.000 | |
| 5 | RYSTTTLLEQ | 0.000 | |

| Table X-V6-A3-10mers: 121P2A3 | | | |
|---|---|---|---|
| Pos | 1234567890 | Score | SeqID |
| 3 | ELLSQVQSLY | 2.700 | |
| 8 | VQSLYTSLLK | 1.200 | |
| 4 | LLSQVQSLYT | 0.200 | |
| 7 | QVQSLYTSLL | 0.180 | |
| 10 | SLYTSLLKQQ | 0.075 | |
| 6 | SQVQSLYTSL | 0.027 | |
| 2 | EELLSQVQSL | 0.002 | |
| 5 | LSQVQSLYTS | 0.001 | |

(continued)

| Table X-V6-A3-10mers: 121P2A3 | | | |
|---|---|---|---|
| Pos | 1234567890 | Score | SeqID |
| 9 | QSLYTSLLKQ | 0.000 | |
| 1 | SEELLSQVQS | 0.000 | |

| Table X-V7-A3-10mers: 121P2A3 | | | |
|---|---|---|---|
| Pos | 1234567890 | Score | SeqID |
| 9 | LLVILKELRK | 60.000 | |
| 8 | QLLVILKELR | 6.000 | |
| 5 | VQHQLLVILK | 3.600 | |
| 4 | HVQHQLLVIL | 0.090 | |
| 7 | HQLLVILKEL | 0.030 | |
| 2 | RQHVQHQLLV | 0.012 | |
| 10 | LVILKELRKA | 0.005 | |
| 3 | QHVQHQLLVI | 0.003 | |
| 1 | DRQHVQHQLL | 0.000 | |
| 6 | QHQLLVILKE | 0.000 | |

| Table X-V8-A3-10mers: 121P2A3 | | | |
|---|---|---|---|
| Pos | 1234567890 | Score | SeqID |
| 7 | ALNGSLVECP | 0.090 | |
| 8 | LNGSLVECPK | 0.060 | |
| 6 | AALNGSLVEC | 0.005 | |
| 3 | SPTAALNGSL | 0.002 | |
| 4 | PTAALNGSLV | 0.001 | |
| 2 | KSPTAALNGS | 0.001 | |
| 5 | TAALNGSLVE | 0.000 | |
| 10 | GSLVECPKCN | 0.000 | |
| 9 | NGSLVECPICC | 0.000 | |
| 1 | PKSPTAALNG | 0.000 | |

| Table XI-V1-A11-9mers: 121P2A3 | | | |
|---|---|---|---|
| Pos | 123456789 | Score | SeqID |
| 378 | HVILKELRK | 6.000 | |
| 64 | RVLEAEKEK | 4.500 | |
| 456 | LVHVEYCSK | 2.000 | |
| 40. | SVDEITSGK | 2.000 | |
| 172 | IQLKDALEK | 1.800 | |
| 359 | CTLDFENEK | 1.500 | |
| 62 | KIRVLEAEK | 1.200 | |
| 193 | VYVKGLLAK | 1.200 | |
| 227 | GYLQEEKQK | 0.900 | |
| 333 | QFLYTSLLK | 0.600 | |
| 84 | IQRLRDQLK | 0.600 | |
| 5 | STKDLIKSK | 0.500 | |
| 9 | LIKSKWGSK | 0.400 | |
| 117 | VLKALSEEK | 0.400 | |
| 199 | LAKIFELEK | 0.400 | |
| 390 | QITQLESLK | 0.400 | |
| 188 | DQQREVYVK | 0.360 | |
| 54 | KERHRLLEK | 0.360 | |
| 300 | KTEKIQKLR | 0.300 | |
| 445 | IQYPATEHR | 0.240 | |
| 74 | AYQLTEKDK | 0.200 | |
| 280 | NQLLYSQRR | 0.180 | |
| 130 | QQLSAATSR | 0.180 | |
| 419 | REKVAASPK | 0.180 | |
| 111 | GERREQVLK | 0.180 | |
| 72 | KNAYQLTEK | 0.120 | |
| 298 | RHKTEKIQK | 0.120 | |
| 259 | SFELSEFRR | 0.120 | |
| 290 | DVQHLEDDR | 0.120 | |
| 86 | RLRDQLKAR | 0.120 | |
| 315 | RGKLEEEKK | 0.060 | |
| 266 | RRKYEETQK | 0.060 | |
| 106 | ETTREGERR | 0.060 | |
| 434 | NESLVECPK | 0.060 | |
| 225 | SEGYLQEEK | 0.060 | |
| 348 | RVALLEQQM | 0.060 | |
| 197 | GLLAKIFEL | 0.054 | |
| 411 | TFQGETENR | 0.040 | |
| 380 | ILKELRKAR | 0.040 | |
| 237 | YNDLLASAK | 0.040 | |
| 3 | SRSTKDLIK | 0.040 | |
| 374 | QHQLHVILK | 0.040 | |
| 21 | SKSETTLEK | 0.040 | |
| 252 | RQTITQLSF | 0.036 | |
| 200 | AKIFELEKK | 0.030 | |
| 214 | HSLPQQTKK | 0.030 | |
| 137 | SRIAELESK | 0.030 | |
| 238 | NDLLASAKK | 0.030 | |
| 23 | SETTLEKLK | 0.030 | |
| 208 | KTETAAHSL | 0.030 | |

| Table XI-V1-A11-9mers: 121P2A3 | | | |
|---|---|---|---|
| Pos | 123456789 | Score | SeqID |
| 50 | KLTDKERHR | 0.024 | |
| 362 | DFENEKLDR | 0.024 | |
| 78 | TEKDKEIQR | 0.024 | |
| 192 | EVYVKGLLA | 0.024 | |
| 449 | ATEHRDLLV | 0.020 | |
| 167 | IHEMEIQLK | 0.020 | |
| 46 | SGKGKLTDK | 0.020 | |
| 30 | LKGEIAHLK | 0.020 | |
| 314 | ARGKLEEEK | 0.020 | |
| 213 | AHSLPQQTK | 0.020 | |
| 121 | LSEEKDVLK | 0.020 | |
| 331 | QVQFLYTSL | 0.020 | |
| 14 | WGSKPSNSK | 0.020 | |
| 437 | LVECPKCNI | 0.020 | |
| 372 | HVQHQLHVI | 0.020 | |
| 194 | YVKGLLAKI | 0.020 | |
| 370 | RQHVQHQLH | 0.018 | |
| 341 | KQQEEQTRV | 0.018 | |
| 233 | KQKCYNDLL | 0.018 | |
| 126 | DVLKQQLSA | 0.018 | |
| 147 | NTLRLSQTV | 0.015 | |
| 42 | DEITSGKGK | 0.013 | |
| 142 | LESKTNTLR | 0.012 | |
| 101 | LEQLEETTR | 0.012 | |
| 328 | LLSQVQFLY | 0.012 | |
| 306 | KLREENDIA | 0.012 | |
| 350 | ALLEQQMQA | 0.012 | |
| 367 | KLDRQHVQH | 0.012 | |
| 254 | TITQLSFEL | 0.012 | |
| 332 | VQFLYTSLL | 0.012 | |
| 29 | KLKGEIAHL | 0.012 | |
| 96 | STTALLEQL | 0.010 | |
| 51 | LTDKERHRL | 0.010 | |
| 316 | GKLEEEKKR | 0.009 | |
| 389 | NQITQLESL | 0.009 | |
| 260 | FELSEFRRK | 0.009 | |
| 258 | LSFELSEFR | 0.008 | |
| 307 | LREENDIAR | 0.008 | |
| 166 | NIHEMEIQL | 0.008 | |
| 355 | QMQACTLDF | 0.008 | |
| 279 | LNQLLYSQR | 0.008 | |
| 183 | QWLVYDQQR | 0.006 | |
| 377 | LHVILKELR | 0.006 | |
| 48 | KGKLTDKER | 0.006 | |
| 56 | RHRLLEKIR | 0.006 | |
| 295 | EDDRHKTEK | 0.006 | |
| 285 | SQRRADVQH | 0.006 | |
| 275 | EVHNLNQLL | 0.006 | |
| 386 | KARNQITQL | 0.006 | |
| 291 | VQHLEDDRH | 0.006 | |

| Table XI-V3-All-9mers: 121P2A3 | | | |
|---|---|---|---|
| Pos | 123456789 | Score | SeqID |
| 6 | KERQRLLEK | 0.360 | |
| 8 | RQRLLEKIR | 0.180 | |
| 2 | KLTDKERQR | 0.024 | |
| 3 | LTDKERQRL | 0.010 | |
| 9 | QRLLEKIRV | 0.001 | |
| 1 | GKLTDKERQ | 0.000 | |
| 7 | ERQRLLEKI | 0.000 | |
| 4 | TDKERQRLL | 0.000 | |
| 5 | DKERQRLLE | 0.000 | |

| Table XI-V4-A11-9mers: 121P2A3 | | | |
|---|---|---|---|
| Pos | 123456789 | Score | SeqID |
| 6 | STTTLLEQL | 0.010 | |
| 2 | KARYSTTTL | 0.006 | |
| 8 | TTLLEQLEE | 0.003 | |
| 4 | RYSTTTLLE | 0.002 | |
| 7 | TTTLLEQLE | 0.001 | |
| 9 | TLLEQLEET | 0.001 | |
| 3 | ARYSTTTLL | 0.000 | |
| 5 | YSTTTLLEQ | 0.000 | |
| 1 | LKARYSTTT | 0.000 | |

| Table XI-V6-A11-9mers: 121P2A3 | | | |
|---|---|---|---|
| Pos | 123456789 | Score | SeqID |
| 8 | QSLYTSLLK | 0.060 | |
| 6 | QVQSLYTSL | 0.020 | |
| 7 | VQSLYTSLL | 0.006 | |
| 3 | LLSQVQSLY | 0.004 | |
| 5 | SQVQSLYTS | 0.002 | |
| 2 | ELLSQVQSL | 0.002 | |
| 9 | SLYTSLLKQ | 0.002 | |
| 4 | LSQVQSLYT | 0.000 | |
| 1 | EELLSQVQS | 0.000 | |

| Table XI-V7-A11-9mers: 121P2A3 | | | |
|---|---|---|---|
| Pos | 123456789 | Score | SeqID |
| 9 | LVILKELRK | 6.000 | |
| 8 | LLVILKELR | 0.120 | |
| 5 | QHQLLVILK | 0.040 | |
| 3 | HVQHQLLVI | 0.040 | |
| 1 | RQHVQHQLL | 0.018 | |
| 4 | VQHQLLVIL | 0.006 | |
| 7 | QLLVILKEL | 0.003 | |
| 6 | HQLLVILKE | 0.002 | |
| 2 | QHVQHQLLV | 0.001 | |

| Table XI-V8-A11-9mers: 121P2A3 | | | |
|---|---|---|---|
| Pos | 123456789 | Score | SeqID |
| 8 | NGSLVECPK | 0.020 | |
| 4 | TAALNGSLV | 0.002 | |
| 3 | PTAALNGSL | 0.001 | |
| 5 | AALNGSLVE | 0.001 | |
| 6 | ALNGSLVEC | 0.000 | |
| 2 | SPTAALNGS | 0.000 | |
| 1 | KSPTAALNG | 0.000 | |
| 9 | GSLVECPKC | 0.000 | |
| 7 | LNGSLVECP | 0.000 | |

| Table XII-V1-A11-10mers:121P2A3 | | | |
|---|---|---|---|
| Pos | 1234567890 | Score | SeqID |
| 116 | QVLKALSEEK | 3.000 | |
| 332 | VQFLYTSLLK | 2.400 | |
| 192 | EVYVKGLLAK | 2.400 | |
| 373 | VQHQLHVILK | 1.200 | |
| 29 | KLKGEIAHLK | 1.200 | |
| 389 | NQITQLESLK | 0.900 | |
| 198 | LLAKIFELEK | 0.800 | |
| 455 | LLVHVEYCSK | 0.600 | |
| 306 | KLREENDIAR | 0.480 | |
| 120 | ALSEEKDVLK | 0.400 | |
| 236 | CYNDLLASAK | 0.400 | |
| 77 | LTEKDKEIQR | 0.400 | |
| 410 | VTFQGETENR | 0.400 | |
| 166 | NIHEMEIQLK | 0.400 | |
| 129 | KQQLSAATSR | 0.360 | |
| 171 | EIQLKDALEK | 0.240 | |
| 182 | QQWLVYDQQR | 0.240 | |
| 212 | AAHSLPQQTK | 0.200 | |
| 313 | IARGKLEEEK | 0.200 | |
| 358 | ACTLDFENEK | 0.200 | |
| 199 | LAKIFELEKK | 0.200 | |
| 73 | NAYQLTEKDK | 0.200 | |
| 8 | DLIKSKWGSK | 0.180 | |
| 83 | EIQRLRDQLK | 0.120 | |
| 444 | NIQYPATEHR | 0.080 | |
| 376 | QLHVILKELR | 0.080 | |
| 100 | LLEQLEETTR | 0.080 | |
| 278 | NLNQLLYSQR | 0.080 | |
| 257 | QLSFELSEFR | 0.080 | |
| 339 | LLKQQEEQTR | 0.080 | |
| 291 | VQHLEDDRHK | 0.060 | |
| 412 | FQGETENREK | 0.060 | |
| 13 | KWGSKPSNSK | 0.060 | |
| 377 | LHVILKELRK | 0.060 | |
| 294 | LEDDRHKTEK | 0.060 | |
| 379 | VILKELRKAR | 0.060 | |
| 253 | QTITQLSFEL | 0.045 | |
| 237 | YNDLLASAKK | 0.040 | |
| 243 | SAKKDLEVER | 0.040 | |
| 2 | SSRSTKDLIK | 0.040 | |
| 20 | NSKSETTLEK | 0.040 | |
| 433 | LNESLVECPK | 0.040 | |
| 187 | YDQQREVYVK | 0.040 | |
| 185 | LVYDQQREVY | 0.040 | |
| 370 | RQHVQHQLHV | 0.036 | |
| 233 | KQKCYNDLLA | 0.036 | |
| 4 | RSTKDLIKSK | 0.030 | |
| 22 | KSETTLEKLK | 0.030 | |
| 39 | TSVDEITSGK | 0.030 | |
| 258 | LSFELSEFRR | 0.024 | |

| Table XII-V1-A11-10mers:121P2A3 | | | |
|---|---|---|---|
| Pos | 1234567890 | Score | SeqID |
| 141 | ELESKTNTLR | 0.024 | |
| 354 | QQMQACTLDF | 0.024 | |
| 136 | TSRIAELESK | 0.020 | |
| 449 | ATEHRDLLVH | 0.020 | |
| 314 | ARGKLEEEKK | 0.020 | |
| 372 | HVQHQLHVIL | 0.020 | |
| 259 | SFELSEFRRK | 0.020 | |
| 331 | QVQFLYTSLL | 0.020 | |
| 213 | AHSLPQQTKK | 0.020 | |
| 418 | NREKVAASPK | 0.020 | |
| 45 | TSGKGKLTDK | 0.020 | |
| 265 | FRRKYEETQK | 0.020 | |
| 308 | REENDIARGK | 0.018 | |
| 361 | LDFENEKLDR | 0.016 | |
| 63 | IRVLEAEKEK | 0.015 | |
| 359 | CTLDFENEKL | 0.015 | |
| 25 | TTLEKLKGEI | 0.015 | |
| 147 | NTLRLSQTVA | 0.015 | |
| 86 | RLRDQLKARY | 0.012 | |
| 275 | EVHNLNQLLY | 0.012 | |
| 268 | KYEETQKEVH | 0.012 | |
| 396 | SLKQLHEFAI | 0.012 | |
| 104 | LEETTREGER | 0.012 | |
| 297 | DRHKTEKIQK | 0.012 | |
| 235 | KCYNDLLASA | 0.012 | |
| 53 | DKERHRLLEK | 0.012 | |
| 84 | IQRLRDQLKA | 0.012 | |
| 50 | KLTDKERHRL | 0.012 | |
| 219 | QTKKPESEGY | 0.010 | |
| 41 | VDEITSGKGK | 0.010 | |
| 194 | YVKGLLAKIF | 0.010 | |
| 5 | STKDLIKSKW | 0.010 | |
| 256 | TQLSFELSEF | 0.009 | |
| 64 | RVLEAEKEKN | 0.009 | |
| 130 | QQLSAATSRI | 0.009 | |
| 392 | TQLESLKQLH | 0.009 | |
| 61 | EKIRVLEAEK | 0.009 | |
| 119 | KALSEEKDVL | 0.009 | |
| 126 | DVLKQQLSAA | 0.009 | |
| 330 | SQVQFLYTSL | 0.009 | |
| 163 | SINNIHEMEI | 0.008 | |
| 282 | LLYSQRRADV | 0.008 | |
| 59 | LLEKIRVLEA | 0.008 | |
| 177 | ALEKNQQWLV | 0.008 | |
| 279 | LNQLLYSQRR | 0.008 | |
| 315 | RGKLEEEKKR | 0.006 | |
| 289 | ADVQHLEDDR | 0.006 | |
| 47 | GKGKLTDKER | 0.006 | |
| 92 | KARYSTTALL | 0.006 | |
| 300 | KTEKIQKLRE | 0.006 | |

| Table XII-V3-A11-10mers: 121P2A3 | | | |
|---|---|---|---|
| Pos | 1234567890 | Score | SeqID |
| 11 | RQRLLEKIRV | 0.036 | |
| 5 | KLTDKERQRL | 0.012 | |
| 8 | DKERQRLLEK | 0.012 | |
| 2 | GKGKLTDKER | 0.006 | |
| 4 | GKLTDKERQR | 0.002 | |
| 9 | KERQRLLEKI | 0.002 | |
| 6 | LTDKERQRLL | 0.001 | |
| 10 | ERQRLLEKIR | 0.001 | |
| 3 | KGKLTDKERQ | 0.000 | |
| 7 | TDKERQRLLE | 0.000 | |
| 12 | QRLLEKIRVL | 0.000 | |
| 1 | SGKGKLTDKE | 0.000 | |

| Table XII-V4-A11-10mers: 121P2A3 | | | |
|---|---|---|---|
| Pos | 1234567890 | Score | SeqID |
| 3 | KARYSTTTLL | 0.006 | |
| 5 | RYSTTTLLEQ | 0.002 | |
| 8 | TTTLLEQLEE | 0.002 | |
| 9 | TTLLEQLEET | 0.002 | |
| 7 | STTTLLEQLE | 0.001 | |
| 10 | TLLEQLEETT | 0.001 | |
| 1 | QLKARYSTTT | 0.000 | |
| 6 | YSTTTLLEQL | 0.000 | |
| 2 | LKARYSTTTL | 0.000 | |
| 4 | ARYSTTTLLE | 0.000 | |

| Table XII-V6-A11-10mers: 121P2A3 | | | |
|---|---|---|---|
| Pos | 1234567890 | Score | SeqID |
| 8 | VQSLYTSLLK | 1.200 | |
| 7 | QVQSLYTSLL | 0.020 | |
| 6 | SQVQSLYTSL | 0.009 | |
| 3 | ELLSQVQSLY | 0.002 | |
| 4 | LLSQVQSLYT | 0.001 | |
| 10 | SLYTSLLKQQ | 0.000 | |

(continued)

| Table XII-V6-A11-10mers: 121P2A3 | | | |
|---|---|---|---|
| Pos | 1234567890 | Score | SeqID |
| 2 | EELLSQVQSL | 0.000 | |
| 9 | QSLYTSLLKQ | 0.000 | |
| 1 | SEELLSQVQS | 0.000 | |
| 5 | LSQVQSLYTS | 0.000 | |

| Table XII-V7-A11-10mers | | | |
|---|---|---|---|
| Pos | 1234567890 | Score | SeqID |
| 9 | LLVILKELRK | 1.200 | |
| 5 | VQHQLLVILK | 1.200 | |
| 8 | QLLVILKELR | 0.120 | |
| 2 | RQHVQHQLLV | 0.036 | |
| 4 | HVQHQLLVIL | 0.020 | |
| 7 | HQLLVILKEL | 0.005 | |
| 10 | LVILKELRKA | 0.003 | |
| 3 | QHVQHQLLVI | 0.001 | |
| 1 | DRQHVQHQLL | 0.000 | |
| 6 | QHQLLVILKE | 0.000 | |

| Table XII-V8-A11-10mers: 121P2A3 | | | |
|---|---|---|---|
| Pos | 1234567890 | Score | SeqID |
| 8 | LNGSLVECPK | 0.040 | |
| 3 | SPTAALNGSL | 0.002 | |
| 4 | PTAALNGSLV | 0.001 | |
| 7 | ALNGSLVECP | 0.000 | |
| 5 | TAALNGSLVE | 0.000 | |
| 6 | AALNGSLVEC | 0.000 | |
| 2 | KSPTAALNGS | 0.000 | |
| 10 | GSLVECPKCN | 0.000 | |
| 9 | NGSLVECPKC | 0.000 | |
| 1 | PKSPTAALNG | 0.000 | |

| Table XIII-V1-A24-9mers:121P2A3 | | | |
|---|---|---|---|
| Pos | 123456789 | Score | SeqID |
| 268 | KYEETQKEV | 19.800 | |
| 58 | RLLEKIRVL | 14.400 | |
| 22 | KSETTLEKL | 13.200 | |
| 208 | KTETAAHSL | 12.000 | |
| 236 | CYNDLLASA | 10.800 | |
| 159 | CFNSSINNI | 9.000 | |
| 92 | KARYSTTAL | 8.000 | |
| 386 | KARNQITQL | 8.000 | |
| 29 | KLKGEIAHL | 8.000 | |
| 233 | KQKCYNDLL | 8.000 | |
| 141 | ELESKTNTL | 7.200 | |
| 177 | ALEKNQQWL | 7.200 | |
| 327 | ELLSQVQFL | 7.200 | |
| 110 | EGERREQVL | 7.200 | |
| 83 | EIQRLRDQL | 7.200 | |
| 392 | TQLESLKQL | 7.200 | |
| 331 | QVQFLYTSL | 7.200 | |
| 197 | GLLAKIFEL | 6.600 | |
| 376 | QLHVILKEL | 6.160 | |
| 353 | EQQMQACTL | 6.000 | |
| 389 | NQITQLESL | 6.000 | |
| 271 | ETQKEVHNL | 6.000 | |
| 275 | EVHNLNQLL | 5.760 | |
| 254 | TITQLSFEL | 5.280 | |
| 283 | LYSQRRADV | 5.000 | |
| 186 | VYDQQREVY | 5.000 | |
| 166 | NIHEMEIQL | 4.800 | |
| 120 | ALSEEKDVL | 4.800 | |
| 373 | VQHQLHVIL | 4.800 | |
| 96 | STTALLEQL | 4.800 | |
| 43 | EITSGKGKL | 4.400 | |
| 310 | ENDIARGKL | 4.400 | |
| 134 | AATSRIAEL | 4.400 | |
| 360 | TLDFENEKL | 4.400 | |
| 252 | RQTITQLSF | 4.000 | |
| 19 | SNSKSETTL | 4.000 | |
| 1 | MSSRSTKDL | 4.000 | |
| 332 | VQFLYTSLL | 4.000 | |
| 447 | YPATEHRDL | 4.000 | |
| 143 | ESKTNTLRL | 4.000 | |
| 51 | LTDKERHRL | 4.000 | |
| 240 | LLASAKKDL | 4.000 | |
| 425 | SPKSPTAAL | 4.000 | |
| 395 | ESLKQLHEF | 3.300 | |
| 355 | QMQACTLDF | 3.000 | |
| 152 | SQTVAPNCF | 2.400 | |
| 404 | AITEPLVTF | 2.400 | |
| 257 | QLSFELSEF | 2.200 | |
| 26 | TLEKLKGEI | 1.980 | |
| 247 | DLEVERQTI | 1.800 | |

| Table XIII-V1-A24-9mers:121P2A3 | | | |
|---|---|---|---|
| Pos | 123456789 | Score | SeqID |
| 113 | RREQVLKAL | 1.680 | |
| 191 | REVYVKGLL | 1.680 | |
| 164 | INNIHEMEI | 1.650 | |
| 372 | HVQHQLHVI | 1.500 | |
| 437 | LVECPKGNI | 1.500 | |
| 156 | APNCFNSSI | 1.500 | |
| 274 | KEVHNLNQL | 1.440 | |
| 221 | KKPESEGYL | 1.440 | |
| 348 | RVALLEQQM | 1.440 | |
| 76 | QLTEKDKEI | 1.320 | |
| 194 | YVKGLLAKI | 1.320 | |
| 383 | ELRKARNQI | 1.200 | |
| 36 | HLKTSVDEI | 1.100 | |
| 2 | SSRSTKDLI | 1.000 | |
| 131 | QLSAATSRI | 1.000 | |
| 94 | RYSTTALLE | 1.000 | |
| 369 | DRQHVQHQL | 0.840 | |
| 162 | SSINNIHEM | 0.825 | |
| 74 | AYQLTEKDK | 0.750 | |
| 446 | QYPATEHRD | 0.750 | |
| 227 | GYLQEEKQK | 0.750 | |
| 193 | VYVKGLLAK | 0.750 | |
| 170 | MEIQLKDAL | 0.720 | |
| 232 | EKQKCYNDL | 0.720 | |
| 299 | HKTEKIQKL | 0.634 | |
| 190 | QREVYVKGL | 0.600 | |
| 344 | EEQTRVALL | 0.600 | |
| 69 | EKEKNAYQL | 0.600 | |
| 335 | LYTSLLKQQ | 0.600 | |
| 343 | QEEQTRVAL | 0.600 | |
| 124 | EKDVLKQQL | 0.576 | |
| 401 | HEFAITEPL | 0.560 | |
| 264 | EFRRKYEET | 0.550 | |
| 402 | EFAITEPLV | 0.500 | |
| 448 | PATEHRDLL | 0.480 | |
| 429 | PTAALNESL | 0.480 | |
| 79 | EKDKEIQRL | 0.480 | |
| 286 | QRRADVQHL | 0.480 | |
| 52 | TDKERHRLL | 0.480 | |
| 320 | EEKKRSEEL | 0.440 | |
| 324 | RSEELLSQV | 0.432 | |
| 250 | VERQTITQL | 0.400 | |
| 93 | ARYSTTALL | 0.400 | |
| 321 | EKKRSEELL | 0.400 | |
| 303 | KIQKLREEN | 0.396 | |
| 398 | KQLHEFAIT | 0.360 | |
| 317 | KLEEEKKRS | 0.360 | |
| 341 | KQQEEQTRV | 0.360 | |
| 31 | KGEIAHLKT | 0.330 | |
| 388 | RNQITQLES | 0.330 | |

| Table XIII-V3-A24-9mers: 121P2A3 | | | |
|---|---|---|---|
| Pos | 123456789 | Score | SeqID |
| 3 | LTDKERQRL | 4.800 | |
| 4 | TDKERQRLL | 0.480 | |
| 7 | ERQRLLEKI | 0.198 | |
| 8 | RQRLLEKIR | 0.024 | |
| 2 | KLTDKERQR | 0.024 | |
| 9 | QRLLEKIRV | 0.015 | |
| 6 | KERQRLLEK | 0.002 | |
| 5 | DKERQRLLE | 0.002 | |
| 1 | GKLTDKERQ | 0.002 | |

| Table XIII-V4-A24-9mers: 121P2A3 | | | |
|---|---|---|---|
| Pos | 123456789 | Score | SeqID |
| 2 | KARYSTTTL | 8.000 | |
| 6 | STTTLLEQL | 4.800 | |
| 4 | RYSTTTLLE | 1.000 | |
| 3 | ARYSTTTLL | 0.400 | |
| 9 | TLLEQLEET | 0.198 | |
| 8 | TTLLEQLEE | 0.017 | |
| 7 | TTTLLEQLE | 0.014 | |
| 5 | YSTTTLLEQ | 0.011 | |
| 1 | LKARYSTTT | 0.010 | |

| Table XIII-V6-A24-9mers: 121P2A3 | | | |
|---|---|---|---|
| Pos | 123456789 | Score | SeqID |
| 6 | QVQSLYTSL | 7.200 | |
| 2 | ELLSQVQSL | 7.200 | |
| 7 | VQSLYTSLL | 4.000 | |
| 5 | SQVQSLYTS | 0.150 | |
| 4 | LSQVQSLYT | 0.150 | |
| 3 | LLSQVQSLY | 0.140 | |
| 8 | QSLYTSLLK | 0.015 | |
| 1 | EELLSQVQS | 0.015 | |
| 9 | SLYTSLLKQ | 0.011 | |

| Table XIII-V7-A24-9mers: 121P2A3 | | | |
|---|---|---|---|
| Pos | 123456789 | Score | SeqID |
| 1 | RQHVQHQLL | 9.600 | |
| 7 | QLLVILKEL | 9.240 | |
| 4 | VQHQLLVIL | 4.800 | |
| 3 | HVQHQLLVI | 1.500 | |
| 6 | HQLLVILKE | 0.023 | |
| 8 | LLVILKELR | 0.018 | |
| 9 | LVILKELRK | 0.015 | |
| 2 | QHVQHQLLV | 0.015 | |
| 5 | QHQLLVILK | 0.002 | |

| Table XIII-V8-A24-9mers: 121P2A3 | | | |
|---|---|---|---|
| Pos | 123456789 | Score | SeqID |
| 3 | PTAALNGSL | 0.480 | |
| 9 | GSLVECPKC | 0.165 | |
| 6 | ALNGSLVEC | 0.165 | |
| 2 | SPTAALNGS | 0.120 | |
| 4 | TAALNGSLV | 0.100 | |
| 1 | KSPTAALNG | 0.030 | |
| 5 | AALNGSLVE | 0.015 | |
| 8 | NGSLVECPK | 0.014 | |
| 7 | LNGSLVECP | 0.012 | |

| Table XIV-V1-A24-10mers:121P2A3 | | | |
|---|---|---|---|
| Pos | 1234567890 | Score | SeqID |
| 446 | QYPATEHRDL | 300.000 | |
| 193 | VYVKGLLAKI | 99.000 | |
| 196 | KGLLAKIFEL | 13.200 | |
| 388 | RNQITQLESL | 12.000 | |
| 119 | KALSEEKDVL | 12.000 | |
| 227 | GYLQEEKQKC | 9.900 | |
| 50 | KLTDKERHRL | 9.600 | |
| 375 | HQLHVILKEL | 9.240 | |
| 176 | DALEKNQQWL | 8.640 | |
| 92 | KARYSTTALL | 8.000 | |
| 253 | QTITQLSFEL | 7.920 | |
| 359 | CTLDFENEKL | 7.920 | |
| 169 | EMEIQLKDAL | 7.200 | |
| 342 | QQEEQTRVAL | 7.200 | |
| 330 | SQVQFLYTSL | 7.200 | |
| 372 | HVQHQLHVIL | 7.200 | |
| 239 | DLLASAKKDL | 6.000 | |
| 249 | EVERQTITQL | 6.000 | |
| 165 | NNIHEMEIQL | 6.000 | |
| 424 | ASPKSPTAAL | 6.000 | |
| 331 | QVQFLYTSLL | 6.000 | |
| 391 | ITQLESLKQL | 6.000 | |
| 186 | VYDQQREVYV | 5.000 | |
| 428 | SPTAALNESL | 4.800 | |
| 95 | YSTTALLEQL | 4.800 | |
| 189 | QQREVYVKGL | 4.800 | |
| 285 | SQRRADVQHL | 4.800 | |
| 133 | SAATSRIAEL | 4.400 | |
| 447 | YPATEHRDLL | 4.000 | |
| 51 | LTDKERHRLL | 4.000 | |
| 151 | LSQTVAPNCF | 3.600 | |
| 256 | TQLSFELSEF | 3.300 | |
| 403 | FAITEPLVTF | 3.000 | |
| 354 | QQMQACTLDF | 3.000 | |
| 194 | YVKGLLAKIF | 2.400 | |
| 25 | TTLEKLKGEI | 2.376 | |
| 436 | SLVECPKCNI | 1.800 | |
| 268 | KYEETQKEVH | 1.800 | |
| 274 | KEVHNLNQLL | 1.728 | |
| 163 | SINNIHEMEI | 1.650 | |
| 75 | YQLTEKDKEI | 1.650 | |
| 130 | QQLSAATSRI | 1.500 | |
| 155 | VAPNCFNSSI | 1.500 | |
| 82 | KEIQRLRDQL | 1.440 | |
| 158 | NCFNSSINNI | 1.200 | |
| 304 | IQKLREENDI | 1.200 | |
| 109 | REGERREQVL | 1.152 | |
| 94 | RYSTTALLEQ | 1.100 | |
| 236 | CYNDLLASAK | 1.080 | |
| 298 | RHKTEKIQKL | 1.056 | |

| Table XIV-V1-A24-10mers:121P2A3 | | | |
|---|---|---|---|
| Pos | 1234567890 | Score | SeqID |
| 1 | MSSRSTKDLI | 1.000 | |
| 396 | SLKQLHEFAI | 1.000 | |
| 207 | KKTETAAHSL | 0.960 | |
| 140 | AELESKTNTL | 0.864 | |
| 190 | QREVYVKGLL | 0.840 | |
| 400 | LHEFAITEPL | 0.840 | |
| 202 | IFELEKKTET | 0.825 | |
| 74 | AYQLTEKDKE | 0.825 | |
| 385 | RKARNQITQL | 0.800 | |
| 309 | EENDIARGKL | 0.792 | |
| 273 | QKEVHNLNQL | 0.720 | |
| 326 | EELLSQVQFL | 0.720 | |
| 335 | LYTSLLKQQE | 0.720 | |
| 123 | EEKDVLKQQL | 0.691 | |
| 112 | ERREQVLKAL | 0.672 | |
| 42 | DEITSGKGKL | 0.660 | |
| 319 | EEEKKRSEEL | 0.660 | |
| 57 | HRLLEKIRVL | 0.600 | |
| 28 | EKLKGEIAHL | 0.600 | |
| 18 | PSNSKSETTL | 0.600 | |
| 352 | LEQQMQACTL | 0.600 | |
| 343 | QEEQTRVALL | 0.600 | |
| 232 | EKQKCYNDLL | 0.600 | |
| 78 | TEKDKEIQRL | 0.576 | |
| 368 | LDRQHVQHQL | 0.560 | |
| 161 | NSSINNIHEM | 0.550 | |
| 21 | SKSETTLEKL | 0.528 | |
| 402 | EFAITEPLVT | 0.500 | |
| 283 | LYSQRRADVQ | 0.500 | |
| 231 | EEKQKCYNDL | 0.480 | |
| 68 | AEKEKNAYQL | 0.480 | |
| 220 | TKKPESEGYL | 0.480 | |
| 246 | KDLEVERQTI | 0.432 | |
| 91 | LKARYSTTAL | 0.400 | |
| 320 | EEKKRSEELL | 0.400 | |
| 142 | LESKTNTLRL | 0.400 | |
| 270 | EETQKEVHNL | 0.400 | |
| 427 | KSPTAALNES | 0.396 | |
| 64 | RVLEAEKEKN | 0.396 | |
| 382 | KELRKARNQI | 0.360 | |
| 341 | KQQEEQTRVA | 0.360 | |
| 251 | ERQTITQLSF | 0.300 | |
| 145 | KTNTLRLSQT | 0.300 | |
| 325 | SEELLSQVQF | 0.300 | |
| 31 | KGEIAHLKTS | 0.300 | |
| 86 | RLRDQLKARY | 0.288 | |
| 150 | RLSQTVAPNC | 0.280 | |
| 54 | KERHRLLEKI | 0.264 | |
| 271 | ETQKEVHNLN | 0.252 | |
| 138 | RIAELESKTN | 0.240 | |

| Table XIV-V3-A24-10mers: 121P2A3 | | | |
|---|---|---|---|
| Pos | 1234567890 | Score | SeqID |
| 5 | KLTDKERQRL | 11.520 | |
| 6 | LTDKERQRLL | 4.000 | |
| 12 | QRLLEKIRVL | 0.600 | |
| 9 | KERQRLLEKI | 0.264 | |
| 11. | RQRLLSKIRV | 0.200 | |
| 3 | KGKLTDKERQ | 0.020 | |
| 1 | SGKGKLTDKE | 0.013 | |
| 10 | ERQRLLEKIR | 0.002 | |
| 8 | DKERQRLLEK | 0.002 | |
| 4 | GKLTDKERQR | 0.002 | |
| 7 | TDKERQRLLE | 0.001 | |
| 2 | GKGKLTDKER | 0.001 | |

| Table XIV-V4-A24-10mers: 121P2A3 | | | |
|---|---|---|---|
| Pos | 1234567890 | Score | SeqID |
| 3 | KARYSTTTLL | 8.000 | |
| 6 | YSTTTLLEQL | 4.800 | |
| 5 | RYSTTTLLEQ | 1.100 | |
| 2 | LKARYSTTTL | 0.400 | |
| 10 | TLLEQLEETT | 0.216 | |
| 9 | TTLLEQLEET | 0.165 | |
| 1 | QLKARYSTTT | 0.100 | |
| 7 | STTTLLEQLE | 0.014 | |
| 8 | TTTLLEQLEE | 0.011 | |
| 4 | ARYSTTTLLE | 0.001 | |

| Table XIV-V6-A24-10mers: 121P2A3 | | | |
|---|---|---|---|
| Pos | 1234567890 | Score | SeqID |
| 6 | SQVQSLYTSL | 7.200 | |
| 7 | QVQSLYTSLL | 6.000 | |
| 2 | EELLSQVQSL | 0.720 | |
| 3 | ELLSQVQSLY | 0.210 | |
| 5 | LSQVQSLYTS | 0.150 | |
| 4 | LLSQVQSLYT | 0.100 | |

(continued)

| Table XIV-V6-A24-10mers: 121P2A3 | | | |
|---|---|---|---|
| Pos | 1234567890 | Score | SeqID |
| 9 | QSLYTSLLKQ | 0.017 | |
| 1 | SEELLSQVQS | 0.015 | |
| 10 | SLYTSLLKQQ | 0.012 | |
| 8 | VQSLYTSLLK | 0.010 | |

| Table XIV-V7-A24-10mers | | | |
|---|---|---|---|
| Pos | 1234567890 | Score | SeqID |
| 7 | HQLLVILKEL | 9.240 | |
| 4 | HVQHQLLVIL | 7.200 | |
| 1 | DRQHVQHQLL | 0.720 | |
| 2 | RQHVQHQLLV | 0.200 | |
| 10 | LVILKELRKA | 0.165 | |
| 3 | QHVQHQLLVI | 0.150 | |
| 8 | QLLVILKELR | 0.018 | |
| 9 | LLVILKELRK | 0.015 | |
| 5 | VQHQLLVILK | 0.012 | |
| 6 | QHQLLVILKE | 0.002 | |

| Table XIV-V8-A24-10mers: 121P2A3 | | | |
|---|---|---|---|
| Pos | 1234567890 | Score | SeqID |
| 3 | SPTAALNGSL | 4.800 | |
| 2 | KSPTAALNGS | 0.360 | |
| 6 | AALNGSLVEC | 0.165 | |
| 10 | GSLVECPKCN | 0.150 | |
| 9 | NGSLVECPKC | 0.110 | |
| 7 | ALNGSLVECP | 0.018 | |
| 8 | LNGSLVECPK | 0.014 | |
| 4 | PTAALNGSLV | 0.010 | |
| 5 | TAALNGSLVE | 0.010 | |
| 1 | PKSPTAALNG | 0.000 | |

| Table XV-V1-B7-9mers: 121P2A3 | | | |
|---|---|---|---|
| Pos | 123456789 | Score | SeqID |
| 92 | KARYSTTAL | 120.000 | |
| 425 | SPKSPTAAL | 120.000 | |
| 386 | KARNQITQL | 120.000 | |
| 447 | YPATEHRDL | 80.000 | |
| 134 | AATSRIAEL | 36.000 | |
| 156 | APNCFNSSI | 24.000 | |
| 331 | QVQFLYTSL | 20.000 | |
| 275 | EVHNLNQLL | 20.000 | |
| 120 | ALSEEKDVL | 12.000 | |
| 383 | ELRKARNQI | 6.000 | |
| 83 | EIQRLRDQL | 6.000 | |
| 348 | RVALLEQQM | 5.000 | |
| 2 | SSRSTKDLI | 4.000 | |
| 389 | NQITQLESL | 4.000 | |
| 376 | QLHVILKEL | 4.000 | |
| 392 | TQLESLKQL | 4.000 | |
| 327 | ELLSQVQFL | 4.000 | |
| 166 | NIHEMEIQL | 4.000 | |
| 197 | GLLAKIFEL | 4.000 | |
| 233 | KQKCYNDLL | 4.000 | |
| 332 | VQFLYTSLL | 4.000 | |
| 58 | RLLEKIRVL | 4.000 | |
| 96 | STTALLEQL | 4.000 | |
| 240 | LLASAKKDL | 4.000 | |
| 254 | TITQLSFEL | 4.000 | |
| 353 | EQQMQACTL | 4.000 | |
| 1 | MSSRSTKDL | 4.000 | |
| 143 | ESKTNTLRL | 4.000 | |
| 29 | KLKGEIAHL | 4.000 | |
| 43 | EITSGKGKL | 4.000 | |
| 19 | SNSKSETTL | 4.000 | |
| 250 | VERQTITQL | 4.000 | |
| 286 | QRRADVQHL | 4.000 | |
| 271 | ETQKEVHNL | 4.000 | |
| 373 | VQHQLHVIL | 4.000 | |
| 177 | ALEKNQQWL | 3.600 | |
| 194 | YVKGLLAKI | 2.000 | |
| 372 | HVQHQLHVI | 2.000 | |
| 17 | KPSNSKSET | 2.000 | |
| 448 | PATEHRDLL | 1.800 | |
| 310 | ENDIARGKL | 1.800 | |
| 51 | LTDKERHRL | 1.800 | |
| 141 | ELESKTNTL | 1.200 | |
| 360 | TLDFENEKL | 1.200 | |
| 93 | ARYSTTALL | 1.200 | |
| 208 | KTETAAHSL | 1.200 | |
| 22 | KSETTLEKL | 1.200 | |
| 110 | EGERREQVL | 1.200 | |
| 162 | SSINNIHEM | 1.000 | |
| 148 | TLRLSQTVA | 1.000 | |

| Table XV-V1-B7-9mers: 121P2A3 | | | |
|---|---|---|---|
| Pos | 123456789 | Score | SeqID |
| 185 | LVYDQQREV | 1.000 | |
| 306 | KLREENDIA | 1.000 | |
| 437 | LVECPKCNI | 0.900 | |
| 212 | AAHSLPQQT | 0.900 | |
| 423 | AASPKSPTA | 0.900 | |
| 242 | ASAKKDLEV | 0.600 | |
| 119 | KALSEEKDV | 0.600 | |
| 430 | TAALNESLV | 0.600 | |
| 126 | DVLKQQLSA | 0.500 | |
| 192 | EVYVKGLLA | 0.500 | |
| 422 | VAASPKSPT | 0.450 | |
| 221 | KKPESEGYL | 0.400 | |
| 191 | REVYVKGLL | 0.400 | |
| 344 | EEQTRVALL | 0.400 | |
| 369 | DRQHVQHQL | 0.400 | |
| 299 | HKTEKIQKL | 0.400 | |
| 429 | PTAALNESL | 0.400 | |
| 164 | INNIHEMEI | 0.400 | |
| 274 | KEVHNLNQL | 0.400 | |
| 131 | QLSAATSRI | 0.400 | |
| 76 | QLTEKDKEI | 0.400 | |
| 36 | HLKTSVDEI | 0.400 | |
| 296 | DDRHKTEKI | 0.400 | |
| 232 | EKQKCYNDL | 0.400 | |
| 170 | MEIQLKDAL | 0.400 | |
| 321 | EKKRSEELL | 0.400 | |
| 401 | HEFAITEPL | 0.400 | |
| 320 | EEKKRSEEL | 0.400 | |
| 52 | TDKERHRLL | 0.400 | |
| 428 | SPTAALNES | 0.400 | |
| 403 | FAITEPLVT | 0.300 | |
| 99 | ALLEQLEET | 0.300 | |
| 424 | ASPKSPTAA | 0.300 | |
| 432 | ALNESLVEC | 0.300 | |
| 313 | IARGKLEEE | 0.300 | |
| 350 | ALLEQQMQA | 0.300 | |
| 136 | TSRIAELES | 0.200 | |
| 440 | CPKCNIQYP | 0.200 | |
| 216 | LPQQTKKPE | 0.200 | |
| 407 | EPLVTFQGE | 0.200 | |
| 451 | EHRDLLVHV | 0.200 | |
| 33 | EIAHLKTSV | 0.200 | |
| 147 | NTLRLSQTV | 0.200 | |
| 417 | ENREKVAAS | 0.200 | |
| 341 | KQQEEQTRV | 0.200 | |
| 343 | QEEQTRVAL | 0.180 | |
| 449 | ATEHRDLLV | 0.180 | |
| 247 | DLEVERQTI | 0.180 | |
| 89 | DQLKARYST | 0.150 | |
| 124 | EKDVLKQQL | 0.120 | |

| Table XV-V3-B7-9mers: 121P2A3 | | | |
|---|---|---|---|
| Pos | 123456789 | Score | SeqID |
| 3 | LTDKERQRL | 1.800 | |
| 4 | TDKERQRLL | 0.400 | |
| 8 | RQRLLEKIR | 0.100 | |
| 7 | ERQRLLEKI | 0.040 | |
| 9 | QRLLEKIRV | 0.020 | |
| 2 | KLTDKERQR | 0.010 | |
| 6 | KERQRLLEK | 0.010 | |
| 1 | GKLTDKERQ | 0.001 | |
| 5 | DKERQRLLE | 0.000 | |

| Table XV-V4-B7-9mers: 121P2A3 | | | |
|---|---|---|---|
| Pos | 123456789 | Score | SeqID |
| 2 | KARYSTTTL | 120.000 | |
| 6 | STTTLLEQL | 4.000 | |
| 3 | ARYSTTTLL | 1.200 | |
| 9 | TLLEQLEET | 0.100 | |
| 8 | TTLLEQLEE | 0.010 | |
| 7 | TTTLLEQLE | 0.010 | |
| 5 | YSTTTLLEQ | 0.010 | |
| 1 | LKARYSTTT | 0.010 | |
| 4 | RYSTTTLLE | 0.001 | |

| Table XV-V6-B7-9mers: 121P2A3 | | | |
|---|---|---|---|
| Pos | 123456789 | Score | SeqID |
| 6 | QVQSLYTSL | 20.000 | |
| 7 | VQSLYTSLL | 4.000 | |
| 2 | ELLSQVQSL | 4.000 | |
| 4 | LSQVQSLYT | 0.100 | |
| 5 | SQVQSLYTS | 0.020 | |
| 3 | LLSQVQSLY | 0.020 | |
| 8 | QSLYTSLLK | 0.010 | |
| 9 | SLYTSLLKQ | 0.010 | |
| 1 | EELLSQVQS | 0.002 | |

| Table XV-V7-B7-9mers: 121P2A3 | | | |
|---|---|---|---|
| Pos | 123456789 | Score | SeqID |
| 1 | RQHVQHQLL | 4.000 | |
| 4 | VQHQLLVIL | 4.000 | |
| 7 | QLLVILKEL | 4.000 | |
| 3 | HVQHQLLVI | 2.000 | |
| 9 | LVILKELRK | 0.050 | |
| 2 | QHVQHQLLV | 0.020 | |
| 6 | HQLLVILKE | 0.010 | |
| 8 | LLVILKELR | 0.010 | |
| 5 | QHQLLVILK | 0.001 | |

| Table XV-V8-B7-9mers: 121P2A3 | | | |
|---|---|---|---|
| Pos | 123456789 | Score | SeqID |
| 4 | TAALNGSLV | 0.600 | |
| 2 | SPTAALNGS | 0.400 | |
| 3 | PTAALNGSL | 0.400 | |
| 6 | ALNGSLVEC | 0.300 | |
| 9 | GSLVECPKC | 0.100 | |
| 5 | AALNGSLVE | 0.090 | |
| 7 | LNGSLVECP | 0.010 | |
| 8 | NGSLVECPK | 0.010 | |
| 1 | KSPTAALNG | 0.010 | |

| Table XVI-V1-B7-10mers: 121P2A3 | | | |
|---|---|---|---|
| Pos | 1234567890 | Score | SeqID |
| 447 | YPATEHRDLL | 120.000 | |
| 92 | KARYSTTALL | 120.000 | |
| 428 | SPTAALNESL | 80.000 | |
| 189 | QQREVYVKGL | 40.000 | |
| 285 | SQRRADVQHL | 40.000 | |
| 372 | HVQHQLHVIL | 20.000 | |
| 331 | QVQFLYTSLL | 20.000 | |
| 424 | ASPKSPTAAL | 18.000 | |
| 176 | DALEKNQQWL | 12.000 | |
| 119 | KALSEEKDVL | 12.000 | |
| 133 | SAATSRIAEL | 12.000 | |
| 249 | EVERQTITQL | 6.000 | |
| 50 | KLTDKERHRL | 6.000 | |
| 388 | RNQITQLESL | 4.000 | |
| 391 | ITQLESLKQL | 4.000 | |
| 368 | LDRQHVQHQL | 4.000 | |
| 196 | KGLLAKIFEL | 4.000 | |
| 253 | QTITQLSFEL | 4.000 | |
| 239 | DLLASAKKDL | 4.000 | |
| 112 | ERREQVLKAL | 4.000 | |
| 359 | CTLDFENEKL | 4.000 | |
| 165 | NNIHEMEIQL | 4.000 | |
| 95 | YSTTALLEQL | 4.000 | |
| 375 | HQLHVILKEL | 4.000 | |
| 330 | SQVQFLYTSL | 4.000 | |
| 17 | KPSNSKSETT | 2.000 | |
| 407 | EPLVTFQGET | 2.000 | |
| 440 | CPKCNIQYPA | 2.000 | |
| 342 | QQEEQTRVAL | 1.800 | |
| 156 | APNCFNSSIN | 1.200 | |
| 140 | AELESKTNTL | 1.200 | |
| 68 | AEKEKNAYQL | 1.200 | |
| 51 | LTDKERHRLL | 1.200 | |
| 155 | VAPNCFNSSI | 1.200 | |
| 169 | EMEIQLKDAL | 1.200 | |
| 84 | IQRLRDQLKA | 1.000 | |
| 161 | NSSINNIHEM | 1.000 | |
| 383 | ELRKARNQIT | 1.000 | |
| 431 | AALNESLVEC | 0.900 | |
| 423 | AASPKSPTAA | 0.900 | |
| 421 | KVAASPKSPT | 0.750 | |
| 241 | LASAKKDLEV | 0.600 | |
| 82 | KEIQRLRDQL | 0.600 | |
| 309 | EENDIARGKL | 0.600 | |
| 436 | SLVECPKCNI | 0.600 | |
| 378 | HVILKELRKA | 0.500 | |
| 126 | DVLKQQLSAA | 0.500 | |
| 216 | LPQQTKKPES | 0.400 | |
| 220 | TKKPESEGYL | 0.400 | |
| 91 | LKARYSTTAL | 0.400 | |

| Table XVI-V1-B7-10mers:121P2A3 | | | |
|---|---|---|---|
| Pos | 1234567890 | Score | SeqID |
| 304 | IQKLREENDI | 0.400 | |
| 274 | KEVHNLNQLL | 0.400 | |
| 130 | QQLSAATSRI | 0.400 | |
| 326 | EELLSQVQFL | 0.400 | |
| 207 | KKTETAAHSL | 0.400 | |
| 18 | PSNSKSETTL | 0.400 | |
| 25 | TTLEKLKGEI | 0.400 | |
| 320 | EEKKRSEELL | 0.400 | |
| 352 | LEQQMQACTL | 0.400 | |
| 425 | SPKSPTAALN | 0.400 | |
| 78 | TEKDKEIQRL | 0.400 | |
| 28 | EKLKGEIAHL | 0.400 | |
| 75 | YQLTEKDKEI | 0.400 | |
| 396 | SLKQLHEFAI | 0.400 | |
| 109 | REGERREQVL | 0.400 | |
| 231 | EEKQKCYNDL | 0.400 | |
| 385 | RKARNQITQL | 0.400 | |
| 142 | LESKTNTLRL | 0.400 | |
| 232 | EKQKCYNDLL | 0.400 | |
| 54 | KERHRLLEKI | 0.400 | |
| 298 | RHKTEKIQKL | 0.400 | |
| 270 | EETQKEVHNL | 0.400 | |
| 42 | DEITSGKGKL | 0.400 | |
| 21 | SKSETTLEKL | 0.400 | |
| 446 | QYPATEHRDL | 0.400 | |
| 1 | MSSRSTKDLI | 0.400 | |
| 57 | HRLLEKIRVL | 0.400 | |
| 158 | NCFNSSINNI | 0.400 | |
| 123 | EEKDVLKQQL | 0.400 | |
| 163 | SINNIHEMEI | 0.400 | |
| 313 | IARGKLEEEK | 0.300 | |
| 98 | TALLEQLEET | 0.300 | |
| 349 | VALLEQQMQA | 0.300 | |
| 282 | LLYSQRRADV | 0.300 | |
| 422 | VAASPKSPTA | 0.300 | |
| 386 | KARNQITQLE | 0.300 | |
| 99 | ALLEQLEETT | 0.300 | |
| 211 | TAAHSLPQQT | 0.300 | |
| 350 | ALLEQQMQAC | 0.300 | |
| 86 | RLRDQLKARY | 0.200 | |
| 56 | RHRLLEKIRV | 0.200 | |
| 184 | WLVYDQQREV | 0.200 | |
| 370 | RQHVQHQLHV | 0.200 | |
| 146 | TNTLRLSQTV | 0.200 | |
| 177 | ALEKNQQWLV | 0.180 | |
| 107 | TTREGERREQ | 0.150 | |
| 185 | LVYDQQREVY | 0.150 | |
| 212 | AAHSLPQQTK | 0.135 | |
| 35 | AHLKTSVDEI | 0.120 | |
| 400 | LHEFAITEPL | 0.120 | |

| Table XVI-V3-B7-10mers: 121P2A3 | | | |
|---|---|---|---|
| Pos | 1234567890 | Score | SeqID |
| 5 | KLTDKERQRL | 6.000 | |
| 11 | RQRLLEKIRV | 2.000 | |
| 6 | LTDKERQRLL | 1.200 | |
| 9 | KERQRLLEKI | 0.400 | |
| 12 | QRLLEKIRVL | 0.400 | |
| 1 | SGKGKLTDKE | 0.010 | |
| 3 | KGKLTDKERQ | 0.010 | |
| 7 | TDKERQRLLE | 0.002 | |
| 4 | GKLTDKERQR | 0.001 | |
| 10 | ERQRLLEKIR | 0.001 | |
| 2 | GKGKLTDKER | 0.001 | |
| 8 | DKERQRLLEK | 0.000 | |

| Table XVI-V4-B7-10mers: 121P2A3 | | | |
|---|---|---|---|
| Pos | 1234567890 | Score | SeqID |
| 3 | KARYSTTTLL | 120.000 | |
| 6 | YSTTTLLEQL | 4.000 | |
| 2 | LKARYSTTTL | 0.400 | |
| 9 | TTLLEQLEET | 0.100 | |
| 1 | QLKARYSTTT | 0.100 | |
| 10 | TLLEQLEETT | 0.100 | |
| 7 | STTTLLEQLE | 0.010 | |
| 8 | TTTLLEQLEE | 0.010 | |
| 4 | ARYSTTTLLE | 0.003 | |
| 5 | RYSTTTLLEQ | 0.001 | |

| Table XVI-V6-B7-10mers: 121P2A3 | | | |
|---|---|---|---|
| Pos | 1234567890 | Score | SeqID |
| 7 | QVQSLYTSLL | 20.000 | |
| 6 | SQVQSLYTSL | 4.000 | |
| 2 | EELLSQVQSL | 0.400 | |
| 4 | LLSQVQSLYT | 0.100 | |
| 5 | LSQVQSLYTS | 0.020 | |
| 3 | ELLSQVQSLY | 0.020 | |
| 10 | SLYTSLLKQQ | 0.010 | |
| 8 | VQSLYTSLLK | 0.010 | |

(continued)

| Table XVI-V6-B7-10mers: 121P2A3 | | | |
|---|---|---|---|
| Pos | 1234567890 | Score | SeqID |
| 9 | QSLYTSLLKQ | 0.010 | |
| 1 | SEELLSQVQS | 0.001 | |

| Table XVI-V7-B7-10mers: 121P2A3 | | | |
|---|---|---|---|
| Pos | 1234567890 | Score | SeqID |
| 4 | HVQHQLLVIL | 20.000 | |
| 7 | HQLLVILKEL | 4.000 | |
| 10 | LVILKELRKA | 0.500 | |
| 1 | DRQHVQHQLL | 0.400 | |
| 2 | RQHVQHQLLV | 0.200 | |
| 3 | QHVQHQLLVI | 0.040 | |
| 8 | QLLVILKELR | 0.010 | |
| 9 | LLVILKELRK | 0.010 | |
| 5 | VQHQLLVILK | 0.010 | |
| 6 | QHQLLVILKE | 0.001 | |

| Table XVI-V8-B7-10mers: 121P2A3 | | | |
|---|---|---|---|
| Pos | 1234567890 | Score | SeqID |
| 3 | SPTAALNGSL | 80.000 | |
| 6 | AALNGSLVEC | 0.900 | |
| 9 | NGSLVECPKC | 0.100 | |
| 7 | ALNGSLVECP | 0.030 | |
| 5 | TAALNGSLVE | 0.030 | |
| 10 | GSLVECPKCN | 0.020 | |
| 4 | PTAALNGSLV | 0.020 | |
| 2 | KSPTAALNGS | 0.020 | |
| 8 | LNGSLVECPK | 0.010 | |
| 1 | PKSPTAALNG | 0.000 | |

| Table XVII-V1-B35-9mers:121P2A3 | | | |
|---|---|---|---|
| Pos | 123456789 | Score | SeqID |
| 425 | SPKSPTAAL | 60.000 | |
| 447 | YPATEHRDL | 30.000 | |
| 92 | KARYSTTAL | 18.000 | |
| 386 | KARNQITQL | 18.000 | |
| 143 | ESKTNTLRL | 15.000 | |
| 162 | SSINNIHEM | 10.000 | |
| 29 | KLKGEIAHL | 9.000 | |
| 156 | APNCFNSSI | 8.000 | |
| 233 | KQKCYNDLL | 6.000 | |
| 2 | SSRSTKDLI | 6.000 | |
| 1 | MSSRSTKDL | 5.000 | |
| 395 | ESLKQLHEF | 5.000 | |
| 348 | RVALLEQQM | 4.000 | |
| 17 | KPSNSKSET | 4.000 | |
| 261 | ELSEFRRKY | 4.000 | |
| 58 | RLLEKIRVL | 4.000 | |
| 120 | ALSEEKDVL | 3.000 | |
| 11 | KSKWGSKPS | 3.000 | |
| 22 | KSETTLEKL | 3.000 | |
| 176 | DALEKNQQW | 3.000 | |
| 134 | AATSRIAEL | 3.000 | |
| 67 | EAEKEKNAY | 2.700 | |
| 428 | SPTAALNES | 2.000 | |
| 439 | ECPKCNIQY | 2.000 | |
| 166 | NIHEMEIQL | 2.000 | |
| 404 | AITEPLVTF | 2.000 | |
| 328 | LLSQVQFLY | 2.000 | |
| 392 | TQLESLKQL | 2.000 | |
| 252 | RQTITQLSF | 2.000 | |
| 119 | KALSEEKDV | 1.800 | |
| 306 | KLREENDIA | 1.800 | |
| 257 | QLSFELSEF | 1.500 | |
| 15 | GSKPSNSKS | 1.500 | |
| 271 | ETQKEVHNL | 1.500 | |
| 4 | RSTKDLIKS | 1.500 | |
| 136 | TSRIAELES | 1.500 | |
| 383 | ELRKARNQI | 1.200 | |
| 36 | HLKTSVDEI | 1.200 | |
| 341 | KQQEEQTRV | 1.200 | |
| 194 | YVKGLLAKI | 1.200 | |
| 229 | LQEEKQKCY | 1.200 | |
| 324 | RSEELLSQV | 1.200 | |
| 43 | EITSGKGKL | 1.000 | |
| 254 | TITQLSFEL | 1.000 | |
| 275 | EVHNLNQLL | 1.000 | |
| 355 | QMQACTLDF | 1.000 | |
| 83 | EIQRLRDQL | 1.000 | |
| 152 | SQTVAPNCF | 1.000 | |
| 331 | QVQFLYTSL | 1.000 | |
| 353 | EQQMQACTL | 1.000 | |

| Table XVII-V1-B35-9mers:121P2A3 | | | |
|---|---|---|---|
| Pos | 123456789 | Score | SeqID |
| 389 | NQITQLESL | 1.000 | |
| 240 | LLASAKKDL | 1.000 | |
| 19 | SNSKSETTL | 1.000 | |
| 376 | QLHVILKEL | 1.000 | |
| 242 | ASAKKDLEV | 1.000 | |
| 373 | VQHQLHVIL | 1.000 | |
| 96 | STTALLEQL | 1.000 | |
| 332 | VQFLYTSLL | 1.000 | |
| 197 | GLLAKIFEL | 1.000 | |
| 327 | ELLSQVQFL | 1.000 | |
| 220 | TKKPESEGY | 0.900 | |
| 76 | QLTEKDKEI | 0.800 | |
| 435 | ESLVECPKC | 0.750 | |
| 417 | ENREKVAAS | 0.600 | |
| 52 | TDKERHRLL | 0.600 | |
| 440 | CPKCNIQYP | 0.600 | |
| 430 | TAALNESLV | 0.600 | |
| 208 | KTETAAHSL | 0.600 | |
| 272 | TQKEVHNLN | 0.600 | |
| 448 | PATEHRDLL | 0.600 | |
| 173 | QLKDALEKN | 0.600 | |
| 424 | ASPKSPTAA | 0.500 | |
| 329 | LSQVQFLYT | 0.500 | |
| 151 | LSQTVAPNC | 0.500 | |
| 132 | LSAATSRIA | 0.500 | |
| 286 | QRRADVQHL | 0.450 | |
| 51 | LTDKERHRL | 0.450 | |
| 360 | TLDFENEKL | 0.450 | |
| 403 | FAITEPLVT | 0.450 | |
| 131 | QLSAATSRI | 0.400 | |
| 138 | RIAELESKT | 0.400 | |
| 201 | KIFELEKKT | 0.400 | |
| 221 | KKPESEGYL | 0.400 | |
| 185 | LVYDQQREV | 0.400 | |
| 164 | INNIHEMEI | 0.400 | |
| 453 | RDLLVHVEY | 0.400 | |
| 372 | HVQHQLHVI | 0.400 | |
| 110 | EGERREQVL | 0.300 | |
| 398 | KQLHEFAIT | 0.300 | |
| 396 | SLKQLHEFA | 0.300 | |
| 155 | VAPNCFNSS | 0.300 | |
| 339 | LLKQQEEQT | 0.300 | |
| 127 | VLKQQLSAA | 0.300 | |
| 177 | ALEKNQQWL | 0.300 | |
| 422 | VAASPKSPT | 0.300 | |
| 310 | ENDIARGKL | 0.300 | |
| 148 | TLRLSQTVA | 0.300 | |
| 250 | VERQTITQL | 0.300 | |
| 212 | AAHSLPQQT | 0.300 | |
| 141 | ELESKTNTL | 0.300 | |

| Table XVII-V3-B35-9mers: 121P2A3 | | | |
|---|---|---|---|
| Pos | 123456789 | Score | SeqID |
| 4 | TDKERQRLL | 0.600 | |
| 3 | LTDKERQRL | 0.450 | |
| 8 | RQRLLEKIR | 0.060 | |
| 7 | ERQRLLEKI | 0.040 | |
| 2 | KLTDKERQR | 0.040 | |
| 9 | QRLLEKIRV | 0.030 | |
| 6 | KERQRLLEK | 0.006 | |
| 1 | GKLTDKERQ | 0.002 | |
| 5 | DKERQRLLE | 0.000 | |

| Table XVII-V4-B35-9mers: 121P2A3 | | | |
|---|---|---|---|
| Pos | 123456789 | Score | SeqID |
| 2 | KARYSTTTL | 18.000 | |
| 6 | STTTLLEQL | 1.000 | |
| 9 | TLLEQLEET | 0.200 | |
| 3 | ARYSTTTLL | 0.100 | |
| 5 | YSTTTLLEQ | 0.050 | |
| 8 | TTLLEQLEE | 0.015 | |
| 7 | TTTLLEQLE | 0.010 | |
| 1 | LKARYSTTT | 0.010 | |
| 4 | RYSTTTLLE | 0.002 | |

| Table XVII-V6-B35-9mers: 121P2A3 | | | |
|---|---|---|---|
| Pos | 123456789 | Score | SeqID |
| 3 | LLSQVQSLY | 2.000 | |
| 2 | ELLSQVQSL | 1.000 | |
| 7 | VQSLYTSLL | 1.000 | |
| 6 | QVQSLYTSL | 1.000 | |
| 4 | LSQVQSLYT | 0.500 | |
| 5 | SQVQSLYTS | 0.100 | |
| 8 | QSLYTSLLK | 0.050 | |
| 9 | SLYTSLLKQ | 0.010 | |
| 1 | EELLSQVQS | 0.010 | |

| Table XVII-V7-B35-9mers: 121P2A3 | | | |
|---|---|---|---|
| Pos | 123456789 | Score | SeqID |
| 1 | RQHVQHQLL | 2.000 | |
| 4 | VQHQLLVIL | 1.000 | |
| 7 | QLLVILKEL | 1.000 | |
| 3 | HVQHQLLVI | 0.400 | |
| 2 | QHVQHQLLV | 0.020 | |
| 8 | LLVILKELR | 0.010 | |
| 6 | HQLLVILKE | 0.010 | |
| 9 | LVILKELRK | 0.010 | |
| 5 | QHQLLVILK | 0.001 | |

| Table XVII-V8-B35-9mers: 121P2A3 | | | |
|---|---|---|---|
| Pos | 123456789 | Score | SeqID |
| 2 | SPTAALNGS | 2.000 | |
| 9 | GSLVECPKC | 0.750 | |
| 4 | TAALNGSLV | 0.600 | |
| 6 | ALNGSLVEC | 0.100 | |
| 1 | KSPTAALNG | 0.100 | |
| 3 | PTAALNGSL | 0.100 | |
| 5 | AALNGSLVE | 0.030 | |
| 7 | LNGSLVECP | 0.010 | |
| 8 | NGSLVECPK | 0.010 | |

| Tbl:XVIII-V1-B35-10mers:121P2A3 | | | |
|---|---|---|---|
| Pos | 1234567890 | Score | SeqID |
| 86 | RLRDQLKARY | 24.000 | |
| 428 | SPTAALNESL | 20.000 | |
| 447 | YPATEHRDLL | 20.000 | |
| 92 | KARYSTTALL | 18.000 | |
| 161 | NSSINNIHEM | 10.000 | |
| 219 | QTKKPESEGY | 9.000 | |
| 119 | KALSEEKDVL | 9.000 | |
| 440 | CPKCNIQYPA | 6.000 | |
| 176 | DALEKNQQWL | 6.000 | |
| 50 | KLTDKERHRL | 6.000 | |
| 425 | SPKSPTAALN | 6.000 | |
| 189 | QQREVYVKGL | 6.000 | |
| 151 | LSQTVAPNCF | 5.000 | |
| 424 | ASPKSPTAAL | 5.000 | |
| 95 | YSTTALLEQL | 5.000 | |
| 285 | SQRRADVQHL | 4.500 | |
| 17 | KPSNSKSETT | 4.000 | |
| 228 | YLQEEKQKCY | 4.000 | |
| 185 | LVYDQQREVY | 4.000 | |
| 11 | KSKWGSKPSN | 3.000 | |
| 133 | SAATSRIAEL | 3.000 | |
| 5 | STKDLIKSKW | 3.000 | |
| 359 | CTLDFENEKL | 3.000 | |
| 194 | YVKGLLAKIF | 3.000 | |
| 403 | FAITEPLVTF | 3.000 | |
| 216 | LPQQTKKPES | 2.000 | |
| 388 | RNQITQLESL | 2.000 | |
| 407 | EPLVTFQGET | 2.000 | |
| 275 | EVHNLNQLLY | 2.000 | |
| 196 | KGLLAKIFEL | 2.000 | |
| 156 | APNCFNSSIN | 2.000 | |
| 1 | MSSRSTKDLI | 2.000 | |
| 327 | ELLSQVQFLY | 2.000 | |
| 304 | IQKLREENDI | 1.800 | |
| 143 | ESKTNTLRLS | 1.500 | |
| 256 | TQLSFELSEF | 1.500 | |
| 396 | SLKQLHEFAI | 1.200 | |
| 155 | VAPNCFNSSI | 1.200 | |
| 165 | NNIHEMEIQL | 1.000 | |
| 372 | HVQHQLHVIL | 1.000 | |
| 427 | KSPTAALNES | 1.000 | |
| 391 | ITQLESLKQL | 1.000 | |
| 330 | SQVQFLYTSL | 1.000 | |
| 354 | QQMQACTLDF | 1.000 | |
| 331 | QVQFLYTSLL | 1.000 | |
| 253 | QTITQLSFEL | 1.000 | |
| 375 | HQLHVILKEL | 1.000 | |
| 239 | DLLASAKKDL | 1.000 | |
| 78 | TEKDKEIQRL | 0.900 | |
| 436 | SLVECPKCNI | 0.800 | |

| Tbl:XVIII-V1-B35-10mers:121P2A3 | | | |
|---|---|---|---|
| Pos | 1234567890 | Score | SeqID |
| 25 | TTLEKLKGEI | 0.800 | |
| 298 | RHKTEKIQKL | 0.600 | |
| 64 | RVLEAEKEKN | 0.600 | |
| 68 | AEKEKNAYQL | 0.600 | |
| 342 | QQEEQTRVAL | 0.600 | |
| 138 | RIAELESKTN | 0.600 | |
| 178 | LEKNQQWLVY | 0.600 | |
| 241 | LASAKKDLEV | 0.600 | |
| 123 | EEKDVLKQQL | 0.600 | |
| 66 | LEAEKEKNAY | 0.600 | |
| 233 | KQKCYNDLLA | 0.600 | |
| 112 | ERREQVLKAL | 0.600 | |
| 380 | ILKELRKARN | 0.600 | |
| 395 | ESLKQLHEFA | 0.500 | |
| 435 | ESLVECPKCN | 0.500 | |
| 18 | PSNSKSETTL | 0.500 | |
| 329 | LSQVQFLYTS | 0.500 | |
| 84 | IQRLRDQLKA | 0.450 | |
| 109 | REGERREQVL | 0.400 | |
| 207 | KKTETAAHSL | 0.400 | |
| 341 | KQQEEQTRVA | 0.400 | |
| 75 | YQLTEKDKEI | 0.400 | |
| 163 | SINNIHEMEI | 0.400 | |
| 370 | RQHVQHQLHV | 0.400 | |
| 130 | QQLSAATSRI | 0.400 | |
| 158 | NCFNSSINNI | 0.400 | |
| 51 | LTDKERHRLL | 0.300 | |
| 231 | EEKQKCYNDL | 0.300 | |
| 98 | TALLEQLEET | 0.300 | |
| 169 | EMEIQLKDAL | 0.300 | |
| 423 | AASPKSPTAA | 0.300 | |
| 422 | VAASPKSPTA | 0.300 | |
| 127 | VLKQQLSAAT | 0.300 | |
| 320 | EEKKRSEELL | 0.300 | |
| 211 | TAAHSLPQQT | 0.300 | |
| 431 | AALNESLVEC | 0.300 | |
| 36 | HLKTSVDEIT | 0.300 | |
| 249 | EVERQTITQL | 0.300 | |
| 383 | ELRKARNQIT | 0.300 | |
| 349 | VALLEQQMQA | 0.300 | |
| 90 | QLKARYSTTA | 0.300 | |
| 368 | LDRQHVQHQL | 0.300 | |
| 220 | TKKPESEGYL | 0.300 | |
| 54 | KERHRLLEKI | 0.240 | |
| 246 | KDLEVERQTI | 0.240 | |
| 136 | TSRIAELESK | 0.225 | |
| 385 | RKARNQITQL | 0.200 | |
| 282 | LLYSQRRADV | 0.200 | |
| 438 | VECPKCNIQY | 0.200 | |
| 82 | KEIQRLRDQL | 0.200 | |

| Table XVIII-V3-B35-10mers: 121P2A3 | | | |
|---|---|---|---|
| Pos | 1234567890 | Score | SeqID |
| 5 | KLTDKERQRL | 6.000 | |
| 11 | RQRLLEKIRV | 1.800 | |
| 6 | LTDKERQRLL | 0.300 | |
| 9 | KERQRLLEKI | 0.240 | |
| 12 | QRLLEKIRVL | 0.100 | |
| 3 | KGKLTDKERQ | 0.090 | |
| 1 | SGKGKLTDKE | 0.030 | |
| 7 | TDKERQRLLE | 0.006 | |
| 4 | GKLTDKERQR | 0.001 | |
| 10 | ERQRLLEKIR | 0.001 | |
| 2 | GKGKLTDKER | 0.001 | |
| 8 | DKERQRLLEK | 0.000 | |

| Table XVIII-V4-B35-10mers: 121P2A3 | | | |
|---|---|---|---|
| Pos | 1234567890 | Score | SeqID |
| 3 | KARYSTTTLL | 18.000 | |
| 6 | YSTTTLLEQL | 5.000 | |
| 1 | QLKARYSTTT | 0.300 | |
| 10 | TLLEQLEETT | 0.200 | |
| 9 | TTLLEQLEET | 0.100 | |
| 2 | LKARYSTTTL | 0.100 | |
| 8 | TTTLLEQLEE | 0.015 | |
| 7 | STTTLLEQLE | 0.010 | |
| 5 | RYSTTTLLEQ | 0.002 | |
| 4 | ARYSTTTLLE | 0.001 | |

| Table XVIII-V6-B35-10mers: 121P2A3 | | | |
|---|---|---|---|
| Pos | 1234567890 | Score | SeqID |
| 3 | ELLSQVQSLY | 2.000 | |
| 7 | QVQSLYTSLL | 1.000 | |
| 6 | SQVQSLYTSL | 1.000 | |
| 5 | LSQVQSLYTS | 0.500 | |
| 2 | EELLSQVQSL | 0.100 | |
| 4 | LLSQVQSLYT | 0.100 | |

(continued)

| Table XVIII-V6-B35-10mers: 121P2A3 | | | |
|---|---|---|---|
| Pos | 1234567890 | Score | SeqID |
| 9 | QSLYTSLLKQ | 0.050 | |
| 10 | SLYTSLLKQQ | 0.010 | |
| 8 | VQSLYTSLLK | 0.010 | |
| 1 | SEELLSQVQS | 0.003 | |

| Table XVIII-V7-B35-10mers: 121P2A3 | | | |
|---|---|---|---|
| Pos | 1234567890 | Score | SeqID |
| 7 | HQLLVILKEL | 1.000 | |
| 4 | HVQHQLLVIL | 1.000 | |
| 2 | RQHVQHQLLV | 0.400 | |
| 10 | LVILKELRKA | 0.150 | |
| 1 | DRQHVQHQLL | 0.100 | |
| 3 | QHVQHQLLVI | 0.040 | |
| 8 | QLLVILKELR | 0.010 | |
| 9 | LLVILKELRK | 0.010 | |
| 5 | VQHQLLVILK | 0.010 | |
| 6 | QHQLLVILKE | 0.001 | |

| Table XVIII-V8-B35-10mers: 121P2A3 | | | |
|---|---|---|---|
| Pos | 1234567890 | Score | SeqID |
| 3 | SPTAALNGSL | 20.000 | |
| 2 | KSPTAALNGS | 1.000 | |
| 10 | GSLVECPKCN | 0.500 | |
| 6 | AALNGSLVEC | 0.300 | |
| 9 | NGSLVECPKC | 0.150 | |
| 5 | TAALNGSLVE | 0.030 | |
| 4 | PTAALNGSLV | 0.020 | |
| 7 | ALNGSLVECP | 0.010 | |
| 8 | LNGSLVECPK | 0.010 | |
| 1 | PKSPTAALNG | 0.000 | |

| Table XIX: Frequently Occurring Motifs | | | |
|---|---|---|---|
| Name | avrg.% identity | Description | Potential Function |
| zf-C2H2 | 34% | Zinc finger, C2H2 type | Nucleic acid-binding protein functions as transcription factor, nuclear location probable |
| cytochrome b N | 68% | Cytochrome b(N-terminal)/b6/petB | membrane bound oxidase, generate superoxide |
| ig | 19% | Immunoglobulin domain | domains are one hundred amino acids long and include a conserved intradomain disulfide bond. |
| WD40 | 18% | WD domain, G-beta repeat | tandem repeats of about 40 residues, each containing a Trp-Asp motif. Function in signal transduction and protein interaction |
| PDZ | 23% | PDZ domain | may function in targeting signaling molecules to sub-membranous sites |
| LRR | 28% | Leucine Rich Repeat | short sequence motifs involved in protein-protein interactions |
| pkinase | 23% | Protein kinase domain | conserved catalytic core common to both serine/threonine and tyrosine protein kinases containing an ATP binding site and a catalytic site |
| PH | 16% | PH domain | pleckstrin homology involved in intracellular signaling or as constituents of the cytoskeleton |
| EGF | 34% | EGF-like domain | 30-40 amino-acid long found in the extracellular domain of membrane-bound proteins or in secreted proteins |
| rvt | 49% | Reverse transcriptase (RNA-dependent DNA polymerase) | |
| ank | 25% | Ank repeat | Cytoplasmic protein, associates integral membrane proteins to the cytoskeleton |
| oxidored q1 | 32% | NADH-Ubiquinone/plastoquin one (complex I), various chains | membrane associated. Involved in proton translocation across the membrane |
| efhand | 24% | EF hand | calcium-binding domain, consists of a12 residue loop flanked on both sides by a 12 residue alpha-helical domain |
| rvp | 79% | Retroviral aspartyl protease | Aspartyl or acid proteases, centered on a catalytic aspartyl residue |
| Collagen | 42% | Collagen triple helix repeat (20 copies) | extracellular structural proteins involved in formation of connective tissue. The sequence consists of the G-X-Y and the polypeptide chains forms a triple helix. |

(continued)

| Table XIX: Frequently Occurring Motifs | | | |
|---|---|---|---|
| **Name** | **avrg.% identity** | **Description** | **Potential Function** |
| fn3 | 20% | Fibronectin type III domain | Located in the extracellular ligand-binding region of receptors and is about 200 amino acid residues long with two pairs of cysteines involved in disulfide bonds |
| 7tm 1 | 19% | 7 transmembrane receptor (rhodopsin family) | seven hydrophobic transmembrane regions, with the N-terminus located extracellularly while theC-terminus is cytoplasmic. Signal through G proteins |

**Table XX: Post Translational Modification of 121P2A3 V.1**

N-glycosylation site
- 161 - 164 NSSI
- 434 - 437 NESL

Glycosaminoglycan attachment site
- 46-49 SGkG

cAMP- and cGMP-dependent protein kinase phosphorylation site
- 322 - 325 KKrS

Protein kinase C phosphorylation site
- 2-4 SsR
- 5 - 7 StK
- 46 - 48 SgK
- 52-54 TdK
- 78 - 80 TeK
- 107 -109 TtR
- 136 -138 TsR
- 148 -150 TlR
- 220-222 TkK
- 243 - 245 SaK
- 272-274 TqK
- 285-287 SqR
- 301- 303 TeK
- 396 - 398 SlK
- 425-427 SpK

Casein kinase II phosphorylation site
- 5 - 8 StkD
- 21 - 24 SksE
- 25 - 28 TtlE
- 39 - 42 TsvD
- 40 - 43 SvdE
- 52 - 55 TdkE
- 78 - 81 TekD

(continued)

107 - 110 TtrE
272 - 275 TqkE
392 - 395 TqlE
436 - 439 SlvE

Tyrosine kinase phosphorylation site
221 - 228 Kkp.EsegY

N-myristoylation site
15 - 20 GSkpSN

**TABLE XXI Features of 121P2A3 protein**

| 121P2A3 var.1 | Bioinformatic Program | URL | Outcome |
|---|---|---|---|
| ORF | ORF finder | | bp 175-1569 (includes stop codon) |
| Protein length | | | 464aa |
| Transmembrane region | TM Pred | URL www.ch.embnet.org/ | no TM |
| | HMMTop | URL www.enzimhu/hmmtop/ | no TM, intracellular |
| | Sosui | URL www.genome.ad.jp/SOSui/ | no TM, soluble protein |
| | TMHMM | URL www.cbs.dtu.dk/services/TMHMM | no TM |
| Signal Peptide | Signal P | URL www.cbs.dtu.dk/services/SignalP/ | no |
| pl | pI/MW tool | URL www.expasy.ch/tools/ | p16.55 |
| Molecular weight | pI/MW tool | URL www.expasy.ch/tools/ | 54.1kDa |
| Localization | PSORT | URL psort.nibb.ac.jp/ | 45% cytoplasm, 30% peroxisome |
| | PSORT II | URL psort.nibb.ac.jp/ | 56.% nuclear, 22% mitochondrial, 17% cytoplasm |
| Motifs | Pfam | URL www.sanger.ac.uk/Pfarn/ | none |
| | Prints | URL www.biochem.ucl.ac.uk/ | none |
| | Blocks | URL www.blocks.fhcrc.org/ | CTF/NF-1 family, chaperonin cpn60 (60kD subunit), clusterin |
| **121P2A3 var.2** | **Bioinformatic Program** | **URL** | **Outcome** |
| ORF | ORF finder | | bp 533-1420 (includes stop codon) |
| Protein length | | | 295aa |
| Transmembrane region | TM Pred | URL www.ch.embnet.org/ | no TM |
| | HMMTop | URL www.enzim.hu/hmmtopl | no TM, extracellular |
| | Sosui | URL www.genome.ad.jp/SOSui/ | no TM, soluble protein |
| | TMHMM | URL www.cbs.dtu.dk/services/TMHMM | no TM |
| Signal Peptide | Signal P | URL www.cbs.dtu.dk/services/SignalP/ | no |
| pl | pI/MW tool | URL www.expasy.ch/tools/ | pI5.8 |
| Molecular weight | pI/MW tool | URL www.expasy.ch/tools/ | 34.9kDa |
| Localization | PSORT | URL psort.nibb.ac.jp/ | 65% cytoplasm |

(continued)

| 121P2A3 var.1 | Bioinformatic Program | URL | Outcome |
|---|---|---|---|
| | PSORT II | URL psort.nibb.ac.jp/ | 56.5% nuclear, 22% cytoplasm |
| Motifs. | Pfam | URL www.sanger.ac.uk/Pfam/ | none |
| | Prints | URL www.biochem.ucl.ac.uk/ | none |
| | Blocks | URL www.blocks.fhcrc:org/ | clusterin, CTF/NF-1 family |

| TABLE XXII 121P2A3 v.1: HLA Peptide Scoring Results A1 9-mers SYFPEITHI | | | |
|---|---|---|---|
| Pos | 1 2 3 4 5 6 7 8 9 | score | SEQ. ID NO. |
| 186 | V Y D Q Q R E V Y | 30 | |
| 67 | E A E K E K N A Y | 25 | |
| 87 | L R D Q L K A R Y | 25 | |
| 229 | L Q E E K Q K C Y | 25 | |
| 449 | A T E H R D L L V | 25 | |
| 179 | E K N Q Q W L V Y | 24 | |
| 276 | V H N L N Q L L Y | 24 | |
| 122 | S E E K D V L K Q | 21 | |
| 405 | I T E P L V T F Q | 21 | |
| 328 | L L S Q V Q F L Y | 20 | |
| 439 | E C P K C N I Q Y | 20 | |
| 53 | D K E R H R L L E | 19 | |
| 81 | D K E I Q R L R D | 19 | |
| 220 | T K K P E S E G Y | 19 | |
| 261 | E L S E F R R K Y | 19 | |
| 31 | K G E I A H L K T | 18 | |
| 288 | R A D V Q H L E D | 18 | |
| 300 | K T E K I Q K L R | 18 | |
| 51 | L T D K E R H R L | 17 | |
| 273 | Q K E V H N L N Q | 17 | |
| 415 | E T E N R E K V A | 17 | |
| 453 | R D L L V H V E Y | 17 | |
| 22 | K S E T T L E K L | 16 | |
| 77 | L T E K D K E I Q | 16 | |
| 121 | L S E E K D V L K | 16 | |
| 208 | K T E T A A H S L | 16 | |
| 224 | E S E G Y L Q E E | 16 | |
| 249 | E V E R Q T I T Q | 16 | |
| 362 | D F E N E K L D R | 16 | |
| 262 | L S E F R R K Y E | 15 | |
| 269 | Y E E T Q K E V H | 15 | |
| 329 | L S Q V Q F L Y T | 15 | |
| 24 | E T T L E K L K G | 14 | |
| 59 | L L E K I R V L E | 14 | |
| 65 | V L E A E K E K N | 14 | |
| 293 | H L E D D R H K T | 14 | |
| 307 | L R E E N D I A R | 14 | |
| 324 | R S E E L L S Q V | 14 | |
| 360 | T L D F E N E K L | 14 | |
| 391 | I T Q L E S L K Q | 14 | |
| 41 | V D E I T S G K G | 13 | |
| 145 | K T N T L R L S Q | 13 | |
| 222 | K P E S E G Y L Q | 13 | |
| 310 | E N D I A R G K L | 13 | |
| 325 | S E E L L S Q V Q | 13 | |
| 342 | Q Q E E Q T R V A | 13 | |
| 351 | L L E Q Q M Q A C | 13 | |
| 367 | K L D R Q H V Q H | 13 | |
| 393 | Q L E S L K Q L H | 13 | |
| 6 | T K D L I K S K W | 12 | |
| 40 | S V D E I T S G K | 12 | |
| 45 | T S G K G K L T D | 12 | |
| 95 | Y S T T A L L E Q | 12 | |
| 108 | T R E G E R R E Q | 12 | |

| TABLE XXII 121P2A3 v.1: HLA Peptide Scoring Results A1 9-mers SYFPEITHI | | | |
|---|---|---|---|
| Pos | 1 2 3 4 5 6 7 8 9 | score | SEQ. ID NO. |
| 113 | R R E Q V L K A L | 12 | |
| 167 | I H E M E I Q L K | 12 | |
| 169 | E M E I Q L K D A | 12 | |
| 177 | A L E K N Q Q W L | 12 | |
| 190 | Q R E V Y V K G L | 12 | |
| 210 | E T A A H S L P Q | 12 | |
| 214 | H S L P Q Q T K K | 12 | |
| 230 | Q E E K Q K C Y N | 12 | |
| 237 | Y N D L L A S A K | 12 | |
| 247 | D L E V E R Q T I | 12 | |
| 259 | S F E L S E F R R | 12 | |
| 346 | Q T R V A L L E Q | 12 | |
| 418 | N R E K V A A S P | 12 | |
| 452 | H R D L L V H V E | 12 | |
| 15 | G S K P S N S K S | 11 | |
| 26 | T L E K L K G E I | 11 | |
| 100 | L L E Q L E E T T | 11 | |
| 103 | Q L E E T T R E G | 11 | |
| 104 | L E E T T R E G E | 11 | |
| 110 | E G E R R E Q V L | 11 | |
| 112 | E R R E Q V L K A | 11 | |
| 141 | E L E S K T N T L | 11 | |
| 204 | E L E K K T E T A | 11 | |
| 242 | A S A K K D L E V | 11 | |
| 245 | K K D L E V E R Q | 11 | |
| 255 | I T Q L S F E L S | 11 | |
| 317 | K L E E E K K R S | 11 | |
| 319 | E E E K K R S E E | 11 | |
| 343 | Q E E Q T R V A L | 11 | |
| 413 | Q G E T E N R E K | 11 | |
| 433 | L N E S L V E C P | 11 | |
| 437 | L V E C P K C N I | 11 | |
| 4 | R S T K D L I K S | 10 | |
| 38 | K T S V D E I T S | 10 | |
| 44 | I T S G K G K L T | 10 | |
| 69 | E K E K N A Y Q L | 10 | |
| 79 | E K D K E I Q R L | 10 | |
| 124 | E K D V L K Q Q L | 10 | |
| 136 | T S R I A E L E S | 10 | |
| 139 | I A E L E S K T N | 10 | |
| 143 | E S K T N T L R L | 10 | |
| 174 | L K D A L E K N Q | 10 | |
| 202 | I F E L E K K T E | 10 | |
| 268 | K Y E E T Q K E V | 10 | |
| 294 | L E D D R H K T E | 10 | |
| 295 | E D D R H K T E K | 10 | |
| 308 | R E E N D I A R G | 10 | |
| 318 | L E E E K K R S E | 10 | |
| 334 | F L Y T S L L K Q | 10 | |
| 345 | E Q T R V A L L E | 10 | |
| 364 | E N E K L D R Q H | 10 | |
| 375 | H Q L H V I L K E | 10 | |
| 381 | L K E L R K A R N | 10 | |
| 400 | L H E F A I T E P | 10 | |
| 427 | K S P T A A L N E | 10 | |
| 135 | A T S R I A E L E | 9 | |

TABLE XXII 121P2A3 v.1: HLA Peptide Scoring Results A1 9-mers SYFPEITHI

| Pos | 1 2 3 4 5 6 7 8 9 | score | SEQ. ID NO. |
|---|---|---|---|
| 161 | N S S I N N I H E | 9 | |
| 192 | E V Y V K G L L A | 9 | |
| 193 | V Y V K G L L A K | 9 | |
| 410 | V T F Q G E T E N | 9 | |
| 2 | S S R S T K D L I | 8 | |
| 3 | S R S T K D L I K | 8 | |
| 5 | S T K D L I K S K | 8 | |
| 21 | S K S E T T L E K | 8 | |
| 85 | Q R L R D Q L K A | 8 | |
| 94 | R Y S T T A L L E | 8 | |
| 96 | S T T A L L E Q L | 8 | |
| 97 | T T A L L E Q L E | 8 | |
| 107 | T T R E G E R R E | 8 | |
| 126 | D V L K Q Q L S A | 8 | |
| 153 | Q T V A P N C F N | 8 | |
| 168 | H E M E I Q L K D | 8 | |
| 223 | P E S E G Y L Q E | 8 | |
| 234 | Q K C Y N D L L A | 8 | |
| 253 | Q T I T Q L S F E | 8 | |
| 277 | H N L N Q L L Y S | 8 | |
| 301 | T E K I Q K L R E | 8 | |
| 312 | D I A R G K L E E | 8 | |
| 322 | K K R S E E L L S | 8 | |
| 333 | Q F L Y T S L L K | 8 | |
| 336 | Y T S L L K Q Q E | 8 | |
| 403 | F A I T E P L V T | 8 | |
| 431 | A A L N E S L V E | 8 | |
| 60 | L E K I R V L E A | 7 | |
| 71 | E K N A Y Q L T E | 7 | |
| 106 | E T T R E G E R R | 7 | |
| 133 | S A A T S R I A E | 7 | |
| 215 | S L P Q Q T K K P | 7 | |
| 219 | Q T K K P E S E G | 7 | |
| 271 | E T Q K E V H N L | 7 | |
| 284 | Y S Q R R A D V Q | 7 | |
| 374 | Q H Q L H V I L K | 7 | |
| 429 | P T A A L N E S L | 7 | |
| 450 | T E H R D L L V H | 7 | |
| 11 | K S K W G S K P S | 6 | |
| 20 | N S K S E T T L E | 6 | |
| 25 | T T L E K L K G E | 6 | |
| 28 | E K L K G E I A H | 6 | |
| 54 | K E R H R L L E K | 6 | |
| 98 | T A L L E Q L E E | 6 | |
| 115 | E Q V L K A L S E | 6 | |
| 147 | N T L R L S Q T V | 6 | |
| 151 | L S Q T V A P N C | 6 | |
| 162 | S S I N N I H E M | 6 | |
| 172 | I Q L K D A L E K | 6 | |
| 198 | L L A K I F E L E | 6 | |
| 199 | L A K I F E L E K | 6 | |
| 235 | K C Y N D L L A S | 6 | |
| 252 | R Q T I T Q L S F | 6 | |
| 256 | T Q L S F E L S E | 6 | |
| 323 | K R S E E L L S Q | 6 | |
| 355 | Q M Q A C T L D F | 6 | |

TABLE XXII 121P2A3 v.1: HLA Peptide Scoring Results A1 9-mers SYFPEITHI

| Pos | 1 2 3 4 5 6 7 8 9 | score | SEQ. ID NO. |
|---|---|---|---|
| 359 | C T L D F E N E K | 6 | |
| 371 | Q H V Q H Q L H V | 6 | |
| 378 | H V I L K E L R K | 6 | |
| 388 | R N Q I T Q L E S | 6 | |
| 394 | L E S L K Q L H E | 6 | |
| 395 | E S L K Q L H E F | 6 | |
| 426 | P K S P T A A L N | 6 | |
| 435 | E S L V E C P K C | 6 | |
| 194 | Y V K G L L A K I | 5 | |
| 228 | Y L Q E E K Q K C | 5 | |
| 338 | S L L K Q Q E E Q | 5 | |
| 399 | Q L H E F A I T E | 5 | |
| 423 | A A S P K S P T A | 5 | |
| 424 | A S P K S P T A A | 5 | |
| 438 | V E C P K C N I Q | 5 | |
| 1 | M S S R S T K D L | 4 | |
| 18 | P S N S K S E T T | 4 | |
| 23 | S E T T L E K L K | 4 | |
| 39 | T S V D E I T S G | 4 | |
| 43 | E I T S G K G K L | 4 | |
| 57 | H R L L E K I R V | 4 | |
| 75 | Y Q L T E K D K E | 4 | |
| 78 | T E K D K E I Q R | 4 | |
| 99 | A L L E Q L E E T | 4 | |
| 127 | V L K Q Q L S A A | 4 | |
| 132 | L S A A T S R I A | 4 | |
| 144 | S K T N T L R L S | 4 | |
| 154 | T V A P N C F N S | 4 | |
| 155 | V A P N C F N S S | 4 | |
| 158 | N C F N S S I N N | 4 | |
| 166 | N I H E M E I Q L | 4 | |
| 178 | L E K N Q Q W L V | 4 | |
| 189 | Q Q R E V Y V K G | 4 | |
| 191 | R E V Y V K G L L | 4 | |
| 196 | K G L L A K I F E | 4 | |
| 226 | E G Y L Q E E K Q | 4 | |
| 233 | K Q K C Y N D L L | 4 | |
| 258 | L S F E L S E F R | 4 | |
| 260 | F E L S E F R R K | 4 | |
| 272 | T Q K E V H N L N | 4 | |
| 282 | L L Y S Q R R A D | 4 | |
| 287 | R R A D V Q H L E | 4 | |
| 298 | R H K T E K I Q K | 4 | |
| 327 | E L L S Q V Q F L | 4 | |
| 332 | V Q F L Y T S L L | 4 | |
| 337 | T S L L K Q Q E E | 4 | |
| 350 | A L L E Q Q M Q A | 4 | |
| 358 | A C T L D F E N E | 4 | |
| 370 | R Q H V Q H Q L H | 4 | |
| 385 | R K A R N Q I T Q | 4 | |
| 445 | I Q Y P A T E H R | 4 | |
| 10 | I K S K W G S K P | 3 | |
| 12 | S K W G S K P S N | 3 | |
| 46 | S G K G K L T D K | 3 | |
| 86 | R L R D Q L K A R | 3 | |
| 93 | A R Y S T T A L L | 3 | |

| TABLE XXII 121P2A3 v.1: HLA Peptide Scoring Results A1 9-mers SYFPEITHI | | | |
|---|---|---|---|
| Pos | 1 2 3 4 5 6 7 8 9 | score | SEQ. ID NO. |
| 120 | A L S E E K D V L | 3 | |
| 142 | L E S K T N T L R | 3 | |
| 163 | S I N N I H E M E | 3 | |
| 173 | Q L K D A L E K N | 3 | |
| 200 | A K I F E L E K K | 3 | |
| 239 | D L L A S A K K D | 3 | |
| 243 | S A K K D L E V E | 3 | |
| 275 | E V H N L N Q L L | 3 | |
| 311 | N D I A R G K L E | 3 | |
| 339 | L L K Q Q E E Q T | 3 | |
| 344 | E E Q T R V A L L | 3 | |
| 372 | H V Q H Q L H V I | 3 | |
| 379 | V I L K E L R K A | 3 | |
| 387 | A R N Q I T Q L E | 3 | |
| 396 | S L K Q L H E F A | 3 | |
| 398 | K Q L H E F A I T | 3 | |
| 407 | E P L V T F Q G E | 3 | |
| 409 | L V T F Q G E T E | 3 | |
| 414 | G E T E N R E K V | 3 | |
| 425 | S P K S P T A A L | 3 | |
| 436 | S L V E C P K C N | 3 | |
| 448 | P A T E H R D L L | 3 | |
| 455 | L L V H V E Y C S | 3 | |
| 16 | S K P S N S K S E | 2 | |
| 19 | S N S K S E T T L | 2 | |
| 29 | K L K G E I A H L | 2 | |
| 30 | L K G E I A H L K | 2 | |
| 36 | H L K T S V D E I | 2 | |
| 37 | L K T S V D E I T | 2 | |
| 47 | G K G K L T D K E | 2 | |
| 52 | T D K E R H R L L | 2 | |
| 55 | E R H R L L E K I | 2 | |
| 58 | R L L E K I R V L | 2 | |
| 61 | E K I R V L E A E | 2 | |
| 63 | I R V L E A E K E | 2 | |
| 68 | A E K E K N A Y Q | 2 | |
| 70 | K E K N A Y Q L T | 2 | |
| 72 | K N A Y Q L T E K | 2 | |
| 73 | N A Y Q L T E K D | 2 | |
| 80 | K D K E I Q R L R | 2 | |
| 84 | I Q R L R D Q L K | 2 | |
| 111 | G E R R E Q V L K | 2 | |
| 114 | R E Q V L K A L S | 2 | |
| 117 | V L K A L S E E K | 2 | |
| 118 | L K A L S E E K D | 2 | |
| 123 | E E K D V L K Q Q | 2 | |
| 125 | K D V L K Q Q L S | 2 | |
| 137 | S R I A E L E S K | 2 | |
| 138 | R I A E L E S K T | 2 | |
| 148 | T L R L S Q T V A | 2 | |
| 149 | L R L S Q T V A P | 2 | |
| 150 | R L S Q T V A P N | 2 | |
| 152 | S Q T V A P N C F | 2 | |
| 156 | A P N C F N S S I | 2 | |
| 160 | F N S S I N N I H | 2 | |
| 171 | E I Q L K D A L E | 2 | |
| 182 | Q Q W L V Y D Q Q | 2 | |
| 185 | L V Y D Q Q R E V | 2 | |
| 187 | Y D Q Q R E V Y V | 2 | |
| 188 | D Q Q R E V Y V K | 2 | |
| 201 | K I F E L E K K T | 2 | |
| 209 | T E T A A H S L P | 2 | |
| 213 | A H S L P Q Q T K | 2 | |
| 225 | S E G Y L Q E E K | 2 | |
| 238 | N D L L A S A K K | 2 | |
| 241 | L A S A K K D L E | 2 | |
| 246 | K D L E V E R Q T | 2 | |
| 251 | E R Q T I T Q L S | 2 | |
| 263 | S E F R R K Y E E | 2 | |
| 265 | F R R K Y E E T Q | 2 | |
| 267 | R K Y E E T Q K E | 2 | |
| 285 | S Q R R A D V Q H | 2 | |
| 296 | D D R H K T E K I | 2 | |
| 299 | H K T E K I Q K L | 2 | |
| 306 | K L R E E N D I A | 2 | |
| 315 | R G K L E E E K K | 2 | |
| 316 | G K L E E E K K R | 2 | |
| 321 | E K K R S E E L L | 2 | |
| 330 | S Q V Q F L Y T S | 2 | |
| 349 | V A L L E Q Q M Q | 2 | |
| 353 | E Q Q M Q A C T L | 2 | |
| 354 | Q Q M Q A C T L D | 2 | |
| 361 | L D F E N E K L D | 2 | |
| 369 | D R Q H V Q H Q L | 2 | |
| 377 | L H V I L K E L R | 2 | |
| 380 | I L K E L R K A R | 2 | |
| 383 | E L R K A R N Q I | 2 | |
| 386 | K A R N Q I T Q L | 2 | |
| 389 | N Q I T Q L E S L | 2 | |
| 390 | Q I T Q L E S L K | 2 | |
| 402 | E F A I T E P L V | 2 | |
| 404 | A I T E P L V T F | 2 | |
| 406 | T E P L V T F Q G | 2 | |
| 420 | E K V A A S P K S | 2 | |
| 422 | V A A S P K S P T | 2 | |
| 428 | S P T A A L N E S | 2 | |
| 430 | T A A L N E S L V | 2 | |
| 432 | A L N E S L V E C | 2 | |
| 434 | N E S L V E C P K | 2 | |
| 442 | K C N I Q Y P A T | 2 | |
| 447 | Y P A T E H R D L | 2 | |
| 454 | D L L V H V E Y C | 2 | |
| 8 | D L I K S K W G S | 1 | |
| 14 | W G S K P S N S K | 1 | |
| 27 | L E K L K G E I A | 1 | |
| 34 | I A H L K T S V D | 1 | |
| 35 | A H L K T S V D E | 1 | |
| 42 | D E I T S G K G K | 1 | |
| 50 | K L T D K E R H R | 1 | |
| 74 | A Y Q L T E K D K | 1 | |
| 76 | Q L T E K D K E I | 1 | |
| 83 | E I Q R L R D Q L | 1 | |

TABLE XXII 121P2A3 v.1: HLA Peptide Scoring Results A1 9-mers SYFPEITHI

| Pos | 1 2 3 4 5 6 7 8 9 | score | SEQ. ID NO. |
|---|---|---|---|
| 89 | DQLKARYST | 1 | |
| 90 | QLKARYSTT | 1 | |
| 91 | LKARYSTTA | 1 | |
| 109 | REGERREQV | 1 | |
| 131 | QLSAATSRI | 1 | |
| 134 | AATSRIAEL | 1 | |
| 140 | AELESKTNT | 1 | |
| 176 | DALEKNQQW | 1 | |
| 181 | NQQWLVYDQ | 1 | |
| 183 | QWLVYDQQR | 1 | |
| 184 | WLVYDQQRE | 1 | |
| 195 | VKGLLAKIF | 1 | |
| 197 | GLLAKIFEL | 1 | |
| 203 | FELEKKTET | 1 | |
| 212 | AAHSLPQQT | 1 | |
| 240 | LLASAKKDL | 1 | |
| 244 | AKKDLEVER | 1 | |
| 250 | VERQTITQL | 1 | |
| 257 | QLSFELSEF | 1 | |
| 278 | NLNQLLYSQ | 1 | |
| 280 | NQLLYSQRR | 1 | |
| 281 | QLLYSQRRA | 1 | |
| 283 | LYSQRRADV | 1 | |
| 286 | QRRADVQHL | 1 | |
| 289 | ADVQHLEDD | 1 | |
| 291 | VQHLEDDRH | 1 | |
| 303 | KIQKLREEN | 1 | |
| 313 | IARGKLEEE | 1 | |
| 314 | ARGKLEEEK | 1 | |
| 363 | FENEKLDRQ | 1 | |
| 365 | NEKLDRQHV | 1 | |
| 366 | EKLDRQHVQ | 1 | |
| 368 | LDRQHVQHQ | 1 | |
| 373 | VQHQLHVIL | 1 | |
| 376 | QLHVILKEL | 1 | |
| 384 | LRKARNQIT | 1 | |
| 408 | PLVTFQGET | 1 | |
| 411 | TFQGETENR | 1 | |
| 412 | FQGETENRE | 1 | |
| 417 | ENREKVAAS | 1 | |
| 443 | CNIQYPATE | 1 | |
| 444 | NIQYPATEH | 1 | |

TABLE XXII 121P2A3 v.3: HLA Peptide Scoring Results A1 9-mers SYFPEITHI

| Pos | 1 2 3 4 5 6 7 8 9 | score | SEQ. ID NO. |
|---|---|---|---|
| 5 | DKERQRLLE | 21 | |
| 3 | LTDKERQRL | 17 | |
| 6 | KERQRLLEK | 6 | |
| 9 | QRLLEKIRV | 4 | |
| 4 | TDKERQRLL | 2 | |
| 7 | ERQRLLEKI | 2 | |
| 2 | KLTDKERQR | 1 | |

TABLE XXII 121P2A3 v.4: HLA Peptide Scoring Results A1 9-mers SYFPEITHI

| Pos | 1 2 3 4 5 6 7 8 9 | score | SEQ. ID NO. |
|---|---|---|---|
| 5 | YSTTTLLEQ | 12 | |
| 8 | TTLLEQLEE | 12 | |
| 4 | RYSTTTLLE | 8 | |
| 6 | STTTLLEQL | 8 | |
| 7 | TTTLLEQLE | 8 | |
| 3 | ARYSTTTLL | 3 | |
| 9 | TLLEQLEET | 3 | |
| 1 | LKARYSTTT | 1 | |

TABLE XXII 121P2A3 v.6: HLA Peptide Scoring Results A1 9-mers SYFPEITHI

| Pos | 1 2 3 4 5 6 7 8 9 | score | SEQ. ID NO. |
|---|---|---|---|
| 3 | LLSQVQSLY | 20 | |
| 4 | LSQVQSLYT | 12 | |
| 8 | QSLYTSLLK | 12 | |
| 9 | SLYTSLLKQ | 11 | |
| 2 | ELLSQVQSL | 4 | |
| 7 | VQSLYTSLL | 4 | |
| 5 | SQVQSLYTS | 2 | |

TABLE XXII 121P2A3 v.7: HLA Peptide Scoring Results A1 9-mers SYFPEITHI

| Pos | 1 2 3 4 5 6 7 8 9 | score | SEQ. ID NO. |
|---|---|---|---|
| 6 | HQLLVILKE | 10 | |
| 3 | HVQHQLLVI | 9 | |
| 2 | QHVQHQLLV | 8 | |
| 5 | QHQLLVILK | 7 | |
| 9 | LVILKELRK | 6 | |
| 1 | RQHVQHQLL | 4 | |
| 8 | LLVILKELR | 3 | |
| 4 | VQHQLLVIL | 1 | |
| 7 | QLLVILKEL | 1 | |

TABLE XXII 121P2A3 v.8: HLA Peptide Scoring Results A1 9-mers SYFPEITHI

| Pos | 1 2 3 4 5 6 7 8 9 | score | SEQ. ID NO. |
|---|---|---|---|
| 1 | KSPTAALNG | 10 | |
| 5 | AALNGSLVE | 10 | |
| 3 | PTAALNGSL | 7 | |
| 9 | GSLVECPKC | 6 | |
| 4 | TAALNGSLV | 3 | |
| 6 | ALNGSLVEC | 3 | |
| 2 | SPTAALNGS | 2 | |
| 8 | NGSLVECPK | 2 | |
| 7 | LNGSLVECP | 1 | |

TABLE XXIII 121P2A3 v.1: HLA Peptide Scoring Results A*0201 9-mers SYFPEITHI

| Pos | 1 2 3 4 5 6 7 8 9 | score | SEQ. ID NO. |
|---|---|---|---|
| 197 | GLLAKIFEL | 30 | |
| 58 | RLLEKIRVL | 29 | |

**TABLE XXIII 121P2A3 v.1: HLA Peptide Scoring Results A*0201 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 29 | K | L | K | G | E | I | A | H | L | 28 | |
| 99 | A | L | L | E | Q | L | E | E | T | 26 | |
| 376 | Q | L | H | V | I | L | K | E | L | 25 | |
| 120 | A | L | S | E | E | K | D | V | L | 24 | |
| 194 | Y | V | K | G | L | L | A | K | I | 24 | |
| 36 | H | L | K | T | S | V | D | E | I | 23 | |
| 134 | A | A | T | S | R | I | A | E | L | 23 | |
| 240 | L | L | A | S | A | K | K | D | L | 23 | |
| 327 | E | L | L | S | Q | V | Q | F | L | 23 | |
| 432 | A | L | N | E | S | L | V | E | C | 23 | |
| 141 | E | L | E | S | K | T | N | T | L | 22 | |
| 76 | Q | L | T | E | K | D | K | E | I | 21 | |
| 177 | A | L | E | K | N | Q | Q | W | L | 21 | |
| 360 | T | L | D | F | E | N | E | K | L | 21 | |
| 379 | V | I | L | K | E | L | R | K | A | 21 | |
| 26 | T | L | E | K | L | K | G | E | I | 20 | |
| 33 | E | I | A | H | L | K | T | S | V | 20 | |
| 96 | S | T | T | A | L | L | E | Q | L | 20 | |
| 131 | Q | L | S | A | A | T | S | R | I | 20 | |
| 166 | N | I | H | E | M | E | I | Q | L | 20 | |
| 185 | L | V | Y | D | Q | Q | R | E | V | 20 | |
| 254 | T | I | T | Q | L | S | F | E | L | 20 | |
| 350 | A | L | L | E | Q | Q | M | Q | A | 20 | |
| 404 | A | I | T | E | P | L | V | T | F | 20 | |
| 127 | V | L | K | Q | Q | L | S | A | A | 19 | |
| 138 | R | I | A | E | L | E | S | K | T | 19 | |
| 147 | N | T | L | R | L | S | Q | T | V | 19 | |
| 278 | N | L | N | Q | L | L | Y | S | Q | 19 | |
| 334 | F | L | Y | T | S | L | L | K | Q | 19 | |
| 386 | K | A | R | N | Q | I | T | Q | L | 19 | |
| 43 | E | I | T | S | G | K | G | K | L | 18 | |
| 100 | L | L | E | Q | L | E | E | T | T | 18 | |
| 201 | K | I | F | E | L | E | K | K | T | 18 | |
| 242 | A | S | A | K | K | D | L | E | V | 18 | |
| 247 | D | L | E | V | E | R | Q | T | I | 18 | |
| 299 | H | K | T | E | K | I | Q | K | L | 18 | |
| 389 | N | Q | I | T | Q | L | E | S | L | 18 | |
| 51 | L | T | D | K | E | R | H | R | L | 17 | |
| 92 | K | A | R | Y | S | T | T | A | L | 17 | |
| 93 | A | R | Y | S | T | T | A | L | L | 17 | |
| 208 | K | T | E | T | A | A | H | S | L | 17 | |
| 293 | H | L | E | D | D | R | H | K | T | 17 | |
| 306 | K | L | R | E | E | N | D | I | A | 17 | |
| 372 | H | V | Q | H | Q | L | H | V | I | 17 | |
| 392 | T | Q | L | E | S | L | K | Q | L | 17 | |
| 425 | S | P | K | S | P | T | A | A | L | 17 | |
| 22 | K | S | E | T | T | L | E | K | L | 16 | |
| 83 | E | I | Q | R | L | R | D | Q | L | 16 | |
| 119 | K | A | L | S | E | E | K | D | V | 16 | |
| 150 | R | L | S | Q | T | V | A | P | N | 16 | |
| 173 | Q | L | K | D | A | L | E | K | N | 16 | |
| 228 | Y | L | Q | E | E | K | Q | K | C | 16 | |
| 271 | E | T | Q | K | E | V | H | N | L | 16 | |
| 274 | K | E | V | H | N | L | N | Q | L | 16 | |
| 324 | R | S | E | E | L | L | S | Q | V | 16 | |
| 331 | Q | V | Q | F | L | Y | T | S | L | 16 | |

**TABLE XXIII 121P2A3 v.1: HLA Peptide Scoring Results A*0201 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 338 | S | L | L | K | Q | Q | E | E | Q | 16 | |
| 383 | E | L | R | K | A | R | N | Q | I | 16 | |
| 396 | S | L | K | Q | L | H | E | F | A | 16 | |
| 423 | A | A | S | P | K | S | P | T | A | 16 | |
| 429 | P | T | A | A | L | N | E | S | L | 16 | |
| 430 | T | A | A | L | N | E | S | L | V | 16 | |
| 449 | A | T | E | H | R | D | L | L | V | 16 | |
| 86 | R | L | R | D | Q | L | K | A | R | 15 | |
| 90 | Q | L | K | A | R | Y | S | T | T | 15 | |
| 103 | Q | L | E | E | T | T | R | E | G | 15 | |
| 162 | S | S | I | N | N | I | H | E | M | 15 | |
| 204 | E | L | E | K | K | T | E | T | A | 15 | |
| 215 | S | L | P | Q | Q | T | K | K | P | 15 | |
| 236 | C | Y | N | D | L | L | A | S | A | 15 | |
| 250 | V | E | R | Q | T | I | T | Q | L | 15 | |
| 257 | Q | L | S | F | E | L | S | E | F | 15 | |
| 281 | Q | L | L | Y | S | Q | R | R | A | 15 | |
| 313 | I | A | R | G | K | L | E | E | E | 15 | |
| 332 | V | Q | F | L | Y | T | S | L | L | 15 | |
| 339 | L | L | K | Q | Q | E | E | Q | T | 15 | |
| 399 | Q | L | H | E | F | A | I | T | E | 15 | |
| 451 | E | H | R | D | L | L | V | H | V | 15 | |
| 454 | D | L | L | V | H | V | E | Y | C | 15 | |
| 19 | S | N | S | K | S | E | T | T | L | 14 | |
| 62 | K | I | R | V | L | E | A | E | K | 14 | |
| 148 | T | L | R | L | S | Q | T | V | A | 14 | |
| 156 | A | P | N | C | F | N | S | S | I | 14 | |
| 159 | C | F | N | S | S | I | N | N | I | 14 | |
| 170 | M | E | I | Q | L | K | D | A | L | 14 | |
| 187 | Y | D | Q | Q | R | E | V | Y | V | 14 | |
| 190 | Q | R | E | V | Y | V | K | G | L | 14 | |
| 198 | L | L | A | K | I | F | E | L | E | 14 | |
| 282 | L | L | Y | S | Q | R | R | A | D | 14 | |
| 283 | L | Y | S | Q | R | R | A | D | V | 14 | |
| 286 | Q | R | R | A | D | V | Q | H | L | 14 | |
| 312 | D | I | A | R | G | K | L | E | E | 14 | |
| 373 | V | Q | H | Q | L | H | V | I | L | 14 | |
| 380 | I | L | K | E | L | R | K | A | R | 14 | |
| 408 | P | L | V | T | F | Q | G | E | T | 14 | |
| 414 | G | E | T | E | N | R | E | K | V | 14 | |
| 437 | L | V | E | C | P | K | C | N | I | 14 | |
| 447 | Y | P | A | T | E | H | R | D | L | 14 | |
| 25 | T | T | L | E | K | L | K | G | E | 13 | |
| 59 | L | L | E | K | I | R | V | L | E | 13 | |
| 113 | R | R | E | Q | V | L | K | A | L | 13 | |
| 117 | V | L | K | A | L | S | E | E | K | 13 | |
| 164 | I | N | N | I | H | E | M | E | I | 13 | |
| 221 | K | K | P | E | S | E | G | Y | L | 13 | |
| 268 | K | Y | E | E | T | Q | K | E | V | 13 | |
| 341 | K | Q | Q | E | E | Q | T | R | V | 13 | |
| 344 | E | E | Q | T | R | V | A | L | L | 13 | |
| 367 | K | L | D | R | Q | H | V | Q | H | 13 | |
| 401 | H | E | F | A | I | T | E | P | L | 13 | |
| 436 | S | L | V | E | C | P | K | C | N | 13 | |
| 455 | L | L | V | H | V | E | Y | C | S | 13 | |
| 5 | S | T | K | D | L | I | K | S | K | 12 | |

| TABLE XXIII 121P2A3 v.1: HLA Peptide Scoring Results A*0201 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 8 | D | L | I | K | S | K | W | G | S | 12 | |
| 9 | L | I | K | S | K | W | G | S | K | 12 | |
| 44 | I | T | S | G | K | G | K | L | T | 12 | |
| 50 | K | L | T | D | K | E | R | H | R | 12 | |
| 55 | E | R | H | R | L | L | E | K | I | 12 | |
| 57 | H | R | L | L | E | K | I | R | V | 12 | |
| 60 | L | E | K | I | R | V | L | E | A | 12 | |
| 65 | V | L | E | A | E | K | E | K | N | 12 | |
| 109 | R | E | G | E | R | R | E | Q | V | 12 | |
| 116 | Q | V | L | K | A | L | S | E | E | 12 | |
| 126 | D | V | L | K | Q | Q | L | S | A | 12 | |
| 203 | F | E | L | E | K | K | T | E | T | 12 | |
| 239 | D | L | L | A | S | A | K | K | D | 12 | |
| 261 | E | L | S | E | F | R | R | K | Y | 12 | |
| 275 | E | V | H | N | L | N | Q | L | L | 12 | |
| 296 | D | D | R | H | K | T | E | K | I | 12 | |
| 303 | K | I | Q | K | L | R | E | E | N | 12 | |
| 328 | L | L | S | Q | V | Q | F | L | Y | 12 | |
| 343 | Q | E | E | Q | T | R | V | A | L | 12 | |
| 351 | L | L | E | Q | Q | M | Q | A | C | 12 | |
| 403 | F | A | I | T | E | P | L | V | T | 12 | |
| 405 | I | T | E | P | L | V | T | F | Q | 12 | |
| 422 | V | A | A | S | P | K | S | P | T | 12 | |
| 448 | P | A | T | E | H | R | D | L | L | 12 | |
| 1 | M | S | S | R | S | T | K | D | L | 11 | |
| 40 | S | V | D | E | I | T | S | G | K | 11 | |
| 52 | T | D | K | E | R | H | R | L | L | 11 | |
| 79 | E | K | D | K | E | I | Q | R | L | 11 | |
| 91 | L | K | A | R | Y | S | T | T | A | 11 | |
| 163 | S | I | N | N | I | H | E | M | E | 11 | |
| 169 | E | M | E | I | Q | L | K | D | A | 11 | |
| 178 | L | E | K | N | Q | Q | W | L | V | 11 | |
| 211 | T | A | A | H | S | L | P | Q | Q | 11 | |
| 212 | A | A | H | S | L | P | Q | Q | T | 11 | |
| 233 | K | Q | K | C | Y | N | D | L | L | 11 | |
| 305 | Q | K | L | R | E | E | N | D | I | 11 | |
| 317 | K | L | E | E | E | K | K | R | S | 11 | |
| 346 | Q | T | R | V | A | L | L | E | Q | 11 | |
| 348 | R | V | A | L | L | E | Q | Q | M | 11 | |
| 355 | Q | M | Q | A | C | T | L | D | F | 11 | |
| 359 | C | T | L | D | F | E | N | E | K | 11 | |
| 371 | Q | H | V | Q | H | Q | L | H | V | 11 | |
| 397 | L | K | Q | L | H | E | F | A | I | 11 | |
| 2 | S | S | R | S | T | K | D | L | I | 10 | |
| 66 | L | E | A | E | K | E | K | N | A | 10 | |
| 73 | N | A | Y | Q | L | T | E | K | D | 10 | |
| 112 | E | R | R | E | Q | V | L | K | A | 10 | |
| 128 | L | K | Q | Q | L | S | A | A | T | 10 | |
| 137 | S | R | I | A | E | L | E | S | K | 10 | |
| 140 | A | E | L | E | S | K | T | N | T | 10 | |
| 145 | K | T | N | T | L | R | L | S | Q | 10 | |
| 253 | Q | T | I | T | Q | L | S | F | E | 10 | |
| 323 | K | R | S | E | E | L | L | S | Q | 10 | |
| 330 | S | Q | V | Q | F | L | Y | T | S | 10 | |
| 369 | D | R | Q | H | V | Q | H | Q | L | 10 | |
| 393 | Q | L | E | S | L | K | Q | L | H | 10 | |
| 402 | E | F | A | I | T | E | P | L | V | 10 | |
| 410 | V | T | F | Q | G | E | T | E | N | 10 | |
| 444 | N | I | Q | Y | P | A | T | E | H | 10 | |
| 21 | S | K | S | E | T | T | L | E | K | 9 | |
| 34 | I | A | H | L | K | T | S | V | D | 9 | |
| 64 | R | V | L | E | A | E | K | E | K | 9 | |
| 85 | Q | R | L | R | D | Q | L | K | A | 9 | |
| 98 | T | A | L | L | E | Q | L | E | E | 9 | |
| 133 | S | A | A | T | S | R | I | A | E | 9 | |
| 143 | E | S | K | T | N | T | L | R | L | 9 | |
| 149 | L | R | L | S | Q | T | V | A | P | 9 | |
| 184 | W | L | V | Y | D | Q | Q | R | E | 9 | |
| 191 | R | E | V | Y | V | K | G | L | L | 9 | |
| 193 | V | Y | V | K | G | L | L | A | K | 9 | |
| 199 | L | A | K | I | F | E | L | E | K | 9 | |
| 200 | A | K | I | F | E | L | E | K | K | 9 | |
| 243 | S | A | K | K | D | L | E | V | E | 9 | |
| 246 | K | D | L | E | V | E | R | Q | T | 9 | |
| 310 | E | N | D | I | A | R | G | K | L | 9 | |
| 353 | E | Q | Q | M | Q | A | C | T | L | 9 | |
| 363 | F | E | N | E | K | L | D | R | Q | 9 | |
| 365 | N | E | K | L | D | R | Q | H | V | 9 | |
| 375 | H | Q | L | H | V | I | L | K | E | 9 | |
| 391 | I | T | Q | L | E | S | L | K | Q | 9 | |
| 398 | K | Q | L | H | E | F | A | I | T | 9 | |
| 431 | A | A | L | N | E | S | L | V | E | 9 | |
| 12 | S | K | W | G | S | K | P | S | N | 8 | |
| 32 | G | E | I | A | H | L | K | T | S | 8 | |
| 46 | S | G | K | G | K | L | T | D | K | 8 | |
| 54 | K | E | R | H | R | L | L | E | K | 8 | |
| 72 | K | N | A | Y | Q | L | T | E | K | 8 | |
| 89 | D | Q | L | K | A | R | Y | S | T | 8 | |
| 95 | Y | S | T | T | A | L | L | E | Q | 8 | |
| 107 | T | T | R | E | G | E | R | R | E | 8 | |
| 122 | S | E | E | K | D | V | L | K | Q | 8 | |
| 124 | E | K | D | V | L | K | Q | Q | L | 8 | |
| 132 | L | S | A | A | T | S | R | I | A | 8 | |
| 154 | T | V | A | P | N | C | F | N | S | 8 | |
| 155 | V | A | P | N | C | F | N | S | S | 8 | |
| 171 | E | I | Q | L | K | D | A | L | E | 8 | |
| 176 | D | A | L | E | K | N | Q | Q | W | 8 | |
| 192 | E | V | Y | V | K | G | L | L | A | 8 | |
| 232 | E | K | Q | K | C | Y | N | D | L | 8 | |
| 235 | K | C | Y | N | D | L | L | A | S | 8 | |
| 248 | L | E | V | E | R | Q | T | I | T | 8 | |
| 320 | E | E | K | K | R | S | E | E | L | 8 | |
| 352 | L | E | Q | Q | M | Q | A | C | T | 8 | |
| 378 | H | V | I | L | K | E | L | R | K | 8 | |
| 390 | Q | I | T | Q | L | E | S | L | K | 8 | |
| 416 | T | E | N | R | E | K | V | A | A | 8 | |
| 421 | K | V | A | A | S | P | K | S | P | 8 | |
| 428 | S | P | T | A | A | L | N | E | S | 8 | |
| 442 | K | C | N | I | Q | Y | P | A | T | 8 | |
| 456 | L | V | H | V | E | Y | C | S | K | 8 | |
| 17 | K | P | S | N | S | K | S | E | T | 7 | |
| 31 | K | G | E | I | A | H | L | K | T | 7 | |

| TABLE XXIII 121P2A3 v.1: HLA Peptide Scoring Results A*0201 9-mers SYFPEITHI | | | |
|---|---|---|---|
| Pos | 1 2 3 4 5 6 7 8 9 | score | SEQ. ID NO. |
| 38 | K T S V D E I T S | 7 | |
| 39 | T S V D E I T S G | 7 | |
| 69 | E K E K N A Y Q L | 7 | |
| 82 | K E I Q R L R D Q | 7 | |
| 97 | T T A L L E Q L E | 7 | |
| 146 | T N T L R L S Q T | 7 | |
| 172 | I Q L K D A L E K | 7 | |
| 180 | K N Q Q W L V Y D | 7 | |
| 241 | L A S A K K D L E | 7 | |
| 244 | A K K D L E V E R | 7 | |
| 267 | R K Y E E T Q K E | 7 | |
| 277 | H N L N Q L L Y S | 7 | |
| 288 | R A D V Q H L E D | 7 | |
| 329 | L S Q V Q F L Y T | 7 | |
| 336 | Y T S L L K Q Q E | 7 | |
| 342 | Q Q E E Q T R V A | 7 | |
| 349 | V A L L E Q Q M Q | 7 | |
| 357 | Q A C T L D F E N | 7 | |
| 417 | E N R E K V A A S | 7 | |
| 424 | A S P K S P T A A | 7 | |
| 443 | C N I Q Y P A T E | 7 | |
| 445 | I Q Y P A T E H R | 7 | |
| 27 | L E K L K G E I A | 6 | |
| 35 | A H L K T S V D E | 6 | |
| 47 | G K G K L T D K E | 6 | |
| 68 | A E K E K N A Y Q | 6 | |
| 75 | Y Q L T E K D K E | 6 | |
| 110 | E G E R R E Q V L | 6 | |
| 118 | L K A L S E E K D | 6 | |
| 121 | L S E E K D V L K | 6 | |
| 135 | A T S R I A E L E | 6 | |
| 139 | I A E L E S K T N | 6 | |
| 168 | H E M E I Q L K D | 6 | |
| 181 | N Q Q W L V Y D Q | 6 | |
| 189 | Q Q R E V Y V K G | 6 | |
| 205 | L E K K T E T A A | 6 | |
| 214 | H S L P Q Q T K K | 6 | |
| 219 | Q T K K P E S E G | 6 | |
| 238 | N D L L A S A K K | 6 | |
| 255 | I T Q L S F E L S | 6 | |
| 256 | T Q L S F E L S E | 6 | |
| 258 | L S F E L S E F R | 6 | |
| 263 | S E F R R K Y E E | 6 | |
| 289 | A D V Q H L E D D | 6 | |
| 302 | E K I Q K L R E E | 6 | |
| 316 | G K L E E E K K R | 6 | |
| 318 | L E E E K K R S E | 6 | |
| 321 | E K K R S E E L L | 6 | |
| 356 | M Q A C T L D F E | 6 | |
| 368 | L D R Q H V Q H Q | 6 | |
| 395 | E S L K Q L H E F | 6 | |
| 400 | L H E F A I T E P | 6 | |
| 409 | L V T F Q G E T E | 6 | |
| 433 | L N E S L V E C P | 6 | |
| 452 | H R D L L V H V E | 6 | |
| 453 | R D L L V H V E Y | 6 | |

| TABLE XXIII 121P2A3 v.1: HLA Peptide Scoring Results A*0201 9-mers SYFPEITHI | | | |
|---|---|---|---|
| Pos | 1 2 3 4 5 6 7 8 9 | score | SEQ. ID NO. |
| 4 | R S T K D L I K S | 5 | |
| 18 | P S N S K S E T T | 5 | |
| 30 | L K G E I A H L K | 5 | |
| 37 | L K T S V D E I T | 5 | |
| 45 | T S G K G K L T D | 5 | |
| 77 | L T E K D K E I Q | 5 | |
| 87 | L R D Q L K A R Y | 5 | |
| 129 | K Q Q L S A A T S | 5 | |
| 144 | S K T N T L R L S | 5 | |
| 153 | Q T V A P N C F N | 5 | |
| 167 | I H E M E I Q L K | 5 | |
| 213 | A H S L P Q Q T K | 5 | |
| 224 | E S E G Y L Q E E | 5 | |
| 245 | K K D L E V E R Q | 5 | |
| 308 | R E E N D I A R G | 5 | |
| 314 | A R G K L E E E K | 5 | |
| 335 | L Y T S L L K Q Q | 5 | |
| 337 | T S L L K Q Q E E | 5 | |
| 347 | T R V A L L E Q Q | 5 | |
| 384 | L R K A R N Q I T | 5 | |
| 387 | A R N Q I T Q L E | 5 | |
| 438 | V E C P K C N I Q | 5 | |
| 450 | T E H R D L L V H | 5 | |
| 3 | S R S T K D L I K | 4 | |
| 10 | I K S K W G S K P | 4 | |
| 14 | W G S K P S N S K | 4 | |
| 15 | G S K P S N S K S | 4 | |
| 61 | E K I R V L E A E | 4 | |
| 63 | I R V L E A E K E | 4 | |
| 70 | K E K N A Y Q L T | 4 | |
| 101 | L E Q L E E T T R | 4 | |
| 130 | Q Q L S A A T S R | 4 | |
| 175 | K D A L E K N Q Q | 4 | |
| 188 | D Q Q R E V Y V K | 4 | |
| 207 | K K T E T A A H S | 4 | |
| 210 | E T A A H S L P Q | 4 | |
| 225 | S E G Y L Q E E K | 4 | |
| 234 | Q K C Y N D L L A | 4 | |
| 237 | Y N D L L A S A K | 4 | |
| 249 | E V E R Q T I T Q | 4 | |
| 264 | E F R R K Y E E T | 4 | |
| 265 | F R R K Y E E T Q | 4 | |
| 285 | S Q R R A D V Q H | 4 | |
| 287 | R R A D V Q H L E | 4 | |
| 290 | D V Q H L E D D R | 4 | |
| 292 | Q H L E D D R H K | 4 | |
| 294 | L E D D R H K T E | 4 | |
| 307 | L R E E N D I A R | 4 | |
| 340 | L K Q Q E E Q T R | 4 | |
| 361 | L D F E N E K L D | 4 | |
| 374 | Q H Q L H V I L K | 4 | |
| 382 | K E L R K A R N Q | 4 | |
| 385 | R K A R N Q I T Q | 4 | |
| 394 | L E S L K Q L H E | 4 | |
| 411 | T F Q G E T E N R | 4 | |
| 412 | F Q G E T E N R E | 4 | |

| TABLE XXIII 121P2A3 v.1: HLA Peptide Scoring Results A*0201 9-mers SYFPEITHI | | | |
|---|---|---|---|
| Pos | 1 2 3 4 5 6 7 8 9 | score | SEQ. ID NO. |
| 7 | K D L I K S K W G | 3 | |
| 16 | S K P S N S K S E | 3 | |
| 24 | E T T L E K L K G | 3 | |
| 49 | G K L T D K E R H | 3 | |
| 102 | E Q L E E T T R E | 3 | |
| 108 | T R E G E R R E Q | 3 | |
| 111 | G E R R E Q V L K | 3 | |
| 114 | R E Q V L K A L S | 3 | |
| 136 | T S R I A E L E S | 3 | |
| 151 | L S Q T V A P N C | 3 | |
| 183 | Q W L V Y D Q Q R | 3 | |
| 196 | K G L L A K I F E | 3 | |
| 229 | L Q E E K Q K C Y | 3 | |
| 260 | F E L S E F R R K | 3 | |
| 276 | V H N L N Q L L Y | 3 | |
| 279 | L N Q L L Y S Q R | 3 | |
| 280 | N Q L L Y S Q R R | 3 | |
| 300 | K T E K I Q K L R | 3 | |
| 377 | L H V I L K E L R | 3 | |
| 381 | L K E L R K A R N | 3 | |
| 388 | R N Q I T Q L E S | 3 | |
| 415 | E T E N R E K V A | 3 | |
| 418 | N R E K V A A S P | 3 | |
| 440 | C P K C N I Q Y P | 3 | |
| 6 | T K D L I K S K W | 2 | |
| 13 | K W G S K P S N S | 2 | |
| 28 | E K L K G E I A H | 2 | |
| 41 | V D E I T S G K G | 2 | |
| 67 | E A E K E K N A Y | 2 | |
| 71 | E K N A Y Q L T E | 2 | |
| 80 | K D K E I Q R L R | 2 | |
| 84 | I Q R L R D Q L K | 2 | |
| 104 | L E E T T R E G E | 2 | |
| 125 | K D V L K Q Q L S | 2 | |
| 142 | L E S K T N T L R | 2 | |
| 152 | S Q T V A P N C F | 2 | |
| 160 | F N S S I N N I H | 2 | |
| 165 | N N I H E M E I Q | 2 | |
| 174 | L K D A L E K N Q | 2 | |
| 202 | I F E L E K K T E | 2 | |
| 218 | Q Q T K K P E S E | 2 | |
| 220 | T K K P E S E G Y | 2 | |
| 223 | P E S E G Y L Q E | 2 | |
| 227 | G Y L Q E E K Q K | 2 | |
| 252 | R Q T I T Q L S F | 2 | |
| 272 | T Q K E V H N L N | 2 | |
| 284 | Y S Q R R A D V Q | 2 | |
| 322 | K K R S E E L L S | 2 | |
| 325 | S E E L L S Q V Q | 2 | |
| 333 | Q F L Y T S L L K | 2 | |
| 406 | T E P L V T F Q G | 2 | |
| 427 | K S P T A A L N E | 2 | |
| 435 | E S L V E C P K C | 2 | |
| 441 | P K C N I Q Y P A | 2 | |
| 11 | K S K W G S K P S | 1 | |
| 20 | N S K S E T T L E | 1 | |

| TABLE XXIII 121P2A3 v.1: HLA Peptide Scoring Results A*0201 9-mers SYFPEITHI | | | |
|---|---|---|---|
| Pos | 1 2 3 4 5 6 7 8 9 | score | SEQ. ID NO. |
| 23 | S E T T L E K L K | 1 | |
| 42 | D E I T S G K G K | 1 | |
| 48 | K G K L T D K E R | 1 | |
| 74 | A Y Q L T E K D K | 1 | |
| 78 | T E K D K E I Q R | 1 | |
| 88 | R D Q L K A R Y S | 1 | |
| 94 | R Y S T T A L L E | 1 | |
| 115 | E Q V L K A L S E | 1 | |
| 158 | N C F N S S I N N | 1 | |
| 182 | Q Q W L V Y D Q Q | 1 | |
| 186 | V Y D Q Q R E V Y | 1 | |
| 195 | V K G L L A K I F | 1 | |
| 206 | E K K T E T A A H | 1 | |
| 216 | L P Q Q T K K P E | 1 | |
| 217 | P Q Q T K K P E S | 1 | |
| 222 | K P E S E G Y L Q | 1 | |
| 259 | S F E L S E F R R | 1 | |
| 269 | Y E E T Q K E V H | 1 | |
| 291 | V Q H L E D D R H | 1 | |
| 304 | I Q K L R E E N D | 1 | |
| 311 | N D I A R G K L E | 1 | |
| 315 | R G K L E E E K K | 1 | |
| 326 | E E L L S Q V Q F | 1 | |
| 354 | Q Q M Q A C T L D | 1 | |
| 358 | A C T L D F E N E | 1 | |
| 366 | E K L D R Q H V Q | 1 | |
| 413 | Q G E T E N R E K | 1 | |
| 426 | P K S P T A A L N | 1 | |
| 446 | Q Y P A T E H R D | 1 | |
| 53 | D K E R H R L L E | -1 | |
| 81 | D K E I Q R L R D | -1 | |
| 179 | E K N Q Q W L V Y | -1 | |
| 209 | T E T A A H S L P | -1 | |
| 230 | Q E E K Q K C Y N | -1 | |
| 251 | E R Q T I T Q L S | -1 | |
| 262 | L S E F R R K Y E | -1 | |
| 270 | E E T Q K E V H N | -1 | |
| 295 | E D D R H K T E K | -1 | |
| 298 | R H K T E K I Q K | -1 | |
| 319 | E E E K K R S E E | -1 | |
| 362 | D F E N E K L D R | -1 | |
| 419 | R E K V A A S P K | -1 | |
| 105 | E E T T R E G E R | -2 | |
| 231 | E E K Q K C Y N D | -2 | |
| 266 | R R K Y E E T Q K | -2 | |
| 297 | D R H K T E K I Q | -2 | |
| 345 | E Q T R V A L L E | -3 | |
| 364 | E N E K L D R Q H | -3 | |
| 439 | E C P K C N I Q Y | -3 | |
| 157 | P N C F N S S I N | -4 | |

| TABLE XXIII 121P2A3 v.3: HLA Peptide Scoring Results A*0201 9-mers SYFPEITHI | | | |
|---|---|---|---|
| Pos | 1 2 3 4 5 6 7 8 9 | score | SEQ. ID NO. |
| 3 | L T D K E R Q R L | 16 | |

TABLE XXIII 121P2A3 v.3: HLA Peptide Scoring Results A*0201 9-mers SYFPEITHI

| Pos | 1 2 3 4 5 6 7 8 9 | score | SEQ. ID NO. |
|---|---|---|---|
| 2 | K L T D K E R Q R | 12 | |
| 7 | E R Q R L L E K I | 12 | |
| 9 | Q R L L E K I R V | 12 | |
| 4 | T D K E R Q R L L | 11 | |
| 6 | K E R Q R L L E K | 8 | |
| 1 | G K L T D K E R Q | 3 | |
| 5 | D K E R Q R L L E | -1 | |

TABLE XXIII 121P2A3 v.4: HLA Peptide Scoring Results A*0201 9-mers SYFPEITHI

| Pos | 1 2 3 4 5 6 7 8 9 | score | SEQ. ID NO. |
|---|---|---|---|
| 9 | T L L E Q L E E T | 24 | |
| 6 | S T T T L L E Q L | 20 | |
| 2 | K A R Y S T T T L | 18 | |
| 3 | A R Y S T T T L L | 15 | |
| 1 | L K A R Y S T T T | 11 | |
| 8 | T T L L E Q L E E | 9 | |
| 5 | Y S T T T L L E Q | 8 | |
| 7 | T T T L L E Q L E | 4 | |
| 4 | R Y S T T T L L E | 3 | |

TABLE XXIII 121P2A3 v.6: HLA Peptide Scoring Results A*0201 9-mers SYFPEITHI

| Pos | 1 2 3 4 5 6 7 8 9 | score | SEQ. ID NO. |
|---|---|---|---|
| 2 | E L L S Q V Q S L | 25 | |
| 9 | S L Y T S L L K Q | 20 | |
| 6 | Q V Q S L Y T S L | 17 | |
| 7 | V Q S L Y T S L L | 14 | |
| 3 | L L S Q V Q S L Y | 12 | |
| 5 | S Q V Q S L Y T S | 9 | |
| 4 | L S Q V Q S L Y T | 7 | |
| 8 | Q S L Y T S L L K | 2 | |
| 1 | E E L L S Q V Q S | 1 | |

TABLE XXIII 121P2A3 v.7: HLA Peptide Scoring Results A*0201 9-mers SYFPEITHI

| Pos | 1 2 3 4 5 6 7 8 9 | score | SEQ. ID NO. |
|---|---|---|---|
| 7 | Q L L V I L K E L | 27 | |
| 4 | V Q H Q L L V I L | 18 | |
| 3 | H V Q H Q L L V I | 17 | |
| 8 | L L V I L K E L R | 13 | |
| 2 | Q H V Q H Q L L V | 11 | |
| 1 | R Q H V Q H Q L L | 10 | |
| 9 | L V I L K E L R K | 10 | |
| 6 | H Q L L V I L K E | 9 | |
| 5 | Q H Q L L V I L K | 5 | |

TABLE XXIII 121P2A3 v.8: HLA Peptide Scoring Results A*0201 9-mers SYFPEITHI

| Pos | 1 2 3 4 5 6 7 8 9 | score | SEQ. ID NO. |
|---|---|---|---|
| 6 | A L N G S L V E C | 23 | |
| 3 | P T A A L N G S L | 16 | |
| 4 | T A A L N G S L V | 16 | |
| 5 | A A L N G S L V E | 11 | |
| 2 | S P T A A L N G S | 7 | |
| 7 | L N G S L V E C P | 7 | |
| 9 | G S L V E C P K C | 6 | |
| 1 | K S P T A A L N G | 2 | |

TABLE XXIV 121P2A3: HLA Peptide Scoring Results A*0202 9-mers SYFPEITHI

| Pos | 1 2 3 4 5 6 7 8 9 | score | SEQ. ID NO. |
|---|---|---|---|
| | NO DATA | | |

TABLE XXV 121P2A3: HLA Peptide Scoring Results A*0203 9-mers SYFPEITHI

| Pos | 1 2 3 4 5 6 7 8 9 | score | SEQ. ID NO. |
|---|---|---|---|
| | NO DATA | | |

TABLE XXVI 121P2A3 v.1: HLA Peptide Scoring Results A3 9-mers SYFPEITHI

| Pos | 1 2 3 4 5 6 7 8 9 | score | SEQ. ID NO. |
|---|---|---|---|
| 378 | H V I L K E L R K | 28 | |
| 62 | K I R V L E A E K | 27 | |
| 64 | R V L E A E K E K | 27 | |
| 367 | K L D R Q H V Q H | 27 | |
| 40 | S V D E I T S G K | 25 | |
| 90 | Q L K A R Y S T T | 24 | |
| 404 | A I T E P L V T F | 24 | |
| 9 | L I K S K W G S K | 23 | |
| 86 | R L R D Q L K A R | 23 | |
| 117 | V L K A L S E E K | 23 | |
| 390 | Q I T Q L E S L K | 23 | |
| 58 | R L L E K I R V L | 22 | |
| 456 | L V H V E Y C S K | 22 | |
| 54 | K E R H R L L E K | 21 | |
| 192 | E V Y V K G L L A | 21 | |
| 419 | R E K V A A S P K | 21 | |
| 111 | G E R R E Q V L K | 20 | |
| 172 | I Q L K D A L E K | 20 | |
| 350 | A L L E Q Q M Q A | 20 | |
| 380 | I L K E L R K A R | 20 | |
| 399 | Q L H E F A I T E | 20 | |
| 29 | K L K G E I A H L | 19 | |
| 50 | K L T D K E R H R | 19 | |
| 148 | T L R L S Q T V A | 19 | |
| 257 | Q L S F E L S E F | 19 | |
| 266 | R R K Y E E T Q K | 19 | |
| 348 | R V A L L E Q Q M | 19 | |
| 421 | K V A A S P K S P | 19 | |
| 84 | I Q R L R D Q L K | 18 | |
| 120 | A L S E E K D V L | 18 | |
| 137 | S R I A E L E S K | 18 | |
| 213 | A H S L P Q Q T K | 18 | |

| TABLE XXVI 121P2A3 v.1: HLA Peptide Scoring Results A3 9-mers SYFPEITHI | | | |
|---|---|---|---|
| Pos | 1 2 3 4 5 6 7 8 9 | score | SEQ. ID NO. |
| 237 | Y N D L L A S A K | 18 | |
| 261 | E L S E F R R K Y | 18 | |
| 285 | S Q R R A D V Q H | 18 | |
| 298 | R H K T E K I Q K | 18 | |
| 432 | A L N E S L V E C | 18 | |
| 453 | R D L L V H V E Y | 18 | |
| 116 | Q V L K A L S E E | 17 | |
| 126 | D V L K Q Q L S A | 17 | |
| 131 | Q L S A A T S R I | 17 | |
| 138 | R I A E L E S K T | 17 | |
| 150 | R L S Q T V A P N | 17 | |
| 188 | D Q Q R E V Y V K | 17 | |
| 239 | D L L A S A K K D | 17 | |
| 249 | E V E R Q T I T Q | 17 | |
| 306 | K L R E E N D I A | 17 | |
| 312 | D I A R G K L E E | 17 | |
| 328 | L L S Q V Q F L Y | 17 | |
| 333 | Q F L Y T S L L K | 17 | |
| 334 | F L Y T S L L K Q | 17 | |
| 383 | E L R K A R N Q I | 17 | |
| 409 | L V T F Q G E T E | 17 | |
| 42 | D E I T S G K G K | 16 | |
| 46 | S G K G K L T D K | 16 | |
| 99 | A L L E Q L E E T | 16 | |
| 121 | L S E E K D V L K | 16 | |
| 193 | V Y V K G L L A K | 16 | |
| 194 | Y V K G L L A K I | 16 | |
| 200 | A K I F E L E K K | 16 | |
| 214 | H S L P Q Q T K K | 16 | |
| 238 | N D L L A S A K K | 16 | |
| 282 | L L Y S Q R R A D | 16 | |
| 315 | R G K L E E E K K | 16 | |
| 393 | Q L E S L K Q L H | 16 | |
| 444 | N I Q Y P A T E H | 16 | |
| 3 | S R S T K D L I K | 15 | |
| 5 | S T K D L I K S K | 15 | |
| 21 | S K S E T T L E K | 15 | |
| 59 | L L E K I R V L E | 15 | |
| 72 | K N A Y Q L T E K | 15 | |
| 83 | E I Q R L R D Q L | 15 | |
| 127 | V L K Q Q L S A A | 15 | |
| 141 | E L E S K T N T L | 15 | |
| 185 | L V Y D Q Q R E V | 15 | |
| 197 | G L L A K I F E L | 15 | |
| 199 | L A K I F E L E K | 15 | |
| 204 | E L E K K T E T A | 15 | |
| 227 | G Y L Q E E K Q K | 15 | |
| 247 | D L E V E R Q T I | 15 | |
| 292 | Q H L E D D R H K | 15 | |
| 331 | Q V Q F L Y T S L | 15 | |
| 30 | L K G E I A H L K | 14 | |
| 74 | A Y Q L T E K D K | 14 | |
| 76 | Q L T E K D K E I | 14 | |
| 100 | L L E Q L E E T T | 14 | |
| 129 | K Q Q L S A A T S | 14 | |
| 130 | Q Q L S A A T S R | 14 | |

| TABLE XXVI 121P2A3 v.1: HLA Peptide Scoring Results A3 9-mers SYFPEITHI | | | |
|---|---|---|---|
| Pos | 1 2 3 4 5 6 7 8 9 | score | SEQ. ID NO. |
| 154 | T V A P N C F N S | 14 | |
| 173 | Q L K D A L E K N | 14 | |
| 177 | A L E K N Q Q W L | 14 | |
| 252 | R Q T I T Q L S F | 14 | |
| 281 | Q L L Y S Q R R A | 14 | |
| 290 | D V Q H L E D D R | 14 | |
| 295 | E D D R H K T E K | 14 | |
| 309 | E E N D I A R G K | 14 | |
| 317 | K L E E E K K R S | 14 | |
| 326 | E E L L S Q V Q F | 14 | |
| 339 | L L K Q Q E E Q T | 14 | |
| 372 | H V Q H Q L H V I | 14 | |
| 450 | T E H R D L L V H | 14 | |
| 8 | D L I K S K W G S | 13 | |
| 103 | Q L E E T T R E G | 13 | |
| 171 | E I Q L K D A L E | 13 | |
| 179 | E K N Q Q W L V Y | 13 | |
| 244 | A K K D L E V E R | 13 | |
| 314 | A R G K L E E E K | 13 | |
| 338 | S L L K Q Q E E Q | 13 | |
| 359 | C T L D F E N E K | 13 | |
| 374 | Q H Q L H V I L K | 13 | |
| 431 | A A L N E S L V E | 13 | |
| 436 | S L V E C P K C N | 13 | |
| 445 | I Q Y P A T E H R | 13 | |
| 14 | W G S K P S N S K | 12 | |
| 33 | E I A H L K T S V | 12 | |
| 56 | R H R L L E K I R | 12 | |
| 65 | V L E A E K E K N | 12 | |
| 166 | N I H E M E I Q L | 12 | |
| 167 | I H E M E I Q L K | 12 | |
| 201 | K I F E L E K K T | 12 | |
| 235 | K C Y N D L L A S | 12 | |
| 260 | F E L S E F R R K | 12 | |
| 278 | N L N Q L L Y S Q | 12 | |
| 284 | Y S Q R R A D V Q | 12 | |
| 293 | H L E D D R H K T | 12 | |
| 303 | K I Q K L R E E N | 12 | |
| 327 | E L L S Q V Q F L | 12 | |
| 376 | Q L H V I L K E L | 12 | |
| 382 | K E L R K A R N Q | 12 | |
| 385 | R K A R N Q I T Q | 12 | |
| 396 | S L K Q L H E F A | 12 | |
| 413 | Q G E T E N R E K | 12 | |
| 434 | N E S L V E C P K | 12 | |
| 454 | D L L V H V E Y C | 12 | |
| 23 | S E T T L E K L K | 11 | |
| 26 | T L E K L K G E I | 11 | |
| 34 | I A H L K T S V D | 11 | |
| 36 | H L K T S V D E I | 11 | |
| 43 | E I T S G K G K L | 11 | |
| 109 | R E G E R R E Q V | 11 | |
| 163 | S I N N I H E M E | 11 | |
| 183 | Q W L V Y D Q Q R | 11 | |
| 186 | V Y D Q Q R E V Y | 11 | |
| 189 | Q Q R E V Y V K G | 11 | |

TABLE XXVI 121P2A3 v.1: HLA Peptide Scoring Results A3 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 198 | L | L | A | K | I | F | E | L | E | 11 | |
| 215 | S | L | P | Q | Q | T | K | K | P | 11 | |
| 225 | S | E | G | Y | L | Q | E | E | K | 11 | |
| 228 | Y | L | Q | E | E | K | Q | K | C | 11 | |
| 240 | L | L | A | S | A | K | K | D | L | 11 | |
| 275 | E | V | H | N | L | N | Q | L | L | 11 | |
| 279 | L | N | Q | L | L | Y | S | Q | R | 11 | |
| 324 | R | S | E | E | L | L | S | Q | V | 11 | |
| 351 | L | L | E | Q | Q | M | Q | A | C | 11 | |
| 355 | Q | M | Q | A | C | T | L | D | F | 11 | |
| 386 | K | A | R | N | Q | I | T | Q | L | 11 | |
| 403 | F | A | I | T | E | P | L | V | T | 11 | |
| 408 | P | L | V | T | F | Q | G | E | T | 11 | |
| 423 | A | A | S | P | K | S | P | T | A | 11 | |
| 443 | C | N | I | Q | Y | P | A | T | E | 11 | |
| 455 | L | L | V | H | V | E | Y | C | S | 11 | |
| 78 | T | E | K | D | K | E | I | Q | R | 10 | |
| 85 | Q | R | L | R | D | Q | L | K | A | 10 | |
| 93 | A | R | Y | S | T | T | A | L | L | 10 | |
| 94 | R | Y | S | T | T | A | L | L | E | 10 | |
| 101 | L | E | Q | L | E | E | T | T | R | 10 | |
| 106 | E | T | T | R | E | G | E | R | R | 10 | |
| 115 | E | Q | V | L | K | A | L | S | E | 10 | |
| 184 | W | L | V | Y | D | Q | Q | R | E | 10 | |
| 207 | K | K | T | E | T | A | A | H | S | 10 | |
| 220 | T | K | K | P | E | S | E | G | Y | 10 | |
| 246 | K | D | L | E | V | E | R | Q | T | 10 | |
| 276 | V | H | N | L | N | Q | L | L | Y | 10 | |
| 311 | N | D | I | A | R | G | K | L | E | 10 | |
| 345 | E | Q | T | R | V | A | L | L | E | 10 | |
| 360 | T | L | D | F | E | N | E | K | L | 10 | |
| 379 | V | I | L | K | E | L | R | K | A | 10 | |
| 437 | L | V | E | C | P | K | C | N | I | 10 | |
| 28 | E | K | L | K | G | E | I | A | H | 9 | |
| 32 | G | E | I | A | H | L | K | T | S | 9 | |
| 35 | A | H | L | K | T | S | V | D | E | 9 | |
| 45 | T | S | G | K | G | K | L | T | D | 9 | |
| 48 | K | G | K | L | T | D | K | E | R | 9 | |
| 68 | A | E | K | E | K | N | A | Y | Q | 9 | |
| 71 | E | K | N | A | Y | Q | L | T | E | 9 | |
| 80 | K | D | K | E | I | Q | R | L | R | 9 | |
| 87 | L | R | D | Q | L | K | A | R | Y | 9 | |
| 88 | R | D | Q | L | K | A | R | Y | S | 9 | |
| 91 | L | K | A | R | Y | S | T | T | A | 9 | |
| 110 | E | G | E | R | R | E | Q | V | L | 9 | |
| 135 | A | T | S | R | I | A | E | L | E | 9 | |
| 145 | K | T | N | T | L | R | L | S | Q | 9 | |
| 146 | T | N | T | L | R | L | S | Q | T | 9 | |
| 147 | N | T | L | R | L | S | Q | T | V | 9 | |
| 206 | E | K | K | T | E | T | A | A | H | 9 | |
| 223 | P | E | S | E | G | Y | L | Q | E | 9 | |
| 267 | R | K | Y | E | E | T | Q | K | E | 9 | |
| 322 | K | K | R | S | E | E | L | L | S | 9 | |
| 323 | K | R | S | E | E | L | L | S | Q | 9 | |
| 366 | E | K | L | D | R | Q | H | V | Q | 9 | |
| 417 | E | N | R | E | K | V | A | A | S | 9 | |

TABLE XXVI 121P2A3 v.1: HLA Peptide Scoring Results A3 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 418 | N | R | E | K | V | A | A | S | P | 9 | |
| 426 | P | K | S | P | T | A | A | L | N | 9 | |
| 427 | K | S | P | T | A | A | L | N | E | 9 | |
| 439 | E | C | P | K | C | N | I | Q | Y | 9 | |
| 7 | K | D | L | I | K | S | K | W | G | 8 | |
| 10 | I | K | S | K | W | G | S | K | P | 8 | |
| 13 | K | W | G | S | K | P | S | N | S | 8 | |
| 44 | I | T | S | G | K | G | K | L | T | 8 | |
| 61 | E | K | I | R | V | L | E | A | E | 8 | |
| 82 | K | E | I | Q | R | L | R | D | Q | 8 | |
| 92 | K | A | R | Y | S | T | T | A | L | 8 | |
| 134 | A | A | T | S | R | I | A | E | L | 8 | |
| 156 | A | P | N | C | F | N | S | S | I | 8 | |
| 175 | K | D | A | L | E | K | N | Q | Q | 8 | |
| 195 | V | K | G | L | L | A | K | I | F | 8 | |
| 208 | K | T | E | T | A | A | H | S | L | 8 | |
| 229 | L | Q | E | E | K | Q | K | C | Y | 8 | |
| 242 | A | S | A | K | K | D | L | E | V | 8 | |
| 253 | Q | T | I | T | Q | L | S | F | E | 8 | |
| 254 | T | I | T | Q | L | S | F | E | L | 8 | |
| 269 | Y | E | E | T | Q | K | E | V | H | 8 | |
| 308 | R | E | E | N | D | I | A | R | G | 8 | |
| 313 | I | A | R | G | K | L | E | E | E | 8 | |
| 340 | L | K | Q | Q | E | E | Q | T | R | 8 | |
| 343 | Q | E | E | Q | T | R | V | A | L | 8 | |
| 346 | Q | T | R | V | A | L | L | E | Q | 8 | |
| 362 | D | F | E | N | E | K | L | D | R | 8 | |
| 370 | R | Q | H | V | Q | H | Q | L | H | 8 | |
| 388 | R | N | Q | I | T | Q | L | E | S | 8 | |
| 398 | K | Q | L | H | E | F | A | I | T | 8 | |
| 416 | T | E | N | R | E | K | V | A | A | 8 | |
| 449 | A | T | E | H | R | D | L | L | V | 8 | |
| 451 | E | H | R | D | L | L | V | H | V | 8 | |
| 15 | G | S | K | P | S | N | S | K | S | 7 | |
| 17 | K | P | S | N | S | K | S | E | T | 7 | |
| 31 | K | G | E | I | A | H | L | K | T | 7 | |
| 38 | K | T | S | V | D | E | I | T | S | 7 | |
| 53 | D | K | E | R | H | R | L | L | E | 7 | |
| 60 | L | E | K | I | R | V | L | E | A | 7 | |
| 67 | E | A | E | K | E | K | N | A | Y | 7 | |
| 105 | E | E | T | T | R | E | G | E | R | 7 | |
| 112 | E | R | R | E | Q | V | L | K | A | 7 | |
| 114 | R | E | Q | V | L | K | A | L | S | 7 | |
| 136 | T | S | R | I | A | E | L | E | S | 7 | |
| 139 | I | A | E | L | E | S | K | T | N | 7 | |
| 140 | A | E | L | E | S | K | T | N | T | 7 | |
| 149 | L | R | L | S | Q | T | V | A | P | 7 | |
| 176 | D | A | L | E | K | N | Q | Q | W | 7 | |
| 180 | K | N | Q | Q | W | L | V | Y | D | 7 | |
| 182 | Q | Q | W | L | V | Y | D | Q | Q | 7 | |
| 202 | I | F | E | L | E | K | K | T | E | 7 | |
| 212 | A | A | H | S | L | P | Q | Q | T | 7 | |
| 250 | V | E | R | Q | T | I | T | Q | L | 7 | |
| 256 | T | Q | L | S | F | E | L | S | E | 7 | |
| 264 | E | F | R | R | K | Y | E | E | T | 7 | |
| 265 | F | R | R | K | Y | E | E | T | Q | 7 | |

TABLE XXVI 121P2A3 v.1: HLA Peptide Scoring Results A3 9-mers SYFPEITHI

| Pos | 1 2 3 4 5 6 7 8 9 | score | SEQ. ID NO. |
|---|---|---|---|
| 280 | N Q L L Y S Q R R | 7 | |
| 286 | Q R R A D V Q H L | 7 | |
| 287 | R R A D V Q H L E | 7 | |
| 288 | R A D V Q H L E D | 7 | |
| 294 | L E D D R H K T E | 7 | |
| 300 | K T E K I Q K L R | 7 | |
| 305 | Q K L R E E N D I | 7 | |
| 316 | G K L E E E K K R | 7 | |
| 342 | Q Q E E Q T R V A | 7 | |
| 353 | E Q Q M Q A C T L | 7 | |
| 364 | E N E K L D R Q H | 7 | |
| 415 | E T E N R E K V A | 7 | |
| 11 | K S K W G S K P S | 6 | |
| 19 | S N S K S E T T L | 6 | |
| 27 | L E K L K G E I A | 6 | |
| 49 | G K L T D K E R H | 6 | |
| 63 | I R V L E A E K E | 6 | |
| 69 | E K E K N A Y Q L | 6 | |
| 96 | S T T A L L E Q L | 6 | |
| 98 | T A L L E Q L E E | 6 | |
| 107 | T T R E G E R R E | 6 | |
| 113 | R R E Q V L K A L | 6 | |
| 123 | E E K D V L K Q Q | 6 | |
| 152 | S Q T V A P N C F | 6 | |
| 187 | Y D Q Q R E V Y V | 6 | |
| 191 | R E V Y V K G L L | 6 | |
| 196 | K G L L A K I F E | 6 | |
| 210 | E T A A H S L P Q | 6 | |
| 218 | Q Q T K K P E S E | 6 | |
| 219 | Q T K K P E S E G | 6 | |
| 222 | K P E S E G Y L Q | 6 | |
| 236 | C Y N D L L A S A | 6 | |
| 243 | S A K K D L E V E | 6 | |
| 259 | S F E L S E F R R | 6 | |
| 263 | S E F R R K Y E E | 6 | |
| 274 | K E V H N L N Q L | 6 | |
| 277 | H N L N Q L L Y S | 6 | |
| 283 | L Y S Q R R A D V | 6 | |
| 304 | I Q K L R E E N D | 6 | |
| 307 | L R E E N D I A R | 6 | |
| 325 | S E E L L S Q V Q | 6 | |
| 375 | H Q L H V I L K E | 6 | |
| 381 | L K E L R K A R N | 6 | |
| 391 | I T Q L E S L K Q | 6 | |
| 392 | T Q L E S L K Q L | 6 | |
| 395 | E S L K Q L H E F | 6 | |
| 405 | I T E P L V T F Q | 6 | |
| 411 | T F Q G E T E N R | 6 | |
| 424 | A S P K S P T A A | 6 | |
| 425 | S P K S P T A A L | 6 | |
| 12 | S K W G S K P S N | 5 | |
| 18 | P S N S K S E T T | 5 | |
| 70 | K E K N A Y Q L T | 5 | |
| 81 | D K E I Q R L R D | 5 | |
| 89 | D Q L K A R Y S T | 5 | |
| 119 | K A L S E E K D V | 5 | |
| 125 | K D V L K Q Q L S | 5 | |
| 142 | L E S K T N T L R | 5 | |
| 162 | S S I N N I H E M | 5 | |
| 203 | F E L E K K T E T | 5 | |
| 209 | T E T A A H S L P | 5 | |
| 221 | K K P E S E G Y L | 5 | |
| 226 | E G Y L Q E E K Q | 5 | |
| 234 | Q K C Y N D L L A | 5 | |
| 258 | L S F E L S E F R | 5 | |
| 270 | E E T Q K E V H N | 5 | |
| 273 | Q K E V H N L N Q | 5 | |
| 291 | V Q H L E D D R H | 5 | |
| 301 | T E K I Q K L R E | 5 | |
| 319 | E E E K K R S E E | 5 | |
| 330 | S Q V Q F L Y T S | 5 | |
| 341 | K Q Q E E Q T R V | 5 | |
| 344 | E E Q T R V A L L | 5 | |
| 347 | T R V A L L E Q Q | 5 | |
| 371 | Q H V Q H Q L H V | 5 | |
| 377 | L H V I L K E L R | 5 | |
| 384 | L R K A R N Q I T | 5 | |
| 389 | N Q I T Q L E S L | 5 | |
| 394 | L E S L K Q L H E | 5 | |
| 406 | T E P L V T F Q G | 5 | |
| 429 | P T A A L N E S L | 5 | |
| 430 | T A A L N E S L V | 5 | |
| 442 | K C N I Q Y P A T | 5 | |
| 452 | H R D L L V H V E | 5 | |
| 2 | S S R S T K D L I | 4 | |
| 4 | R S T K D L I K S | 4 | |
| 6 | T K D L I K S K W | 4 | |
| 24 | E T T L E K L K G | 4 | |
| 25 | T T L E K L K G E | 4 | |
| 39 | T S V D E I T S G | 4 | |
| 52 | T D K E R H R L L | 4 | |
| 73 | N A Y Q L T E K D | 4 | |
| 102 | E Q L E E T T R E | 4 | |
| 122 | S E E K D V L K Q | 4 | |
| 128 | L K Q Q L S A A T | 4 | |
| 133 | S A A T S R I A E | 4 | |
| 143 | E S K T N T L R L | 4 | |
| 151 | L S Q T V A P N C | 4 | |
| 153 | Q T V A P N C F N | 4 | |
| 160 | F N S S I N N I H | 4 | |
| 164 | I N N I H E M E I | 4 | |
| 168 | H E M E I Q L K D | 4 | |
| 170 | M E I Q L K D A L | 4 | |
| 211 | T A A H S L P Q Q | 4 | |
| 233 | K Q K C Y N D L L | 4 | |
| 245 | K K D L E V E R Q | 4 | |
| 248 | L E V E R Q T I T | 4 | |
| 268 | K Y E E T Q K E V | 4 | |
| 296 | D D R H K T E K I | 4 | |
| 302 | E K I Q K L R E E | 4 | |
| 318 | L E E E K K R S E | 4 | |
| 321 | E K K R S E E L L | 4 | |

TABLE XXVI 121P2A3 v.1: HLA Peptide Scoring Results A3 9-mers SYFPEITHI

| Pos | 1 2 3 4 5 6 7 8 9 | score | SEQ. ID NO. |
|---|---|---|---|
| 332 | V Q F L Y T S L L | 4 | |
| 336 | Y T S L L K Q Q E | 4 | |
| 337 | T S L L K Q Q E E | 4 | |
| 349 | V A L L E Q Q M Q | 4 | |
| 358 | A C T L D F E N E | 4 | |
| 369 | D R Q H V Q H Q L | 4 | |
| 373 | V Q H Q L H V I L | 4 | |
| 422 | V A A S P K S P T | 4 | |
| 438 | V E C P K C N I Q | 4 | |
| 446 | Q Y P A T E H R D | 4 | |
| 1 | M S S R S T K D L | 3 | |
| 16 | S K P S N S K S E | 3 | |
| 22 | K S E T T L E K L | 3 | |
| 55 | E R H R L L E K I | 3 | |
| 57 | H R L L E K I R V | 3 | |
| 66 | L E A E K E K N A | 3 | |
| 95 | Y S T T A L L E Q | 3 | |
| 108 | T R E G E R R E Q | 3 | |
| 124 | E K D V L K Q Q L | 3 | |
| 132 | L S A A T S R I A | 3 | |
| 155 | V A P N C F N S S | 3 | |
| 157 | P N C F N S S I N | 3 | |
| 165 | N N I H E M E I Q | 3 | |
| 205 | L E K K T E T A A | 3 | |
| 241 | L A S A K K D L E | 3 | |
| 255 | I T Q L S F E L S | 3 | |
| 262 | L S E F R R K Y E | 3 | |
| 272 | T Q K E V H N L N | 3 | |
| 289 | A D V Q H L E D D | 3 | |
| 310 | E N D I A R G K L | 3 | |
| 320 | E E K K R S E E L | 3 | |
| 329 | L S Q V Q F L Y T | 3 | |
| 335 | L Y T S L L K Q Q | 3 | |
| 352 | L E Q Q M Q A C T | 3 | |
| 354 | Q Q M Q A C T L D | 3 | |
| 365 | N E K L D R Q H V | 3 | |
| 368 | L D R Q H V Q H Q | 3 | |
| 387 | A R N Q I T Q L E | 3 | |
| 410 | V T F Q G E T E N | 3 | |
| 428 | S P T A A L N E S | 3 | |
| 20 | N S K S E T T L E | 2 | |
| 41 | V D E I T S G K G | 2 | |
| 75 | Y Q L T E K D K E | 2 | |
| 97 | T T A L L E Q L E | 2 | |
| 118 | L K A L S E E K D | 2 | |
| 144 | S K T N T L R L S | 2 | |
| 174 | L K D A L E K N Q | 2 | |
| 178 | L E K N Q Q W L V | 2 | |
| 190 | Q R E V Y V K G L | 2 | |
| 224 | E S E G Y L Q E E | 2 | |
| 230 | Q E E K Q K C Y N | 2 | |
| 231 | E E K Q K C Y N D | 2 | |
| 299 | H K T E K I Q K L | 2 | |
| 357 | Q A C T L D F E N | 2 | |
| 363 | F E N E K L D R Q | 2 | |
| 397 | L K Q L H E F A I | 2 | |

TABLE XXVI 121P2A3 v.1: HLA Peptide Scoring Results A3 9-mers SYFPEITHI

| Pos | 1 2 3 4 5 6 7 8 9 | score | SEQ. ID NO. |
|---|---|---|---|
| 401 | H E F A I T E P L | 2 | |
| 402 | E F A I T E P L V | 2 | |
| 407 | E P L V T F Q G E | 2 | |
| 414 | G E T E N R E K V | 2 | |
| 420 | E K V A A S P K S | 2 | |
| 435 | E S L V E C P K C | 2 | |
| 448 | P A T E H R D L L | 2 | |
| 37 | L K T S V D E I T | 1 | |
| 51 | L T D K E R H R L | 1 | |
| 104 | L E E T T R E G E | 1 | |
| 158 | N C F N S S I N N | 1 | |
| 161 | N S S I N N I H E | 1 | |
| 169 | E M E I Q L K D A | 1 | |
| 217 | P Q Q T K K P E S | 1 | |
| 356 | M Q A C T L D F E | 1 | |
| 361 | L D F E N E K L D | 1 | |
| 400 | L H E F A I T E P | 1 | |
| 412 | F Q G E T E N R E | 1 | |
| 440 | C P K C N I Q Y P | 1 | |
| 441 | P K C N I Q Y P A | 1 | |
| 447 | Y P A T E H R D L | 1 | |

TABLE XXVI 121P2A3 v.3: HLA Peptide Scoring Results A3 9-mers SYFPEITHI

| Pos | 1 2 3 4 5 6 7 8 9 | score | SEQ. ID NO. |
|---|---|---|---|
| 2 | K L T D K E R Q R | 22 | |
| 6 | K E R Q R L L E K | 21 | |
| 8 | R Q R L L E K I R | 12 | |
| 5 | D K E R Q R L L E | 7 | |
| 9 | Q R L L E K I R V | 5 | |
| 4 | T D K E R Q R L L | 4 | |
| 7 | E R Q R L L E K I | 3 | |
| 1 | G K L T D K E R Q | 2 | |
| 3 | L T D K E R Q R L | 1 | |

TABLE XXVI 121P2A3 v.4: HLA Peptide Scoring Results A3 9-mers SYFPEITHI

| Pos | 1 2 3 4 5 6 7 8 9 | score | SEQ. ID NO. |
|---|---|---|---|
| 9 | T L L E Q L E E T | 13 | |
| 2 | K A R Y S T T T L | 11 | |
| 1 | L K A R Y S T T T | 9 | |
| 3 | A R Y S T T T L L | 8 | |
| 4 | R Y S T T T L L E | 7 | |
| 8 | T T L L E Q L E E | 6 | |
| 6 | S T T T L L E Q L | 4 | |
| 5 | Y S T T T L L E Q | 3 | |
| 7 | T T T L L E Q L E | 1 | |

TABLE XXVI 121P2A3 v.6: HLA Peptide Scoring Results A3 9-mers SYFPEITHI

| Pos | 1 2 3 4 5 6 7 8 9 | score | SEQ. ID NO. |
|---|---|---|---|
| 3 | L L S Q V Q S L Y | 20 | |
| 9 | S L Y T S L L K Q | 18 | |

| TABLE XXVI 121P2A3 v.6: HLA Peptide Scoring Results A3 9-mers SYFPEITHI | | | |
|---|---|---|---|
| Pos | 1 2 3 4 5 6 7 8 9 | score | SEQ. ID NO. |
| 8 | Q S L Y T S L L K | 17 | |
| 6 | Q V Q S L Y T S L | 15 | |
| 2 | E L L S Q V Q S L | 14 | |
| 1 | E E L L S Q V Q S | 10 | |
| 5 | S Q V Q S L Y T S | 5 | |
| 7 | V Q S L Y T S L L | 4 | |
| 4 | L S Q V Q S L Y T | 3 | |

| TABLE XXVI 121P2A3 v.7: HLA Peptide Scoring Results A3 9-mers SYFPEITHI | | | |
|---|---|---|---|
| Pos | 1 2 3 4 5 6 7 8 9 | score | SEQ. ID NO. |
| 9 | L V I L K E L R K | 28 | |
| 3 | H V Q H Q L L V I | 17 | |
| 8 | L L V I L K E L R | 15 | |
| 7 | Q L L V I L K E L | 14 | |
| 5 | Q H Q L L V I L K | 13 | |
| 6 | H Q L L V I L K E | 7 | |
| 2 | Q H V Q H Q L L V | 5 | |
| 1 | R Q H V Q H Q L L | 4 | |
| 4 | V Q H Q L L V I L | 4 | |

| TABLE XXVI 121P2A3 v.8: HLA Peptide Scoring Results A3 9-mers SYFPEITHI | | | |
|---|---|---|---|
| Pos | 1 2 3 4 5 6 7 8 9 | score | SEQ. ID NO. |
| 6 | A L N G S L V E C | 19 | |
| 5 | A A L N G S L V E | 15 | |
| 8 | N G S L V E C P K | 12 | |
| 1 | K S P T A A L N G | 9 | |
| 4 | T A A L N G S L V | 8 | |
| 3 | P T A A L N G S L | 6 | |
| 2 | S P T A A L N G S | 3 | |
| 9 | G S L V E C P K C | 2 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 271 | E T Q K E V H N L | 30 | |
| 327 | E L L S Q V Q F L | 29 | |
| 261 | E L S E F R R K Y | 27 | |
| 404 | A I T E P L V T F | 27 | |
| 43 | E I T S G K G K L | 26 | |
| 83 | E I Q R L R D Q L | 26 | |
| 275 | E V H N L N Q L L | 26 | |
| 29 | K L K G E I A H L | 25 | |
| 96 | S T T A L L E Q L | 24 | |
| 141 | E L E S K T N T L | 24 | |
| 257 | Q L S F E L S E F | 24 | |
| 331 | Q V Q F L Y T S L | 24 | |
| 58 | R L L E K I R V L | 23 | |
| 395 | E S L K Q L H E F | 23 | |
| 79 | E K D K E I Q R L | 22 | |
| 197 | G L L A K I F E L | 22 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 348 | R V A L L E Q Q M | 22 | |
| 51 | L T D K E R H R L | 21 | |
| 166 | N I H E M E I Q L | 21 | |
| 254 | T I T Q L S F E L | 21 | |
| 376 | Q L H V I L K E L | 21 | |
| 429 | P T A A L N E S L | 21 | |
| 194 | Y V K G L L A K I | 20 | |
| 8 | D L I K S K W G S | 19 | |
| 33 | E I A H L K T S V | 19 | |
| 126 | D V L K Q Q L S A | 19 | |
| 208 | K T E T A A H S L | 19 | |
| 232 | E K Q K C Y N D L | 19 | |
| 326 | E E L L S Q V Q F | 19 | |
| 328 | L L S Q V Q F L Y | 19 | |
| 344 | E E Q T R V A L L | 19 | |
| 439 | E C P K C N I Q Y | 19 | |
| 5 | S T K D L I K S K | 18 | |
| 25 | T T L E K L K G E | 18 | |
| 67 | E A E K E K N A Y | 18 | |
| 120 | A L S E E K D V L | 18 | |
| 124 | E K D V L K Q Q L | 18 | |
| 171 | E I Q L K D A L E | 18 | |
| 177 | A L E K N Q Q W L | 18 | |
| 179 | E K N Q Q W L V Y | 18 | |
| 240 | L L A S A K K D L | 18 | |
| 253 | Q T I T Q L S F E | 18 | |
| 264 | E F R R K Y E E T | 18 | |
| 312 | D I A R G K L E E | 18 | |
| 360 | T L D F E N E K L | 18 | |
| 454 | D L L V H V E Y C | 18 | |
| 24 | E T T L E K L K G | 17 | |
| 106 | E T T R E G E R R | 17 | |
| 116 | Q V L K A L S E E | 17 | |
| 127 | V L K Q Q L S A A | 17 | |
| 192 | E V Y V K G L L A | 17 | |
| 210 | E T A A H S L P Q | 17 | |
| 249 | E V E R Q T I T Q | 17 | |
| 290 | D V Q H L E D D R | 17 | |
| 299 | H K T E K I Q K L | 17 | |
| 320 | E E K K R S E E L | 17 | |
| 405 | I T E P L V T F Q | 17 | |
| 432 | A L N E S L V E C | 17 | |
| 69 | E K E K N A Y Q L | 16 | |
| 99 | A L L E Q L E E T | 16 | |
| 138 | R I A E L E S K T | 16 | |
| 143 | E S K T N T L R L | 16 | |
| 201 | K I F E L E K K T | 16 | |
| 204 | E L E K K T E T A | 16 | |
| 239 | D L L A S A K K D | 16 | |
| 310 | E N D I A R G K L | 16 | |
| 321 | E K K R S E E L L | 16 | |
| 351 | L L E Q Q M Q A C | 16 | |
| 379 | V I L K E L R K A | 16 | |
| 383 | E L R K A R N Q I | 16 | |
| 389 | N Q I T Q L E S L | 16 | |
| 392 | T Q L E S L K Q L | 16 | |

TABLE XXVII 121P2A3 v.1: HLA Peptide Scoring Results A26 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 415 | E | T | E | N | R | E | K | V | A | 16 | |
| 36 | H | L | K | T | S | V | D | E | I | 15 | |
| 40 | S | V | D | E | I | T | S | G | K | 15 | |
| 86 | R | L | R | D | Q | L | K | A | R | 15 | |
| 87 | L | R | D | Q | L | K | A | R | Y | 15 | |
| 110 | E | G | E | R | R | E | Q | V | L | 15 | |
| 134 | A | A | T | S | R | I | A | E | L | 15 | |
| 162 | S | S | I | N | N | I | H | E | M | 15 | |
| 173 | Q | L | K | D | A | L | E | K | N | 15 | |
| 247 | D | L | E | V | E | R | Q | T | I | 15 | |
| 255 | I | T | Q | L | S | F | E | L | S | 15 | |
| 278 | N | L | N | Q | L | L | Y | S | Q | 15 | |
| 302 | E | K | I | Q | K | L | R | E | E | 15 | |
| 346 | Q | T | R | V | A | L | L | E | Q | 15 | |
| 353 | E | Q | Q | M | Q | A | C | T | L | 15 | |
| 369 | D | R | Q | H | V | Q | H | Q | L | 15 | |
| 372 | H | V | Q | H | Q | L | H | V | I | 15 | |
| 417 | E | N | R | E | K | V | A | A | S | 15 | |
| 456 | L | V | H | V | E | Y | C | S | K | 15 | |
| 9 | L | I | K | S | K | W | G | S | K | 14 | |
| 90 | Q | L | K | A | R | Y | S | T | T | 14 | |
| 112 | E | R | R | E | Q | V | L | K | A | 14 | |
| 113 | R | R | E | Q | V | L | K | A | L | 14 | |
| 150 | R | L | S | Q | T | V | A | P | N | 14 | |
| 154 | T | V | A | P | N | C | F | N | S | 14 | |
| 198 | L | L | A | K | I | F | E | L | E | 14 | |
| 219 | Q | T | K | K | P | E | S | E | G | 14 | |
| 220 | T | K | K | P | E | S | E | G | Y | 14 | |
| 224 | E | S | E | G | Y | L | Q | E | E | 14 | |
| 250 | V | E | R | Q | T | I | T | Q | L | 14 | |
| 286 | Q | R | R | A | D | V | Q | H | L | 14 | |
| 334 | F | L | Y | T | S | L | L | K | Q | 14 | |
| 378 | H | V | I | L | K | E | L | R | K | 14 | |
| 386 | K | A | R | N | Q | I | T | Q | L | 14 | |
| 402 | E | F | A | I | T | E | P | L | V | 14 | |
| 410 | V | T | F | Q | G | E | T | E | N | 14 | |
| 451 | E | H | R | D | L | L | V | H | V | 14 | |
| 22 | K | S | E | T | T | L | E | K | L | 13 | |
| 44 | I | T | S | G | K | G | K | L | T | 13 | |
| 61 | E | K | I | R | V | L | E | A | E | 13 | |
| 62 | K | I | R | V | L | E | A | E | K | 13 | |
| 64 | R | V | L | E | A | E | K | E | K | 13 | |
| 97 | T | T | A | L | L | E | Q | L | E | 13 | |
| 107 | T | T | R | E | G | E | R | R | E | 13 | |
| 123 | E | E | K | D | V | L | K | Q | Q | 13 | |
| 159 | C | F | N | S | S | I | N | N | I | 13 | |
| 170 | M | E | I | Q | L | K | D | A | L | 13 | |
| 185 | L | V | Y | D | Q | Q | R | E | V | 13 | |
| 186 | V | Y | D | Q | Q | R | E | V | Y | 13 | |
| 190 | Q | R | E | V | Y | V | K | G | L | 13 | |
| 229 | L | Q | E | E | K | Q | K | C | Y | 13 | |
| 274 | K | E | V | H | N | L | N | Q | L | 13 | |
| 303 | K | I | Q | K | L | R | E | E | N | 13 | |
| 336 | Y | T | S | L | L | K | Q | Q | E | 13 | |
| 350 | A | L | L | E | Q | Q | M | Q | A | 13 | |
| 380 | I | L | K | E | L | R | K | A | R | 13 | |

TABLE XXVII 121P2A3 v.1: HLA Peptide Scoring Results A26 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 390 | Q | I | T | Q | L | E | S | L | K | 13 | |
| 421 | K | V | A | A | S | P | K | S | P | 13 | |
| 55 | E | R | H | R | L | L | E | K | I | 12 | |
| 76 | Q | L | T | E | K | D | K | E | I | 12 | |
| 145 | K | T | N | T | L | R | L | S | Q | 12 | |
| 147 | N | T | L | R | L | S | Q | T | V | 12 | |
| 215 | S | L | P | Q | Q | T | K | K | P | 12 | |
| 221 | K | K | P | E | S | E | G | Y | L | 12 | |
| 228 | Y | L | Q | E | E | K | Q | K | C | 12 | |
| 339 | L | L | K | Q | Q | E | E | Q | T | 12 | |
| 359 | C | T | L | D | F | E | N | E | K | 12 | |
| 362 | D | F | E | N | E | K | L | D | R | 12 | |
| 367 | K | L | D | R | Q | H | V | Q | H | 12 | |
| 407 | E | P | L | V | T | F | Q | G | E | 12 | |
| 425 | S | P | K | S | P | T | A | A | L | 12 | |
| 453 | R | D | L | L | V | H | V | E | Y | 12 | |
| 26 | T | L | E | K | L | K | G | E | I | 11 | |
| 50 | K | L | T | D | K | E | R | H | R | 11 | |
| 52 | T | D | K | E | R | H | R | L | L | 11 | |
| 77 | L | T | E | K | D | K | E | I | Q | 11 | |
| 103 | Q | L | E | E | T | T | R | E | G | 11 | |
| 117 | V | L | K | A | L | S | E | E | K | 11 | |
| 163 | S | I | N | N | I | H | E | M | E | 11 | |
| 169 | E | M | E | I | Q | L | K | D | A | 11 | |
| 176 | D | A | L | E | K | N | Q | Q | W | 11 | |
| 195 | V | K | G | L | L | A | K | I | F | 11 | |
| 252 | R | Q | T | I | T | Q | L | S | F | 11 | |
| 282 | L | L | Y | S | Q | R | R | A | D | 11 | |
| 300 | K | T | E | K | I | Q | K | L | R | 11 | |
| 317 | K | L | E | E | E | K | K | R | S | 11 | |
| 332 | V | Q | F | L | Y | T | S | L | L | 11 | |
| 338 | S | L | L | K | Q | Q | E | E | Q | 11 | |
| 343 | Q | E | E | Q | T | R | V | A | L | 11 | |
| 373 | V | Q | H | Q | L | H | V | I | L | 11 | |
| 391 | I | T | Q | L | E | S | L | K | Q | 11 | |
| 393 | Q | L | E | S | L | K | Q | L | H | 11 | |
| 396 | S | L | K | Q | L | H | E | F | A | 11 | |
| 409 | L | V | T | F | Q | G | E | T | E | 11 | |
| 437 | L | V | E | C | P | K | C | N | I | 11 | |
| 444 | N | I | Q | Y | P | A | T | E | H | 11 | |
| 19 | S | N | S | K | S | E | T | T | L | 10 | |
| 38 | K | T | S | V | D | E | I | T | S | 10 | |
| 42 | D | E | I | T | S | G | K | G | K | 10 | |
| 59 | L | L | E | K | I | R | V | L | E | 10 | |
| 92 | K | A | R | Y | S | T | T | A | L | 10 | |
| 93 | A | R | Y | S | T | T | A | L | L | 10 | |
| 100 | L | L | E | Q | L | E | E | T | T | 10 | |
| 131 | Q | L | S | A | A | T | S | R | I | 10 | |
| 135 | A | T | S | R | I | A | E | L | E | 10 | |
| 152 | S | Q | T | V | A | P | N | C | F | 10 | |
| 153 | Q | T | V | A | P | N | C | F | N | 10 | |
| 184 | W | L | V | Y | D | Q | Q | R | E | 10 | |
| 188 | D | Q | Q | R | E | V | Y | V | K | 10 | |
| 231 | E | E | K | Q | K | C | Y | N | D | 10 | |
| 270 | E | E | T | Q | K | E | V | H | N | 10 | |
| 276 | V | H | N | L | N | Q | L | L | Y | 10 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 281 | Q L L Y S Q R R A | 10 | |
| 293 | H L E D D R H K T | 10 | |
| 306 | K L R E E N D I A | 10 | |
| 319 | E E E K K R S E E | 10 | |
| 355 | Q M Q A C T L D F | 10 | |
| 399 | Q L H E F A I T E | 10 | |
| 401 | H E F A I T E P L | 10 | |
| 436 | S L V E C P K C N | 10 | |
| 448 | P A T E H R D L L | 10 | |
| 449 | A T E H R D L L V | 10 | |
| 1 | M S S R S T K D L | 9 | |
| 65 | V L E A E K E K N | 9 | |
| 102 | E Q L E E T T R E | 9 | |
| 148 | T L R L S Q T V A | 9 | |
| 206 | E K K T E T A A H | 9 | |
| 233 | K Q K C Y N D L L | 9 | |
| 324 | R S E E L L S Q V | 9 | |
| 364 | E N E K L D R Q H | 9 | |
| 408 | P L V T F Q G E T | 9 | |
| 411 | T F Q G E T E N R | 9 | |
| 447 | Y P A T E H R D L | 9 | |
| 28 | E K L K G E I A H | 8 | |
| 46 | S G K G K L T D K | 8 | |
| 81 | D K E I Q R L R D | 8 | |
| 89 | D Q L K A R Y S T | 8 | |
| 115 | E Q V L K A L S E | 8 | |
| 189 | Q Q R E V Y V K G | 8 | |
| 191 | R E V Y V K G L L | 8 | |
| 211 | T A A H S L P Q Q | 8 | |
| 226 | E G Y L Q E E K Q | 8 | |
| 236 | C Y N D L L A S A | 8 | |
| 259 | S F E L S E F R R | 8 | |
| 295 | E D D R H K T E K | 8 | |
| 296 | D D R H K T E K I | 8 | |
| 309 | E E N D I A R G K | 8 | |
| 313 | I A R G K L E E E | 8 | |
| 363 | F E N E K L D R Q | 8 | |
| 420 | E K V A A S P K S | 8 | |
| 435 | E S L V E C P K C | 8 | |
| 455 | L L V H V E Y C S | 8 | |
| 72 | K N A Y Q L T E K | 7 | |
| 82 | K E I Q R L R D Q | 7 | |
| 105 | E E T T R E G E R | 7 | |
| 137 | S R I A E L E S K | 7 | |
| 200 | A K I F E L E K K | 7 | |
| 223 | P E S E G Y L Q E | 7 | |
| 251 | E R Q T I T Q L S | 7 | |
| 279 | L N Q L L Y S Q R | 7 | |
| 297 | D R H K T E K I Q | 7 | |
| 323 | K R S E E L L S Q | 7 | |
| 335 | L Y T S L L K Q Q | 7 | |
| 366 | E K L D R Q H V Q | 7 | |
| 368 | L D R Q H V Q H Q | 7 | |
| 442 | K C N I Q Y P A T | 7 | |
| 4 | R S T K D L I K S | 6 | |
| 15 | G S K P S N S K S | 6 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 32 | G E I A H L K T S | 6 | |
| 39 | T S V D E I T S G | 6 | |
| 53 | D K E R H R L L E | 6 | |
| 60 | L E K I R V L E A | 6 | |
| 70 | K E K N A Y Q L T | 6 | |
| 71 | E K N A Y Q L T E | 6 | |
| 122 | S E E K D V L K Q | 6 | |
| 146 | T N T L R L S Q T | 6 | |
| 155 | V A P N C F N S S | 6 | |
| 165 | N N I H E M E I Q | 6 | |
| 180 | K N Q Q W L V Y D | 6 | |
| 193 | V Y V K G L L A K | 6 | |
| 202 | I F E L E K K T E | 6 | |
| 243 | S A K K D L E V E | 6 | |
| 245 | K K D L E V E R Q | 6 | |
| 260 | F E L S E F R R K | 6 | |
| 308 | R E E N D I A R G | 6 | |
| 333 | Q F L Y T S L L K | 6 | |
| 345 | E Q T R V A L L E | 6 | |
| 347 | T R V A L L E Q Q | 6 | |
| 433 | L N E S L V E C P | 6 | |
| 440 | C P K C N I Q Y P | 6 | |
| 452 | H R D L L V H V E | 6 | |
| 12 | S K W G S K P S N | 5 | |
| 54 | K E R H R L L E K | 5 | |
| 66 | L E A E K E K N A | 5 | |
| 68 | A E K E K N A Y Q | 5 | |
| 95 | Y S T T A L L E Q | 5 | |
| 167 | I H E M E I Q L K | 5 | |
| 181 | N Q Q W L V Y D Q | 5 | |
| 182 | Q Q W L V Y D Q Q | 5 | |
| 235 | K C Y N D L L A S | 5 | |
| 244 | A K K D L E V E R | 5 | |
| 258 | L S F E L S E F R | 5 | |
| 277 | H N L N Q L L Y S | 5 | |
| 289 | A D V Q H L E D D | 5 | |
| 329 | L S Q V Q F L Y T | 5 | |
| 330 | S Q V Q F L Y T S | 5 | |
| 356 | M Q A C T L D F E | 5 | |
| 358 | A C T L D F E N E | 5 | |
| 375 | H Q L H V I L K E | 5 | |
| 398 | K Q L H E F A I T | 5 | |
| 400 | L H E F A I T E P | 5 | |
| 428 | S P T A A L N E S | 5 | |
| 13 | K W G S K P S N S | 4 | |
| 78 | T E K D K E I Q R | 4 | |
| 121 | L S E E K D V L K | 4 | |
| 128 | L K Q Q L S A A T | 4 | |
| 140 | A E L E S K T N T | 4 | |
| 144 | S K T N T L R L S | 4 | |
| 149 | L R L S Q T V A P | 4 | |
| 158 | N C F N S S I N N | 4 | |
| 203 | F E L E K K T E T | 4 | |
| 207 | K K T E T A A H S | 4 | |
| 212 | A A H S L P Q Q T | 4 | |
| 214 | H S L P Q Q T K K | 4 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 237 | Y N D L L A S A K | 4 | |
| 242 | A S A K K D L E V | 4 | |
| 267 | R K Y E E T Q K E | 4 | |
| 318 | L E E E K K R S E | 4 | |
| 361 | L D F E N E K L D | 4 | |
| 374 | Q H Q L H V I L K | 4 | |
| 423 | A A S P K S P T A | 4 | |
| 424 | A S P K S P T A A | 4 | |
| 443 | C N I Q Y P A T E | 4 | |
| 450 | T E H R D L L V H | 4 | |
| 6 | T K D L I K S K W | 3 | |
| 10 | I K S K W G S K P | 3 | |
| 16 | S K P S N S K S E | 3 | |
| 17 | K P S N S K S E T | 3 | |
| 21 | S K S E T T L E K | 3 | |
| 47 | G K G K L T D K E | 3 | |
| 73 | N A Y Q L T E K D | 3 | |
| 80 | K D K E I Q R L R | 3 | |
| 108 | T R E G E R R E Q | 3 | |
| 109 | R E G E R R E Q V | 3 | |
| 118 | L K A L S E E K D | 3 | |
| 133 | S A A T S R I A E | 3 | |
| 168 | H E M E I Q L K D | 3 | |
| 174 | L K D A L E K N Q | 3 | |
| 205 | L E K K T E T A A | 3 | |
| 217 | P Q Q T K K P E S | 3 | |
| 218 | Q Q T K K P E S E | 3 | |
| 246 | K D L E V E R Q T | 3 | |
| 263 | S E F R R K Y E E | 3 | |
| 268 | K Y E E T Q K E V | 3 | |
| 272 | T Q K E V H N L N | 3 | |
| 288 | R A D V Q H L E D | 3 | |
| 292 | Q H L E D D R H K | 3 | |
| 301 | T E K I Q K L R E | 3 | |
| 307 | L R E E N D I A R | 3 | |
| 314 | A R G K L E E E K | 3 | |
| 316 | G K L E E E K K R | 3 | |
| 337 | T S L L K Q Q E E | 3 | |
| 341 | K Q Q E E Q T R V | 3 | |
| 342 | Q Q E E Q T R V A | 3 | |
| 365 | N E K L D R Q H V | 3 | |
| 412 | F Q G E T E N R E | 3 | |
| 414 | G E T E N R E K V | 3 | |
| 418 | N R E K V A A S P | 3 | |
| 422 | V A A S P K S P T | 3 | |
| 426 | P K S P T A A L N | 3 | |
| 438 | V E C P K C N I Q | 3 | |
| 445 | I Q Y P A T E H R | 3 | |
| 14 | W G S K P S N S K | 2 | |
| 18 | P S N S K S E T T | 2 | |
| 20 | N S K S E T T L E | 2 | |
| 27 | L E K L K G E I A | 2 | |
| 30 | L K G E I A H L K | 2 | |
| 34 | I A H L K T S V D | 2 | |
| 35 | A H L K T S V D E | 2 | |
| 48 | K G K L T D K E R | 2 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 114 | R E Q V L K A L S | 2 | |
| 125 | K D V L K Q Q L S | 2 | |
| 129 | K Q Q L S A A T S | 2 | |
| 130 | Q Q L S A A T S R | 2 | |
| 151 | L S Q T V A P N C | 2 | |
| 156 | A P N C F N S S I | 2 | |
| 164 | I N N I H E M E I | 2 | |
| 172 | I Q L K D A L E K | 2 | |
| 175 | K D A L E K N Q Q | 2 | |
| 178 | L E K N Q Q W L V | 2 | |
| 183 | Q W L V Y D Q Q R | 2 | |
| 187 | Y D Q Q R E V Y V | 2 | |
| 199 | L A K I F E L E K | 2 | |
| 225 | S E G Y L Q E E K | 2 | |
| 227 | G Y L Q E E K Q K | 2 | |
| 230 | Q E E K Q K C Y N | 2 | |
| 238 | N D L L A S A K K | 2 | |
| 248 | L E V E R Q T I T | 2 | |
| 256 | T Q L S F E L S E | 2 | |
| 266 | R R K Y E E T Q K | 2 | |
| 280 | N Q L L Y S Q R R | 2 | |
| 283 | L Y S Q R R A D V | 2 | |
| 285 | S Q R R A D V Q H | 2 | |
| 287 | R R A D V Q H L E | 2 | |
| 294 | L E D D R H K T E | 2 | |
| 298 | R H K T E K I Q K | 2 | |
| 304 | I Q K L R E E N D | 2 | |
| 311 | N D I A R G K L E | 2 | |
| 315 | R G K L E E E K K | 2 | |
| 325 | S E E L L S Q V Q | 2 | |
| 340 | L K Q Q E E Q T R | 2 | |
| 349 | V A L L E Q Q M Q | 2 | |
| 352 | L E Q Q M Q A C T | 2 | |
| 357 | Q A C T L D F E N | 2 | |
| 370 | R Q H V Q H Q L H | 2 | |
| 371 | Q H V Q H Q L H V | 2 | |
| 381 | L K E L R K A R N | 2 | |
| 382 | K E L R K A R N Q | 2 | |
| 384 | L R K A R N Q I T | 2 | |
| 387 | A R N Q I T Q L E | 2 | |
| 394 | L E S L K Q L H E | 2 | |
| 403 | F A I T E P L V T | 2 | |
| 406 | T E P L V T F Q G | 2 | |
| 416 | T E N R E K V A A | 2 | |
| 419 | R E K V A A S P K | 2 | |
| 427 | K S P T A A L N E | 2 | |
| 430 | T A A L N E S L V | 2 | |
| 446 | Q Y P A T E H R D | 2 | |
| 2 | S S R S T K D L I | 1 | |
| 3 | S R S T K D L I K | 1 | |
| 7 | K D L I K S K W G | 1 | |
| 11 | K S K W G S K P S | 1 | |
| 23 | S E T T L E K L K | 1 | |
| 31 | K G E I A H L K T | 1 | |
| 37 | L K T S V D E I T | 1 | |
| 41 | V D E I T S G K G | 1 | |

| TABLE XXVII 121P2A3 v.1: HLA Peptide Scoring Results A26 9-mers SYFPEITHI | | | |
|---|---|---|---|
| Pos | 1 2 3 4 5 6 7 8 9 | score | SEQ. ID NO. |
| 45 | T S G K G K L T D | 1 | |
| 49 | G K L T D K E R H | 1 | |
| 56 | R H R L L E K I R | 1 | |
| 57 | H R L L E K I R V | 1 | |
| 63 | I R V L E A E K E | 1 | |
| 75 | Y Q L T E K D K E | 1 | |
| 84 | I Q R L R D Q L K | 1 | |
| 85 | Q R L R D Q L K A | 1 | |
| 88 | R D Q L K A R Y S | 1 | |
| 91 | L K A R Y S T T A | 1 | |
| 98 | T A L L E Q L E E | 1 | |
| 104 | L E E T T R E G E | 1 | |
| 111 | G E R R E Q V L K | 1 | |
| 119 | K A L S E E K D V | 1 | |
| 132 | L S A A T S R I A | 1 | |
| 136 | T S R I A E L E S | 1 | |
| 142 | L E S K T N T L R | 1 | |
| 157 | P N C F N S S I N | 1 | |
| 160 | F N S S I N N I H | 1 | |
| 161 | N S S I N N I H E | 1 | |
| 209 | T E T A A H S L P | 1 | |
| 213 | A H S L P Q Q T K | 1 | |
| 216 | L P Q Q T K K P E | 1 | |
| 222 | K P E S E G Y L Q | 1 | |
| 241 | L A S A K K D L E | 1 | |
| 265 | F R R K Y E E T Q | 1 | |
| 269 | Y E E T Q K E V H | 1 | |
| 273 | Q K E V H N L N Q | 1 | |
| 291 | V Q H L E D D R H | 1 | |
| 322 | K K R S E E L L S | 1 | |
| 354 | Q Q M Q A C T L D | 1 | |
| 377 | L H V I L K E L R | 1 | |
| 385 | R K A R N Q I T Q | 1 | |
| 388 | R N Q I T Q L E S | 1 | |
| 397 | L K Q L H E F A I | 1 | |
| 413 | Q G E T E N R E K | 1 | |

| TABLE XXVII 121P2A3 v.3: HLA Peptide Scoring Results A26 9-mers SYFPEITHI | | | |
|---|---|---|---|
| Pos | 1 2 3 4 5 6 7 8 9 | score | SEQ. ID NO. |
| 3 | L T D K E R Q R L | 22 | |
| 7 | E R Q R L L E K I | 12 | |
| 2 | K L T D K E R Q R | 11 | |
| 4 | T D K E R Q R L L | 11 | |
| 5 | D K E R Q R L L E | 7 | |
| 6 | K E R Q R L L E K | 6 | |
| 1 | G K L T D K E R Q | 1 | |
| 8 | R Q R L L E K I R | 1 | |
| 9 | Q R L L E K I R V | 1 | |

| TABLE XXVII 121P2A3 v.4: HLA Peptide Scoring Results A26 9-mers SYFPEITHI | | | |
|---|---|---|---|
| Pos | 1 2 3 4 5 6 7 8 9 | score | SEQ. ID NO. |
| 6 | S T T T L L E Q L | 24 | |
| 9 | T L L E Q L E E T | 16 | |
| 7 | T T T L L E Q L E | 12 | |
| 8 | T T L L E Q L E E | 11 | |
| 2 | K A R Y S T T T L | 9 | |
| 3 | A R Y S T T T L L | 9 | |
| 5 | Y S T T T L L E Q | 5 | |
| 1 | L K A R Y S T T T | 1 | |

| TABLE XXVII 121P2A3 v.6: HLA Peptide Scoring Results A26 9-mers SYFPEITHI | | | |
|---|---|---|---|
| Pos | 1 2 3 4 5 6 7 8 9 | score | SEQ. ID NO. |
| 2 | E L L S Q V Q S L | 30 | |
| 6 | Q V Q S L Y T S L | 25 | |
| 3 | L L S Q V Q S L Y | 20 | |
| 9 | S L Y T S L L K Q | 14 | |
| 1 | E E L L S Q V Q S | 9 | |
| 7 | V Q S L Y T S L L | 9 | |
| 5 | S Q V Q S L Y T S | 6 | |
| 4 | L S Q V Q S L Y T | 1 | |

| TABLE XXVII 121P2A3 v.7: HLA Peptide Scoring Results A26 9-mers SYFPEITHI | | | |
|---|---|---|---|
| Pos | 1 2 3 4 5 6 7 8 9 | score | SEQ. ID NO. |
| 7 | Q L L V I L K E L | 21 | |
| 3 | H V Q H Q L L V I | 15 | |
| 4 | V Q H Q L L V I L | 15 | |
| 9 | L V I L K E L R K | 14 | |
| 1 | R Q H V Q H Q L L | 10 | |
| 8 | L L V I L K E L R | 9 | |
| 5 | Q H Q L L V I L K | 5 | |
| 6 | H Q L L V I L K E | 5 | |
| 2 | Q H V Q H Q L L V | 1 | |

| TABLE XXVII 121P2A3 v.8: HLA Peptide Scoring Results A26 9-mers SYFPEITHI | | | |
|---|---|---|---|
| Pos | 1 2 3 4 5 6 7 8 9 | score | SEQ. ID NO. |
| 3 | P T A A L N G S L | 21 | |
| 6 | A L N G S L V E C | 17 | |
| 7 | L N G S L V E C P | 6 | |
| 2 | S P T A A L N G S | 5 | |
| 1 | K S P T A A L N G | 2 | |
| 4 | T A A L N G S L V | 2 | |
| 9 | G S L V E C P K C | 2 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 425 | S P K S P T A A L | 26 | |
| 447 | Y P A T E H R D L | 21 | |
| 17 | K P S N S K S E T | 19 | |
| 156 | A P N C F N S S I | 18 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 92 | K A R Y S T T A L | 16 | |
| 120 | A L S E E K D V L | 15 | |
| 19 | S N S K S E T T L | 14 | |
| 29 | K L K G E I A H L | 14 | |
| 93 | A R Y S T T A L L | 14 | |
| 143 | E S K T N T L R L | 14 | |
| 286 | Q R R A D V Q H L | 14 | |
| 343 | Q E E Q T R V A L | 14 | |
| 386 | K A R N Q I T Q L | 14 | |
| 1 | M S S R S T K D L | 13 | |
| 51 | L T D K E R H R L | 13 | |
| 79 | E K D K E I Q R L | 13 | |
| 134 | A A T S R I A E L | 13 | |
| 177 | A L E K N Q Q W L | 13 | |
| 250 | V E R Q T I T Q L | 13 | |
| 271 | E T Q K E V H N L | 13 | |
| 310 | E N D I A R G K L | 13 | |
| 327 | E L L S Q V Q F L | 13 | |
| 344 | E E Q T R V A L L | 13 | |
| 401 | H E F A I T E P L | 13 | |
| 404 | A I T E P L V T F | 13 | |
| 44 | I T S G K G K L T | 12 | |
| 58 | R L L E K I R V L | 12 | |
| 69 | E K E K N A Y Q L | 12 | |
| 83 | E I Q R L R D Q L | 12 | |
| 110 | E G E R R E Q V L | 12 | |
| 112 | E R R E Q V L K A | 12 | |
| 113 | R R E Q V L K A L | 12 | |
| 124 | E K D V L K Q Q L | 12 | |
| 141 | E L E S K T N T L | 12 | |
| 222 | K P E S E G Y L Q | 12 | |
| 232 | E K Q K C Y N D L | 12 | |
| 242 | A S A K K D L E V | 12 | |
| 320 | E E K K R S E E L | 12 | |
| 373 | V Q H Q L H V I L | 12 | |
| 407 | E P L V T F Q G E | 12 | |
| 423 | A A S P K S P T A | 12 | |
| 429 | P T A A L N E S L | 12 | |
| 22 | K S E T T L E K L | 11 | |
| 43 | E I T S G K G K L | 11 | |
| 96 | S T T A L L E Q L | 11 | |
| 170 | M E I Q L K D A L | 11 | |
| 190 | Q R E V Y V K G L | 11 | |
| 191 | R E V Y V K G L L | 11 | |
| 197 | G L L A K I F E L | 11 | |
| 208 | K T E T A A H S L | 11 | |
| 216 | L P Q Q T K K P E | 11 | |
| 221 | K K P E S E G Y L | 11 | |
| 233 | K Q K C Y N D L L | 11 | |
| 240 | L L A S A K K D L | 11 | |
| 274 | K E V H N L N Q L | 11 | |
| 275 | E V H N L N Q L L | 11 | |
| 321 | E K K R S E E L L | 11 | |
| 331 | Q V Q F L Y T S L | 11 | |
| 332 | V Q F L Y T S L L | 11 | |
| 353 | E Q Q M Q A C T L | 11 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 360 | T L D F E N E K L | 11 | |
| 383 | E L R K A R N Q I | 11 | |
| 422 | V A A S P K S P T | 11 | |
| 424 | A S P K S P T A A | 11 | |
| 428 | S P T A A L N E S | 11 | |
| 448 | P A T E H R D L L | 11 | |
| 451 | E H R D L L V H V | 11 | |
| 52 | T D K E R H R L L | 10 | |
| 131 | Q L S A A T S R I | 10 | |
| 148 | T L R L S Q T V A | 10 | |
| 166 | N I H E M E I Q L | 10 | |
| 192 | E V Y V K G L L A | 10 | |
| 254 | T I T Q L S F E L | 10 | |
| 283 | L Y S Q R R A D V | 10 | |
| 299 | H K T E K I Q K L | 10 | |
| 355 | Q M Q A C T L D F | 10 | |
| 369 | D R Q H V Q H Q L | 10 | |
| 376 | Q L H V I L K E L | 10 | |
| 389 | N Q I T Q L E S L | 10 | |
| 392 | T Q L E S L K Q L | 10 | |
| 440 | C P K C N I Q Y P | 10 | |
| 449 | A T E H R D L L V | 10 | |
| 31 | K G E I A H L K T | 9 | |
| 33 | E I A H L K T S V | 9 | |
| 60 | L E K I R V L E A | 9 | |
| 109 | R E G E R R E Q V | 9 | |
| 126 | D V L K Q Q L S A | 9 | |
| 140 | A E L E S K T N T | 9 | |
| 194 | Y V K G L L A K I | 9 | |
| 204 | E L E K K T E T A | 9 | |
| 205 | L E K K T E T A A | 9 | |
| 252 | R Q T I T Q L S F | 9 | |
| 264 | E F R R K Y E E T | 9 | |
| 296 | D D R H K T E K I | 9 | |
| 306 | K L R E E N D I A | 9 | |
| 326 | E E L L S Q V Q F | 9 | |
| 329 | L S Q V Q F L Y T | 9 | |
| 398 | K Q L H E F A I T | 9 | |
| 402 | E F A I T E P L V | 9 | |
| 403 | F A I T E P L V T | 9 | |
| 416 | T E N R E K V A A | 9 | |
| 437 | L V E C P K C N I | 9 | |
| 442 | K C N I Q Y P A T | 9 | |
| 2 | S S R S T K D L I | 8 | |
| 85 | Q R L R D Q L K A | 8 | |
| 89 | D Q L K A R Y S T | 8 | |
| 91 | L K A R Y S T T A | 8 | |
| 99 | A L L E Q L E E T | 8 | |
| 128 | L K Q Q L S A A T | 8 | |
| 132 | L S A A T S R I A | 8 | |
| 138 | R I A E L E S K T | 8 | |
| 187 | Y D Q Q R E V Y V | 8 | |
| 212 | A A H S L P Q Q T | 8 | |
| 234 | Q K C Y N D L L A | 8 | |
| 257 | Q L S F E L S E F | 8 | |
| 341 | K Q Q E E Q T R V | 8 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 342 | Q Q E E Q T R V A | 8 | |
| 348 | R V A L L E Q Q M | 8 | |
| 350 | A L L E Q Q M Q A | 8 | |
| 371 | Q H V Q H Q L H V | 8 | |
| 395 | E S L K Q L H E F | 8 | |
| 415 | E T E N R E K V A | 8 | |
| 26 | T L E K L K G E I | 7 | |
| 27 | L E K L K G E I A | 7 | |
| 36 | H L K T S V D E I | 7 | |
| 55 | E R H R L L E K I | 7 | |
| 66 | L E A E K E K N A | 7 | |
| 70 | K E K N A Y Q L T | 7 | |
| 90 | Q L K A R Y S T T | 7 | |
| 119 | K A L S E E K D V | 7 | |
| 127 | V L K Q Q L S A A | 7 | |
| 164 | I N N I H E M E I | 7 | |
| 169 | E M E I Q L K D A | 7 | |
| 195 | V K G L L A K I F | 7 | |
| 201 | K I F E L E K K T | 7 | |
| 203 | F E L E K K T E T | 7 | |
| 213 | A H S L P Q Q T K | 7 | |
| 236 | C Y N D L L A S A | 7 | |
| 246 | K D L E V E R Q T | 7 | |
| 247 | D L E V E R Q T I | 7 | |
| 248 | L E V E R Q T I T | 7 | |
| 268 | K Y E E T Q K E V | 7 | |
| 293 | H L E D D R H K T | 7 | |
| 324 | R S E E L L S Q V | 7 | |
| 352 | L E Q Q M Q A C T | 7 | |
| 365 | N E K L D R Q H V | 7 | |
| 372 | H V Q H Q L H V I | 7 | |
| 379 | V I L K E L R K A | 7 | |
| 384 | L R K A R N Q I T | 7 | |
| 396 | S L K Q L H E F A | 7 | |
| 397 | L K Q L H E F A I | 7 | |
| 414 | G E T E N R E K V | 7 | |
| 430 | T A A L N E S L V | 7 | |
| 441 | P K C N I Q Y P A | 7 | |
| 14 | W G S K P S N S K | 6 | |
| 18 | P S N S K S E T T | 6 | |
| 37 | L K T S V D E I T | 6 | |
| 57 | H R L L E K I R V | 6 | |
| 76 | Q L T E K D K E I | 6 | |
| 100 | L L E Q L E E T T | 6 | |
| 146 | T N T L R L S Q T | 6 | |
| 147 | N T L R L S Q T V | 6 | |
| 150 | R L S Q T V A P N | 6 | |
| 152 | S Q T V A P N C F | 6 | |
| 159 | C F N S S I N N I | 6 | |
| 162 | S S I N N I H E M | 6 | |
| 178 | L E K N Q Q W L V | 6 | |
| 185 | L V Y D Q Q R E V | 6 | |
| 210 | E T A A H S L P Q | 6 | |
| 281 | Q L L Y S Q R R A | 6 | |
| 305 | Q K L R E E N D I | 6 | |
| 339 | L L K Q Q E E Q T | 6 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 408 | P L V T F Q G E T | 6 | |
| 417 | E N R E K V A A S | 6 | |
| 10 | I K S K W G S K P | 5 | |
| 21 | S K S E T T L E K | 5 | |
| 35 | A H L K T S V D E | 5 | |
| 54 | K E R H R L L E K | 5 | |
| 94 | R Y S T T A L L E | 5 | |
| 153 | Q T V A P N C F N | 5 | |
| 313 | I A R G K L E E E | 5 | |
| 322 | K K R S E E L L S | 5 | |
| 323 | K R S E E L L S Q | 5 | |
| 405 | I T E P L V T F Q | 5 | |
| 431 | A A L N E S L V E | 5 | |
| 3 | S R S T K D L I K | 4 | |
| 45 | T S G K G K L T D | 4 | |
| 59 | L L E K I R V L E | 4 | |
| 62 | K I R V L E A E K | 4 | |
| 71 | E K N A Y Q L T E | 4 | |
| 86 | R L R D Q L K A R | 4 | |
| 111 | G E R R E Q V L K | 4 | |
| 122 | S E E K D V L K Q | 4 | |
| 135 | A T S R I A E L E | 4 | |
| 136 | T S R I A E L E S | 4 | |
| 142 | L E S K T N T L R | 4 | |
| 145 | K T N T L R L S Q | 4 | |
| 149 | L R L S Q T V A P | 4 | |
| 172 | I Q L K D A L E K | 4 | |
| 179 | E K N Q Q W L V Y | 4 | |
| 189 | Q Q R E V Y V K G | 4 | |
| 193 | V Y V K G L L A K | 4 | |
| 206 | E K K T E T A A H | 4 | |
| 219 | Q T K K P E S E G | 4 | |
| 223 | P E S E G Y L Q E | 4 | |
| 235 | K C Y N D L L A S | 4 | |
| 244 | A K K D L E V E R | 4 | |
| 261 | E L S E F R R K Y | 4 | |
| 285 | S Q R R A D V Q H | 4 | |
| 288 | R A D V Q H L E D | 4 | |
| 314 | A R G K L E E E K | 4 | |
| 346 | Q T R V A L L E Q | 4 | |
| 367 | K L D R Q H V Q H | 4 | |
| 394 | L E S L K Q L H E | 4 | |
| 426 | P K S P T A A L N | 4 | |
| 432 | A L N E S L V E C | 4 | |
| 444 | N I Q Y P A T E H | 4 | |
| 12 | S K W G S K P S N | 3 | |
| 24 | E T T L E K L K G | 3 | |
| 28 | E K L K G E I A H | 3 | |
| 34 | I A H L K T S V D | 3 | |
| 38 | K T S V D E I T S | 3 | |
| 46 | S G K G K L T D K | 3 | |
| 47 | G K G K L T D K E | 3 | |
| 53 | D K E R H R L L E | 3 | |
| 56 | R H R L L E K I R | 3 | |
| 67 | E A E K E K N A Y | 3 | |
| 68 | A E K E K N A Y Q | 3 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 72 | K N A Y Q L T E K | 3 | |
| 81 | D K E I Q R L R D | 3 | |
| 84 | I Q R L R D Q L K | 3 | |
| 102 | E Q L E E T T R E | 3 | |
| 108 | T R E G E R R E Q | 3 | |
| 115 | E Q V L K A L S E | 3 | |
| 133 | S A A T S R I A E | 3 | |
| 168 | H E M E I Q L K D | 3 | |
| 180 | K N Q Q W L V Y D | 3 | |
| 186 | V Y D Q Q R E V Y | 3 | |
| 198 | L L A K I F E L E | 3 | |
| 224 | E S E G Y L Q E E | 3 | |
| 241 | L A S A K K D L E | 3 | |
| 245 | K K D L E V E R Q | 3 | |
| 265 | F R R K Y E E T Q | 3 | |
| 270 | E E T Q K E V H N | 3 | |
| 295 | E D D R H K T E K | 3 | |
| 303 | K I Q K L R E E N | 3 | |
| 312 | D I A R G K L E E | 3 | |
| 319 | E E E K K R S E E | 3 | |
| 345 | E Q T R V A L L E | 3 | |
| 358 | A C T L D F E N E | 3 | |
| 368 | L D R Q H V Q H Q | 3 | |
| 380 | I L K E L R K A R | 3 | |
| 387 | A R N Q I T Q L E | 3 | |
| 388 | R N Q I T Q L E S | 3 | |
| 391 | I T Q L E S L K Q | 3 | |
| 411 | T F Q G E T E N R | 3 | |
| 420 | E K V A A S P K S | 3 | |
| 421 | K V A A S P K S P | 3 | |
| 427 | K S P T A A L N E | 3 | |
| 434 | N E S L V E C P K | 3 | |
| 445 | I Q Y P A T E H R | 3 | |
| 450 | T E H R D L L V H | 3 | |
| 452 | H R D L L V H V E | 3 | |
| 453 | R D L L V H V E Y | 3 | |
| 4 | R S T K D L I K S | 2 | |
| 11 | K S K W G S K P S | 2 | |
| 13 | K W G S K P S N S | 2 | |
| 20 | N S K S E T T L E | 2 | |
| 40 | S V D E I T S G K | 2 | |
| 61 | E K I R V L E A E | 2 | |
| 74 | A Y Q L T E K D K | 2 | |
| 87 | L R D Q L K A R Y | 2 | |
| 95 | Y S T T A L L E Q | 2 | |
| 98 | T A L L E Q L E E | 2 | |
| 106 | E T T R E G E R R | 2 | |
| 107 | T T R E G E R R E | 2 | |
| 114 | R E Q V L K A L S | 2 | |
| 121 | L S E E K D V L K | 2 | |
| 129 | K Q Q L S A A T S | 2 | |
| 154 | T V A P N C F N S | 2 | |
| 160 | F N S S I N N I H | 2 | |
| 161 | N S S I N N I H E | 2 | |
| 171 | E I Q L K D A L E | 2 | |
| 174 | L K D A L E K N Q | 2 | |

| TABLE XXVIII 121P2A3 v.1: HLA Peptide Scoring Results B*0702 9-mers SYFPEITHI | | | |
|---|---|---|---|
| Pos | 1 2 3 4 5 6 7 8 9 | score | SEQ. ID NO. |
| 175 | K D A L E K N Q Q | 2 | |
| 188 | D Q Q R E V Y V K | 2 | |
| 196 | K G L L A K I F E | 2 | |
| 199 | L A K I F E L E K | 2 | |
| 200 | A K I F E L E K K | 2 | |
| 207 | K K T E T A A H S | 2 | |
| 211 | T A A H S L P Q Q | 2 | |
| 214 | H S L P Q Q T K K | 2 | |
| 237 | Y N D L L A S A K | 2 | |
| 243 | S A K K D L E V E | 2 | |
| 249 | E V E R Q T I T Q | 2 | |
| 251 | E R Q T I T Q L S | 2 | |
| 255 | I T Q L S F E L S | 2 | |
| 256 | T Q L S F E L S E | 2 | |
| 266 | R R K Y E E T Q K | 2 | |
| 267 | R K Y E E T Q K E | 2 | |
| 273 | Q K E V H N L N Q | 2 | |
| 276 | V H N L N Q L L Y | 2 | |
| 277 | H N L N Q L L Y S | 2 | |
| 282 | L L Y S Q R R A D | 2 | |
| 287 | R R A D V Q H L E | 2 | |
| 289 | A D V Q H L E D D | 2 | |
| 300 | K T E K I Q K L R | 2 | |
| 301 | T E K I Q K L R E | 2 | |
| 308 | R E E N D I A R G | 2 | |
| 311 | N D I A R G K L E | 2 | |
| 328 | L L S Q V Q F L Y | 2 | |
| 333 | Q F L Y T S L L K | 2 | |
| 334 | F L Y T S L L K Q | 2 | |
| 336 | Y T S L L K Q Q E | 2 | |
| 354 | Q Q M Q A C T L D | 2 | |
| 356 | M Q A C T L D F E | 2 | |
| 362 | D F E N E K L D R | 2 | |
| 364 | E N E K L D R Q H | 2 | |
| 366 | E K L D R Q H V Q | 2 | |
| 375 | H Q L H V I L K E | 2 | |
| 378 | H V I L K E L R K | 2 | |
| 385 | R K A R N Q I T Q | 2 | |
| 418 | N R E K V A A S P | 2 | |
| 419 | R E K V A A S P K | 2 | |
| 435 | E S L V E C P K C | 2 | |
| 439 | E C P K C N I Q Y | 2 | |
| 6 | T K D L I K S K W | 1 | |
| 7 | K D L I K S K W G | 1 | |
| 8 | D L I K S K W G S | 1 | |
| 15 | G S K P S N S K S | 1 | |
| 30 | L K G E I A H L K | 1 | |
| 32 | G E I A H L K T S | 1 | |
| 39 | T S V D E I T S G | 1 | |
| 42 | D E I T S G K G K | 1 | |
| 48 | K G K L T D K E R | 1 | |
| 50 | K L T D K E R H R | 1 | |
| 63 | I R V L E A E K E | 1 | |
| 64 | R V L E A E K E K | 1 | |
| 65 | V L E A E K E K N | 1 | |
| 73 | N A Y Q L T E K D | 1 | |

| TABLE XXVIII 121P2A3 v.1: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 77 | L | T | E | K | D | K | E | I | Q | 1 | |
| 80 | K | D | K | E | I | Q | R | L | R | 1 | |
| 82 | K | E | I | Q | R | L | R | D | Q | 1 | |
| 88 | R | D | Q | L | K | A | R | Y | S | 1 | |
| 97 | T | T | A | L | L | E | Q | L | E | 1 | |
| 101 | L | E | Q | L | E | E | T | T | R | 1 | |
| 103 | Q | L | E | E | T | T | R | E | G | 1 | |
| 104 | L | E | E | T | T | R | E | G | E | 1 | |
| 105 | E | E | T | T | R | E | G | E | R | 1 | |
| 116 | Q | V | L | K | A | L | S | E | E | 1 | |
| 117 | V | L | K | A | L | S | E | E | K | 1 | |
| 118 | L | K | A | L | S | E | E | K | D | 1 | |
| 123 | E | E | K | D | V | L | K | Q | Q | 1 | |
| 125 | K | D | V | L | K | Q | Q | L | S | 1 | |
| 130 | Q | Q | L | S | A | A | T | S | R | 1 | |
| 137 | S | R | I | A | E | L | E | S | K | 1 | |
| 139 | I | A | E | L | E | S | K | T | N | 1 | |
| 151 | L | S | Q | T | V | A | P | N | C | 1 | |
| 155 | V | A | P | N | C | F | N | S | S | 1 | |
| 167 | I | H | E | M | E | I | Q | L | K | 1 | |
| 181 | N | Q | Q | W | L | V | Y | D | Q | 1 | |
| 202 | I | F | E | L | E | K | K | T | E | 1 | |
| 209 | T | E | T | A | A | H | S | L | P | 1 | |
| 215 | S | L | P | Q | Q | T | K | K | P | 1 | |
| 217 | P | Q | Q | T | K | K | P | E | S | 1 | |
| 218 | Q | Q | T | K | K | P | E | S | E | 1 | |
| 220 | T | K | K | P | E | S | E | G | Y | 1 | |
| 225 | S | E | G | Y | L | Q | E | E | K | 1 | |
| 226 | E | G | Y | L | Q | E | E | K | Q | 1 | |
| 230 | Q | E | E | K | Q | K | C | Y | N | 1 | |
| 231 | E | E | K | Q | K | C | Y | N | D | 1 | |
| 238 | N | D | L | L | A | S | A | K | K | 1 | |
| 239 | D | L | L | A | S | A | K | K | D | 1 | |
| 253 | Q | T | I | T | Q | L | S | F | E | 1 | |
| 258 | L | S | F | E | L | S | E | F | R | 1 | |
| 260 | F | E | L | S | E | F | R | R | K | 1 | |
| 262 | L | S | E | F | R | R | K | Y | E | 1 | |
| 269 | Y | E | E | T | Q | K | E | V | H | 1 | |
| 284 | Y | S | Q | R | R | A | D | V | Q | 1 | |
| 294 | L | E | D | D | R | H | K | T | E | 1 | |
| 297 | D | R | H | K | T | E | K | I | Q | 1 | |
| 298 | R | H | K | T | E | K | I | Q | K | 1 | |
| 302 | E | K | I | Q | K | L | R | E | E | 1 | |
| 304 | I | Q | K | L | R | E | E | N | D | 1 | |
| 307 | L | R | E | E | N | D | I | A | R | 1 | |
| 309 | E | E | N | D | I | A | R | G | K | 1 | |
| 315 | R | G | K | L | E | E | E | K | K | 1 | |
| 317 | K | L | E | E | E | K | K | R | S | 1 | |
| 325 | S | E | E | L | L | S | Q | V | Q | 1 | |
| 338 | S | L | L | K | Q | Q | E | E | Q | 1 | |
| 347 | T | R | V | A | L | L | E | Q | Q | 1 | |
| 351 | L | L | E | Q | Q | M | Q | A | C | 1 | |
| 370 | R | Q | H | V | Q | H | Q | L | H | 1 | |
| 374 | Q | H | Q | L | H | V | I | L | K | 1 | |
| 381 | L | K | E | L | R | K | A | R | N | 1 | |
| 382 | K | E | L | R | K | A | R | N | Q | 1 | |

| TABLE XXVIII 121P2A3 v.1: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 400 | L | H | E | F | A | I | T | E | P | 1 | |
| 406 | T | E | P | L | V | T | F | Q | G | 1 | |
| 409 | L | V | T | F | Q | G | E | T | E | 1 | |
| 410 | V | T | F | Q | G | E | T | E | N | 1 | |
| 412 | F | Q | G | E | T | E | N | R | E | 1 | |
| 433 | L | N | E | S | L | V | E | C | P | 1 | |
| 438 | V | E | C | P | K | C | N | I | Q | 1 | |
| 443 | C | N | I | Q | Y | P | A | T | E | 1 | |
| 446 | Q | Y | P | A | T | E | H | R | D | 1 | |
| 454 | D | L | L | V | H | V | E | Y | C | 1 | |

| TABLE XXVIII 121P2A3 v.3: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 3 | L | T | D | K | E | R | Q | R | L | 13 | |
| 4 | T | D | K | E | R | Q | R | L | L | 10 | |
| 7 | E | R | Q | R | L | L | E | K | I | 7 | |
| 9 | Q | R | L | L | E | K | I | R | V | 6 | |
| 6 | K | E | R | Q | R | L | L | E | K | 5 | |
| 5 | D | K | E | R | Q | R | L | L | E | 3 | |
| 8 | R | Q | R | L | L | E | K | I | R | 3 | |
| 2 | K | L | T | D | K | E | R | Q | R | 1 | |

| TABLE XXVIII 121P2A3 v.4: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 2 | K | A | R | Y | S | T | T | T | L | 15 | |
| 3 | A | R | Y | S | T | T | T | L | L | 14 | |
| 6 | S | T | T | T | L | L | E | Q | L | 10 | |
| 1 | L | K | A | R | Y | S | T | T | T | 8 | |
| 4 | R | Y | S | T | T | T | L | L | E | 6 | |
| 9 | T | L | L | E | Q | L | E | E | T | 6 | |
| 5 | Y | S | T | T | T | L | L | E | Q | 2 | |
| 8 | T | T | L | L | E | Q | L | E | E | 2 | |

| TABLE XXVIII 121P2A3 v.6: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 7 | V | Q | S | L | Y | T | S | L | L | 13 | |
| 2 | E | L | L | S | Q | V | Q | S | L | 12 | |
| 6 | Q | V | Q | S | L | Y | T | S | L | 11 | |
| 4 | L | S | Q | V | Q | S | L | Y | T | 8 | |
| 1 | E | E | L | L | S | Q | V | Q | S | 3 | |
| 3 | L | L | S | Q | V | Q | S | L | Y | 2 | |
| 8 | Q | S | L | Y | T | S | L | L | K | 2 | |
| 9 | S | L | Y | T | S | L | L | K | Q | 2 | |

| TABLE XXVIII 121P2A3 v.7: HLA Peptide Scoring Results B*0702 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 4 | V | Q | H | Q | L | L | V | I | L | 12 | |

| TABLE XXVIII 121P2A3 v.7: HLA Peptide Scoring Results B*0702 9-mers SYFPEITHI | | | |
|---|---|---|---|
| Pos | 1 2 3 4 5 6 7 8 9 | score | SEQ. ID NO. |
| 1 | R Q H V Q H Q L L | 11 | |
| 7 | Q L L V I L K E L | 10 | |
| 3 | H V Q H Q L L V I | 9 | |
| 2 | Q H V Q H Q L L V | 8 | |
| 6 | H Q L L V I L K E | 2 | |
| 9 | L V I L K E L R K | 2 | |
| 5 | Q H Q L L V I L K | 1 | |

| TABLE XXVIII 121P2A3 v.8: HLA Peptide Scoring Results B*0702 9-mers SYFPEITHI | | | |
|---|---|---|---|
| Pos | 1 2 3 4 5 6 7 8 9 | score | SEQ. ID NO. |
| 3 | P T A A L N G S L | 12 | |
| 2 | S P T A A L N G S | 11 | |
| 4 | T A A L N G S L V | 8 | |
| 6 | A L N G S L V E C | 6 | |
| 5 | A A L N G S L V E | 5 | |
| 1 | K S P T A A L N G | 3 | |
| 8 | N G S L V E C P K | 3 | |
| 7 | L N G S L V E C P | 2 | |
| 9 | G S L V E C P K C | 1 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 320 | E E K K R S E E L | 31 | |
| 52 | T D K E R H R L L | 30 | |
| 58 | R L L E K I R V L | 29 | |
| 197 | G L L A K I F E L | 29 | |
| 425 | S P K S P T A A L | 29 | |
| 141 | E L E S K T N T L | 28 | |
| 29 | K L K G E I A H L | 27 | |
| 76 | Q L T E K D K E I | 26 | |
| 36 | H L K T S V D E I | 25 | |
| 9 | L I K S K W G S K | 24 | |
| 90 | Q L K A R Y S T T | 24 | |
| 134 | A A T S R I A E L | 24 | |
| 299 | H K T E K I Q K L | 24 | |
| 68 | A E K E K N A Y Q | 23 | |
| 46 | S G K G K L T D K | 22 | |
| 92 | K A R Y S T T A L | 22 | |
| 143 | E S K T N T L R L | 22 | |
| 231 | E E K Q K C Y N D | 22 | |
| 296 | D D R H K T E K I | 22 | |
| 313 | I A R G K L E E E | 22 | |
| 321 | E K K R S E E L L | 22 | |
| 417 | E N R E K V A A S | 22 | |
| 27 | L E K L K G E I A | 21 | |
| 233 | K Q K C Y N D L L | 21 | |
| 344 | E E Q T R V A L L | 21 | |
| 380 | I L K E L R K A R | 21 | |
| 386 | K A R N Q I T Q L | 21 | |
| 60 | L E K I R V L E A | 20 | |
| 78 | T E K D K E I Q R | 20 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 110 | E G E R R E Q V L | 20 | |
| 304 | I Q K L R E E N D | 20 | |
| 383 | E L R K A R N Q I | 20 | |
| 264 | E F R R K Y E E T | 19 | |
| 327 | E L L S Q V Q F L | 19 | |
| 376 | Q L H V I L K E L | 19 | |
| 384 | L R K A R N Q I T | 19 | |
| 117 | V L K A L S E E K | 18 | |
| 120 | A L S E E K D V L | 18 | |
| 127 | V L K Q Q L S A A | 18 | |
| 204 | E L E K K T E T A | 18 | |
| 250 | V E R Q T I T Q L | 18 | |
| 396 | S L K Q L H E F A | 18 | |
| 171 | E I Q L K D A L E | 17 | |
| 176 | D A L E K N Q Q W | 17 | |
| 240 | L L A S A K K D L | 17 | |
| 286 | Q R R A D V Q H L | 17 | |
| 360 | T L D F E N E K L | 17 | |
| 440 | C P K C N I Q Y P | 17 | |
| 34 | I A H L K T S V D | 16 | |
| 43 | E I T S G K G K L | 16 | |
| 50 | K L T D K E R H R | 16 | |
| 83 | E I Q R L R D Q L | 16 | |
| 84 | I Q R L R D Q L K | 16 | |
| 166 | N I H E M E I Q L | 16 | |
| 173 | Q L K D A L E K N | 16 | |
| 177 | A L E K N Q Q W L | 16 | |
| 194 | Y V K G L L A K I | 16 | |
| 243 | S A K K D L E V E | 16 | |
| 339 | L L K Q Q E E Q T | 16 | |
| 447 | Y P A T E H R D L | 16 | |
| 448 | P A T E H R D L L | 16 | |
| 54 | K E R H R L L E K | 15 | |
| 199 | L A K I F E L E K | 15 | |
| 254 | T I T Q L S F E L | 15 | |
| 306 | K L R E E N D I A | 15 | |
| 2 | S S R S T K D L I | 14 | |
| 5 | S T K D L I K S K | 14 | |
| 86 | R L R D Q L K A R | 14 | |
| 203 | F E L E K K T E T | 14 | |
| 241 | L A S A K K D L E | 14 | |
| 423 | A A S P K S P T A | 14 | |
| 25 | T T L E K L K G E | 13 | |
| 26 | T L E K L K G E I | 13 | |
| 48 | K G K L T D K E R | 13 | |
| 66 | L E A E K E K N A | 13 | |
| 79 | E K D K E I Q R L | 13 | |
| 115 | E Q V L K A L S E | 13 | |
| 121 | L S E E K D V L K | 13 | |
| 123 | E E K D V L K Q Q | 13 | |
| 124 | E K D V L K Q Q L | 13 | |
| 148 | T L R L S Q T V A | 13 | |
| 156 | A P N C F N S S I | 13 | |
| 190 | Q R E V Y V K G L | 13 | |
| 192 | E V Y V K G L L A | 13 | |
| 206 | E K K T E T A A H | 13 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 229 | L Q E E K Q K C Y | 13 | |
| 247 | D L E V E R Q T I | 13 | |
| 257 | Q L S F E L S E F | 13 | |
| 270 | E E T Q K E V H N | 13 | |
| 271 | E T Q K E V H N L | 13 | |
| 275 | E V H N L N Q L L | 13 | |
| 302 | E K I Q K L R E E | 13 | |
| 319 | E E E K K R S E E | 13 | |
| 363 | F E N E K L D R Q | 13 | |
| 392 | T Q L E S L K Q L | 13 | |
| 404 | A I T E P L V T F | 13 | |
| 3 | S R S T K D L I K | 12 | |
| 15 | G S K P S N S K S | 12 | |
| 19 | S N S K S E T T L | 12 | |
| 44 | I T S G K G K L T | 12 | |
| 62 | K I R V L E A E K | 12 | |
| 69 | E K E K N A Y Q L | 12 | |
| 80 | K D K E I Q R L R | 12 | |
| 96 | S T T A L L E Q L | 12 | |
| 113 | R R E Q V L K A L | 12 | |
| 131 | Q L S A A T S R I | 12 | |
| 221 | K K P E S E G Y L | 12 | |
| 232 | E K Q K C Y N D L | 12 | |
| 244 | A K K D L E V E R | 12 | |
| 272 | T Q K E V H N L N | 12 | |
| 310 | E N D I A R G K L | 12 | |
| 318 | L E E E K K R S E | 12 | |
| 332 | V Q F L Y T S L L | 12 | |
| 337 | T S L L K Q Q E E | 12 | |
| 343 | Q E E Q T R V A L | 12 | |
| 353 | E Q Q M Q A C T L | 12 | |
| 373 | V Q H Q L H V I L | 12 | |
| 378 | H V I L K E L R K | 12 | |
| 1 | M S S R S T K D L | 11 | |
| 7 | K D L I K S K W G | 11 | |
| 11 | K S K W G S K P S | 11 | |
| 88 | R D Q L K A R Y S | 11 | |
| 105 | E E T T R E G E R | 11 | |
| 112 | E R R E Q V L K A | 11 | |
| 125 | K D V L K Q Q L S | 11 | |
| 170 | M E I Q L K D A L | 11 | |
| 191 | R E V Y V K G L L | 11 | |
| 205 | L E K K T E T A A | 11 | |
| 217 | P Q Q T K K P E S | 11 | |
| 219 | Q T K K P E S E G | 11 | |
| 242 | A S A K K D L E V | 11 | |
| 263 | S E F R R K Y E E | 11 | |
| 266 | R R K Y E E T Q K | 11 | |
| 315 | R G K L E E E K K | 11 | |
| 365 | N E K L D R Q H V | 11 | |
| 394 | L E S L K Q L H E | 11 | |
| 401 | H E F A I T E P L | 11 | |
| 415 | E T E N R E K V A | 11 | |
| 419 | R E K V A A S P K | 11 | |
| 429 | P T A A L N E S L | 11 | |
| 436 | S L V E C P K C N | 11 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 438 | V E C P K C N I Q | 11 | |
| 13 | K W G S K P S N S | 10 | |
| 18 | P S N S K S E T T | 10 | |
| 20 | N S K S E T T L E | 10 | |
| 22 | K S E T T L E K L | 10 | |
| 51 | L T D K E R H R L | 10 | |
| 70 | K E K N A Y Q L T | 10 | |
| 93 | A R Y S T T A L L | 10 | |
| 109 | R E G E R R E Q V | 10 | |
| 178 | L E K N Q Q W L V | 10 | |
| 208 | K T E T A A H S L | 10 | |
| 218 | Q Q T K K P E S E | 10 | |
| 220 | T K K P E S E G Y | 10 | |
| 248 | L E V E R Q T I T | 10 | |
| 261 | E L S E F R R K Y | 10 | |
| 274 | K E V H N L N Q L | 10 | |
| 298 | R H K T E K I Q K | 10 | |
| 301 | T E K I Q K L R E | 10 | |
| 326 | E E L L S Q V Q F | 10 | |
| 331 | Q V Q F L Y T S L | 10 | |
| 366 | E K L D R Q H V Q | 10 | |
| 369 | D R Q H V Q H Q L | 10 | |
| 382 | K E L R K A R N Q | 10 | |
| 389 | N Q I T Q L E S L | 10 | |
| 432 | A L N E S L V E C | 10 | |
| 99 | A L L E Q L E E T | 9 | |
| 103 | Q L E E T T R E G | 9 | |
| 146 | T N T L R L S Q T | 9 | |
| 152 | S Q T V A P N C F | 9 | |
| 187 | Y D Q Q R E V Y V | 9 | |
| 189 | Q Q R E V Y V K G | 9 | |
| 195 | V K G L L A K I F | 9 | |
| 215 | S L P Q Q T K K P | 9 | |
| 262 | L S E F R R K Y E | 9 | |
| 285 | S Q R R A D V Q H | 9 | |
| 294 | L E D D R H K T E | 9 | |
| 311 | N D I A R G K L E | 9 | |
| 317 | K L E E E K K R S | 9 | |
| 338 | S L L K Q Q E E Q | 9 | |
| 381 | L K E L R K A R N | 9 | |
| 395 | E S L K Q L H E F | 9 | |
| 407 | E P L V T F Q G E | 9 | |
| 428 | S P T A A L N E S | 9 | |
| 451 | E H R D L L V H V | 9 | |
| 55 | E R H R L L E K I | 8 | |
| 56 | R H R L L E K I R | 8 | |
| 82 | K E I Q R L R D Q | 8 | |
| 107 | T T R E G E R R E | 8 | |
| 111 | G E R R E Q V L K | 8 | |
| 164 | I N N I H E M E I | 8 | |
| 228 | Y L Q E E K Q K C | 8 | |
| 283 | L Y S Q R R A D V | 8 | |
| 284 | Y S Q R R A D V Q | 8 | |
| 346 | Q T R V A L L E Q | 8 | |
| 350 | A L L E Q Q M Q A | 8 | |
| 397 | L K Q L H E F A I | 8 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 399 | Q L H E F A I T E | 8 | |
| 449 | A T E H R D L L V | 8 | |
| 454 | D L L V H V E Y C | 8 | |
| 17 | K P S N S K S E T | 7 | |
| 59 | L L E K I R V L E | 7 | |
| 65 | V L E A E K E K N | 7 | |
| 67 | E A E K E K N A Y | 7 | |
| 133 | S A A T S R I A E | 7 | |
| 136 | T S R I A E L E S | 7 | |
| 139 | I A E L E S K T N | 7 | |
| 150 | R L S Q T V A P N | 7 | |
| 159 | C F N S S I N N I | 7 | |
| 184 | W L V Y D Q Q R E | 7 | |
| 201 | K I F E L E K K T | 7 | |
| 216 | L P Q Q T K K P E | 7 | |
| 239 | D L L A S A K K D | 7 | |
| 265 | F R R K Y E E T Q | 7 | |
| 281 | Q L L Y S Q R R A | 7 | |
| 282 | L L Y S Q R R A D | 7 | |
| 293 | H L E D D R H K T | 7 | |
| 334 | F L Y T S L L K Q | 7 | |
| 351 | L L E Q Q M Q A C | 7 | |
| 367 | K L D R Q H V Q H | 7 | |
| 368 | L D R Q H V Q H Q | 7 | |
| 372 | H V Q H Q L H V I | 7 | |
| 408 | P L V T F Q G E T | 7 | |
| 8 | D L I K S K W G S | 6 | |
| 33 | E I A H L K T S V | 6 | |
| 98 | T A L L E Q L E E | 6 | |
| 100 | L L E Q L E E T T | 6 | |
| 138 | R I A E L E S K T | 6 | |
| 163 | S I N N I H E M E | 6 | |
| 198 | L L A K I F E L E | 6 | |
| 222 | K P E S E G Y L Q | 6 | |
| 252 | R Q T I T Q L S F | 6 | |
| 278 | N L N Q L L Y S Q | 6 | |
| 288 | R A D V Q H L E D | 6 | |
| 305 | Q K L R E E N D I | 6 | |
| 322 | K K R S E E L L S | 6 | |
| 328 | L L S Q V Q F L Y | 6 | |
| 349 | V A L L E Q Q M Q | 6 | |
| 355 | Q M Q A C T L D F | 6 | |
| 393 | Q L E S L K Q L H | 6 | |
| 437 | L V E C P K C N I | 6 | |
| 444 | N I Q Y P A T E H | 6 | |
| 455 | L L V H V E Y C S | 6 | |
| 21 | S K S E T T L E K | 5 | |
| 119 | K A L S E E K D V | 5 | |
| 155 | V A P N C F N S S | 5 | |
| 303 | K I Q K L R E E N | 5 | |
| 312 | D I A R G K L E E | 5 | |
| 357 | Q A C T L D F E N | 5 | |
| 379 | V I L K E L R K A | 5 | |
| 403 | F A I T E P L V T | 5 | |
| 422 | V A A S P K S P T | 5 | |
| 430 | T A A L N E S L V | 5 | |

| TABLE XXIX 121P2A3 v.1: HLA Peptide Scoring Results B*08 9-mers SYFPEITHI | | | |
|---|---|---|---|
| Pos | 1 2 3 4 5 6 7 8 9 | score | SEQ. ID NO. |
| 40 | S V D E I T S G K | 4 | |
| 64 | R V L E A E K E K | 4 | |
| 73 | N A Y Q L T E K D | 4 | |
| 102 | E Q L E E T T R E | 4 | |
| 162 | S S I N N I H E M | 4 | |
| 211 | T A A H S L P Q Q | 4 | |
| 212 | A A H S L P Q Q T | 4 | |
| 225 | S E G Y L Q E E K | 4 | |
| 268 | K Y E E T Q K E V | 4 | |
| 316 | G K L E E E K K R | 4 | |
| 390 | Q I T Q L E S L K | 4 | |
| 431 | A A L N E S L V E | 4 | |
| 16 | S K P S N S K S E | 3 | |
| 23 | S E T T L E K L K | 3 | |
| 24 | E T T L E K L K G | 3 | |
| 28 | E K L K G E I A H | 3 | |
| 61 | E K I R V L E A E | 3 | |
| 122 | S E E K D V L K Q | 3 | |
| 168 | H E M E I Q L K D | 3 | |
| 169 | E M E I Q L K D A | 3 | |
| 202 | I F E L E K K T E | 3 | |
| 224 | E S E G Y L Q E E | 3 | |
| 226 | E G Y L Q E E K Q | 3 | |
| 259 | S F E L S E F R R | 3 | |
| 295 | E D D R H K T E K | 3 | |
| 307 | L R E E N D I A R | 3 | |
| 325 | S E E L L S Q V Q | 3 | |
| 361 | L D F E N E K L D | 3 | |
| 412 | F Q G E T E N R E | 3 | |
| 414 | G E T E N R E K V | 3 | |
| 435 | E S L V E C P K C | 3 | |
| 439 | E C P K C N I Q Y | 3 | |
| 452 | H R D L L V H V E | 3 | |
| 453 | R D L L V H V E Y | 3 | |
| 12 | S K W G S K P S N | 2 | |
| 30 | L K G E I A H L K | 2 | |
| 32 | G E I A H L K T S | 2 | |
| 57 | H R L L E K I R V | 2 | |
| 63 | I R V L E A E K E | 2 | |
| 71 | E K N A Y Q L T E | 2 | |
| 72 | K N A Y Q L T E K | 2 | |
| 74 | A Y Q L T E K D K | 2 | |
| 106 | E T T R E G E R R | 2 | |
| 137 | S R I A E L E S K | 2 | |
| 140 | A E L E S K T N T | 2 | |
| 144 | S K T N T L R L S | 2 | |
| 149 | L R L S Q T V A P | 2 | |
| 160 | F N S S I N N I H | 2 | |
| 167 | I H E M E I Q L K | 2 | |
| 172 | I Q L K D A L E K | 2 | |
| 179 | E K N Q Q W L V Y | 2 | |
| 193 | V Y V K G L L A K | 2 | |
| 207 | K K T E T A A H S | 2 | |
| 210 | E T A A H S L P Q | 2 | |
| 223 | P E S E G Y L Q E | 2 | |
| 227 | G Y L Q E E K Q K | 2 | |

TABLE XXIX 121P2A3 v.1: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 237 | Y | N | D | L | L | A | S | A | K | 2 | |
| 245 | K | K | D | L | E | V | E | R | Q | 2 | |
| 246 | K | D | L | E | V | E | R | Q | T | 2 | |
| 249 | E | V | E | R | Q | T | I | T | Q | 2 | |
| 251 | E | R | Q | T | I | T | Q | L | S | 2 | |
| 255 | I | T | Q | L | S | F | E | L | S | 2 | |
| 258 | L | S | F | E | L | S | E | F | R | 2 | |
| 267 | R | K | Y | E | E | T | Q | K | E | 2 | |
| 273 | Q | K | E | V | H | N | L | N | Q | 2 | |
| 276 | V | H | N | L | N | Q | L | L | Y | 2 | |
| 291 | V | Q | H | L | E | D | D | R | H | 2 | |
| 292 | Q | H | L | E | D | D | R | H | K | 2 | |
| 309 | E | E | N | D | I | A | R | G | K | 2 | |
| 323 | K | R | S | E | E | L | L | S | Q | 2 | |
| 324 | R | S | E | E | L | L | S | Q | V | 2 | |
| 330 | S | Q | V | Q | F | L | Y | T | S | 2 | |
| 341 | K | Q | Q | E | E | Q | T | R | V | 2 | |
| 342 | Q | Q | E | E | Q | T | R | V | A | 2 | |
| 345 | E | Q | T | R | V | A | L | L | E | 2 | |
| 364 | E | N | E | K | L | D | R | Q | H | 2 | |
| 374 | Q | H | Q | L | H | V | I | L | K | 2 | |
| 375 | H | Q | L | H | V | I | L | K | E | 2 | |
| 391 | I | T | Q | L | E | S | L | K | Q | 2 | |
| 400 | L | H | E | F | A | I | T | E | P | 2 | |
| 402 | E | F | A | I | T | E | P | L | V | 2 | |
| 405 | I | T | E | P | L | V | T | F | Q | 2 | |
| 410 | V | T | F | Q | G | E | T | E | N | 2 | |
| 413 | Q | G | E | T | E | N | R | E | K | 2 | |
| 416 | T | E | N | R | E | K | V | A | A | 2 | |
| 420 | E | K | V | A | A | S | P | K | S | 2 | |
| 6 | T | K | D | L | I | K | S | K | W | 1 | |
| 10 | I | K | S | K | W | G | S | K | P | 1 | |
| 35 | A | H | L | K | T | S | V | D | E | 1 | |
| 38 | K | T | S | V | D | E | I | T | S | 1 | |
| 39 | T | S | V | D | E | I | T | S | G | 1 | |
| 41 | V | D | E | I | T | S | G | K | G | 1 | |
| 42 | D | E | I | T | S | G | K | G | K | 1 | |
| 47 | G | K | G | K | L | T | D | K | E | 1 | |
| 49 | G | K | L | T | D | K | E | R | H | 1 | |
| 95 | Y | S | T | T | A | L | L | E | Q | 1 | |
| 97 | T | T | A | L | L | E | Q | L | E | 1 | |
| 101 | L | E | Q | L | E | E | T | T | R | 1 | |
| 108 | T | R | E | G | E | R | R | E | Q | 1 | |
| 114 | R | E | Q | V | L | K | A | L | S | 1 | |
| 116 | Q | V | L | K | A | L | S | E | E | 1 | |
| 118 | L | K | A | L | S | E | E | K | D | 1 | |
| 128 | L | K | Q | Q | L | S | A | A | T | 1 | |
| 129 | K | Q | Q | L | S | A | A | T | S | 1 | |
| 142 | L | E | S | K | T | N | T | L | R | 1 | |
| 153 | Q | T | V | A | P | N | C | F | N | 1 | |
| 161 | N | S | S | I | N | N | I | H | E | 1 | |
| 174 | L | K | D | A | L | E | K | N | Q | 1 | |
| 175 | K | D | A | L | E | K | N | Q | Q | 1 | |
| 180 | K | N | Q | Q | W | L | V | Y | D | 1 | |
| 181 | N | Q | Q | W | L | V | Y | D | Q | 1 | |
| 182 | Q | Q | W | L | V | Y | D | Q | Q | 1 | |

TABLE XXIX 121P2A3 v.1: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 183 | Q | W | L | V | Y | D | Q | Q | R | 1 | |
| 185 | L | V | Y | D | Q | Q | R | E | V | 1 | |
| 186 | V | Y | D | Q | Q | R | E | V | Y | 1 | |
| 188 | D | Q | Q | R | E | V | Y | V | K | 1 | |
| 196 | K | G | L | L | A | K | I | F | E | 1 | |
| 213 | A | H | S | L | P | Q | Q | T | K | 1 | |
| 214 | H | S | L | P | Q | Q | T | K | K | 1 | |
| 234 | Q | K | C | Y | N | D | L | L | A | 1 | |
| 235 | K | C | Y | N | D | L | L | A | S | 1 | |
| 238 | N | D | L | L | A | S | A | K | K | 1 | |
| 260 | F | E | L | S | E | F | R | R | K | 1 | |
| 277 | H | N | L | N | Q | L | L | Y | S | 1 | |
| 279 | L | N | Q | L | L | Y | S | Q | R | 1 | |
| 280 | N | Q | L | L | Y | S | Q | R | R | 1 | |
| 297 | D | R | H | K | T | E | K | I | Q | 1 | |
| 300 | K | T | E | K | I | Q | K | L | R | 1 | |
| 308 | R | E | E | N | D | I | A | R | G | 1 | |
| 314 | A | R | G | K | L | E | E | E | K | 1 | |
| 329 | L | S | Q | V | Q | F | L | Y | T | 1 | |
| 333 | Q | F | L | Y | T | S | L | L | K | 1 | |
| 335 | L | Y | T | S | L | L | K | Q | Q | 1 | |
| 336 | Y | T | S | L | L | K | Q | Q | E | 1 | |
| 348 | R | V | A | L | L | E | Q | Q | M | 1 | |
| 358 | A | C | T | L | D | F | E | N | E | 1 | |
| 359 | C | T | L | D | F | E | N | E | K | 1 | |
| 370 | R | Q | H | V | Q | H | Q | L | H | 1 | |
| 388 | R | N | Q | I | T | Q | L | E | S | 1 | |
| 406 | T | E | P | L | V | T | F | Q | G | 1 | |
| 421 | K | V | A | A | S | P | K | S | P | 1 | |
| 424 | A | S | P | K | S | P | T | A | A | 1 | |
| 433 | L | N | E | S | L | V | E | C | P | 1 | |
| 434 | N | E | S | L | V | E | C | P | K | 1 | |
| 442 | K | C | N | I | Q | Y | P | A | T | 1 | |
| 445 | I | Q | Y | P | A | T | E | H | R | 1 | |
| 456 | L | V | H | V | E | Y | C | S | K | 1 | |

TABLE XXIX 121P2A3 v.3: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 4 | T | D | K | E | R | Q | R | L | L | 30 | |
| 2 | K | L | T | D | K | E | R | Q | R | 16 | |
| 6 | K | E | R | Q | R | L | L | E | K | 15 | |
| 3 | L | T | D | K | E | R | Q | R | L | 10 | |
| 7 | E | R | Q | R | L | L | E | K | I | 8 | |
| 8 | R | Q | R | L | L | E | K | I | R | 8 | |
| 1 | G | K | L | T | D | K | E | R | Q | 1 | |
| 9 | Q | R | L | L | E | K | I | R | V | 1 | |

TABLE XXIX 121P2A3 v.4: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 2 | K | A | R | Y | S | T | T | T | L | 21 | |
| 6 | S | T | T | T | L | L | E | Q | L | 12 | |
| 3 | A | R | Y | S | T | T | T | L | L | 10 | |

TABLE XXIX 121P2A3 v.4: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 9 | T | L | L | E | Q | L | E | E | T | 9 | |
| 8 | T | T | L | L | E | Q | L | E | E | 2 | |
| 5 | Y | S | T | T | T | L | L | E | Q | 1 | |
| 7 | T | T | T | L | L | E | Q | L | E | 1 | |

TABLE XXIX 121P2A3 v.6: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 2 | E | L | L | S | Q | V | Q | S | L | 19 | |
| 7 | V | Q | S | L | Y | T | S | L | L | 12 | |
| 6 | Q | V | Q | S | L | Y | T | S | L | 10 | |
| 9 | S | L | Y | T | S | L | L | K | Q | 8 | |
| 3 | L | L | S | Q | V | Q | S | L | Y | 6 | |
| 1 | E | E | L | L | S | Q | V | Q | S | 4 | |
| 5 | S | Q | V | Q | S | L | Y | T | S | 2 | |
| 4 | L | S | Q | V | Q | S | L | Y | T | 1 | |
| 8 | Q | S | L | Y | T | S | L | L | K | 1 | |

TABLE XXIX 121P2A3 v.7: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 7 | Q | L | L | V | I | L | K | E | L | 19 | |
| 4 | V | Q | H | Q | L | L | V | I | L | 12 | |
| 1 | R | Q | H | V | Q | H | Q | L | L | 11 | |
| 9 | L | V | I | L | K | E | L | R | K | 11 | |
| 3 | H | V | Q | H | Q | L | L | V | I | 7 | |
| 8 | L | L | V | I | L | K | E | L | R | 6 | |
| 6 | H | Q | L | L | V | I | L | K | E | 3 | |
| 5 | Q | H | Q | L | L | V | I | L | K | 2 | |

TABLE XXIX 121P2A3 v.8: HLA Peptide Scoring Results B*08 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 3 | P | T | A | A | L | N | G | S | L | 11 | |
| 2 | S | P | T | A | A | L | N | G | S | 8 | |
| 6 | A | L | N | G | S | L | V | E | C | 8 | |
| 4 | T | A | A | L | N | G | S | L | V | 5 | |
| 5 | A | A | L | N | G | S | L | V | E | 4 | |
| 9 | G | S | L | V | E | C | P | K | C | 2 | |
| 7 | L | N | G | S | L | V | E | C | P | 1 | |
| 8 | N | G | S | L | V | E | C | P | K | 1 | |

TABLE XXX 121P2A3 v.1: HLA Peptide Scoring Results B*1510 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 58 | R | L | L | E | K | I | R | V | L | 16 | |
| 343 | Q | E | E | Q | T | R | V | A | L | 16 | |
| 79 | E | K | D | K | E | I | Q | R | L | 15 | |
| 120 | A | L | S | E | E | K | D | V | L | 15 | |
| 51 | L | T | D | K | E | R | H | R | L | 14 | |
| 52 | T | D | K | E | R | H | R | L | L | 14 | |

TABLE XXX 121P2A3 v.1: HLA Peptide Scoring Results B*1510 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 69 | E | K | E | K | N | A | Y | Q | L | 14 | |
| 110 | E | G | E | R | R | E | Q | V | L | 14 | |
| 143 | E | S | K | T | N | T | L | R | L | 14 | |
| 167 | I | H | E | M | E | I | Q | L | K | 14 | |
| 447 | Y | P | A | T | E | H | R | D | L | 14 | |
| 451 | E | H | R | D | L | L | V | H | V | 14 | |
| 19 | S | N | S | K | S | E | T | T | L | 13 | |
| 35 | A | H | L | K | T | S | V | D | E | 13 | |
| 43 | E | I | T | S | G | K | G | K | L | 13 | |
| 113 | R | R | E | Q | V | L | K | A | L | 13 | |
| 124 | E | K | D | V | L | K | Q | Q | L | 13 | |
| 141 | E | L | E | S | K | T | N | T | L | 13 | |
| 170 | M | E | I | Q | L | K | D | A | L | 13 | |
| 177 | A | L | E | K | N | Q | Q | W | L | 13 | |
| 190 | Q | R | E | V | Y | V | K | G | L | 13 | |
| 197 | G | L | L | A | K | I | F | E | L | 13 | |
| 213 | A | H | S | L | P | Q | Q | T | K | 13 | |
| 254 | T | I | T | Q | L | S | F | E | L | 13 | |
| 271 | E | T | Q | K | E | V | H | N | L | 13 | |
| 292 | Q | H | L | E | D | D | R | H | K | 13 | |
| 320 | E | E | K | K | R | S | E | E | L | 13 | |
| 373 | V | Q | H | Q | L | H | V | I | L | 13 | |
| 400 | L | H | E | F | A | I | T | E | P | 13 | |
| 29 | K | L | K | G | E | I | A | H | L | 12 | |
| 83 | E | I | Q | R | L | R | D | Q | L | 12 | |
| 134 | A | A | T | S | R | I | A | E | L | 12 | |
| 232 | E | K | Q | K | C | Y | N | D | L | 12 | |
| 240 | L | L | A | S | A | K | K | D | L | 12 | |
| 250 | V | E | R | Q | T | I | T | Q | L | 12 | |
| 299 | H | K | T | E | K | I | Q | K | L | 12 | |
| 310 | E | N | D | I | A | R | G | K | L | 12 | |
| 327 | E | L | L | S | Q | V | Q | F | L | 12 | |
| 344 | E | E | Q | T | R | V | A | L | L | 12 | |
| 353 | E | Q | Q | M | Q | A | C | T | L | 12 | |
| 376 | Q | L | H | V | I | L | K | E | L | 12 | |
| 392 | T | Q | L | E | S | L | K | Q | L | 12 | |
| 425 | S | P | K | S | P | T | A | A | L | 12 | |
| 448 | P | A | T | E | H | R | D | L | L | 12 | |
| 1 | M | S | S | R | S | T | K | D | L | 11 | |
| 22 | K | S | E | T | T | L | E | K | L | 11 | |
| 92 | K | A | R | Y | S | T | T | A | L | 11 | |
| 166 | N | I | H | E | M | E | I | Q | L | 11 | |
| 191 | R | E | V | Y | V | K | G | L | L | 11 | |
| 208 | K | T | E | T | A | A | H | S | L | 11 | |
| 221 | K | K | P | E | S | E | G | Y | L | 11 | |
| 274 | K | E | V | H | N | L | N | Q | L | 11 | |
| 275 | E | V | H | N | L | N | Q | L | L | 11 | |
| 276 | V | H | N | L | N | Q | L | L | Y | 11 | |
| 286 | Q | R | R | A | D | V | Q | H | L | 11 | |
| 298 | R | H | K | T | E | K | I | Q | K | 11 | |
| 321 | E | K | K | R | S | E | E | L | L | 11 | |
| 360 | T | L | D | F | E | N | E | K | L | 11 | |
| 371 | Q | H | V | Q | H | Q | L | H | V | 11 | |
| 374 | Q | H | Q | L | H | V | I | L | K | 11 | |
| 377 | L | H | V | I | L | K | E | L | R | 11 | |
| 386 | K | A | R | N | Q | I | T | Q | L | 11 | |

| TABLE XXX 121P2A3 v.1: HLA Peptide Scoring Results B*1510 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 404 | A | I | T | E | P | L | V | T | F | 11 | |
| 429 | P | T | A | A | L | N | E | S | L | 11 | |
| 56 | R | H | R | L | L | E | K | I | R | 10 | |
| 93 | A | R | Y | S | T | T | A | L | L | 10 | |
| 96 | S | T | T | A | L | L | E | Q | L | 10 | |
| 233 | K | Q | K | C | Y | N | D | L | L | 10 | |
| 331 | Q | V | Q | F | L | Y | T | S | L | 10 | |
| 332 | V | Q | F | L | Y | T | S | L | L | 10 | |
| 369 | D | R | Q | H | V | Q | H | Q | L | 10 | |
| 389 | N | Q | I | T | Q | L | E | S | L | 10 | |
| 401 | H | E | F | A | I | T | E | P | L | 10 | |
| 162 | S | S | I | N | N | I | H | E | M | 9 | |
| 326 | E | E | L | L | S | Q | V | Q | F | 9 | |
| 395 | E | S | L | K | Q | L | H | E | F | 9 | |
| 152 | S | Q | T | V | A | P | N | C | F | 8 | |
| 257 | Q | L | S | F | E | L | S | E | F | 8 | |
| 107 | T | T | R | E | G | E | R | R | E | 7 | |
| 108 | T | R | E | G | E | R | R | E | Q | 7 | |
| 348 | R | V | A | L | L | E | Q | Q | M | 7 | |
| 195 | V | K | G | L | L | A | K | I | F | 6 | |
| 252 | R | Q | T | I | T | Q | L | S | F | 6 | |
| 261 | E | L | S | E | F | R | R | K | Y | 6 | |
| 342 | Q | Q | E | E | Q | T | R | V | A | 6 | |
| 355 | Q | M | Q | A | C | T | L | D | F | 6 | |
| 405 | I | T | E | P | L | V | T | F | Q | 6 | |
| 26 | T | L | E | K | L | K | G | E | I | 5 | |
| 45 | T | S | G | K | G | K | L | T | D | 5 | |
| 59 | L | L | E | K | I | R | V | L | E | 5 | |
| 67 | E | A | E | K | E | K | N | A | Y | 5 | |
| 103 | Q | L | E | E | T | T | R | E | G | 5 | |
| 154 | T | V | A | P | N | C | F | N | S | 5 | |
| 202 | I | F | E | L | E | K | K | T | E | 5 | |
| 269 | Y | E | E | T | Q | K | E | V | H | 5 | |
| 302 | E | K | I | Q | K | L | R | E | E | 5 | |
| 317 | K | L | E | E | E | K | K | R | S | 5 | |
| 318 | L | E | E | E | K | K | R | S | E | 5 | |
| 319 | E | E | E | K | K | R | S | E | E | 5 | |
| 364 | E | N | E | K | L | D | R | Q | H | 5 | |
| 380 | I | L | K | E | L | R | K | A | R | 5 | |
| 416 | T | E | N | R | E | K | V | A | A | 5 | |
| 423 | A | A | S | P | K | S | P | T | A | 5 | |
| 10 | I | K | S | K | W | G | S | K | P | 4 | |
| 28 | E | K | L | K | G | E | I | A | H | 4 | |
| 34 | I | A | H | L | K | T | S | V | D | 4 | |
| 44 | I | T | S | G | K | G | K | L | T | 4 | |
| 49 | G | K | L | T | D | K | E | R | H | 4 | |
| 81 | D | K | E | I | Q | R | L | R | D | 4 | |
| 87 | L | R | D | Q | L | K | A | R | Y | 4 | |
| 102 | E | Q | L | E | E | T | T | R | E | 4 | |
| 121 | L | S | E | E | K | D | V | L | K | 4 | |
| 139 | I | A | E | L | E | S | K | T | N | 4 | |
| 172 | I | Q | L | K | D | A | L | E | K | 4 | |
| 179 | E | K | N | Q | Q | W | L | V | Y | 4 | |
| 185 | L | V | Y | D | Q | Q | R | E | V | 4 | |
| 186 | V | Y | D | Q | Q | R | E | V | Y | 4 | |
| 187 | Y | D | Q | Q | R | E | V | Y | V | 4 | |

| TABLE XXX 121P2A3 v.1: HLA Peptide Scoring Results B*1510 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 204 | E | L | E | K | K | T | E | T | A | 4 | |
| 224 | E | S | E | G | Y | L | Q | E | E | 4 | |
| 244 | A | K | K | D | L | E | V | E | R | 4 | |
| 247 | D | L | E | V | E | R | Q | T | I | 4 | |
| 260 | F | E | L | S | E | F | R | R | K | 4 | |
| 270 | E | E | T | Q | K | E | V | H | N | 4 | |
| 281 | Q | L | L | Y | S | Q | R | R | A | 4 | |
| 282 | L | L | Y | S | Q | R | R | A | D | 4 | |
| 301 | T | E | K | I | Q | K | L | R | E | 4 | |
| 307 | L | R | E | E | N | D | I | A | R | 4 | |
| 308 | R | E | E | N | D | I | A | R | G | 4 | |
| 309 | E | E | N | D | I | A | R | G | K | 4 | |
| 312 | D | I | A | R | G | K | L | E | E | 4 | |
| 313 | I | A | R | G | K | L | E | E | E | 4 | |
| 351 | L | L | E | Q | Q | M | Q | A | C | 4 | |
| 366 | E | K | L | D | R | Q | H | V | Q | 4 | |
| 381 | L | K | E | L | R | K | A | R | N | 4 | |
| 413 | Q | G | E | T | E | N | R | E | K | 4 | |
| 414 | G | E | T | E | N | R | E | K | V | 4 | |
| 415 | E | T | E | N | R | E | K | V | A | 4 | |
| 417 | E | N | R | E | K | V | A | A | S | 4 | |
| 432 | A | L | N | E | S | L | V | E | C | 4 | |
| 445 | I | Q | Y | P | A | T | E | H | R | 4 | |
| 12 | S | K | W | G | S | K | P | S | N | 3 | |
| 14 | W | G | S | K | P | S | N | S | K | 3 | |
| 15 | G | S | K | P | S | N | S | K | S | 3 | |
| 17 | K | P | S | N | S | K | S | E | T | 3 | |
| 21 | S | K | S | E | T | T | L | E | K | 3 | |
| 33 | E | I | A | H | L | K | T | S | V | 3 | |
| 38 | K | T | S | V | D | E | I | T | S | 3 | |
| 39 | T | S | V | D | E | I | T | S | G | 3 | |
| 50 | K | L | T | D | K | E | R | H | R | 3 | |
| 57 | H | R | L | L | E | K | I | R | V | 3 | |
| 77 | L | T | E | K | D | K | E | I | Q | 3 | |
| 80 | K | D | K | E | I | Q | R | L | R | 3 | |
| 82 | K | E | I | Q | R | L | R | D | Q | 3 | |
| 100 | L | L | E | Q | L | E | E | T | T | 3 | |
| 106 | E | T | T | R | E | G | E | R | R | 3 | |
| 111 | G | E | R | R | E | Q | V | L | K | 3 | |
| 112 | E | R | R | E | Q | V | L | K | A | 3 | |
| 122 | S | E | E | K | D | V | L | K | Q | 3 | |
| 131 | Q | L | S | A | A | T | S | R | I | 3 | |
| 132 | L | S | A | A | T | S | R | I | A | 3 | |
| 133 | S | A | A | T | S | R | I | A | E | 3 | |
| 148 | T | L | R | L | S | Q | T | V | A | 3 | |
| 149 | L | R | L | S | Q | T | V | A | P | 3 | |
| 150 | R | L | S | Q | T | V | A | P | N | 3 | |
| 164 | I | N | N | I | H | E | M | E | I | 3 | |
| 180 | K | N | Q | Q | W | L | V | Y | D | 3 | |
| 188 | D | Q | Q | R | E | V | Y | V | K | 3 | |
| 189 | Q | Q | R | E | V | Y | V | K | G | 3 | |
| 193 | V | Y | V | K | G | L | L | A | K | 3 | |
| 203 | F | E | L | E | K | K | T | E | T | 3 | |
| 211 | T | A | A | H | S | L | P | Q | Q | 3 | |
| 217 | P | Q | Q | T | K | K | P | E | S | 3 | |
| 219 | Q | T | K | K | P | E | S | E | G | 3 | |

| TABLE XXX 121P2A3 v.1: HLA Peptide Scoring Results B*1510 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 220 | T | K | K | P | E | S | E | G | Y | 3 | |
| 223 | P | E | S | E | G | Y | L | Q | E | 3 | |
| 228 | Y | L | Q | E | E | K | Q | K | C | 3 | |
| 230 | Q | E | E | K | Q | K | C | Y | N | 3 | |
| 242 | A | S | A | K | K | D | L | E | V | 3 | |
| 243 | S | A | K | K | D | L | E | V | E | 3 | |
| 245 | K | K | D | L | E | V | E | R | Q | 3 | |
| 246 | K | D | L | E | V | E | R | Q | T | 3 | |
| 249 | E | V | E | R | Q | T | I | T | Q | 3 | |
| 259 | S | F | E | L | S | E | F | R | R | 3 | |
| 268 | K | Y | E | E | T | Q | K | E | V | 3 | |
| 283 | L | Y | S | Q | R | R | A | D | V | 3 | |
| 284 | Y | S | Q | R | R | A | D | V | Q | 3 | |
| 293 | H | L | E | D | D | R | H | K | T | 3 | |
| 295 | E | D | D | R | H | K | T | E | K | 3 | |
| 303 | K | I | Q | K | L | R | E | E | N | 3 | |
| 325 | S | E | E | L | L | S | Q | V | Q | 3 | |
| 336 | Y | T | S | L | L | K | Q | Q | E | 3 | |
| 341 | K | Q | Q | E | E | Q | T | R | V | 3 | |
| 363 | F | E | N | E | K | L | D | R | Q | 3 | |
| 379 | V | I | L | K | E | L | R | K | A | 3 | |
| 383 | E | L | R | K | A | R | N | Q | I | 3 | |
| 385 | R | K | A | R | N | Q | I | T | Q | 3 | |
| 402 | E | F | A | I | T | E | P | L | V | 3 | |
| 403 | F | A | I | T | E | P | L | V | T | 3 | |
| 410 | V | T | F | Q | G | E | T | E | N | 3 | |
| 412 | F | Q | G | E | T | E | N | R | E | 3 | |
| 422 | V | A | A | S | P | K | S | P | T | 3 | |
| 424 | A | S | P | K | S | P | T | A | A | 3 | |
| 426 | P | K | S | P | T | A | A | L | N | 3 | |
| 430 | T | A | A | L | N | E | S | L | V | 3 | |
| 435 | E | S | L | V | E | C | P | K | C | 3 | |
| 437 | L | V | E | C | P | K | C | N | I | 3 | |
| 443 | C | N | I | Q | Y | P | A | T | E | 3 | |
| 450 | T | E | H | R | D | L | L | V | H | 3 | |
| 452 | H | R | D | L | L | V | H | V | E | 3 | |
| 453 | R | D | L | L | V | H | V | E | Y | 3 | |
| 5 | S | T | K | D | L | I | K | S | K | 2 | |
| 6 | T | K | D | L | I | K | S | K | W | 2 | |
| 8 | D | L | I | K | S | K | W | G | S | 2 | |
| 24 | E | T | T | L | E | K | L | K | G | 2 | |
| 25 | T | T | L | E | K | L | K | G | E | 2 | |
| 32 | G | E | I | A | H | L | K | T | S | 2 | |
| 36 | H | L | K | T | S | V | D | E | I | 2 | |
| 47 | G | K | G | K | L | T | D | K | E | 2 | |
| 53 | D | K | E | R | H | R | L | L | E | 2 | |
| 60 | L | E | K | I | R | V | L | E | A | 2 | |
| 61 | E | K | I | R | V | L | E | A | E | 2 | |
| 62 | K | I | R | V | L | E | A | E | K | 2 | |
| 63 | I | R | V | L | E | A | E | K | E | 2 | |
| 64 | R | V | L | E | A | E | K | E | K | 2 | |
| 65 | V | L | E | A | E | K | E | K | N | 2 | |
| 66 | L | E | A | E | K | E | K | N | A | 2 | |
| 71 | E | K | N | A | Y | Q | L | T | E | 2 | |
| 72 | K | N | A | Y | Q | L | T | E | K | 2 | |
| 75 | Y | Q | L | T | E | K | D | K | E | 2 | |

| TABLE XXX 121P2A3 v.1: HLA Peptide Scoring Results B*1510 9-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | score | SEQ. ID NO. |
| 76 | Q | L | T | E | K | D | K | E | I | 2 | |
| 78 | T | E | K | D | K | E | I | Q | R | 2 | |
| 84 | I | Q | R | L | R | D | Q | L | K | 2 | |
| 86 | R | L | R | D | Q | L | K | A | R | 2 | |
| 88 | R | D | Q | L | K | A | R | Y | S | 2 | |
| 89 | D | Q | L | K | A | R | Y | S | T | 2 | |
| 91 | L | K | A | R | Y | S | T | T | A | 2 | |
| 95 | Y | S | T | T | A | L | L | E | Q | 2 | |
| 97 | T | T | A | L | L | E | Q | L | E | 2 | |
| 98 | T | A | L | L | E | Q | L | E | E | 2 | |
| 99 | A | L | L | E | Q | L | E | E | T | 2 | |
| 104 | L | E | E | T | T | R | E | G | E | 2 | |
| 105 | E | E | T | T | R | E | G | E | R | 2 | |
| 109 | R | E | G | E | R | R | E | Q | V | 2 | |
| 114 | R | E | Q | V | L | K | A | L | S | 2 | |
| 116 | Q | V | L | K | A | L | S | E | E | 2 | |
| 123 | E | E | K | D | V | L | K | Q | Q | 2 | |
| 127 | V | L | K | Q | Q | L | S | A | A | 2 | |
| 128 | L | K | Q | Q | L | S | A | A | T | 2 | |
| 136 | T | S | R | I | A | E | L | E | S | 2 | |
| 138 | R | I | A | E | L | E | S | K | T | 2 | |
| 140 | A | E | L | E | S | K | T | N | T | 2 | |
| 142 | L | E | S | K | T | N | T | L | R | 2 | |
| 144 | S | K | T | N | T | L | R | L | S | 2 | |
| 161 | N | S | S | I | N | N | I | H | E | 2 | |
| 169 | E | M | E | I | Q | L | K | D | A | 2 | |
| 175 | K | D | A | L | E | K | N | Q | Q | 2 | |
| 184 | W | L | V | Y | D | Q | Q | R | E | 2 | |
| 192 | E | V | Y | V | K | G | L | L | A | 2 | |
| 194 | Y | V | K | G | L | L | A | K | I | 2 | |
| 198 | L | L | A | K | I | F | E | L | E | 2 | |
| 205 | L | E | K | K | T | E | T | A | A | 2 | |
| 206 | E | K | K | T | E | T | A | A | H | 2 | |
| 210 | E | T | A | A | H | S | L | P | Q | 2 | |
| 214 | H | S | L | P | Q | Q | T | K | K | 2 | |
| 216 | L | P | Q | Q | T | K | K | P | E | 2 | |
| 218 | Q | Q | T | K | K | P | E | S | E | 2 | |
| 227 | G | Y | L | Q | E | E | K | Q | K | 2 | |
| 229 | L | Q | E | E | K | Q | K | C | Y | 2 | |
| 231 | E | E | K | Q | K | C | Y | N | D | 2 | |
| 237 | Y | N | D | L | L | A | S | A | K | 2 | |
| 241 | L | A | S | A | K | K | D | L | E | 2 | |
| 255 | I | T | Q | L | S | F | E | L | S | 2 | |
| 262 | L | S | E | F | R | R | K | Y | E | 2 | |
| 263 | S | E | F | R | R | K | Y | E | E | 2 | |
| 264 | E | F | R | R | K | Y | E | E | T | 2 | |
| 265 | F | R | R | K | Y | E | E | T | Q | 2 | |
| 272 | T | Q | K | E | V | H | N | L | N | 2 | |
| 273 | Q | K | E | V | H | N | L | N | Q | 2 | |
| 280 | N | Q | L | L | Y | S | Q | R | R | 2 | |
| 285 | S | Q | R | R | A | D | V | Q | H | 2 | |
| 287 | R | R | A | D | V | Q | H | L | E | 2 | |
| 288 | R | A | D | V | Q | H | L | E | D | 2 | |
| 291 | V | Q | H | L | E | D | D | R | H | 2 | |
| 294 | L | E | D | D | R | H | K | T | E | 2 | |
| 300 | K | T | E | K | I | Q | K | L | R | 2 | |

| TABLE XXX 121P2A3 v.1: HLA Peptide Scoring Results B*1510 9-mers SYFPEITHI | | | |
|---|---|---|---|
| Pos | 1 2 3 4 5 6 7 8 9 | score | SEQ. ID NO. |
| 304 | I Q K L R E E N D | 2 | |
| 314 | A R G K L E E E K | 2 | |
| 316 | G K L E E E K K R | 2 | |
| 323 | K R S E E L L S Q | 2 | |
| 324 | R S E E L L S Q V | 2 | |
| 328 | L L S Q V Q F L Y | 2 | |
| 330 | S Q V Q F L Y T S | 2 | |
| 337 | T S L L K Q Q E E | 2 | |
| 338 | S L L K Q Q E E Q | 2 | |
| 340 | L K Q Q E E Q T R | 2 | |
| 346 | Q T R V A L L E Q | 2 | |
| 359 | C T L D F E N E K | 2 | |
| 361 | L D F E N E K L D | 2 | |
| 362 | D F E N E K L D R | 2 | |
| 367 | K L D R Q H V Q H | 2 | |
| 368 | L D R Q H V Q H Q | 2 | |
| 372 | H V Q H Q L H V I | 2 | |
| 378 | H V I L K E L R K | 2 | |
| 382 | K E L R K A R N Q | 2 | |
| 391 | I T Q L E S L K Q | 2 | |
| 393 | Q L E S L K Q L H | 2 | |
| 399 | Q L H E F A I T E | 2 | |
| 407 | E P L V T F Q G E | 2 | |
| 411 | T F Q G E T E N R | 2 | |
| 418 | N R E K V A A S P | 2 | |
| 420 | E K V A A S P K S | 2 | |
| 421 | K V A A S P K S P | 2 | |
| 431 | A A L N E S L V E | 2 | |
| 433 | L N E S L V E C P | 2 | |
| 436 | S L V E C P K C N | 2 | |
| 438 | V E C P K C N I Q | 2 | |
| 439 | E C P K C N I Q Y | 2 | |
| 440 | C P K C N I Q Y P | 2 | |
| 442 | K C N I Q Y P A T | 2 | |
| 444 | N I Q Y P A T E H | 2 | |
| 446 | Q Y P A T E H R D | 2 | |
| 454 | D L L V H V E Y C | 2 | |
| 2 | S S R S T K D L I | 1 | |
| 3 | S R S T K D L I K | 1 | |
| 4 | R S T K D L I K S | 1 | |
| 11 | K S K W G S K P S | 1 | |
| 13 | K W G S K P S N S | 1 | |
| 16 | S K P S N S K S E | 1 | |
| 18 | P S N S K S E T T | 1 | |
| 30 | L K G E I A H L K | 1 | |
| 31 | K G E I A H L K T | 1 | |
| 40 | S V D E I T S G K | 1 | |
| 41 | V D E I T S G K G | 1 | |
| 46 | S G K G K L T D K | 1 | |
| 48 | K G K L T D K E R | 1 | |
| 54 | K E R H R L L E K | 1 | |
| 55 | E R H R L L E K I | 1 | |
| 68 | A E K E K N A Y Q | 1 | |
| 73 | N A Y Q L T E K D | 1 | |
| 90 | Q L K A R Y S T T | 1 | |
| 94 | R Y S T T A L L E | 1 | |

| TABLE XXX 121P2A3 v.1: HLA Peptide Scoring Results B*1510 9-mers SYFPEITHI | | | |
|---|---|---|---|
| Pos | 1 2 3 4 5 6 7 8 9 | score | SEQ. ID NO. |
| 101 | L E Q L E E T T R | 1 | |
| 115 | E Q V L K A L S E | 1 | |
| 117 | V L K A L S E E K | 1 | |
| 118 | L K A L S E E K D | 1 | |
| 126 | D V L K Q Q L S A | 1 | |
| 129 | K Q Q L S A A T S | 1 | |
| 135 | A T S R I A E L E | 1 | |
| 145 | K T N T L R L S Q | 1 | |
| 146 | T N T L R L S Q T | 1 | |
| 147 | N T L R L S Q T V | 1 | |
| 151 | L S Q T V A P N C | 1 | |
| 153 | Q T V A P N C F N | 1 | |
| 157 | P N C F N S S I N | 1 | |
| 159 | C F N S S I N N I | 1 | |
| 160 | F N S S I N N I H | 1 | |
| 165 | N N I H E M E I Q | 1 | |
| 168 | H E M E I Q L K D | 1 | |
| 171 | E I Q L K D A L E | 1 | |
| 173 | Q L K D A L E K N | 1 | |
| 176 | D A L E K N Q Q W | 1 | |
| 181 | N Q Q W L V Y D Q | 1 | |
| 183 | Q W L V Y D Q Q R | 1 | |
| 196 | K G L L A K I F E | 1 | |
| 199 | L A K I F E L E K | 1 | |
| 201 | K I F E L E K K T | 1 | |
| 207 | K K T E T A A H S | 1 | |
| 209 | T E T A A H S L P | 1 | |
| 212 | A A H S L P Q Q T | 1 | |
| 215 | S L P Q Q T K K P | 1 | |
| 222 | K P E S E G Y L Q | 1 | |
| 225 | S E G Y L Q E E K | 1 | |
| 226 | E G Y L Q E E K Q | 1 | |
| 234 | Q K C Y N D L L A | 1 | |
| 235 | K C Y N D L L A S | 1 | |
| 236 | C Y N D L L A S A | 1 | |
| 248 | L E V E R Q T I T | 1 | |
| 251 | E R Q T I T Q L S | 1 | |
| 256 | T Q L S F E L S E | 1 | |
| 258 | L S F E L S E F R | 1 | |
| 267 | R K Y E E T Q K E | 1 | |
| 278 | N L N Q L L Y S Q | 1 | |
| 289 | A D V Q H L E D D | 1 | |
| 297 | D R H K T E K I Q | 1 | |
| 306 | K L R E E N D I A | 1 | |
| 329 | L S Q V Q F L Y T | 1 | |
| 339 | L L K Q Q E E Q T | 1 | |
| 345 | E Q T R V A L L E | 1 | |
| 347 | T R V A L L E Q Q | 1 | |
| 350 | A L L E Q Q M Q A | 1 | |
| 352 | L E Q Q M Q A C T | 1 | |
| 354 | Q Q M Q A C T L D | 1 | |
| 356 | M Q A C T L D F E | 1 | |
| 357 | Q A C T L D F E N | 1 | |
| 365 | N E K L D R Q H V | 1 | |
| 370 | R Q H V Q H Q L H | 1 | |
| 375 | H Q L H V I L K E | 1 | |

| Pos | 1 2 3 4 5 6 7 8 9 | score | SEQ. ID NO. |
|---|---|---|---|
| TABLE XXX 121P2A3 v.1: HLA Peptide Scoring Results B*1510 9-mers SYFPEITHI | | | |
| 387 | A R N Q I T Q L E | 1 | |
| 388 | R N Q I T Q L E S | 1 | |
| 390 | Q I T Q L E S L K | 1 | |
| 394 | L E S L K Q L H E | 1 | |
| 396 | S L K Q L H E F A | 1 | |
| 397 | L K Q L H E F A I | 1 | |
| 406 | T E P L V T F Q G | 1 | |
| 408 | P L V T F Q G E T | 1 | |
| 409 | L V T F Q G E T E | 1 | |
| 419 | R E K V A A S P K | 1 | |
| 428 | S P T A A L N E S | 1 | |
| 434 | N E S L V E C P K | 1 | |
| 449 | A T E H R D L L V | 1 | |
| 456 | L V H V E Y C S K | 1 | |

| Pos | 1 2 3 4 5 6 7 8 9 | score | SEQ. ID NO. |
|---|---|---|---|
| TABLE XXX 121P2A3 v.3: HLA Peptide Scoring Results B*1510 9-mers SYFPEITHI | | | |
| 3 | L T D K E R Q R L | 14 | |
| 4 | T D K E R Q R L L | 14 | |
| 1 | G K L T D K E R Q | 4 | |
| 2 | K L T D K E R Q R | 3 | |
| 9 | Q R L L E K I R V | 3 | |
| 5 | D K E R Q R L L E | 2 | |
| 6 | K E R Q R L L E K | 2 | |
| 7 | E R Q R L L E K I | 1 | |

| Pos | 1 2 3 4 5 6 7 8 9 | score | SEQ. ID NO. |
|---|---|---|---|
| TABLE XXX 121P2A3 v.4: HLA Peptide Scoring Results B*1510 9-mers SYFPEITHI | | | |
| 2 | K A R Y S T T T L | 11 | |
| 3 | A R Y S T T T L L | 10 | |
| 6 | S T T T L L E Q L | 10 | |
| 9 | T L L E Q L E E T | 3 | |
| 1 | L K A R Y S T T T | 2 | |
| 5 | Y S T T T L L E Q | 2 | |
| 8 | T T L L E Q L E E | 2 | |
| 4 | R Y S T T T L L E | 1 | |
| 7 | T T T L L E Q L E | 1 | |

| Pos | 1 2 3 4 5 6 7 8 9 | score | SEQ. ID NO. |
|---|---|---|---|
| TABLE XXX 121P2A3 v.6: HLA Peptide Scoring Results B*1510 9-mers SYFPEITHI | | | |
| 2 | E L L S Q V Q S L | 12 | |
| 7 | V Q S L Y T S L L | 11 | |
| 6 | Q V Q S L Y T S L | 10 | |
| 1 | E E L L S Q V Q S | 3 | |
| 3 | L L S Q V Q S L Y | 2 | |
| 5 | S Q V Q S L Y T S | 2 | |
| 4 | L S Q V Q S L Y T | 1 | |

| Pos | 1 2 3 4 5 6 7 8 9 | score | SEQ. ID NO. |
|---|---|---|---|
| TABLE XXX 121P2A3 v.7: HLA Peptide Scoring Results B*1510 9-mers SYFPEITHI | | | |
| 4 | V Q H Q L L V I L | 13 | |
| 7 | Q L L V I L K E L | 12 | |
| 1 | R Q H V Q H Q L L | 11 | |
| 2 | Q H V Q H Q L L V | 11 | |
| 5 | Q H Q L L V I L K | 11 | |
| 3 | H V Q H Q L L V I | 2 | |
| 9 | L V I L K E L R K | 2 | |
| 6 | H Q L L V I L K E | 1 | |
| 8 | L L V I L K E L R | 1 | |

| Pos | 1 2 3 4 5 6 7 8 9 | score | SEQ. ID NO. |
|---|---|---|---|
| TABLE XXX 121P2A3 v.8: HLA Peptide Scoring Results B*1510 9-mers SYFPEITHI | | | |
| 3 | P T A A L N G S L | 11 | |
| 6 | A L N G S L V E C | 4 | |
| 4 | T A A L N G S L V | 3 | |
| 5 | A A L N G S L V E | 3 | |
| 9 | G S L V E C P K C | 3 | |
| 7 | L N G S L V E C P | 1 | |
| 8 | N G S L V E C P K | 1 | |

| Pos | 1 2 3 4 5 6 7 8 9 | score | SEQ. ID NO. |
|---|---|---|---|
| TABLE XXXI 121P2A3 v.1: HLA Peptide Scoring Results B*2705 9-mers SYFPEITHI | | | |
| 113 | R R E Q V L K A L | 28 | |
| 266 | R R K Y E E T Q K | 28 | |
| 87 | L R D Q L K A R Y | 25 | |
| 137 | S R I A E L E S K | 25 | |
| 314 | A R G K L E E E K | 25 | |
| 93 | A R Y S T T A L L | 24 | |
| 369 | D R Q H V Q H Q L | 24 | |
| 3 | S R S T K D L I K | 23 | |
| 307 | L R E E N D I A R | 23 | |
| 58 | R L L E K I R V L | 22 | |
| 190 | Q R E V Y V K G L | 21 | |
| 286 | Q R R A D V Q H L | 21 | |
| 55 | E R H R L L E K I | 20 | |
| 197 | G L L A K I F E L | 20 | |
| 29 | K L K G E I A H L | 19 | |
| 214 | H S L P Q Q T K K | 19 | |
| 227 | G Y L Q E E K Q K | 19 | |
| 316 | G K L E E E K K R | 19 | |
| 57 | H R L L E K I R V | 18 | |
| 64 | R V L E A E K E K | 18 | |
| 79 | E K D K E I Q R L | 18 | |
| 172 | I Q L K D A L E K | 18 | |
| 250 | V E R Q T I T Q L | 18 | |
| 252 | R Q T I T Q L S F | 18 | |
| 298 | R H K T E K I Q K | 18 | |
| 315 | R G K L E E E K K | 18 | |
| 326 | E E L L S Q V Q F | 18 | |
| 386 | K A R N Q I T Q L | 18 | |
| 453 | R D L L V H V E Y | 18 | |

TABLE XXXI 121P2A3 v.1: HLA Peptide Scoring Results B*2705 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 63 | I | R | V | L | E | A | E | K | E | 17 | |
| 85 | Q | R | L | R | D | Q | L | K | A | 17 | |
| 111 | G | E | R | R | E | Q | V | L | K | 17 | |
| 191 | R | E | V | Y | V | K | G | L | L | 17 | |
| 193 | V | Y | V | K | G | L | L | A | K | 17 | |
| 200 | A | K | I | F | E | L | E | K | K | 17 | |
| 238 | N | D | L | L | A | S | A | K | K | 17 | |
| 287 | R | R | A | D | V | Q | H | L | E | 17 | |
| 299 | H | K | T | E | K | I | Q | K | L | 17 | |
| 300 | K | T | E | K | I | Q | K | L | R | 17 | |
| 323 | K | R | S | E | E | L | L | S | Q | 17 | |
| 378 | H | V | I | L | K | E | L | R | K | 17 | |
| 14 | W | G | S | K | P | S | N | S | K | 16 | |
| 19 | S | N | S | K | S | E | T | T | L | 16 | |
| 46 | S | G | K | G | K | L | T | D | K | 16 | |
| 49 | G | K | L | T | D | K | E | R | H | 16 | |
| 56 | R | H | R | L | L | E | K | I | R | 16 | |
| 72 | K | N | A | Y | Q | L | T | E | K | 16 | |
| 80 | K | D | K | E | I | Q | R | L | R | 16 | |
| 130 | Q | Q | L | S | A | A | T | S | R | 16 | |
| 134 | A | A | T | S | R | I | A | E | L | 16 | |
| 170 | M | E | I | Q | L | K | D | A | L | 16 | |
| 213 | A | H | S | L | P | Q | Q | T | K | 16 | |
| 258 | L | S | F | E | L | S | E | F | R | 16 | |
| 271 | E | T | Q | K | E | V | H | N | L | 16 | |
| 274 | K | E | V | H | N | L | N | Q | L | 16 | |
| 280 | N | Q | L | L | Y | S | Q | R | R | 16 | |
| 392 | T | Q | L | E | S | L | K | Q | L | 16 | |
| 395 | E | S | L | K | Q | L | H | E | F | 16 | |
| 404 | A | I | T | E | P | L | V | T | F | 16 | |
| 418 | N | R | E | K | V | A | A | S | P | 16 | |
| 419 | R | E | K | V | A | A | S | P | K | 16 | |
| 5 | S | T | K | D | L | I | K | S | K | 15 | |
| 22 | K | S | E | T | T | L | E | K | L | 15 | |
| 28 | E | K | L | K | G | E | I | A | H | 15 | |
| 43 | E | I | T | S | G | K | G | K | L | 15 | |
| 48 | K | G | K | L | T | D | K | E | R | 15 | |
| 51 | L | T | D | K | E | R | H | R | L | 15 | |
| 54 | K | E | R | H | R | L | L | E | K | 15 | |
| 62 | K | I | R | V | L | E | A | E | K | 15 | |
| 69 | E | K | E | K | N | A | Y | Q | L | 15 | |
| 86 | R | L | R | D | Q | L | K | A | R | 15 | |
| 92 | K | A | R | Y | S | T | T | A | L | 15 | |
| 101 | L | E | Q | L | E | E | T | T | R | 15 | |
| 112 | E | R | R | E | Q | V | L | K | A | 15 | |
| 120 | A | L | S | E | E | K | D | V | L | 15 | |
| 142 | L | E | S | K | T | N | T | L | R | 15 | |
| 167 | I | H | E | M | E | I | Q | L | K | 15 | |
| 177 | A | L | E | K | N | Q | Q | W | L | 15 | |
| 254 | T | I | T | Q | L | S | F | E | L | 15 | |
| 260 | F | E | L | S | E | F | R | R | K | 15 | |
| 332 | V | Q | F | L | Y | T | S | L | L | 15 | |
| 348 | R | V | A | L | L | E | Q | Q | M | 15 | |
| 411 | T | F | Q | G | E | T | E | N | R | 15 | |
| 108 | T | R | E | G | E | R | R | E | Q | 14 | |
| 121 | L | S | E | E | K | D | V | L | K | 14 | |

TABLE XXXI 121P2A3 v.1: HLA Peptide Scoring Results B*2705 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 162 | S | S | I | N | N | I | H | E | M | 14 | |
| 183 | Q | W | L | V | Y | D | Q | Q | R | 14 | |
| 188 | D | Q | Q | R | E | V | Y | V | K | 14 | |
| 208 | K | T | E | T | A | A | H | S | L | 14 | |
| 221 | K | K | P | E | S | E | G | Y | L | 14 | |
| 225 | S | E | G | Y | L | Q | E | E | K | 14 | |
| 237 | Y | N | D | L | L | A | S | A | K | 14 | |
| 244 | A | K | K | D | L | E | V | E | R | 14 | |
| 257 | Q | L | S | F | E | L | S | E | F | 14 | |
| 259 | S | F | E | L | S | E | F | R | R | 14 | |
| 279 | L | N | Q | L | L | Y | S | Q | R | 14 | |
| 292 | Q | H | L | E | D | D | R | H | K | 14 | |
| 295 | E | D | D | R | H | K | T | E | K | 14 | |
| 320 | E | E | K | K | R | S | E | E | L | 14 | |
| 331 | Q | V | Q | F | L | Y | T | S | L | 14 | |
| 333 | Q | F | L | Y | T | S | L | L | K | 14 | |
| 340 | L | K | Q | Q | E | E | Q | T | R | 14 | |
| 347 | T | R | V | A | L | L | E | Q | Q | 14 | |
| 359 | C | T | L | D | F | E | N | E | K | 14 | |
| 360 | T | L | D | F | E | N | E | K | L | 14 | |
| 373 | V | Q | H | Q | L | H | V | I | L | 14 | |
| 387 | A | R | N | Q | I | T | Q | L | E | 14 | |
| 389 | N | Q | I | T | Q | L | E | S | L | 14 | |
| 390 | Q | I | T | Q | L | E | S | L | K | 14 | |
| 401 | H | E | F | A | I | T | E | P | L | 14 | |
| 445 | I | Q | Y | P | A | T | E | H | R | 14 | |
| 452 | H | R | D | L | L | V | H | V | E | 14 | |
| 21 | S | K | S | E | T | T | L | E | K | 13 | |
| 30 | L | K | G | E | I | A | H | L | K | 13 | |
| 40 | S | V | D | E | I | T | S | G | K | 13 | |
| 42 | D | E | I | T | S | G | K | G | K | 13 | |
| 50 | K | L | T | D | K | E | R | H | R | 13 | |
| 67 | E | A | E | K | E | K | N | A | Y | 13 | |
| 78 | T | E | K | D | K | E | I | Q | R | 13 | |
| 84 | I | Q | R | L | R | D | Q | L | K | 13 | |
| 96 | S | T | T | A | L | L | E | Q | L | 13 | |
| 106 | E | T | T | R | E | G | E | R | R | 13 | |
| 117 | V | L | K | A | L | S | E | E | K | 13 | |
| 124 | E | K | D | V | L | K | Q | Q | L | 13 | |
| 141 | E | L | E | S | K | T | N | T | L | 13 | |
| 143 | E | S | K | T | N | T | L | R | L | 13 | |
| 149 | L | R | L | S | Q | T | V | A | P | 13 | |
| 159 | C | F | N | S | S | I | N | N | I | 13 | |
| 166 | N | I | H | E | M | E | I | Q | L | 13 | |
| 194 | Y | V | K | G | L | L | A | K | I | 13 | |
| 195 | V | K | G | L | L | A | K | I | F | 13 | |
| 232 | E | K | Q | K | C | Y | N | D | L | 13 | |
| 233 | K | Q | K | C | Y | N | D | L | L | 13 | |
| 265 | F | R | R | K | Y | E | E | T | Q | 13 | |
| 291 | V | Q | H | L | E | D | D | R | H | 13 | |
| 327 | E | L | L | S | Q | V | Q | F | L | 13 | |
| 328 | L | L | S | Q | V | Q | F | L | Y | 13 | |
| 343 | Q | E | E | Q | T | R | V | A | L | 13 | |
| 362 | D | F | E | N | E | K | L | D | R | 13 | |
| 364 | E | N | E | K | L | D | R | Q | H | 13 | |
| 374 | Q | H | Q | L | H | V | I | L | K | 13 | |

| TABLE XXXI 121P2A3 v.1: HLA Peptide Scoring Results B*2705 9-mers SYFPEITHI | | | |
|---|---|---|---|
| Pos | 1 2 3 4 5 6 7 8 9 | score | SEQ. ID NO. |
| 376 | Q L H V I L K E L | 13 | |
| 377 | L H V I L K E L R | 13 | |
| 380 | I L K E L R K A R | 13 | |
| 413 | Q G E T E N R E K | 13 | |
| 429 | P T A A L N E S L | 13 | |
| 439 | E C P K C N I Q Y | 13 | |
| 444 | N I Q Y P A T E H | 13 | |
| 9 | L I K S K W G S K | 12 | |
| 74 | A Y Q L T E K D K | 12 | |
| 83 | E I Q R L R D Q L | 12 | |
| 179 | E K N Q Q W L V Y | 12 | |
| 199 | L A K I F E L E K | 12 | |
| 229 | L Q E E K Q K C Y | 12 | |
| 275 | E V H N L N Q L L | 12 | |
| 276 | V H N L N Q L L Y | 12 | |
| 297 | D R H K T E K I Q | 12 | |
| 309 | E E N D I A R G K | 12 | |
| 310 | E N D I A R G K L | 12 | |
| 344 | E E Q T R V A L L | 12 | |
| 353 | E Q Q M Q A C T L | 12 | |
| 367 | K L D R Q H V Q H | 12 | |
| 370 | R Q H V Q H Q L H | 12 | |
| 425 | S P K S P T A A L | 12 | |
| 434 | N E S L V E C P K | 12 | |
| 456 | L V H V E Y C S K | 12 | |
| 1 | M S S R S T K D L | 11 | |
| 23 | S E T T L E K L K | 11 | |
| 26 | T L E K L K G E I | 11 | |
| 52 | T D K E R H R L L | 11 | |
| 105 | E E T T R E G E R | 11 | |
| 110 | E G E R R E Q V L | 11 | |
| 152 | S Q T V A P N C F | 11 | |
| 160 | F N S S I N N I H | 11 | |
| 186 | V Y D Q Q R E V Y | 11 | |
| 220 | T K K P E S E G Y | 11 | |
| 240 | L L A S A K K D L | 11 | |
| 251 | E R Q T I T Q L S | 11 | |
| 261 | E L S E F R R K Y | 11 | |
| 285 | S Q R R A D V Q H | 11 | |
| 290 | D V Q H L E D D R | 11 | |
| 321 | E K K R S E E L L | 11 | |
| 355 | Q M Q A C T L D F | 11 | |
| 384 | L R K A R N Q I T | 11 | |
| 447 | Y P A T E H R D L | 11 | |
| 448 | P A T E H R D L L | 11 | |
| 450 | T E H R D L L V H | 11 | |
| 4 | R S T K D L I K S | 10 | |
| 76 | Q L T E K D K E I | 10 | |
| 140 | A E L E S K T N T | 10 | |
| 156 | A P N C F N S S I | 10 | |
| 206 | E K K T E T A A H | 10 | |
| 267 | R K Y E E T Q K E | 10 | |
| 269 | Y E E T Q K E V H | 10 | |
| 305 | Q K L R E E N D I | 10 | |
| 308 | R E E N D I A R G | 10 | |
| 341 | K Q Q E E Q T R V | 10 | |
| 383 | E L R K A R N Q I | 10 | |
| 393 | Q L E S L K Q L H | 10 | |
| 410 | V T F Q G E T E N | 10 | |
| 437 | L V E C P K C N I | 10 | |
| 15 | G S K P S N S K S | 9 | |
| 36 | H L K T S V D E I | 9 | |
| 131 | Q L S A A T S R I | 9 | |
| 138 | R I A E L E S K T | 9 | |
| 164 | I N N I H E M E I | 9 | |
| 201 | K I F E L E K K T | 9 | |
| 296 | D D R H K T E K I | 9 | |
| 324 | R S E E L L S Q V | 9 | |
| 375 | H Q L H V I L K E | 9 | |
| 39 | T S V D E I T S G | 8 | |
| 47 | G K G K L T D K E | 8 | |
| 98 | T A L L E Q L E E | 8 | |
| 102 | E Q L E E T T R E | 8 | |
| 126 | D V L K Q Q L S A | 8 | |
| 150 | R L S Q T V A P N | 8 | |
| 158 | N C F N S S I N N | 8 | |
| 203 | F E L E K K T E T | 8 | |
| 247 | D L E V E R Q T I | 8 | |
| 350 | A L L E Q Q M Q A | 8 | |
| 372 | H V Q H Q L H V I | 8 | |
| 379 | V I L K E L R K A | 8 | |
| 382 | K E L R K A R N Q | 8 | |
| 385 | R K A R N Q I T Q | 8 | |
| 388 | R N Q I T Q L E S | 8 | |
| 2 | S S R S T K D L I | 7 | |
| 8 | D L I K S K W G S | 7 | |
| 32 | G E I A H L K T S | 7 | |
| 35 | A H L K T S V D E | 7 | |
| 45 | T S G K G K L T D | 7 | |
| 73 | N A Y Q L T E K D | 7 | |
| 82 | K E I Q R L R D Q | 7 | |
| 88 | R D Q L K A R Y S | 7 | |
| 114 | R E Q V L K A L S | 7 | |
| 116 | Q V L K A L S E E | 7 | |
| 125 | K D V L K Q Q L S | 7 | |
| 129 | K Q Q L S A A T S | 7 | |
| 148 | T L R L S Q T V A | 7 | |
| 168 | H E M E I Q L K D | 7 | |
| 175 | K D A L E K N Q Q | 7 | |
| 196 | K G L L A K I F E | 7 | |
| 242 | A S A K K D L E V | 7 | |
| 245 | K K D L E V E R Q | 7 | |
| 246 | K D L E V E R Q T | 7 | |
| 281 | Q L L Y S Q R R A | 7 | |
| 302 | E K I Q K L R E E | 7 | |
| 317 | K L E E E K K R S | 7 | |
| 334 | F L Y T S L L K Q | 7 | |
| 337 | T S L L K Q Q E E | 7 | |
| 338 | S L L K Q Q E E Q | 7 | |
| 391 | I T Q L E S L K Q | 7 | |
| 397 | L K Q L H E F A I | 7 | |
| 431 | A A L N E S L V E | 7 | |

| TABLE XXXI 121P2A3 v.1: HLA Peptide Scoring Results B*2705 9-mers SYFPEITHI | | | |
|---|---|---|---|
| Pos | 1 2 3 4 5 6 7 8 9 | score | SEQ. ID NO. |
| 6 | T K D L I K S K W | 6 | |
| 7 | K D L I K S K W G | 6 | |
| 10 | I K S K W G S K P | 6 | |
| 12 | S K W G S K P S N | 6 | |
| 13 | K W G S K P S N S | 6 | |
| 17 | K P S N S K S E T | 6 | |
| 24 | E T T L E K L K G | 6 | |
| 75 | Y Q L T E K D K E | 6 | |
| 89 | D Q L K A R Y S T | 6 | |
| 94 | R Y S T T A L L E | 6 | |
| 99 | A L L E Q L E E T | 6 | |
| 109 | R E G E R R E Q V | 6 | |
| 115 | E Q V L K A L S E | 6 | |
| 122 | S E E K D V L K Q | 6 | |
| 176 | D A L E K N Q Q W | 6 | |
| 180 | K N Q Q W L V Y D | 6 | |
| 231 | E E K Q K C Y N D | 6 | |
| 235 | K C Y N D L L A S | 6 | |
| 239 | D L L A S A K K D | 6 | |
| 248 | L E V E R Q T I T | 6 | |
| 263 | S E F R R K Y E E | 6 | |
| 268 | K Y E E T Q K E V | 6 | |
| 278 | N L N Q L L Y S Q | 6 | |
| 288 | R A D V Q H L E D | 6 | |
| 303 | K I Q K L R E E N | 6 | |
| 313 | I A R G K L E E E | 6 | |
| 330 | S Q V Q F L Y T S | 6 | |
| 349 | V A L L E Q Q M Q | 6 | |
| 371 | Q H V Q H Q L H V | 6 | |
| 398 | K Q L H E F A I T | 6 | |
| 400 | L H E F A I T E P | 6 | |
| 405 | I T E P L V T F Q | 6 | |
| 414 | G E T E N R E K V | 6 | |
| 423 | A A S P K S P T A | 6 | |
| 424 | A S P K S P T A A | 6 | |
| 427 | K S P T A A L N E | 6 | |
| 432 | A L N E S L V E C | 6 | |
| 435 | E S L V E C P K C | 6 | |
| 443 | C N I Q Y P A T E | 6 | |
| 11 | K S K W G S K P S | 5 | |
| 25 | T T L E K L K G E | 5 | |
| 31 | K G E I A H L K T | 5 | |
| 33 | E I A H L K T S V | 5 | |
| 34 | I A H L K T S V D | 5 | |
| 68 | A E K E K N A Y Q | 5 | |
| 107 | T T R E G E R R E | 5 | |
| 119 | K A L S E E K D V | 5 | |
| 128 | L K Q Q L S A A T | 5 | |
| 139 | I A E L E S K T N | 5 | |
| 145 | K T N T L R L S Q | 5 | |
| 147 | N T L R L S Q T V | 5 | |
| 151 | L S Q T V A P N C | 5 | |
| 184 | W L V Y D Q Q R E | 5 | |
| 185 | L V Y D Q Q R E V | 5 | |
| 189 | Q Q R E V Y V K G | 5 | |
| 202 | I F E L E K K T E | 5 | |

| TABLE XXXI 121P2A3 v.1: HLA Peptide Scoring Results B*2705 9-mers SYFPEITHI | | | |
|---|---|---|---|
| Pos | 1 2 3 4 5 6 7 8 9 | score | SEQ. ID NO. |
| 207 | K K T E T A A H S | 5 | |
| 219 | Q T K K P E S E G | 5 | |
| 223 | P E S E G Y L Q E | 5 | |
| 226 | E G Y L Q E E K Q | 5 | |
| 228 | Y L Q E E K Q K C | 5 | |
| 289 | A D V Q H L E D D | 5 | |
| 306 | K L R E E N D I A | 5 | |
| 312 | D I A R G K L E E | 5 | |
| 319 | E E E K K R S E E | 5 | |
| 352 | L E Q Q M Q A C T | 5 | |
| 363 | F E N E K L D R Q | 5 | |
| 366 | E K L D R Q H V Q | 5 | |
| 381 | L K E L R K A R N | 5 | |
| 394 | L E S L K Q L H E | 5 | |
| 403 | F A I T E P L V T | 5 | |
| 420 | E K V A A S P K S | 5 | |
| 38 | K T S V D E I T S | 4 | |
| 44 | I T S G K G K L T | 4 | |
| 59 | L L E K I R V L E | 4 | |
| 61 | E K I R V L E A E | 4 | |
| 65 | V L E A E K E K N | 4 | |
| 66 | L E A E K E K N A | 4 | |
| 71 | E K N A Y Q L T E | 4 | |
| 91 | L K A R Y S T T A | 4 | |
| 100 | L L E Q L E E T T | 4 | |
| 118 | L K A L S E E K D | 4 | |
| 127 | V L K Q Q L S A A | 4 | |
| 146 | T N T L R L S Q T | 4 | |
| 171 | E I Q L K D A L E | 4 | |
| 192 | E V Y V K G L L A | 4 | |
| 204 | E L E K K T E T A | 4 | |
| 205 | L E K K T E T A A | 4 | |
| 211 | T A A H S L P Q Q | 4 | |
| 215 | S L P Q Q T K K P | 4 | |
| 217 | P Q Q T K K P E S | 4 | |
| 218 | Q Q T K K P E S E | 4 | |
| 222 | K P E S E G Y L Q | 4 | |
| 224 | E S E G Y L Q E E | 4 | |
| 236 | C Y N D L L A S A | 4 | |
| 243 | S A K K D L E V E | 4 | |
| 253 | Q T I T Q L S F E | 4 | |
| 256 | T Q L S F E L S E | 4 | |
| 270 | E E T Q K E V H N | 4 | |
| 273 | Q K E V H N L N Q | 4 | |
| 277 | H N L N Q L L Y S | 4 | |
| 301 | T E K I Q K L R E | 4 | |
| 304 | I Q K L R E E N D | 4 | |
| 318 | L E E E K K R S E | 4 | |
| 322 | K K R S E E L L S | 4 | |
| 325 | S E E L L S Q V Q | 4 | |
| 336 | Y T S L L K Q Q E | 4 | |
| 354 | Q Q M Q A C T L D | 4 | |
| 358 | A C T L D F E N E | 4 | |
| 361 | L D F E N E K L D | 4 | |
| 399 | Q L H E F A I T E | 4 | |
| 408 | P L V T F Q G E T | 4 | |

| TABLE XXXI 121P2A3 v.1: HLA Peptide Scoring Results B*2705 9-mers SYFPEITHI | | | |
|---|---|---|---|
| Pos | 1 2 3 4 5 6 7 8 9 | score | SEQ. ID NO. |
| 412 | F Q G E T E N R E | 4 | |
| 417 | E N R E K V A A S | 4 | |
| 421 | K V A A S P K S P | 4 | |
| 441 | P K C N I Q Y P A | 4 | |
| 18 | P S N S K S E T T | 3 | |
| 20 | N S K S E T T L E | 3 | |
| 41 | V D E I T S G K G | 3 | |
| 60 | L E K I R V L E A | 3 | |
| 77 | L T E K D K E I Q | 3 | |
| 81 | D K E I Q R L R D | 3 | |
| 95 | Y S T T A L L E Q | 3 | |
| 123 | E E K D V L K Q Q | 3 | |
| 135 | A T S R I A E L E | 3 | |
| 153 | Q T V A P N C F N | 3 | |
| 154 | T V A P N C F N S | 3 | |
| 165 | N N I H E M E I Q | 3 | |
| 173 | Q L K D A L E K N | 3 | |
| 174 | L K D A L E K N Q | 3 | |
| 178 | L E K N Q Q W L V | 3 | |
| 181 | N Q Q W L V Y D Q | 3 | |
| 182 | Q Q W L V Y D Q Q | 3 | |
| 187 | Y D Q Q R E V Y V | 3 | |
| 198 | L L A K I F E L E | 3 | |
| 212 | A A H S L P Q Q T | 3 | |
| 216 | L P Q Q T K K P E | 3 | |
| 230 | Q E E K Q K C Y N | 3 | |
| 255 | I T Q L S F E L S | 3 | |
| 272 | T Q K E V H N L N | 3 | |
| 283 | L Y S Q R R A D V | 3 | |
| 335 | L Y T S L L K Q Q | 3 | |
| 339 | L L K Q Q E E Q T | 3 | |
| 345 | E Q T R V A L L E | 3 | |
| 346 | Q T R V A L L E Q | 3 | |
| 357 | Q A C T L D F E N | 3 | |
| 368 | L D R Q H V Q H Q | 3 | |
| 396 | S L K Q L H E F A | 3 | |
| 406 | T E P L V T F Q G | 3 | |
| 416 | T E N R E K V A A | 3 | |
| 422 | V A A S P K S P T | 3 | |
| 426 | P K S P T A A L N | 3 | |
| 428 | S P T A A L N E S | 3 | |
| 438 | V E C P K C N I Q | 3 | |
| 440 | C P K C N I Q Y P | 3 | |
| 442 | K C N I Q Y P A T | 3 | |
| 449 | A T E H R D L L V | 3 | |
| 451 | E H R D L L V H V | 3 | |
| 454 | D L L V H V E Y C | 3 | |
| 455 | L L V H V E Y C S | 3 | |
| 16 | S K P S N S K S E | 2 | |
| 27 | L E K L K G E I A | 2 | |
| 37 | L K T S V D E I T | 2 | |
| 70 | K E K N A Y Q L T | 2 | |
| 90 | Q L K A R Y S T T | 2 | |
| 97 | T T A L L E Q L E | 2 | |
| 103 | Q L E E T T R E G | 2 | |
| 133 | S A A T S R I A E | 2 | |

| TABLE XXXI 121P2A3 v.1: HLA Peptide Scoring Results B*2705 9-mers SYFPEITHI | | | |
|---|---|---|---|
| Pos | 1 2 3 4 5 6 7 8 9 | score | SEQ. ID NO. |
| 136 | T S R I A E L E S | 2 | |
| 155 | V A P N C F N S S | 2 | |
| 157 | P N C F N S S I N | 2 | |
| 161 | N S S I N N I H E | 2 | |
| 210 | E T A A H S L P Q | 2 | |
| 234 | Q K C Y N D L L A | 2 | |
| 241 | L A S A K K D L E | 2 | |
| 249 | E V E R Q T I T Q | 2 | |
| 264 | E F R R K Y E E T | 2 | |
| 282 | L L Y S Q R R A D | 2 | |
| 284 | Y S Q R R A D V Q | 2 | |
| 293 | H L E D D R H K T | 2 | |
| 311 | N D I A R G K L E | 2 | |
| 342 | Q Q E E Q T R V A | 2 | |
| 351 | L L E Q Q M Q A C | 2 | |
| 407 | E P L V T F Q G E | 2 | |
| 430 | T A A L N E S L V | 2 | |
| 433 | L N E S L V E C P | 2 | |
| 436 | S L V E C P K C N | 2 | |
| 446 | Q Y P A T E H R D | 2 | |
| 53 | D K E R H R L L E | 1 | |
| 132 | L S A A T S R I A | 1 | |
| 144 | S K T N T L R L S | 1 | |
| 163 | S I N N I H E M E | 1 | |
| 169 | E M E I Q L K D A | 1 | |
| 209 | T E T A A H S L P | 1 | |
| 294 | L E D D R H K T E | 1 | |
| 329 | L S Q V Q F L Y T | 1 | |
| 356 | M Q A C T L D F E | 1 | |
| 365 | N E K L D R Q H V | 1 | |
| 402 | E F A I T E P L V | 1 | |
| 409 | L V T F Q G E T E | 1 | |
| 415 | E T E N R E K V A | 1 | |

| TABLE XXXI 121P2A3 v.3: HLA Peptide Scoring Results B*2705 9-mers SYFPEITHI | | | |
|---|---|---|---|
| Pos | 1 2 3 4 5 6 7 8 9 | score | SEQ. ID NO. |
| 7 | E R Q R L L E K I | 20 | |
| 9 | Q R L L E K I R V | 18 | |
| 6 | K E R Q R L L E K | 17 | |
| 8 | R Q R L L E K I R | 16 | |
| 3 | L T D K E R Q R L | 15 | |
| 2 | K L T D K E R Q R | 14 | |
| 4 | T D K E R Q R L L | 13 | |
| 1 | G K L T D K E R Q | 8 | |
| 5 | D K E R Q R L L E | 1 | |

| TABLE XXXI 121P2A3 v.4: HLA Peptide Scoring Results B*2705 9-mers SYFPEITHI | | | |
|---|---|---|---|
| Pos | 1 2 3 4 5 6 7 8 9 | score | SEQ. ID NO. |
| 3 | A R Y S T T T L L | 25 | |
| 2 | K A R Y S T T T L | 16 | |
| 6 | S T T T L L E Q L | 13 | |
| 8 | T T L L E Q L E E | 8 | |

**TABLE XXXI 121P2A3 v.4: HLA Peptide Scoring Results B*2705 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 4 | R | Y | S | T | T | T | L | L | E | 5 | |
| 9 | T | L | L | E | Q | L | E | E | T | 5 | |
| 1 | L | K | A | R | Y | S | T | T | T | 4 | |
| 5 | Y | S | T | T | T | L | L | E | Q | 3 | |
| 7 | T | T | T | L | L | E | Q | L | E | 2 | |

**TABLE XXXI 121P2A3 v.6: HLA Peptide Scoring Results B*2705 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 2 | E | L | L | S | Q | V | Q | S | L | 14 | |
| 3 | L | L | S | Q | V | Q | S | L | Y | 14 | |
| 6 | Q | V | Q | S | L | Y | T | S | L | 14 | |
| 8 | Q | S | L | Y | T | S | L | L | K | 14 | |
| 7 | V | Q | S | L | Y | T | S | L | L | 12 | |
| 1 | E | E | L | L | S | Q | V | Q | S | 8 | |
| 5 | S | Q | V | Q | S | L | Y | T | S | 7 | |
| 9 | S | L | Y | T | S | L | L | K | Q | 7 | |
| 4 | L | S | Q | V | Q | S | L | Y | T | 2 | |

**TABLE XXXI 121P2A3 v.7: HLA Peptide Scoring Results B*2705 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 9 | L | V | I | L | K | E | L | R | K | 17 | |
| 4 | V | Q | H | Q | L | L | V | I | L | 15 | |
| 7 | Q | L | L | V | I | L | K | E | L | 15 | |
| 1 | R | Q | H | V | Q | H | Q | L | L | 14 | |
| 5 | Q | H | Q | L | L | V | I | L | K | 14 | |
| 8 | L | L | V | I | L | K | E | L | R | 13 | |
| 6 | H | Q | L | L | V | I | L | K | E | 10 | |
| 3 | H | V | Q | H | Q | L | L | V | I | 9 | |
| 2 | Q | H | V | Q | H | Q | L | L | V | 6 | |

**TABLE XXXI 121P2A3 v.8: HLA Peptide Scoring Results B*2705 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 3 | P | T | A | A | L | N | G | S | L | 13 | |
| 8 | N | G | S | L | V | E | C | P | K | 12 | |
| 9 | G | S | L | V | E | C | P | K | C | 9 | |
| 5 | A | A | L | N | G | S | L | V | E | 8 | |
| 6 | A | L | N | G | S | L | V | E | C | 7 | |
| 1 | K | S | P | T | A | A | L | N | G | 6 | |
| 2 | S | P | T | A | A | L | N | G | S | 2 | |
| 4 | T | A | A | L | N | G | S | L | V | 2 | |
| 7 | L | N | G | S | L | V | E | C | P | 2 | |
| | | | | | | | | | | | |

**TABLE XXXII 121P2A3 v.1: HLA Peptide Scoring Results B*2709 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 93 | A | R | Y | S | T | T | A | L | L | 24 | |
| 113 | R | R | E | Q | V | L | K | A | L | 24 | |

**TABLE XXXII 121P2A3 v.1: HLA Peptide Scoring Results B*2709 9-mers SYFPEITHI**

| Pos | 1 | 2 | 3 | 4 | 5 | 6 | 7 | 8 | 9 | score | SEQ. ID NO. |
|---|---|---|---|---|---|---|---|---|---|---|---|
| 286 | Q | R | R | A | D | V | Q | H | L | 22 | |
| 57 | H | R | L | L | E | K | I | R | V | 21 | |
| 190 | Q | R | E | V | Y | V | K | G | L | 21 | |
| 369 | D | R | Q | H | V | Q | H | Q | L | 21 | |
| 55 | E | R | H | R | L | L | E | K | I | 18 | |
| 58 | R | L | L | E | K | I | R | V | L | 16 | |
| 191 | R | E | V | Y | V | K | G | L | L | 15 | |
| 197 | G | L | L | A | K | I | F | E | L | 15 | |
| 266 | R | R | K | Y | E | E | T | Q | K | 15 | |
| 85 | Q | R | L | R | D | Q | L | K | A | 14 | |
| 149 | L | R | L | S | Q | T | V | A | P | 14 | |
| 252 | R | Q | T | I | T | Q | L | S | F | 14 | |
| 274 | K | E | V | H | N | L | N | Q | L | 14 | |
| 287 | R | R | A | D | V | Q | H | L | E | 14 | |
| 327 | E | L | L | S | Q | V | Q | F | L | 14 | |
| 63 | I | R | V | L | E | A | E | K | E | 13 | |
| 69 | E | K | E | K | N | A | Y | Q | L | 13 | |
| 109 | R | E | G | E | R | R | E | Q | V | 13 | |
| 119 | K | A | L | S | E | E | K | D | V | 13 | |
| 134 | A | A | T | S | R | I | A | E | L | 13 | |
| 208 | K | T | E | T | A | A | H | S | L | 13 | |
| 323 | K | R | S | E | E | L | L | S | Q | 13 | |
| 324 | R | S | E | E | L | L | S | Q | V | 13 | |
| 348 | R | V | A | L | L | E | Q | Q | M | 13 | |
| 386 | K | A | R | N | Q | I | T | Q | L | 13 | |
| 392 | T | Q | L | E | S | L | K | Q | L | 13 | |
| 401 | H | E | F | A | I | T | E | P | L | 13 | |
| 22 | K | S | E | T | T | L | E | K | L | 12 | |
| 29 | K | L | K | G | E | I | A | H | L | 12 | |
| 43 | E | I | T | S | G | K | G | K | L | 12 | |
| 92 | K | A | R | Y | S | T | T | A | L | 12 | |
| 96 | S | T | T | A | L | L | E | Q | L | 12 | |
| 112 | E | R | R | E | Q | V | L | K | A | 12 | |
| 143 | E | S | K | T | N | T | L | R | L | 12 | |
| 221 | K | K | P | E | S | E | G | Y | L | 12 | |
| 233 | K | Q | K | C | Y | N | D | L | L | 12 | |
| 271 | E | T | Q | K | E | V | H | N | L | 12 | |
| 326 | E | E | L | L | S | Q | V | Q | F | 12 | |
| 332 | V | Q | F | L | Y | T | S | L | L | 12 | |
| 347 | T | R | V | A | L | L | E | Q | Q | 12 | |
| 387 | A | R | N | Q | I | T | Q | L | E | 12 | |
| 414 | G | E | T | E | N | R | E | K | V | 12 | |
| 3 | S | R | S | T | K | D | L | I | K | 11 | |
| 51 | L | T | D | K | E | R | H | R | L | 11 | |
| 79 | E | K | D | K | E | I | Q | R | L | 11 | |
| 83 | E | I | Q | R | L | R | D | Q | L | 11 | |
| 87 | L | R | D | Q | L | K | A | R | Y | 11 | |
| 120 | A | L | S | E | E | K | D | V | L | 11 | |
| 124 | E | K | D | V | L | K | Q | Q | L | 11 | |
| 137 | S | R | I | A | E | L | E | S | K | 11 | |
| 141 | E | L | E | S | K | T | N | T | L | 11 | |
| 166 | N | I | H | E | M | E | I | Q | L | 11 | |
| 170 | M | E | I | Q | L | K | D | A | L | 11 | |
| 177 | A | L | E | K | N | Q | Q | W | L | 11 | |
| 240 | L | L | A | S | A | K | K | D | L | 11 | |
| 250 | V | E | R | Q | T | I | T | Q | L | 11 | |

| TABLE XXXII 121P2A3 v.1: HLA Peptide Scoring Results B*2709 9-mers SYFPEITHI | | | |
|---|---|---|---|
| Pos | 1 2 3 4 5 6 7 8 9 | score | SEQ. ID NO. |
| 251 | E R Q T I T Q L S | 11 | |
| 254 | T I T Q L S F E L | 11 | |
| 299 | H K T E K I Q K L | 11 | |
| 314 | A R G K L E E E K | 11 | |
| 341 | K Q Q E E Q T R V | 11 | |
| 343 | Q E E Q T R V A L | 11 | |
| 344 | E E Q T R V A L L | 11 | |
| 353 | E Q Q M Q A C T L | 11 | |
| 373 | V Q H Q L H V I L | 11 | |
| 376 | Q L H V I L K E L | 11 | |
| 389 | N Q I T Q L E S L | 11 | |
| 404 | A I T E P L V T F | 11 | |
| 418 | N R E K V A A S P | 11 | |
| 425 | S P K S P T A A L | 11 | |
| 448 | P A T E H R D L L | 11 | |
| 452 | H R D L L V H V E | 11 | |
| 1 | M S S R S T K D L | 10 | |
| 19 | S N S K S E T T L | 10 | |
| 52 | T D K E R H R L L | 10 | |
| 108 | T R E G E R R E Q | 10 | |
| 110 | E G E R R E Q V L | 10 | |
| 147 | N T L R L S Q T V | 10 | |
| 159 | C F N S S I N N I | 10 | |
| 185 | L V Y D Q Q R E V | 10 | |
| 232 | E K Q K C Y N D L | 10 | |
| 242 | A S A K K D L E V | 10 | |
| 265 | F R R K Y E E T Q | 10 | |
| 268 | K Y E E T Q K E V | 10 | |
| 275 | E V H N L N Q L L | 10 | |
| 297 | D R H K T E K I Q | 10 | |
| 305 | Q K L R E E N D I | 10 | |
| 307 | L R E E N D I A R | 10 | |
| 310 | E N D I A R G K L | 10 | |
| 320 | E E K K R S E E L | 10 | |
| 321 | E K K R S E E L L | 10 | |
| 331 | Q V Q F L Y T S L | 10 | |
| 360 | T L D F E N E K L | 10 | |
| 371 | Q H V Q H Q L H V | 10 | |
| 384 | L R K A R N Q I T | 10 | |
| 395 | E S L K Q L H E F | 10 | |
| 429 | P T A A L N E S L | 10 | |
| 447 | Y P A T E H R D L | 10 | |
| 449 | A T E H R D L L V | 10 | |
| 2 | S S R S T K D L I | 9 | |
| 36 | H L K T S V D E I | 9 | |
| 76 | Q L T E K D K E I | 9 | |
| 131 | Q L S A A T S R I | 9 | |
| 152 | S Q T V A P N C F | 9 | |
| 156 | A P N C F N S S I | 9 | |
| 162 | S S I N N I H E M | 9 | |
| 164 | I N N I H E M E I | 9 | |
| 187 | Y D Q Q R E V Y V | 9 | |
| 195 | V K G L L A K I F | 9 | |
| 355 | Q M Q A C T L D F | 9 | |
| 365 | N E K L D R Q H V | 9 | |
| 383 | E L R K A R N Q I | 9 | |

| TABLE XXXII 121P2A3 v.1: HLA Peptide Scoring Results B*2709 9-mers SYFPEITHI | | | |
|---|---|---|---|
| Pos | 1 2 3 4 5 6 7 8 9 | score | SEQ. ID NO. |
| 402 | E F A I T E P L V | 9 | |
| 437 | L V E C P K C N I | 9 | |
| 451 | E H R D L L V H V | 9 | |
| 26 | T L E K L K G E I | 8 | |
| 33 | E I A H L K T S V | 8 | |
| 178 | L E K N Q Q W L V | 8 | |
| 194 | Y V K G L L A K I | 8 | |
| 247 | D L E V E R Q T I | 8 | |
| 257 | Q L S F E L S E F | 8 | |
| 283 | L Y S Q R R A D V | 8 | |
| 296 | D D R H K T E K I | 8 | |
| 372 | H V Q H Q L H V I | 8 | |
| 397 | L K Q L H E F A I | 8 | |
| 430 | T A A L N E S L V | 8 | |
| 49 | G K L T D K E R H | 7 | |
| 453 | R D L L V H V E Y | 7 | |
| 4 | R S T K D L I K S | 6 | |
| 64 | R V L E A E K E K | 6 | |
| 94 | R Y S T T A L L E | 6 | |
| 172 | I Q L K D A L E K | 6 | |
| 227 | G Y L Q E E K Q K | 6 | |
| 235 | K C Y N D L L A S | 6 | |
| 267 | R K Y E E T Q K E | 6 | |
| 308 | R E E N D I A R G | 6 | |
| 382 | K E L R K A R N Q | 6 | |
| 88 | R D Q L K A R Y S | 5 | |
| 150 | R L S Q T V A P N | 5 | |
| 246 | K D L E V E R Q T | 5 | |
| 288 | R A D V Q H L E D | 5 | |
| 298 | R H K T E K I Q K | 5 | |
| 316 | G K L E E E K K R | 5 | |
| 388 | R N Q I T Q L E S | 5 | |
| 419 | R E K V A A S P K | 5 | |
| 427 | K S P T A A L N E | 5 | |
| 7 | K D L I K S K W G | 4 | |
| 13 | K W G S K P S N S | 4 | |
| 15 | G S K P S N S K S | 4 | |
| 32 | G E I A H L K T S | 4 | |
| 35 | A H L K T S V D E | 4 | |
| 56 | R H R L L E K I R | 4 | |
| 86 | R L R D Q L K A R | 4 | |
| 111 | G E R R E Q V L K | 4 | |
| 114 | R E Q V L K A L S | 4 | |
| 130 | Q Q L S A A T S R | 4 | |
| 138 | R I A E L E S K T | 4 | |
| 140 | A E L E S K T N T | 4 | |
| 196 | K G L L A K I F E | 4 | |
| 201 | K I F E L E K K T | 4 | |
| 207 | K K T E T A A H S | 4 | |
| 222 | K P E S E G Y L Q | 4 | |
| 245 | K K D L E V E R Q | 4 | |
| 256 | T Q L S F E L S E | 4 | |
| 260 | F E L S E F R R K | 4 | |
| 315 | R G K L E E E K K | 4 | |
| 322 | K K R S E E L L S | 4 | |
| 350 | A L L E Q Q M Q A | 4 | |

TABLE XXXII 121P2A3 v.1: HLA Peptide Scoring Results B*2709 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 358 | A | C | T | L | D | F | E | N | E | 4 | |
| 367 | K | L | D | R | Q | H | V | Q | H | 4 | |
| 370 | R | Q | H | V | Q | H | Q | L | H | 4 | |
| 385 | R | K | A | R | N | Q | I | T | Q | 4 | |
| 398 | K | Q | L | H | E | F | A | I | T | 4 | |
| 431 | A | A | L | N | E | S | L | V | E | 4 | |
| 445 | I | Q | Y | P | A | T | E | H | R | 4 | |
| 31 | K | G | E | I | A | H | L | K | T | 3 | |
| 38 | K | T | S | V | D | E | I | T | S | 3 | |
| 47 | G | K | G | K | L | T | D | K | E | 3 | |
| 50 | K | L | T | D | K | E | R | H | R | 3 | |
| 54 | K | E | R | H | R | L | L | E | K | 3 | |
| 80 | K | D | K | E | I | Q | R | L | R | 3 | |
| 82 | K | E | I | Q | R | L | R | D | Q | 3 | |
| 89 | D | Q | L | K | A | R | Y | S | T | 3 | |
| 98 | T | A | L | L | E | Q | L | E | E | 3 | |
| 99 | A | L | L | E | Q | L | E | E | T | 3 | |
| 102 | E | Q | L | E | E | T | T | R | E | 3 | |
| 115 | E | Q | V | L | K | A | L | S | E | 3 | |
| 122 | S | E | E | K | D | V | L | K | Q | 3 | |
| 125 | K | D | V | L | K | Q | Q | L | S | 3 | |
| 126 | D | V | L | K | Q | Q | L | S | A | 3 | |
| 129 | K | Q | Q | L | S | A | A | T | S | 3 | |
| 135 | A | T | S | R | I | A | E | L | E | 3 | |
| 145 | K | T | N | T | L | R | L | S | Q | 3 | |
| 151 | L | S | Q | T | V | A | P | N | C | 3 | |
| 158 | N | C | F | N | S | S | I | N | N | 3 | |
| 175 | K | D | A | L | E | K | N | Q | Q | 3 | |
| 176 | D | A | L | E | K | N | Q | Q | W | 3 | |
| 180 | K | N | Q | Q | W | L | V | Y | D | 3 | |
| 183 | Q | W | L | V | Y | D | Q | Q | R | 3 | |
| 184 | W | L | V | Y | D | Q | Q | R | E | 3 | |
| 192 | E | V | Y | V | K | G | L | L | A | 3 | |
| 212 | A | A | H | S | L | P | Q | Q | T | 3 | |
| 214 | H | S | L | P | Q | Q | T | K | K | 3 | |
| 239 | D | L | L | A | S | A | K | K | D | 3 | |
| 263 | S | E | F | R | R | K | Y | E | E | 3 | |
| 277 | H | N | L | N | Q | L | L | Y | S | 3 | |
| 280 | N | Q | L | L | Y | S | Q | R | R | 3 | |
| 281 | Q | L | L | Y | S | Q | R | R | A | 3 | |
| 282 | L | L | Y | S | Q | R | R | A | D | 3 | |
| 292 | Q | H | L | E | D | D | R | H | K | 3 | |
| 300 | K | T | E | K | I | Q | K | L | R | 3 | |
| 317 | K | L | E | E | E | K | K | R | S | 3 | |
| 333 | Q | F | L | Y | T | S | L | L | K | 3 | |
| 334 | F | L | Y | T | S | L | L | K | Q | 3 | |
| 335 | L | Y | T | S | L | L | K | Q | Q | 3 | |
| 345 | E | Q | T | R | V | A | L | L | E | 3 | |
| 375 | H | Q | L | H | V | I | L | K | E | 3 | |
| 378 | H | V | I | L | K | E | L | R | K | 3 | |
| 405 | I | T | E | P | L | V | T | F | Q | 3 | |
| 423 | A | A | S | P | K | S | P | T | A | 3 | |
| 435 | E | S | L | V | E | C | P | K | C | 3 | |
| 442 | K | C | N | I | Q | Y | P | A | T | 3 | |
| 454 | D | L | L | V | H | V | E | Y | C | 3 | |
| 11 | K | S | K | W | G | S | K | P | S | 2 | |

TABLE XXXII 121P2A3 v.1: HLA Peptide Scoring Results B*2709 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 17 | K | P | S | N | S | K | S | E | T | 2 | |
| 24 | E | T | T | L | E | K | L | K | G | 2 | |
| 25 | T | T | L | E | K | L | K | G | E | 2 | |
| 28 | E | K | L | K | G | E | I | A | H | 2 | |
| 30 | L | K | G | E | I | A | H | L | K | 2 | |
| 37 | L | K | T | S | V | D | E | I | T | 2 | |
| 48 | K | G | K | L | T | D | K | E | R | 2 | |
| 59 | L | L | E | K | I | R | V | L | E | 2 | |
| 60 | L | E | K | I | R | V | L | E | A | 2 | |
| 62 | K | I | R | V | L | E | A | E | K | 2 | |
| 70 | K | E | K | N | A | Y | Q | L | T | 2 | |
| 72 | K | N | A | Y | Q | L | T | E | K | 2 | |
| 73 | N | A | Y | Q | L | T | E | K | D | 2 | |
| 75 | Y | Q | L | T | E | K | D | K | E | 2 | |
| 81 | D | K | E | I | Q | R | L | R | D | 2 | |
| 95 | Y | S | T | T | A | L | L | E | Q | 2 | |
| 106 | E | T | T | R | E | G | E | R | R | 2 | |
| 116 | Q | V | L | K | A | L | S | E | E | 2 | |
| 146 | T | N | T | L | R | L | S | Q | T | 2 | |
| 154 | T | V | A | P | N | C | F | N | S | 2 | |
| 160 | F | N | S | S | I | N | N | I | H | 2 | |
| 168 | H | E | M | E | I | Q | L | K | D | 2 | |
| 181 | N | Q | Q | W | L | V | Y | D | Q | 2 | |
| 188 | D | Q | Q | R | E | V | Y | V | K | 2 | |
| 193 | V | Y | V | K | G | L | L | A | K | 2 | |
| 200 | A | K | I | F | E | L | E | K | K | 2 | |
| 203 | F | E | L | E | K | K | T | E | T | 2 | |
| 211 | T | A | A | H | S | L | P | Q | Q | 2 | |
| 223 | P | E | S | E | G | Y | L | Q | E | 2 | |
| 226 | E | G | Y | L | Q | E | E | K | Q | 2 | |
| 231 | E | E | K | Q | K | C | Y | N | D | 2 | |
| 238 | N | D | L | L | A | S | A | K | K | 2 | |
| 244 | A | K | K | D | L | E | V | E | R | 2 | |
| 258 | L | S | F | E | L | S | E | F | R | 2 | |
| 270 | E | E | T | Q | K | E | V | H | N | 2 | |
| 273 | Q | K | E | V | H | N | L | N | Q | 2 | |
| 285 | S | Q | R | R | A | D | V | Q | H | 2 | |
| 289 | A | D | V | Q | H | L | E | D | D | 2 | |
| 301 | T | E | K | I | Q | K | L | R | E | 2 | |
| 303 | K | I | Q | K | L | R | E | E | N | 2 | |
| 304 | I | Q | K | L | R | E | E | N | D | 2 | |
| 306 | K | L | R | E | E | N | D | I | A | 2 | |
| 309 | E | E | N | D | I | A | R | G | K | 2 | |
| 330 | S | Q | V | Q | F | L | Y | T | S | 2 | |
| 337 | T | S | L | L | K | Q | Q | E | E | 2 | |
| 338 | S | L | L | K | Q | Q | E | E | Q | 2 | |
| 349 | V | A | L | L | E | Q | Q | M | Q | 2 | |
| 359 | C | T | L | D | F | E | N | E | K | 2 | |
| 361 | L | D | F | E | N | E | K | L | D | 2 | |
| 366 | E | K | L | D | R | Q | H | V | Q | 2 | |
| 379 | V | I | L | K | E | L | R | K | A | 2 | |
| 391 | I | T | Q | L | E | S | L | K | Q | 2 | |
| 403 | F | A | I | T | E | P | L | V | T | 2 | |
| 407 | E | P | L | V | T | F | Q | G | E | 2 | |
| 410 | V | T | F | Q | G | E | T | E | N | 2 | |
| 420 | E | K | V | A | A | S | P | K | S | 2 | |

| TABLE XXXII 121P2A3 v.1: HLA Peptide Scoring Results B*2709 9-mers SYFPEITHI | | | |
|---|---|---|---|
| Pos | 1 2 3 4 5 6 7 8 9 | score | SEQ. ID NO. |
| 421 | K V A A S P K S P | 2 | |
| 426 | P K S P T A A L N | 2 | |
| 432 | A L N E S L V E C | 2 | |
| 433 | L N E S L V E C P | 2 | |
| 441 | P K C N I Q Y P A | 2 | |
| 450 | T E H R D L L V H | 2 | |
| 455 | L L V H V E Y C S | 2 | |
| 6 | T K D L I K S K W | 1 | |
| 8 | D L I K S K W G S | 1 | |
| 9 | L I K S K W G S K | 1 | |
| 10 | I K S K W G S K P | 1 | |
| 12 | S K W G S K P S N | 1 | |
| 16 | S K P S N S K S E | 1 | |
| 18 | P S N S K S E T T | 1 | |
| 20 | N S K S E T T L E | 1 | |
| 21 | S K S E T T L E K | 1 | |
| 23 | S E T T L E K L K | 1 | |
| 34 | I A H L K T S V D | 1 | |
| 39 | T S V D E I T S G | 1 | |
| 40 | S V D E I T S G K | 1 | |
| 42 | D E I T S G K G K | 1 | |
| 44 | I T S G K G K L T | 1 | |
| 45 | T S G K G K L T D | 1 | |
| 53 | D K E R H R L L E | 1 | |
| 61 | E K I R V L E A E | 1 | |
| 66 | L E A E K E K N A | 1 | |
| 68 | A E K E K N A Y Q | 1 | |
| 71 | E K N A Y Q L T E | 1 | |
| 74 | A Y Q L T E K D K | 1 | |
| 77 | L T E K D K E I Q | 1 | |
| 78 | T E K D K E I Q R | 1 | |
| 84 | I Q R L R D Q L K | 1 | |
| 105 | E E T T R E G E R | 1 | |
| 107 | T T R E G E R R E | 1 | |
| 121 | L S E E K D V L K | 1 | |
| 123 | E E K D V L K Q Q | 1 | |
| 136 | T S R I A E L E S | 1 | |
| 139 | I A E L E S K T N | 1 | |
| 144 | S K T N T L R L S | 1 | |
| 153 | Q T V A P N C F N | 1 | |
| 163 | S I N N I H E M E | 1 | |
| 165 | N N I H E M E I Q | 1 | |
| 167 | I H E M E I Q L K | 1 | |
| 174 | L K D A L E K N Q | 1 | |
| 179 | E K N Q Q W L V Y | 1 | |
| 182 | Q Q W L V Y D Q Q | 1 | |
| 189 | Q Q R E V Y V K G | 1 | |
| 198 | L L A K I F E L E | 1 | |
| 199 | L A K I F E L E K | 1 | |
| 202 | I F E L E K K T E | 1 | |
| 209 | T E T A A H S L P | 1 | |
| 210 | E T A A H S L P Q | 1 | |
| 213 | A H S L P Q Q T K | 1 | |
| 217 | P Q Q T K K P E S | 1 | |
| 218 | Q Q T K K P E S E | 1 | |
| 220 | T K K P E S E G Y | 1 | |

| TABLE XXXII 121P2A3 v.1: HLA Peptide Scoring Results B*2709 9-mers SYFPEITHI | | | |
|---|---|---|---|
| Pos | 1 2 3 4 5 6 7 8 9 | score | SEQ. ID NO. |
| 234 | Q K C Y N D L L A | 1 | |
| 237 | Y N D L L A S A K | 1 | |
| 243 | S A K K D L E V E | 1 | |
| 248 | L E V E R Q T I T | 1 | |
| 253 | Q T I T Q L S F E | 1 | |
| 255 | I T Q L S F E L S | 1 | |
| 259 | S F E L S E F R R | 1 | |
| 276 | V H N L N Q L L Y | 1 | |
| 278 | N L N Q L L Y S Q | 1 | |
| 279 | L N Q L L Y S Q R | 1 | |
| 284 | Y S Q R R A D V Q | 1 | |
| 291 | V Q H L E D D R H | 1 | |
| 293 | H L E D D R H K T | 1 | |
| 302 | E K I Q K L R E E | 1 | |
| 311 | N D I A R G K L E | 1 | |
| 312 | D I A R G K L E E | 1 | |
| 313 | I A R G K L E E E | 1 | |
| 329 | L S Q V Q F L Y T | 1 | |
| 336 | Y T S L L K Q Q E | 1 | |
| 339 | L L K Q Q E E Q T | 1 | |
| 346 | Q T R V A L L E Q | 1 | |
| 362 | D F E N E K L D R | 1 | |
| 363 | F E N E K L D R Q | 1 | |
| 364 | E N E K L D R Q H | 1 | |
| 368 | L D R Q H V Q H Q | 1 | |
| 374 | Q H Q L H V I L K | 1 | |
| 377 | L H V I L K E L R | 1 | |
| 380 | I L K E L R K A R | 1 | |
| 381 | L K E L R K A R N | 1 | |
| 390 | Q I T Q L E S L K | 1 | |
| 393 | Q L E S L K Q L H | 1 | |
| 394 | L E S L K Q L H E | 1 | |
| 399 | Q L H E F A I T E | 1 | |
| 406 | T E P L V T F Q G | 1 | |
| 408 | P L V T F Q G E T | 1 | |
| 409 | L V T F Q G E T E | 1 | |
| 411 | T F Q G E T E N R | 1 | |
| 412 | F Q G E T E N R E | 1 | |
| 416 | T E N R E K V A A | 1 | |
| 417 | E N R E K V A A S | 1 | |
| 422 | V A A S P K S P T | 1 | |
| 424 | A S P K S P T A A | 1 | |
| 428 | S P T A A L N E S | 1 | |
| 436 | S L V E C P K C N | 1 | |
| 438 | V E C P K C N I Q | 1 | |
| 439 | E C P K C N I Q Y | 1 | |
| 443 | C N I Q Y P A T E | 1 | |
| 444 | N I Q Y P A T E H | 1 | |
| 446 | Q Y P A T E H R D | 1 | |

| TABLE XXXII 121P2A3 v.3: HLA Peptide Scoring Results B*2709 9-mers SYFPEITHI | | | |
|---|---|---|---|
| Pos | 1 2 3 4 5 6 7 8 9 | score | SEQ. ID NO. |
| 9 | Q R L L E K I R V | 21 | |
| 7 | E R Q R L L E K I | 18 | |

| TABLE XXXII 121P2A3 v.3: HLA Peptide Scoring Results B*2709 9-mers SYFPEITHI | | | |
|---|---|---|---|
| Pos | 1 2 3 4 5 6 7 8 9 | score | SEQ. ID NO. |
| 3 | L T D K E R Q R L | 11 | |
| 4 | T D K E R Q R L L | 10 | |
| 1 | G K L T D K E R Q | 7 | |
| 2 | K L T D K E R Q R | 4 | |
| 8 | R Q R L L E K I R | 4 | |
| 6 | K E R Q R L L E K | 3 | |
| 5 | D K E R Q R L L E | 1 | |

| TABLE XXXII 121P2A3 v.4: HLA Peptide Scoring Results B*2709 9-mers SYFPEITHI | | | |
|---|---|---|---|
| Pos | 1 2 3 4 5 6 7 8 9 | score | SEQ. ID NO. |
| 3 | A R Y S T T T L L | 24 | |
| 2 | K A R Y S T T T L | 12 | |
| 6 | S T T T L L E Q L | 12 | |
| 4 | R Y S T T T L L E | 5 | |
| 8 | T T L L E Q L E E | 3 | |
| 5 | Y S T T T L L E Q | 2 | |
| 9 | T L L E Q L E E T | 2 | |
| 7 | T T T L L E Q L E | 1 | |

| TABLE XXXII 121P2A3 v.6: HLA Peptide Scoring Results B*2709 9-mers SYFPEITHI | | | |
|---|---|---|---|
| Pos | 1 2 3 4 5 6 7 8 9 | score | SEQ. ID NO. |
| 2 | E L L S Q V Q S L | 14 | |
| 6 | Q V Q S L Y T S L | 11 | |
| 7 | V Q S L Y T S L L | 10 | |
| 1 | E E L L S Q V Q S | 4 | |
| 8 | Q S L Y T S L L K | 3 | |
| 9 | S L Y T S L L K Q | 3 | |
| 5 | S Q V Q S L Y T S | 2 | |
| 4 | L S Q V Q S L Y T | 1 | |

| TABLE XXXII 121P2A3 v.7: HLA Peptide Scoring Results B*2709 9-mers SYFPEITHI | | | |
|---|---|---|---|
| Pos | 1 2 3 4 5 6 7 8 9 | score | SEQ. ID NO. |
| 1 | R Q H V Q H Q L L | 14 | |
| 7 | Q L L V I L K E L | 13 | |
| 4 | V Q H Q L L V I L | 11 | |
| 2 | Q H V Q H Q L L V | 10 | |
| 3 | H V Q H Q L L V I | 9 | |
| 6 | H Q L L V I L K E | 3 | |
| 9 | L V I L K E L R K | 3 | |
| 5 | Q H Q L L V I L K | 1 | |
| 8 | L L V I L K E L R | 1 | |

| TABLE XXXII 121P2A3 v.8: HLA Peptide Scoring Results B*2709 9-mers SYFPEITHI | | | |
|---|---|---|---|
| Pos | 1 2 3 4 5 6 7 8 9 | score | SEQ. ID NO. |
| 3 | P T A A L N G S L | 10 | |
| 4 | T A A L N G S L V | 8 | |
| 9 | G S L V E C P K C | 6 | |

| TABLE XXXII 121P2A3 v.8: HLA Peptide Scoring Results B*2709 9-mers SYFPEITHI | | | |
|---|---|---|---|
| Pos | 1 2 3 4 5 6 7 8 9 | score | SEQ. ID NO. |
| 1 | K S P T A A L N G | 5 | |
| 5 | A A L N G S L V E | 4 | |
| 6 | A L N G S L V E C | 2 | |
| 7 | L N G S L V E C P | 2 | |
| 2 | S P T A A L N G S | 1 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 326 | E E L L S Q V Q F | 26 | |
| 344 | E E Q T R V A L L | 26 | |
| 170 | M E I Q L K D A L | 25 | |
| 274 | K E V H N L N Q L | 25 | |
| 250 | V E R Q T I T Q L | 24 | |
| 343 | Q E E Q T R V A L | 24 | |
| 401 | H E F A I T E P L | 24 | |
| 320 | E E K K R S E E L | 23 | |
| 191 | R E V Y V K G L L | 21 | |
| 32 | G E I A H L K T S | 17 | |
| 82 | K E I Q R L R D Q | 17 | |
| 123 | E E K D V L K Q Q | 17 | |
| 134 | A A T S R I A E L | 17 | |
| 42 | D E I T S G K G K | 16 | |
| 58 | R L L E K I R V L | 16 | |
| 79 | E K D K E I Q R L | 16 | |
| 143 | E S K T N T L R L | 16 | |
| 261 | E L S E F R R K Y | 16 | |
| 294 | L E D D R H K T E | 16 | |
| 309 | E E N D I A R G K | 16 | |
| 386 | K A R N Q I T Q L | 16 | |
| 389 | N Q I T Q L E S L | 16 | |
| 404 | A I T E P L V T F | 16 | |
| 1 | M S S R S T K D L | 15 | |
| 23 | S E T T L E K L K | 15 | |
| 29 | K L K G E I A H L | 15 | |
| 67 | E A E K E K N A Y | 15 | |
| 69 | E K E K N A Y Q L | 15 | |
| 83 | E I Q R L R D Q L | 15 | |
| 93 | A R Y S T T A L L | 15 | |
| 110 | E G E R R E Q V L | 15 | |
| 113 | R R E Q V L K A L | 15 | |
| 120 | A L S E E K D V L | 15 | |
| 141 | E L E S K T N T L | 15 | |
| 263 | S E F R R K Y E E | 15 | |
| 310 | E N D I A R G K L | 15 | |
| 332 | V Q F L Y T S L L | 15 | |
| 382 | K E L R K A R N Q | 15 | |
| 392 | T Q L E S L K Q L | 15 | |
| 395 | E S L K Q L H E F | 15 | |
| 439 | E C P K C N I Q Y | 15 | |
| 6 | T K D L I K S K W | 14 | |
| 22 | K S E T T L E K L | 14 | |
| 54 | K E R H R L L E K | 14 | |
| 92 | K A R Y S T T A L | 14 | |
| 105 | E E T T R E G E R | 14 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 122 | S E E K D V L K Q | 14 | |
| 124 | E K D V L K Q Q L | 14 | |
| 140 | A E L E S K T N T | 14 | |
| 168 | H E M E I Q L K D | 14 | |
| 177 | A L E K N Q Q W L | 14 | |
| 179 | E K N Q Q W L V Y | 14 | |
| 195 | V K G L L A K I F | 14 | |
| 197 | G L L A K I F E L | 14 | |
| 208 | K T E T A A H S L | 14 | |
| 271 | E T Q K E V H N L | 14 | |
| 275 | E V H N L N Q L L | 14 | |
| 276 | V H N L N Q L L Y | 14 | |
| 299 | H K T E K I Q K L | 14 | |
| 308 | R E E N D I A R G | 14 | |
| 321 | E K K R S E E L L | 14 | |
| 327 | E L L S Q V Q F L | 14 | |
| 353 | E Q Q M Q A C T L | 14 | |
| 376 | Q L H V I L K E L | 14 | |
| 406 | T E P L V T F Q G | 14 | |
| 416 | T E N R E K V A A | 14 | |
| 425 | S P K S P T A A L | 14 | |
| 438 | V E C P K C N I Q | 14 | |
| 450 | T E H R D L L V H | 14 | |
| 19 | S N S K S E T T L | 13 | |
| 43 | E I T S G K G K L | 13 | |
| 51 | L T D K E R H R L | 13 | |
| 52 | T D K E R H R L L | 13 | |
| 55 | E R H R L L E K I | 13 | |
| 60 | L E K I R V L E A | 13 | |
| 68 | A E K E K N A Y Q | 13 | |
| 70 | K E K N A Y Q L T | 13 | |
| 78 | T E K D K E I Q R | 13 | |
| 96 | S T T A L L E Q L | 13 | |
| 109 | R E G E R R E Q V | 13 | |
| 111 | G E R R E Q V L K | 13 | |
| 152 | S Q T V A P N C F | 13 | |
| 166 | N I H E M E I Q L | 13 | |
| 186 | V Y D Q Q R E V Y | 13 | |
| 190 | Q R E V Y V K G L | 13 | |
| 220 | T K K P E S E G Y | 13 | |
| 223 | P E S E G Y L Q E | 13 | |
| 232 | E K Q K C Y N D L | 13 | |
| 260 | F E L S E F R R K | 13 | |
| 270 | E E T Q K E V H N | 13 | |
| 319 | E E E K K R S E E | 13 | |
| 325 | S E E L L S Q V Q | 13 | |
| 365 | N E K L D R Q H V | 13 | |
| 383 | E L R K A R N Q I | 13 | |
| 394 | L E S L K Q L H E | 13 | |
| 414 | G E T E N R E K V | 13 | |
| 434 | N E S L V E C P K | 13 | |
| 453 | R D L L V H V E Y | 13 | |
| 66 | L E A E K E K N A | 12 | |
| 101 | L E Q L E E T T R | 12 | |
| 104 | L E E T T R E G E | 12 | |
| 142 | L E S K T N T L R | 12 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 159 | C F N S S I N N I | 12 | |
| 176 | D A L E K N Q Q W | 12 | |
| 178 | L E K N Q Q W L V | 12 | |
| 205 | L E K K T E T A A | 12 | |
| 221 | K K P E S E G Y L | 12 | |
| 229 | L Q E E K Q K C Y | 12 | |
| 231 | E E K Q K C Y N D | 12 | |
| 233 | K Q K C Y N D L L | 12 | |
| 240 | L L A S A K K D L | 12 | |
| 257 | Q L S F E L S E F | 12 | |
| 269 | Y E E T Q K E V H | 12 | |
| 328 | L L S Q V Q F L Y | 12 | |
| 355 | Q M Q A C T L D F | 12 | |
| 360 | T L D F E N E K L | 12 | |
| 369 | D R Q H V Q H Q L | 12 | |
| 447 | Y P A T E H R D L | 12 | |
| 448 | P A T E H R D L L | 12 | |
| 27 | L E K L K G E I A | 11 | |
| 87 | L R D Q L K A R Y | 11 | |
| 114 | R E Q V L K A L S | 11 | |
| 156 | A P N C F N S S I | 11 | |
| 194 | Y V K G L L A K I | 11 | |
| 203 | F E L E K K T E T | 11 | |
| 209 | T E T A A H S L P | 11 | |
| 225 | S E G Y L Q E E K | 11 | |
| 248 | L E V E R Q T I T | 11 | |
| 252 | R Q T I T Q L S F | 11 | |
| 286 | Q R R A D V Q H L | 11 | |
| 301 | T E K I Q K L R E | 11 | |
| 305 | Q K L R E E N D I | 11 | |
| 318 | L E E E K K R S E | 11 | |
| 363 | F E N E K L D R Q | 11 | |
| 372 | H V Q H Q L H V I | 11 | |
| 373 | V Q H Q L H V I L | 11 | |
| 397 | L K Q L H E F A I | 11 | |
| 419 | R E K V A A S P K | 11 | |
| 2 | S S R S T K D L I | 10 | |
| 36 | H L K T S V D E I | 10 | |
| 76 | Q L T E K D K E I | 10 | |
| 131 | Q L S A A T S R I | 10 | |
| 162 | S S I N N I H E M | 10 | |
| 230 | Q E E K Q K C Y N | 10 | |
| 247 | D L E V E R Q T I | 10 | |
| 254 | T I T Q L S F E L | 10 | |
| 296 | D D R H K T E K I | 10 | |
| 331 | Q V Q F L Y T S L | 10 | |
| 352 | L E Q Q M Q A C T | 10 | |
| 429 | P T A A L N E S L | 10 | |
| 61 | E K I R V L E A E | 9 | |
| 26 | T L E K L K G E I | 8 | |
| 135 | A T S R I A E L E | 8 | |
| 164 | I N N I H E M E I | 8 | |
| 200 | A K I F E L E K K | 8 | |
| 311 | N D I A R G K L E | 8 | |
| 423 | A A S P K S P T A | 8 | |
| 437 | L V E C P K C N I | 8 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 5 | S T K D L I K S K | 7 | |
| 302 | E K I Q K L R E E | 7 | |
| 366 | E K L D R Q H V Q | 7 | |
| 403 | F A I T E P L V T | 7 | |
| 415 | E T E N R E K V A | 7 | |
| 424 | A S P K S P T A A | 7 | |
| 431 | A A L N E S L V E | 7 | |
| 28 | E K L K G E I A H | 6 | |
| 74 | A Y Q L T E K D K | 6 | |
| 86 | R L R D Q L K A R | 6 | |
| 133 | S A A T S R I A E | 6 | |
| 201 | K I F E L E K K T | 6 | |
| 206 | E K K T E T A A H | 6 | |
| 213 | A H S L P Q Q T K | 6 | |
| 215 | S L P Q Q T K K P | 6 | |
| 235 | K C Y N D L L A S | 6 | |
| 249 | E V E R Q T I T Q | 6 | |
| 251 | E R Q T I T Q L S | 6 | |
| 345 | E Q T R V A L L E | 6 | |
| 361 | L D F E N E K L D | 6 | |
| 375 | H Q L H V I L K E | 6 | |
| 387 | A R N Q I T Q L E | 6 | |
| 426 | P K S P T A A L N | 6 | |
| 449 | A T E H R D L L V | 6 | |
| 16 | S K P S N S K S E | 5 | |
| 24 | E T T L E K L K G | 5 | |
| 35 | A H L K T S V D E | 5 | |
| 44 | I T S G K G K L T | 5 | |
| 94 | R Y S T T A L L E | 5 | |
| 99 | A L L E Q L E E T | 5 | |
| 112 | E R R E Q V L K A | 5 | |
| 115 | E Q V L K A L S E | 5 | |
| 137 | S R I A E L E S K | 5 | |
| 144 | S K T N T L R L S | 5 | |
| 149 | L R L S Q T V A P | 5 | |
| 158 | N C F N S S I N N | 5 | |
| 169 | E M E I Q L K D A | 5 | |
| 212 | A A H S L P Q Q T | 5 | |
| 214 | H S L P Q Q T K K | 5 | |
| 237 | Y N D L L A S A K | 5 | |
| 239 | D L L A S A K K D | 5 | |
| 244 | A K K D L E V E R | 5 | |
| 253 | Q T I T Q L S F E | 5 | |
| 300 | K T E K I Q K L R | 5 | |
| 350 | A L L E Q Q M Q A | 5 | |
| 358 | A C T L D F E N E | 5 | |
| 367 | K L D R Q H V Q H | 5 | |
| 378 | H V I L K E L R K | 5 | |
| 380 | I L K E L R K A R | 5 | |
| 417 | E N R E K V A A S | 5 | |
| 427 | K S P T A A L N E | 5 | |
| 432 | A L N E S L V E C | 5 | |
| 443 | C N I Q Y P A T E | 5 | |
| 451 | E H R D L L V H V | 5 | |
| 452 | H R D L L V H V E | 5 | |
| 3 | S R S T K D L I K | 4 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 7 | K D L I K S K W G | 4 | |
| 15 | G S K P S N S K S | 4 | |
| 21 | S K S E T T L E K | 4 | |
| 25 | T T L E K L K G E | 4 | |
| 30 | L K G E I A H L K | 4 | |
| 38 | K T S V D E I T S | 4 | |
| 39 | T S V D E I T S G | 4 | |
| 40 | S V D E I T S G K | 4 | |
| 48 | K G K L T D K E R | 4 | |
| 53 | D K E R H R L L E | 4 | |
| 59 | L L E K I R V L E | 4 | |
| 71 | E K N A Y Q L T E | 4 | |
| 80 | K D K E I Q R L R | 4 | |
| 85 | Q R L R D Q L K A | 4 | |
| 102 | E Q L E E T T R E | 4 | |
| 119 | K A L S E E K D V | 4 | |
| 129 | K Q Q L S A A T S | 4 | |
| 139 | I A E L E S K T N | 4 | |
| 145 | K T N T L R L S Q | 4 | |
| 146 | T N T L R L S Q T | 4 | |
| 147 | N T L R L S Q T V | 4 | |
| 154 | T V A P N C F N S | 4 | |
| 155 | V A P N C F N S S | 4 | |
| 161 | N S S I N N I H E | 4 | |
| 165 | N N I H E M E I Q | 4 | |
| 167 | I H E M E I Q L K | 4 | |
| 171 | E I Q L K D A L E | 4 | |
| 175 | K D A L E K N Q Q | 4 | |
| 192 | E V Y V K G L L A | 4 | |
| 193 | V Y V K G L L A K | 4 | |
| 196 | K G L L A K I F E | 4 | |
| 202 | I F E L E K K T E | 4 | |
| 204 | E L E K K T E T A | 4 | |
| 224 | E S E G Y L Q E E | 4 | |
| 226 | E G Y L Q E E K Q | 4 | |
| 227 | G Y L Q E E K Q K | 4 | |
| 238 | N D L L A S A K K | 4 | |
| 242 | A S A K K D L E V | 4 | |
| 245 | K K D L E V E R Q | 4 | |
| 246 | K D L E V E R Q T | 4 | |
| 262 | L S E F R R K Y E | 4 | |
| 277 | H N L N Q L L Y S | 4 | |
| 282 | L L Y S Q R R A D | 4 | |
| 284 | Y S Q R R A D V Q | 4 | |
| 285 | S Q R R A D V Q H | 4 | |
| 289 | A D V Q H L E D D | 4 | |
| 293 | H L E D D R H K T | 4 | |
| 295 | E D D R H K T E K | 4 | |
| 307 | L R E E N D I A R | 4 | |
| 316 | G K L E E E K K R | 4 | |
| 323 | K R S E E L L S Q | 4 | |
| 334 | F L Y T S L L K Q | 4 | |
| 335 | L Y T S L L K Q Q | 4 | |
| 364 | E N E K L D R Q H | 4 | |
| 374 | Q H Q L H V I L K | 4 | |
| 379 | V I L K E L R K A | 4 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 385 | R K A R N Q I T Q | 4 | |
| 405 | I T E P L V T F Q | 4 | |
| 407 | E P L V T F Q G E | 4 | |
| 421 | K V A A S P K S P | 4 | |
| 435 | E S L V E C P K C | 4 | |
| 436 | S L V E C P K C N | 4 | |
| 440 | C P K C N I Q Y P | 4 | |
| 445 | I Q Y P A T E H R | 4 | |
| 4 | R S T K D L I K S | 3 | |
| 8 | D L I K S K W G S | 3 | |
| 11 | K S K W G S K P S | 3 | |
| 17 | K P S N S K S E T | 3 | |
| 20 | N S K S E T T L E | 3 | |
| 31 | K G E I A H L K T | 3 | |
| 34 | I A H L K T S V D | 3 | |
| 46 | S G K G K L T D K | 3 | |
| 50 | K L T D K E R H R | 3 | |
| 56 | R H R L L E K I R | 3 | |
| 57 | H R L L E K I R V | 3 | |
| 63 | I R V L E A E K E | 3 | |
| 64 | R V L E A E K E K | 3 | |
| 73 | N A Y Q L T E K D | 3 | |
| 75 | Y Q L T E K D K E | 3 | |
| 84 | I Q R L R D Q L K | 3 | |
| 88 | R D Q L K A R Y S | 3 | |
| 95 | Y S T T A L L E Q | 3 | |
| 98 | T A L L E Q L E E | 3 | |
| 106 | E T T R E G E R R | 3 | |
| 108 | T R E G E R R E Q | 3 | |
| 125 | K D V L K Q Q L S | 3 | |
| 127 | V L K Q Q L S A A | 3 | |
| 160 | F N S S I N N I H | 3 | |
| 163 | S I N N I H E M E | 3 | |
| 172 | I Q L K D A L E K | 3 | |
| 173 | Q L K D A L E K N | 3 | |
| 174 | L K D A L E K N Q | 3 | |
| 180 | K N Q Q W L V Y D | 3 | |
| 188 | D Q Q R E V Y V K | 3 | |
| 198 | L L A K I F E L E | 3 | |
| 210 | E T A A H S L P Q | 3 | |
| 234 | Q K C Y N D L L A | 3 | |
| 243 | S A K K D L E V E | 3 | |
| 255 | I T Q L S F E L S | 3 | |
| 264 | E F R R K Y E E T | 3 | |
| 268 | K Y E E T Q K E V | 3 | |
| 280 | N Q L L Y S Q R R | 3 | |
| 283 | L Y S Q R R A D V | 3 | |
| 287 | R R A D V Q H L E | 3 | |
| 298 | R H K T E K I Q K | 3 | |
| 314 | A R G K L E E E K | 3 | |
| 317 | K L E E E K K R S | 3 | |
| 322 | K K R S E E L L S | 3 | |
| 330 | S Q V Q F L Y T S | 3 | |
| 333 | Q F L Y T S L L K | 3 | |
| 336 | Y T S L L K Q Q E | 3 | |
| 342 | Q Q E E Q T R V A | 3 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 359 | C T L D F E N E K | 3 | |
| 391 | I T Q L E S L K Q | 3 | |
| 398 | K Q L H E F A I T | 3 | |
| 399 | Q L H E F A I T E | 3 | |
| 400 | L H E F A I T E P | 3 | |
| 402 | E F A I T E P L V | 3 | |
| 420 | E K V A A S P K S | 3 | |
| 428 | S P T A A L N E S | 3 | |
| 430 | T A A L N E S L V | 3 | |
| 442 | K C N I Q Y P A T | 3 | |
| 10 | I K S K W G S K P | 2 | |
| 12 | S K W G S K P S N | 2 | |
| 13 | K W G S K P S N S | 2 | |
| 14 | W G S K P S N S K | 2 | |
| 33 | E I A H L K T S V | 2 | |
| 45 | T S G K G K L T D | 2 | |
| 47 | G K G K L T D K E | 2 | |
| 49 | G K L T D K E R H | 2 | |
| 72 | K N A Y Q L T E K | 2 | |
| 81 | D K E I Q R L R D | 2 | |
| 89 | D Q L K A R Y S T | 2 | |
| 90 | Q L K A R Y S T T | 2 | |
| 91 | L K A R Y S T T A | 2 | |
| 97 | T T A L L E Q L E | 2 | |
| 103 | Q L E E T T R E G | 2 | |
| 116 | Q V L K A L S E E | 2 | |
| 118 | L K A L S E E K D | 2 | |
| 121 | L S E E K D V L K | 2 | |
| 126 | D V L K Q Q L S A | 2 | |
| 128 | L K Q Q L S A A T | 2 | |
| 130 | Q Q L S A A T S R | 2 | |
| 148 | T L R L S Q T V A | 2 | |
| 150 | R L S Q T V A P N | 2 | |
| 151 | L S Q T V A P N C | 2 | |
| 181 | N Q Q W L V Y D Q | 2 | |
| 182 | Q Q W L V Y D Q Q | 2 | |
| 183 | Q W L V Y D Q Q R | 2 | |
| 185 | L V Y D Q Q R E V | 2 | |
| 187 | Y D Q Q R E V Y V | 2 | |
| 189 | Q Q R E V Y V K G | 2 | |
| 207 | K K T E T A A H S | 2 | |
| 211 | T A A H S L P Q Q | 2 | |
| 216 | L P Q Q T K K P E | 2 | |
| 222 | K P E S E G Y L Q | 2 | |
| 236 | C Y N D L L A S A | 2 | |
| 241 | L A S A K K D L E | 2 | |
| 256 | T Q L S F E L S E | 2 | |
| 258 | L S F E L S E F R | 2 | |
| 259 | S F E L S E F R R | 2 | |
| 267 | R K Y E E T Q K E | 2 | |
| 272 | T Q K E V H N L N | 2 | |
| 278 | N L N Q L L Y S Q | 2 | |
| 279 | L N Q L L Y S Q R | 2 | |
| 281 | Q L L Y S Q R R A | 2 | |
| 288 | R A D V Q H L E D | 2 | |
| 292 | Q H L E D D R H K | 2 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 297 | D R H K T E K I Q | 2 | |
| 303 | K I Q K L R E E N | 2 | |
| 304 | I Q K L R E E N D | 2 | |
| 312 | D I A R G K L E E | 2 | |
| 313 | I A R G K L E E E | 2 | |
| 315 | R G K L E E E K K | 2 | |
| 324 | R S E E L L S Q V | 2 | |
| 329 | L S Q V Q F L Y T | 2 | |
| 337 | T S L L K Q Q E E | 2 | |
| 338 | S L L K Q Q E E Q | 2 | |
| 346 | Q T R V A L L E Q | 2 | |
| 347 | T R V A L L E Q Q | 2 | |
| 348 | R V A L L E Q Q M | 2 | |
| 349 | V A L L E Q Q M Q | 2 | |
| 351 | L L E Q Q M Q A C | 2 | |
| 354 | Q Q M Q A C T L D | 2 | |
| 362 | D F E N E K L D R | 2 | |
| 381 | L K E L R K A R N | 2 | |
| 410 | V T F Q G E T E N | 2 | |
| 411 | T F Q G E T E N R | 2 | |
| 413 | Q G E T E N R E K | 2 | |
| 418 | N R E K V A A S P | 2 | |
| 441 | P K C N I Q Y P A | 2 | |
| 444 | N I Q Y P A T E H | 2 | |
| 446 | Q Y P A T E H R D | 2 | |
| 454 | D L L V H V E Y C | 2 | |
| 18 | P S N S K S E T T | 1 | |
| 41 | V D E I T S G K G | 1 | |
| 62 | K I R V L E A E K | 1 | |
| 65 | V L E A E K E K N | 1 | |
| 100 | L L E Q L E E T T | 1 | |
| 107 | T T R E G E R R E | 1 | |
| 132 | L S A A T S R I A | 1 | |
| 136 | T S R I A E L E S | 1 | |
| 138 | R I A E L E S K T | 1 | |
| 157 | P N C F N S S I N | 1 | |
| 199 | L A K I F E L E K | 1 | |
| 217 | P Q Q T K K P E S | 1 | |
| 218 | Q Q T K K P E S E | 1 | |
| 228 | Y L Q E E K Q K C | 1 | |
| 265 | F R R K Y E E T Q | 1 | |
| 266 | R R K Y E E T Q K | 1 | |
| 273 | Q K E V H N L N Q | 1 | |
| 291 | V Q H L E D D R H | 1 | |
| 306 | K L R E E N D I A | 1 | |
| 339 | L L K Q Q E E Q T | 1 | |
| 340 | L K Q Q E E Q T R | 1 | |
| 341 | K Q Q E E Q T R V | 1 | |
| 356 | M Q A C T L D F E | 1 | |
| 357 | Q A C T L D F E N | 1 | |
| 368 | L D R Q H V Q H Q | 1 | |
| 370 | R Q H V Q H Q L H | 1 | |
| 371 | Q H V Q H Q L H V | 1 | |
| 377 | L H V I L K E L R | 1 | |
| 384 | L R K A R N Q I T | 1 | |
| 388 | R N Q I T Q L E S | 1 | |

| TABLE XXXIII 121P2A3 v.1: HLA Peptide Scoring Results B*4402 9-mers SYFPEITHI | | | |
|---|---|---|---|
| Pos | 1 2 3 4 5 6 7 8 9 | score | SEQ. ID NO. |
| 390 | Q I T Q L E S L K | 1 | |
| 393 | Q L E S L K Q L H | 1 | |
| 396 | S L K Q L H E F A | 1 | |
| 408 | P L V T F Q G E T | 1 | |
| 409 | L V T F Q G E T E | 1 | |
| 412 | F Q G E T E N R E | 1 | |
| 433 | L N E S L V E C P | 1 | |

| TABLE XXXIII 121P2A3 v.3: HLA Peptide Scoring Results B*4402 9-mers SYFPEITHI | | | |
|---|---|---|---|
| Pos | 1 2 3 4 5 6 7 8 9 | score | SEQ. ID NO. |
| 6 | K E R Q R L L E K | 14 | |
| 4 | T D K E R Q R L L | 13 | |
| 7 | E R Q R L L E K I | 13 | |
| 3 | L T D K E R Q R L | 12 | |
| 2 | K L T D K E R Q R | 4 | |
| 5 | D K E R Q R L L E | 4 | |
| 8 | R Q R L L E K I R | 3 | |
| 9 | Q R L L E K I R V | 3 | |
| 1 | G K L T D K E R Q | 2 | |

| TABLE XXXIII 121P2A3 v.4: HLA Peptide Scoring Results B*4402 9-mers SYFPEITHI | | | |
|---|---|---|---|
| Pos | 1 2 3 4 5 6 7 8 9 | score | SEQ. ID NO. |
| 3 | A R Y S T T T L L | 15 | |
| 6 | S T T T L L E Q L | 14 | |
| 2 | K A R Y S T T T L | 13 | |
| 4 | R Y S T T T L L E | 5 | |
| 5 | Y S T T T L L E Q | 3 | |
| 8 | T T L L E Q L E E | 3 | |
| 1 | L K A R Y S T T T | 2 | |
| 7 | T T T L L E Q L E | 2 | |
| 9 | T L L E Q L E E T | 2 | |

| TABLE XXXIII 121P2A3 v.6: HLA Peptide Scoring Results B*4402 9-mers SYFPEITHI | | | |
|---|---|---|---|
| Pos | 1 2 3 4 5 6 7 8 9 | score | SEQ. ID NO. |
| 1 | E E L L S Q V Q S | 16 | |
| 2 | E L L S Q V Q S L | 14 | |
| 7 | V Q S L Y T S L L | 14 | |
| 3 | L L S Q V Q S L Y | 12 | |
| 6 | Q V Q S L Y T S L | 10 | |
| 9 | S L Y T S L L K Q | 5 | |
| 5 | S Q V Q S L Y T S | 3 | |
| 8 | Q S L Y T S L L K | 3 | |
| 4 | L S Q V Q S L Y T | 1 | |

| TABLE XXXIII 121P2A3 v.7: HLA Peptide Scoring Results B*4402 9-mers SYFPEITHI | | | |
|---|---|---|---|
| Pos | 1 2 3 4 5 6 7 8 9 | score | SEQ. ID NO. |
| 7 | Q L L V I L K E L | 15 | |

| TABLE XXXIII 121P2A3 v.7: HLA Peptide Scoring Results B*4402 9-mers SYFPEITHI | | | |
|---|---|---|---|
| Pos | 1 2 3 4 5 6 7 8 9 | score | SEQ. ID NO. |
| 4 | V Q H Q L L V I L | 12 | |
| 1 | R Q H V Q H Q L L | 11 | |
| 3 | H V Q H Q L L V I | 11 | |
| 6 | H Q L L V I L K E | 7 | |
| 9 | L V I L K E L R K | 5 | |
| 5 | Q H Q L L V I L K | 4 | |
| 2 | Q H V Q H Q L L V | 2 | |
| 8 | L L V I L K E L R | 1 | |

| TABLE XXXIII 121P2A3 v.8: HLA Peptide Scoring Results B*4402 9-mers SYFPEITHI | | | |
|---|---|---|---|
| Pos | 1 2 3 4 5 6 7 8 9 | score | SEQ. ID NO. |
| 3 | P T A A L N G S L | 10 | |
| 5 | A A L N G S L V E | 7 | |
| 6 | A L N G S L V E C | 6 | |
| 1 | K S P T A A L N G | 5 | |
| 2 | S P T A A L N G S | 3 | |
| 4 | T A A L N G S L V | 3 | |
| 8 | N G S L V E C P K | 3 | |
| 9 | G S L V E C P K C | 2 | |
| 7 | L N G S L V E C P | 1 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 119 | K A L S E E K D V | 21 | |
| 156 | A P N C F N S S I | 21 | |
| 176 | D A L E K N Q Q W | 20 | |
| 447 | Y P A T E H R D L | 20 | |
| 430 | T A A L N E S L V | 19 | |
| 92 | K A R Y S T T A L | 18 | |
| 386 | K A R N Q I T Q L | 18 | |
| 448 | P A T E H R D L L | 18 | |
| 73 | N A Y Q L T E K D | 17 | |
| 134 | A A T S R I A E L | 17 | |
| 296 | D D R H K T E K I | 17 | |
| 403 | F A I T E P L V T | 17 | |
| 34 | I A H L K T S V D | 16 | |
| 58 | R L L E K I R V L | 16 | |
| 185 | L V Y D Q Q R E V | 16 | |
| 194 | Y V K G L L A K I | 16 | |
| 247 | D L E V E R Q T I | 16 | |
| 425 | S P K S P T A A L | 16 | |
| 431 | A A L N E S L V E | 16 | |
| 110 | E G E R R E Q V L | 15 | |
| 139 | I A E L E S K T N | 15 | |
| 243 | S A K K D L E V E | 15 | |
| 313 | I A R G K L E E E | 15 | |
| 36 | H L K T S V D E I | 14 | |
| 76 | Q L T E K D K E I | 14 | |
| 98 | T A L L E Q L E E | 14 | |
| 155 | V A P N C F N S S | 14 | |
| 216 | L P Q Q T K K P E | 14 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 241 | L A S A K K D L E | 14 | |
| 372 | H V Q H Q L H V I | 14 | |
| 392 | T Q L E S L K Q L | 14 | |
| 407 | E P L V T F Q G E | 14 | |
| 55 | E R H R L L E K I | 13 | |
| 133 | S A A T S R I A E | 13 | |
| 147 | N T L R L S Q T V | 13 | |
| 159 | C F N S S I N N I | 13 | |
| 199 | L A K I F E L E K | 13 | |
| 211 | T A A H S L P Q Q | 13 | |
| 305 | Q K L R E E N D I | 13 | |
| 349 | V A L L E Q Q M Q | 13 | |
| 423 | A A S P K S P T A | 13 | |
| 428 | S P T A A L N E S | 13 | |
| 26 | T L E K L K G E I | 12 | |
| 57 | H R L L E K I R V | 12 | |
| 67 | E A E K E K N A Y | 12 | |
| 131 | Q L S A A T S R I | 12 | |
| 164 | I N N I H E M E I | 12 | |
| 226 | E G Y L Q E E K Q | 12 | |
| 239 | D L L A S A K K D | 12 | |
| 268 | K Y E E T Q K E V | 12 | |
| 299 | H K T E K I Q K L | 12 | |
| 341 | K Q Q E E Q T R V | 12 | |
| 369 | D R Q H V Q H Q L | 12 | |
| 383 | E L R K A R N Q I | 12 | |
| 397 | L K Q L H E F A I | 12 | |
| 414 | G E T E N R E K V | 12 | |
| 437 | L V E C P K C N I | 12 | |
| 451 | E H R D L L V H V | 12 | |
| 2 | S S R S T K D L I | 11 | |
| 17 | K P S N S K S E T | 11 | |
| 19 | S N S K S E T T L | 11 | |
| 22 | K S E T T L E K L | 11 | |
| 93 | A R Y S T T A L L | 11 | |
| 120 | A L S E E K D V L | 11 | |
| 166 | N I H E M E I Q L | 11 | |
| 187 | Y D Q Q R E V Y V | 11 | |
| 197 | G L L A K I F E L | 11 | |
| 212 | A A H S L P Q Q T | 11 | |
| 242 | A S A K K D L E V | 11 | |
| 288 | R A D V Q H L E D | 11 | |
| 324 | R S E E L L S Q V | 11 | |
| 334 | F L Y T S L L K Q | 11 | |
| 357 | Q A C T L D F E N | 11 | |
| 422 | V A A S P K S P T | 11 | |
| 440 | C P K C N I Q Y P | 11 | |
| 46 | S G K G K L T D K | 10 | |
| 51 | L T D K E R H R L | 10 | |
| 52 | T D K E R H R L L | 10 | |
| 109 | R E G E R R E Q V | 10 | |
| 113 | R R E Q V L K A L | 10 | |
| 141 | E L E S K T N T L | 10 | |
| 178 | L E K N Q Q W L V | 10 | |
| 190 | Q R E V Y V K G L | 10 | |
| 221 | K K P E S E G Y L | 10 | |

TABLE XXXIV 121P2A3 v.1: HLA Peptide Scoring Results B*5101 9-mers SYFPEITHI

| Pos | 1 2 3 4 5 6 7 8 9 | score | SEQ. ID NO. |
|---|---|---|---|
| 222 | K P E S E G Y L Q | 10 | |
| 250 | V E R Q T I T Q L | 10 | |
| 283 | L Y S Q R R A D V | 10 | |
| 286 | Q R R A D V Q H L | 10 | |
| 327 | E L L S Q V Q F L | 10 | |
| 360 | T L D F E N E K L | 10 | |
| 365 | N E K L D R Q H V | 10 | |
| 371 | Q H V Q H Q L H V | 10 | |
| 1 | M S S R S T K D L | 9 | |
| 29 | K L K G E I A H L | 9 | |
| 31 | K G E I A H L K T | 9 | |
| 33 | E I A H L K T S V | 9 | |
| 79 | E K D K E I Q R L | 9 | |
| 143 | E S K T N T L R L | 9 | |
| 188 | D Q Q R E V Y V K | 9 | |
| 196 | K G L L A K I F E | 9 | |
| 240 | L L A S A K K D L | 9 | |
| 271 | E T Q K E V H N L | 9 | |
| 332 | V Q F L Y T S L L | 9 | |
| 344 | E E Q T R V A L L | 9 | |
| 353 | E Q Q M Q A C T L | 9 | |
| 389 | N Q I T Q L E S L | 9 | |
| 402 | E F A I T E P L V | 9 | |
| 413 | Q G E T E N R E K | 9 | |
| 449 | A T E H R D L L V | 9 | |
| 14 | W G S K P S N S K | 8 | |
| 25 | T T L E K L K G E | 8 | |
| 43 | E I T S G K G K L | 8 | |
| 48 | K G K L T D K E R | 8 | |
| 69 | E K E K N A Y Q L | 8 | |
| 96 | S T T A L L E Q L | 8 | |
| 126 | D V L K Q Q L S A | 8 | |
| 191 | R E V Y V K G L L | 8 | |
| 208 | K T E T A A H S L | 8 | |
| 232 | E K Q K C Y N D L | 8 | |
| 233 | K Q K C Y N D L L | 8 | |
| 267 | R K Y E E T Q K E | 8 | |
| 274 | K E V H N L N Q L | 8 | |
| 310 | E N D I A R G K L | 8 | |
| 315 | R G K L E E E K K | 8 | |
| 343 | Q E E Q T R V A L | 8 | |
| 373 | V Q H Q L H V I L | 8 | |
| 375 | H Q L H V I L K E | 8 | |
| 376 | Q L H V I L K E L | 8 | |
| 379 | V I L K E L R K A | 8 | |
| 401 | H E F A I T E P L | 8 | |
| 429 | P T A A L N E S L | 8 | |
| 454 | D L L V H V E Y C | 8 | |
| 42 | D E I T S G K G K | 7 | |
| 75 | Y Q L T E K D K E | 7 | |
| 89 | D Q L K A R Y S T | 7 | |
| 112 | E R R E Q V L K A | 7 | |
| 170 | M E I Q L K D A L | 7 | |
| 172 | I Q L K D A L E K | 7 | |
| 177 | A L E K N Q Q W L | 7 | |
| 189 | Q Q R E V Y V K G | 7 | |
| 203 | F E L E K K T E T | 7 | |
| 246 | K D L E V E R Q T | 7 | |
| 254 | T I T Q L S F E L | 7 | |
| 275 | E V H N L N Q L L | 7 | |
| 282 | L L Y S Q R R A D | 7 | |
| 297 | D R H K T E K I Q | 7 | |
| 316 | G K L E E E K K R | 7 | |
| 320 | E E K K R S E E L | 7 | |
| 321 | E K K R S E E L L | 7 | |
| 331 | Q V Q F L Y T S L | 7 | |
| 445 | I Q Y P A T E H R | 7 | |
| 8 | D L I K S K W G S | 6 | |
| 66 | L E A E K E K N A | 6 | |
| 81 | D K E I Q R L R D | 6 | |
| 83 | E I Q R L R D Q L | 6 | |
| 91 | L K A R Y S T T A | 6 | |
| 102 | E Q L E E T T R E | 6 | |
| 121 | L S E E K D V L K | 6 | |
| 122 | S E E K D V L K Q | 6 | |
| 124 | E K D V L K Q Q L | 6 | |
| 140 | A E L E S K T N T | 6 | |
| 142 | L E S K T N T L R | 6 | |
| 148 | T L R L S Q T V A | 6 | |
| 151 | L S Q T V A P N C | 6 | |
| 192 | E V Y V K G L L A | 6 | |
| 201 | K I F E L E K K T | 6 | |
| 228 | Y L Q E E K Q K C | 6 | |
| 229 | L Q E E K Q K C Y | 6 | |
| 235 | K C Y N D L L A S | 6 | |
| 260 | F E L S E F R R K | 6 | |
| 269 | Y E E T Q K E V H | 6 | |
| 272 | T Q K E V H N L N | 6 | |
| 284 | Y S Q R R A D V Q | 6 | |
| 292 | Q H L E D D R H K | 6 | |
| 294 | L E D D R H K T E | 6 | |
| 318 | L E E E K K R S E | 6 | |
| 342 | Q Q E E Q T R V A | 6 | |
| 361 | L D F E N E K L D | 6 | |
| 363 | F E N E K L D R Q | 6 | |
| 366 | E K L D R Q H V Q | 6 | |
| 399 | Q L H E F A I T E | 6 | |
| 404 | A I T E P L V T F | 6 | |
| 412 | F Q G E T E N R E | 6 | |
| 450 | T E H R D L L V H | 6 | |
| 4 | R S T K D L I K S | 5 | |
| 10 | I K S K W G S K P | 5 | |
| 30 | L K G E I A H L K | 5 | |
| 32 | G E I A H L K T S | 5 | |
| 35 | A H L K T S V D E | 5 | |
| 44 | I T S G K G K L T | 5 | |
| 45 | T S G K G K L T D | 5 | |
| 53 | D K E R H R L L E | 5 | |
| 60 | L E K I R V L E A | 5 | |
| 63 | I R V L E A E K E | 5 | |
| 64 | R V L E A E K E K | 5 | |
| 71 | E K N A Y Q L T E | 5 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 85 | Q R L R D Q L K A | 5 | |
| 95 | Y S T T A L L E Q | 5 | |
| 99 | A L L E Q L E E T | 5 | |
| 101 | L E Q L E E T T R | 5 | |
| 107 | T T R E G E R R E | 5 | |
| 118 | L K A L S E E K D | 5 | |
| 123 | E E K D V L K Q Q | 5 | |
| 132 | L S A A T S R I A | 5 | |
| 149 | L R L S Q T V A P | 5 | |
| 168 | H E M E I Q L K D | 5 | |
| 202 | I F E L E K K T E | 5 | |
| 205 | L E K K T E T A A | 5 | |
| 207 | K K T E T A A H S | 5 | |
| 214 | H S L P Q Q T K K | 5 | |
| 238 | N D L L A S A K K | 5 | |
| 248 | L E V E R Q T I T | 5 | |
| 258 | L S F E L S E F R | 5 | |
| 261 | E L S E F R R K Y | 5 | |
| 265 | F R R K Y E E T Q | 5 | |
| 280 | N Q L L Y S Q R R | 5 | |
| 281 | Q L L Y S Q R R A | 5 | |
| 307 | L R E E N D I A R | 5 | |
| 312 | D I A R G K L E E | 5 | |
| 362 | D F E N E K L D R | 5 | |
| 368 | L D R Q H V Q H Q | 5 | |
| 380 | I L K E L R K A R | 5 | |
| 382 | K E L R K A R N Q | 5 | |
| 391 | I T Q L E S L K Q | 5 | |
| 394 | L E S L K Q L H E | 5 | |
| 405 | I T E P L V T F Q | 5 | |
| 417 | E N R E K V A A S | 5 | |
| 418 | N R E K V A A S P | 5 | |
| 424 | A S P K S P T A A | 5 | |
| 432 | A L N E S L V E C | 5 | |
| 452 | H R D L L V H V E | 5 | |
| 453 | R D L L V H V E Y | 5 | |
| 7 | K D L I K S K W G | 4 | |
| 15 | G S K P S N S K S | 4 | |
| 21 | S K S E T T L E K | 4 | |
| 28 | E K L K G E I A H | 4 | |
| 37 | L K T S V D E I T | 4 | |
| 39 | T S V D E I T S G | 4 | |
| 41 | V D E I T S G K G | 4 | |
| 49 | G K L T D K E R H | 4 | |
| 50 | K L T D K E R H R | 4 | |
| 59 | L L E K I R V L E | 4 | |
| 65 | V L E A E K E K N | 4 | |
| 68 | A E K E K N A Y Q | 4 | |
| 78 | T E K D K E I Q R | 4 | |
| 94 | R Y S T T A L L E | 4 | |
| 100 | L L E Q L E E T T | 4 | |
| 103 | Q L E E T T R E G | 4 | |
| 104 | L E E T T R E G E | 4 | |
| 116 | Q V L K A L S E E | 4 | |
| 129 | K Q Q L S A A T S | 4 | |
| 130 | Q Q L S A A T S R | 4 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 138 | R I A E L E S K T | 4 | |
| 158 | N C F N S S I N N | 4 | |
| 161 | N S S I N N I H E | 4 | |
| 173 | Q L K D A L E K N | 4 | |
| 174 | L K D A L E K N Q | 4 | |
| 179 | E K N Q Q W L V Y | 4 | |
| 186 | V Y D Q Q R E V Y | 4 | |
| 193 | V Y V K G L L A K | 4 | |
| 198 | L L A K I F E L E | 4 | |
| 204 | E L E K K T E T A | 4 | |
| 215 | S L P Q Q T K K P | 4 | |
| 255 | I T Q L S F E L S | 4 | |
| 256 | T Q L S F E L S E | 4 | |
| 277 | H N L N Q L L Y S | 4 | |
| 290 | D V Q H L E D D R | 4 | |
| 317 | K L E E E K K R S | 4 | |
| 323 | K R S E E L L S Q | 4 | |
| 326 | E E L L S Q V Q F | 4 | |
| 328 | L L S Q V Q F L Y | 4 | |
| 329 | L S Q V Q F L Y T | 4 | |
| 333 | Q F L Y T S L L K | 4 | |
| 335 | L Y T S L L K Q Q | 4 | |
| 337 | T S L L K Q Q E E | 4 | |
| 340 | L K Q Q E E Q T R | 4 | |
| 345 | E Q T R V A L L E | 4 | |
| 350 | A L L E Q Q M Q A | 4 | |
| 359 | C T L D F E N E K | 4 | |
| 384 | L R K A R N Q I T | 4 | |
| 395 | E S L K Q L H E F | 4 | |
| 400 | L H E F A I T E P | 4 | |
| 406 | T E P L V T F Q G | 4 | |
| 409 | L V T F Q G E T E | 4 | |
| 415 | E T E N R E K V A | 4 | |
| 421 | K V A A S P K S P | 4 | |
| 427 | K S P T A A L N E | 4 | |
| 433 | L N E S L V E C P | 4 | |
| 435 | E S L V E C P K C | 4 | |
| 436 | S L V E C P K C N | 4 | |
| 439 | E C P K C N I Q Y | 4 | |
| 443 | C N I Q Y P A T E | 4 | |
| 446 | Q Y P A T E H R D | 4 | |
| 455 | L L V H V E Y C S | 4 | |
| 3 | S R S T K D L I K | 3 | |
| 5 | S T K D L I K S K | 3 | |
| 6 | T K D L I K S K W | 3 | |
| 9 | L I K S K W G S K | 3 | |
| 12 | S K W G S K P S N | 3 | |
| 18 | P S N S K S E T T | 3 | |
| 24 | E T T L E K L K G | 3 | |
| 27 | L E K L K G E I A | 3 | |
| 38 | K T S V D E I T S | 3 | |
| 47 | G K G K L T D K E | 3 | |
| 61 | E K I R V L E A E | 3 | |
| 77 | L T E K D K E I Q | 3 | |
| 80 | K D K E I Q R L R | 3 | |
| 86 | R L R D Q L K A R | 3 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 87 | L R D Q L K A R Y | 3 | |
| 88 | R D Q L K A R Y S | 3 | |
| 90 | Q L K A R Y S T T | 3 | |
| 108 | T R E G E R R E Q | 3 | |
| 115 | E Q V L K A L S E | 3 | |
| 144 | S K T N T L R L S | 3 | |
| 150 | R L S Q T V A P N | 3 | |
| 160 | F N S S I N N I H | 3 | |
| 167 | I H E M E I Q L K | 3 | |
| 181 | N Q Q W L V Y D Q | 3 | |
| 182 | Q Q W L V Y D Q Q | 3 | |
| 183 | Q W L V Y D Q Q R | 3 | |
| 195 | V K G L L A K I F | 3 | |
| 200 | A K I F E L E K K | 3 | |
| 218 | Q Q T K K P E S E | 3 | |
| 223 | P E S E G Y L Q E | 3 | |
| 224 | E S E G Y L Q E E | 3 | |
| 227 | G Y L Q E E K Q K | 3 | |
| 237 | Y N D L L A S A K | 3 | |
| 245 | K K D L E V E R Q | 3 | |
| 249 | E V E R Q T I T Q | 3 | |
| 262 | L S E F R R K Y E | 3 | |
| 270 | E E T Q K E V H N | 3 | |
| 287 | R R A D V Q H L E | 3 | |
| 293 | H L E D D R H K T | 3 | |
| 301 | T E K I Q K L R E | 3 | |
| 302 | E K I Q K L R E E | 3 | |
| 306 | K L R E E N D I A | 3 | |
| 309 | E E N D I A R G K | 3 | |
| 311 | N D I A R G K L E | 3 | |
| 325 | S E E L L S Q V Q | 3 | |
| 330 | S Q V Q F L Y T S | 3 | |
| 338 | S L L K Q Q E E Q | 3 | |
| 347 | T R V A L L E Q Q | 3 | |
| 352 | L E Q Q M Q A C T | 3 | |
| 356 | M Q A C T L D F E | 3 | |
| 374 | Q H Q L H V I L K | 3 | |
| 378 | H V I L K E L R K | 3 | |
| 381 | L K E L R K A R N | 3 | |
| 385 | R K A R N Q I T Q | 3 | |
| 398 | K Q L H E F A I T | 3 | |
| 410 | V T F Q G E T E N | 3 | |
| 420 | E K V A A S P K S | 3 | |
| 434 | N E S L V E C P K | 3 | |
| 438 | V E C P K C N I Q | 3 | |
| 444 | N I Q Y P A T E H | 3 | |
| 13 | K W G S K P S N S | 2 | |
| 16 | S K P S N S K S E | 2 | |
| 20 | N S K S E T T L E | 2 | |
| 23 | S E T T L E K L K | 2 | |
| 40 | S V D E I T S G K | 2 | |
| 54 | K E R H R L L E K | 2 | |
| 72 | K N A Y Q L T E K | 2 | |
| 74 | A Y Q L T E K D K | 2 | |
| 82 | K E I Q R L R D Q | 2 | |
| 84 | I Q R L R D Q L K | 2 | |

| TABLE XXXIV 121P2A3 v.1: HLA Peptide Scoring Results B*5101 9-mers SYFPEITHI | | | |
|---|---|---|---|
| Pos | 1 2 3 4 5 6 7 8 9 | score | SEQ. ID NO. |
| 117 | V L K A L S E E K | 2 | |
| 127 | V L K Q Q L S A A | 2 | |
| 128 | L K Q Q L S A A T | 2 | |
| 136 | T S R I A E L E S | 2 | |
| 137 | S R I A E L E S K | 2 | |
| 145 | K T N T L R L S Q | 2 | |
| 146 | T N T L R L S Q T | 2 | |
| 152 | S Q T V A P N C F | 2 | |
| 153 | Q T V A P N C F N | 2 | |
| 169 | E M E I Q L K D A | 2 | |
| 180 | K N Q Q W L V Y D | 2 | |
| 209 | T E T A A H S L P | 2 | |
| 210 | E T A A H S L P Q | 2 | |
| 213 | A H S L P Q Q T K | 2 | |
| 217 | P Q Q T K K P E S | 2 | |
| 236 | C Y N D L L A S A | 2 | |
| 244 | A K K D L E V E R | 2 | |
| 252 | R Q T I T Q L S F | 2 | |
| 253 | Q T I T Q L S F E | 2 | |
| 259 | S F E L S E F R R | 2 | |
| 273 | Q K E V H N L N Q | 2 | |
| 276 | V H N L N Q L L Y | 2 | |
| 278 | N L N Q L L Y S Q | 2 | |
| 279 | L N Q L L Y S Q R | 2 | |
| 291 | V Q H L E D D R H | 2 | |
| 298 | R H K T E K I Q K | 2 | |
| 300 | K T E K I Q K L R | 2 | |
| 304 | I Q K L R E E N D | 2 | |
| 308 | R E E N D I A R G | 2 | |
| 319 | E E E K K R S E E | 2 | |
| 336 | Y T S L L K Q Q E | 2 | |
| 339 | L L K Q Q E E Q T | 2 | |
| 346 | Q T R V A L L E Q | 2 | |
| 351 | L L E Q Q M Q A C | 2 | |
| 355 | Q M Q A C T L D F | 2 | |
| 364 | E N E K L D R Q H | 2 | |
| 377 | L H V I L K E L R | 2 | |
| 388 | R N Q I T Q L E S | 2 | |
| 411 | T F Q G E T E N R | 2 | |
| 416 | T E N R E K V A A | 2 | |
| 426 | P K S P T A A L N | 2 | |
| 456 | L V H V E Y C S K | 2 | |
| 11 | K S K W G S K P S | 1 | |
| 56 | R H R L L E K I R | 1 | |
| 97 | T T A L L E Q L E | 1 | |
| 105 | E E T T R E G E R | 1 | |
| 106 | E T T R E G E R R | 1 | |
| 111 | G E R R E Q V L K | 1 | |
| 125 | K D V L K Q Q L S | 1 | |
| 135 | A T S R I A E L E | 1 | |
| 154 | T V A P N C F N S | 1 | |
| 162 | S S I N N I H E M | 1 | |
| 165 | N N I H E M E I Q | 1 | |
| 171 | E I Q L K D A L E | 1 | |
| 175 | K D A L E K N Q Q | 1 | |
| 184 | W L V Y D Q Q R E | 1 | |

TABLE XXXIV 121P2A3 v.1: HLA Peptide Scoring Results B*5101 9-mers SYFPEITHI

| Pos | 1 | 2 | 3 | 4 | 5 | 6 | 7 | 8 | 9 | score | SEQ. ID NO. |
|---|---|---|---|---|---|---|---|---|---|---|---|
| 206 | E | K | K | T | E | T | A | A | H | 1 | |
| 219 | Q | T | K | K | P | E | S | E | G | 1 | |
| 220 | T | K | K | P | E | S | E | G | Y | 1 | |
| 230 | Q | E | E | K | Q | K | C | Y | N | 1 | |
| 231 | E | E | K | Q | K | C | Y | N | D | 1 | |
| 234 | Q | K | C | Y | N | D | L | L | A | 1 | |
| 251 | E | R | Q | T | I | T | Q | L | S | 1 | |
| 257 | Q | L | S | F | E | L | S | E | F | 1 | |
| 263 | S | E | F | R | R | K | Y | E | E | 1 | |
| 264 | E | F | R | R | K | Y | E | E | T | 1 | |
| 266 | R | R | K | Y | E | E | T | Q | K | 1 | |
| 285 | S | Q | R | R | A | D | V | Q | H | 1 | |
| 289 | A | D | V | Q | H | L | E | D | D | 1 | |
| 295 | E | D | D | R | H | K | T | E | K | 1 | |
| 303 | K | I | Q | K | L | R | E | E | N | 1 | |
| 314 | A | R | G | K | L | E | E | E | K | 1 | |
| 322 | K | K | R | S | E | E | L | L | S | 1 | |
| 354 | Q | Q | M | Q | A | C | T | L | D | 1 | |
| 358 | A | C | T | L | D | F | E | N | E | 1 | |
| 367 | K | L | D | R | Q | H | V | Q | H | 1 | |
| 408 | P | L | V | T | F | Q | G | E | T | 1 | |
| 419 | R | E | K | V | A | A | S | P | K | 1 | |

TABLE XXXV 121P2A3 v.1: HLA Peptide Scoring Results A1 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 438 | V | E | C | P | K | C | N | I | Q | Y | 25 | |
| 452 | H | R | D | L | L | V | H | V | E | Y | 25 | |
| 449 | A | T | E | H | R | D | L | L | V | H | 24 | |
| 121 | L | S | E | E | K | D | V | L | K | Q | 23 | |
| 275 | E | V | H | N | L | N | Q | L | L | Y | 23 | |
| 178 | L | E | K | N | Q | Q | W | L | V | Y | 22 | |
| 300 | K | T | E | K | I | Q | K | L | R | E | 22 | |
| 219 | Q | T | K | K | P | E | S | E | G | Y | 21 | |
| 77 | L | T | E | K | D | K | E | I | Q | R | 20 | |
| 405 | I | T | E | P | L | V | T | F | Q | G | 20 | |
| 260 | F | E | L | S | E | F | R | R | K | Y | 19 | |
| 51 | L | T | D | K | E | R | H | R | L | L | 18 | |
| 59 | L | L | E | K | I | R | V | L | E | A | 18 | |
| 167 | I | H | E | M | E | I | Q | L | K | D | 18 | |
| 208 | K | T | E | T | A | A | H | S | L | P | 18 | |
| 228 | Y | L | Q | E | E | K | Q | K | C | Y | 18 | |
| 327 | E | L | L | S | Q | V | Q | F | L | Y | 18 | |
| 185 | L | V | Y | D | Q | Q | R | E | V | Y | 17 | |
| 393 | Q | L | E | S | L | K | Q | L | H | E | 17 | |
| 22 | K | S | E | T | T | L | E | K | L | K | 16 | |
| 53 | D | K | E | R | H | R | L | L | E | K | 16 | |
| 86 | R | L | R | D | Q | L | K | A | R | Y | 16 | |
| 222 | K | P | E | S | E | G | Y | L | Q | E | 16 | |
| 415 | E | T | E | N | R | E | K | V | A | A | 16 | |
| 66 | L | E | A | E | K | E | K | N | A | Y | 15 | |
| 224 | E | S | E | G | Y | L | Q | E | E | K | 15 | |
| 259 | S | F | E | L | S | E | F | R | R | K | 15 | |
| 324 | R | S | E | E | L | L | S | Q | V | Q | 15 | |
| 40 | S | V | D | E | I | T | S | G | K | G | 14 | |

TABLE XXXV 121P2A3 v.1: HLA Peptide Scoring Results A1 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 141 | E | L | E | S | K | T | N | T | L | R | 14 | |
| 177 | A | L | E | K | N | Q | Q | W | L | V | 14 | |
| 237 | Y | N | D | L | L | A | S | A | K | K | 14 | |
| 262 | L | S | E | F | R | R | K | Y | E | E | 14 | |
| 44 | I | T | S | G | K | G | K | L | T | D | 13 | |
| 135 | A | T | S | R | I | A | E | L | E | S | 13 | |
| 310 | E | N | D | I | A | R | G | K | L | E | 13 | |
| 343 | Q | E | E | Q | T | R | V | A | L | L | 13 | |
| 360 | T | L | D | F | E | N | E | K | L | D | 13 | |
| 413 | Q | G | E | T | E | N | R | E | K | V | 13 | |
| 2 | S | S | R | S | T | K | D | L | I | K | 12 | |
| 26 | T | L | E | K | L | K | G | E | I | A | 12 | |
| 41 | V | D | E | I | T | S | G | K | G | K | 12 | |
| 65 | V | L | E | A | E | K | E | K | N | A | 12 | |
| 69 | E | K | E | K | N | A | Y | Q | L | T | 12 | |
| 79 | E | K | D | K | E | I | Q | R | L | R | 12 | |
| 97 | T | T | A | L | L | E | Q | L | E | E | 12 | |
| 103 | Q | L | E | E | T | T | R | E | G | E | 12 | |
| 108 | T | R | E | G | E | R | R | E | Q | V | 12 | |
| 110 | E | G | E | R | R | E | Q | V | L | K | 12 | |
| 113 | R | R | E | Q | V | L | K | A | L | S | 12 | |
| 122 | S | E | E | K | D | V | L | K | Q | Q | 12 | |
| 124 | E | K | D | V | L | K | Q | Q | L | S | 12 | |
| 190 | Q | R | E | V | Y | V | K | G | L | L | 12 | |
| 255 | I | T | Q | L | S | F | E | L | S | E | 12 | |
| 269 | Y | E | E | T | Q | K | E | V | H | N | 12 | |
| 295 | E | D | D | R | H | K | T | E | K | I | 12 | |
| 325 | S | E | E | L | L | S | Q | V | Q | F | 12 | |
| 367 | K | L | D | R | Q | H | V | Q | H | Q | 12 | |
| 67 | E | A | E | K | E | K | N | A | Y | Q | 11 | |
| 100 | L | L | E | Q | L | E | E | T | T | R | 11 | |
| 186 | V | Y | D | Q | Q | R | E | V | Y | V | 11 | |
| 204 | E | L | E | K | K | T | E | T | A | A | 11 | |
| 247 | D | L | E | V | E | R | Q | T | I | T | 11 | |
| 268 | K | Y | E | E | T | Q | K | E | V | H | 11 | |
| 293 | H | L | E | D | D | R | H | K | T | E | 11 | |
| 317 | K | L | E | E | E | K | K | R | S | E | 11 | |
| 318 | L | E | E | E | K | K | R | S | E | E | 11 | |
| 342 | Q | Q | E | E | Q | T | R | V | A | L | 11 | |
| 351 | L | L | E | Q | Q | M | Q | A | C | T | 11 | |
| 364 | E | N | E | K | L | D | R | Q | H | V | 11 | |
| 5 | S | T | K | D | L | I | K | S | K | W | 10 | |
| 6 | T | K | D | L | I | K | S | K | W | G | 10 | |
| 20 | N | S | K | S | E | T | T | L | E | K | 10 | |
| 23 | S | E | T | T | L | E | K | L | K | G | 10 | |
| 31 | K | G | E | I | A | H | L | K | T | S | 10 | |
| 81 | D | K | E | I | Q | R | L | R | D | Q | 10 | |
| 87 | L | R | D | Q | L | K | A | R | Y | S | 10 | |
| 96 | S | T | T | A | L | L | E | Q | L | E | 10 | |
| 104 | L | E | E | T | T | R | E | G | E | R | 10 | |
| 139 | I | A | E | L | E | S | K | T | N | T | 10 | |
| 169 | E | M | E | I | Q | L | K | D | A | L | 10 | |
| 174 | L | K | D | A | L | E | K | N | Q | Q | 10 | |
| 202 | I | F | E | L | E | K | K | T | E | T | 10 | |
| 214 | H | S | L | P | Q | Q | T | K | K | P | 10 | |
| 229 | L | Q | E | E | K | Q | K | C | Y | N | 10 | |

| TABLE XXXV 121P2A3 v.1: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 230 | Q | E | E | K | Q | K | C | Y | N | D | 10 | |
| 245 | K | K | D | L | E | V | E | R | Q | T | 10 | |
| 249 | E | V | E | R | Q | T | I | T | Q | L | 10 | |
| 273 | Q | K | E | V | H | N | L | N | Q | L | 10 | |
| 288 | R | A | D | V | Q | H | L | E | D | D | 10 | |
| 294 | L | E | D | D | R | H | K | T | E | K | 10 | |
| 307 | L | R | E | E | N | D | I | A | R | G | 10 | |
| 308 | R | E | E | N | D | I | A | R | G | K | 10 | |
| 319 | E | E | E | K | K | R | S | E | E | L | 10 | |
| 328 | L | L | S | Q | V | Q | F | L | Y | T | 10 | |
| 362 | D | F | E | N | E | K | L | D | R | Q | 10 | |
| 381 | L | K | E | L | R | K | A | R | N | Q | 10 | |
| 400 | L | H | E | F | A | I | T | E | P | L | 10 | |
| 418 | N | R | E | K | V | A | A | S | P | K | 10 | |
| 426 | P | K | S | P | T | A | A | L | N | E | 10 | |
| 433 | L | N | E | S | L | V | E | C | P | K | 10 | |
| 437 | L | V | E | C | P | K | C | N | I | Q | 10 | |
| 52 | T | D | K | E | R | H | R | L | L | E | 9 | |
| 93 | A | R | Y | S | T | T | A | L | L | E | 9 | |
| 111 | G | E | R | R | E | Q | V | L | K | A | 9 | |
| 132 | L | S | A | A | T | S | R | I | A | E | 9 | |
| 144 | S | K | T | N | T | L | R | L | S | Q | 9 | |
| 191 | R | E | V | Y | V | K | G | L | L | A | 9 | |
| 332 | V | Q | F | L | Y | T | S | L | L | K | 9 | |
| 359 | C | T | L | D | F | E | N | E | K | L | 9 | |
| 3 | S | R | S | T | K | D | L | I | K | S | 8 | |
| 15 | G | S | K | P | S | N | S | K | S | E | 8 | |
| 30 | L | K | G | E | I | A | H | L | K | T | 8 | |
| 84 | I | Q | R | L | R | D | Q | L | K | A | 8 | |
| 233 | K | Q | K | C | Y | N | D | L | L | A | 8 | |
| 271 | E | T | Q | K | E | V | H | N | L | N | 8 | |
| 321 | E | K | K | R | S | E | E | L | L | S | 8 | |
| 333 | Q | F | L | Y | T | S | L | L | K | Q | 8 | |
| 336 | Y | T | S | L | L | K | Q | Q | E | E | 8 | |
| 344 | E | E | Q | T | R | V | A | L | L | E | 8 | |
| 373 | V | Q | H | Q | L | H | V | I | L | K | 8 | |
| 374 | Q | H | Q | L | H | V | I | L | K | E | 8 | |
| 390 | Q | I | T | Q | L | E | S | L | K | Q | 8 | |
| 429 | P | T | A | A | L | N | E | S | L | V | 8 | |
| 448 | P | A | T | E | H | R | D | L | L | V | 8 | |
| 80 | K | D | K | E | I | Q | R | L | R | D | 7 | |
| 107 | T | T | R | E | G | E | R | R | E | Q | 7 | |
| 147 | N | T | L | R | L | S | Q | T | V | A | 7 | |
| 154 | T | V | A | P | N | C | F | N | S | S | 7 | |
| 162 | S | S | I | N | N | I | H | E | M | E | 7 | |
| 198 | L | L | A | K | I | F | E | L | E | K | 7 | |
| 272 | T | Q | K | E | V | H | N | L | N | Q | 7 | |
| 276 | V | H | N | L | N | Q | L | L | Y | S | 7 | |
| 387 | A | R | N | Q | I | T | Q | L | E | S | 7 | |
| 402 | E | F | A | I | T | E | P | L | V | T | 7 | |
| 410 | V | T | F | Q | G | E | T | E | N | R | 7 | |
| 430 | T | A | A | L | N | E | S | L | V | E | 7 | |
| 1 | M | S | S | R | S | T | K | D | L | I | 6 | |
| 24 | E | T | T | L | E | K | L | K | G | E | 6 | |
| 25 | T | T | L | E | K | L | K | G | E | I | 6 | |
| 38 | K | T | S | V | D | E | I | T | S | G | 6 | |
| 70 | K | E | K | N | A | Y | Q | L | T | E | 6 | |
| 94 | R | Y | S | T | T | A | L | L | E | Q | 6 | |
| 95 | Y | S | T | T | A | L | L | E | Q | L | 6 | |
| 106 | E | T | T | R | E | G | E | R | R | E | 6 | |
| 114 | R | E | Q | V | L | K | A | L | S | E | 6 | |
| 125 | K | D | V | L | K | Q | Q | L | S | A | 6 | |
| 142 | L | E | S | K | T | N | T | L | R | L | 6 | |
| 143 | E | S | K | T | N | T | L | R | L | S | 6 | |
| 145 | K | T | N | T | L | R | L | S | Q | T | 6 | |
| 153 | Q | T | V | A | P | N | C | F | N | S | 6 | |
| 160 | F | N | S | S | I | N | N | I | H | E | 6 | |
| 171 | E | I | Q | L | K | D | A | L | E | K | 6 | |
| 192 | E | V | Y | V | K | G | L | L | A | K | 6 | |
| 197 | G | L | L | A | K | I | F | E | L | E | 6 | |
| 209 | T | E | T | A | A | H | S | L | P | Q | 6 | |
| 210 | E | T | A | A | H | S | L | P | Q | Q | 6 | |
| 234 | Q | K | C | Y | N | D | L | L | A | S | 6 | |
| 241 | L | A | S | A | K | K | D | L | E | V | 6 | |
| 242 | A | S | A | K | K | D | L | E | V | E | 6 | |
| 251 | E | R | Q | T | I | T | Q | L | S | F | 6 | |
| 253 | Q | T | I | T | Q | L | S | F | E | L | 6 | |
| 284 | Y | S | Q | R | R | A | D | V | Q | H | 6 | |
| 287 | R | R | A | D | V | Q | H | L | E | D | 6 | |
| 311 | N | D | I | A | R | G | K | L | E | E | 6 | |
| 322 | K | K | R | S | E | E | L | L | S | Q | 6 | |
| 345 | E | Q | T | R | V | A | L | L | E | Q | 6 | |
| 346 | Q | T | R | V | A | L | L | E | Q | Q | 6 | |
| 354 | Q | Q | M | Q | A | C | T | L | D | F | 6 | |
| 361 | L | D | F | E | N | E | K | L | D | R | 6 | |
| 370 | R | Q | H | V | Q | H | Q | L | H | V | 6 | |
| 377 | L | H | V | I | L | K | E | L | R | K | 6 | |
| 391 | I | T | Q | L | E | S | L | K | Q | L | 6 | |
| 46 | S | G | K | G | K | L | T | D | K | E | 5 | |
| 195 | V | K | G | L | L | A | K | I | F | E | 5 | |
| 254 | T | I | T | Q | L | S | F | E | L | S | 5 | |
| 306 | K | L | R | E | E | N | D | I | A | R | 5 | |
| 423 | A | A | S | P | K | S | P | T | A | A | 5 | |
| 424 | A | S | P | K | S | P | T | A | A | L | 5 | |
| 447 | Y | P | A | T | E | H | R | D | L | L | 5 | |
| 4 | R | S | T | K | D | L | I | K | S | K | 4 | |
| 11 | K | S | K | W | G | S | K | P | S | N | 4 | |
| 18 | P | S | N | S | K | S | E | T | T | L | 4 | |
| 19 | S | N | S | K | S | E | T | T | L | E | 4 | |
| 21 | S | K | S | E | T | T | L | E | K | L | 4 | |
| 27 | L | E | K | L | K | G | E | I | A | H | 4 | |
| 29 | K | L | K | G | E | I | A | H | L | K | 4 | |
| 37 | L | K | T | S | V | D | E | I | T | S | 4 | |
| 39 | T | S | V | D | E | I | T | S | G | K | 4 | |
| 45 | T | S | G | K | G | K | L | T | D | K | 4 | |
| 56 | R | H | R | L | L | E | K | I | R | V | 4 | |
| 58 | R | L | L | E | K | I | R | V | L | E | 4 | |
| 117 | V | L | K | A | L | S | E | E | K | D | 4 | |
| 120 | A | L | S | E | E | K | D | V | L | K | 4 | |
| 136 | T | S | R | I | A | E | L | E | S | K | 4 | |
| 137 | S | R | I | A | E | L | E | S | K | T | 4 | |
| 151 | L | S | Q | T | V | A | P | N | C | F | 4 | |

TABLE XXXV 121P2A3 v.1: HLA Peptide Scoring Results A1 10-mers SYFPEITHI

| Pos | 1 2 3 4 5 6 7 8 9 0 | score | SEQ. ID NO. |
|---|---|---|---|
| 157 | P N C F N S S I N N | 4 | |
| 161 | N S S I N N I H E M | 4 | |
| 165 | N N I H E M E I Q L | 4 | |
| 187 | Y D Q Q R E V Y V K | 4 | |
| 220 | T K K P E S E G Y L | 4 | |
| 225 | S E G Y L Q E E K Q | 4 | |
| 248 | L E V E R Q T I T Q | 4 | |
| 258 | L S F E L S E F R R | 4 | |
| 297 | D R H K T E K I Q K | 4 | |
| 329 | L S Q V Q F L Y T S | 4 | |
| 337 | T S L L K Q Q E E Q | 4 | |
| 384 | L R K A R N Q I T Q | 4 | |
| 395 | E S L K Q L H E F A | 4 | |
| 398 | K Q L H E F A I T E | 4 | |
| 425 | S P K S P T A A L N | 4 | |
| 427 | K S P T A A L N E S | 4 | |
| 435 | E S L V E C P K C N | 4 | |
| 445 | I Q Y P A T E H R D | 4 | |
| 9 | L I K S K W G S K P | 3 | |
| 12 | S K W G S K P S N S | 3 | |
| 14 | W G S K P S N S K S | 3 | |
| 43 | E I T S G K G K L T | 3 | |
| 74 | A Y Q L T E K D K E | 3 | |
| 134 | A A T S R I A E L E | 3 | |
| 152 | S Q T V A P N C F N | 3 | |
| 193 | V Y V K G L L A K I | 3 | |
| 194 | Y V K G L L A K I F | 3 | |
| 200 | A K I F E L E K K T | 3 | |
| 213 | A H S L P Q Q T K K | 3 | |
| 215 | S L P Q Q T K K P E | 3 | |
| 221 | K K P E S E G Y L Q | 3 | |
| 232 | E K Q K C Y N D L L | 3 | |
| 240 | L L A S A K K D L E | 3 | |
| 250 | V E R Q T I T Q L S | 3 | |
| 274 | K E V H N L N Q L L | 3 | |
| 285 | S Q R R A D V Q H L | 3 | |
| 292 | Q H L E D D R H K T | 3 | |
| 309 | E E N D I A R G K L | 3 | |
| 314 | A R G K L E E E K K | 3 | |
| 338 | S L L K Q Q E E Q T | 3 | |
| 357 | Q A C T L D F E N E | 3 | |
| 376 | Q L H V I L K E L R | 3 | |
| 396 | S L K Q L H E F A I | 3 | |
| 397 | L K Q L H E F A I T | 3 | |
| 406 | T E P L V T F Q G E | 3 | |
| 428 | S P T A A L N E S L | 3 | |
| 432 | A L N E S L V E C P | 3 | |
| 436 | S L V E C P K C N I | 3 | |
| 16 | S K P S N S K S E T | 2 | |
| 35 | A H L K T S V D E I | 2 | |
| 42 | D E I T S G K G K L | 2 | |
| 50 | K L T D K E R H R L | 2 | |
| 54 | K E R H R L L E K I | 2 | |
| 62 | K I R V L E A E K E | 2 | |
| 64 | R V L E A E K E K N | 2 | |
| 72 | K N A Y Q L T E K D | 2 | |
| 75 | Y Q L T E K D K E I | 2 | |
| 83 | E I Q R L R D Q L K | 2 | |
| 92 | K A R Y S T T A L L | 2 | |
| 99 | A L L E Q L E E T T | 2 | |
| 127 | V L K Q Q L S A A T | 2 | |
| 133 | S A A T S R I A E L | 2 | |
| 155 | V A P N C F N S S I | 2 | |
| 163 | S I N N I H E M E I | 2 | |
| 166 | N I H E M E I Q L K | 2 | |
| 170 | M E I Q L K D A L E | 2 | |
| 172 | I Q L K D A L E K N | 2 | |
| 188 | D Q Q R E V Y V K G | 2 | |
| 199 | L A K I F E L E K K | 2 | |
| 227 | G Y L Q E E K Q K C | 2 | |
| 238 | N D L L A S A K K D | 2 | |
| 243 | S A K K D L E V E R | 2 | |
| 244 | A K K D L E V E R Q | 2 | |
| 261 | E L S E F R R K Y E | 2 | |
| 263 | S E F R R K Y E E T | 2 | |
| 266 | R R K Y E E T Q K E | 2 | |
| 281 | Q L L Y S Q R R A D | 2 | |
| 282 | L L Y S Q R R A D V | 2 | |
| 286 | Q R R A D V Q H L E | 2 | |
| 298 | R H K T E K I Q K L | 2 | |
| 299 | H K T E K I Q K L R | 2 | |
| 315 | R G K L E E E K K R | 2 | |
| 320 | E E K K R S E E L L | 2 | |
| 330 | S Q V Q F L Y T S L | 2 | |
| 331 | Q V Q F L Y T S L L | 2 | |
| 334 | F L Y T S L L K Q Q | 2 | |
| 350 | A L L E Q Q M Q A C | 2 | |
| 353 | E Q Q M Q A C T L D | 2 | |
| 369 | D R Q H V Q H Q L H | 2 | |
| 378 | H V I L K E L R K A | 2 | |
| 379 | V I L K E L R K A R | 2 | |
| 383 | E L R K A R N Q I T | 2 | |
| 386 | K A R N Q I T Q L E | 2 | |
| 389 | N Q I T Q L E S L K | 2 | |
| 392 | T Q L E S L K Q L H | 2 | |
| 401 | H E F A I T E P L V | 2 | |
| 404 | A I T E P L V T F Q | 2 | |
| 408 | P L V T F Q G E T E | 2 | |
| 412 | F Q G E T E N R E K | 2 | |
| 419 | R E K V A A S P K S | 2 | |
| 434 | N E S L V E C P K C | 2 | |
| 451 | E H R D L L V H V E | 2 | |
| 8 | D L I K S K W G S K | 1 | |
| 33 | E I A H L K T S V D | 1 | |
| 36 | H L K T S V D E I T | 1 | |
| 57 | H R L L E K I R V L | 1 | |
| 68 | A E K E K N A Y Q L | 1 | |
| 76 | Q L T E K D K E I Q | 1 | |
| 82 | K E I Q R L R D Q L | 1 | |
| 88 | R D Q L K A R Y S T | 1 | |
| 90 | Q L K A R Y S T T A | 1 | |
| 105 | E E T T R E G E R R | 1 | |

| TABLE XXXV 121P2A3 v.1: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 109 | R | E | G | E | R | R | E | Q | V | L | 1 | |
| 119 | K | A | L | S | E | E | K | D | V | L | 1 | |
| 131 | Q | L | S | A | A | T | S | R | I | A | 1 | |
| 140 | A | E | L | E | S | K | T | N | T | L | 1 | |
| 148 | T | L | R | L | S | Q | T | V | A | P | 1 | |
| 149 | L | R | L | S | Q | T | V | A | P | N | 1 | |
| 150 | R | L | S | Q | T | V | A | P | N | C | 1 | |
| 156 | A | P | N | C | F | N | S | S | I | N | 1 | |
| 159 | C | F | N | S | S | I | N | N | I | H | 1 | |
| 173 | Q | L | K | D | A | L | E | K | N | Q | 1 | |
| 175 | K | D | A | L | E | K | N | Q | Q | W | 1 | |
| 180 | K | N | Q | Q | W | L | V | Y | D | Q | 1 | |
| 184 | W | L | V | Y | D | Q | Q | R | E | V | 1 | |
| 189 | Q | Q | R | E | V | Y | V | K | G | L | 1 | |
| 203 | F | E | L | E | K | K | T | E | T | A | 1 | |
| 212 | A | A | H | S | L | P | Q | Q | T | K | 1 | |
| 239 | D | L | L | A | S | A | K | K | D | L | 1 | |
| 246 | K | D | L | E | V | E | R | Q | T | I | 1 | |
| 257 | Q | L | S | F | E | L | S | E | F | R | 1 | |
| 265 | F | R | R | K | Y | E | E | T | Q | K | 1 | |
| 270 | E | E | T | Q | K | E | V | H | N | L | 1 | |
| 278 | N | L | N | Q | L | L | Y | S | Q | R | 1 | |
| 283 | L | Y | S | Q | R | R | A | D | V | Q | 1 | |
| 289 | A | D | V | Q | H | L | E | D | D | R | 1 | |
| 291 | V | Q | H | L | E | D | D | R | H | K | 1 | |
| 302 | E | K | I | Q | K | L | R | E | E | N | 1 | |
| 305 | Q | K | L | R | E | E | N | D | I | A | 1 | |
| 313 | I | A | R | G | K | L | E | E | E | K | 1 | |
| 326 | E | E | L | L | S | Q | V | Q | F | L | 1 | |
| 339 | L | L | K | Q | Q | E | E | Q | T | R | 1 | |
| 341 | K | Q | Q | E | E | Q | T | R | V | A | 1 | |
| 348 | R | V | A | L | L | E | Q | Q | M | Q | 1 | |
| 349 | V | A | L | L | E | Q | Q | M | Q | A | 1 | |
| 358 | A | C | T | L | D | F | E | N | E | K | 1 | |
| 363 | F | E | N | E | K | L | D | R | Q | H | 1 | |
| 365 | N | E | K | L | D | R | Q | H | V | Q | 1 | |
| 371 | Q | H | V | Q | H | Q | L | H | V | I | 1 | |
| 380 | I | L | K | E | L | R | K | A | R | N | 1 | |
| 382 | K | E | L | R | K | A | R | N | Q | I | 1 | |
| 399 | Q | L | H | E | F | A | I | T | E | P | 1 | |
| 403 | F | A | I | T | E | P | L | V | T | F | 1 | |
| 411 | T | F | Q | G | E | T | E | N | R | E | 1 | |
| 414 | G | E | T | E | N | R | E | K | V | A | 1 | |
| 416 | T | E | N | R | E | K | V | A | A | S | 1 | |
| 422 | V | A | A | S | P | K | S | P | T | A | 1 | |
| 431 | A | A | L | N | E | S | L | V | E | C | 1 | |
| 453 | R | D | L | L | V | H | V | E | Y | C | 1 | |
| 454 | D | L | L | V | H | V | E | Y | C | S | 1 | |
| 455 | L | L | V | H | V | E | Y | C | S | K | 1 | |

| TABLE XXXV 121P2A3 v.3: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 6 | L | T | D | K | E | R | Q | R | L | L | 18 | |

| TABLE XXXV 121P2A3 v.3: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 8 | D | K | E | R | Q | R | L | L | E | K | 16 | |
| 7 | T | D | K | E | R | Q | R | L | L | E | 9 | |
| 1 | S | G | K | G | K | L | T | D | K | E | 5 | |
| 11 | R | Q | R | L | L | E | K | I | R | V | 4 | |
| 5 | K | L | T | D | K | E | R | Q | R | L | 2 | |
| 9 | K | E | R | Q | R | L | L | E | K | I | 2 | |
| 12 | Q | R | L | L | E | K | I | R | V | L | 1 | |

| TABLE XXXV 121P2A3 v.4: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 8 | T | T | T | L | L | E | Q | L | E | E | 12 | |
| 7 | S | T | T | T | L | L | E | Q | L | E | 10 | |
| 4 | A | R | Y | S | T | T | T | L | L | E | 9 | |
| 5 | R | Y | S | T | T | T | L | L | E | Q | 6 | |
| 6 | Y | S | T | T | T | L | L | E | Q | L | 6 | |
| 9 | T | T | L | L | E | Q | L | E | E | T | 6 | |
| 3 | K | A | R | Y | S | T | T | T | L | L | 2 | |
| 1 | Q | L | K | A | R | Y | S | T | T | T | 1 | |
| 10 | T | L | L | E | Q | L | E | E | T | T | 1 | |

| TABLE XXXV 121P2A3 v.6: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 3 | E | L | L | S | Q | V | Q | S | L | Y | 18 | |
| 1 | S | E | E | L | L | S | Q | V | Q | S | 12 | |
| 9 | Q | S | L | Y | T | S | L | L | K | Q | 12 | |
| 8 | V | Q | S | L | Y | T | S | L | L | K | 9 | |
| 4 | L | L | S | Q | V | Q | S | L | Y | T | 7 | |
| 5 | L | S | Q | V | Q | S | L | Y | T | S | 4 | |
| 10 | S | L | Y | T | S | L | L | K | Q | Q | 3 | |
| 6 | S | Q | V | Q | S | L | Y | T | S | L | 2 | |
| 7 | Q | V | Q | S | L | Y | T | S | L | L | 2 | |
| 2 | E | E | L | L | S | Q | V | Q | S | L | 1 | |

| TABLE XXXV 121P2A3 v.7: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 2 | R | Q | H | V | Q | H | Q | L | L | V | 8 | |
| 5 | V | Q | H | Q | L | L | V | I | L | K | 8 | |
| 6 | Q | H | Q | L | L | V | I | L | K | E | 8 | |
| 3 | Q | H | V | Q | H | Q | L | L | V | I | 7 | |
| 9 | L | L | V | I | L | K | E | L | R | K | 7 | |
| 8 | Q | L | L | V | I | L | K | E | L | R | 3 | |
| 1 | D | R | Q | H | V | Q | H | Q | L | L | 2 | |
| 10 | L | V | I | L | K | E | L | R | K | A | 2 | |

| TABLE XXXV 121P2A3 v.8: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 1 | P | K | S | P | T | A | A | L | N | G | 10 | |

| TABLE XXXV 121P2A3 v.8: HLA Peptide Scoring Results A1 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 4 | P | T | A | A | L | N | G | S | L | V | 9 | |
| 5 | T | A | A | L | N | G | S | L | V | E | 7 | |
| 2 | K | S | P | T | A | A | L | N | G | S | 4 | |
| 7 | A | L | N | G | S | L | V | E | C | P | 4 | |
| 10 | G | S | L | V | E | C | P | K | C | N | 4 | |
| 3 | S | P | T | A | A | L | N | G | S | L | 3 | |
| 9 | N | G | S | L | V | E | C | P | K | C | 2 | |
| 6 | A | A | L | N | G | S | L | V | E | C | 1 | |

| TABLE XXXVI 121P2A3 v.1: HLA Peptide Scoring Results A*0201 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 133 | S | A | A | T | S | R | I | A | E | L | 26 | |
| 282 | L | L | Y | S | Q | R | R | A | D | V | 25 | |
| 50 | K | L | T | D | K | E | R | H | R | L | 22 | |
| 59 | L | L | E | K | I | R | V | L | E | A | 22 | |
| 99 | A | L | L | E | Q | L | E | E | T | T | 22 | |
| 436 | S | L | V | E | C | P | K | C | N | I | 22 | |
| 163 | S | I | N | N | I | H | E | M | E | I | 21 | |
| 177 | A | L | E | K | N | Q | Q | W | L | V | 21 | |
| 184 | W | L | V | Y | D | Q | Q | R | E | V | 21 | |
| 21 | S | K | S | E | T | T | L | E | K | L | 20 | |
| 239 | D | L | L | A | S | A | K | K | D | L | 20 | |
| 396 | S | L | K | Q | L | H | E | F | A | I | 20 | |
| 140 | A | E | L | E | S | K | T | N | T | L | 19 | |
| 196 | K | G | L | L | A | K | I | F | E | L | 19 | |
| 241 | L | A | S | A | K | K | D | L | E | V | 19 | |
| 359 | C | T | L | D | F | E | N | E | K | L | 19 | |
| 391 | I | T | Q | L | E | S | L | K | Q | L | 19 | |
| 432 | A | L | N | E | S | L | V | E | C | P | 19 | |
| 25 | T | T | L | E | K | L | K | G | E | I | 18 | |
| 35 | A | H | L | K | T | S | V | D | E | I | 18 | |
| 253 | Q | T | I | T | Q | L | S | F | E | L | 18 | |
| 338 | S | L | L | K | Q | Q | E | E | Q | T | 18 | |
| 399 | Q | L | H | E | F | A | I | T | E | P | 18 | |
| 119 | K | A | L | S | E | E | K | D | V | L | 17 | |
| 127 | V | L | K | Q | Q | L | S | A | A | T | 17 | |
| 176 | D | A | L | E | K | N | Q | Q | W | L | 17 | |
| 193 | V | Y | V | K | G | L | L | A | K | I | 17 | |
| 198 | L | L | A | K | I | F | E | L | E | K | 17 | |
| 323 | K | R | S | E | E | L | L | S | Q | V | 17 | |
| 328 | L | L | S | Q | V | Q | F | L | Y | T | 17 | |
| 350 | A | L | L | E | Q | Q | M | Q | A | C | 17 | |
| 351 | L | L | E | Q | Q | M | Q | A | C | T | 17 | |
| 375 | H | Q | L | H | V | I | L | K | E | L | 17 | |
| 51 | L | T | D | K | E | R | H | R | L | L | 16 | |
| 57 | H | R | L | L | E | K | I | R | V | L | 16 | |
| 58 | R | L | L | E | K | I | R | V | L | E | 16 | |
| 92 | K | A | R | Y | S | T | T | A | L | L | 16 | |
| 98 | T | A | L | L | E | Q | L | E | E | T | 16 | |
| 120 | A | L | S | E | E | K | D | V | L | K | 16 | |
| 189 | Q | Q | R | E | V | Y | V | K | G | L | 16 | |
| 285 | S | Q | R | R | A | D | V | Q | H | L | 16 | |
| 372 | H | V | Q | H | Q | L | H | V | I | L | 16 | |

| TABLE XXXVI 121P2A3 v.1: HLA Peptide Scoring Results A*0201 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 378 | H | V | I | L | K | E | L | R | K | A | 16 | |
| 385 | R | K | A | R | N | Q | I | T | Q | L | 16 | |
| 388 | R | N | Q | I | T | Q | L | E | S | L | 16 | |
| 431 | A | A | L | N | E | S | L | V | E | C | 16 | |
| 450 | T | E | H | R | D | L | L | V | H | V | 16 | |
| 28 | E | K | L | K | G | E | I | A | H | L | 15 | |
| 32 | G | E | I | A | H | L | K | T | S | V | 15 | |
| 54 | K | E | R | H | R | L | L | E | K | I | 15 | |
| 90 | Q | L | K | A | R | Y | S | T | T | A | 15 | |
| 91 | L | K | A | R | Y | S | T | T | A | L | 15 | |
| 95 | Y | S | T | T | A | L | L | E | Q | L | 15 | |
| 155 | V | A | P | N | C | F | N | S | S | I | 15 | |
| 169 | E | M | E | I | Q | L | K | D | A | L | 15 | |
| 207 | K | K | T | E | T | A | A | H | S | L | 15 | |
| 246 | K | D | L | E | V | E | R | Q | T | I | 15 | |
| 298 | R | H | K | T | E | K | I | Q | K | L | 15 | |
| 312 | D | I | A | R | G | K | L | E | E | E | 15 | |
| 334 | F | L | Y | T | S | L | L | K | Q | Q | 15 | |
| 343 | Q | E | E | Q | T | R | V | A | L | L | 15 | |
| 367 | K | L | D | R | Q | H | V | Q | H | Q | 15 | |
| 380 | I | L | K | E | L | R | K | A | R | N | 15 | |
| 403 | F | A | I | T | E | P | L | V | T | F | 15 | |
| 404 | A | I | T | E | P | L | V | T | F | Q | 15 | |
| 424 | A | S | P | K | S | P | T | A | A | L | 15 | |
| 68 | A | E | K | E | K | N | A | Y | Q | L | 14 | |
| 78 | T | E | K | D | K | E | I | Q | R | L | 14 | |
| 126 | D | V | L | K | Q | Q | L | S | A | A | 14 | |
| 131 | Q | L | S | A | A | T | S | R | I | A | 14 | |
| 145 | K | T | N | T | L | R | L | S | Q | T | 14 | |
| 158 | N | C | F | N | S | S | I | N | N | I | 14 | |
| 166 | N | I | H | E | M | E | I | Q | L | K | 14 | |
| 235 | K | C | Y | N | D | L | L | A | S | A | 14 | |
| 240 | L | L | A | S | A | K | K | D | L | E | 14 | |
| 249 | E | V | E | R | Q | T | I | T | Q | L | 14 | |
| 267 | R | K | Y | E | E | T | Q | K | E | V | 14 | |
| 273 | Q | K | E | V | H | N | L | N | Q | L | 14 | |
| 306 | K | L | R | E | E | N | D | I | A | R | 14 | |
| 317 | K | L | E | E | E | K | K | R | S | E | 14 | |
| 330 | S | Q | V | Q | F | L | Y | T | S | L | 14 | |
| 342 | Q | Q | E | E | Q | T | R | V | A | L | 14 | |
| 352 | L | E | Q | Q | M | Q | A | C | T | L | 14 | |
| 422 | V | A | A | S | P | K | S | P | T | A | 14 | |
| 447 | Y | P | A | T | E | H | R | D | L | L | 14 | |
| 455 | L | L | V | H | V | E | Y | C | S | K | 14 | |
| 8 | D | L | I | K | S | K | W | G | S | K | 13 | |
| 26 | T | L | E | K | L | K | G | E | I | A | 13 | |
| 29 | K | L | K | G | E | I | A | H | L | K | 13 | |
| 36 | H | L | K | T | S | V | D | E | I | T | 13 | |
| 42 | D | E | I | T | S | G | K | G | K | L | 13 | |
| 65 | V | L | E | A | E | K | E | K | N | A | 13 | |
| 75 | Y | Q | L | T | E | K | D | K | E | I | 13 | |
| 82 | K | E | I | Q | R | L | R | D | Q | L | 13 | |
| 86 | R | L | R | D | Q | L | K | A | R | Y | 13 | |
| 100 | L | L | E | Q | L | E | E | T | T | R | 13 | |
| 112 | E | R | R | E | Q | V | L | K | A | L | 13 | |
| 118 | L | K | A | L | S | E | E | K | D | V | 13 | |

TABLE XXXVI 121P2A3 v.1: HLA Peptide Scoring Results A*0201 10-mers SYFPEITHI

| Pos | 1 2 3 4 5 6 7 8 9 0 | score | SEQ. ID NO. |
|---|---|---|---|
| 138 | R I A E L E S K T N | 13 | |
| 142 | L E S K T N T L R L | 13 | |
| 148 | T L R L S Q T V A P | 13 | |
| 186 | V Y D Q Q R E V Y V | 13 | |
| 197 | G L L A K I F E L E | 13 | |
| 201 | K I F E L E K K T E | 13 | |
| 228 | Y L Q E E K Q K C Y | 13 | |
| 326 | E E L L S Q V Q F L | 13 | |
| 331 | Q V Q F L Y T S L L | 13 | |
| 339 | L L K Q Q E E Q T R | 13 | |
| 368 | L D R Q H V Q H Q L | 13 | |
| 371 | Q H V Q H Q L H V I | 13 | |
| 423 | A A S P K S P T A A | 13 | |
| 428 | S P T A A L N E S L | 13 | |
| 429 | P T A A L N E S L V | 13 | |
| 448 | P A T E H R D L L V | 13 | |
| 103 | Q L E E T T R E G E | 12 | |
| 109 | R E G E R R E Q V L | 12 | |
| 111 | G E R R E Q V L K A | 12 | |
| 117 | V L K A L S E E K D | 12 | |
| 165 | N N I H E M E I Q L | 12 | |
| 247 | D L E V E R Q T I T | 12 | |
| 281 | Q L L Y S Q R R A D | 12 | |
| 340 | L K Q Q E E Q T R V | 12 | |
| 355 | Q M Q A C T L D F E | 12 | |
| 382 | K E L R K A R N Q I | 12 | |
| 401 | H E F A I T E P L V | 12 | |
| 454 | D L L V H V E Y C S | 12 | |
| 9 | L I K S K W G S K P | 11 | |
| 30 | L K G E I A H L K T | 11 | |
| 38 | K T S V D E I T S G | 11 | |
| 44 | I T S G K G K L T D | 11 | |
| 62 | K I R V L E A E K E | 11 | |
| 76 | Q L T E K D K E I Q | 11 | |
| 108 | T R E G E R R E Q V | 11 | |
| 146 | T N T L R L S Q T V | 11 | |
| 150 | R L S Q T V A P N C | 11 | |
| 154 | T V A P N C F N S S | 11 | |
| 161 | N S S I N N I H E M | 11 | |
| 199 | L A K I F E L E K K | 11 | |
| 203 | F E L E K K T E T A | 11 | |
| 204 | E L E K K T E T A A | 11 | |
| 215 | S L P Q Q T K K P E | 11 | |
| 220 | T K K P E S E G Y L | 11 | |
| 270 | E E T Q K E V H N L | 11 | |
| 274 | K E V H N L N Q L L | 11 | |
| 278 | N L N Q L L Y S Q R | 11 | |
| 292 | Q H L E D D R H K T | 11 | |
| 293 | H L E D D R H K T E | 11 | |
| 309 | E E N D I A R G K L | 11 | |
| 349 | V A L L E Q Q M Q A | 11 | |
| 370 | R Q H V Q H Q L H V | 11 | |
| 379 | V I L K E L R K A R | 11 | |
| 383 | E L R K A R N Q I T | 11 | |
| 413 | Q G E T E N R E K V | 11 | |
| 421 | K V A A S P K S P T | 11 | |

TABLE XXXVI 121P2A3 v.1: HLA Peptide Scoring Results A*0201 10-mers SYFPEITHI

| Pos | 1 2 3 4 5 6 7 8 9 0 | score | SEQ. ID NO. |
|---|---|---|---|
| 18 | P S N S K S E T T L | 10 | |
| 40 | S V D E I T S G K G | 10 | |
| 56 | R H R L L E K I R V | 10 | |
| 64 | R V L E A E K E K N | 10 | |
| 121 | L S E E K D V L K Q | 10 | |
| 130 | Q Q L S A A T S R I | 10 | |
| 137 | S R I A E L E S K T | 10 | |
| 147 | N T L R L S Q T V A | 10 | |
| 149 | L R L S Q T V A P N | 10 | |
| 168 | H E M E I Q L K D A | 10 | |
| 172 | I Q L K D A L E K N | 10 | |
| 211 | T A A H S L P Q Q T | 10 | |
| 304 | I Q K L R E E N D I | 10 | |
| 313 | I A R G K L E E E K | 10 | |
| 376 | Q L H V I L K E L R | 10 | |
| 390 | Q I T Q L E S L K Q | 10 | |
| 393 | Q L E S L K Q L H E | 10 | |
| 400 | L H E F A I T E P L | 10 | |
| 446 | Q Y P A T E H R D L | 10 | |
| 449 | A T E H R D L L V H | 10 | |
| 84 | I Q R L R D Q L K A | 9 | |
| 97 | T T A L L E Q L E E | 9 | |
| 135 | A T S R I A E L E S | 9 | |
| 139 | I A E L E S K T N T | 9 | |
| 173 | Q L K D A L E K N Q | 9 | |
| 190 | Q R E V Y V K G L L | 9 | |
| 243 | S A K K D L E V E R | 9 | |
| 255 | I T Q L S F E L S E | 9 | |
| 257 | Q L S F E L S E F R | 9 | |
| 263 | S E F R R K Y E E T | 9 | |
| 277 | H N L N Q L L Y S Q | 9 | |
| 303 | K I Q K L R E E N D | 9 | |
| 307 | L R E E N D I A R G | 9 | |
| 327 | E L L S Q V Q F L Y | 9 | |
| 329 | L S Q V Q F L Y T S | 9 | |
| 394 | L E S L K Q L H E F | 9 | |
| 408 | P L V T F Q G E T E | 9 | |
| 1 | M S S R S T K D L I | 8 | |
| 3 | S R S T K D L I K S | 8 | |
| 5 | S T K D L I K S K W | 8 | |
| 16 | S K P S N S K S E T | 8 | |
| 34 | I A H L K T S V D E | 8 | |
| 66 | L E A E K E K N A Y | 8 | |
| 107 | T T R E G E R R E Q | 8 | |
| 116 | Q V L K A L S E E K | 8 | |
| 125 | K D V L K Q Q L S A | 8 | |
| 171 | E I Q L K D A L E K | 8 | |
| 185 | L V Y D Q Q R E V Y | 8 | |
| 192 | E V Y V K G L L A K | 8 | |
| 200 | A K I F E L E K K T | 8 | |
| 210 | E T A A H S L P Q Q | 8 | |
| 212 | A A H S L P Q Q T K | 8 | |
| 242 | A S A K K D L E V E | 8 | |
| 256 | T Q L S F E L S E F | 8 | |
| 261 | E L S E F R R K Y E | 8 | |
| 288 | R A D V Q H L E D D | 8 | |

| TABLE XXXVI 121P2A3 v.1: HLA Peptide Scoring Results A*0201 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 319 | E | E | E | K | K | R | S | E | E | L | 8 | |
| 322 | K | K | R | S | E | E | L | L | S | Q | 8 | |
| 333 | Q | F | L | Y | T | S | L | L | K | Q | 8 | |
| 336 | Y | T | S | L | L | K | Q | Q | E | E | 8 | |
| 346 | Q | T | R | V | A | L | L | E | Q | Q | 8 | |
| 360 | T | L | D | F | E | N | E | K | L | D | 8 | |
| 397 | L | K | Q | L | H | E | F | A | I | T | 8 | |
| 416 | T | E | N | R | E | K | V | A | A | S | 8 | |
| 427 | K | S | P | T | A | A | L | N | E | S | 8 | |
| 17 | K | P | S | N | S | K | S | E | T | T | 7 | |
| 33 | E | I | A | H | L | K | T | S | V | D | 7 | |
| 43 | E | I | T | S | G | K | G | K | L | T | 7 | |
| 88 | R | D | Q | L | K | A | R | Y | S | T | 7 | |
| 89 | D | Q | L | K | A | R | Y | S | T | T | 7 | |
| 94 | R | Y | S | T | T | A | L | L | E | Q | 7 | |
| 123 | E | E | K | D | V | L | K | Q | Q | L | 7 | |
| 187 | Y | D | Q | Q | R | E | V | Y | V | K | 7 | |
| 202 | I | F | E | L | E | K | K | T | E | T | 7 | |
| 231 | E | E | K | Q | K | C | Y | N | D | L | 7 | |
| 232 | E | K | Q | K | C | Y | N | D | L | L | 7 | |
| 254 | T | I | T | Q | L | S | F | E | L | S | 7 | |
| 276 | V | H | N | L | N | Q | L | L | Y | S | 7 | |
| 295 | E | D | D | R | H | K | T | E | K | I | 7 | |
| 341 | K | Q | Q | E | E | Q | T | R | V | A | 7 | |
| 374 | Q | H | Q | L | H | V | I | L | K | E | 7 | |
| 405 | I | T | E | P | L | V | T | F | Q | G | 7 | |
| 415 | E | T | E | N | R | E | K | V | A | A | 7 | |
| 430 | T | A | A | L | N | E | S | L | V | E | 7 | |
| 444 | N | I | Q | Y | P | A | T | E | H | R | 7 | |
| 60 | L | E | K | I | R | V | L | E | A | E | 6 | |
| 85 | Q | R | L | R | D | Q | L | K | A | R | 6 | |
| 128 | L | K | Q | Q | L | S | A | A | T | S | 6 | |
| 136 | T | S | R | I | A | E | L | E | S | K | 6 | |
| 141 | E | L | E | S | K | T | N | T | L | R | 6 | |
| 180 | K | N | Q | Q | W | L | V | Y | D | Q | 6 | |
| 194 | Y | V | K | G | L | L | A | K | I | F | 6 | |
| 237 | Y | N | D | L | L | A | S | A | K | K | 6 | |
| 244 | A | K | K | D | L | E | V | E | R | Q | 6 | |
| 248 | L | E | V | E | R | Q | T | I | T | Q | 6 | |
| 280 | N | Q | L | L | Y | S | Q | R | R | A | 6 | |
| 287 | R | R | A | D | V | Q | H | L | E | D | 6 | |
| 305 | Q | K | L | R | E | E | N | D | I | A | 6 | |
| 320 | E | E | K | K | R | S | E | E | L | L | 6 | |
| 348 | R | V | A | L | L | E | Q | Q | M | Q | 6 | |
| 361 | L | D | F | E | N | E | K | L | D | R | 6 | |
| 364 | E | N | E | K | L | D | R | Q | H | V | 6 | |
| 387 | A | R | N | Q | I | T | Q | L | E | S | 6 | |
| 409 | L | V | T | F | Q | G | E | T | E | N | 6 | |
| 410 | V | T | F | Q | G | E | T | E | N | R | 6 | |
| 437 | L | V | E | C | P | K | C | N | I | Q | 6 | |
| 442 | K | C | N | I | Q | Y | P | A | T | E | 6 | |
| 445 | I | Q | Y | P | A | T | E | H | R | D | 6 | |
| 453 | R | D | L | L | V | H | V | E | Y | C | 6 | |
| 12 | S | K | W | G | S | K | P | S | N | S | 5 | |
| 31 | K | G | E | I | A | H | L | K | T | S | 5 | |
| 46 | S | G | K | G | K | L | T | D | K | E | 5 | |

| TABLE XXXVI 121P2A3 v.1: HLA Peptide Scoring Results A*0201 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 53 | D | K | E | R | H | R | L | L | E | K | 5 | |
| 77 | L | T | E | K | D | K | E | I | Q | R | 5 | |
| 83 | E | I | Q | R | L | R | D | Q | L | K | 5 | |
| 96 | S | T | T | A | L | L | E | Q | L | E | 5 | |
| 132 | L | S | A | A | T | S | R | I | A | E | 5 | |
| 134 | A | A | T | S | R | I | A | E | L | E | 5 | |
| 214 | H | S | L | P | Q | Q | T | K | K | P | 5 | |
| 216 | L | P | Q | Q | T | K | K | P | E | S | 5 | |
| 223 | P | E | S | E | G | Y | L | Q | E | E | 5 | |
| 227 | G | Y | L | Q | E | E | K | Q | K | C | 5 | |
| 233 | K | Q | K | C | Y | N | D | L | L | A | 5 | |
| 234 | Q | K | C | Y | N | D | L | L | A | S | 5 | |
| 245 | K | K | D | L | E | V | E | R | Q | T | 5 | |
| 258 | L | S | F | E | L | S | E | F | R | R | 5 | |
| 260 | F | E | L | S | E | F | R | R | K | Y | 5 | |
| 294 | L | E | D | D | R | H | K | T | E | K | 5 | |
| 300 | K | T | E | K | I | Q | K | L | R | E | 5 | |
| 301 | T | E | K | I | Q | K | L | R | E | E | 5 | |
| 311 | N | D | I | A | R | G | K | L | E | E | 5 | |
| 318 | L | E | E | E | K | K | R | S | E | E | 5 | |
| 373 | V | Q | H | Q | L | H | V | I | L | K | 5 | |
| 386 | K | A | R | N | Q | I | T | Q | L | E | 5 | |
| 398 | K | Q | L | H | E | F | A | I | T | E | 5 | |
| 407 | E | P | L | V | T | F | Q | G | E | T | 5 | |
| 412 | F | Q | G | E | T | E | N | R | E | K | 5 | |
| 414 | G | E | T | E | N | R | E | K | V | A | 5 | |
| 4 | R | S | T | K | D | L | I | K | S | K | 4 | |
| 7 | K | D | L | I | K | S | K | W | G | S | 4 | |
| 13 | K | W | G | S | K | P | S | N | S | K | 4 | |
| 23 | S | E | T | T | L | E | K | L | K | G | 4 | |
| 24 | E | T | T | L | E | K | L | K | G | E | 4 | |
| 45 | T | S | G | K | G | K | L | T | D | K | 4 | |
| 71 | E | K | N | A | Y | Q | L | T | E | K | 4 | |
| 72 | K | N | A | Y | Q | L | T | E | K | D | 4 | |
| 73 | N | A | Y | Q | L | T | E | K | D | K | 4 | |
| 74 | A | Y | Q | L | T | E | K | D | K | E | 4 | |
| 93 | A | R | Y | S | T | T | A | L | L | E | 4 | |
| 122 | S | E | E | K | D | V | L | K | Q | Q | 4 | |
| 144 | S | K | T | N | T | L | R | L | S | Q | 4 | |
| 153 | Q | T | V | A | P | N | C | F | N | S | 4 | |
| 162 | S | S | I | N | N | I | H | E | M | E | 4 | |
| 167 | I | H | E | M | E | I | Q | L | K | D | 4 | |
| 170 | M | E | I | Q | L | K | D | A | L | E | 4 | |
| 179 | E | K | N | Q | Q | W | L | V | Y | D | 4 | |
| 191 | R | E | V | Y | V | K | G | L | L | A | 4 | |
| 205 | L | E | K | K | T | E | T | A | A | H | 4 | |
| 208 | K | T | E | T | A | A | H | S | L | P | 4 | |
| 213 | A | H | S | L | P | Q | Q | T | K | K | 4 | |
| 219 | Q | T | K | K | P | E | S | E | G | Y | 4 | |
| 221 | K | K | P | E | S | E | G | Y | L | Q | 4 | |
| 284 | Y | S | Q | R | R | A | D | V | Q | H | 4 | |
| 314 | A | R | G | K | L | E | E | E | K | K | 4 | |
| 316 | G | K | L | E | E | E | K | K | R | S | 4 | |
| 337 | T | S | L | L | K | Q | Q | E | E | Q | 4 | |
| 347 | T | R | V | A | L | L | E | Q | Q | M | 4 | |
| 356 | M | Q | A | C | T | L | D | F | E | N | 4 | |

TABLE XXXVI 121P2A3 v.1: HLA Peptide Scoring Results A*0201 10-mers SYFPEITHI

| Pos | 1 2 3 4 5 6 7 8 9 0 | score | SEQ. ID NO. |
|---|---|---|---|
| 357 | Q A C T L D F E N E | 4 | |
| 358 | A C T L D F E N E K | 4 | |
| 363 | F E N E K L D R Q H | 4 | |
| 392 | T Q L E S L K Q L H | 4 | |
| 402 | E F A I T E P L V T | 4 | |
| 440 | C P K C N I Q Y P A | 4 | |
| 443 | C N I Q Y P A T E H | 4 | |
| 452 | H R D L L V H V E Y | 4 | |
| 2 | S S R S T K D L I K | 3 | |
| 11 | K S K W G S K P S N | 3 | |
| 15 | G S K P S N S K S E | 3 | |
| 37 | L K T S V D E I T S | 3 | |
| 49 | G K L T D K E R H R | 3 | |
| 61 | E K I R V L E A E K | 3 | |
| 63 | I R V L E A E K E K | 3 | |
| 70 | K E K N A Y Q L T E | 3 | |
| 80 | K D K E I Q R L R D | 3 | |
| 102 | E Q L E E T T R E G | 3 | |
| 104 | L E E T T R E G E R | 3 | |
| 114 | R E Q V L K A L S E | 3 | |
| 115 | E Q V L K A L S E E | 3 | |
| 129 | K Q Q L S A A T S R | 3 | |
| 151 | L S Q T V A P N C F | 3 | |
| 156 | A P N C F N S S I N | 3 | |
| 164 | I N N I H E M E I Q | 3 | |
| 174 | L K D A L E K N Q Q | 3 | |
| 175 | K D A L E K N Q Q W | 3 | |
| 188 | D Q Q R E V Y V K G | 3 | |
| 225 | S E G Y L Q E E K Q | 3 | |
| 229 | L Q E E K Q K C Y N | 3 | |
| 236 | C Y N D L L A S A K | 3 | |
| 238 | N D L L A S A K K D | 3 | |
| 252 | R Q T I T Q L S F E | 3 | |
| 262 | L S E F R R K Y E E | 3 | |
| 269 | Y E E T Q K E V H N | 3 | |
| 290 | D V Q H L E D D R H | 3 | |
| 325 | S E E L L S Q V Q F | 3 | |
| 332 | V Q F L Y T S L L K | 3 | |
| 345 | E Q T R V A L L E Q | 3 | |
| 377 | L H V I L K E L R K | 3 | |
| 384 | L R K A R N Q I T Q | 3 | |
| 395 | E S L K Q L H E F A | 3 | |
| 411 | T F Q G E T E N R E | 3 | |
| 417 | E N R E K V A A S P | 3 | |
| 425 | S P K S P T A A L N | 3 | |
| 434 | N E S L V E C P K C | 3 | |
| 438 | V E C P K C N I Q Y | 3 | |
| 441 | P K C N I Q Y P A T | 3 | |
| 451 | E H R D L L V H V E | 3 | |
| 14 | W G S K P S N S K S | 2 | |
| 19 | S N S K S E T T L E | 2 | |
| 20 | N S K S E T T L E K | 2 | |
| 27 | L E K L K G E I A H | 2 | |
| 39 | T S V D E I T S G K | 2 | |
| 47 | G K G K L T D K E R | 2 | |
| 52 | T D K E R H R L L E | 2 | |

TABLE XXXVI 121P2A3 v.1: HLA Peptide Scoring Results A*0201 10-mers SYFPEITHI

| Pos | 1 2 3 4 5 6 7 8 9 0 | score | SEQ. ID NO. |
|---|---|---|---|
| 67 | E A E K E K N A Y Q | 2 | |
| 81 | D K E I Q R L R D Q | 2 | |
| 87 | L R D Q L K A R Y S | 2 | |
| 101 | L E Q L E E T T R E | 2 | |
| 159 | C F N S S I N N I H | 2 | |
| 178 | L E K N Q Q W L V Y | 2 | |
| 183 | Q W L V Y D Q Q R E | 2 | |
| 268 | K Y E E T Q K E V H | 2 | |
| 271 | E T Q K E V H N L N | 2 | |
| 272 | T Q K E V H N L N Q | 2 | |
| 275 | E V H N L N Q L L Y | 2 | |
| 279 | L N Q L L Y S Q R R | 2 | |
| 283 | L Y S Q R R A D V Q | 2 | |
| 289 | A D V Q H L E D D R | 2 | |
| 354 | Q Q M Q A C T L D F | 2 | |
| 362 | D F E N E K L D R Q | 2 | |
| 366 | E K L D R Q H V Q H | 2 | |
| 389 | N Q I T Q L E S L K | 2 | |
| 419 | R E K V A A S P K S | 2 | |
| 10 | I K S K W G S K P S | 1 | |
| 113 | R R E Q V L K A L S | 1 | |
| 152 | S Q T V A P N C F N | 1 | |
| 160 | F N S S I N N I H E | 1 | |
| 182 | Q Q W L V Y D Q Q R | 1 | |
| 195 | V K G L L A K I F E | 1 | |
| 218 | Q Q T K K P E S E G | 1 | |
| 222 | K P E S E G Y L Q E | 1 | |
| 250 | V E R Q T I T Q L S | 1 | |
| 259 | S F E L S E F R R K | 1 | |
| 265 | F R R K Y E E T Q K | 1 | |
| 286 | Q R R A D V Q H L E | 1 | |
| 291 | V Q H L E D D R H K | 1 | |
| 302 | E K I Q K L R E E N | 1 | |
| 324 | R S E E L L S Q V Q | 1 | |
| 335 | L Y T S L L K Q Q E | 1 | |
| 381 | L K E L R K A R N Q | 1 | |
| 406 | T E P L V T F Q G E | 1 | |
| 433 | L N E S L V E C P K | 1 | |
| 439 | E C P K C N I Q Y P | 1 | |
| 6 | T K D L I K S K W G | -1 | |
| 22 | K S E T T L E K L K | -1 | |
| 217 | P Q Q T K K P E S E | -1 | |
| 264 | E F R R K Y E E T Q | -1 | |
| 297 | D R H K T E K I Q K | -1 | |
| 369 | D R Q H V Q H Q L H | -1 | |
| 418 | N R E K V A A S P K | -1 | |
| 110 | E G E R R E Q V L K | -2 | |
| 206 | E K K T E T A A H S | -2 | |
| 296 | D D R H K T E K I Q | -2 | |
| 344 | E E Q T R V A L L E | -2 | |
| 420 | E K V A A S P K S P | -2 | |
| 435 | E S L V E C P K C N | -2 | |
| 79 | E K D K E I Q R L R | -3 | |
| 124 | E K D V L K Q Q L S | -3 | |
| 226 | E G Y L Q E E K Q K | -3 | |
| 321 | E K K R S E E L L S | -3 | |

| TABLE XXXVI 121P2A3 v.1: HLA Peptide Scoring Results A*0201 10-mers SYFPEITHI | | | |
|---|---|---|---|
| Pos | 1 2 3 4 5 6 7 8 9 0 | score | SEQ. ID NO. |
| 353 | E Q Q M Q A C T L D | -3 | |
| 55 | E R H R L L E K I R | -4 | |
| 105 | E E T T R E G E R R | -4 | |
| 157 | P N C F N S S I N N | -4 | |
| 310 | E N D I A R G K L E | -4 | |

| TABLE XXXVI 121P2A3 v.3: HLA Peptide Scoring Results A*0201 10-mers SYFPEITHI | | | |
|---|---|---|---|
| Pos | 1 2 3 4 5 6 7 8 9 0 | score | SEQ. ID NO. |
| 5 | K L T D K E R Q R L | 21 | |
| 6 | L T D K E R Q R L L | 16 | |
| 12 | Q R L L E K I R V L | 16 | |
| 9 | K E R Q R L L E K I | 15 | |
| 11 | R Q R L L E K I R V | 10 | |
| 1 | S G K G K L T D K E | 5 | |
| 8 | D K E R Q R L L E K | 5 | |
| 4 | G K L T D K E R Q R | 3 | |
| 2 | G K G K L T D K E R | 2 | |
| 7 | T D K E R Q R L L E | 2 | |
| 10 | E R Q R L L E K I R | -4 | |

| TABLE XXXVI 121P2A3 v.4: HLA Peptide Scoring Results A*0201 10-mers SYFPEITHI | | | |
|---|---|---|---|
| Pos | 1 2 3 4 5 6 7 8 9 0 | score | SEQ. ID NO. |
| 10 | T L L E Q L E E T T | 20 | |
| 2 | L K A R Y S T T T L | 16 | |
| 9 | T T L L E Q L E E T | 16 | |
| 1 | Q L K A R Y S T T T | 15 | |
| 6 | Y S T T T L L E Q L | 15 | |
| 3 | K A R Y S T T T L L | 14 | |
| 5 | R Y S T T T L L E Q | 7 | |
| 8 | T T T L L E Q L E E | 6 | |
| 7 | S T T T L L E Q L E | 5 | |
| 4 | A R Y S T T T L L E | 4 | |

| TABLE XXXVI 121P2A3 v.6: HLA Peptide Scoring Results A*0201 10-mers SYFPEITHI | | | |
|---|---|---|---|
| Pos | 1 2 3 4 5 6 7 8 9 0 | score | SEQ. ID NO. |
| 4 | L L S Q V Q S L Y T | 17 | |
| 10 | S L Y T S L L K Q Q | 16 | |
| 2 | E E L L S Q V Q S L | 15 | |
| 6 | S Q V Q S L Y T S L | 14 | |
| 7 | Q V Q S L Y T S L L | 14 | |
| 3 | E L L S Q V Q S L Y | 9 | |
| 5 | L S Q V Q S L Y T S | 9 | |
| 9 | Q S L Y T S L L K Q | 8 | |
| 1 | S E E L L S Q V Q S | 3 | |
| 8 | V Q S L Y T S L L K | 2 | |

| TABLE XXXVI 121P2A3 v.7: HLA Peptide Scoring Results A*0201 10-mers SYFPEITHI | | | |
|---|---|---|---|
| Pos | 1 2 3 4 5 6 7 8 9 0 | score | SEQ. ID NO. |
| 4 | H V Q H Q L L V I L | 20 | |
| 10 | L V I L K E L R K A | 18 | |
| 7 | H Q L L V I L K E L | 17 | |
| 3 | Q H V Q H Q L L V I | 13 | |
| 9 | L L V I L K E L R K | 13 | |
| 8 | Q L L V I L K E L R | 12 | |
| 2 | R Q H V Q H Q L L V | 11 | |
| 1 | D R Q H V Q H Q L L | 9 | |
| 6 | Q H Q L L V I L K E | 7 | |
| 5 | V Q H Q L L V I L K | 5 | |

| TABLE XXXVI 121P2A3 v.8: HLA Peptide Scoring Results A*0201 10-mers SYFPEITHI | | | |
|---|---|---|---|
| Pos | 1 2 3 4 5 6 7 8 9 0 | score | SEQ. ID NO. |
| 7 | A L N G S L V E C P | 19 | |
| 6 | A A L N G S L V E C | 16 | |
| 3 | S P T A A L N G S L | 13 | |
| 4 | P T A A L N G S L V | 13 | |
| 2 | K S P T A A L N G S | 7 | |
| 5 | T A A L N G S L V E | 7 | |
| 9 | N G S L V E C P K C | 3 | |
| 8 | L N G S L V E C P K | 2 | |
| 10 | G S L V E C P K C N | 2 | |

| TABLE XXXVII 121P2A3 v.1: HLA Peptide Scoring Results A*0202 10-mers SYFPEITHI | | | |
|---|---|---|---|
| Pos | 1 2 3 4 5 6 7 8 9 0 | score | SEQ. ID NO. |
| 133 | S A A T S R I A E L | 5 | |
| 211 | T A A H S L P Q Q T | 5 | |
| 422 | V A A S P K S P T A | 5 | |
| 430 | T A A L N E S L V E | 5 | |
| 242 | A S A K K D L E V E | 4 | |
| 33 | E I A H L K T S V D | 3 | |
| 66 | L E A E K E K N A Y | 3 | |
| 72 | K N A Y Q L T E K D | 3 | |
| 91 | L K A R Y S T T A L | 3 | |
| 97 | T T A L L E Q L E E | 3 | |
| 118 | L K A L S E E K D V | 3 | |
| 132 | L S A A T S R I A E | 3 | |
| 134 | A A T S R I A E L E | 3 | |
| 138 | R I A E L E S K T N | 3 | |
| 154 | T V A P N C F N S S | 3 | |
| 175 | K D A L E K N Q Q W | 3 | |
| 198 | L L A K I F E L E K | 3 | |
| 210 | E T A A H S L P Q Q | 3 | |
| 212 | A A H S L P Q Q T K | 3 | |
| 240 | L L A S A K K D L E | 3 | |
| 287 | R R A D V Q H L E D | 3 | |
| 312 | D I A R G K L E E E | 3 | |
| 348 | R V A L L E Q Q M Q | 3 | |
| 356 | M Q A C T L D F E N | 3 | |
| 385 | R K A R N Q I T Q L | 3 | |
| 402 | E F A I T E P L V T | 3 | |

| TABLE XXXVII 121P2A3 v.1: HLA Peptide Scoring Results A*0202 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 421 | K | V | A | A | S | P | K | S | P | T | 3 | |
| 423 | A | A | S | P | K | S | P | T | A | A | 3 | |
| 429 | P | T | A | A | L | N | E | S | L | V | 3 | |
| 431 | A | A | L | N | E | S | L | V | E | C | 3 | |
| 447 | Y | P | A | T | E | H | R | D | L | L | 3 | |
| 34 | I | A | H | L | K | T | S | V | D | E | 2 | |
| 67 | E | A | E | K | E | K | N | A | Y | Q | 2 | |
| 73 | N | A | Y | Q | L | T | E | K | D | K | 2 | |
| 92 | K | A | R | Y | S | T | T | A | L | L | 2 | |
| 98 | T | A | L | L | E | Q | L | E | E | T | 2 | |
| 119 | K | A | L | S | E | E | K | D | V | L | 2 | |
| 139 | I | A | E | L | E | S | K | T | N | T | 2 | |
| 155 | V | A | P | N | C | F | N | S | S | I | 2 | |
| 176 | D | A | L | E | K | N | Q | Q | W | L | 2 | |
| 199 | L | A | K | I | F | E | L | E | K | K | 2 | |
| 241 | L | A | S | A | K | K | D | L | E | V | 2 | |
| 243 | S | A | K | K | D | L | E | V | E | R | 2 | |
| 288 | R | A | D | V | Q | H | L | E | D | D | 2 | |
| 313 | I | A | R | G | K | L | E | E | E | K | 2 | |
| 349 | V | A | L | L | E | Q | Q | M | Q | A | 2 | |
| 357 | Q | A | C | T | L | D | F | E | N | E | 2 | |
| 386 | K | A | R | N | Q | I | T | Q | L | E | 2 | |
| 403 | F | A | I | T | E | P | L | V | T | F | 2 | |
| 448 | P | A | T | E | H | R | D | L | L | V | 2 | |
| 35 | A | H | L | K | T | S | V | D | E | I | 1 | |
| 68 | A | E | K | E | K | N | A | Y | Q | L | 1 | |
| 74 | A | Y | Q | L | T | E | K | D | K | E | 1 | |
| 93 | A | R | Y | S | T | T | A | L | L | E | 1 | |
| 99 | A | L | L | E | Q | L | E | E | T | T | 1 | |
| 120 | A | L | S | E | E | K | D | V | L | K | 1 | |
| 135 | A | T | S | R | I | A | E | L | E | S | 1 | |
| 140 | A | E | L | E | S | K | T | N | T | L | 1 | |
| 156 | A | P | N | C | F | N | S | S | I | N | 1 | |
| 177 | A | L | E | K | N | Q | Q | W | L | V | 1 | |
| 200 | A | K | I | F | E | L | E | K | K | T | 1 | |
| 213 | A | H | S | L | P | Q | Q | T | K | K | 1 | |
| 244 | A | K | K | D | L | E | V | E | R | Q | 1 | |
| 289 | A | D | V | Q | H | L | E | D | D | R | 1 | |
| 314 | A | R | G | K | L | E | E | E | K | K | 1 | |
| 350 | A | L | L | E | Q | Q | M | Q | A | C | 1 | |
| 358 | A | C | T | L | D | F | E | N | E | K | 1 | |
| 387 | A | R | N | Q | I | T | Q | L | E | S | 1 | |
| 404 | A | I | T | E | P | L | V | T | F | Q | 1 | |
| 424 | A | S | P | K | S | P | T | A | A | L | 1 | |
| 432 | A | L | N | E | S | L | V | E | C | P | 1 | |
| 449 | A | T | E | H | R | D | L | L | V | H | 1 | |

| TABLE XXXVII 121P2A3 v.4: HLA Peptide Scoring Results A*0202 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 2 | L | K | A | R | Y | S | T | T | T | L | 3 | |
| 3 | K | A | R | Y | S | T | T | T | L | L | 2 | |
| 4 | A | R | Y | S | T | T | T | L | L | E | 1 | |

| TABLE XXXVII 121P2A3 v.8: HLA Peptide Scoring Results A*0202 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 5 | T | A | A | L | N | G | S | L | V | E | 5 | |
| 4 | P | T | A | A | L | N | G | S | L | V | 3 | |
| 6 | A | A | L | N | G | S | L | V | E | C | 3 | |
| 7 | A | L | N | G | S | L | V | E | C | P | 1 | |

| TABLE XXXVIII 121P2A3 v.1: HLA Peptide Scoring Results A*0203 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 126 | D | V | L | K | Q | Q | L | S | A | A | 19 | |
| 204 | E | L | E | K | K | T | E | T | A | A | 19 | |
| 415 | E | T | E | N | R | E | K | V | A | A | 19 | |
| 423 | A | A | S | P | K | S | P | T | A | A | 19 | |
| 235 | K | C | Y | N | D | L | L | A | S | A | 18 | |
| 127 | V | L | K | Q | Q | L | S | A | A | T | 17 | |
| 205 | L | E | K | K | T | E | T | A | A | H | 17 | |
| 416 | T | E | N | R | E | K | V | A | A | S | 17 | |
| 424 | A | S | P | K | S | P | T | A | A | L | 17 | |
| 26 | T | L | E | K | L | K | G | E | I | A | 10 | |
| 59 | L | L | E | K | I | R | V | L | E | A | 10 | |
| 65 | V | L | E | A | E | K | E | K | N | A | 10 | |
| 84 | I | Q | R | L | R | D | Q | L | K | A | 10 | |
| 90 | Q | L | K | A | R | Y | S | T | T | A | 10 | |
| 111 | G | E | R | R | E | Q | V | L | K | A | 10 | |
| 125 | K | D | V | L | K | Q | Q | L | S | A | 10 | |
| 131 | Q | L | S | A | A | T | S | R | I | A | 10 | |
| 147 | N | T | L | R | L | S | Q | T | V | A | 10 | |
| 168 | H | E | M | E | I | Q | L | K | D | A | 10 | |
| 191 | R | E | V | Y | Y | V | K | G | L | L | 10 | |
| 203 | F | E | L | E | K | K | T | E | T | A | 10 | |
| 233 | K | Q | K | C | Y | N | D | L | L | A | 10 | |
| 280 | N | Q | L | L | Y | S | Q | R | R | A | 10 | |
| 305 | Q | K | L | R | E | E | N | D | I | A | 10 | |
| 341 | K | Q | Q | E | E | Q | T | R | V | A | 10 | |
| 349 | V | A | L | L | E | Q | Q | M | Q | A | 10 | |
| 378 | H | V | I | L | K | E | L | R | K | A | 10 | |
| 395 | E | S | L | K | Q | L | H | E | F | A | 10 | |
| 414 | G | E | T | E | N | R | E | K | V | A | 10 | |
| 422 | V | A | A | S | P | K | S | P | T | A | 10 | |
| 440 | C | P | K | C | N | I | Q | Y | P | A | 10 | |
| 27 | L | E | K | L | K | G | E | I | A | H | 9 | |
| 60 | L | E | K | I | R | V | L | E | A | E | 9 | |
| 66 | L | E | A | E | K | E | K | N | A | Y | 9 | |
| 85 | Q | R | L | R | D | Q | L | K | A | R | 9 | |
| 91 | L | K | A | R | Y | S | T | T | A | L | 9 | |
| 112 | E | R | R | E | Q | V | L | K | A | L | 9 | |
| 132 | L | S | A | A | T | S | R | I | A | E | 9 | |
| 148 | T | L | R | L | S | Q | T | V | A | P | 9 | |
| 169 | E | M | E | I | Q | L | K | D | A | L | 9 | |
| 192 | E | V | Y | Y | V | K | G | L | L | A | 9 | |
| 234 | Q | K | C | Y | N | D | L | L | A | S | 9 | |
| 236 | C | Y | N | D | L | L | A | S | A | K | 9 | |
| 281 | Q | L | L | Y | S | Q | R | R | A | D | 9 | |
| 306 | K | L | R | E | E | N | D | I | A | R | 9 | |
| 342 | Q | Q | E | E | Q | T | R | V | A | L | 9 | |

| TABLE XXXVIII 121P2A3 v.1: HLA Peptide Scoring Results A*0203 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 350 | A | L | L | E | Q | Q | M | Q | A | C | 9 | |
| 379 | V | I | L | K | E | L | R | K | A | R | 9 | |
| 396 | S | L | K | Q | L | H | E | F | A | I | 9 | |
| 441 | P | K | C | N | I | Q | Y | P | A | T | 9 | |
| 28 | E | K | L | K | G | E | I | A | H | L | 8 | |
| 61 | E | K | I | R | V | L | E | A | E | K | 8 | |
| 67 | E | A | E | K | E | K | N | A | Y | Q | 8 | |
| 86 | R | L | R | D | Q | L | K | A | R | Y | 8 | |
| 92 | K | A | R | Y | S | T | T | A | L | L | 8 | |
| 113 | R | R | E | Q | V | L | K | A | L | S | 8 | |
| 128 | L | K | Q | Q | L | S | A | A | T | S | 8 | |
| 133 | S | A | A | T | S | R | I | A | E | L | 8 | |
| 149 | L | R | L | S | Q | T | V | A | P | N | 8 | |
| 170 | M | E | I | Q | L | K | D | A | L | E | 8 | |
| 193 | V | Y | V | K | G | L | L | A | K | I | 8 | |
| 206 | E | K | K | T | E | T | A | A | H | S | 8 | |
| 237 | Y | N | D | L | L | A | S | A | K | K | 8 | |
| 282 | L | L | Y | S | Q | R | R | A | D | V | 8 | |
| 307 | L | R | E | E | N | D | I | A | R | G | 8 | |
| 343 | Q | E | E | Q | T | R | V | A | L | L | 8 | |
| 351 | L | L | E | Q | Q | M | Q | A | C | T | 8 | |
| 380 | I | L | K | E | L | R | K | A | R | N | 8 | |
| 397 | L | K | Q | L | H | E | F | A | I | T | 8 | |
| 417 | E | N | R | E | K | V | A | A | S | P | 8 | |
| 425 | S | P | K | S | P | T | A | A | L | N | 8 | |
| 442 | K | C | N | I | Q | Y | P | A | T | E | 8 | |

| TABLE XXXVIII 121P2A3 v.7: HLA Peptide Scoring Results A*0203 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 10 | L | V | I | L | K | E | L | R | K | A | 10 | |

| TABLE XXXIX 121P2A3 v.1: HLA Peptide Scoring Results A3 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 29 | K | L | K | G | E | I | A | H | L | K | 29 | |
| 120 | A | L | S | E | E | K | D | V | L | K | 28 | |
| 192 | E | V | Y | V | K | G | L | L | A | K | 28 | |
| 86 | R | L | R | D | Q | L | K | A | R | Y | 27 | |
| 8 | D | L | I | K | S | K | W | G | S | K | 26 | |
| 185 | L | V | Y | D | Q | Q | R | E | V | Y | 26 | |
| 171 | E | I | Q | L | K | D | A | L | E | K | 25 | |
| 116 | Q | V | L | K | A | L | S | E | E | K | 24 | |
| 198 | L | L | A | K | I | F | E | L | E | K | 24 | |
| 58 | R | L | L | E | K | I | R | V | L | E | 22 | |
| 306 | K | L | R | E | E | N | D | I | A | R | 22 | |
| 455 | L | L | V | H | V | E | Y | C | S | K | 22 | |
| 83 | E | I | Q | R | L | R | D | Q | L | K | 21 | |
| 90 | Q | L | K | A | R | Y | S | T | T | A | 20 | |
| 99 | A | L | L | E | Q | L | E | E | T | T | 20 | |
| 194 | Y | V | K | G | L | L | A | K | I | F | 20 | |
| 275 | E | V | H | N | L | N | Q | L | L | Y | 20 | |
| 278 | N | L | N | Q | L | L | Y | S | Q | R | 20 | |

| TABLE XXXIX 121P2A3 v.1: HLA Peptide Scoring Results A3 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 61 | E | K | I | R | V | L | E | A | E | K | 19 | |
| 100 | L | L | E | Q | L | E | E | T | T | R | 19 | |
| 148 | T | L | R | L | S | Q | T | V | A | P | 19 | |
| 166 | N | I | H | E | M | E | I | Q | L | K | 19 | |
| 339 | L | L | K | Q | Q | E | E | Q | T | R | 19 | |
| 421 | K | V | A | A | S | P | K | S | P | T | 19 | |
| 138 | R | I | A | E | L | E | S | K | T | N | 18 | |
| 226 | E | G | Y | L | Q | E | E | K | Q | K | 18 | |
| 236 | C | Y | N | D | L | L | A | S | A | K | 18 | |
| 282 | L | L | Y | S | Q | R | R | A | D | V | 18 | |
| 308 | R | E | E | N | D | I | A | R | G | K | 18 | |
| 327 | E | L | L | S | Q | V | Q | F | L | Y | 18 | |
| 2 | S | S | R | S | T | K | D | L | I | K | 17 | |
| 62 | K | I | R | V | L | E | A | E | K | E | 17 | |
| 64 | R | V | L | E | A | E | K | E | K | N | 17 | |
| 110 | E | G | E | R | R | E | Q | V | L | K | 17 | |
| 150 | R | L | S | Q | T | V | A | P | N | C | 17 | |
| 212 | A | A | H | S | L | P | Q | Q | T | K | 17 | |
| 228 | Y | L | Q | E | E | K | Q | K | C | Y | 17 | |
| 249 | E | V | E | R | Q | T | I | T | Q | L | 17 | |
| 265 | F | R | R | K | Y | E | E | T | Q | K | 17 | |
| 313 | I | A | R | G | K | L | E | E | E | K | 17 | |
| 334 | F | L | Y | T | S | L | L | K | Q | Q | 17 | |
| 348 | R | V | A | L | L | E | Q | Q | M | Q | 17 | |
| 350 | A | L | L | E | Q | Q | M | Q | A | C | 17 | |
| 380 | I | L | K | E | L | R | K | A | R | N | 17 | |
| 408 | P | L | V | T | F | Q | G | E | T | E | 17 | |
| 4 | R | S | T | K | D | L | I | K | S | K | 16 | |
| 33 | E | I | A | H | L | K | T | S | V | D | 16 | |
| 53 | D | K | E | R | H | R | L | L | E | K | 16 | |
| 126 | D | V | L | K | Q | Q | L | S | A | A | 16 | |
| 136 | T | S | R | I | A | E | L | E | S | K | 16 | |
| 154 | T | V | A | P | N | C | F | N | S | S | 16 | |
| 201 | K | I | F | E | L | E | K | K | T | E | 16 | |
| 257 | Q | L | S | F | E | L | S | E | F | R | 16 | |
| 281 | Q | L | L | Y | S | Q | R | R | A | D | .16 | |
| 317 | K | L | E | E | E | K | K | R | S | E | 16 | |
| 338 | S | L | L | K | Q | Q | E | E | Q | T | 16 | |
| 367 | K | L | D | R | Q | H | V | Q | H | Q | 16 | |
| 376 | Q | L | H | V | I | L | K | E | L | R | 16 | |
| 379 | V | I | L | K | E | L | R | K | A | R | 16 | |
| 389 | N | Q | I | T | Q | L | E | S | L | K | 16 | |
| 418 | N | R | E | K | V | A | A | S | P | K | 16 | |
| 9 | L | I | K | S | K | W | G | S | K | P | 15 | |
| 13 | K | W | G | S | K | P | S | N | S | K | 15 | |
| 50 | K | L | T | D | K | E | R | H | R | L | 15 | |
| 127 | V | L | K | Q | Q | L | S | A | A | T | 15 | |
| 141 | E | L | E | S | K | T | N | T | L | R | 15 | |
| 178 | L | E | K | N | Q | Q | W | L | V | Y | 15 | |
| 213 | A | H | S | L | P | Q | Q | T | K | K | 15 | |
| 293 | H | L | E | D | D | R | H | K | T | E | 15 | |
| 331 | Q | V | Q | F | L | Y | T | S | L | L | 15 | |
| 393 | Q | L | E | S | L | K | Q | L | H | E | 15 | |
| 403 | F | A | I | T | E | P | L | V | T | F | 15 | |
| 20 | N | S | K | S | E | T | T | L | E | K | 14 | |
| 39 | T | S | V | D | E | I | T | S | G | K | 14 | |

| TABLE XXXIX 121P2A3 v.1: HLA Peptide Scoring Results A3 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 41 | V | D | E | I | T | S | G | K | G | K | 14 | |
| 73 | N | A | Y | Q | L | T | E | K | D | K | 14 | |
| 103 | Q | L | E | E | T | T | R | E | G | E | 14 | |
| 129 | K | Q | Q | L | S | A | A | T | S | R | 14 | |
| 131 | Q | L | S | A | A | T | S | R | I | A | 14 | |
| 173 | Q | L | K | D | A | L | E | K | N | Q | 14 | |
| 187 | Y | D | Q | Q | R | E | V | Y | V | K | 14 | |
| 197 | G | L | L | A | K | I | F | E | L | E | 14 | |
| 237 | Y | N | D | L | L | A | S | A | K | K | 14 | |
| 239 | D | L | L | A | S | A | K | K | D | L | 14 | |
| 284 | Y | S | Q | R | R | A | D | V | Q | H | 14 | |
| 290 | D | V | Q | H | L | E | D | D | R | H | 14 | |
| 332 | V | Q | F | L | Y | T | S | L | L | K | 14 | |
| 358 | A | C | T | L | D | F | E | N | E | K | 14 | |
| 366 | E | K | L | D | R | Q | H | V | Q | H | 14 | |
| 372 | H | V | Q | H | Q | L | H | V | I | L | 14 | |
| 377 | L | H | V | I | L | K | E | L | R | K | 14 | |
| 378 | H | V | I | L | K | E | L | R | K | A | 14 | |
| 399 | Q | L | H | E | F | A | I | T | E | P | 14 | |
| 432 | A | L | N | E | S | L | V | E | C | P | 14 | |
| 449 | A | T | E | H | R | D | L | L | V | H | 14 | |
| 22 | K | S | E | T | T | L | E | K | L | K | 13 | |
| 26 | T | L | E | K | L | K | G | E | I | A | 13 | |
| 40 | S | V | D | E | I | T | S | G | K | G | 13 | |
| 59 | L | L | E | K | I | R | V | L | E | A | 13 | |
| 63 | I | R | V | L | E | A | E | K | E | K | 13 | |
| 71 | E | K | N | A | Y | Q | L | T | E | K | 13 | |
| 76 | Q | L | T | E | K | D | K | E | I | Q | 13 | |
| 93 | A | R | Y | S | T | T | A | L | L | E | 13 | |
| 117 | V | L | K | A | L | S | E | E | K | D | 13 | |
| 177 | A | L | E | K | N | Q | Q | W | L | V | 13 | |
| 235 | K | C | Y | N | D | L | L | A | S | A | 13 | |
| 297 | D | R | H | K | T | E | K | I | Q | K | 13 | |
| 314 | A | R | G | K | L | E | E | E | K | K | 13 | |
| 325 | S | E | E | L | L | S | Q | V | Q | F | 13 | |
| 328 | L | L | S | Q | V | Q | F | L | Y | T | 13 | |
| 351 | L | L | E | Q | Q | M | Q | A | C | T | 13 | |
| 383 | E | L | R | K | A | R | N | Q | I | T | 13 | |
| 390 | Q | I | T | Q | L | E | S | L | K | Q | 13 | |
| 396 | S | L | K | Q | L | H | E | F | A | I | 13 | |
| 404 | A | I | T | E | P | L | V | T | F | Q | 13 | |
| 65 | V | L | E | A | E | K | E | K | N | A | 12 | |
| 70 | K | E | K | N | A | Y | Q | L | T | E | 12 | |
| 85 | Q | R | L | R | D | Q | L | K | A | R | 12 | |
| 114 | R | E | Q | V | L | K | A | L | S | E | 12 | |
| 199 | L | A | K | I | F | E | L | E | K | K | 12 | |
| 204 | E | L | E | K | K | T | E | T | A | A | 12 | |
| 224 | E | S | E | G | Y | L | Q | E | E | K | 12 | |
| 259 | S | F | E | L | S | E | F | R | R | K | 12 | |
| 261 | E | L | S | E | F | R | R | K | Y | E | 12 | |
| 268 | K | Y | E | E | T | Q | K | E | V | H | 12 | |
| 294 | L | E | D | D | R | H | K | T | E | K | 12 | |
| 312 | D | I | A | R | G | K | L | E | E | E | 12 | |
| 382 | K | E | L | R | K | A | R | N | Q | I | 12 | |
| 385 | R | K | A | R | N | Q | I | T | Q | L | 12 | |
| 398 | K | Q | L | H | E | F | A | I | T | E | 12 | |

| TABLE XXXIX 121P2A3 v.1: HLA Peptide Scoring Results A3 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 417 | E | N | R | E | K | V | A | A | S | P | 12 | |
| 436 | S | L | V | E | C | P | K | C | N | I | 12 | |
| 438 | V | E | C | P | K | C | N | I | Q | Y | 12 | |
| 442 | K | C | N | I | Q | Y | P | A | T | E | 12 | |
| 444 | N | I | Q | Y | P | A | T | E | H | R | 12 | |
| 454 | D | L | L | V | H | V | E | Y | C | S | 12 | |
| 36 | H | L | K | T | S | V | D | E | I | T | 11 | |
| 43 | E | I | T | S | G | K | G | K | L | T | 11 | |
| 44 | I | T | S | G | K | G | K | L | T | D | 11 | |
| 45 | T | S | G | K | G | K | L | T | D | K | 11 | |
| 68 | A | E | K | E | K | N | A | Y | Q | L | 11 | |
| 163 | S | I | N | N | I | H | E | M | E | I | 11 | |
| 215 | S | L | P | Q | Q | T | K | K | P | E | 11 | |
| 240 | L | L | A | S | A | K | K | D | L | E | 11 | |
| 246 | K | D | L | E | V | E | R | Q | T | I | 11 | |
| 247 | D | L | E | V | E | R | Q | T | I | T | 11 | |
| 291 | V | Q | H | L | E | D | D | R | H | K | 11 | |
| 303 | K | I | Q | K | L | R | E | E | N | D | 11 | |
| 322 | K | K | R | S | E | E | L | L | S | Q | 11 | |
| 409 | L | V | T | F | Q | G | E | T | E | N | 11 | |
| 412 | F | Q | G | E | T | E | N | R | E | K | 11 | |
| 443 | C | N | I | Q | Y | P | A | T | E | H | 11 | |
| 452 | H | R | D | L | L | V | H | V | E | Y | 11 | |
| 82 | K | E | I | Q | R | L | R | D | Q | L | 10 | |
| 128 | L | K | Q | Q | L | S | A | A | T | S | 10 | |
| 140 | A | E | L | E | S | K | T | N | T | L | 10 | |
| 147 | N | T | L | R | L | S | Q | T | V | A | 10 | |
| 182 | Q | Q | W | L | V | Y | D | Q | Q | R | 10 | |
| 184 | W | L | V | Y | D | Q | Q | R | E | V | 10 | |
| 191 | R | E | V | Y | V | K | G | L | L | A | 10 | |
| 243 | S | A | K | K | D | L | E | V | E | R | 10 | |
| 264 | E | F | R | R | K | Y | E | E | T | Q | 10 | |
| 315 | R | G | K | L | E | E | E | K | K | R | 10 | |
| 323 | K | R | S | E | E | L | L | S | Q | V | 10 | |
| 360 | T | L | D | F | E | N | E | K | L | D | 10 | |
| 373 | V | Q | H | Q | L | H | V | I | L | K | 10 | |
| 402 | E | F | A | I | T | E | P | L | V | T | 10 | |
| 431 | A | A | L | N | E | S | L | V | E | C | 10 | |
| 433 | L | N | E | S | L | V | E | C | P | K | 10 | |
| 437 | L | V | E | C | P | K | C | N | I | Q | 10 | |
| 48 | K | G | K | L | T | D | K | E | R | H | 9 | |
| 66 | L | E | A | E | K | E | K | N | A | Y | 9 | |
| 80 | K | D | K | E | I | Q | R | L | R | D | 9 | |
| 84 | I | Q | R | L | R | D | Q | L | K | A | 9 | |
| 89 | D | Q | L | K | A | R | Y | S | T | T | 9 | |
| 92 | K | A | R | Y | S | T | T | A | L | L | 9 | |
| 109 | R | E | G | E | R | R | E | Q | V | L | 9 | |
| 111 | G | E | R | R | E | Q | V | L | K | A | 9 | |
| 119 | K | A | L | S | E | E | K | D | V | L | 9 | |
| 125 | K | D | V | L | K | Q | Q | L | S | A | 9 | |
| 135 | A | T | S | R | I | A | E | L | E | S | 9 | |
| 137 | S | R | I | A | E | L | E | S | K | T | 9 | |
| 145 | K | T | N | T | L | R | L | S | Q | T | 9 | |
| 205 | L | E | K | K | T | E | T | A | A | H | 9 | |
| 207 | K | K | T | E | T | A | A | H | S | L | 9 | |
| 219 | Q | T | K | K | P | E | S | E | G | Y | 9 | |

| TABLE XXXIX 121P2A3 v.1: HLA Peptide Scoring Results A3 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 222 | K | P | E | S | E | G | Y | L | Q | E | 9 | |
| 251 | E | R | Q | T | I | T | Q | L | S | F | 9 | |
| 256 | T | Q | L | S | F | E | L | S | E | F | 9 | |
| 260 | F | E | L | S | E | F | R | R | K | Y | 9 | |
| 267 | R | K | Y | E | E | T | Q | K | E | V | 9 | |
| 287 | R | R | A | D | V | Q | H | L | E | D | 9 | |
| 324 | R | S | E | E | L | L | S | Q | V | Q | 9 | |
| 342 | Q | Q | E | E | Q | T | R | V | A | L | 9 | |
| 354 | Q | Q | M | Q | A | C | T | L | D | F | 9 | |
| 423 | A | A | S | P | K | S | P | T | A | A | 9 | |
| 426 | P | K | S | P | T | A | A | L | N | E | 9 | |
| 430 | T | A | A | L | N | E | S | L | V | E | 9 | |
| 445 | I | Q | Y | P | A | T | E | H | R | D | 9 | |
| 17 | K | P | S | N | S | K | S | E | T | T | 8 | |
| 31 | K | G | E | I | A | H | L | K | T | S | 8 | |
| 32 | G | E | I | A | H | L | K | T | S | V | 8 | |
| 56 | R | H | R | L | L | E | K | I | R | V | 8 | |
| 88 | R | D | Q | L | K | A | R | Y | S | T | 8 | |
| 144 | S | K | T | N | T | L | R | L | S | Q | 8 | |
| 175 | K | D | A | L | E | K | N | Q | Q | W | 8 | |
| 242 | A | S | A | K | K | D | L | E | V | E | 8 | |
| 252 | R | Q | T | I | T | Q | L | S | F | E | 8 | |
| 254 | T | I | T | Q | L | S | F | E | L | S | 8 | |
| 255 | I | T | Q | L | S | F | E | L | S | E | 8 | |
| 311 | N | D | I | A | R | G | K | L | E | E | 8 | |
| 341 | K | Q | Q | E | E | Q | T | R | V | A | 8 | |
| 346 | Q | T | R | V | A | L | L | E | Q | Q | 8 | |
| 361 | L | D | F | E | N | E | K | L | D | R | 8 | |
| 363 | F | E | N | E | K | L | D | R | Q | H | 8 | |
| 405 | I | T | E | P | L | V | T | F | Q | G | 8 | |
| 425 | S | P | K | S | P | T | A | A | L | N | 8 | |
| 451 | E | H | R | D | L | L | V | H | V | E | 8 | |
| 12 | S | K | W | G | S | K | P | S | N | S | 7 | |
| 27 | L | E | K | L | K | G | E | I | A | H | 7 | |
| 34 | I | A | H | L | K | T | S | V | D | E | 7 | |
| 55 | E | R | H | R | L | L | E | K | I | R | 7 | |
| 57 | H | R | L | L | E | K | I | R | V | L | 7 | |
| 77 | L | T | E | K | D | K | E | I | Q | R | 7 | |
| 94 | R | Y | S | T | T | A | L | L | E | Q | 7 | |
| 105 | E | E | T | T | R | E | G | E | R | R | 7 | |
| 113 | R | R | E | Q | V | L | K | A | L | S | 7 | |
| 130 | Q | Q | L | S | A | A | T | S | R | I | 7 | |
| 188 | D | Q | Q | R | E | V | Y | V | K | G | 7 | |
| 203 | F | E | L | E | K | K | T | E | T | A | 7 | |
| 206 | E | K | K | T | E | T | A | A | H | S | 7 | |
| 221 | K | K | P | E | S | E | G | Y | L | Q | 7 | |
| 233 | K | Q | K | C | Y | N | D | L | L | A | 7 | |
| 245 | K | K | D | L | E | V | E | R | Q | T | 7 | |
| 283 | L | Y | S | Q | R | R | A | D | V | Q | 7 | |
| 285 | S | Q | R | R | A | D | V | Q | H | L | 7 | |
| 286 | Q | R | R | A | D | V | Q | H | L | E | 7 | |
| 289 | A | D | V | Q | H | L | E | D | D | R | 7 | |
| 305 | Q | K | L | R | E | E | N | D | I | A | 7 | |
| 321 | E | K | K | R | S | E | E | L | L | S | 7 | |
| 333 | Q | F | L | Y | T | S | L | L | K | Q | 7 | |
| 349 | V | A | L | L | E | Q | Q | M | Q | A | 7 | |

| TABLE XXXIX 121P2A3 v.1: HLA Peptide Scoring Results A3 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 352 | L | E | Q | Q | M | Q | A | C | T | L | 7 | |
| 370 | R | Q | H | V | Q | H | Q | L | H | V | 7 | |
| 384 | L | R | K | A | R | N | Q | I | T | Q | 7 | |
| 386 | K | A | R | N | Q | I | T | Q | L | E | 7 | |
| 388 | R | N | Q | I | T | Q | L | E | S | L | 7 | |
| 392 | T | Q | L | E | S | L | K | Q | L | H | 7 | |
| 416 | T | E | N | R | E | K | V | A | A | S | 7 | |
| 424 | A | S | P | K | S | P | T | A | A | L | 7 | |
| 450 | T | E | H | R | D | L | L | V | H | V | 7 | |
| 453 | R | D | L | L | V | H | V | E | Y | C | 7 | |
| 5 | S | T | K | D | L | I | K | S | K | W | 6 | |
| 7 | K | D | L | I | K | S | K | W | G | S | 6 | |
| 11 | K | S | K | W | G | S | K | P | S | N | 6 | |
| 15 | G | S | K | P | S | N | S | K | S | E | 6 | |
| 38 | K | T | S | V | D | E | I | T | S | G | 6 | |
| 49 | G | K | L | T | D | K | E | R | H | R | 6 | |
| 52 | T | D | K | E | R | H | R | L | L | E | 6 | |
| 54 | K | E | R | H | R | L | L | E | K | I | 6 | |
| 91 | L | K | A | R | Y | S | T | T | A | L | 6 | |
| 108 | T | R | E | G | E | R | R | E | Q | V | 6 | |
| 134 | A | A | T | S | R | I | A | E | L | E | 6 | |
| 155 | V | A | P | N | C | F | N | S | S | I | 6 | |
| 156 | A | P | N | C | F | N | S | S | I | N | 6 | |
| 162 | S | S | I | N | N | I | H | E | M | E | 6 | |
| 165 | N | N | I | H | E | M | E | I | Q | L | 6 | |
| 170 | M | E | I | Q | L | K | D | A | L | E | 6 | |
| 189 | Q | Q | R | E | V | Y | V | K | G | L | 6 | |
| 196 | K | G | L | L | A | K | I | F | E | L | 6 | |
| 200 | A | K | I | F | E | L | E | K | K | T | 6 | |
| 208 | K | T | E | T | A | A | H | S | L | P | 6 | |
| 210 | E | T | A | A | H | S | L | P | Q | Q | 6 | |
| 234 | Q | K | C | Y | N | D | L | L | A | S | 6 | |
| 238 | N | D | L | L | A | S | A | K | K | D | 6 | |
| 292 | Q | H | L | E | D | D | R | H | K | T | 6 | |
| 300 | K | T | E | K | I | Q | K | L | R | E | 6 | |
| 344 | E | E | Q | T | R | V | A | L | L | E | 6 | |
| 345 | E | Q | T | R | V | A | L | L | E | Q | 6 | |
| 365 | N | E | K | L | D | R | Q | H | V | Q | 6 | |
| 368 | L | D | R | Q | H | V | Q | H | Q | L | 6 | |
| 371 | Q | H | V | Q | H | Q | L | H | V | I | 6 | |
| 374 | Q | H | Q | L | H | V | I | L | K | E | 6 | |
| 387 | A | R | N | Q | I | T | Q | L | E | S | 6 | |
| 391 | I | T | Q | L | E | S | L | K | Q | L | 6 | |
| 415 | E | T | E | N | R | E | K | V | A | A | 6 | |
| 429 | P | T | A | A | L | N | E | S | L | V | 6 | |
| 18 | P | S | N | S | K | S | E | T | T | L | 5 | |
| 28 | E | K | L | K | G | E | I | A | H | L | 5 | |
| 35 | A | H | L | K | T | S | V | D | E | I | 5 | |
| 97 | T | T | A | L | L | E | Q | L | E | E | 5 | |
| 104 | L | E | E | T | T | R | E | G | E | R | 5 | |
| 107 | T | T | R | E | G | E | R | R | E | Q | 5 | |
| 112 | E | R | R | E | Q | V | L | K | A | L | 5 | |
| 123 | E | E | K | D | V | L | K | Q | Q | L | 5 | |
| 132 | L | S | A | A | T | S | R | I | A | E | 5 | |
| 133 | S | A | A | T | S | R | I | A | E | L | 5 | |
| 146 | T | N | T | L | R | L | S | Q | T | V | 5 | |

| TABLE XXXIX 121P2A3 v.1: HLA Peptide Scoring Results A3 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 149 | L | R | L | S | Q | T | V | A | P | N | 5 | |
| 151 | L | S | Q | T | V | A | P | N | C | F | 5 | |
| 167 | I | H | E | M | E | I | Q | L | K | D | 5 | |
| 174 | L | K | D | A | L | E | K | N | Q | Q | 5 | |
| 209 | T | E | T | A | A | H | S | L | P | Q | 5 | |
| 211 | T | A | A | H | S | L | P | Q | Q | T | 5 | |
| 214 | H | S | L | P | Q | Q | T | K | K | P | 5 | |
| 218 | Q | Q | T | K | K | P | E | S | E | G | 5 | |
| 220 | T | K | K | P | E | S | E | G | Y | L | 5 | |
| 241 | L | A | S | A | K | K | D | L | E | V | 5 | |
| 244 | A | K | K | D | L | E | V | E | R | Q | 5 | |
| 248 | L | E | V | E | R | Q | T | I | T | Q | 5 | |
| 253 | Q | T | I | T | Q | L | S | F | E | L | 5 | |
| 258 | L | S | F | E | L | S | E | F | R | R | 5 | |
| 266 | R | R | K | Y | E | E | T | Q | K | E | 5 | |
| 272 | T | Q | K | E | V | H | N | L | N | Q | 5 | |
| 273 | Q | K | E | V | H | N | L | N | Q | L | 5 | |
| 277 | H | N | L | N | Q | L | L | Y | S | Q | 5 | |
| 279 | L | N | Q | L | L | Y | S | Q | R | R | 5 | |
| 298 | R | H | K | T | E | K | I | Q | K | L | 5 | |
| 299 | H | K | T | E | K | I | Q | K | L | R | 5 | |
| 318 | L | E | E | E | K | K | R | S | E | E | 5 | |
| 343 | Q | E | E | Q | T | R | V | A | L | L | 5 | |
| 347 | T | R | V | A | L | L | E | Q | Q | M | 5 | |
| 394 | L | E | S | L | K | Q | L | H | E | F | 5 | |
| 414 | G | E | T | E | N | R | E | K | V | A | 5 | |
| 419 | R | E | K | V | A | A | S | P | K | S | 5 | |
| 420 | E | K | V | A | A | S | P | K | S | P | 5 | |
| 422 | V | A | A | S | P | K | S | P | T | A | 5 | |
| 428 | S | P | T | A | A | L | N | E | S | L | 5 | |
| 16 | S | K | P | S | N | S | K | S | E | T | 4 | |
| 23 | S | E | T | T | L | E | K | L | K | G | 4 | |
| 25 | T | T | L | E | K | L | K | G | E | I | 4 | |
| 30 | L | K | G | E | I | A | H | L | K | T | 4 | |
| 37 | L | K | T | S | V | D | E | I | T | S | 4 | |
| 42 | D | E | I | T | S | G | K | G | K | L | 4 | |
| 46 | S | G | K | G | K | L | T | D | K | E | 4 | |
| 47 | G | K | G | K | L | T | D | K | E | R | 4 | |
| 72 | K | N | A | Y | Q | L | T | E | K | D | 4 | |
| 74 | A | Y | Q | L | T | E | K | D | K | E | 4 | |
| 79 | E | K | D | K | E | I | Q | R | L | R | 4 | |
| 115 | E | Q | V | L | K | A | L | S | E | E | 4 | |
| 121 | L | S | E | E | K | D | V | L | K | Q | 4 | |
| 122 | S | E | E | K | D | V | L | K | Q | Q | 4 | |
| 139 | I | A | E | L | E | S | K | T | N | T | 4 | |
| 153 | Q | T | V | A | P | N | C | F | N | S | 4 | |
| 159 | C | F | N | S | S | I | N | N | I | H | 4 | |
| 172 | I | Q | L | K | D | A | L | E | K | N | 4 | |
| 176 | D | A | L | E | K | N | Q | Q | W | L | 4 | |
| 179 | E | K | N | Q | Q | W | L | V | Y | D | 4 | |
| 183 | Q | W | L | V | Y | D | Q | Q | R | E | 4 | |
| 186 | V | Y | D | Q | Q | R | E | V | Y | V | 4 | |
| 202 | I | F | E | L | E | K | K | T | E | T | 4 | |
| 263 | S | E | F | R | R | K | Y | E | E | T | 4 | |
| 269 | Y | E | E | T | Q | K | E | V | H | N | 4 | |
| 276 | V | H | N | L | N | Q | L | L | Y | S | 4 | |

| TABLE XXXIX 121P2A3 v.1: HLA Peptide Scoring Results A3 10-mers SYFPEITHI | | | | | | | | | | | | |
|---|---|---|---|---|---|---|---|---|---|---|---|---|
| Pos | 1 | 2 | 3 | 4 | 5 | 6 | 7 | 8 | 9 | 0 | score | SEQ. ID NO. |
| 288 | R | A | D | V | Q | H | L | E | D | D | 4 | |
| 302 | E | K | I | Q | K | L | R | E | E | N | 4 | |
| 304 | I | Q | K | L | R | E | E | N | D | I | 4 | |
| 307 | L | R | E | E | N | D | I | A | R | G | 4 | |
| 310 | E | N | D | I | A | R | G | K | L | E | 4 | |
| 329 | L | S | Q | V | Q | F | L | Y | T | S | 4 | |
| 330 | S | Q | V | Q | F | L | Y | T | S | L | 4 | |
| 355 | Q | M | Q | A | C | T | L | D | F | E | 4 | |
| 369 | D | R | Q | H | V | Q | H | Q | L | H | 4 | |
| 381 | L | K | E | L | R | K | A | R | N | Q | 4 | |
| 397 | L | K | Q | L | H | E | F | A | I | T | 4 | |
| 410 | V | T | F | Q | G | E | T | E | N | R | 4 | |
| 446 | Q | Y | P | A | T | E | H | R | D | L | 4 | |
| 448 | P | A | T | E | H | R | D | L | L | V | 4 | |
| 1 | M | S | S | R | S | T | K | D | L | I | 3 | |
| 14 | W | G | S | K | P | S | N | S | K | S | 3 | |
| 60 | L | E | K | I | R | V | L | E | A | E | 3 | |
| 67 | E | A | E | K | E | K | N | A | Y | Q | 3 | |
| 87 | L | R | D | Q | L | K | A | R | Y | S | 3 | |
| 95 | Y | S | T | T | A | L | L | E | Q | L | 3 | |
| 96 | S | T | T | A | L | L | E | Q | L | E | 3 | |
| 98 | T | A | L | L | E | Q | L | E | E | T | 3 | |
| 102 | E | Q | L | E | E | T | T | R | E | G | 3 | |
| 106 | E | T | T | R | E | G | E | R | R | E | 3 | |
| 142 | L | E | S | K | T | N | T | L | R | L | 3 | |
| 164 | I | N | N | I | H | E | M | E | I | Q | 3 | |
| 180 | K | N | Q | Q | W | L | V | Y | D | Q | 3 | |
| 181 | N | Q | Q | W | L | V | Y | D | Q | Q | 3 | |
| 190 | Q | R | E | V | Y | V | K | G | L | L | 3 | |
| 193 | V | Y | V | K | G | L | L | A | K | I | 3 | |
| 217 | P | Q | Q | T | K | K | P | E | S | E | 3 | |
| 250 | V | E | R | Q | T | I | T | Q | L | S | 3 | |
| 274 | K | E | V | H | N | L | N | Q | L | L | 3 | |
| 280 | N | Q | L | L | Y | S | Q | R | R | A | 3 | |
| 295 | E | D | D | R | H | K | T | E | K | I | 3 | |
| 296 | D | D | R | H | K | T | E | K | I | Q | 3 | |
| 309 | E | E | N | D | I | A | R | G | K | L | 3 | |
| 316 | G | K | L | E | E | E | K | K | R | S | 3 | |
| 326 | E | E | L | L | S | Q | V | Q | F | L | 3 | |
| 335 | L | Y | T | S | L | L | K | Q | Q | E | 3 | |
| 337 | T | S | L | L | K | Q | Q | E | E | Q | 3 | |
| 359 | C | T | L | D | F | E | N | E | K | L | 3 | |
| 407 | E | P | L | V | T | F | Q | G | E | T | 3 | |
| 413 | Q | G | E | T | E | N | R | E | K | V | 3 | |
| 427 | K | S | P | T | A | A | L | N | E | S | 3 | |
| 435 | E | S | L | V | E | C | P | K | C | N | 3 | |
| 6 | T | K | D | L | I | K | S | K | W | G | 2 | |
| 10 | I | K | S | K | W | G | S | K | P | S | 2 | |
| 19 | S | N | S | K | S | E | T | T | L | E | 2 | |
| 21 | S | K | S | E | T | T | L | E | K | L | 2 | |
| 75 | Y | Q | L | T | E | K | D | K | E | I | 2 | |
| 78 | T | E | K | D | K | E | I | Q | R | L | 2 | |
| 101 | L | E | Q | L | E | E | T | T | R | E | 2 | |
| 118 | L | K | A | L | S | E | E | K | D | V | 2 | |
| 169 | E | M | E | I | Q | L | K | D | A | L | 2 | |
| 227 | G | Y | L | Q | E | E | K | Q | K | C | 2 | |

| TABLE XXXIX 121P2A3 v.1: HLA Peptide Scoring Results A3 10-mers SYFPEITHI | | | |
|---|---|---|---|
| Pos | 1 2 3 4 5 6 7 8 9 0 | score | SEQ. ID NO. |
| 230 | Q E E K Q K C Y N D | 2 | |
| 301 | T E K I Q K L R E E | 2 | |
| 357 | Q A C T L D F E N E | 2 | |
| 375 | H Q L H V I L K E L | 2 | |
| 395 | E S L K Q L H E F A | 2 | |
| 401 | H E F A I T E P L V | 2 | |
| 411 | T F Q G E T E N R E | 2 | |
| 441 | P K C N I Q Y P A T | 2 | |
| 447 | Y P A T E H R D L L | 2 | |
| 3 | S R S T K D L I K S | 1 | |
| 24 | E T T L E K L K G E | 1 | |
| 81 | D K E I Q R L R D Q | 1 | |
| 143 | E S K T N T L R L S | 1 | |
| 152 | S Q T V A P N C F N | 1 | |
| 158 | N C F N S S I N N I | 1 | |
| 161 | N S S I N N I H E M | 1 | |
| 168 | H E M E I Q L K D A | 1 | |
| 195 | V K G L L A K I F E | 1 | |
| 223 | P E S E G Y L Q E E | 1 | |
| 225 | S E G Y L Q E E K Q | 1 | |
| 229 | L Q E E K Q K C Y N | 1 | |
| 231 | E E K Q K C Y N D L | 1 | |
| 320 | E E K K R S E E L L | 1 | |
| 336 | Y T S L L K Q Q E E | 1 | |
| 340 | L K Q Q E E Q T R V | 1 | |
| 353 | E Q Q M Q A C T L D | 1 | |
| 356 | M Q A C T L D F E N | 1 | |
| 362 | D F E N E K L D R Q | 1 | |
| 364 | E N E K L D R Q H V | 1 | |
| 406 | T E P L V T F Q G E | 1 | |
| 434 | N E S L V E C P K C | 1 | |
| 440 | C P K C N I Q Y P A | 1 | |

| TABLE XXXIX 121P2A3 v.3: HLA Peptide Scoring Results A3 10-mers SYFPEITHI | | | |
|---|---|---|---|
| Pos | 1 2 3 4 5 6 7 8 9 0 | score | SEQ. ID NO. |
| 8 | D K E R Q R L L E K | 16 | |
| 5 | K L T D K E R Q R L | 15 | |
| 4 | G K L T D K E R Q R | 9 | |
| 12 | Q R L L E K I R V L | 9 | |
| 11 | R Q R L L E K I R V | 8 | |
| 10 | E R Q R L L E K I R | 7 | |
| 7 | T D K E R Q R L L E | 6 | |
| 9 | K E R Q R L L E K I | 6 | |
| 3 | K G K L T D K E R Q | 5 | |
| 1 | S G K G K L T D K E | 4 | |
| 2 | G K G K L T D K E R | 4 | |

| TABLE XXXIX 121P2A3 v.4: HLA Peptide Scoring Results A3 10-mers SYFPEITHI | | | |
|---|---|---|---|
| Pos | 1 2 3 4 5 6 7 8 9 0 | score | SEQ. ID NO. |
| 1 | Q L K A R Y S T T T | 20 | |
| 10 | T L L E Q L E E T T | 17 | |
| 4 | A R Y S T T T L L E | 10 | |
| 2 | L K A R Y S T T T L | 9 | |
| 3 | K A R Y S T T T L L | 7 | |
| 5 | R Y S T T T L L E Q | 7 | |
| 8 | T T T L L E Q L E E | 4 | |
| 6 | Y S T T T L L E Q L | 3 | |
| 9 | T T L L E Q L E E T | 3 | |
| 7 | S T T T L L E Q L E | 1 | |

| TABLE XXXIX 121P2A3 v.6: HLA Peptide Scoring Results A3 10-mers SYFPEITHI | | | |
|---|---|---|---|
| Pos | 1 2 3 4 5 6 7 8 9 0 | score | SEQ. ID NO. |
| 3 | E L L S Q V Q S L Y | 21 | |
| 10 | S L Y T S L L K Q Q | 18 | |
| 7 | Q V Q S L Y T S L L | 15 | |
| 8 | V Q S L Y T S L L K | 14 | |
| 4 | L L S Q V Q S L Y T | 13 | |
| 1 | S E E L L S Q V Q S | 9 | |
| 9 | Q S L Y T S L L K Q | 7 | |
| 2 | E E L L S Q V Q S L | 5 | |
| 5 | L S Q V Q S L Y T S | 4 | |
| 6 | S Q V Q S L Y T S L | 4 | |

| TABLE XXXIX 121P2A3 v.7: HLA Peptide Scoring Results A3 10-mers SYFPEITHI | | | |
|---|---|---|---|
| Pos | 1 2 3 4 5 6 7 8 9 0 | score | SEQ. ID NO. |
| 9 | L L V I L K E L R K | 24 | |
| 8 | Q L L V I L K E L R | 18 | |
| 4 | H V Q H Q L L V I L | 14 | |
| 10 | L V I L K E L R K A | 14 | |
| 5 | V Q H Q L L V I L K | 10 | |
| 3 | Q H V Q H Q L L V I | 9 | |
| 2 | R Q H V Q H Q L L V | 7 | |
| 6 | Q H Q L L V I L K E | 6 | |
| 7 | H Q L L V I L K E L | 3 | |

| TABLE XXXIX 121P2A3 v.8: HLA Peptide Scoring Results A3 10-mers SYFPEITHI | | | |
|---|---|---|---|
| Pos | 1 2 3 4 5 6 7 8 9 0 | score | SEQ. ID NO. |
| 7 | A L N G S L V E C P | 15 | |
| 6 | A A L N G S L V E C | 10 | |
| 8 | L N G S L V E C P K | 10 | |
| 1 | P K S P T A A L N G | 9 | |
| 4 | P T A A L N G S L V | 9 | |
| 5 | T A A L N G S L V E | 9 | |
| 3 | S P T A A L N G S L | 6 | |
| 2 | K S P T A A L N G S | 3 | |
| 10 | G S L V E C P K C N | 3 | |
| 9 | N G S L V E C P K C | 1 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 249 | E V E R Q T I T Q L | 29 | |
| 275 | E V H N L N Q L L Y | 26 | |
| 327 | E L L S Q V Q F L Y | 25 | |
| 126 | D V L K Q Q L S A A | 23 | |
| 194 | Y V K G L L A K I F | 23 | |
| 210 | E T A A H S L P Q Q | 23 | |
| 219 | Q T K K P E S E G Y | 23 | |
| 391 | I T Q L E S L K Q L | 23 | |
| 239 | D L L A S A K K D L | 22 | |
| 312 | D I A R G K L E E E | 22 | |
| 24 | E T T L E K L K G E | 21 | |
| 28 | E K L K G E I A H L | 21 | |
| 86 | R L R D Q L K A R Y | 21 | |
| 112 | E R R E Q V L K A L | 21 | |
| 185 | L V Y D Q Q R E V Y | 21 | |
| 192 | E V Y V K G L L A K | 21 | |
| 253 | Q T I T Q L S F E L | 21 | |
| 51 | L T D K E R H R L L | 20 | |
| 228 | Y L Q E E K Q K C Y | 20 | |
| 231 | E E K Q K C Y N D L | 20 | |
| 270 | E E T Q K E V H N L | 20 | |
| 359 | C T L D F E N E K L | 20 | |
| 326 | E E L L S Q V Q F L | 19 | |
| 331 | Q V Q F L Y T S L L | 19 | |
| 372 | H V Q H Q L H V I L | 19 | |
| 8 | D L I K S K W G S K | 18 | |
| 33 | E I A H L K T S V D | 18 | |
| 50 | K L T D K E R H R L | 18 | |
| 123 | E E K D V L K Q Q L | 18 | |
| 154 | T V A P N C F N S S | 18 | |
| 415 | E T E N R E K V A A | 18 | |
| 42 | D E I T S G K G K L | 17 | |
| 43 | E I T S G K G K L T | 17 | |
| 83 | E I Q R L R D Q L K | 17 | |
| 106 | E T T R E G E R R E | 17 | |
| 166 | N I H E M E I Q L K | 17 | |
| 171 | E I Q L K D A L E K | 17 | |
| 176 | D A L E K N Q Q W L | 17 | |
| 251 | E R Q T I T Q L S F | 17 | |
| 256 | T Q L S F E L S E F | 17 | |
| 271 | E T Q K E V H N L N | 17 | |
| 290 | D V Q H L E D D R H | 17 | |
| 362 | D F E N E K L D R Q | 17 | |
| 378 | H V I L K E L R K A | 17 | |
| 403 | F A I T E P L V T F | 17 | |
| 78 | T E K D K E I Q R L | 16 | |
| 145 | K T N T L R L S Q T | 16 | |
| 204 | E L E K K T E T A A | 16 | |
| 261 | E L S E F R R K Y E | 16 | |
| 309 | E E N D I A R G K L | 16 | |
| 319 | E E E K K R S E E L | 16 | |
| 320 | E E K K R S E E L L | 16 | |
| 350 | A L L E Q Q M Q A C | 16 | |
| 383 | E L R K A R N Q I T | 16 | |
| 404 | A I T E P L V T F Q | 16 | |
| 21 | S K S E T T L E K L | 15 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 38 | K T S V D E I T S G | 15 | |
| 133 | S A A T S R I A E L | 15 | |
| 141 | E L E S K T N T L R | 15 | |
| 169 | E M E I Q L K D A L | 15 | |
| 189 | Q Q R E V Y V K G L | 15 | |
| 232 | E K Q K C Y N D L L | 15 | |
| 254 | T I T Q L S F E L S | 15 | |
| 278 | N L N Q L L Y S Q R | 15 | |
| 298 | R H K T E K I Q K L | 15 | |
| 346 | Q T R V A L L E Q Q | 15 | |
| 367 | K L D R Q H V Q H Q | 15 | |
| 394 | L E S L K Q L H E F | 15 | |
| 399 | Q L H E F A I T E P | 15 | |
| 5 | S T K D L I K S K W | 14 | |
| 9 | L I K S K W G S K P | 14 | |
| 40 | S V D E I T S G K G | 14 | |
| 59 | L L E K I R V L E A | 14 | |
| 66 | L E A E K E K N A Y | 14 | |
| 201 | K I F E L E K K T E | 14 | |
| 247 | D L E V E R Q T I T | 14 | |
| 285 | S Q R R A D V Q H L | 14 | |
| 330 | S Q V Q F L Y T S L | 14 | |
| 343 | Q E E Q T R V A L L | 14 | |
| 385 | R K A R N Q I T Q L | 14 | |
| 410 | V T F Q G E T E N R | 14 | |
| 432 | A L N E S L V E C P | 14 | |
| 449 | A T E H R D L L V H | 14 | |
| 454 | D L L V H V E Y C S | 14 | |
| 25 | T T L E K L K G E I | 13 | |
| 64 | R V L E A E K E K N | 13 | |
| 95 | Y S T T A L L E Q L | 13 | |
| 107 | T T R E G E R R E Q | 13 | |
| 138 | R I A E L E S K T N | 13 | |
| 161 | N S S I N N I H E M | 13 | |
| 196 | K G L L A K I F E L | 13 | |
| 273 | Q K E V H N L N Q L | 13 | |
| 328 | L L S Q V Q F L Y T | 13 | |
| 334 | F L Y T S L L K Q Q | 13 | |
| 348 | R V A L L E Q Q M Q | 13 | |
| 375 | H Q L H V I L K E L | 13 | |
| 380 | I L K E L R K A R N | 13 | |
| 388 | R N Q I T Q L E S L | 13 | |
| 402 | E F A I T E P L V T | 13 | |
| 405 | I T E P L V T F Q G | 13 | |
| 438 | V E C P K C N I Q Y | 13 | |
| 439 | E C P K C N I Q Y P | 13 | |
| 452 | H R D L L V H V E Y | 13 | |
| 455 | L L V H V E Y C S K | 13 | |
| 44 | I T S G K G K L T D | 12 | |
| 57 | H R L L E K I R V L | 12 | |
| 68 | A E K E K N A Y Q L | 12 | |
| 69 | E K E K N A Y Q L T | 12 | |
| 77 | L T E K D K E I Q R | 12 | |
| 82 | K E I Q R L R D Q L | 12 | |
| 89 | D Q L K A R Y S T T | 12 | |
| 97 | T T A L L E Q L E E | 12 | |

TABLE XL 121P2A3 v.1: HLA Peptide Scoring Results A26 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 115 | E | Q | V | L | K | A | L | S | E | E | 12 | |
| 116 | Q | V | L | K | A | L | S | E | E | K | 12 | |
| 127 | V | L | K | Q | Q | L | S | A | A | T | 12 | |
| 163 | S | I | N | N | I | H | E | M | E | I | 12 | |
| 179 | E | K | N | Q | Q | W | L | V | Y | D | 12 | |
| 188 | D | Q | Q | R | E | V | Y | V | K | G | 12 | |
| 197 | G | L | L | A | K | I | F | E | L | E | 12 | |
| 260 | F | E | L | S | E | F | R | R | K | Y | 12 | |
| 264 | E | F | R | R | K | Y | E | E | T | Q | 12 | |
| 300 | K | T | E | K | I | Q | K | L | R | E | 12 | |
| 303 | K | I | Q | K | L | R | E | E | N | D | 12 | |
| 325 | S | E | E | L | L | S | Q | V | Q | F | 12 | |
| 336 | Y | T | S | L | L | K | Q | Q | E | E | 12 | |
| 342 | Q | Q | E | E | Q | T | R | V | A | L | 12 | |
| 379 | V | I | L | K | E | L | R | K | A | R | 12 | |
| 390 | Q | I | T | Q | L | E | S | L | K | Q | 12 | |
| 421 | K | V | A | A | S | P | K | S | P | T | 12 | |
| 424 | A | S | P | K | S | P | T | A | A | L | 12 | |
| 429 | P | T | A | A | L | N | E | S | L | V | 12 | |
| 29 | K | L | K | G | E | I | A | H | L | K | 11 | |
| 53 | D | K | E | R | H | R | L | L | E | K | 11 | |
| 58 | R | L | L | E | K | I | R | V | L | E | 11 | |
| 62 | K | I | R | V | L | E | A | E | K | E | 11 | |
| 71 | E | K | N | A | Y | Q | L | T | E | K | 11 | |
| 96 | S | T | T | A | L | L | E | Q | L | E | 11 | |
| 103 | Q | L | E | E | T | T | R | E | G | E | 11 | |
| 109 | R | E | G | E | R | R | E | Q | V | L | 11 | |
| 120 | A | L | S | E | E | K | D | V | L | K | 11 | |
| 135 | A | T | S | R | I | A | E | L | E | S | 11 | |
| 143 | E | S | K | T | N | T | L | R | L | S | 11 | |
| 150 | R | L | S | Q | T | V | A | P | N | C | 11 | |
| 153 | Q | T | V | A | P | N | C | F | N | S | 11 | |
| 165 | N | N | I | H | E | M | E | I | Q | L | 11 | |
| 173 | Q | L | K | D | A | L | E | K | N | Q | 11 | |
| 178 | L | E | K | N | Q | Q | W | L | V | Y | 11 | |
| 198 | L | L | A | K | I | F | E | L | E | K | 11 | |
| 207 | K | K | T | E | T | A | A | H | S | L | 11 | |
| 208 | K | T | E | T | A | A | H | S | L | P | 11 | |
| 220 | T | K | K | P | E | S | E | G | Y | L | 11 | |
| 255 | I | T | Q | L | S | F | E | L | S | E | 11 | |
| 259 | S | F | E | L | S | E | F | R | R | K | 11 | |
| 306 | K | L | R | E | E | N | D | I | A | R | 11 | |
| 317 | K | L | E | E | E | K | K | R | S | E | 11 | |
| 333 | Q | F | L | Y | T | S | L | L | K | Q | 11 | |
| 339 | L | L | K | Q | Q | E | E | Q | T | R | 11 | |
| 345 | E | Q | T | R | V | A | L | L | E | Q | 11 | |
| 354 | Q | Q | M | Q | A | C | T | L | D | F | 11 | |
| 396 | S | L | K | Q | L | H | E | F | A | I | 11 | |
| 409 | L | V | T | F | Q | G | E | T | E | N | 11 | |
| 436 | S | L | V | E | C | P | K | C | N | I | 11 | |
| 444 | N | I | Q | Y | P | A | T | E | H | R | 11 | |
| 451 | E | H | R | D | L | L | V | H | V | E | 11 | |
| 61 | E | K | I | R | V | L | E | A | E | K | 10 | |
| 76 | Q | L | T | E | K | D | K | E | I | Q | 10 | |
| 81 | D | K | E | I | Q | R | L | R | D | Q | 10 | |
| 91 | L | K | A | R | Y | S | T | T | A | L | 10 | |

TABLE XL 121P2A3 v.1: HLA Peptide Scoring Results A26 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 99 | A | L | L | E | Q | L | E | E | T | T | 10 | |
| 117 | V | L | K | A | L | S | E | E | K | D | 10 | |
| 140 | A | E | L | E | S | K | T | N | T | L | 10 | |
| 142 | L | E | S | K | T | N | T | L | R | L | 10 | |
| 147 | N | T | L | R | L | S | Q | T | V | A | 10 | |
| 148 | T | L | R | L | S | Q | T | V | A | P | 10 | |
| 151 | L | S | Q | T | V | A | P | N | C | F | 10 | |
| 215 | S | L | P | Q | Q | T | K | K | P | E | 10 | |
| 240 | L | L | A | S | A | K | K | D | L | E | 10 | |
| 282 | L | L | Y | S | Q | R | R | A | D | V | 10 | |
| 302 | E | K | I | Q | K | L | R | E | E | N | 10 | |
| 338 | S | L | L | K | Q | Q | E | E | Q | T | 10 | |
| 347 | T | R | V | A | L | L | E | Q | Q | M | 10 | |
| 351 | L | L | E | Q | Q | M | Q | A | C | T | 10 | |
| 368 | L | D | R | Q | H | V | Q | H | Q | L | 10 | |
| 393 | Q | L | E | S | L | K | Q | L | H | E | 10 | |
| 417 | E | N | R | E | K | V | A | A | S | P | 10 | |
| 437 | L | V | E | C | P | K | C | N | I | Q | 10 | |
| 18 | P | S | N | S | K | S | E | T | T | L | 9 | |
| 26 | T | L | E | K | L | K | G | E | I | A | 9 | |
| 36 | H | L | K | T | S | V | D | E | I | T | 9 | |
| 65 | V | L | E | A | E | K | E | K | N | A | 9 | |
| 90 | Q | L | K | A | R | Y | S | T | T | A | 9 | |
| 92 | K | A | R | Y | S | T | T | A | L | L | 9 | |
| 100 | L | L | E | Q | L | E | E | T | T | R | 9 | |
| 102 | E | Q | L | E | E | T | T | R | E | G | 9 | |
| 119 | K | A | L | S | E | E | K | D | V | L | 9 | |
| 177 | A | L | E | K | N | Q | Q | W | L | V | 9 | |
| 184 | W | L | V | Y | D | Q | Q | R | E | V | 9 | |
| 206 | E | K | K | T | E | T | A | A | H | S | 9 | |
| 224 | E | S | E | G | Y | L | Q | E | E | K | 9 | |
| 274 | K | E | V | H | N | L | N | Q | L | L | 9 | |
| 281 | Q | L | L | Y | S | Q | R | R | A | D | 9 | |
| 293 | H | L | E | D | D | R | H | K | T | E | 9 | |
| 352 | L | E | Q | Q | M | Q | A | C | T | L | 9 | |
| 360 | T | L | D | F | E | N | E | K | L | D | 9 | |
| 364 | E | N | E | K | L | D | R | Q | H | V | 9 | |
| 366 | E | K | L | D | R | Q | H | V | Q | H | 9 | |
| 411 | T | F | Q | G | E | T | E | N | R | E | 9 | |
| 428 | S | P | T | A | A | L | N | E | S | L | 9 | |
| 446 | Q | Y | P | A | T | E | H | R | D | L | 9 | |
| 447 | Y | P | A | T | E | H | R | D | L | L | 9 | |
| 67 | E | A | E | K | E | K | N | A | Y | Q | 8 | |
| 79 | E | K | D | K | E | I | Q | R | L | R | 8 | |
| 124 | E | K | D | V | L | K | Q | Q | L | S | 8 | |
| 131 | Q | L | S | A | A | T | S | R | I | A | 8 | |
| 190 | Q | R | E | V | Y | V | K | G | L | L | 8 | |
| 223 | P | E | S | E | G | Y | L | Q | E | E | 8 | |
| 257 | Q | L | S | F | E | L | S | E | F | R | 8 | |
| 295 | E | D | D | R | H | K | T | E | K | I | 8 | |
| 297 | D | R | H | K | T | E | K | I | Q | K | 8 | |
| 307 | L | R | E | E | N | D | I | A | R | G | 8 | |
| 323 | K | R | S | E | E | L | L | S | Q | V | 8 | |
| 376 | Q | L | H | V | I | L | K | E | L | R | 8 | |
| 400 | L | H | E | F | A | I | T | E | P | L | 8 | |
| 408 | P | L | V | T | F | Q | G | E | T | E | 8 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 105 | E E T T R E G E R R | 7 | |
| 110 | E G E R R E Q V L K | 7 | |
| 121 | L S E E K D V L K Q | 7 | |
| 158 | N C F N S S I N N I | 7 | |
| 159 | C F N S S I N N I H | 7 | |
| 193 | V Y V K G L L A K I | 7 | |
| 202 | I F E L E K K T E T | 7 | |
| 226 | E G Y L Q E E K Q K | 7 | |
| 244 | A K K D L E V E R Q | 7 | |
| 263 | S E F R R K Y E E T | 7 | |
| 310 | E N D I A R G K L E | 7 | |
| 321 | E K K R S E E L L S | 7 | |
| 369 | D R Q H V Q H Q L H | 7 | |
| 395 | E S L K Q L H E F A | 7 | |
| 406 | T E P L V T F Q G E | 7 | |
| 407 | E P L V T F Q G E T | 7 | |
| 420 | E K V A A S P K S P | 7 | |
| 4 | R S T K D L I K S K | 6 | |
| 35 | A H L K T S V D E I | 6 | |
| 55 | E R H R L L E K I R | 6 | |
| 60 | L E K I R V L E A E | 6 | |
| 122 | S E E K D V L K Q Q | 6 | |
| 149 | L R L S Q T V A P N | 6 | |
| 168 | H E M E I Q L K D A | 6 | |
| 172 | I Q L K D A L E K N | 6 | |
| 199 | L A K I F E L E K K | 6 | |
| 222 | K P E S E G Y L Q E | 6 | |
| 235 | K C Y N D L L A S A | 6 | |
| 242 | A S A K K D L E V E | 6 | |
| 296 | D D R H K T E K I Q | 6 | |
| 301 | T E K I Q K L R E E | 6 | |
| 322 | K K R S E E L L S Q | 6 | |
| 344 | E E Q T R V A L L E | 6 | |
| 353 | E Q Q M Q A C T L D | 6 | |
| 416 | T E N R E K V A A S | 6 | |
| 427 | K S P T A A L N E S | 6 | |
| 431 | A A L N E S L V E C | 6 | |
| 435 | E S L V E C P K C N | 6 | |
| 441 | P K C N I Q Y P A T | 6 | |
| 450 | T E H R D L L V H V | 6 | |
| 3 | S R S T K D L I K S | 5 | |
| 45 | T S G K G K L T D K | 5 | |
| 54 | K E R H R L L E K I | 5 | |
| 85 | Q R L R D Q L K A R | 5 | |
| 94 | R Y S T T A L L E Q | 5 | |
| 98 | T A L L E Q L E E T | 5 | |
| 111 | G E R R E Q V L K A | 5 | |
| 136 | T S R I A E L E S K | 5 | |
| 180 | K N Q Q W L V Y D Q | 5 | |
| 181 | N Q Q W L V Y D Q Q | 5 | |
| 187 | Y D Q Q R E V Y V K | 5 | |
| 234 | Q K C Y N D L L A S | 5 | |
| 252 | R Q T I T Q L S F E | 5 | |
| 258 | L S F E L S E F R R | 5 | |
| 277 | H N L N Q L L Y S Q | 5 | |
| 288 | R A D V Q H L E D D | 5 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 357 | Q A C T L D F E N E | 5 | |
| 371 | Q H V Q H Q L H V I | 5 | |
| 373 | V Q H Q L H V I L K | 5 | |
| 374 | Q H Q L H V I L K E | 5 | |
| 397 | L K Q L H E F A I T | 5 | |
| 12 | S K W G S K P S N S | 4 | |
| 15 | G S K P S N S K S E | 4 | |
| 16 | S K P S N S K S E T | 4 | |
| 31 | K G E I A H L K T S | 4 | |
| 46 | S G K G K L T D K E | 4 | |
| 80 | K D K E I Q R L R D | 4 | |
| 137 | S R I A E L E S K T | 4 | |
| 164 | I N N I H E M E I Q | 4 | |
| 170 | M E I Q L K D A L E | 4 | |
| 205 | L E K K T E T A A H | 4 | |
| 243 | S A K K D L E V E R | 4 | |
| 272 | T Q K E V H N L N Q | 4 | |
| 276 | V H N L N Q L L Y S | 4 | |
| 318 | L E E E K K R S E E | 4 | |
| 329 | L S Q V Q F L Y T S | 4 | |
| 355 | Q M Q A C T L D F E | 4 | |
| 361 | L D F E N E K L D R | 4 | |
| 443 | C N I Q Y P A T E H | 4 | |
| 453 | R D L L V H V E Y C | 4 | |
| 11 | K S K W G S K P S N | 3 | |
| 14 | W G S K P S N S K S | 3 | |
| 20 | N S K S E T T L E K | 3 | |
| 30 | L K G E I A H L K T | 3 | |
| 32 | G E I A H L K T S V | 3 | |
| 39 | T S V D E I T S G K | 3 | |
| 52 | T D K E R H R L L E | 3 | |
| 108 | T R E G E R R E Q V | 3 | |
| 130 | Q Q L S A A T S R I | 3 | |
| 155 | V A P N C F N S S I | 3 | |
| 162 | S S I N N I H E M E | 3 | |
| 175 | K D A L E K N Q Q W | 3 | |
| 186 | V Y D Q Q R E V Y V | 3 | |
| 200 | A K I F E L E K K T | 3 | |
| 211 | T A A H S L P Q Q T | 3 | |
| 214 | H S L P Q Q T K K P | 3 | |
| 216 | L P Q Q T K K P E S | 3 | |
| 218 | Q Q T K K P E S E G | 3 | |
| 221 | K K P E S E G Y L Q | 3 | |
| 227 | G Y L Q E E K Q K C | 3 | |
| 236 | C Y N D L L A S A K | 3 | |
| 237 | Y N D L L A S A K K | 3 | |
| 246 | K D L E V E R Q T I | 3 | |
| 266 | R R K Y E E T Q K E | 3 | |
| 267 | R K Y E E T Q K E V | 3 | |
| 287 | R R A D V Q H L E D | 3 | |
| 292 | Q H L E D D R H K T | 3 | |
| 294 | L E D D R H K T E K | 3 | |
| 311 | N D I A R G K L E E | 3 | |
| 313 | I A R G K L E E E K | 3 | |
| 316 | G K L E E E K K R S | 3 | |
| 324 | R S E E L L S Q V Q | 3 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 335 | L Y T S L L K Q Q E | 3 | |
| 363 | F E N E K L D R Q H | 3 | |
| 389 | N Q I T Q L E S L K | 3 | |
| 392 | T Q L E S L K Q L H | 3 | |
| 401 | H E F A I T E P L V | 3 | |
| 412 | F Q G E T E N R E K | 3 | |
| 419 | R E K V A A S P K S | 3 | |
| 422 | V A A S P K S P T A | 3 | |
| 423 | A A S P K S P T A A | 3 | |
| 425 | S P K S P T A A L N | 3 | |
| 426 | P K S P T A A L N E | 3 | |
| 445 | I Q Y P A T E H R D | 3 | |
| 13 | K W G S K P S N S K | 2 | |
| 27 | L E K L K G E I A H | 2 | |
| 47 | G K G K L T D K E R | 2 | |
| 48 | K G K L T D K E R H | 2 | |
| 72 | K N A Y Q L T E K D | 2 | |
| 87 | L R D Q L K A R Y S | 2 | |
| 113 | R R E Q V L K A L S | 2 | |
| 128 | L K Q Q L S A A T S | 2 | |
| 132 | L S A A T S R I A E | 2 | |
| 160 | F N S S I N N I H E | 2 | |
| 174 | L K D A L E K N Q Q | 2 | |
| 182 | Q Q W L V Y D Q Q R | 2 | |
| 183 | Q W L V Y D Q Q R E | 2 | |
| 203 | F E L E K K T E T A | 2 | |
| 212 | A A H S L P Q Q T K | 2 | |
| 229 | L Q E E K Q K C Y N | 2 | |
| 230 | Q E E K Q K C Y N D | 2 | |
| 245 | K K D L E V E R Q T | 2 | |
| 248 | L E V E R Q T I T Q | 2 | |
| 250 | V E R Q T I T Q L S | 2 | |
| 265 | F R R K Y E E T Q K | 2 | |
| 268 | K Y E E T Q K E V H | 2 | |
| 269 | Y E E T Q K E V H N | 2 | |
| 279 | L N Q L L Y S Q R R | 2 | |
| 284 | Y S Q R R A D V Q H | 2 | |
| 299 | H K T E K I Q K L R | 2 | |
| 314 | A R G K L E E E K K | 2 | |
| 315 | R G K L E E E K K R | 2 | |
| 332 | V Q F L Y T S L L K | 2 | |
| 340 | L K Q Q E E Q T R V | 2 | |
| 341 | K Q Q E E Q T R V A | 2 | |
| 349 | V A L L E Q Q M Q A | 2 | |
| 356 | M Q A C T L D F E N | 2 | |
| 387 | A R N Q I T Q L E S | 2 | |
| 414 | G E T E N R E K V A | 2 | |
| 418 | N R E K V A A S P K | 2 | |
| 434 | N E S L V E C P K C | 2 | |
| 448 | P A T E H R D L L V | 2 | |
| 2 | S S R S T K D L I K | 1 | |
| 6 | T K D L I K S K W G | 1 | |
| 7 | K D L I K S K W G S | 1 | |
| 10 | I K S K W G S K P S | 1 | |
| 19 | S N S K S E T T L E | 1 | |
| 23 | S E T T L E K L K G | 1 | |

| TABLE XL 121P2A3 v.1: HLA Peptide Scoring Results A26 10-mers SYFPEITHI | | | |
|---|---|---|---|
| Pos | 1 2 3 4 5 6 7 8 9 0 | score | SEQ. ID NO. |
| 34 | I A H L K T S V D E | 1 | |
| 37 | L K T S V D E I T S | 1 | |
| 41 | V D E I T S G K G K | 1 | |
| 49 | G K L T D K E R H R | 1 | |
| 56 | R H R L L E K I R V | 1 | |
| 63 | I R V L E A E K E K | 1 | |
| 70 | K E K N A Y Q L T E | 1 | |
| 73 | N A Y Q L T E K D K | 1 | |
| 74 | A Y Q L T E K D K E | 1 | |
| 75 | Y Q L T E K D K E I | 1 | |
| 84 | I Q R L R D Q L K A | 1 | |
| 88 | R D Q L K A R Y S T | 1 | |
| 93 | A R Y S T T A L L E | 1 | |
| 101 | L E Q L E E T T R E | 1 | |
| 104 | L E E T T R E G E R | 1 | |
| 114 | R E Q V L K A L S E | 1 | |
| 118 | L K A L S E E K D V | 1 | |
| 125 | K D V L K Q Q L S A | 1 | |
| 129 | K Q Q L S A A T S R | 1 | |
| 134 | A A T S R I A E L E | 1 | |
| 139 | I A E L E S K T N T | 1 | |
| 144 | S K T N T L R L S Q | 1 | |
| 146 | T N T L R L S Q T V | 1 | |
| 156 | A P N C F N S S I N | 1 | |
| 157 | P N C F N S S I N N | 1 | |
| 167 | I H E M E I Q L K D | 1 | |
| 213 | A H S L P Q Q T K K | 1 | |
| 217 | P Q Q T K K P E S E | 1 | |
| 225 | S E G Y L Q E E K Q | 1 | |
| 233 | K Q K C Y N D L L A | 1 | |
| 238 | N D L L A S A K K D | 1 | |
| 241 | L A S A K K D L E V | 1 | |
| 262 | L S E F R R K Y E E | 1 | |
| 280 | N Q L L Y S Q R R A | 1 | |
| 283 | L Y S Q R R A D V Q | 1 | |
| 289 | A D V Q H L E D D R | 1 | |
| 291 | V Q H L E D D R H K | 1 | |
| 304 | I Q K L R E E N D I | 1 | |
| 308 | R E E N D I A R G K | 1 | |
| 337 | T S L L K Q Q E E Q | 1 | |
| 358 | A C T L D F E N E K | 1 | |
| 365 | N E K L D R Q H V Q | 1 | |
| 370 | R Q H V Q H Q L H V | 1 | |
| 377 | L H V I L K E L R K | 1 | |
| 381 | L K E L R K A R N Q | 1 | |
| 382 | K E L R K A R N Q I | 1 | |
| 384 | L R K A R N Q I T Q | 1 | |
| 386 | K A R N Q I T Q L E | 1 | |
| 413 | Q G E T E N R E K V | 1 | |
| 430 | T A A L N E S L V E | 1 | |
| 433 | L N E S L V E C P K | 1 | |
| 440 | C P K C N I Q Y P A | 1 | |
| 442 | K C N I Q Y P A T E | 1 | |

| TABLE XL 121P2A3 v.3: HLA Peptide Scoring Results A26 10-mers SYFPEITHI | | | |
|---|---|---|---|
| Pos | 1 2 3 4 5 6 7 8 9 0 | score | SEQ. ID NO. |
| 6 | L T D K E R Q R L L | 20 | |
| 5 | K L T D K E R Q R L | 19 | |
| 12 | Q R L L E K I R V L | 12 | |
| 8 | D K E R Q R L L E K | 11 | |
| 9 | K E R Q R L L E K I | 6 | |
| 10 | E R Q R L L E K I R | 6 | |
| 1 | S G K G K L T D K E | 4 | |
| 7 | T D K E R Q R L L E | 3 | |
| 2 | G K G K L T D K E R | 2 | |
| 3 | K G K L T D K E R Q | 2 | |
| 4 | G K L T D K E R Q R | 1 | |
| 11 | R Q R L L E K I R V | 1 | |

| TABLE XL 121P2A3 v.4: HLA Peptide Scoring Results A26 10-mers SYFPEITHI | | | |
|---|---|---|---|
| Pos | 1 2 3 4 5 6 7 8 9 0 | score | SEQ. ID NO. |
| 9 | T T L L E Q L E E T | 15 | |
| 6 | Y S T T T L L E Q L | 13 | |
| 7 | S T T T L L E Q L E | 11 | |
| 8 | T T T L L E Q L E E | 11 | |
| 10 | T L L E Q L E E T T | 10 | |
| 1 | Q L K A R Y S T T T | 9 | |
| 2 | L K A R Y S T T T L | 9 | |
| 3 | K A R Y S T T T L L | 8 | |
| 5 | R Y S T T T L L E Q | 5 | |
| 4 | A R Y S T T T L L E | 1 | |

| TABLE XL 121P2A3 v.6: HLA Peptide Scoring Results A26 10-mers SYFPEITHI | | | |
|---|---|---|---|
| Pos | 1 2 3 4 5 6 7 8 9 0 | score | SEQ. ID NO. |
| 3 | E L L S Q V Q S L Y | 26 | |
| 2 | E E L L S Q V Q S L | 20 | |
| 7 | Q V Q S L Y T S L L | 20 | |
| 6 | S Q V Q S L Y T S L | 14 | |
| 10 | S L Y T S L L K Q Q | 13 | |
| 4 | L L S Q V Q S L Y T | 9 | |
| 9 | Q S L Y T S L L K Q | 5 | |
| 5 | L S Q V Q S L Y T S | 4 | |
| 1 | S E E L L S Q V Q S | 2 | |

| TABLE XL 121P2A3 v.7: HLA Peptide Scoring Results A26 10-mers SYFPEITHI | | | |
|---|---|---|---|
| Pos | 1 2 3 4 5 6 7 8 9 0 | score | SEQ. ID NO. |
| 4 | H V Q H Q L L V I L | 23 | |
| 10 | L V I L K E L R K A | 17 | |
| 1 | D R Q H V Q H Q L L | 15 | |
| 7 | H Q L L V I L K E L | 13 | |
| 9 | L L V I L K E L R K | 9 | |
| 8 | Q L L V I L K E L R | 8 | |
| 3 | Q H V Q H Q L L V I | 5 | |
| 5 | V Q H Q L L V I L K | 5 | |
| 6 | Q H Q L L V I L K E | 5 | |

| TABLE XL 121P2A3 v.8: HLA Peptide Scoring Results A26 10-mers SYFPEITHI | | | |
|---|---|---|---|
| Pos | 1 2 3 4 5 6 7 8 9 0 | score | SEQ. ID NO. |
| 7 | A L N G S L V E C P | 14 | |
| 4 | P T A A L N G S L V | 12 | |
| 3 | S P T A A L N G S L | 9 | |
| 2 | K S P T A A L N G S | 6 | |
| 6 | A A L N G S L V E C | 6 | |
| 1 | P K S P T A A L N G | 3 | |
| 9 | N G S L V E C P K C | 2 | |
| 5 | T A A L N G S L V E | 1 | |
| 8 | L N G S L V E C P K | 1 | |

| TABLE XLI 121P2A3 v.1: HLA Peptide Scoring Results B*0702 10-mers SYFPEITHI | | | |
|---|---|---|---|
| Pos | 1 2 3 4 5 6 7 8 9 0 | score | SEQ. ID NO. |
| 447 | Y P A T E H R D L L | 22 | |
| 428 | S P T A A L N E S L | 21 | |
| 17 | K P S N S K S E T T | 19 | |
| 407 | E P L V T F Q G E T | 17 | |
| 440 | C P K C N I Q Y P A | 17 | |
| 142 | L E S K T N T L R L | 16 | |
| 424 | A S P K S P T A A L | 16 | |
| 92 | K A R Y S T T A L L | 15 | |
| 91 | L K A R Y S T T A L | 14 | |
| 112 | E R R E Q V L K A L | 14 | |
| 342 | Q Q E E Q T R V A L | 14 | |
| 28 | E K L K G E I A H L | 13 | |
| 109 | R E G E R R E Q V L | 13 | |
| 140 | A E L E S K T N T L | 13 | |
| 189 | Q Q R E V Y V K G L | 13 | |
| 222 | K P E S E G Y L Q E | 13 | |
| 285 | S Q R R A D V Q H L | 13 | |
| 326 | E E L L S Q V Q F L | 13 | |
| 385 | R K A R N Q I T Q L | 13 | |
| 423 | A A S P K S P T A A | 13 | |
| 21 | S K S E T T L E K L | 12 | |
| 50 | K L T D K E R H R L | 12 | |
| 51 | L T D K E R H R L L | 12 | |
| 68 | A E K E K N A Y Q L | 12 | |
| 82 | K E I Q R L R D Q L | 12 | |
| 119 | K A L S E E K D V L | 12 | |
| 133 | S A A T S R I A E L | 12 | |
| 156 | A P N C F N S S I N | 12 | |
| 169 | E M E I Q L K D A L | 12 | |
| 232 | E K Q K C Y N D L L | 12 | |
| 249 | E V E R Q T I T Q L | 12 | |
| 270 | E E T Q K E V H N L | 12 | |
| 309 | E E N D I A R G K L | 12 | |
| 319 | E E E K K R S E E L | 12 | |
| 320 | E E K K R S E E L L | 12 | |
| 343 | Q E E Q T R V A L L | 12 | |
| 368 | L D R Q H V Q H Q L | 12 | |
| 372 | H V Q H Q L H V I L | 12 | |
| 400 | L H E F A I T E P L | 12 | |
| 18 | P S N S K S E T T L | 11 | |

TABLE XLI 121P2A3 v.1: HLA Peptide Scoring Results B*0702 10-mers SYFPEITHI

| Pos | 1 2 3 4 5 6 7 8 9 0 | score | SEQ. ID NO. |
|---|---|---|---|
| 57 | H R L L E K I R V L | 11 | |
| 84 | I Q R L R D Q L K A | 11 | |
| 111 | G E R R E Q V L K A | 11 | |
| 123 | E E K D V L K Q Q L | 11 | |
| 196 | K G L L A K I F E L | 11 | |
| 207 | K K T E T A A H S L | 11 | |
| 216 | L P Q Q T K K P E S | 11 | |
| 220 | T K K P E S E G Y L | 11 | |
| 231 | E E K Q K C Y N D L | 11 | |
| 239 | D L L A S A K K D L | 11 | |
| 241 | L A S A K K D L E V | 11 | |
| 274 | K E V H N L N Q L L | 11 | |
| 298 | R H K T E K I Q K L | 11 | |
| 328 | L L S Q V Q F L Y T | 11 | |
| 330 | S Q V Q F L Y T S L | 11 | |
| 331 | Q V Q F L Y T S L L | 11 | |
| 388 | R N Q I T Q L E S L | 11 | |
| 391 | I T Q L E S L K Q L | 11 | |
| 402 | E F A I T E P L V T | 11 | |
| 425 | S P K S P T A A L N | 11 | |
| 446 | Q Y P A T E H R D L | 11 | |
| 35 | A H L K T S V D E I | 10 | |
| 42 | D E I T S G K G K L | 10 | |
| 59 | L L E K I R V L E A | 10 | |
| 78 | T E K D K E I Q R L | 10 | |
| 95 | Y S T T A L L E Q L | 10 | |
| 165 | N N I H E M E I Q L | 10 | |
| 176 | D A L E K N Q Q W L | 10 | |
| 190 | Q R E V Y Y V K G L L | 10 | |
| 204 | E L E K K T E T A A | 10 | |
| 253 | Q T I T Q L S F E L | 10 | |
| 273 | Q K E V H N L N Q L | 10 | |
| 352 | L E Q Q M Q A C T L | 10 | |
| 354 | Q Q M Q A C T L D F | 10 | |
| 359 | C T L D F E N E K L | 10 | |
| 375 | H Q L H V I L K E L | 10 | |
| 383 | E L R K A R N Q I T | 10 | |
| 415 | E T E N R E K V A A | 10 | |
| 421 | K V A A S P K S P T | 10 | |
| 30 | L K G E I A H L K T | 9 | |
| 54 | K E R H R L L E K I | 9 | |
| 56 | R H R L L E K I R V | 9 | |
| 108 | T R E G E R R E Q V | 9 | |
| 125 | K D V L K Q Q L S A | 9 | |
| 131 | Q L S A A T S R I A | 9 | |
| 177 | A L E K N Q Q W L V | 9 | |
| 186 | V Y D Q Q R E V Y V | 9 | |
| 191 | R E V Y V K G L L A | 9 | |
| 233 | K Q K C Y N D L L A | 9 | |
| 251 | E R Q T I T Q L S F | 9 | |
| 295 | E D D R H K T E K I | 9 | |
| 323 | K R S E E L L S Q V | 9 | |
| 341 | K Q Q E E Q T R V A | 9 | |
| 364 | E N E K L D R Q H V | 9 | |
| 370 | R Q H V Q H Q L H V | 9 | |
| 395 | E S L K Q L H E F A | 9 | |
| 1 | M S S R S T K D L I | 8 | |
| 32 | G E I A H L K T S V | 8 | |
| 69 | E K E K N A Y Q L T | 8 | |
| 88 | R D Q L K A R Y S T | 8 | |
| 90 | Q L K A R Y S T T A | 8 | |
| 99 | A L L E Q L E E T T | 8 | |
| 126 | D V L K Q Q L S A A | 8 | |
| 127 | V L K Q Q L S A A T | 8 | |
| 139 | I A E L E S K T N T | 8 | |
| 147 | N T L R L S Q T V A | 8 | |
| 161 | N S S I N N I H E M | 8 | |
| 193 | V Y V K G L L A K I | 8 | |
| 194 | Y V K G L L A K I F | 8 | |
| 200 | A K I F E L E K K T | 8 | |
| 202 | I F E L E K K T E T | 8 | |
| 235 | K C Y N D L L A S A | 8 | |
| 245 | K K D L E V E R Q T | 8 | |
| 246 | K D L E V E R Q T I | 8 | |
| 282 | L L Y S Q R R A D V | 8 | |
| 325 | S E E L L S Q V Q F | 8 | |
| 382 | K E L R K A R N Q I | 8 | |
| 394 | L E S L K Q L H E F | 8 | |
| 397 | L K Q L H E F A I T | 8 | |
| 401 | H E F A I T E P L V | 8 | |
| 403 | F A I T E P L V T F | 8 | |
| 422 | V A A S P K S P T A | 8 | |
| 429 | P T A A L N E S L V | 8 | |
| 441 | P K C N I Q Y P A T | 8 | |
| 448 | P A T E H R D L L V | 8 | |
| 450 | T E H R D L L V H V | 8 | |
| 26 | T L E K L K G E I A | 7 | |
| 43 | E I T S G K G K L T | 7 | |
| 44 | I T S G K G K L T D | 7 | |
| 65 | V L E A E K E K N A | 7 | |
| 89 | D Q L K A R Y S T T | 7 | |
| 118 | L K A L S E E K D V | 7 | |
| 130 | Q Q L S A A T S R I | 7 | |
| 137 | S R I A E L E S K T | 7 | |
| 145 | K T N T L R L S Q T | 7 | |
| 168 | H E M E I Q L K D A | 7 | |
| 203 | F E L E K K T E T A | 7 | |
| 211 | T A A H S L P Q Q T | 7 | |
| 247 | D L E V E R Q T I T | 7 | |
| 267 | R K Y E E T Q K E V | 7 | |
| 292 | Q H L E D D R H K T | 7 | |
| 304 | I Q K L R E E N D I | 7 | |
| 338 | S L L K Q Q E E Q T | 7 | |
| 340 | L K Q Q E E Q T R V | 7 | |
| 347 | T R V A L L E Q Q M | 7 | |
| 351 | L L E Q Q M Q A C T | 7 | |
| 371 | Q H V Q H Q L H V I | 7 | |
| 396 | S L K Q L H E F A I | 7 | |
| 413 | Q G E T E N R E K V | 7 | |
| 414 | G E T E N R E K V A | 7 | |
| 16 | S K P S N S K S E T | 6 | |
| 25 | T T L E K L K G E I | 6 | |

| TABLE XLI 121P2A3 v.1: HLA Peptide Scoring Results B*0702 10-mers SYFPEITHI | | | |
|---|---|---|---|
| Pos | 1 2 3 4 5 6 7 8 9 0 | score | SEQ. ID NO. |
| 36 | H L K T S V D E I T | 6 | |
| 75 | Y Q L T E K D K E I | 6 | |
| 98 | T A L L E Q L E E T | 6 | |
| 120 | A L S E E K D V L K | 6 | |
| 135 | A T S R I A E L E S | 6 | |
| 146 | T N T L R L S Q T V | 6 | |
| 148 | T L R L S Q T V A P | 6 | |
| 151 | L S Q T V A P N C F | 6 | |
| 155 | V A P N C F N S S I | 6 | |
| 158 | N C F N S S I N N I | 6 | |
| 163 | S I N N I H E M E I | 6 | |
| 184 | W L V Y D Q Q R E V | 6 | |
| 256 | T Q L S F E L S E F | 6 | |
| 263 | S E F R R K Y E E T | 6 | |
| 280 | N Q L L Y S Q R R A | 6 | |
| 305 | Q K L R E E N D I A | 6 | |
| 349 | V A L L E Q Q M Q A | 6 | |
| 378 | H V I L K E L R K A | 6 | |
| 436 | S L V E C P K C N I | 6 | |
| 10 | I K S K W G S K P S | 5 | |
| 19 | S N S K S E T T L E | 5 | |
| 94 | R Y S T T A L L E Q | 5 | |
| 213 | A H S L P Q Q T K K | 5 | |
| 242 | A S A K K D L E V E | 5 | |
| 313 | I A R G K L E E E K | 5 | |
| 322 | K K R S E E L L S Q | 5 | |
| 404 | A I T E P L V T F Q | 5 | |
| 426 | P K S P T A A L N E | 5 | |
| 449 | A T E H R D L L V H | 5 | |
| 451 | E H R D L L V H V E | 5 | |
| 2 | S S R S T K D L I K | 4 | |
| 33 | E I A H L K T S V D | 4 | |
| 38 | K T S V D E I T S G | 4 | |
| 58 | R L L E K I R V L E | 4 | |
| 80 | K D K E I Q R L R D | 4 | |
| 86 | R L R D Q L K A R Y | 4 | |
| 93 | A R Y S T T A L L E | 4 | |
| 132 | L S A A T S R I A E | 4 | |
| 150 | R L S Q T V A P N C | 4 | |
| 192 | E V Y V K G L L A K | 4 | |
| 198 | L L A K I F E L E K | 4 | |
| 205 | L E K K T E T A A H | 4 | |
| 209 | T E T A A H S L P Q | 4 | |
| 210 | E T A A H S L P Q Q | 4 | |
| 261 | E L S E F R R K Y E | 4 | |
| 265 | F R R K Y E E T Q K | 4 | |
| 287 | R R A D V Q H L E D | 4 | |
| 300 | K T E K I Q K L R E | 4 | |
| 306 | K L R E E N D I A R | 4 | |
| 314 | A R G K L E E E K K | 4 | |
| 386 | K A R N Q I T Q L E | 4 | |
| 387 | A R N Q I T Q L E S | 4 | |
| 417 | E N R E K V A A S P | 4 | |
| 430 | T A A L N E S L V E | 4 | |
| 431 | A A L N E S L V E C | 4 | |
| 14 | W G S K P S N S K S | 3 | |

| TABLE XLI 121P2A3 v.1: HLA Peptide Scoring Results B*0702 10-mers SYFPEITHI | | | |
|---|---|---|---|
| Pos | 1 2 3 4 5 6 7 8 9 0 | score | SEQ. ID NO. |
| 20 | N S K S E T T L E K | 3 | |
| 29 | K L K G E I A H L K | 3 | |
| 34 | I A H L K T S V D E | 3 | |
| 45 | T S G K G K L T D K | 3 | |
| 46 | S G K G K L T D K E | 3 | |
| 52 | T D K E R H R L L E | 3 | |
| 62 | K I R V L E A E K E | 3 | |
| 67 | E A E K E K N A Y Q | 3 | |
| 70 | K E K N A Y Q L T E | 3 | |
| 71 | E K N A Y Q L T E K | 3 | |
| 72 | K N A Y Q L T E K D | 3 | |
| 79 | E K D K E I Q R L R | 3 | |
| 97 | T T A L L E Q L E E | 3 | |
| 107 | T T R E G E R R E Q | 3 | |
| 110 | E G E R R E Q V L K | 3 | |
| 114 | R E Q V L K A L S E | 3 | |
| 121 | L S E E K D V L K Q | 3 | |
| 144 | S K T N T L R L S Q | 3 | |
| 149 | L R L S Q T V A P N | 3 | |
| 154 | T V A P N C F N S S | 3 | |
| 167 | I H E M E I Q L K D | 3 | |
| 171 | E I Q L K D A L E K | 3 | |
| 178 | L E K N Q Q W L V Y | 3 | |
| 179 | E K N Q Q W L V Y D | 3 | |
| 224 | E S E G Y L Q E E K | 3 | |
| 234 | Q K C Y N D L L A S | 3 | |
| 243 | S A K K D L E V E R | 3 | |
| 244 | A K K D L E V E R Q | 3 | |
| 250 | V E R Q T I T Q L S | 3 | |
| 255 | I T Q L S F E L S E | 3 | |
| 257 | Q L S F E L S E F R | 3 | |
| 264 | E F R R K Y E E T Q | 3 | |
| 275 | E V H N L N Q L L Y | 3 | |
| 283 | L Y S Q R R A D V Q | 3 | |
| 286 | Q R R A D V Q H L E | 3 | |
| 311 | N D I A R G K L E E | 3 | |
| 321 | E K K R S E E L L S | 3 | |
| 344 | E E Q T R V A L L E | 3 | |
| 345 | E Q T R V A L L E Q | 3 | |
| 350 | A L L E Q Q M Q A C | 3 | |
| 366 | E K L D R Q H V Q H | 3 | |
| 367 | K L D R Q H V Q H Q | 3 | |
| 379 | V I L K E L R K A R | 3 | |
| 405 | I T E P L V T F Q G | 3 | |
| 416 | T E N R E K V A A S | 3 | |
| 432 | A L N E S L V E C P | 3 | |
| 434 | N E S L V E C P K C | 3 | |
| 452 | H R D L L V H V E Y | 3 | |
| 3 | S R S T K D L I K S | 2 | |
| 4 | R S T K D L I K S K | 2 | |
| 11 | K S K W G S K P S N | 2 | |
| 12 | S K W G S K P S N S | 2 | |
| 13 | K W G S K P S N S K | 2 | |
| 23 | S E T T L E K L K G | 2 | |
| 47 | G K G K L T D K E R | 2 | |
| 53 | D K E R H R L L E K | 2 | |

| TABLE XLI 121P2A3 v.1: HLA Peptide Scoring Results B*0702 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 61 | E | K | I | R | V | L | E | A | E | K | 2 | |
| 66 | L | E | A | E | K | E | K | N | A | Y | 2 | |
| 74 | A | Y | Q | L | T | E | K | D | K | E | 2 | |
| 101 | L | E | Q | L | E | E | T | T | R | E | 2 | |
| 102 | E | Q | L | E | E | T | T | R | E | G | 2 | |
| 113 | R | R | E | Q | V | L | K | A | L | S | 2 | |
| 124 | E | K | D | V | L | K | Q | Q | L | S | 2 | |
| 129 | K | Q | Q | L | S | A | A | T | S | R | 2 | |
| 134 | A | A | T | S | R | I | A | E | L | E | 2 | |
| 136 | T | S | R | I | A | E | L | E | S | K | 2 | |
| 138 | R | I | A | E | L | E | S | K | T | N | 2 | |
| 141 | E | L | E | S | K | T | N | T | L | R | 2 | |
| 160 | F | N | S | S | I | N | N | I | H | E | 2 | |
| 172 | I | Q | L | K | D | A | L | E | K | N | 2 | |
| 174 | L | K | D | A | L | E | K | N | Q | Q | 2 | |
| 175 | K | D | A | L | E | K | N | Q | Q | W | 2 | |
| 180 | K | N | Q | Q | W | L | V | Y | D | Q | 2 | |
| 185 | L | V | Y | D | Q | Q | R | E | V | Y | 2 | |
| 187 | Y | D | Q | Q | R | E | V | Y | V | K | 2 | |
| 188 | D | Q | Q | R | E | V | Y | V | K | G | 2 | |
| 195 | V | K | G | L | L | A | K | I | F | E | 2 | |
| 197 | G | L | L | A | K | I | F | E | L | E | 2 | |
| 206 | E | K | K | T | E | T | A | A | H | S | 2 | |
| 212 | A | A | H | S | L | P | Q | Q | T | K | 2 | |
| 214 | H | S | L | P | Q | Q | T | K | K | P | 2 | |
| 223 | P | E | S | E | G | Y | L | Q | E | E | 2 | |
| 237 | Y | N | D | L | L | A | S | A | K | K | 2 | |
| 252 | R | Q | T | I | T | Q | L | S | F | E | 2 | |
| 266 | R | R | K | Y | E | E | T | Q | K | E | 2 | |
| 268 | K | Y | E | E | T | Q | K | E | V | H | 2 | |
| 269 | Y | E | E | T | Q | K | E | V | H | N | 2 | |
| 271 | E | T | Q | K | E | V | H | N | L | N | 2 | |
| 272 | T | Q | K | E | V | H | N | L | N | Q | 2 | |
| 276 | V | H | N | L | N | Q | L | L | Y | S | 2 | |
| 281 | Q | L | L | Y | S | Q | R | R | A | D | 2 | |
| 284 | Y | S | Q | R | R | A | D | V | Q | H | 2 | |
| 288 | R | A | D | V | Q | H | L | E | D | D | 2 | |
| 289 | A | D | V | Q | H | L | E | D | D | R | 2 | |
| 294 | L | E | D | D | R | H | K | T | E | K | 2 | |
| 296 | D | D | R | H | K | T | E | K | I | Q | 2 | |
| 302 | E | K | I | Q | K | L | R | E | E | N | 2 | |
| 303 | K | I | Q | K | L | R | E | E | N | D | 2 | |
| 310 | E | N | D | I | A | R | G | K | L | E | 2 | |
| 324 | R | S | E | E | L | L | S | Q | V | Q | 2 | |
| 332 | V | Q | F | L | Y | T | S | L | L | K | 2 | |
| 333 | Q | F | L | Y | T | S | L | L | K | Q | 2 | |
| 336 | Y | T | S | L | L | K | Q | Q | E | E | 2 | |
| 346 | Q | T | R | V | A | L | L | E | Q | Q | 2 | |
| 348 | R | V | A | L | L | E | Q | Q | M | Q | 2 | |
| 353 | E | Q | Q | M | Q | A | C | T | L | D | 2 | |
| 355 | Q | M | Q | A | C | T | L | D | F | E | 2 | |
| 358 | A | C | T | L | D | F | E | N | E | K | 2 | |
| 361 | L | D | F | E | N | E | K | L | D | R | 2 | |
| 374 | Q | H | Q | L | H | V | I | L | K | E | 2 | |
| 377 | L | H | V | I | L | K | E | L | R | K | 2 | |
| 380 | I | L | K | E | L | R | K | A | R | N | 2 | |

| TABLE XLI 121P2A3 v.1: HLA Peptide Scoring Results B*0702 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 390 | Q | I | T | Q | L | E | S | L | K | Q | 2 | |
| 393 | Q | L | E | S | L | K | Q | L | H | E | 2 | |
| 411 | T | F | Q | G | E | T | E | N | R | E | 2 | |
| 418 | N | R | E | K | V | A | A | S | P | K | 2 | |
| 419 | R | E | K | V | A | A | S | P | K | S | 2 | |
| 420 | E | K | V | A | A | S | P | K | S | P | 2 | |
| 439 | E | C | P | K | C | N | I | Q | Y | P | 2 | |
| 442 | K | C | N | I | Q | Y | P | A | T | E | 2 | |
| 445 | I | Q | Y | P | A | T | E | H | R | D | 2 | |
| 453 | R | D | L | L | V | H | V | E | Y | C | 2 | |
| 6 | T | K | D | L | I | K | S | K | W | G | 1 | |
| 7 | K | D | L | I | K | S | K | W | G | S | 1 | |
| 8 | D | L | I | K | S | K | W | G | S | K | 1 | |
| 15 | G | S | K | P | S | N | S | K | S | E | 1 | |
| 22 | K | S | E | T | T | L | E | K | L | K | 1 | |
| 24 | E | T | T | L | E | K | L | K | G | E | 1 | |
| 27 | L | E | K | L | K | G | E | I | A | H | 1 | |
| 31 | K | G | E | I | A | H | L | K | T | S | 1 | |
| 39 | T | S | V | D | E | I | T | S | G | K | 1 | |
| 40 | S | V | D | E | I | T | S | G | K | G | 1 | |
| 48 | K | G | K | L | T | D | K | E | R | H | 1 | |
| 55 | E | R | H | R | L | L | E | K | I | R | 1 | |
| 60 | L | E | K | I | R | V | L | E | A | E | 1 | |
| 63 | I | R | V | L | E | A | E | K | E | K | 1 | |
| 64 | R | V | L | E | A | E | K | E | K | N | 1 | |
| 77 | L | T | E | K | D | K | E | I | Q | R | 1 | |
| 83 | E | I | Q | R | L | R | D | Q | L | K | 1 | |
| 85 | Q | R | L | R | D | Q | L | K | A | R | 1 | |
| 87 | L | R | D | Q | L | K | A | R | Y | S | 1 | |
| 96 | S | T | T | A | L | L | E | Q | L | E | 1 | |
| 100 | L | L | E | Q | L | E | E | T | T | R | 1 | |
| 103 | Q | L | E | E | T | T | R | E | G | E | 1 | |
| 105 | E | E | T | T | R | E | G | E | R | R | 1 | |
| 106 | E | T | T | R | E | G | E | R | R | E | 1 | |
| 115 | E | Q | V | L | K | A | L | S | E | E | 1 | |
| 116 | Q | V | L | K | A | L | S | E | E | K | 1 | |
| 117 | V | L | K | A | L | S | E | E | K | D | 1 | |
| 122 | S | E | E | K | D | V | L | K | Q | Q | 1 | |
| 128 | L | K | Q | Q | L | S | A | A | T | S | 1 | |
| 143 | E | S | K | T | N | T | L | R | L | S | 1 | |
| 152 | S | Q | T | V | A | P | N | C | F | N | 1 | |
| 153 | Q | T | V | A | P | N | C | F | N | S | 1 | |
| 164 | I | N | N | I | H | E | M | E | I | Q | 1 | |
| 170 | M | E | I | Q | L | K | D | A | L | E | 1 | |
| 201 | K | I | F | E | L | E | K | K | T | E | 1 | |
| 208 | K | T | E | T | A | A | H | S | L | P | 1 | |
| 215 | S | L | P | Q | Q | T | K | K | P | E | 1 | |
| 218 | Q | Q | T | K | K | P | E | S | E | G | 1 | |
| 219 | Q | T | K | K | P | E | S | E | G | Y | 1 | |
| 221 | K | K | P | E | S | E | G | Y | L | Q | 1 | |
| 225 | S | E | G | Y | L | Q | E | E | K | Q | 1 | |
| 226 | E | G | Y | L | Q | E | E | K | Q | K | 1 | |
| 230 | Q | E | E | K | Q | K | C | Y | N | D | 1 | |
| 236 | C | Y | N | D | L | L | A | S | A | K | 1 | |
| 240 | L | L | A | S | A | K | K | D | L | E | 1 | |
| 248 | L | E | V | E | R | Q | T | I | T | Q | 1 | |

| TABLE XLI 121P2A3 v.1: HLA Peptide Scoring Results B*0702 10-mers SYFPEITHI | | | |
|---|---|---|---|
| Pos | 1 2 3 4 5 6 7 8 9 0 | score | SEQ. ID NO. |
| 254 | T I T Q L S F E L S | 1 | |
| 259 | S F E L S E F R R K | 1 | |
| 260 | F E L S E F R R K Y | 1 | |
| 297 | D R H K T E K I Q K | 1 | |
| 307 | L R E E N D I A R G | 1 | |
| 308 | R E E N D I A R G K | 1 | |
| 312 | D I A R G K L E E E | 1 | |
| 315 | R G K L E E E K K R | 1 | |
| 317 | K L E E E K K R S E | 1 | |
| 318 | L E E E K K R S E E | 1 | |
| 327 | E L L S Q V Q F L Y | 1 | |
| 356 | M Q A C T L D F E N | 1 | |
| 357 | Q A C T L D F E N E | 1 | |
| 360 | T L D F E N E K L D | 1 | |
| 365 | N E K L D R Q H V Q | 1 | |
| 373 | V Q H Q L H V I L K | 1 | |
| 384 | L R K A R N Q I T Q | 1 | |
| 398 | K Q L H E F A I T E | 1 | |
| 399 | Q L H E F A I T E P | 1 | |
| 406 | T E P L V T F Q G E | 1 | |
| 409 | L V T F Q G E T E N | 1 | |
| 410 | V T F Q G E T E N R | 1 | |
| 412 | F Q G E T E N R E K | 1 | |
| 427 | K S P T A A L N E S | 1 | |
| 433 | L N E S L V E C P K | 1 | |
| 435 | E S L V E C P K C N | 1 | |
| 438 | V E C P K C N I Q Y | 1 | |
| 443 | C N I Q Y P A T E H | 1 | |
| 444 | N I Q Y P A T E H R | 1 | |

| TABLE XLI 121P2A3 v.3: HLA Peptide Scoring Results B*0702 10-mers SYFPEITHI | | | |
|---|---|---|---|
| Pos | 1 2 3 4 5 6 7 8 9 0 | score | SEQ. ID NO. |
| 5 | K L T D K E R Q R L | 12 | |
| 6 | L T D K E R Q R L L | 12 | |
| 12 | Q R L L E K I R V L | 11 | |
| 9 | K E R Q R L L E K I | 9 | |
| 11 | R Q R L L E K I R V | 9 | |

| TABLE XLI 121P2A3 v.4: HLA Peptide Scoring Results B*0702 10-mers SYFPEITHI | | | |
|---|---|---|---|
| Pos | 1 2 3 4 5 6 7 8 9 0 | score | SEQ. ID NO. |
| 3 | K A R Y S T T T L L | 15 | |
| 2 | L K A R Y S T T T L | 13 | |
| 6 | Y S T T T L L E Q L | 10 | |
| 1 | Q L K A R Y S T T T | 8 | |
| 9 | T T L L E Q L E E T | 6 | |
| 10 | T L L E Q L E E T T | 6 | |
| 4 | A R Y S T T T L L E | 5 | |
| 5 | R Y S T T T L L E Q | 5 | |
| 8 | T T T L L E Q L E E | 2 | |

| TABLE XLI 121P2A3 v.6: HLA Peptide Scoring Results B*0702 10-mers SYFPEITHI | | | |
|---|---|---|---|
| Pos | 1 2 3 4 5 6 7 8 9 0 | score | SEQ. ID NO. |
| 2 | E E L L S Q V Q S L | 12 | |
| 6 | S Q V Q S L Y T S L | 11 | |
| 7 | Q V Q S L Y T S L L | 11 | |
| 4 | L L S Q V Q S L Y T | 10 | |
| 8 | V Q S L Y T S L L K | 4 | |
| 1 | S E E L L S Q V Q S | 2 | |
| 9 | Q S L Y T S L L K Q | 2 | |
| 3 | E L L S Q V Q S L Y | | |

| TABLE XLI 121P2A3 v.7: HLA Peptide Scoring Results B*0702 10-mers SYFPEITHI | | | |
|---|---|---|---|
| Pos | 1 2 3 4 5 6 7 8 9 0 | score | SEQ. ID NO. |
| 4 | H V Q H Q L L V I L | 12 | |
| 1 | D R Q H V Q H Q L L | 10 | |
| 7 | H Q L L V I L K E L | 10 | |
| 2 | R Q H V Q H Q L L V | 9 | |
| 3 | Q H V Q H Q L L V I | 9 | |
| 10 | L V I L K E L R K A | 6 | |
| 6 | Q H Q L L V I L K E | 2 | |
| 9 | L L V I L K E L R K | 2 | |
| 5 | V Q H Q L L V I L K | 1 | |

| TABLE XLI 121P2A3 v.8: HLA Peptide Scoring Results B*0702 10-mers SYFPEITHI | | | |
|---|---|---|---|
| Pos | 1 2 3 4 5 6 7 8 9 0 | score | SEQ. ID NO. |
| 3 | S P T A A L N G S L | 21 | |
| 4 | P T A A L N G S L V | 8 | |
| 1 | P K S P T A A L N G | 5 | |
| 7 | A L N G S L V E C P | 5 | |
| 5 | T A A L N G S L V E | 4 | |
| 6 | A A L N G S L V E C | 4 | |
| 9 | N G S L V E C P K C | 3 | |
| 8 | L N G S L V E C P K | 2 | |
| 2 | K S P T A A L N G S | 1 | |

| TABLE XLII 121P2A3: HLA Peptide Scoring Results B*08 10-mers SYFPEITHI | | | |
|---|---|---|---|
| Pos | 1 2 3 4 5 6 7 8 9 0 | score | SEQ. ID NO. |
| | NO DATA | | |

| TABLE XLIII 121P2A3: HLA Peptide Scoring Results B*1510 10-mers SYFPEITHI | | | |
|---|---|---|---|
| Pos | 1 2 3 4 5 6 7 8 9 0 | score | SEQ. ID NO. |
| | NO DATA | | |

| TABLE XLIV 121P2A3: HLA Peptide Scoring Results B*2705 10-mers SYFPEITHI | | | |
|---|---|---|---|
| Pos | 1 2 3 4 5 6 7 8 9 0 | score | SEQ. ID NO. |
| | NO DATA | | |

TABLE XLV 121P2A3: HLA Peptide Scoring Results B*2709 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| | NO DATA | | | | | | | | | | | |

TABLE XLVI 121P2A3 v.1: HLA Peptide Scoring Results B*4402 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 140 | A | E | L | E | S | K | T | N | T | L | 27 | |
| 82 | K | E | I | Q | R | L | R | D | Q | L | 26 | |
| 42 | D | E | I | T | S | G | K | G | K | L | 25 | |
| 68 | A | E | K | E | K | N | A | Y | Q | L | 25 | |
| 309 | E | E | N | D | I | A | R | G | K | L | 25 | |
| 326 | E | E | L | L | S | Q | V | Q | F | L | 25 | |
| 438 | V | E | C | P | K | C | N | I | Q | Y | 25 | |
| 78 | T | E | K | D | K | E | I | Q | R | L | 24 | |
| 123 | E | E | K | D | V | L | K | Q | Q | L | 24 | |
| 260 | F | E | L | S | E | F | R | R | K | Y | 24 | |
| 270 | E | E | T | Q | K | E | V | H | N | L | 24 | |
| 325 | S | E | E | L | L | S | Q | V | Q | F | 24 | |
| 382 | K | E | L | R | K | A | R | N | Q | I | 24 | |
| 394 | L | E | S | L | K | Q | L | H | E | F | 24 | |
| 66 | L | E | A | E | K | E | K | N | A | Y | 23 | |
| 142 | L | E | S | K | T | N | T | L | R | L | 23 | |
| 178 | L | E | K | N | Q | Q | W | L | V | Y | 23 | |
| 231 | E | E | K | Q | K | C | Y | N | D | L | 23 | |
| 320 | E | E | K | K | R | S | E | E | L | L | 23 | |
| 274 | K | E | V | H | N | L | N | Q | L | L | 22 | |
| 319 | E | E | E | K | K | R | S | E | E | L | 22 | |
| 343 | Q | E | E | Q | T | R | V | A | L | L | 22 | |
| 352 | L | E | Q | Q | M | Q | A | C | T | L | 22 | |
| 54 | K | E | R | H | R | L | L | E | K | I | 21 | |
| 109 | R | E | G | E | R | R | E | Q | V | L | 21 | |
| 57 | H | R | L | L | E | K | I | R | V | L | 17 | |
| 112 | E | R | R | E | Q | V | L | K | A | L | 17 | |
| 249 | E | V | E | R | Q | T | I | T | Q | L | 17 | |
| 403 | F | A | I | T | E | P | L | V | T | F | 17 | |
| 424 | A | S | P | K | S | P | T | A | A | L | 17 | |
| 28 | E | K | L | K | G | E | I | A | H | L | 16 | |
| 158 | N | C | F | N | S | S | I | N | N | I | 16 | |
| 165 | N | N | I | H | E | M | E | I | Q | L | 16 | |
| 196 | K | G | L | L | A | K | I | F | E | L | 16 | |
| 344 | E | E | Q | T | R | V | A | L | L | E | 16 | |
| 122 | S | E | E | K | D | V | L | K | Q | Q | 15 | |
| 133 | S | A | A | T | S | R | I | A | E | L | 15 | |
| 275 | E | V | H | N | L | N | Q | L | L | Y | 15 | |
| 298 | R | H | K | T | E | K | I | Q | K | L | 15 | |
| 385 | R | K | A | R | N | Q | I | T | Q | L | 15 | |
| 391 | I | T | Q | L | E | S | L | K | Q | L | 15 | |
| 21 | S | K | S | E | T | T | L | E | K | L | 14 | |
| 23 | S | E | T | T | L | E | K | L | K | G | 14 | |
| 27 | L | E | K | L | K | G | E | I | A | H | 14 | |
| 50 | K | L | T | D | K | E | R | H | R | L | 14 | |
| 70 | K | E | K | N | A | Y | Q | L | T | E | 14 | |
| 91 | L | K | A | R | Y | S | T | T | A | L | 14 | |
| 111 | G | E | R | R | E | Q | V | L | K | A | 14 | |
| 168 | H | E | M | E | I | Q | L | K | D | A | 14 | |
| 169 | E | M | E | I | Q | L | K | D | A | L | 14 | |
| 170 | M | E | I | Q | L | K | D | A | L | E | 14 | |
| 194 | Y | V | K | G | L | L | A | K | I | F | 14 | |
| 251 | E | R | Q | T | I | T | Q | L | S | F | 14 | |
| 253 | Q | T | I | T | Q | L | S | F | E | L | 14 | |
| 263 | S | E | F | R | R | K | Y | E | E | T | 14 | |
| 295 | E | D | D | R | H | K | T | E | K | I | 14 | |
| 327 | E | L | L | S | Q | V | Q | F | L | Y | 14 | |
| 342 | Q | Q | E | E | Q | T | R | V | A | L | 14 | |
| 365 | N | E | K | L | D | R | Q | H | V | Q | 14 | |
| 375 | H | Q | L | H | V | I | L | K | E | L | 14 | |
| 416 | T | E | N | R | E | K | V | A | A | S | 14 | |
| 434 | N | E | S | L | V | E | C | P | K | C | 14 | |
| 5 | S | T | K | D | L | I | K | S | K | W | 13 | |
| 32 | G | E | I | A | H | L | K | T | S | V | 13 | |
| 35 | A | H | L | K | T | S | V | D | E | I | 13 | |
| 51 | L | T | D | K | E | R | H | R | L | L | 13 | |
| 60 | L | E | K | I | R | V | L | E | A | E | 13 | |
| 92 | K | A | R | Y | S | T | T | A | L | L | 13 | |
| 95 | Y | S | T | T | A | L | L | E | Q | L | 13 | |
| 105 | E | E | T | T | R | E | G | E | R | R | 13 | |
| 119 | K | A | L | S | E | E | K | D | V | L | 13 | |
| 151 | L | S | Q | T | V | A | P | N | C | F | 13 | |
| 175 | K | D | A | L | E | K | N | Q | Q | W | 13 | |
| 185 | L | V | Y | D | Q | Q | R | E | V | Y | 13 | |
| 189 | Q | Q | R | E | V | Y | V | K | G | L | 13 | |
| 203 | F | E | L | E | K | K | T | E | T | A | 13 | |
| 205 | L | E | K | K | T | E | T | A | A | H | 13 | |
| 207 | K | K | T | E | T | A | A | H | S | L | 13 | |
| 220 | T | K | K | P | E | S | E | G | Y | L | 13 | |
| 232 | E | K | Q | K | C | Y | N | D | L | L | 13 | |
| 239 | D | L | L | A | S | A | K | K | D | L | 13 | |
| 248 | L | E | V | E | R | Q | T | I | T | Q | 13 | |
| 273 | Q | K | E | V | H | N | L | N | Q | L | 13 | |
| 285 | S | Q | R | R | A | D | V | Q | H | L | 13 | |
| 308 | R | E | E | N | D | I | A | R | G | K | 13 | |
| 354 | Q | Q | M | Q | A | C | T | L | D | F | 13 | |
| 359 | C | T | L | D | F | E | N | E | K | L | 13 | |
| 401 | H | E | F | A | I | T | E | P | L | V | 13 | |
| 406 | T | E | P | L | V | T | F | Q | G | E | 13 | |
| 414 | G | E | T | E | N | R | E | K | V | A | 13 | |
| 447 | Y | P | A | T | E | H | R | D | L | L | 13 | |
| 450 | T | E | H | R | D | L | L | V | H | V | 13 | |
| 452 | H | R | D | L | L | V | H | V | E | Y | 13 | |
| 75 | Y | Q | L | T | E | K | D | K | E | I | 12 | |
| 101 | L | E | Q | L | E | E | T | T | R | E | 12 | |
| 114 | R | E | Q | V | L | K | A | L | S | E | 12 | |
| 191 | R | E | V | Y | V | K | G | L | L | A | 12 | |
| 223 | P | E | S | E | G | Y | L | Q | E | E | 12 | |
| 225 | S | E | G | Y | L | Q | E | E | K | Q | 12 | |
| 228 | Y | L | Q | E | E | K | Q | K | C | Y | 12 | |
| 246 | K | D | L | E | V | E | R | Q | T | I | 12 | |
| 250 | V | E | R | Q | T | I | T | Q | L | S | 12 | |
| 256 | T | Q | L | S | F | E | L | S | E | F | 12 | |
| 269 | Y | E | E | T | Q | K | E | V | H | N | 12 | |
| 294 | L | E | D | D | R | H | K | T | E | K | 12 | |

| TABLE XLVI 121P2A3 v.1: HLA Peptide Scoring Results B*4402 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 301 | T | E | K | I | Q | K | L | R | E | E | 12 | |
| 331 | Q | V | Q | F | L | Y | T | S | L | L | 12 | |
| 363 | F | E | N | E | K | L | D | R | Q | H | 12 | |
| 368 | L | D | R | Q | H | V | Q | H | Q | L | 12 | |
| 446 | Q | Y | P | A | T | E | H | R | D | L | 12 | |
| 1 | M | S | S | R | S | T | K | D | L | I | 11 | |
| 18 | P | S | N | S | K | S | E | T | T | L | 11 | |
| 86 | R | L | R | D | Q | L | K | A | R | Y | 11 | |
| 104 | L | E | E | T | T | R | E | G | E | R | 11 | |
| 176 | D | A | L | E | K | N | Q | Q | W | L | 11 | |
| 190 | Q | R | E | V | Y | V | K | G | L | L | 11 | |
| 209 | T | E | T | A | A | H | S | L | P | Q | 11 | |
| 318 | L | E | E | E | K | K | R | S | E | E | 11 | |
| 330 | S | Q | V | Q | F | L | Y | T | S | L | 11 | |
| 371 | Q | H | V | Q | H | Q | L | H | V | I | 11 | |
| 372 | H | V | Q | H | Q | L | H | V | I | L | 11 | |
| 388 | R | N | Q | I | T | Q | L | E | S | L | 11 | |
| 396 | S | L | K | Q | L | H | E | F | A | I | 11 | |
| 400 | L | H | E | F | A | I | T | E | P | L | 11 | |
| 419 | R | E | K | V | A | A | S | P | K | S | 11 | |
| 428 | S | P | T | A | A | L | N | E | S | L | 11 | |
| 130 | Q | Q | L | S | A | A | T | S | R | I | 10 | |
| 155 | V | A | P | N | C | F | N | S | S | I | 10 | |
| 163 | S | I | N | N | I | H | E | M | E | I | 10 | |
| 193 | V | Y | V | K | G | L | L | A | K | I | 10 | |
| 219 | Q | T | K | K | P | E | S | E | G | Y | 10 | |
| 230 | Q | E | E | K | Q | K | C | Y | N | D | 10 | |
| 304 | I | Q | K | L | R | E | E | N | D | I | 10 | |
| 423 | A | A | S | P | K | S | P | T | A | A | 10 | |
| 25 | T | T | L | E | K | L | K | G | E | I | 9 | |
| 200 | A | K | I | F | E | L | E | K | K | T | 9 | |
| 436 | S | L | V | E | C | P | K | C | N | I | 9 | |
| 214 | H | S | L | P | Q | Q | T | K | K | P | 8 | |
| 85 | Q | R | L | R | D | Q | L | K | A | R | 7 | |
| 238 | N | D | L | L | A | S | A | K | K | D | 7 | |
| 378 | H | V | I | L | K | E | L | R | K | A | 7 | |
| 431 | A | A | L | N | E | S | L | V | E | C | 7 | |
| 3 | S | R | S | T | K | D | L | I | K | S | 6 | |
| 24 | E | T | T | L | E | K | L | K | G | E | 6 | |
| 61 | E | K | I | R | V | L | E | A | E | K | 6 | |
| 93 | A | R | Y | S | T | T | A | L | L | E | 6 | |
| 120 | A | L | S | E | E | K | D | V | L | K | 6 | |
| 135 | A | T | S | R | I | A | E | L | E | S | 6 | |
| 143 | E | S | K | T | N | T | L | R | L | S | 6 | |
| 161 | N | S | S | I | N | N | I | H | E | M | 6 | |
| 192 | E | V | Y | V | K | G | L | L | A | K | 6 | |
| 201 | K | I | F | E | L | E | K | K | T | E | 6 | |
| 213 | A | H | S | L | P | Q | Q | T | K | K | 6 | |
| 226 | E | G | Y | L | Q | E | E | K | Q | K | 6 | |
| 261 | E | L | S | E | F | R | R | K | Y | E | 6 | |
| 302 | E | K | I | Q | K | L | R | E | E | N | 6 | |
| 310 | E | N | D | I | A | R | G | K | L | E | 6 | |
| 350 | A | L | L | E | Q | Q | M | Q | A | C | 6 | |
| 366 | E | K | L | D | R | Q | H | V | Q | H | 6 | |
| 374 | Q | H | Q | L | H | V | I | L | K | E | 6 | |
| 379 | V | I | L | K | E | L | R | K | A | R | 6 | |

| TABLE XLVI 121P2A3 v.1: HLA Peptide Scoring Results B*4402 10-mers SYFPEITHI | | | | | | | | | | | |
|---|---|---|---|---|---|---|---|---|---|---|---|
| Pos | 1 | 2 | 3 | 4 | 5 | 6 | 7 | 8 | 9 | 0 | score | SEQ. ID NO. |
| 389 | N | Q | I | T | Q | L | E | S | L | K | 6 | |
| 415 | E | T | E | N | R | E | K | V | A | A | 6 | |
| 426 | P | K | S | P | T | A | A | L | N | E | 6 | |
| 435 | E | S | L | V | E | C | P | K | C | N | 6 | |
| 439 | E | C | P | K | C | N | I | Q | Y | P | 6 | |
| 449 | A | T | E | H | R | D | L | L | V | H | 6 | |
| 451 | E | H | R | D | L | L | V | H | V | E | 6 | |
| 4 | R | S | T | K | D | L | I | K | S | K | 5 | |
| 15 | G | S | K | P | S | N | S | K | S | E | 5 | |
| 22 | K | S | E | T | T | L | E | K | L | K | 5 | |
| 29 | K | L | K | G | E | I | A | H | L | K | 5 | |
| 31 | K | G | E | I | A | H | L | K | T | S | 5 | |
| 38 | K | T | S | V | D | E | I | T | S | G | 5 | |
| 43 | E | I | T | S | G | K | G | K | L | T | 5 | |
| 55 | E | R | H | R | L | L | E | K | I | R | 5 | |
| 74 | A | Y | Q | L | T | E | K | D | K | E | 5 | |
| 79 | E | K | D | K | E | I | Q | R | L | R | 5 | |
| 99 | A | L | L | E | Q | L | E | E | T | T | 5 | |
| 102 | E | Q | L | E | E | T | T | R | E | G | 5 | |
| 110 | E | G | E | R | R | E | Q | V | L | K | 5 | |
| 134 | A | A | T | S | R | I | A | E | L | E | 5 | |
| 137 | S | R | I | A | E | L | E | S | K | T | 5 | |
| 148 | T | L | R | L | S | Q | T | V | A | P | 5 | |
| 154 | T | V | A | P | N | C | F | N | S | S | 5 | |
| 162 | S | S | I | N | N | I | H | E | M | E | 5 | |
| 166 | N | I | H | E | M | E | I | Q | L | K | 5 | |
| 171 | E | I | Q | L | K | D | A | L | E | K | 5 | |
| 242 | A | S | A | K | K | D | L | E | V | E | 5 | |
| 244 | A | K | K | D | L | E | V | E | R | Q | 5 | |
| 245 | K | K | D | L | E | V | E | R | Q | T | 5 | |
| 281 | Q | L | L | Y | S | Q | R | R | A | D | 5 | |
| 311 | N | D | I | A | R | G | K | L | E | E | 5 | |
| 321 | E | K | K | R | S | E | E | L | L | S | 5 | |
| 332 | V | Q | F | L | Y | T | S | L | L | K | 5 | |
| 334 | F | L | Y | T | S | L | L | K | Q | Q | 5 | |
| 345 | E | Q | T | R | V | A | L | L | E | Q | 5 | |
| 358 | A | C | T | L | D | F | E | N | E | K | 5 | |
| 398 | K | Q | L | H | E | F | A | I | T | E | 5 | |
| 402 | E | F | A | I | T | E | P | L | V | T | 5 | |
| 405 | I | T | E | P | L | V | T | F | Q | G | 5 | |
| 420 | E | K | V | A | A | S | P | K | S | P | 5 | |
| 427 | K | S | P | T | A | A | L | N | E | S | 5 | |
| 6 | T | K | D | L | I | K | S | K | W | G | 4 | |
| 17 | K | P | S | N | S | K | S | E | T | T | 4 | |
| 19 | S | N | S | K | S | E | T | T | L | E | 4 | |
| 33 | E | I | A | H | L | K | T | S | V | D | 4 | |
| 44 | I | T | S | G | K | G | K | L | T | D | 4 | |
| 46 | S | G | K | G | K | L | T | D | K | E | 4 | |
| 58 | R | L | L | E | K | I | R | V | L | E | 4 | |
| 83 | E | I | Q | R | L | R | D | Q | L | K | 4 | |
| 94 | R | Y | S | T | T | A | L | L | E | Q | 4 | |
| 106 | E | T | T | R | E | G | E | R | R | E | 4 | |
| 115 | E | Q | V | L | K | A | L | S | E | E | 4 | |
| 124 | E | K | D | V | L | K | Q | Q | L | S | 4 | |
| 126 | D | V | L | K | Q | Q | L | S | A | A | 4 | |
| 132 | L | S | A | A | T | S | R | I | A | E | 4 | |

TABLE XLVI 121P2A3 v.1: HLA Peptide Scoring Results B*4402 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 144 | S | K | T | N | T | L | R | L | S | Q | 4 | |
| 145 | K | T | N | T | L | R | L | S | Q | T | 4 | |
| 147 | N | T | L | R | L | S | Q | T | V | A | 4 | |
| 177 | A | L | E | K | N | Q | Q | W | L | V | 4 | |
| 179 | E | K | N | Q | Q | W | L | V | Y | D | 4 | |
| 204 | E | L | E | K | K | T | E | T | A | A | 4 | |
| 206 | E | K | K | T | E | T | A | A | H | S | 4 | |
| 210 | E | T | A | A | H | S | L | P | Q | Q | 4 | |
| 212 | A | A | H | S | L | P | Q | Q | T | K | 4 | |
| 215 | S | L | P | Q | Q | T | K | K | P | E | 4 | |
| 234 | Q | K | C | Y | N | D | L | L | A | S | 4 | |
| 236 | C | Y | N | D | L | L | A | S | A | K | 4 | |
| 259 | S | F | E | L | S | E | F | R | R | K | 4 | |
| 264 | E | F | R | R | K | Y | E | E | T | Q | 4 | |
| 271 | E | T | Q | K | E | V | H | N | L | N | 4 | |
| 280 | N | Q | L | L | Y | S | Q | R | R | A | 4 | |
| 292 | Q | H | L | E | D | D | R | H | K | T | 4 | |
| 293 | H | L | E | D | D | R | H | K | T | E | 4 | |
| 306 | K | L | R | E | E | N | D | I | A | R | 4 | |
| 314 | A | R | G | K | L | E | E | E | K | K | 4 | |
| 315 | R | G | K | L | E | E | E | K | K | R | 4 | |
| 323 | K | R | S | E | E | L | L | S | Q | V | 4 | |
| 333 | Q | F | L | Y | T | S | L | L | K | Q | 4 | |
| 341 | K | Q | Q | E | E | Q | T | R | V | A | 4 | |
| 360 | T | L | D | F | E | N | E | K | L | D | 4 | |
| 367 | K | L | D | R | Q | H | V | Q | H | Q | 4 | |
| 386 | K | A | R | N | Q | I | T | Q | L | E | 4 | |
| 387 | A | R | N | Q | I | T | Q | L | E | S | 4 | |
| 404 | A | I | T | E | P | L | V | T | F | Q | 4 | |
| 432 | A | L | N | E | S | L | V | E | C | P | 4 | |
| 443 | C | N | I | Q | Y | P | A | T | E | H | 4 | |
| 445 | I | Q | Y | P | A | T | E | H | R | D | 4 | |
| 8 | D | L | I | K | S | K | W | G | S | K | 3 | |
| 10 | I | K | S | K | W | G | S | K | P | S | 3 | |
| 12 | S | K | W | G | S | K | P | S | N | S | 3 | |
| 20 | N | S | K | S | E | T | T | L | E | K | 3 | |
| 40 | S | V | D | E | I | T | S | G | K | G | 3 | |
| 49 | G | K | L | T | D | K | E | R | H | R | 3 | |
| 52 | T | D | K | E | R | H | R | L | L | E | 3 | |
| 53 | D | K | E | R | H | R | L | L | E | K | 3 | |
| 62 | K | I | R | V | L | E | A | E | K | E | 3 | |
| 63 | I | R | V | L | E | A | E | K | E | K | 3 | |
| 69 | E | K | E | K | N | A | Y | Q | L | T | 3 | |
| 71 | E | K | N | A | Y | Q | L | T | E | K | 3 | |
| 72 | K | N | A | Y | Q | L | T | E | K | D | 3 | |
| 73 | N | A | Y | Q | L | T | E | K | D | K | 3 | |
| 80 | K | D | K | E | I | Q | R | L | R | D | 3 | |
| 84 | I | Q | R | L | R | D | Q | L | K | A | 3 | |
| 87 | L | R | D | Q | L | K | A | R | Y | S | 3 | |
| 98 | T | A | L | L | E | Q | L | E | E | T | 3 | |
| 108 | T | R | E | G | E | R | R | E | Q | V | 3 | |
| 118 | L | K | A | L | S | E | E | K | D | V | 3 | |
| 121 | L | S | E | E | K | D | V | L | K | Q | 3 | |
| 125 | K | D | V | L | K | Q | Q | L | S | A | 3 | |
| 129 | K | Q | Q | L | S | A | A | T | S | R | 3 | |
| 138 | R | I | A | E | L | E | S | K | T | N | 3 | |
| 141 | E | L | E | S | K | T | N | T | L | R | 3 | |
| 156 | A | P | N | C | F | N | S | S | I | N | 3 | |
| 172 | I | Q | L | K | D | A | L | E | K | N | 3 | |
| 173 | Q | L | K | D | A | L | E | K | N | Q | 3 | |
| 174 | L | K | D | A | L | E | K | N | Q | Q | 3 | |
| 188 | D | Q | Q | R | E | V | Y | V | K | G | 3 | |
| 197 | G | L | L | A | K | I | F | E | L | E | 3 | |
| 208 | K | T | E | T | A | A | H | S | L | P | 3 | |
| 221 | K | K | P | E | S | E | G | Y | L | Q | 3 | |
| 222 | K | P | E | S | E | G | Y | L | Q | E | 3 | |
| 224 | E | S | E | G | Y | L | Q | E | E | K | 3 | |
| 233 | K | Q | K | C | Y | N | D | L | L | A | 3 | |
| 235 | K | C | Y | N | D | L | L | A | S | A | 3 | |
| 237 | Y | N | D | L | L | A | S | A | K | K | 3 | |
| 276 | V | H | N | L | N | Q | L | L | Y | S | 3 | |
| 277 | H | N | L | N | Q | L | L | Y | S | Q | 3 | |
| 283 | L | Y | S | Q | R | R | A | D | V | Q | 3 | |
| 284 | Y | S | Q | R | R | A | D | V | Q | H | 3 | |
| 289 | A | D | V | Q | H | L | E | D | D | R | 3 | |
| 299 | H | K | T | E | K | I | Q | K | L | R | 3 | |
| 307 | L | R | E | E | N | D | I | A | R | G | 3 | |
| 316 | G | K | L | E | E | E | K | K | R | S | 3 | |
| 322 | K | K | R | S | E | E | L | L | S | Q | 3 | |
| 328 | L | L | S | Q | V | Q | F | L | Y | T | 3 | |
| 338 | S | L | L | K | Q | Q | E | E | Q | T | 3 | |
| 349 | V | A | L | L | E | Q | Q | M | Q | A | 3 | |
| 353 | E | Q | Q | M | Q | A | C | T | L | D | 3 | |
| 361 | L | D | F | E | N | E | K | L | D | R | 3 | |
| 364 | E | N | E | K | L | D | R | Q | H | V | 3 | |
| 373 | V | Q | H | Q | L | H | V | I | L | K | 3 | |
| 381 | L | K | E | L | R | K | A | R | N | Q | 3 | |
| 395 | E | S | L | K | Q | L | H | E | F | A | 3 | |
| 399 | Q | L | H | E | F | A | I | T | E | P | 3 | |
| 407 | E | P | L | V | T | F | Q | G | E | T | 3 | |
| 410 | V | T | F | Q | G | E | T | E | N | R | 3 | |
| 413 | Q | G | E | T | E | N | R | E | K | V | 3 | |
| 417 | E | N | R | E | K | V | A | A | S | P | 3 | |
| 425 | S | P | K | S | P | T | A | A | L | N | 3 | |
| 430 | T | A | A | L | N | E | S | L | V | E | 3 | |
| 441 | P | K | C | N | I | Q | Y | P | A | T | 3 | |
| 442 | K | C | N | I | Q | Y | P | A | T | E | 3 | |
| 453 | R | D | L | L | V | H | V | E | Y | C | 3 | |
| 2 | S | S | R | S | T | K | D | L | I | K | 2 | |
| 7 | K | D | L | I | K | S | K | W | G | S | 2 | |
| 13 | K | W | G | S | K | P | S | N | S | K | 2 | |
| 14 | W | G | S | K | P | S | N | S | K | S | 2 | |
| 16 | S | K | P | S | N | S | K | S | E | T | 2 | |
| 30 | L | K | G | E | I | A | H | L | K | T | 2 | |
| 34 | I | A | H | L | K | T | S | V | D | E | 2 | |
| 37 | L | K | T | S | V | D | E | I | T | S | 2 | |
| 39 | T | S | V | D | E | I | T | S | G | K | 2 | |
| 41 | V | D | E | I | T | S | G | K | G | K | 2 | |
| 47 | G | K | G | K | L | T | D | K | E | R | 2 | |
| 48 | K | G | K | L | T | D | K | E | R | H | 2 | |
| 56 | R | H | R | L | L | E | K | I | R | V | 2 | |
| 59 | L | L | E | K | I | R | V | L | E | A | 2 | |

TABLE XLVI 121P2A3 v.1: HLA Peptide Scoring Results B*4402 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 64 | R | V | L | E | A | E | K | E | K | N | 2 | |
| 65 | V | L | E | A | E | K | E | K | N | A | 2 | |
| 67 | E | A | E | K | E | K | N | A | Y | Q | 2 | |
| 77 | L | T | E | K | D | K | E | I | Q | R | 2 | |
| 81 | D | K | E | I | Q | R | L | R | D | Q | 2 | |
| 88 | R | D | Q | L | K | A | R | Y | S | T | 2 | |
| 89 | D | Q | L | K | A | R | Y | S | T | T | 2 | |
| 96 | S | T | T | A | L | L | E | Q | L | E | 2 | |
| 97 | T | T | A | L | L | E | Q | L | E | E | 2 | |
| 107 | T | T | R | E | G | E | R | R | E | Q | 2 | |
| 127 | V | L | K | Q | Q | L | S | A | A | T | 2 | |
| 128 | L | K | Q | Q | L | S | A | A | T | S | 2 | |
| 131 | Q | L | S | A | A | T | S | R | I | A | 2 | |
| 146 | T | N | T | L | R | L | S | Q | T | V | 2 | |
| 149 | L | R | L | S | Q | T | V | A | P | N | 2 | |
| 150 | R | L | S | Q | T | V | A | P | N | C | 2 | |
| 160 | F | N | S | S | I | N | N | I | H | E | 2 | |
| 167 | I | H | E | M | E | I | Q | L | K | D | 2 | |
| 180 | K | N | Q | Q | W | L | V | Y | D | Q | 2 | |
| 181 | N | Q | Q | W | L | V | Y | D | Q | Q | 2 | |
| 182 | Q | Q | W | L | V | Y | D | Q | Q | R | 2 | |
| 186 | V | Y | D | Q | Q | R | E | V | Y | V | 2 | |
| 187 | Y | D | Q | Q | R | E | V | Y | V | K | 2 | |
| 195 | V | K | G | L | L | A | K | I | F | E | 2 | |
| 199 | L | A | K | I | F | E | L | E | K | K | 2 | |
| 211 | T | A | A | H | S | L | P | Q | Q | T | 2 | |
| 217 | P | Q | Q | T | K | K | P | E | S | E | 2 | |
| 227 | G | Y | L | Q | E | E | K | Q | K | C | 2 | |
| 241 | L | A | S | A | K | K | D | L | E | V | 2 | |
| 243 | S | A | K | K | D | L | E | V | E | R | 2 | |
| 254 | T | I | T | Q | L | S | F | E | L | S | 2 | |
| 255 | I | T | Q | L | S | F | E | L | S | E | 2 | |
| 258 | L | S | F | E | L | S | E | F | R | R | 2 | |
| 266 | R | R | K | Y | E | E | T | Q | K | E | 2 | |
| 267 | R | K | Y | E | E | T | Q | K | E | V | 2 | |
| 268 | K | Y | E | E | T | Q | K | E | V | H | 2 | |
| 278 | N | L | N | Q | L | L | Y | S | Q | R | 2 | |
| 286 | Q | R | R | A | D | V | Q | H | L | E | 2 | |
| 287 | R | R | A | D | V | Q | H | L | E | D | 2 | |
| 288 | R | A | D | V | Q | H | L | E | D | D | 2 | |
| 291 | V | Q | H | L | E | D | D | R | H | K | 2 | |
| 296 | D | D | R | H | K | T | E | K | I | Q | 2 | |
| 297 | D | R | H | K | T | E | K | I | Q | K | 2 | |
| 300 | K | T | E | K | I | Q | K | L | R | E | 2 | |
| 305 | Q | K | L | R | E | E | N | D | I | A | 2 | |
| 312 | D | I | A | R | G | K | L | E | E | E | 2 | |
| 317 | K | L | E | E | E | K | K | R | S | E | 2 | |
| 329 | L | S | Q | V | Q | F | L | Y | T | S | 2 | |
| 336 | Y | T | S | L | L | K | Q | Q | E | E | 2 | |
| 337 | T | S | L | L | K | Q | Q | E | E | Q | 2 | |
| 346 | Q | T | R | V | A | L | L | E | Q | Q | 2 | |
| 357 | Q | A | C | T | L | D | F | E | N | E | 2 | |
| 362 | D | F | E | N | E | K | L | D | R | Q | 2 | |
| 383 | E | L | R | K | A | R | N | Q | I | T | 2 | |
| 384 | L | R | K | A | R | N | Q | I | T | Q | 2 | |
| 390 | Q | I | T | Q | L | E | S | L | K | Q | 2 | |

TABLE XLVI 121P2A3 v.1: HLA Peptide Scoring Results B*4402 10-mers SYFPEITHI

| Pos | 1 | 2 | 3 | 4 | 5 | 6 | 7 | 8 | 9 | 0 | score | SEQ. ID NO. |
|---|---|---|---|---|---|---|---|---|---|---|---|---|
| 392 | T | Q | L | E | S | L | K | Q | L | H | 2 | |
| 397 | L | K | Q | L | H | E | F | A | I | T | 2 | |
| 408 | P | L | V | T | F | Q | G | E | T | E | 2 | |
| 411 | T | F | Q | G | E | T | E | N | R | E | 2 | |
| 418 | N | R | E | K | V | A | A | S | P | K | 2 | |
| 444 | N | I | Q | Y | P | A | T | E | H | R | 2 | |
| 448 | P | A | T | E | H | R | D | L | L | V | 2 | |
| 9 | L | I | K | S | K | W | G | S | K | P | 1 | |
| 11 | K | S | K | W | G | S | K | P | S | N | 1 | |
| 36 | H | L | K | T | S | V | D | E | I | T | 1 | |
| 45 | T | S | G | K | G | K | L | T | D | K | 1 | |
| 90 | Q | L | K | A | R | Y | S | T | T | A | 1 | |
| 100 | L | L | E | Q | L | E | E | T | T | R | 1 | |
| 103 | Q | L | E | E | T | T | R | E | G | E | 1 | |
| 113 | R | R | E | Q | V | L | K | A | L | S | 1 | |
| 116 | Q | V | L | K | A | L | S | E | E | K | 1 | |
| 117 | V | L | K | A | L | S | E | E | K | D | 1 | |
| 136 | T | S | R | I | A | E | L | E | S | K | 1 | |
| 139 | I | A | E | L | E | S | K | T | N | T | 1 | |
| 152 | S | Q | T | V | A | P | N | C | F | N | 1 | |
| 157 | P | N | C | F | N | S | S | I | N | N | 1 | |
| 159 | C | F | N | S | S | I | N | N | I | H | 1 | |
| 183 | Q | W | L | V | Y | D | Q | Q | R | E | 1 | |
| 184 | W | L | V | Y | D | Q | Q | R | E | V | 1 | |
| 198 | L | L | A | K | I | F | E | L | E | K | 1 | |
| 202 | I | F | E | L | E | K | K | T | E | T | 1 | |
| 240 | L | L | A | S | A | K | K | D | L | E | 1 | |
| 252 | R | Q | T | I | T | Q | L | S | F | E | 1 | |
| 257 | Q | L | S | F | E | L | S | E | F | R | 1 | |
| 265 | F | R | R | K | Y | E | E | T | Q | K | 1 | |
| 272 | T | Q | K | E | V | H | N | L | N | Q | 1 | |
| 279 | L | N | Q | L | L | Y | S | Q | R | R | 1 | |
| 282 | L | L | Y | S | Q | R | R | A | D | V | 1 | |
| 303 | K | I | Q | K | L | R | E | E | N | D | 1 | |
| 313 | I | A | R | G | K | L | E | E | E | K | 1 | |
| 324 | R | S | E | E | L | L | S | Q | V | Q | 1 | |
| 335 | L | Y | T | S | L | L | K | Q | Q | E | 1 | |
| 339 | L | L | K | Q | Q | E | E | Q | T | R | 1 | |
| 347 | T | R | V | A | L | L | E | Q | Q | M | 1 | |
| 348 | R | V | A | L | L | E | Q | Q | M | Q | 1 | |
| 355 | Q | M | Q | A | C | T | L | D | F | E | 1 | |
| 369 | D | R | Q | H | V | Q | H | Q | L | H | 1 | |
| 370 | R | Q | H | V | Q | H | Q | L | H | V | 1 | |
| 376 | Q | L | H | V | I | L | K | E | L | R | 1 | |
| 377 | L | H | V | I | L | K | E | L | R | K | 1 | |
| 393 | Q | L | E | S | L | K | Q | L | H | E | 1 | |
| 412 | F | Q | G | E | T | E | N | R | E | K | 1 | |
| 421 | K | V | A | A | S | P | K | S | P | T | 1 | |
| 422 | V | A | A | S | P | K | S | P | T | A | 1 | |
| 429 | P | T | A | A | L | N | E | S | L | V | 1 | |
| 437 | L | V | E | C | P | K | C | N | I | Q | 1 | |
| 454 | D | L | L | V | H | V | E | Y | C | S | 1 | |

| TABLE XLVII 121P2A3: HLA Peptide Scoring Results B*5101 10-mers SYFPEITHI | | | | | | | | | | | | |
|---|---|---|---|---|---|---|---|---|---|---|---|---|
| Pos | 1 NO | 2 | 3 | 4 | 5 | 6 | 7 | 8 | 9 | 0 | score | SEQ. ID NO. |
| | DATA | | | | | | | | | | | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 24 | E T T L E K L K G E I A H L K | 34 | |
| 192 | E V Y V K G L L A K I F E L E | 34 | |
| 329 | L S Q V Q F L Y T S L L K Q Q | 31 | |
| 60 | L E K I R V L E A E K E K N A | 28 | |
| 234 | Q K C Y N D L L A S A K K D L | 27 | |
| 7 | K D L I K S K W G S K P S N S | 26 | |
| 85 | Q R L R D Q L K A R Y S T T A | 25 | |
| 146 | T N T L R L S Q T V A P N C F | 25 | |
| 167 | I H E M E I Q L K D A L E K N | 25 | |
| 252 | R Q T I T Q L S F E L S E F R | 25 | |
| 388 | R N Q I T Q L E S L K Q L H E | 25 | |
| 394 | L E S L K Q L H E F A I T E P | 25 | |
| 57 | H R L L E K I R V L E A E K E | 24 | |
| 88 | R D Q L K A R Y S T T A L L E | 24 | |
| 124 | E K D V L K Q Q L S A A T S R | 24 | |
| 126 | D V L K Q Q L S A A T S R I A | 24 | |
| 136 | T S R I A E L E S K T N T L R | 24 | |
| 184 | W L V Y D Q Q R E V Y V K G L | 24 | |
| 129 | K Q Q L S A A T S R I A E L E | 23 | |
| 161 | N S S I N N I H E M E I Q L K | 23 | |
| 189 | Q Q R E V Y V K G L L A K I F | 23 | |
| 247 | D L E V E R Q T I T Q L S F E | 23 | |
| 31 | K G E I A H L K T S V D E I T | 22 | |
| 191 | R E V Y V K G L L A K I F E L | 22 | |
| 212 | A A H S L P Q Q T K K P E S E | 22 | |
| 280 | N Q L L Y S Q R R A D V Q H L | 22 | |
| 350 | A L L E Q Q M Q A C T L D F E | 22 | |
| 370 | R Q H V Q H Q L H V I L K E L | 22 | |
| 38 | K T S V D E I T S G K G K L T | 21 | |
| 125 | K D V L K Q Q L S A A T S R I | 21 | |
| 54 | K E R H R L L E K I R V L E A | 20 | |
| 149 | L R L S Q T V A P N C F N S S | 20 | |
| 251 | E R Q T I T Q L S F E L S E F | 20 | |
| 376 | Q L H V I L K E L R K A R N Q | 20 | |
| 400 | L H E F A I T E P L V T F Q G | 20 | |
| 444 | N I Q Y P A T E H R D L L V H | 20 | |
| 187 | Y D Q Q R E V Y V K G L L A K | 19 | |
| 202 | I F E L E K K T E T A A H S L | 19 | |
| 333 | Q F L Y T S L L K Q Q E E Q T | 19 | |
| 8 | D L I K S K W G S K P S N S K | 18 | |
| 41 | V D E I T S G K G K L T D K E | 18 | |
| 48 | K G K L T D K E R H R L L E K | 18 | |
| 98 | T A L L E Q L E E T T R E G E | 18 | |
| 115 | E Q V L K A L S E E K D V L K | 18 | |
| 175 | K D A L E K N Q Q W L V Y D Q | 18 | |
| 182 | Q Q W L V Y D Q Q R E V Y V K | 18 | |
| 199 | L A K I F E L E K K T E T A A | 18 | |
| 281 | Q L L Y S Q R R A D V Q H L E | 18 | |
| 301 | T E K I Q K L R E E N D I A R | 18 | |
| 346 | Q T R V A L L E Q Q M Q A C T | 18 | |
| 362 | D F E N E K L D R Q H V Q H Q | 18 | |
| 407 | E P L V T F Q G E T E N R E K | 18 | |
| 415 | E T E N R E K V A A S P K S P | 18 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 27 | L E K L K G E I A H L K T S V | 17 | |
| 63 | I R V L E A E K E K N A Y Q L | 17 | |
| 66 | L E A E K E K N A Y Q L T E K | 17 | |
| 110 | E G E R R E Q V L K A L S E E | 17 | |
| 145 | K T N T L R L S Q T V A P N C | 17 | |
| 169 | E M E I Q L K D A L E K N Q Q | 17 | |
| 204 | E L E K K T E T A A H S L P Q | 17 | |
| 205 | L E K K T E T A A H S L P Q Q | 17 | |
| 237 | Y N D L L A S A K K D L E V E | 17 | |
| 276 | V H N L N Q L L Y S Q R R A D | 17 | |
| 288 | R A D V Q H L E D D R H K T E | 17 | |
| 323 | K R S E E L L S Q V Q F L Y T | 17 | |
| 345 | E Q T R V A L L E Q Q M Q A C | 17 | |
| 378 | H V I L K E L R K A R N Q I T | 17 | |
| 397 | L K Q L H E F A I T E P L V T | 17 | |
| 406 | T E P L V T F Q G E T E N R E | 17 | |
| 430 | T A A L N E S L V E C P K C N | 17 | |
| 434 | N E S L V E C P K C N I Q Y P | 17 | |
| 11 | K S K W G S K P S N S K S E T | 16 | |
| 81 | D K E I Q R L R D Q L K A R Y | 16 | |
| 84 | I Q R L R D Q L K A R Y S T T | 16 | |
| 90 | Q L K A R Y S T T A L L E Q L | 16 | |
| 112 | E R R E Q V L K A L S E E K D | 16 | |
| 114 | R E Q V L K A L S E E K D V L | 16 | |
| 122 | S E E K D V L K Q Q L S A A T | 16 | |
| 140 | A E L E S K T N T L R L S Q T | 16 | |
| 144 | S K T N T L R L S Q T V A P N | 16 | |
| 148 | T L R L S Q T V A P N C F N S | 16 | |
| 152 | S Q T V A P N C F N S S I N N | 16 | |
| 196 | K G L L A K I F E L E K K T E | 16 | |
| 235 | K C Y N D L L A S A K K D L E | 16 | |
| 248 | L E V E R Q T I T Q L S F E L | 16 | |
| 273 | Q K E V H N L N Q L L Y S Q R | 16 | |
| 307 | L R E E N D I A R G K L E E E | 16 | |
| 317 | K L E E E K K R S E E L L S Q | 16 | |
| 336 | Y T S L L K Q Q E E Q T R V A | 16 | |
| 337 | T S L L K Q Q E E Q T R V A L | 16 | |
| 391 | I T Q L E S L K Q L H E F A I | 16 | |
| 393 | Q L E S L K Q L H E F A I T E | 16 | |
| 416 | T E N R E K V A A S P K S P T | 16 | |
| 417 | E N R E K V A A S P K S P T A | 16 | |
| 418 | N R E K V A A S P K S P T A A | 16 | |
| 422 | V A A S P K S P T A A L N E S | 16 | |
| 427 | K S P T A A L N E S L V E C P | 16 | |
| 440 | C P K C N I Q Y P A T E H R D | 16 | |
| 35 | A H L K T S V D E I T S G K G | 15 | |
| 89 | D Q L K A R Y S T T A L L E Q | 15 | |
| 121 | L S E E K D V L K Q Q L S A A | 15 | |
| 164 | I N N I H E M E I Q L K D A L | 15 | |
| 166 | N I H E M E I Q L K D A L E K | 15 | |
| 213 | A H S L P Q Q T K K P E S E G | 15 | |
| 244 | A K K D L E V E R Q T I T Q L | 15 | |
| 255 | I T Q L S F E L S E F R R K Y | 15 | |
| 277 | H N L N Q L L Y S Q R R A D V | 15 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 293 | H L E D D R H K T E K I Q K L | 15 | |
| 339 | L L K Q Q E E Q T R V A L L E | 15 | |
| 373 | V Q H Q L H V I L K E L R K A | 15 | |
| 385 | R K A R N Q I T Q L E S L K Q | 15 | |
| 398 | K Q L H E F A I T E P L V T F | 15 | |
| 421 | K V A A S P K S P T A A L N E | 15 | |
| 4 | R S T K D L I K S K W G S K P | 14 | |
| 10 | I K S K W G S K P S N S K S E | 14 | |
| 39 | T S V D E I T S G K G K L T D | 14 | |
| 97 | T T A L L E Q L E E T T R E G | 14 | |
| 111 | G E R R E Q V L K A L S E E K | 14 | |
| 128 | L K Q Q L S A A T S R I A E L | 14 | |
| 133 | S A A T S R I A E L E S K T N | 14 | |
| 138 | R I A E L E S K T N T L R L S | 14 | |
| 183 | Q W L V Y D Q Q R E V Y V K G | 14 | |
| 201 | K I F E L E K K T E T A A H S | 14 | |
| 249 | E V E R Q T I T Q L S F E L S | 14 | |
| 322 | K K R S E E L L S Q V Q F L Y | 14 | |
| 325 | S E E L L S Q V Q F L Y T S L | 14 | |
| 326 | E E L L S Q V Q F L Y T S L L | 14 | |
| 327 | E L L S Q V Q F L Y T S L L K | 14 | |
| 340 | L K Q Q E E Q T R V A L L E Q | 14 | |
| 348 | R V A L L E Q Q M Q A C T L D | 14 | |
| 349 | V A L L E Q Q M Q A C T L D F | 14 | |
| 352 | L E Q Q M Q A C T L D F E N E | 14 | |
| 365 | N E K L D R Q H V Q H Q L H V | 14 | |
| 368 | L D R Q H V Q H Q L H V I L K | 14 | |
| 374 | Q H Q L H V I L K E L R K A R | 14 | |
| 384 | L R K A R N Q I T Q L E S L K | 14 | |
| 399 | Q L H E F A I T E P L V T F Q | 14 | |
| 403 | F A I T E P L V T F Q G E T E | 14 | |
| 420 | E K V A A S P K S P T A A L N | 14 | |
| 442 | K C N I Q Y P A T E H R D L L | 14 | |
| 5 | S T K D L I K S K W G S K P S | 13 | |
| 70 | K E K N A Y Q L T E K D K E I | 13 | |
| 72 | K N A Y Q L T E K D K E I Q R | 13 | |
| 257 | Q L S F E L S E F R R K Y E E | 13 | |
| 29 | K L K G E I A H L K T S V D E | 12 | |
| 82 | K E I Q R L R D Q L K A R Y S | 12 | |
| 200 | A K I F E L E K K T E T A A H | 12 | |
| 217 | P Q Q T K K P E S E G Y L Q E | 12 | |
| 225 | S E G Y L Q E E K Q K C Y N D | 12 | |
| 262 | L S E F R R K Y E E T Q K E V | 12 | |
| 308 | R E E N D I A R G K L E E E K | 12 | |
| 312 | D I A R G K L E E E K K R S E | 12 | |
| 375 | H Q L H V I L K E L R K A R N | 12 | |
| 381 | L K E L R K A R N Q I T Q L E | 12 | |
| 12 | S K W G S K P S N S K S E T T | 11 | |
| 21 | S K S E T T L E K L K G E I A | 11 | |
| 26 | T L E K L K G E I A H L K T S | 11 | |
| 45 | T S G K G K L T D K E R H R L | 11 | |
| 49 | G K L T D K E R H R L L E K I | 11 | |
| 52 | T D K E R H R L L E K I R V L | 11 | |
| 109 | R E G E R R E Q V L K A L S E | 11 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 116 | Q V L K A L S E E K D V L K Q | 11 | |
| 157 | P N C F N S S I N N I H E M E | 11 | |
| 159 | C F N S S I N N I H E M E I Q | 11 | |
| 236 | C Y N D L L A S A K K D L E V | 11 | |
| 299 | H K T E K I Q K L R E E N D I | 11 | |
| 302 | E K I Q K L R E E N D I A R G | 11 | |
| 306 | K L R E E N D I A R G K L E E | 11 | |
| 318 | L E E E K K R S E E L L S Q V | 11 | |
| 331 | Q V Q F L Y T S L L K Q Q E E | 11 | |
| 377 | L H V I L K E L R K A R N Q I | 11 | |
| 386 | K A R N Q I T Q L E S L K Q L | 11 | |
| 3 | S R S T K D L I K S K W G S K | 10 | |
| 6 | T K D L I K S K W G S K P S N | 10 | |
| 16 | S K P S N S K S E T T L E K L | 10 | |
| 17 | K P S N S K S E T T L E K L K | 10 | |
| 33 | E I A H L K T S V D E I T S G | 10 | |
| 40 | S V D E I T S G K G K L T D K | 10 | |
| 55 | E R H R L L E K I R V L E A E | 10 | |
| 62 | K I R V L E A E K E K N A Y Q | 10 | |
| 76 | Q L T E K D K E I Q R L R D Q | 10 | |
| 79 | E K D K E I Q R L R D Q L K A | 10 | |
| 92 | K A R Y S T T A L L E Q L E E | 10 | |
| 95 | Y S T T A L L E Q L E E T T R | 10 | |
| 101 | L E Q L E E T T R E G E R R E | 10 | |
| 103 | Q L E E T T R E G E R R E Q V | 10 | |
| 123 | E E K D V L K Q Q L S A A T S | 10 | |
| 127 | V L K Q Q L S A A T S R I A E | 10 | |
| 141 | E L E S K T N T L R L S Q T V | 10 | |
| 171 | E I Q L K D A L E K N Q Q W L | 10 | |
| 181 | N Q Q W L V Y D Q Q R E V Y V | 10 | |
| 203 | F E L E K K T E T A A H S L P | 10 | |
| 230 | Q E E K Q K C Y N D L L A S A | 10 | |
| 242 | A S A K K D L E V E R Q T I T | 10 | |
| 266 | R R K Y E E T Q K E V H N L N | 10 | |
| 268 | K Y E E T Q K E V H N L N Q L | 10 | |
| 274 | K E V H N L N Q L L Y S Q R R | 10 | |
| 278 | N L N Q L L Y S Q R R A D V Q | 10 | |
| 283 | L Y S Q R R A D V Q H L E D D | 10 | |
| 296 | D D R H K T E K I Q K L R E E | 10 | |
| 304 | I Q K L R E E N D I A R G K L | 10 | |
| 314 | A R G K L E E E K K R S E E L | 10 | |
| 319 | E E E K K R S E E L L S Q V Q | 10 | |
| 328 | L L S Q V Q F L Y T S L L K Q | 10 | |
| 338 | S L L K Q Q E E Q T R V A L L | 10 | |
| 357 | Q A C T L D F E N E K L D R Q | 10 | |
| 358 | A C T L D F E N E K L D R Q H | 10 | |
| 360 | T L D F E N E K L D R Q H V Q | 10 | |
| 366 | E K L D R Q H V Q H Q L H V I | 10 | |
| 379 | V I L K E L R K A R N Q I T Q | 10 | |
| 389 | N Q I T Q L E S L K Q L H E F | 10 | |
| 402 | E F A I T E P L V T F Q G E T | 10 | |
| 409 | L V T F Q G E T E N R E K V A | 10 | |
| 412 | F Q G E T E N R E K V A A S P | 10 | |
| 437 | L V E C P K C N I Q Y P A T E | 10 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 449 | A T E H R D L L V H V E Y C S | 10 | |
| 1 | M S S R S T K D L I K S K W G | 9 | |
| 19 | S N S K S E T T L E K L K G E | 9 | |
| 30 | L K G E I A H L K T S V D E I | 9 | |
| 34 | I A H L K T S V D E I T S G K | 9 | |
| 37 | L K T S V D E I T S G K G K L | 9 | |
| 53 | D K E R H R L L E K I R V L E | 9 | |
| 71 | E K N A Y Q L T E K D K E I Q | 9 | |
| 73 | N A Y Q L T E K D K E I Q R L | 9 | |
| 74 | A Y Q L T E K D K E I Q R L R | 9 | |
| 93 | A R Y S T T A L L E Q L E E T | 9 | |
| 94 | R Y S T T A L L E Q L E E T T | 9 | |
| 99 | A L L E Q L E E T T R E G E R | 9 | |
| 117 | V L K A L S E E K D V L K Q Q | 9 | |
| 132 | L S A A T S R I A E L E S K T | 9 | |
| 139 | I A E L E S K T N T L R L S Q | 9 | |
| 143 | E S K T N T L R L S Q T V A P | 9 | |
| 151 | L S Q T V A P N C F N S S I N | 9 | |
| 172 | I Q L K D A L E K N Q Q W L V | 9 | |
| 190 | Q R E V Y V K G L L A K I F E | 9 | |
| 193 | V Y V K G L L A K I F E L E K | 9 | |
| 194 | Y V K G L L A K I F E L E K K | 9 | |
| 218 | Q Q T K K P E S E G Y L Q E E | 9 | |
| 219 | Q T K K P E S E G Y L Q E E K | 9 | |
| 223 | P E S E G Y L Q E E K Q K C Y | 9 | |
| 224 | E S E G Y L Q E E K Q K C Y N | 9 | |
| 226 | E G Y L Q E E K Q K C Y N D L | 9 | |
| 229 | L Q E E K Q K C Y N D L L A S | 9 | |
| 231 | E E K Q K C Y N D L L A S A K | 9 | |
| 245 | K K D L E V E R Q T I T Q L S | 9 | |
| 254 | T I T Q L S F E L S E F R R K | 9 | |
| 259 | S F E L S E F R R K Y E E T Q | 9 | |
| 264 | E F R R K Y E E T Q K E V H N | 9 | |
| 265 | F R R K Y E E T Q K E V H N L | 9 | |
| 272 | T Q K E V H N L N Q L L Y S Q | 9 | |
| 279 | L N Q L L Y S Q R R A D V Q H | 9 | |
| 291 | V Q H L E D D R H K T E K I Q | 9 | |
| 303 | K I Q K L R E E N D I A R G K | 9 | |
| 315 | R G K L E E E K K R S E E L L | 9 | |
| 324 | R S E E L L S Q V Q F L Y T S | 9 | |
| 332 | V Q F L Y T S L L K Q Q E E Q | 9 | |
| 334 | F L Y T S L L K Q Q E E Q T R | 9 | |
| 347 | T R V A L L E Q Q M Q A C T L | 9 | |
| 369 | D R Q H V Q H Q L H V I L K E | 9 | |
| 383 | E L R K A R N Q I T Q L E S L | 9 | |
| 392 | T Q L E S L K Q L H E F A I T | 9 | |
| 404 | A I T E P L V T F Q G E T E N | 9 | |
| 411 | T F Q G E T E N R E K V A A S | 9 | |
| 413 | Q G E T E N R E K V A A S P K | 9 | |
| 414 | G E T E N R E K V A A S P K S | 9 | |
| 423 | A A S P K S P T A A L N E S L | 9 | |
| 426 | P K S P T A A L N E S L V E C | 9 | |
| 432 | A L N E S L V E C P K C N I Q | 9 | |
| 438 | V E C P K C N I Q Y P A T E H | 9 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 441 | P K C N I Q Y P A T E H R D L | 9 | |
| 446 | Q Y P A T E H R D L L V H V E | 9 | |
| 450 | T E H R D L L V H V E Y C S K | 9 | |
| 13 | K W G S K P S N S K S E T T L | 8 | |
| 23 | S E T T L E K L K G E I A H L | 8 | |
| 44 | I T S G K G K L T D K E R H R | 8 | |
| 56 | R H R L L E K I R V L E A E K | 8 | |
| 80 | K D K E I Q R L R D Q L K A R | 8 | |
| 91 | L K A R Y S T T A L L E Q L E | 8 | |
| 106 | E T T R E G E R R E Q V L K A | 8 | |
| 107 | T T R E G E R R E Q V L K A L | 8 | |
| 118 | L K A L S E E K D V L K Q Q L | 8 | |
| 131 | Q L S A A T S R I A E L E S K | 8 | |
| 153 | Q T V A P N C F N S S I N N I | 8 | |
| 156 | A P N C F N S S I N N I H E M | 8 | |
| 163 | S I N N I H E M E I Q L K D A | 8 | |
| 168 | H E M E I Q L K D A L E K N Q | 8 | |
| 174 | L K D A L E K N Q Q W L V Y D | 8 | |
| 179 | E K N Q Q W L V Y D Q Q R E V | 8 | |
| 188 | D Q Q R E V Y V K G L L A K I | 8 | |
| 195 | V K G L L A K I F E L E K K T | 8 | |
| 206 | E K K T E T A A H S L P Q Q T | 8 | |
| 210 | E T A A H S L P Q Q T K K P E | 8 | |
| 214 | H S L P Q Q T K K P E S E G Y | 8 | |
| 228 | Y L Q E E K Q K C Y N D L L A | 8 | |
| 232 | E K Q K C Y N D L L A S A K K | 8 | |
| 233 | K Q K C Y N D L L A S A K K D | 8 | |
| 238 | N D L L A S A K K D L E V E R | 8 | |
| 239 | D L L A S A K K D L E V E R Q | 8 | |
| 256 | T Q L S F E L S E F R R K Y E | 8 | |
| 271 | E T Q K E V H N L N Q L L Y S | 8 | |
| 298 | R H K T E K I Q K L R E E N D | 8 | |
| 310 | E N D I A R G K L E E E K K R | 8 | |
| 321 | E K K R S E E L L S Q V Q F L | 8 | |
| 341 | K Q Q E E Q T R V A L L E Q Q | 8 | |
| 342 | Q Q E E Q T R V A L L E Q Q M | 8 | |
| 351 | L L E Q Q M Q A C T L D F E N | 8 | |
| 353 | E Q Q M Q A C T L D F E N E K | 8 | |
| 355 | Q M Q A C T L D F E N E K L D | 8 | |
| 371 | Q H V Q H Q L H V I L K E L R | 8 | |
| 380 | I L K E L R K A R N Q I T Q L | 8 | |
| 396 | S L K Q L H E F A I T E P L V | 8 | |
| 401 | H E F A I T E P L V T F Q G E | 8 | |
| 419 | R E K V A A S P K S P T A A L | 8 | |
| 424 | A S P K S P T A A L N E S L V | 8 | |
| 425 | S P K S P T A A L N E S L V E | 8 | |
| 428 | S P T A A L N E S L V E C P K | 8 | |
| 431 | A A L N E S L V E C P K C N I | 8 | |
| 435 | E S L V E C P K C N I Q Y P A | 8 | |
| 445 | I Q Y P A T E H R D L L V H V | 8 | |
| 448 | P A T E H R D L L V H V E Y C | 8 | |
| 18 | P S N S K S E T T L E K L K G | 7 | |
| 28 | E K L K G E I A H L K T S V D | 7 | |
| 32 | G E I A H L K T S V D E I T S | 7 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 78 | T E K D K E I Q R L R D Q L K | 7 | |
| 100 | L L E Q L E E T T R E G E R R | 7 | |
| 154 | T V A P N C F N S S I N N I H | 7 | |
| 155 | V A P N C F N S S I N N I H E | 7 | |
| 176 | D A L E K N Q Q W L V Y D Q Q | 7 | |
| 177 | A L E K N Q Q W L V Y D Q Q R | 7 | |
| 180 | K N Q Q W L V Y D Q Q R E V Y | 7 | |
| 207 | K K T E T A A H S L P Q Q T K | 7 | |
| 246 | K D L E V E R Q T I T Q L S F | 7 | |
| 270 | E E T Q K E V H N L N Q L L Y | 7 | |
| 330 | S Q V Q F L Y T S L L K Q Q E | 7 | |
| 343 | Q E E Q T R V A L L E Q Q M Q | 7 | |
| 408 | P L V T F Q G E T E N R E K V | 7 | |
| 433 | L N E S L V E C P K C N I Q Y | 7 | |
| 15 | G S K P S N S K S E T T L E K | 6 | |
| 59 | L L E K I R V L E A E K E K N | 6 | |
| 134 | A A T S R I A E L E S K T N T | 6 | |
| 147 | N T L R L S Q T V A P N C F N | 6 | |
| 158 | N C F N S S I N N I H E M E I | 6 | |
| 197 | G L L A K I F E L E K K T E T | 6 | |
| 209 | T E T A A H S L P Q Q T K K P | 6 | |
| 215 | S L P Q Q T K K P E S E G Y L | 6 | |
| 263 | S E F R R K Y E E T Q K E V H | 6 | |
| 267 | R K Y E E T Q K E V H N L N Q | 6 | |
| 275 | E V H N L N Q L L Y S Q R R A | 6 | |
| 285 | S Q R R A D V Q H L E D D R H | 6 | |
| 286 | Q R R A D V Q H L E D D R H K | 6 | |
| 367 | K L D R Q H V Q H Q L H V I L | 6 | |
| 387 | A R N Q I T Q L E S L K Q L H | 6 | |
| 439 | E C P K C N I Q Y P A T E H R | 6 | |
| 22 | K S E T T L E K L K G E I A H | 5 | |
| 162 | S S I N N I H E M E I Q L K D | 5 | |
| 313 | I A R G K L E E E K K R S E E | 5 | |
| 2 | S S R S T K D L I K S K W G S | 4 | |
| 58 | R L L E K I R V L E A E K E K | 4 | |
| 105 | E E T T R E G E R R E Q V L K | 4 | |
| 250 | V E R Q T I T Q L S F E L S E | 4 | |
| 25 | T T L E K L K G E I A H L K T | 3 | |
| 43 | E I T S G K G K L T D K E R H | 3 | |
| 61 | E K I R V L E A E K E K N A Y | 3 | |
| 77 | L T E K D K E I Q R L R D Q L | 3 | |
| 104 | L E E T T R E G E R R E Q V L | 3 | |
| 120 | A L S E E K D V L K Q Q L S A | 3 | |
| 170 | M E I Q L K D A L E K N Q Q W | 3 | |
| 198 | L L A K I F E L E K K T E T A | 3 | |
| 211 | T A A H S L P Q Q T K K P E S | 3 | |
| 216 | L P Q Q T K K P E S E G Y L Q | 3 | |
| 241 | L A S A K K D L E V E R Q T I | 3 | |
| 294 | L E D D R H K T E K I Q K L R | 3 | |
| 295 | E D D R H K T E K I Q K L R E | 3 | |
| 320 | E E K K R S E E L L S Q V Q F | 3 | |
| 36 | H L K T S V D E I T S G K G K | 2 | |
| 46 | S G K G K L T D K E R H R L L | 2 | |
| 65 | V L E A E K E K N A Y Q L T E | 2 | |

| TABLE XLVIII 121P2A3 v.1: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 67 | E A E K E K N A Y Q L T E K D | 2 | |
| 69 | E K E K N A Y Q L T E K D K E | 2 | |
| 75 | Y Q L T E K D K E I Q R L R D | 2 | |
| 87 | L R D Q L K A R Y S T T A L L | 2 | |
| 113 | R R E Q V L K A L S E E K D V | 2 | |
| 137 | S R I A E L E S K T N T L R L | 2 | |
| 165 | N N I H E M E I Q L K D A L E | 2 | |
| 240 | L L A S A K K D L E V E R Q T | 2 | |
| 243 | S A K K D L E V E R Q T I T Q | 2 | |
| 258 | L S F E L S E F R R K Y E E T | 2 | |
| 269 | Y E E T Q K E V H N L N Q L L | 2 | |
| 284 | Y S Q R R A D V Q H L E D D R | 2 | |
| 289 | A D V Q H L E D D R H K T E K | 2 | |
| 297 | D R H K T E K I Q K L R E E N | 2 | |
| 311 | N D I A R G K L E E E K K R S | 2 | |
| 344 | E E Q T R V A L L E Q Q M Q A | 2 | |
| 356 | M Q A C T L D F E N E K L D R | 2 | |
| 361 | L D F E N E K L D R Q H V Q H | 2 | |
| 363 | F E N E K L D R Q H V Q H Q L | 2 | |
| 372 | H V Q H Q L H V I L K E L R K | 2 | |
| 395 | E S L K Q L H E F A I T E P L | 2 | |
| 410 | V T F Q G E T E N R E K V A A | 2 | |
| 9 | L I K S K W G S K P S N S K S | 1 | |
| 20 | N S K S E T T L E K L K G E I | 1 | |
| 42 | D E I T S G K G K L T D K E R | 1 | |
| 47 | G K G K L T D K E R H R L L E | 1 | |
| 50 | K L T D K E R H R L L E K I R | 1 | |
| 64 | R V L E A E K E K N A Y Q L T | 1 | |
| 68 | A E K E K N A Y Q L T E K D K | 1 | |
| 83 | E I Q R L R D Q L K A R Y S T | 1 | |
| 86 | R L R D Q L K A R Y S T T A L | 1 | |
| 96 | S T T A L L E Q L E E T T R E | 1 | |
| 108 | T R E G E R R E Q V L K A L S | 1 | |
| 119 | K A L S E E K D V L K Q Q L S | 1 | |
| 135 | A T S R I A E L E S K T N T L | 1 | |
| 142 | L E S K T N T L R L S Q T V A | 1 | |
| 150 | R L S Q T V A P N C F N S S I | 1 | |
| 173 | Q L K D A L E K N Q Q W L V Y | 1 | |
| 185 | L V Y D Q Q R E V Y V K G L L | 1 | |
| 186 | V Y D Q Q R E V Y V K G L L A | 1 | |
| 220 | T K K P E S E G Y L Q E E K Q | 1 | |
| 253 | Q T I T Q L S F E L S E F R R | 1 | |
| 260 | F E L S E F R R K Y E E T Q K | 1 | |
| 287 | R R A D V Q H L E D D R H K T | 1 | |
| 290 | D V Q H L E D D R H K T E K I | 1 | |
| 292 | Q H L E D D R H K T E K I Q K | 1 | |
| 309 | E E N D I A R G K L E E E K K | 1 | |
| 335 | L Y T S L L K Q Q E E Q T R V | 1 | |
| 359 | C T L D F E N E K L D R Q H V | 1 | |
| 382 | K E L R K A R N Q I T Q L E S | 1 | |

| TABLE XLVIII 121P2A3 v.3: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 15 | Q R L L E K I R V L E A E K E | 24 | |
| 12 | K E R Q R L L E K I R V L E A | 20 | |
| 6 | K G K L T D K E R Q R L L E K | 18 | |
| 3 | T S G K G K L T D K E R Q R L | 11 | |
| 7 | G K L T D K E R Q R L L E K I | 11 | |
| 10 | T D K E R Q R L L E K I R V L | 11 | |
| 13 | E R Q R L L E K I R V L E A E | 10 | |
| 11 | D K E R Q R L L E K I R V L E | 9 | |
| 2 | I T S G K G K L T D K E R Q R | 8 | |
| 14 | R Q R L L E K I R V L E A E K | 8 | |
| 9 | L T D K E R Q R L L E K I R V | 6 | |
| 1 | E I T S G K G K L T D K E R Q | 3 | |
| 4 | S G K G K L T D K E R Q R L L | 2 | |
| 5 | G K G K L T D K E R Q R L L E | 1 | |
| 8 | K L T D K E R Q R L L E K I R | 1 | |

| TABLE XLVIII 121P2A3 v.4: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 1 | Q R L R D Q L K A R Y S T T T | 25 | |
| 14 | T T L L E Q L E E T T R E G E | 18 | |
| 6 | Q L K A R Y S T T T L L E Q L | 17 | |
| 4 | R D Q L K A R Y S T T T L L E | 16 | |
| 5 | D Q L K A R Y S T T T L L E Q | 15 | |
| 13 | T T T L L E Q L E E T T R E G | 14 | |
| 11 | Y S T T T L L E Q L E E T T R | 11 | |
| 8 | K A R Y S T T T L L E Q L E E | 10 | |
| 9 | A R Y S T T T L L E Q L E E T | 9 | |
| 10 | R Y S T T T L L E Q L E E T T | 9 | |
| 7 | L K A R Y S T T T L L E Q L E | 6 | |
| 3 | L R D Q L K A R Y S T T T L L | 2 | |
| 2 | R L R D Q L K A R Y S T T T L | 1 | |
| 12 | S T T T L L E Q L E E T T R E | 1 | |

| TABLE XLVIII 121P2A3 v.6: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 10 | L S Q V Q S L Y T S L L K Q Q | 30 | |
| 7 | E E L L S Q V Q S L Y T S L L | 20 | |
| 14 | Q S L Y T S L L K Q Q E E Q T | 19 | |
| 4 | K R S E E L L S Q V Q S L Y T | 17 | |
| 3 | K K R S E E L L S Q V Q S L Y | 14 | |
| 6 | S E E L L S Q V Q S L Y T S L | 14 | |
| 8 | E L L S Q V Q S L Y T S L L K | 14 | |
| 9 | L L S Q V Q S L Y T S L L K Q | 10 | |
| 5 | R S E E L L S Q V Q S L Y T S | 9 | |
| 13 | V Q S L Y T S L L K Q Q E E Q | 9 | |
| 15 | S L Y T S L L K Q Q E E Q T R | 9 | |
| 2 | E K K R S E E L L S Q V Q S L | 8 | |
| 11 | S Q V Q S L Y T S L L K Q Q E | 7 | |
| 1 | E E K K R S E E L L S Q V Q S | 3 | |
| 12 | Q V Q S L Y T S L L K Q Q E E | 1 | |

| TABLE XLVIII 121P2A3 v.7: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 7 | R Q H V Q H Q L L V I L K E L | 22 | |
| 12 | H Q L L V I L K E L R K A R N | 20 | |
| 13 | Q L L V I L K E L R K A R N Q | 20 | |
| 15 | L V I L K E L R K A R N Q I T | 17 | |
| 10 | V Q H Q L L V I L K E L R K A | 16 | |
| 2 | N E K L D R Q H V Q H Q L L V | 14 | |
| 4 | K L D R Q H V Q H Q L L V I L | 14 | |
| 5 | L D R Q H V Q H Q L L V I L K | 14 | |
| 11 | Q H Q L L V I L K E L R K A R | 14 | |
| 14 | L L V I L K E L R K A R N Q I | 11 | |
| 3 | E K L D R Q H V Q H Q L L V I | 10 | |
| 9 | H V Q H Q L L V I L K E L R K | 10 | |
| 6 | D R Q H V Q H Q L L V I L K E | 9 | |
| 8 | Q H V Q H Q L L V I L K E L R | 8 | |

| TABLE XLVIII 121P2A3 v.8: HLA Peptide Scoring Results DRB1*0101 15 - mers SYFPEITHI | | | |
|---|---|---|---|
| Pos | 1 2 3 4 5 6 7 8 9 0 1 2 3 4 5 | score | SEQ. ID NO. |
| 7 | K S P T A A L N G S L V E C P | 24 | |
| 10 | T A A L N G S L V E C P K C N | 17 | |
| 14 | N G S L V E C P K C N I Q Y P | 17 | |
| 2 | V A A S P K S P T A A L N G S | 16 | |
| 1 | K V A A S P K S P T A A L N G | 15 | |
| 5 | S P K S P T A A L N G S L V E | 10 | |
| 3 | A A S P K S P T A A L N G S L | 9 | |
| 6 | P K S P T A A L N G S L V E C | 9 | |
| 12 | A L N G S L V E C P K C N I Q | 9 | |
| 4 | A S P K S P T A A L N G S L V | 8 | |
| 8 | S P T A A L N G S L V E C P K | 8 | |
| 11 | A A L N G S L V E C P K C N I | 8 | |
| 15 | G S L V E C P K C N I Q Y P A | 8 | |
| 13 | L N G S L V E C P K C N I Q Y | 7 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 325 | S E E L L S Q V Q F L Y T S L | 28 | |
| 84 | I Q R L R D Q L K A R Y S T T | 27 | |
| 182 | Q Q W L V Y D Q Q R E V Y V K | 27 | |
| 48 | K G K L T D K E R H R L L E K | 26 | |
| 167 | I H E M E I Q L K D A L E K N | 26 | |
| 226 | E G Y L Q E E K Q K C Y N D L | 26 | |
| 237 | Y N D L L A S A K K D L E V E | 26 | |
| 273 | Q K E V H N L N Q L L Y S Q R | 26 | |
| 183 | Q W L V Y D Q Q R E V Y V K G | 24 | |
| 56 | R H R L L E K I R V L E A E K | 21 | |
| 62 | K I R V L E A E K E K N A Y Q | 21 | |
| 97 | T T A L L E Q L E E T T R E G | 20 | |
| 192 | E V Y V K G L L A K I F E L E | 20 | |
| 290 | D V Q H L E D D R H K T E K I | 20 | |
| 291 | V Q H L E D D R H K T E K I Q | 20 | |
| 370 | R Q H V Q H Q L H V I L K E L | 20 | |
| 377 | L H V I L K E L R K A R N Q I | 20 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 434 | N E S L V E C P K C N I Q Y P | 20 | |
| 47 | G K G K L T D K E R H R L L E | 19 | |
| 115 | E Q V L K A L S E E K D V L K | 19 | |
| 171 | E I Q L K D A L E K N Q Q W L | 19 | |
| 238 | N D L L A S A K K D L E V E R | 19 | |
| 241 | L A S A K K D L E V E R Q T I | 19 | |
| 279 | L N Q L L Y S Q R R A D V Q H | 19 | |
| 329 | L S Q V Q F L Y T S L L K Q Q | 19 | |
| 337 | T S L L K Q Q E E Q T R V A L | 19 | |
| 346 | Q T R V A L L E Q Q M Q A C T | 19 | |
| 356 | M Q A C T L D F E N E K L D R | 19 | |
| 378 | H V I L K E L R K A R N Q I T | 19 | |
| 391 | I T Q L E S L K Q L H E F A I | 19 | |
| 63 | I R V L E A E K E K N A Y Q L | 18 | |
| 64 | R V L E A E K E K N A Y Q L T | 18 | |
| 74 | A Y Q L T E K D K E I Q R L R | 18 | |
| 117 | V L K A L S E E K D V L K Q Q | 18 | |
| 139 | I A E L E S K T N T L R L S Q | 18 | |
| 174 | L K D A L E K N Q Q W L V Y D | 18 | |
| 199 | L A K I F E L E K K T E T A A | 18 | |
| 247 | D L E V E R Q T I T Q L S F E | 18 | |
| 258 | L S F E L S E F R R K Y E E T | 18 | |
| 272 | T Q K E V H N L N Q L L Y S Q | 18 | |
| 280 | N Q L L Y S Q R R A D V Q H L | 18 | |
| 284 | Y S Q R R A D V Q H L E D D R | 18 | |
| 315 | R G K L E E E K K R S E E L L | 18 | |
| 336 | Y T S L L K Q Q E E Q T R V A | 18 | |
| 357 | Q A C T L D F E N E K L D R Q | 18 | |
| 363 | F E N E K L D R Q H V Q H Q L | 18 | |
| 374 | Q H Q L H V I L K E L R K A R | 18 | |
| 381 | L K E L R K A R N Q I T Q L E | 18 | |
| 394 | L E S L K Q L H E F A I T E P | 18 | |
| 407 | E P L V T F Q G E T E N R E K | 18 | |
| 40 | S V D E I T S G K G K L T D K | 17 | |
| 75 | Y Q L T E K D K E I Q R L R D | 17 | |
| 98 | T A L L E Q L E E T T R E G E | 17 | |
| 101 | L E Q L E E T T R E G E R R E | 17 | |
| 121 | L S E E K D V L K Q Q L S A A | 17 | |
| 175 | K D A L E K N Q Q W L V Y D Q | 17 | |
| 196 | K G L L A K I F E L E K K T E | 17 | |
| 202 | I F E L E K K T E T A A H S L | 17 | |
| 218 | Q Q T K K P E S E G Y L Q E E | 17 | |
| 259 | S F E L S E F R R K Y E E T Q | 17 | |
| 301 | T E K I Q K L R E E N D I A R | 17 | |
| 318 | L E E E K K R S E E L L S Q V | 17 | |
| 323 | K R S E E L L S Q V Q F L Y T | 17 | |
| 340 | L K Q Q E E Q T R V A L L E Q | 17 | |
| 349 | V A L L E Q Q M Q A C T L D F | 17 | |
| 358 | A C T L D F E N E K L D R Q H | 17 | |
| 419 | R E K V A A S P K S P T A A L | 17 | |
| 16 | S K P S N S K S E T T L E K L | 16 | |
| 80 | K D K E I Q R L R D Q L K A R | 16 | |
| 107 | T T R E G E R R E Q V L K A L | 16 | |
| 157 | P N C F N S S I N N I H E M E | 16 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 161 | N S S I N N I H E M E I Q L K | 16 | |
| 200 | A K I F E L E K K T E T A A H | 16 | |
| 213 | A H S L P Q Q T K K P E S E G | 16 | |
| 245 | K K D L E V E R Q T I T Q L S | 16 | |
| 426 | P K S P T A A L N E S L V E C | 16 | |
| 163 | S I N N I H E M E I Q L K D A | 15 | |
| 188 | D Q Q R E V Y V K G L L A K I | 15 | |
| 230 | Q E E K Q K C Y N D L L A S A | 15 | |
| 249 | E V E R Q T I T Q L S F E L S | 15 | |
| 262 | L S E F R R K Y E E T Q K E V | 15 | |
| 307 | L R E E N D I A R G K L E E E | 15 | |
| 348 | R V A L L E Q Q M Q A C T L D | 15 | |
| 366 | E K L D R Q H V Q H Q L H V I | 15 | |
| 409 | L V T F Q G E T E N R E K V A | 15 | |
| 436 | S L V E C P K C N I Q Y P A T | 15 | |
| 445 | I Q Y P A T E H R D L L V H V | 15 | |
| 41 | V D E I T S G K G K L T D K E | 14 | |
| 118 | L K A L S E E K D V L K Q Q L | 14 | |
| 125 | K D V L K Q Q L S A A T S R I | 14 | |
| 146 | T N T L R L S Q T V A P N C F | 14 | |
| 164 | I N N I H E M E I Q L K D A L | 14 | |
| 376 | Q L H V I L K E L R K A R N Q | 14 | |
| 388 | R N Q I T Q L E S L K Q L H E | 14 | |
| 7 | K D L I K S K W G S K P S N S | 13 | |
| 60 | L E K I R V L E A E K E K N A | 13 | |
| 83 | E I Q R L R D Q L K A R Y S T | 13 | |
| 114 | R E Q V L K A L S E E K D V L | 13 | |
| 124 | E K D V L K Q Q L S A A T S R | 13 | |
| 138 | R I A E L E S K T N T L R L S | 13 | |
| 170 | M E I Q L K D A L E K N Q Q W | 13 | |
| 190 | Q R E V Y V K G L L A K I F E | 13 | |
| 195 | V K G L L A K I F E L E K K T | 13 | |
| 252 | R Q T I T Q L S F E L S E F R | 13 | |
| 304 | I Q K L R E E N D I A R G K L | 13 | |
| 331 | Q V Q F L Y T S L L K Q Q E E | 13 | |
| 402 | E F A I T E P L V T F Q G E T | 13 | |
| 2 | S S R S T K D L I K S K W G S | 12 | |
| 6 | T K D L I K S K W G S K P S N | 12 | |
| 26 | T L E K L K G E I A H L K T S | 12 | |
| 27 | L E K L K G E I A H L K T S V | 12 | |
| 38 | K T S V D E I T S G K G K L T | 12 | |
| 55 | E R H R L L E K I R V L E A E | 12 | |
| 81 | D K E I Q R L R D Q L K A R Y | 12 | |
| 136 | T S R I A E L E S K T N T L R | 12 | |
| 152 | S Q T V A P N C F N S S I N N | 12 | |
| 169 | E M E I Q L K D A L E K N Q Q | 12 | |
| 194 | Y V K G L L A K I F E L E K K | 12 | |
| 233 | K Q K C Y N D L L A S A K K D | 12 | |
| 254 | T I T Q L S F E L S E F R R K | 12 | |
| 296 | D D R H K T E K I Q K L R E E | 12 | |
| 324 | R S E E L L S Q V Q F L Y T S | 12 | |
| 390 | Q I T Q L E S L K Q L H E F A | 12 | |
| 430 | T A A L N E S L V E C P K C N | 12 | |
| 435 | E S L V E C P K C N I Q Y P A | 12 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 448 | P A T E H R D L L V H V E Y C | 12 | |
| 24 | E T T L E K L K G E I A H L K | 11 | |
| 31 | K G E I A H L K T S V D E I T | 11 | |
| 34 | I A H L K T S V D E I T S G K | 11 | |
| 36 | H L K T S V D E I T S G K G K | 11 | |
| 57 | H R L L E K I R V L E A E K E | 11 | |
| 76 | Q L T E K D K E I Q R L R D Q | 11 | |
| 88 | R D Q L K A R Y S T T A L L E | 11 | |
| 90 | Q L K A R Y S T T A L L E Q L | 11 | |
| 110 | E G E R R E Q V L K A L S E E | 11 | |
| 148 | T L R L S Q T V A P N C F N S | 11 | |
| 225 | S E G Y L Q E E K Q K C Y N D | 11 | |
| 244 | A K K D L E V E R Q T I T Q L | 11 | |
| 255 | I T Q L S F E L S E F R R K Y | 11 | |
| 268 | K Y E E T Q K E V H N L N Q L | 11 | |
| 276 | V H N L N Q L L Y S Q R R A D | 11 | |
| 288 | R A D V Q H L E D D R H K T E | 11 | |
| 310 | E N D I A R G K L E E E K K R | 11 | |
| 326 | E E L L S Q V Q F L Y T S L L | 11 | |
| 332 | V Q F L Y T S L L K Q Q E E Q | 11 | |
| 365 | N E K L D R Q H V Q H Q L H V | 11 | |
| 397 | L K Q L H E F A I T E P L V T | 11 | |
| 401 | H E F A I T E P L V T F Q G E | 11 | |
| 406 | T E P L V T F Q G E T E N R E | 11 | |
| 422 | V A A S P K S P T A A L N E S | 11 | |
| 19 | S N S K S E T T L E K L K G E | 10 | |
| 21 | S K S E T T L E K L K G E I A | 10 | |
| 23 | S E T T L E K L K G E I A H L | 10 | |
| 25 | T T L E K L K G E I A H L K T | 10 | |
| 50 | K L T D K E R H R L L E K I R | 10 | |
| 66 | L E A E K E K N A Y Q L T E K | 10 | |
| 82 | K E I Q R L R D Q L K A R Y S | 10 | |
| 89 | D Q L K A R Y S T T A L L E Q | 10 | |
| 93 | A R Y S T T A L L E Q L E E T | 10 | |
| 94 | R Y S T T A L L E Q L E E T T | 10 | |
| 120 | A L S E E K D V L K Q Q L S A | 10 | |
| 129 | K Q Q L S A A T S R I A E L E | 10 | |
| 176 | D A L E K N Q Q W L V Y D Q Q | 10 | |
| 187 | Y D Q Q R E V Y V K G L L A K | 10 | |
| 205 | L E K K T E T A A H S L P Q Q | 10 | |
| 217 | P Q Q T K K P E S E G Y L Q E | 10 | |
| 229 | L Q E E K Q K C Y N D L L A S | 10 | |
| 251 | E R Q T I T Q L S F E L S E F | 10 | |
| 257 | Q L S F E L S E F R R K Y E E | 10 | |
| 270 | E E T Q K E V H N L N Q L L Y | 10 | |
| 278 | N L N Q L L Y S Q R R A D V Q | 10 | |
| 283 | L Y S Q R R A D V Q H L E D D | 10 | |
| 302 | E K I Q K L R E E N D I A R G | 10 | |
| 306 | K L R E E N D I A R G K L E E | 10 | |
| 313 | I A R G K L E E E K K R S E E | 10 | |
| 314 | A R G K L E E E K K R S E E L | 10 | |
| 319 | E E E K K R S E E L L S Q V Q | 10 | |
| 328 | L L S Q V Q F L Y T S L L K Q | 10 | |
| 333 | Q F L Y T S L L K Q Q E E Q T | 10 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 341 | K Q Q E E Q T R V A L L E Q Q | 10 | |
| 353 | E Q Q M Q A C T L D F E N E K | 10 | |
| 373 | V Q H Q L H V I L K E L R K A | 10 | |
| 389 | N Q I T Q L E S L K Q L H E F | 10 | |
| 400 | L H E F A I T E P L V T F Q G | 10 | |
| 442 | K C N I Q Y P A T E H R D L L | 10 | |
| 450 | T E H R D L L V H V E Y C S K | 10 | |
| 5 | S T K D L I K S K W G S K P S | 9 | |
| 49 | G K L T D K E R H R L L E K I | 9 | |
| 54 | K E R H R L L E K I R V L E A | 9 | |
| 59 | L L E K I R V L E A E K E K N | 9 | |
| 72 | K N A Y Q L T E K D K E I Q R | 9 | |
| 78 | T E K D K E I Q R L R D Q L K | 9 | |
| 95 | Y S T T A L L E Q L E E T T R | 9 | |
| 105 | E E T T R E G E R R E Q V L K | 9 | |
| 108 | T R E G E R R E Q V L K A L S | 9 | |
| 111 | G E R R E Q V L K A L S E E K | 9 | |
| 122 | S E E K D V L K Q Q L S A A T | 9 | |
| 123 | E E K D V L K Q Q L S A A T S | 9 | |
| 131 | Q L S A A T S R I A E L E S K | 9 | |
| 135 | A T S R I A E L E S K T N T L | 9 | |
| 137 | S R I A E L E S K T N T L R L | 9 | |
| 140 | A E L E S K T N T L R L S Q T | 9 | |
| 142 | L E S K T N T L R L S Q T V A | 9 | |
| 145 | K T N T L R L S Q T V A P N C | 9 | |
| 149 | L R L S Q T V A P N C F N S S | 9 | |
| 165 | N N I H E M E I Q L K D A L E | 9 | |
| 173 | Q L K D A L E K N Q Q W L V Y | 9 | |
| 181 | N Q Q W L V Y D Q Q R E V Y V | 9 | |
| 198 | L L A K I F E L E K K T E T A | 9 | |
| 210 | E T A A H S L P Q Q T K K P E | 9 | |
| 271 | E T Q K E V H N L N Q L L Y S | 9 | |
| 297 | D R H K T E K I Q K L R E E N | 9 | |
| 303 | K I Q K L R E E N D I A R G K | 9 | |
| 309 | E E N D I A R G K L E E E K K | 9 | |
| 334 | F L Y T S L L K Q Q E E Q T R | 9 | |
| 335 | L Y T S L L K Q Q E E Q T R V | 9 | |
| 345 | E Q T R V A L L E Q Q M Q A C | 9 | |
| 347 | T R V A L L E Q Q M Q A C T L | 9 | |
| 352 | L E Q Q M Q A C T L D F E N E | 9 | |
| 359 | C T L D F E N E K L D R Q H V | 9 | |
| 360 | T L D F E N E K L D R Q H V Q | 9 | |
| 375 | H Q L H V I L K E L R K A R N | 9 | |
| 380 | I L K E L R K A R N Q I T Q L | 9 | |
| 387 | A R N Q I T Q L E S L K Q L H | 9 | |
| 392 | T Q L E S L K Q L H E F A I T | 9 | |
| 413 | Q G E T E N R E K V A A S P K | 9 | |
| 444 | N I Q Y P A T E H R D L L V H | 9 | |
| 3 | S R S T K D L I K S K W G S K | 8 | |
| 9 | L I K S K W G S K P S N S K S | 8 | |
| 12 | S K W G S K P S N S K S E T T | 8 | |
| 14 | W G S K P S N S K S E T T L E | 8 | |
| 20 | N S K S E T T L E K L K G E I | 8 | |
| 30 | L K G E I A H L K T S V D E I | 8 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 35 | A H L K T S V D E I T S G K G | 8 | |
| 42 | D E I T S G K G K L T D K E R | 8 | |
| 46 | S G K G K L T D K E R H R L L | 8 | |
| 61 | E K I R V L E A E K E K N A Y | 8 | |
| 65 | V L E A E K E K N A Y Q L T E | 8 | |
| 68 | A E K E K N A Y Q L T E K D K | 8 | |
| 104 | L E E T T R E G E R R E Q V L | 8 | |
| 116 | Q V L K A L S E E K D V L K Q | 8 | |
| 130 | Q Q L S A A T S R I A E L E S | 8 | |
| 150 | R L S Q T V A P N C F N S S I | 8 | |
| 172 | I Q L K D A L E K N Q Q W L V | 8 | |
| 211 | T A A H S L P Q Q T K K P E S | 8 | |
| 216 | L P Q Q T K K P E S E G Y L Q | 8 | |
| 224 | E S E G Y L Q E E K Q K C Y N | 8 | |
| 253 | Q T I T Q L S F E L S E F R R | 8 | |
| 256 | T Q L S F E L S E F R R K Y E | 8 | |
| 263 | S E F R R K Y E E T Q K E V H | 8 | |
| 267 | R K Y E E T Q K E V H N L N Q | 8 | |
| 287 | R R A D V Q H L E D D R H K T | 8 | |
| 300 | K T E K I Q K L R E E N D I A | 8 | |
| 311 | N D I A R G K L E E E K K R S | 8 | |
| 312 | D I A R G K L E E E K K R S E | 8 | |
| 316 | G K L E E E K K R S E E L L S | 8 | |
| 317 | K L E E E K K R S E E L L S Q | 8 | |
| 350 | A L L E Q Q M Q A C T L D F E | 8 | |
| 383 | E L R K A R N Q I T Q L E S L | 8 | |
| 386 | K A R N Q I T Q L E S L K Q L | 8 | |
| 398 | K Q L H E F A I T E P L V T F | 8 | |
| 405 | I T E P L V T F Q G E T E N R | 8 | |
| 410 | V T F Q G E T E N R E K V A A | 8 | |
| 412 | F Q G E T E N R E K V A A S P | 8 | |
| 53 | D K E R H R L L E K I R V L E | 7 | |
| 77 | L T E K D K E I Q R L R D Q L | 7 | |
| 86 | R L R D Q L K A R Y S T T A L | 7 | |
| 133 | S A A T S R I A E L E S K T N | 7 | |
| 158 | N C F N S S I N N I H E M E I | 7 | |
| 184 | W L V Y D Q Q R E V Y V K G L | 7 | |
| 193 | V Y V K G L L A K I F E L E K | 7 | |
| 214 | H S L P Q Q T K K P E S E G Y | 7 | |
| 222 | K P E S E G Y L Q E E K Q K C | 7 | |
| 223 | P E S E G Y L Q E E K Q K C Y | 7 | |
| 227 | G Y L Q E E K Q K C Y N D L L | 7 | |
| 243 | S A K K D L E V E R Q T I T Q | 7 | |
| 265 | F R R K Y E E T Q K E V H N L | 7 | |
| 266 | R R K Y E E T Q K E V H N L N | 7 | |
| 294 | L E D D R H K T E K I Q K L R | 7 | |
| 295 | E D D R H K T E K I Q K L R E | 7 | |
| 298 | R H K T E K I Q K L R E E N D | 7 | |
| 338 | S L L K Q Q E E Q T R V A L L | 7 | |
| 362 | D F E N E K L D R Q H V Q H Q | 7 | |
| 368 | L D R Q H V Q H Q L H V I L K | 7 | |
| 382 | K E L R K A R N Q I T Q L E S | 7 | |
| 385 | R K A R N Q I T Q L E S L K Q | 7 | |
| 399 | Q L H E F A I T E P L V T F Q | 7 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 411 | T F Q G E T E N R E K V A A S | 7 | |
| 427 | K S P T A A L N E S L V E C P | 7 | |
| 431 | A A L N E S L V E C P K C N I | 7 | |
| 438 | V E C P K C N I Q Y P A T E H | 7 | |
| 443 | C N I Q Y P A T E H R D L L V | 7 | |
| 71 | E K N A Y Q L T E K D K E I Q | 6 | |
| 102 | E Q L E E T T R E G E R R E Q | 6 | |
| 106 | E T T R E G E R R E Q V L K A | 6 | |
| 153 | Q T V A P N C F N S S I N N I | 6 | |
| 260 | F E L S E F R R K Y E E T Q K | 6 | |
| 292 | Q H L E D D R H K T E K I Q K | 6 | |
| 236 | C Y N D L L A S A K K D L E V | 5 | |
| 144 | S K T N T L R L S Q T V A P N | 4 | |
| 147 | N T L R L S Q T V A P N C F N | 4 | |
| 168 | H E M E I Q L K D A L E K N Q | 4 | |
| 189 | Q Q R E V Y V K G L L A K I F | 4 | |
| 191 | R E V Y V K G L L A K I F E L | 4 | |
| 201 | K I F E L E K K T E T A A H S | 4 | |
| 203 | F E L E K K T E T A A H S L P | 4 | |
| 212 | A A H S L P Q Q T K K P E S E | 4 | |
| 264 | E F R R K Y E E T Q K E V H N | 4 | |
| 320 | E E K K R S E E L L S Q V Q F | 4 | |
| 423 | A A S P K S P T A A L N E S L | 4 | |
| 429 | P T A A L N E S L V E C P K C | 4 | |
| 446 | Q Y P A T E H R D L L V H V E | 4 | |
| 11 | K S K W G S K P S N S K S E T | 3 | |
| 18 | P S N S K S E T T L E K L K G | 3 | |
| 22 | K S E T T L E K L K G E I A H | 3 | |
| 28 | E K L K G E I A H L K T S V D | 3 | |
| 33 | E I A H L K T S V D E I T S G | 3 | |
| 44 | I T S G K G K L T D K E R H R | 3 | |
| 52 | T D K E R H R L L E K I R V L | 3 | |
| 73 | N A Y Q L T E K D K E I Q R L | 3 | |
| 91 | L K A R Y S T T A L L E Q L E | 3 | |
| 96 | S T T A L L E Q L E E T T R E | 3 | |
| 100 | L L E Q L E E T T R E G E R R | 3 | |
| 109 | R E G E R R E Q V L K A L S E | 3 | |
| 119 | K A L S E E K D V L K Q Q L S | 3 | |
| 128 | L K Q Q L S A A T S R I A E L | 3 | |
| 132 | L S A A T S R I A E L E S K T | 3 | |
| 156 | A P N C F N S S I N N I H E M | 3 | |
| 197 | G L L A K I F E L E K K T E T | 3 | |
| 219 | Q T K K P E S E G Y L Q E E K | 3 | |
| 220 | T K K P E S E G Y L Q E E K Q | 3 | |
| 231 | E E K Q K C Y N D L L A S A K | 3 | |
| 235 | K C Y N D L L A S A K K D L E | 3 | |
| 248 | L E V E R Q T I T Q L S F E L | 3 | |
| 275 | E V H N L N Q L L Y S Q R R A | 3 | |
| 322 | K K R S E E L L S Q V Q F L Y | 3 | |
| 330 | S Q V Q F L Y T S L L K Q Q E | 3 | |
| 364 | E N E K L D R Q H V Q H Q L H | 3 | |
| 372 | H V Q H Q L H V I L K E L R K | 3 | |
| 379 | V I L K E L R K A R N Q I T Q | 3 | |
| 384 | L R K A R N Q I T Q L E S L K | 3 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 393 | Q L E S L K Q L H E F A I T E | 3 | |
| 395 | E S L K Q L H E F A I T E P L | 3 | |
| 414 | G E T E N R E K V A A S P K S | 3 | |
| 415 | E T E N R E K V A A S P K S P | 3 | |
| 421 | K V A A S P K S P T A A L N E | 3 | |
| 424 | A S P K S P T A A L N E S L V | 3 | |
| 428 | S P T A A L N E S L V E C P K | 3 | |
| 432 | A L N E S L V E C P K C N I Q | 3 | |
| 4 | R S T K D L I K S K W G S K P | 2 | |
| 10 | I K S K W G S K P S N S K S E | 2 | |
| 13 | K W G S K P S N S K S E T T L | 2 | |
| 17 | K P S N S K S E T T L E K L K | 2 | |
| 32 | G E I A H L K T S V D E I T S | 2 | |
| 58 | R L L E K I R V L E A E K E K | 2 | |
| 67 | E A E K E K N A Y Q L T E K D | 2 | |
| 70 | K E K N A Y Q L T E K D K E I | 2 | |
| 87 | L R D Q L K A R Y S T T A L L | 2 | |
| 99 | A L L E Q L E E T T R E G E R | 2 | |
| 103 | Q L E E T T R E G E R R E Q V | 2 | |
| 112 | E R R E Q V L K A L S E E K D | 2 | |
| 113 | R R E Q V L K A L S E E K D V | 2 | |
| 126 | D V L K Q Q L S A A T S R I A | 2 | |
| 134 | A A T S R I A E L E S K T N T | 2 | |
| 141 | E L E S K T N T L R L S Q T V | 2 | |
| 151 | L S Q T V A P N C F N S S I N | 2 | |
| 155 | V A P N C F N S S I N N I H E | 2 | |
| 180 | K N Q Q W L V Y D Q Q R E V Y | 2 | |
| 186 | V Y D Q Q R E V Y V K G L L A | 2 | |
| 204 | E L E K K T E T A A H S L P Q | 2 | |
| 207 | K K T E T A A H S L P Q Q T K | 2 | |
| 209 | T E T A A H S L P Q Q T K K P | 2 | |
| 221 | K K P E S E G Y L Q E E K Q K | 2 | |
| 228 | Y L Q E E K Q K C Y N D L L A | 2 | |
| 232 | E K Q K C Y N D L L A S A K K | 2 | |
| 234 | Q K C Y N D L L A S A K K D L | 2 | |
| 239 | D L L A S A K K D L E V E R Q | 2 | |
| 242 | A S A K K D L E V E R Q T I T | 2 | |
| 246 | K D L E V E R Q T I T Q L S F | 2 | |
| 269 | Y E E T Q K E V H N L N Q L L | 2 | |
| 274 | K E V H N L N Q L L Y S Q R R | 2 | |
| 277 | H N L N Q L L Y S Q R R A D V | 2 | |
| 293 | H L E D D R H K T E K I Q K L | 2 | |
| 299 | H K T E K I Q K L R E E N D I | 2 | |
| 305 | Q K L R E E N D I A R G K L E | 2 | |
| 321 | E K K R S E E L L S Q V Q F L | 2 | |
| 342 | Q Q E E Q T R V A L L E Q Q M | 2 | |
| 343 | Q E E Q T R V A L L E Q Q M Q | 2 | |
| 344 | E E Q T R V A L L E Q Q M Q A | 2 | |
| 351 | L L E Q Q M Q A C T L D F E N | 2 | |
| 361 | L D F E N E K L D R Q H V Q H | 2 | |
| 367 | K L D R Q H V Q H Q L H V I L | 2 | |
| 396 | S L K Q L H E F A I T E P L V | 2 | |
| 404 | A I T E P L V T F Q G E T E N | 2 | |
| 408 | P L V T F Q G E T E N R E K V | 2 | |

| TABLE XLIX 121P2A3 v.1: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 417 | E N R E K V A A S P K S P T A | 2 | |
| 418 | N R E K V A A S P K S P T A A | 2 | |
| 420 | E K V A A S P K S P T A A L N | 2 | |
| 433 | L N E S L V E C P K C N I Q Y | 2 | |
| 441 | P K C N I Q Y P A T E H R D L | 2 | |
| 449 | A T E H R D L L V H V E Y C S | 2 | |
| 1 | M S S R S T K D L I K S K W G | 1 | |
| 8 | D L I K S K W G S K P S N S K | 1 | |
| 15 | G S K P S N S K S E T T L E K | 1 | |
| 37 | L K T S V D E I T S G K G K L | 1 | |
| 39 | T S V D E I T S G K G K L T D | 1 | |
| 43 | E I T S G K G K L T D K E R H | 1 | |
| 51 | L T D K E R H R L L E K I R V | 1 | |
| 85 | Q R L R D Q L K A R Y S T T A | 1 | |
| 92 | K A R Y S T T A L L E Q L E E | 1 | |
| 127 | V L K Q Q L S A A T S R I A E | 1 | |
| 160 | F N S S I N N I H E M E I Q L | 1 | |
| 162 | S S I N N I H E M E I Q L K D | 1 | |
| 166 | N I H E M E I Q L K D A L E K | 1 | |
| 177 | A L E K N Q Q W L V Y D Q Q R | 1 | |
| 178 | L E K N Q Q W L V Y D Q Q R E | 1 | |
| 185 | L V Y D Q Q R E V Y V K G L L | 1 | |
| 206 | E K K T E T A A H S L P Q Q T | 1 | |
| 208 | K T E T A A H S L P Q Q T K K | 1 | |
| 215 | S L P Q Q T K K P E S E G Y L | 1 | |
| 250 | V E R Q T I T Q L S F E L S E | 1 | |
| 261 | E L S E F R R K Y E E T Q K E | 1 | |
| 282 | L L Y S Q R R A D V Q H L E D | 1 | |
| 285 | S Q R R A D V Q H L E D D R H | 1 | |
| 289 | A D V Q H L E D D R H K T E K | 1 | |
| 308 | R E E N D I A R G K L E E E K | 1 | |
| 339 | L L K Q Q E E Q T R V A L L E | 1 | |
| 354 | Q Q M Q A C T L D F E N E K L | 1 | |
| 355 | Q M Q A C T L D F E N E K L D | 1 | |
| 369 | D R Q H V Q H Q L H V I L K E | 1 | |
| 371 | Q H V Q H Q L H V I L K E L R | 1 | |
| 403 | F A I T E P L V T F Q G E T E | 1 | |
| 425 | S P K S P T A A L N E S L V E | 1 | |
| 437 | L V E C P K C N I Q Y P A T E | 1 | |
| 439 | E C P K C N I Q Y P A T E H R | 1 | |
| 440 | C P K C N I Q Y P A T E H R D | 1 | |
| 447 | Y P A T E H R D L L V H V E Y | 1 | |

| TABLE XLIX 121P2A3 v.3: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 6 | K G K L T D K E R Q R L L E K | 27 | |
| 14 | R Q R L L E K I R V L E A E K | 21 | |
| 5 | G K G K L T D K E R Q R L L E | 19 | |
| 7 | G K L T D K E R Q R L L E K I | 15 | |
| 13 | E R Q R L L E K I R V L E A E | 12 | |
| 15 | Q R L L E K I R V L E A E K E | 11 | |
| 8 | K L T D K E R Q R L L E K I R | 10 | |
| 12 | K E R Q R L L E K I R V L E A | 9 | |

| TABLE XLIX 121P2A3 v.3: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 4 | S G K G K L T D K E R Q R L L | 8 | |
| 11 | D K E R Q R L L E K I R V L E | 7 | |
| 2 | I T S G K G K L T D K E R Q R | 3 | |
| 10 | T D K E R Q R L L E K I R V L | 3 | |
| 1 | E I T S G K G K L T D K E R Q | 1 | |
| 9 | L T D K E R Q R L L E K I R V | 1 | |

| TABLE XLIX 121P2A3 v.4: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 13 | T T T L L E Q L E E T T R E G | 20 | |
| 14 | T T L L E Q L E E T T R E G E | 17 | |
| 4 | R D Q L K A R Y S T T T L L E | 11 | |
| 6 | Q L K A R Y S T T T L L E Q L | 10 | |
| 9 | A R Y S T T T L L E Q L E E T | 10 | |
| 10 | R Y S T T T L L E Q L E E T T | 10 | |
| 11 | Y S T T T L L E Q L E E T T R | 10 | |
| 5 | D Q L K A R Y S T T T L L E Q | 9 | |
| 2 | R L R D Q L K A R Y S T T T L | 7 | |
| 7 | L K A R Y S T T T L L E Q L E | 3 | |
| 12 | S T T T L L E Q L E E T T R E | 3 | |
| 3 | L R D Q L K A R Y S T T T L L | 2 | |
| 1 | Q R L R D Q L K A R Y S T T T | 1 | |
| 8 | K A R Y S T T T L L E Q L E E | 1 | |

| TABLE XLIX 121P2A3 v.6: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 6 | S E E L L S Q V Q S L Y T S L | 28 | |
| 10 | L S Q V Q S L Y T S L L K Q Q | 20 | |
| 5 | R S E E L L S Q V Q S L Y T S | 12 | |
| 7 | E E L L S Q V Q S L Y T S L L | 11 | |
| 13 | V Q S L Y T S L L K Q Q E E Q | 11 | |
| 9 | L L S Q V Q S L Y T S L L K Q | 10 | |
| 14 | Q S L Y T S L L K Q Q E E Q T | 10 | |
| 4 | K R S E E L L S Q V Q S L Y T | 9 | |
| 15 | S L Y T S L L K Q Q E E Q T R | 9 | |
| 12 | Q V Q S L Y T S L L K Q Q E E | 5 | |
| 1 | E E K K R S E E L L S Q V Q S | 4 | |
| 3 | K K R S E E L L S Q V Q S L Y | 3 | |
| 11 | S Q V Q S L Y T S L L K Q Q E | 3 | |
| 2 | E K K R S E E L L S Q V Q S L | 2 | |
| 8 | E L L S Q V Q S L Y T S L L K | 1 | |

| TABLE XLIX 121P2A3 v.7: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 7 | R Q H V Q H Q L L V I L K E L | 20 | |
| 11 | Q H Q L L V I L K E L R K A R | 20 | |
| 14 | L L V I L K E L R K A R N Q I | 20 | |
| 12 | H Q L L V I L K E L R K A R N | 19 | |
| 15 | L V I L K E L R K A R N Q I T | 19 | |
| 3 | E K L D R Q H V Q H Q L L V I | 15 | |

| TABLE XLIX 121P2A3 v.7: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 13 | Q L L V I L K E L R K A R N Q | 14 | |
| 2 | N E K L D R Q H V Q H Q L L V | 11 | |
| 10 | V Q H Q L L V I L K E L R K A | 11 | |
| 4 | K L D R Q H V Q H Q L L V I L | 10 | |
| 5 | L D R Q H V Q H Q L L V I L K | 8 | |
| 1 | E N E K L D R Q H V Q H Q L L | 3 | |
| 9 | H V Q H Q L L V I L K E L R K | 3 | |
| 6 | D R Q H V Q H Q L L V I L K E | 2 | |
| 8 | Q H V Q H Q L L V I L K E L R | 2 | |

| TABLE XLIX 121P2A3 v.8: HLA Peptide Scoring Results DRB1*0301 15 - mers SYFPEITHI | | | |
|---|---|---|---|
| Pos | 1 2 3 4 5 6 7 8 9 0 1 2 3 4 5 | score | SEQ. ID NO. |
| 14 | N G S L V E C P K C N I Q Y P | 20 | |
| 6 | P K S P T A A L N G S L V E C | 16 | |
| 15 | G S L V E C P K C N I Q Y P A | 12 | |
| 2 | V A A S P K S P T A A L N G S | 11 | |
| 10 | T A A L N G S L V E C P K C N | 11 | |
| 11 | A A L N G S L V E C P K C N I | 7 | |
| 3 | A A S P K S P T A A L N G S L | 4 | |
| 9 | P T A A L N G S L V E C P K C | 4 | |
| 1 | K V A A S P K S P T A A L N G | 3 | |
| 4 | A S P K S P T A A L N G S L V | 3 | |
| 8 | S P T A A L N G S L V E C P K | 3 | |
| 12 | A L N G S L V E C P K C N I Q | 3 | |
| 13 | L N G S L V E C P K C N I Q Y | 2 | |
| 5 | S P K S P T A A L N G S L V E | 1 | |
| 7 | K S P T A A L N G S L V E C P | 1 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 114 | R E Q V L K A L S E E K D V L | 26 | |
| 129 | K Q Q L S A A T S R I A E L E | 26 | |
| 136 | T S R I A E L E S K T N T L R | 26 | |
| 182 | Q Q W L V Y D Q Q R E V Y V K | 26 | |
| 245 | K K D L E V E R Q T I T Q L S | 26 | |
| 329 | L S Q V Q F L Y T S L L K Q Q | 26 | |
| 346 | Q T R V A L L E Q Q M Q A C T | 26 | |
| 381 | L K E L R K A R N Q I T Q L E | 26 | |
| 388 | R N Q I T Q L E S L K Q L H E | 26 | |
| 200 | A K I F E L E K K T E T A A H | 22 | |
| 234 | Q K C Y N D L L A S A K K D L | 22 | |
| 360 | T L D F E N E K L D R Q H V Q | 22 | |
| 444 | N I Q Y P A T E H R D L L V H | 22 | |
| 24 | E T T L E K L K G E I A H L K | 20 | |
| 27 | L E K L K G E I A H L K T S V | 20 | |
| 31 | K G E I A H L K T S V D E I T | 20 | |
| 38 | K T S V D E I T S G K G K L T | 20 | |
| 57 | H R L L E K I R V L E A E K E | 20 | |
| 62 | K I R V L E A E K E K N A Y Q | 20 | |
| 63 | I R V L E A E K E K N A Y Q L | 20 | |
| 81 | D K E I Q R L R D Q L K A R Y | 20 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 118 | L K A L S E E K D V L K Q Q L | 20 | |
| 125 | K D V L K Q Q L S A A T S R I | 20 | |
| 161 | N S S I N N I H E M E I Q L K | 20 | |
| 171 | E I Q L K D A L E K N Q Q W L | 20 | |
| 175 | K D A L E K N Q Q W L V Y D Q | 20 | |
| 196 | K G L L A K I F E L E K K T E | 20 | |
| 226 | E G Y L Q E E K Q K C Y N D L | 20 | |
| 252 | R Q T I T Q L S F E L S E F R | 20 | |
| 255 | I T Q L S F E L S E F R R K Y | 20 | |
| 259 | S F E L S E F R R K Y E E T Q | 20 | |
| 273 | Q K E V H N L N Q L L Y S Q R | 20 | |
| 280 | N Q L L Y S Q R R A D V Q H L | 20 | |
| 291 | V Q H L E D D R H K T E K I Q | 20 | |
| 326 | E E L L S Q V Q F L Y T S L L | 20 | |
| 349 | V A L L E Q Q M Q A C T L D F | 20 | |
| 370 | R Q H V Q H Q L H V I L K E L | 20 | |
| 377 | L H V I L K E L R K A R N Q I | 20 | |
| 378 | H V I L K E L R K A R N Q I T | 20 | |
| 391 | I T Q L E S L K Q L H E F A I | 20 | |
| 394 | L E S L K Q L H E F A I T E P | 20 | |
| 18 | P S N S K S E T T L E K L K G | 18 | |
| 28 | E K L K G E I A H L K T S V D | 18 | |
| 37 | L K T S V D E I T S G K G K L | 18 | |
| 49 | G K L T D K E R H R L L E K I | 18 | |
| 65 | V L E A E K E K N A Y Q L T E | 18 | |
| 106 | E T T R E G E R R E Q V L K A | 18 | |
| 122 | S E E K D V L K Q Q L S A A T | 18 | |
| 128 | L K Q Q L S A A T S R I A E L | 18 | |
| 145 | K T N T L R L S Q T V A P N C | 18 | |
| 150 | R L S Q T V A P N C F N S S I | 18 | |
| 154 | T V A P N C F N S S I N N I H | 18 | |
| 207 | K K T E T A A H S L P Q Q T K | 18 | |
| 210 | E T A A H S L P Q Q T K K P E | 18 | |
| 235 | K C Y N D L L A S A K K D L E | 18 | |
| 244 | A K K D L E V E R Q T I T Q L | 18 | |
| 265 | F R R K Y E E T Q K E V H N L | 18 | |
| 269 | Y E E T Q K E V H N L N Q L L | 18 | |
| 270 | E E T Q K E V H N L N Q L L Y | 18 | |
| 290 | D V Q H L E D D R H K T E K I | 18 | |
| 303 | K I Q K L R E E N D I A R G K | 18 | |
| 307 | L R E E N D I A R G K L E E E | 18 | |
| 322 | K K R S E E L L S Q V Q F L Y | 18 | |
| 338 | S L L K Q Q E E Q T R V A L L | 18 | |
| 339 | L L K Q Q E E Q T R V A L L E | 18 | |
| 347 | T R V A L L E Q Q M Q A C T L | 18 | |
| 357 | Q A C T L D F E N E K L D R Q | 18 | |
| 362 | D F E N E K L D R Q H V Q H Q | 18 | |
| 363 | F E N E K L D R Q H V Q H Q L | 18 | |
| 385 | R K A R N Q I T Q L E S L K Q | 18 | |
| 398 | K Q L H E F A I T E P L V T F | 18 | |
| 411 | T F Q G E T E N R E K V A A S | 18 | |
| 417 | E N R E K V A A S P K S P T A | 18 | |
| 426 | P K S P T A A L N E S L V E C | 18 | |
| 445 | I Q Y P A T E H R D L L V H V | 18 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 11 | K S K W G S K P S N S K S E T | 17 | |
| 92 | K A R Y S T T A L L E Q L E E | 16 | |
| 157 | P N C F N S S I N N I H E M E | 16 | |
| 181 | N Q Q W L V Y D Q Q R E V Y V | 16 | |
| 184 | W L V Y D Q Q R E V Y V K G L | 16 | |
| 191 | R E V Y V K G L L A K I F E L | 16 | |
| 225 | S E G Y L Q E E K Q K C Y N D | 16 | |
| 257 | Q L S F E L S E F R R K Y E E | 16 | |
| 331 | Q V Q F L Y T S L L K Q Q E E | 16 | |
| 333 | Q F L Y T S L L K Q Q E E Q T | 16 | |
| 400 | L H E F A I T E P L V T F Q G | 16 | |
| 409 | L V T F Q G E T E N R E K V A | 16 | |
| 48 | K G K L T D K E R H R L L E K | 15 | |
| 88 | R D Q L K A R Y S T T A L L E | 15 | |
| 6 | T K D L I K S K W G S K P S N | 14 | |
| 34 | I A H L K T S V D E I T S G K | 14 | |
| 41 | V D E I T S G K G K L T D K E | 14 | |
| 60 | L E K I R V L E A E K E K N A | 14 | |
| 97 | T T A L L E Q L E E T T R E G | 14 | |
| 98 | T A L L E Q L E E T T R E G E | 14 | |
| 101 | L E Q L E E T T R E G E R R E | 14 | |
| 115 | E Q V L K A L S E E K D V L K | 14 | |
| 124 | E K D V L K Q Q L S A A T S R | 14 | |
| 146 | T N T L R L S Q T V A P N C F | 14 | |
| 152 | S Q T V A P N C F N S S I N N | 14 | |
| 164 | I N N I H E M E I Q L K D A L | 14 | |
| 167 | I H E M E I Q L K D A L E K N | 14 | |
| 183 | Q W L V Y D Q Q R E V Y V K G | 14 | |
| 192 | E V Y V K G L L A K I F E L E | 14 | |
| 199 | L A K I F E L E K K T E T A A | 14 | |
| 237 | Y N D L L A S A K K D L E V E | 14 | |
| 238 | N D L L A S A K K D L E V E R | 14 | |
| 247 | D L E V E R Q T I T Q L S F E | 14 | |
| 276 | V H N L N Q L L Y S Q R R A D | 14 | |
| 279 | L N Q L L Y S Q R R A D V Q H | 14 | |
| 288 | R A D V Q H L E D D R H K T E | 14 | |
| 301 | T E K I Q K L R E E N D I A R | 14 | |
| 304 | I Q K L R E E N D I A R G K L | 14 | |
| 315 | R G K L E E E K K R S E E L L | 14 | |
| 325 | S E E L L S Q V Q F L Y T S L | 14 | |
| 332 | V Q F L Y T S L L K Q Q E E Q | 14 | |
| 336 | Y T S L L K Q Q E E Q T R V A | 14 | |
| 358 | A C T L D F E N E K L D R Q H | 14 | |
| 365 | N E K L D R Q H V Q H Q L H V | 14 | |
| 374 | Q H Q L H V I L K E L R K A R | 14 | |
| 397 | L K Q L H E F A I T E P L V T | 14 | |
| 402 | E F A I T E P L V T F Q G E T | 14 | |
| 406 | T E P L V T F Q G E T E N R E | 14 | |
| 407 | E P L V T F Q G E T E N R E K | 14 | |
| 419 | R E K V A A S P K S P T A A L | 14 | |
| 434 | N E S L V E C P K C N I Q Y P | 14 | |
| 435 | E S L V E C P K C N I Q Y P A | 14 | |
| 442 | K C N I Q Y P A T E H R D L L | 14 | |
| 2 | S S R S T K D L I K S K W G S | 12 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 4 | R S T K D L I K S K W G S K P | 12 | |
| 8 | D L I K S K W G S K P S N S K | 12 | |
| 12 | S K W G S K P S N S K S E T T | 12 | |
| 15 | G S K P S N S K S E T T L E K | 12 | |
| 29 | K L K G E I A H L K T S V D E | 12 | |
| 35 | A H L K T S V D E I T S G K G | 12 | |
| 54 | K E R H R L L E K I R V L E A | 12 | |
| 55 | E R H R L L E K I R V L E A E | 12 | |
| 59 | L L E K I R V L E A E K E K N | 12 | |
| 61 | E K I R V L E A E K E K N A Y | 12 | |
| 68 | A E K E K N A Y Q L T E K D K | 12 | |
| 71 | E K N A Y Q L T E K D K E I Q | 12 | |
| 73 | N A Y Q L T E K D K E I Q R L | 12 | |
| 77 | L T E K D K E I Q R L R D Q L | 12 | |
| 78 | T E K D K E I Q R L R D Q L K | 12 | |
| 85 | Q R L R D Q L K A R Y S T T A | 12 | |
| 87 | L R D Q L K A R Y S T T A L L | 12 | |
| 89 | D Q L K A R Y S T T A L L E Q | 12 | |
| 90 | Q L K A R Y S T T A L L E Q L | 12 | |
| 93 | A R Y S T T A L L E Q L E E T | 12 | |
| 95 | Y S T T A L L E Q L E E T T R | 12 | |
| 96 | S T T A L L E Q L E E T T R E | 12 | |
| 99 | A L L E Q L E E T T R E G E R | 12 | |
| 100 | L L E Q L E E T T R E G E R R | 12 | |
| 105 | E E T T R E G E R R E Q V L K | 12 | |
| 109 | R E G E R R E Q V L K A L S E | 12 | |
| 111 | G E R R E Q V L K A L S E E K | 12 | |
| 117 | V L K A L S E E K D V L K Q Q | 12 | |
| 120 | A L S E E K D V L K Q Q L S A | 12 | |
| 121 | L S E E K D V L K Q Q L S A A | 12 | |
| 126 | D V L K Q Q L S A A T S R I A | 12 | |
| 133 | S A A T S R I A E L E S K T N | 12 | |
| 134 | A A T S R I A E L E S K T N T | 12 | |
| 135 | A T S R I A E L E S K T N T L | 12 | |
| 137 | S R I A E L E S K T N T L R L | 12 | |
| 138 | R I A E L E S K T N T L R L S | 12 | |
| 140 | A E L E S K T N T L R L S Q T | 12 | |
| 142 | L E S K T N T L R L S Q T V A | 12 | |
| 143 | E S K T N T L R L S Q T V A P | 12 | |
| 153 | Q T V A P N C F N S S I N N I | 12 | |
| 155 | V A P N C F N S S I N N I H E | 12 | |
| 158 | N C F N S S I N N I H E M E I | 12 | |
| 163 | S I N N I H E M E I Q L K D A | 12 | |
| 165 | N N I H E M E I Q L K D A L E | 12 | |
| 166 | N I H E M E I Q L K D A L E K | 12 | |
| 170 | M E I Q L K D A L E K N Q Q W | 12 | |
| 172 | I Q L K D A L E K N Q Q W L V | 12 | |
| 173 | Q L K D A L E K N Q Q W L V Y | 12 | |
| 179 | E K N Q Q W L V Y D Q Q R E V | 12 | |
| 187 | Y D Q Q R E V Y V K G L L A K | 12 | |
| 189 | Q Q R E V Y V K G L L A K I F | 12 | |
| 194 | Y V K G L L A K I F E L E K K | 12 | |
| 197 | G L L A K I F E L E K K T E T | 12 | |
| 198 | L L A K I F E L E K K T E T A | 12 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 204 | E L E K K T E T A A H S L P Q | 12 | |
| 206 | E K K T E T A A H S L P Q Q T | 12 | |
| 211 | T A A H S L P Q Q T K K P E S | 12 | |
| 218 | Q Q T K K P E S E G Y L Q E E | 12 | |
| 222 | K P E S E G Y L Q E E K Q K C | 12 | |
| 223 | P E S E G Y L Q E E K Q K C Y | 12 | |
| 230 | Q E E K Q K C Y N D L L A S A | 12 | |
| 233 | K Q K C Y N D L L A S A K K D | 12 | |
| 242 | A S A K K D L E V E R Q T I T | 12 | |
| 243 | S A K K D L E V E R Q T I T Q | 12 | |
| 248 | L E V E R Q T I T Q L S F E L | 12 | |
| 249 | E V E R Q T I T Q L S F E L S | 12 | |
| 251 | E R Q T I T Q L S F E L S E F | 12 | |
| 258 | L S F E L S E F R R K Y E E T | 12 | |
| 264 | E F R R K Y E E T Q K E V H N | 12 | |
| 272 | T Q K E V H N L N Q L L Y S Q | 12 | |
| 277 | H N L N Q L L Y S Q R R A D V | 12 | |
| 278 | N L N Q L L Y S Q R R A D V Q | 12 | |
| 283 | L Y S Q R R A D V Q H L E D D | 12 | |
| 284 | Y S Q R R A D V Q H L E D D R | 12 | |
| 285 | S Q R R A D V Q H L E D D R H | 12 | |
| 289 | A D V Q H L E D D R H K T E K | 12 | |
| 293 | H L E D D R H K T E K I Q K L | 12 | |
| 296 | D D R H K T E K I Q K L R E E | 12 | |
| 306 | K L R E E N D I A R G K L E E | 12 | |
| 312 | D I A R G K L E E E K K R S E | 12 | |
| 314 | A R G K L E E E K K R S E E L | 12 | |
| 320 | E E K K R S E E L L S Q V Q F | 12 | |
| 321 | E K K R S E E L L S Q V Q F L | 12 | |
| 323 | K R S E E L L S Q V Q F L Y T | 12 | |
| 328 | L L S Q V Q F L Y T S L L K Q | 12 | |
| 330 | S Q V Q F L Y T S L L K Q Q E | 12 | |
| 334 | F L Y T S L L K Q Q E E Q T R | 12 | |
| 340 | L K Q Q E E Q T R V A L L E Q | 12 | |
| 343 | Q E E Q T R V A L L E Q Q M Q | 12 | |
| 344 | E E Q T R V A L L E Q Q M Q A | 12 | |
| 345 | E Q T R V A L L E Q Q M Q A C | 12 | |
| 352 | L E Q Q M Q A C T L D F E N E | 12 | |
| 356 | M Q A C T L D F E N E K L D R | 12 | |
| 366 | E K L D R Q H V Q H Q L H V I | 12 | |
| 367 | K L D R Q H V Q H Q L H V I L | 12 | |
| 368 | L D R Q H V Q H Q L H V I L K | 12 | |
| 371 | Q H V Q H Q L H V I L K E L R | 12 | |
| 373 | V Q H Q L H V I L K E L R K A | 12 | |
| 375 | H Q L H V I L K E L R K A R N | 12 | |
| 389 | N Q I T Q L E S L K Q L H E F | 12 | |
| 393 | Q L E S L K Q L H E F A I T E | 12 | |
| 399 | Q L H E F A I T E P L V T F Q | 12 | |
| 403 | F A I T E P L V T F Q G E T E | 12 | |
| 405 | I T E P L V T F Q G E T E N R | 12 | |
| 410 | V T F Q G E T E N R E K V A A | 12 | |
| 414 | G E T E N R E K V A A S P K S | 12 | |
| 416 | T E N R E K V A A S P K S P T | 12 | |
| 420 | E K V A A S P K S P T A A L N | 12 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 422 | V A A S P K S P T A A L N E S | 12 | |
| 425 | S P K S P T A A L N E S L V E | 12 | |
| 427 | K S P T A A L N E S L V E C P | 12 | |
| 431 | A A L N E S L V E C P K C N I | 12 | |
| 433 | L N E S L V E C P K C N I Q Y | 12 | |
| 438 | V E C P K C N I Q Y P A T E H | 12 | |
| 439 | E C P K C N I Q Y P A T E H R | 12 | |
| 448 | P A T E H R D L L V H V E Y C | 12 | |
| 450 | T E H R D L L V H V E Y C S K | 12 | |
| 262 | L S E F R R K Y E E T Q K E V | 11 | |
| 281 | Q L L Y S Q R R A D V Q H L E | 11 | |
| 72 | K N A Y Q L T E K D K E I Q R | 10 | |
| 266 | R R K Y E E T Q K E V H N L N | 10 | |
| 56 | R H R L L E K I R V L E A E K | 9 | |
| 74 | A Y Q L T E K D K E I Q R L R | 9 | |
| 139 | I A E L E S K T N T L R L S Q | 9 | |
| 169 | E M E I Q L K D A L E K N Q Q | 9 | |
| 190 | Q R E V Y V K G L L A K I F E | 9 | |
| 202 | I F E L E K K T E T A A H S L | 9 | |
| 376 | Q L H V I L K E L R K A R N Q | 9 | |
| 84 | I Q R L R D Q L K A R Y S T T | 8 | |
| 148 | T L R L S Q T V A P N C F N S | 8 | |
| 213 | A H S L P Q Q T K K P E S E G | 8 | |
| 310 | E N D I A R G K L E E E K K R | 8 | |
| 337 | T S L L K Q Q E E Q T R V A L | 8 | |
| 348 | R V A L L E Q Q M Q A C T L D | 8 | |
| 353 | E Q Q M Q A C T L D F E N E K | 8 | |
| 430 | T A A L N E S L V E C P K C N | 8 | |
| 80 | K D K E I Q R L R D Q L K A R | 7 | |
| 82 | K E I Q R L R D Q L K A R Y S | 7 | |
| 86 | R L R D Q L K A R Y S T T A L | 7 | |
| 108 | T R E G E R R E Q V L K A L S | 7 | |
| 123 | E E K D V L K Q Q L S A A T S | 7 | |
| 144 | S K T N T L R L S Q T V A P N | 7 | |
| 174 | L K D A L E K N Q Q W L V Y D | 7 | |
| 201 | K I F E L E K K T E T A A H S | 7 | |
| 246 | K D L E V E R Q T I T Q L S F | 7 | |
| 300 | K T E K I Q K L R E E N D I A | 7 | |
| 317 | K L E E E K K R S E E L L S Q | 7 | |
| 335 | L Y T S L L K Q Q E E Q T R V | 7 | |
| 380 | I L K E L R K A R N Q I T Q L | 7 | |
| 382 | K E L R K A R N Q I T Q L E S | 7 | |
| 3 | S R S T K D L I K S K W G S K | 6 | |
| 9 | L I K S K W G S K P S N S K S | 6 | |
| 10 | I K S K W G S K P S N S K S E | 6 | |
| 13 | K W G S K P S N S K S E T T L | 6 | |
| 14 | W G S K P S N S K S E T T L E | 6 | |
| 17 | K P S N S K S E T T L E K L K | 6 | |
| 19 | S N S K S E T T L E K L K G E | 6 | |
| 20 | N S K S E T T L E K L K G E I | 6 | |
| 21 | S K S E T T L E K L K G E I A | 6 | |
| 22 | K S E T T L E K L K G E I A H | 6 | |
| 26 | T L E K L K G E I A H L K T S | 6 | |
| 30 | L K G E I A H L K T S V D E I | 6 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 33 | E I A H L K T S V D E I T S G | 6 | |
| 36 | H L K T S V D E I T S G K G K | 6 | |
| 39 | T S V D E I T S G K G K L T D | 6 | |
| 40 | S V D E I T S G K G K L T D K | 6 | |
| 43 | E I T S G K G K L T D K E R H | 6 | |
| 45 | T S G K G K L T D K E R H R L | 6 | |
| 47 | G K G K L T D K E R H R L L E | 6 | |
| 51 | L T D K E R H R L L E K I R V | 6 | |
| 53 | D K E R H R L L E K I R V L E | 6 | |
| 67 | E A E K E K N A Y Q L T E K D | 6 | |
| 69 | E K E K N A Y Q L T E K D K E | 6 | |
| 70 | K E K N A Y Q L T E K D K E I | 6 | |
| 75 | Y Q L T E K D K E I Q R L R D | 6 | |
| 79 | E K D K E I Q R L R D Q L K A | 6 | |
| 83 | E I Q R L R D Q L K A R Y S T | 6 | |
| 94 | R Y S T T A L L E Q L E E T T | 6 | |
| 104 | L E E T T R E G E R R E Q V L | 6 | |
| 110 | E G E R R E Q V L K A L S E E | 6 | |
| 112 | E R R E Q V L K A L S E E K D | 6 | |
| 116 | Q V L K A L S E E K D V L K Q | 6 | |
| 130 | Q Q L S A A T S R I A E L E S | 6 | |
| 131 | Q L S A A T S R I A E L E S K | 6 | |
| 141 | E L E S K T N T L R L S Q T V | 6 | |
| 147 | N T L R L S Q T V A P N C F N | 6 | |
| 149 | L R L S Q T V A P N C F N S S | 6 | |
| 151 | L S Q T V A P N C F N S S I N | 6 | |
| 156 | A P N C F N S S I N N I H E M | 6 | |
| 159 | C F N S S I N N I H E M E I Q | 6 | |
| 160 | F N S S I N N I H E M E I Q L | 6 | |
| 162 | S S I N N I H E M E I Q L K D | 6 | |
| 168 | H E M E I Q L K D A L E K N Q | 6 | |
| 178 | L E K N Q Q W L V Y D Q Q R E | 6 | |
| 180 | K N Q Q W L V Y D Q Q R E V Y | 6 | |
| 186 | V Y D Q Q R E V Y V K G L L A | 6 | |
| 188 | D Q Q R E V Y V K G L L A K I | 6 | |
| 193 | V Y V K G L L A K I F E L E K | 6 | |
| 203 | F E L E K K T E T A A H S L P | 6 | |
| 205 | L E K K T E T A A H S L P Q Q | 6 | |
| 208 | K T E T A A H S L P Q Q T K K | 6 | |
| 209 | T E T A A H S L P Q Q T K K P | 6 | |
| 212 | A A H S L P Q Q T K K P E S E | 6 | |
| 214 | H S L P Q Q T K K P E S E G Y | 6 | |
| 217 | P Q Q T K K P E S E G Y L Q E | 6 | |
| 219 | Q T K K P E S E G Y L Q E E K | 6 | |
| 220 | T K K P E S E G Y L Q E E K Q | 6 | |
| 224 | E S E G Y L Q E E K Q K C Y N | 6 | |
| 231 | E E K Q K C Y N D L L A S A K | 6 | |
| 232 | E K Q K C Y N D L L A S A K K | 6 | |
| 236 | C Y N D L L A S A K K D L E V | 6 | |
| 241 | L A S A K K D L E V E R Q T I | 6 | |
| 254 | T I T Q L S F E L S E F R R K | 6 | |
| 256 | T Q L S F E L S E F R R K Y E | 6 | |
| 263 | S E F R R K Y E E T Q K E V H | 6 | |
| 267 | R K Y E E T Q K E V H N L N Q | 6 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 271 | E T Q K E V H N L N Q L L Y S | 6 | |
| 274 | K E V H N L N Q L L Y S Q R R | 6 | |
| 275 | E V H N L N Q L L Y S Q R R A | 6 | |
| 295 | E D D R H K T E K I Q K L R E | 6 | |
| 298 | R H K T E K I Q K L R E E N D | 6 | |
| 308 | R E E N D I A R G K L E E E K | 6 | |
| 313 | I A R G K L E E E K K R S E E | 6 | |
| 319 | E E E K K R S E E L L S Q V Q | 6 | |
| 324 | R S E E L L S Q V Q F L Y T S | 6 | |
| 327 | E L L S Q V Q F L Y T S L L K | 6 | |
| 341 | K Q Q E E Q T R V A L L E Q Q | 6 | |
| 350 | A L L E Q Q M Q A C T L D F E | 6 | |
| 354 | Q Q M Q A C T L D F E N E K L | 6 | |
| 355 | Q M Q A C T L D F E N E K L D | 6 | |
| 369 | D R Q H V Q H Q L H V I L K E | 6 | |
| 383 | E L R K A R N Q I T Q L E S L | 6 | |
| 384 | L R K A R N Q I T Q L E S L K | 6 | |
| 386 | K A R N Q I T Q L E S L K Q L | 6 | |
| 387 | A R N Q I T Q L E S L K Q L H | 6 | |
| 395 | E S L K Q L H E F A I T E P L | 6 | |
| 396 | S L K Q L H E F A I T E P L V | 6 | |
| 401 | H E F A I T E P L V T F Q G E | 6 | |
| 404 | A I T E P L V T F Q G E T E N | 6 | |
| 408 | P L V T F Q G E T E N R E K V | 6 | |
| 412 | F Q G E T E N R E K V A A S P | 6 | |
| 418 | N R E K V A A S P K S P T A A | 6 | |
| 423 | A A S P K S P T A A L N E S L | 6 | |
| 428 | S P T A A L N E S L V E C P K | 6 | |
| 429 | P T A A L N E S L V E C P K C | 6 | |
| 432 | A L N E S L V E C P K C N I Q | 6 | |
| 443 | C N I Q Y P A T E H R D L L V | 6 | |
| 446 | Q Y P A T E H R D L L V H V E | 6 | |
| 449 | A T E H R D L L V H V E Y C S | 6 | |
| 7 | K D L I K S K W G S K P S N S | 3 | |
| 195 | V K G L L A K I F E L E K K T | 3 | |
| 1 | M S S R S T K D L I K S K W G | 1 | |
| 5 | S T K D L I K S K W G S K P S | 1 | |
| 16 | S K P S N S K S E T T L E K L | 1 | |
| 23 | S E T T L E K L K G E I A H L | 1 | |
| 32 | G E I A H L K T S V D E I T S | 1 | |
| 44 | I T S G K G K L T D K E R H R | 1 | |
| 50 | K L T D K E R H R L L E K I R | 1 | |
| 52 | T D K E R H R L L E K I R V L | 1 | |
| 66 | L E A E K E K N A Y Q L T E K | 1 | |
| 76 | Q L T E K D K E I Q R L R D Q | 1 | |
| 107 | T T R E G E R R E Q V L K A L | 1 | |
| 119 | K A L S E E K D V L K Q Q L S | 1 | |
| 185 | L V Y D Q Q R E V Y V K G L L | 1 | |
| 227 | G Y L Q E E K Q K C Y N D L L | 1 | |
| 229 | L Q E E K Q K C Y N D L L A S | 1 | |
| 239 | D L L A S A K K D L E V E R Q | 1 | |
| 261 | E L S E F R R K Y E E T Q K E | 1 | |
| 268 | K Y E E T Q K E V H N L N Q L | 1 | |
| 292 | Q H L E D D R H K T E K I Q K | 1 | |

| TABLE L 121P2A3 v.1: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 294 | L E D D R H K T E K I Q K L R | 1 | |
| 297 | D R H K T E K I Q K L R E E N | 1 | |
| 302 | E K I Q K L R E E N D I A R G | 1 | |
| 311 | N D I A R G K L E E E K K R S | 1 | |
| 316 | G K L E E E K K R S E E L L S | 1 | |
| 318 | L E E E K K R S E E L L S Q V | 1 | |
| 364 | E N E K L D R Q H V Q H Q L H | 1 | |
| 379 | V I L K E L R K A R N Q I T Q | 1 | |
| 413 | Q G E T E N R E K V A A S P K | 1 | |
| 421 | K V A A S P K S P T A A L N E | 1 | |
| 436 | S L V E C P K C N I Q Y P A T | 1 | |
| 25 | T T L E K L K G E I A H L K T | -5 | |
| 42 | D E I T S G K G K L T D K E R | -5 | |
| 58 | R L L E K I R V L E A E K E K | -5 | |
| 64 | R V L E A E K E K N A Y Q L T | -5 | |
| 103 | Q L E E T T R E G E R R E Q V | -5 | |
| 113 | R R E Q V L K A L S E E K D V | -5 | |
| 132 | L S A A T S R I A E L E S K T | -5 | |
| 215 | S L P Q Q T K K P E S E G Y L | -5 | |
| 216 | L P Q Q T K K P E S E G Y L Q | -5 | |
| 240 | L L A S A K K D L E V E R Q T | -5 | |
| 260 | F E L S E F R R K Y E E T Q K | -5 | |
| 282 | L L Y S Q R R A D V Q H L E D | -5 | |
| 309 | E E N D I A R G K L E E E K K | -5 | |
| 342 | Q Q E E Q T R V A L L E Q Q M | -5 | |
| 361 | L D F E N E K L D R Q H V Q H | -5 | |
| 392 | T Q L E S L K Q L H E F A I T | -5 | |
| 415 | E T E N R E K V A A S P K S P | -5 | |
| 447 | Y P A T E H R D L L V H V E Y | -5 | |

| TABLE L 121P2A3 v.3: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 15 | Q R L L E K I R V L E A E K E | 20 | |
| 7 | G K L T D K E R Q R L L E K I | 18 | |
| 6 | K G K L T D K E R Q R L L E K | 15 | |
| 12 | K E R Q R L L E K I R V L E A | 12 | |
| 13 | E R Q R L L E K I R V L E A E | 12 | |
| 14 | R Q R L L E K I R V L E A E K | 9 | |
| 1 | E I T S G K G K L T D K E R Q | 6 | |
| 3 | T S G K G K L T D K E R Q R L | 6 | |
| 4 | S G K G K L T D K E R Q R L L | 6 | |
| 5 | G K G K L T D K E R Q R L L E | 6 | |
| 9 | L T D K E R Q R L L E K I R V | 6 | |
| 11 | D K E R Q R L L E K I R V L E | 6 | |
| 2 | I T S G K G K L T D K E R Q R | 1 | |
| 8 | K L T D K E R Q R L L E K I R | 1 | |
| 10 | T D K E R Q R L L E K I R V L | 1 | |

| TABLE L 121P2A3 v.4: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 8 | K A R Y S T T T L L E Q L E E | 16 | |

| TABLE L 121P2A3 v.4: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 4 | R D Q L K A R Y S T T T L L E | 15 | |
| 13 | T T T L L E Q L E E T T R E G | 14 | |
| 14 | T T L L E Q L E E T T R E G E | 14 | |
| 1 | Q R L R D Q L K A R Y S T T T | 12 | |
| 3 | L R D Q L K A R Y S T T T L L | 12 | |
| 5 | D Q L K A R Y S T T T L L E Q | 12 | |
| 6 | Q L K A R Y S T T T L L E Q L | 12 | |
| 11 | Y S T T T L L E Q L E E T T R | 12 | |
| 12 | S T T T L L E Q L E E T T R E | 12 | |
| 2 | R L R D Q L K A R Y S T T T L | 7 | |
| 7 | L K A R Y S T T T L L E Q L E | 6 | |
| 9 | A R Y S T T T L L E Q L E E T | 6 | |
| 10 | R Y S T T T L L E Q L E E T T | 6 | |

| TABLE L 121P2A3 v.6: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 7 | E E L L S Q V Q S L Y T S L L | 26 | |
| 10 | L S Q V Q S L Y T S L L K Q Q | 26 | |
| 3 | K K R S E E L L S Q V Q S L Y | 18 | |
| 4 | K R S E E L L S Q V Q S L Y T | 18 | |
| 14 | Q S L Y T S L L K Q Q E E Q T | 16 | |
| 6 | S E E L L S Q V Q S L Y T S L | 14 | |
| 13 | V Q S L Y T S L L K Q Q E E Q | 14 | |
| 1 | E E K K R S E E L L S Q V Q S | 12 | |
| 2 | E K K R S E E L L S Q V Q S L | 12 | |
| 11 | S Q V Q S L Y T S L L K Q Q E | 12 | |
| 15 | S L Y T S L L K Q Q E E Q T R | 12 | |

| TABLE L 121P2A3 v.7: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 12 | H Q L L V I L K E L R K A R N | 20 | |
| 14 | L L V I L K E L R K A R N Q I | 20 | |
| 15 | L V I L K E L R K A R N Q I T | 20 | |
| 4 | K L D R Q H V Q H Q L L V I L | 18 | |
| 2 | N E K L D R Q H V Q H Q L L V | 14 | |
| 7 | R Q H V Q H Q L L V I L K E L | 14 | |
| 11 | Q H Q L L V I L K E L R K A R | 14 | |
| 3 | E K L D R Q H V Q H Q L L V I | 12 | |
| 5 | L D R Q H V Q H Q L L V I L K | 12 | |
| 8 | Q H V Q H Q L L V I L K E L R | 12 | |
| 10 | V Q H Q L L V I L K E L R K A | 12 | |
| 13 | Q L L V I L K E L R K A R N Q | 9 | |
| 6 | D R Q H V Q H Q L L V I L K E | 6 | |
| 9 | H V Q H Q L L V I L K E L R K | 6 | |
| 1 | E N E K L D R Q H V Q H Q L L | 1 | |

| TABLE L 121P2A3 v.8: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 6 | P K S P T A A L N G S L V E C | 18 | |
| 14 | N G S L V E C P K C N I Q Y P | 14 | |

| TABLE L 121P2A3 v.8: HLA Peptide Scoring Results DRB1*0401 15 - mers SYFPEITHI | | | |
|---|---|---|---|
| Pos | 1 2 3 4 5 6 7 8 9 0 1 2 3 4 5 | score | SEQ. ID NO. |
| 15 | G S L V E C P K C N I Q Y P A | 14 | |
| 2 | V A A S P K S P T A A L N G S | 12 | |
| 5 | S P K S P T A A L N G S L V E | 12 | |
| 7 | K S P T A A L N G S L V E C P | 12 | |
| 11 | A A L N G S L V E C P K C N I | 12 | |
| 13 | L N G S L V E C P K C N I Q Y | 12 | |
| 10 | T A A L N G S L V E C P K C N | 8 | |
| 3 | A A S P K S P T A A L N G S L | 6 | |
| 8 | S P T A A L N G S L V E C P K | 6 | |
| 12 | A L N G S L V E C P K C N I Q | 6 | |
| 1 | K V A A S P K S P T A A L N G | | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 333 | Q F L Y T S L L K Q Q E E Q T | 26 | |
| 21 | S K S E T T L E K L K G E I A | 23 | |
| 374 | Q H Q L H V I L K E L R K A R | 22 | |
| 378 | H V I L K E L R K A R N Q I T | 22 | |
| 111 | G E R R E Q V L K A L S E E K | 21 | |
| 199 | L A K I F E L E K K T E T A A | 21 | |
| 252 | R Q T I T Q L S F E L S E F R | 21 | |
| 24 | E T T L E K L K G E I A H L K | 19 | |
| 38 | K T S V D E I T S G K G K L T | 19 | |
| 72 | K N A Y Q L T E K D K E I Q R | 19 | |
| 225 | S E G Y L Q E E K Q K C Y N D | 19 | |
| 266 | R R K Y E E T Q K E V H N L N | 19 | |
| 444 | N I Q Y P A T E H R D L L V H | 19 | |
| 48 | K G K L T D K E R H R L L E K | 18 | |
| 57 | H R L L E K I R V L E A E K E | 18 | |
| 200 | A K I F E L E K K T E T A A H | 18 | |
| 74 | A Y Q L T E K D K E I Q R L R | 17 | |
| 54 | K E R H R L L E K I R V L E A | 16 | |
| 78 | T E K D K E I Q R L R D Q L K | 16 | |
| 84 | I Q R L R D Q L K A R Y S T T | 16 | |
| 234 | Q K C Y N D L L A S A K K D L | 16 | |
| 298 | R H K T E K I Q K L R E E N D | 16 | |
| 314 | A R G K L E E E K K R S E E L | 16 | |
| 450 | T E H R D L L V H V E Y C S K | 16 | |
| 3 | S R S T K D L I K S K W G S K | 15 | |
| 28 | E K L K G E I A H L K T S V D | 15 | |
| 56 | R H R L L E K I R V L E A E K | 15 | |
| 62 | K I R V L E A E K E K N A Y Q | 15 | |
| 183 | Q W L V Y D Q Q R E V Y V K G | 15 | |
| 206 | E K K T E T A A H S L P Q Q T | 15 | |
| 238 | N D L L A S A K K D L E V E R | 15 | |
| 279 | L N Q L L Y S Q R R A D V Q H | 15 | |
| 307 | L R E E N D I A R G K L E E E | 15 | |
| 315 | R G K L E E E K K R S E E L L | 15 | |
| 367 | K L D R Q H V Q H Q L H V I L | 15 | |
| 370 | R Q H V Q H Q L H V I L K E L | 15 | |
| 388 | R N Q I T Q L E S L K Q L H E | 15 | |
| 5 | S T K D L I K S K W G S K P S | 14 | |
| 35 | A H L K T S V D E I T S G K G | 14 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 60 | L E K I R V L E A E K E K N A | 14 | |
| 101 | L E Q L E E T T R E G E R R E | 14 | |
| 121 | L S E E K D V L K Q Q L S A A | 14 | |
| 122 | S E E K D V L K Q Q L S A A T | 14 | |
| 129 | K Q Q L S A A T S R I A E L E | 14 | |
| 167 | I H E M E I Q L K D A L E K N | 14 | |
| 172 | I Q L K D A L E K N Q Q W L V | 14 | |
| 193 | V Y V K G L L A K I F E L E K | 14 | |
| 213 | A H S L P Q Q T K K P E S E G | 14 | |
| 214 | H S L P Q Q T K K P E S E G Y | 14 | |
| 237 | Y N D L L A S A K K D L E V E | 14 | |
| 244 | A K K D L E V E R Q T I T Q L | 14 | |
| 259 | S F E L S E F R R K Y E E T Q | 14 | |
| 280 | N Q L L Y S Q R R A D V Q H L | 14 | |
| 285 | S Q R R A D V Q H L E D D R H | 14 | |
| 288 | R A D V Q H L E D D R H K T E | 14 | |
| 291 | V Q H L E D D R H K T E K I Q | 14 | |
| 362 | D F E N E K L D R Q H V Q H Q | 14 | |
| 376 | Q L H V I L K E L R K A R N Q | 14 | |
| 377 | L H V I L K E L R K A R N Q I | 14 | |
| 393 | Q L E S L K Q L H E F A I T E | 14 | |
| 403 | F A I T E P L V T F Q G E T E | 14 | |
| 413 | Q G E T E N R E K V A A S P K | 14 | |
| 419 | R E K V A A S P K S P T A A L | 14 | |
| 434 | N E S L V E C P K C N I Q Y P | 14 | |
| 7 | K D L I K S K W G S K P S N S | 13 | |
| 31 | K G E I A H L K T S V D E I T | 13 | |
| 81 | D K E I Q R L R D Q L K A R Y | 13 | |
| 85 | Q R L R D Q L K A R Y S T T A | 13 | |
| 115 | E Q V L K A L S E E K D V L K | 13 | |
| 145 | K T N T L R L S Q T V A P N C | 13 | |
| 164 | I N N I H E M E I Q L K D A L | 13 | |
| 181 | N Q Q W L V Y D Q Q R E V Y V | 13 | |
| 189 | Q Q R E V Y V K G L L A K I F | 13 | |
| 192 | E V Y V K G L L A K I F E L E | 13 | |
| 257 | Q L S F E L S E F R R K Y E E | 13 | |
| 273 | Q K E V H N L N Q L L Y S Q R | 13 | |
| 329 | L S Q V Q F L Y T S L L K Q Q | 13 | |
| 4 | R S T K D L I K S K W G S K P | 12 | |
| 63 | I R V L E A E K E K N A Y Q L | 12 | |
| 88 | R D Q L K A R Y S T T A L L E | 12 | |
| 98 | T A L L E Q L E E T T R E G E | 12 | |
| 124 | E K D V L K Q Q L S A A T S R | 12 | |
| 126 | D V L K Q Q L S A A T S R I A | 12 | |
| 133 | S A A T S R I A E L E S K T N | 12 | |
| 136 | T S R I A E L E S K T N T L R | 12 | |
| 146 | T N T L R L S Q T V A P N C F | 12 | |
| 152 | S Q T V A P N C F N S S I N N | 12 | |
| 161 | N S S I N N I H E M E I Q L K | 12 | |
| 166 | N I H E M E I Q L K D A L E K | 12 | |
| 188 | D Q Q R E V Y V K G L L A K I | 12 | |
| 196 | K G L L A K I F E L E K K T E | 12 | |
| 202 | I F E L E K K T E T A A H S L | 12 | |
| 276 | V H N L N Q L L Y S Q R R A D | 12 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 301 | T E K I Q K L R E E N D I A R | 12 | |
| 326 | E E L L S Q V Q F L Y T S L L | 12 | |
| 346 | Q T R V A L L E Q Q M Q A C T | 12 | |
| 360 | T L D F E N E K L D R Q H V Q | 12 | |
| 385 | R K A R N Q I T Q L E S L K Q | 12 | |
| 391 | I T Q L E S L K Q L H E F A I | 12 | |
| 394 | L E S L K Q L H E F A I T E P | 12 | |
| 397 | L K Q L H E F A I T E P L V T | 12 | |
| 409 | L V T F Q G E T E N R E K V A | 12 | |
| 430 | T A A L N E S L V E C P K C N | 12 | |
| 157 | P N C F N S S I N N I H E M E | 11 | |
| 191 | R E V Y V K G L L A K I F E L | 11 | |
| 269 | Y E E T Q K E V H N L N Q L L | 11 | |
| 281 | Q L L Y S Q R R A D V Q H L E | 11 | |
| 331 | Q V Q F L Y T S L L K Q Q E E | 11 | |
| 400 | L H E F A I T E P L V T F Q G | 11 | |
| 11 | K S K W G S K P S N S K S E T | 10 | |
| 46 | S G K G K L T D K E R H R L L | 10 | |
| 49 | G K L T D K E R H R L L E K I | 10 | |
| 92 | K A R Y S T T A L L E Q L E E | 10 | |
| 142 | L E S K T N T L R L S Q T V A | 10 | |
| 184 | W L V Y D Q Q R E V Y V K G L | 10 | |
| 258 | L S F E L S E F R R K Y E E T | 10 | |
| 262 | L S E F R R K Y E E T Q K E V | 10 | |
| 34 | I A H L K T S V D E I T S G K | 9 | |
| 40 | S V D E I T S G K G K L T D K | 9 | |
| 41 | V D E I T S G K G K L T D K E | 9 | |
| 50 | K L T D K E R H R L L E K I R | 9 | |
| 94 | R Y S T T A L L E Q L E E T T | 9 | |
| 105 | E E T T R E G E R R E Q V L K | 9 | |
| 117 | V L K A L S E E K D V L K Q Q | 9 | |
| 186 | V Y D Q Q R E V Y V K G L L A | 9 | |
| 256 | T Q L S F E L S E F R R K Y E | 9 | |
| 278 | N L N Q L L Y S Q R R A D V Q | 9 | |
| 290 | D V Q H L E D D R H K T E K I | 9 | |
| 325 | S E E L L S Q V Q F L Y T S L | 9 | |
| 359 | C T L D F E N E K L D R Q H V | 9 | |
| 390 | Q I T Q L E S L K Q L H E F A | 9 | |
| 411 | T F Q G E T E N R E K V A A S | 9 | |
| 412 | F Q G E T E N R E K V A A S P | 9 | |
| 445 | I Q Y P A T E H R D L L V H V | 9 | |
| 9 | L I K S K W G S K P S N S K S | 8 | |
| 14 | W G S K P S N S K S E T T L E | 8 | |
| 23 | S E T T L E K L K G E I A H L | 8 | |
| 30 | L K G E I A H L K T S V D E I | 8 | |
| 42 | D E I T S G K G K L T D K E R | 8 | |
| 64 | R V L E A E K E K N A Y Q L T | 8 | |
| 80 | K D K E I Q R L R D Q L K A R | 8 | |
| 86 | R L R D Q L K A R Y S T T A L | 8 | |
| 106 | E T T R E G E R R E Q V L K A | 8 | |
| 130 | Q Q L S A A T S R I A E L E S | 8 | |
| 137 | S R I A E L E S K T N T L R L | 8 | |
| 141 | E L E S K T N T L R L S Q T V | 8 | |
| 148 | T L R L S Q T V A P N C F N S | 8 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 160 | F N S S I N N I H E M E I Q L | 8 | |
| 179 | E K N Q Q W L V Y D Q Q R E V | 8 | |
| 216 | L P Q Q T K K P E S E G Y L Q | 8 | |
| 227 | G Y L Q E E K Q K C Y N D L L | 8 | |
| 242 | A S A K K D L E V E R Q T I T | 8 | |
| 245 | K K D L E V E R Q T I T Q L S | 8 | |
| 260 | F E L S E F R R K Y E E T Q K | 8 | |
| 277 | H N L N Q L L Y S Q R R A D V | 8 | |
| 292 | Q H L E D D R H K T E K I Q K | 8 | |
| 295 | E D D R H K T E K I Q K L R E | 8 | |
| 300 | K T E K I Q K L R E E N D I A | 8 | |
| 305 | Q K L R E E N D I A R G K L E | 8 | |
| 309 | E E N D I A R G K L E E E K K | 8 | |
| 316 | G K L E E E K K R S E E L L S | 8 | |
| 339 | L L K Q Q E E Q T R V A L L E | 8 | |
| 340 | L K Q Q E E Q T R V A L L E Q | 8 | |
| 343 | Q E E Q T R V A L L E Q Q M Q | 8 | |
| 364 | E N E K L D R Q H V Q H Q L H | 8 | |
| 375 | H Q L H V I L K E L R K A R N | 8 | |
| 380 | I L K E L R K A R N Q I T Q L | 8 | |
| 399 | Q L H E F A I T E P L V T F Q | 8 | |
| 402 | E F A I T E P L V T F Q G E T | 8 | |
| 407 | E P L V T F Q G E T E N R E K | 8 | |
| 415 | E T E N R E K V A A S P K S P | 8 | |
| 417 | E N R E K V A A S P K S P T A | 8 | |
| 431 | A A L N E S L V E C P K C N I | 8 | |
| 1 | M S S R S T K D L I K S K W G | 7 | |
| 12 | S K W G S K P S N S K S E T T | 7 | |
| 27 | L E K L K G E I A H L K T S V | 7 | |
| 36 | H L K T S V D E I T S G K G K | 7 | |
| 53 | D K E R H R L L E K I R V L E | 7 | |
| 82 | K E I Q R L R D Q L K A R Y S | 7 | |
| 100 | L L E Q L E E T T R E G E R R | 7 | |
| 109 | R E G E R R E Q V L K A L S E | 7 | |
| 112 | E R R E Q V L K A L S E E K D | 7 | |
| 118 | L K A L S E E K D V L K Q Q L | 7 | |
| 139 | I A E L E S K T N T L R L S Q | 7 | |
| 168 | H E M E I Q L K D A L E K N Q | 7 | |
| 175 | K D A L E K N Q Q W L V Y D Q | 7 | |
| 182 | Q Q W L V Y D Q Q R E V Y V K | 7 | |
| 190 | Q R E V Y V K G L L A K I F E | 7 | |
| 195 | V K G L L A K I F E L E K K T | 7 | |
| 212 | A A H S L P Q Q T K K P E S E | 7 | |
| 223 | P E S E G Y L Q E E K Q K C Y | 7 | |
| 231 | E E K Q K C Y N D L L A S A K | 7 | |
| 235 | K C Y N D L L A S A K K D L E | 7 | |
| 248 | L E V E R Q T I T Q L S F E L | 7 | |
| 303 | K I Q K L R E E N D I A R G K | 7 | |
| 312 | D I A R G K L E E E K K R S E | 7 | |
| 319 | E E E K K R S E E L L S Q V Q | 7 | |
| 322 | K K R S E E L L S Q V Q F L Y | 7 | |
| 327 | E L L S Q V Q F L Y T S L L K | 7 | |
| 358 | A C T L D F E N E K L D R Q H | 7 | |
| 371 | Q H V Q H Q L H V I L K E L R | 7 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 381 | LKELRKARNQITQLE | 7 | |
| 427 | KSPTAALNESLVECP | 7 | |
| 432 | ALNESLVECPKCNIQ | 7 | |
| 435 | ESLVECPKCNIQYPA | 7 | |
| 449 | ATEHRDLLVHVEYCS | 7 | |
| 6 | TKDLIKSKWGSKPSN | 6 | |
| 8 | DLIKSKWGSKPSNSK | 6 | |
| 10 | IKSKWGSKPSNSKSE | 6 | |
| 29 | KLKGEIAHLKTSVDE | 6 | |
| 45 | TSGKGKLTDKERHRL | 6 | |
| 59 | LLEKIRVLEAEKEKN | 6 | |
| 71 | EKNAYQLTEKDKEIQ | 6 | |
| 95 | YSTTALLEQLEETTR | 6 | |
| 97 | TTALLEQLEETTREG | 6 | |
| 114 | REQVLKALSEEKDVL | 6 | |
| 123 | EEKDVLKQQLSAATS | 6 | |
| 125 | KDVLKQQLSAATSRI | 6 | |
| 143 | ESKTNTLRLSQTVAP | 6 | |
| 149 | LRLSQTVAPNCFNSS | 6 | |
| 151 | LSQTVAPNCFNSSIN | 6 | |
| 158 | NCFNSSINNIHEMEI | 6 | |
| 169 | EMEIQLKDALEKNQQ | 6 | |
| 171 | EIQLKDALEKNQQWL | 6 | |
| 180 | KNQQWLVYDQQREVY | 6 | |
| 187 | YDQQREVYVKGLLAK | 6 | |
| 201 | KIFELEKKTETAAHS | 6 | |
| 204 | ELEKKTETAAHSLPQ | 6 | |
| 210 | ETAAHSLPQQTKKPE | 6 | |
| 226 | EGYLQEEKQKCYNDL | 6 | |
| 232 | EKQKCYNDLLASAKK | 6 | |
| 233 | KQKCYNDLLASAKKD | 6 | |
| 247 | DLEVERQTITQLSFE | 6 | |
| 249 | EVERQTITQLSFELS | 6 | |
| 255 | ITQLSFELSEFRRKY | 6 | |
| 270 | EETQKEVHNLNQLLY | 6 | |
| 274 | KEVHNLNQLLYSQRR | 6 | |
| 304 | IQKLREENDIARGKL | 6 | |
| 310 | ENDIARGKLEEEKKR | 6 | |
| 323 | KRSEELLSQVQFLYT | 6 | |
| 332 | VQFLYTSLLKQQEEQ | 6 | |
| 334 | FLYTSLLKQQEEQTR | 6 | |
| 336 | YTSLLKQQEEQTRVA | 6 | |
| 337 | TSLLKQQEEQTRVAL | 6 | |
| 345 | EQTRVALLEQQMQAC | 6 | |
| 347 | TRVALLEQQMQACTL | 6 | |
| 348 | RVALLEQQMQACTLD | 6 | |
| 349 | VALLEQQMQACTLDF | 6 | |
| 350 | ALLEQQMQACTLDFE | 6 | |
| 353 | EQQMQACTLDFENEK | 6 | |
| 355 | QMQACTLDFENEKLD | 6 | |
| 365 | NEKLDRQHVQHQLHV | 6 | |
| 373 | VQHQLHVILKELRKA | 6 | |
| 404 | AITEPLVTFQGETEN | 6 | |
| 406 | TEPLVTFQGETENRE | 6 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 414 | G E T E N R E K V A A S P K S | 6 | |
| 416 | T E N R E K V A A S P K S P T | 6 | |
| 418 | N R E K V A A S P K S P T A A | 6 | |
| 420 | E K V A A S P K S P T A A L N | 6 | |
| 421 | K V A A S P K S P T A A L N E | 6 | |
| 425 | S P K S P T A A L N E S L V E | 6 | |
| 437 | L V E C P K C N I Q Y P A T E | 6 | |
| 438 | V E C P K C N I Q Y P A T E H | 6 | |
| 439 | E C P K C N I Q Y P A T E H R | 6 | |
| 442 | K C N I Q Y P A T E H R D L L | 6 | |
| 104 | L E E T T R E G E R R E Q V L | 5 | |
| 103 | Q L E E T T R E G E R R E Q V | 4 | |
| 132 | L S A A T S R I A E L E S K T | 3 | |
| 150 | R L S Q T V A P N C F N S S I | 3 | |
| 243 | S A K K D L E V E R Q T I T Q | 3 | |
| 284 | Y S Q R R A D V Q H L E D D R | 3 | |
| 297 | D R H K T E K I Q K L R E E N | 3 | |
| 342 | Q Q E E Q T R V A L L E Q Q M | 3 | |
| 372 | H V Q H Q L H V I L K E L R K | 3 | |
| 446 | Q Y P A T E H R D L L V H V E | 3 | |
| 2 | S S R S T K D L I K S K W G S | 2 | |
| 22 | K S E T T L E K L K G E I A H | 2 | |
| 55 | E R H R L L E K I R V L E A E | 2 | |
| 58 | R L L E K I R V L E A E K E K | 2 | |
| 76 | Q L T E K D K E I Q R L R D Q | 2 | |
| 77 | L T E K D K E I Q R L R D Q L | 2 | |
| 93 | A R Y S T T A L L E Q L E E T | 2 | |
| 99 | A L L E Q L E E T T R E G E R | 2 | |
| 110 | E G E R R E Q V L K A L S E E | 2 | |
| 120 | A L S E E K D V L K Q Q L S A | 2 | |
| 128 | L K Q Q L S A A T S R I A E L | 2 | |
| 140 | A E L E S K T N T L R L S Q T | 2 | |
| 144 | S K T N T L R L S Q T V A P N | 2 | |
| 205 | L E K K T E T A A H S L P Q Q | 2 | |
| 207 | K K T E T A A H S L P Q Q T K | 2 | |
| 250 | V E R Q T I T Q L S F E L S E | 2 | |
| 268 | K Y E E T Q K E V H N L N Q L | 2 | |
| 330 | S Q V Q F L Y T S L L K Q Q E | 2 | |
| 338 | S L L K Q Q E E Q T R V A L L | 2 | |
| 356 | M Q A C T L D F E N E K L D R | 2 | |
| 366 | E K L D R Q H V Q H Q L H V I | 2 | |
| 426 | P K S P T A A L N E S L V E C | 2 | |
| 443 | C N I Q Y P A T E H R D L L V | 2 | |
| 17 | K P S N S K S E T T L E K L K | 1 | |
| 19 | S N S K S E T T L E K L K G E | 1 | |
| 20 | N S K S E T T L E K L K G E I | 1 | |
| 44 | I T S G K G K L T D K E R H R | 1 | |
| 47 | G K G K L T D K E R H R L L E | 1 | |
| 52 | T D K E R H R L L E K I R V L | 1 | |
| 67 | E A E K E K N A Y Q L T E K D | 1 | |
| 70 | K E K N A Y Q L T E K D K E I | 1 | |
| 79 | E K D K E I Q R L R D Q L K A | 1 | |
| 90 | Q L K A R Y S T T A L L E Q L | 1 | |
| 91 | L K A R Y S T T A L L E Q L E | 1 | |

| TABLE LI 121P2A3 v.1: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 107 | T T R E G E R R E Q V L K A L | 1 | |
| 108 | T R E G E R R E Q V L K A L S | 1 | |
| 119 | K A L S E E K D V L K Q Q L S | 1 | |
| 135 | A T S R I A E L E S K T N T L | 1 | |
| 154 | T V A P N C F N S S I N N I H | 1 | |
| 156 | A P N C F N S S I N N I H E M | 1 | |
| 162 | S S I N N I H E M E I Q L K D | 1 | |
| 165 | N N I H E M E I Q L K D A L E | 1 | |
| 170 | M E I Q L K D A L E K N Q Q W | 1 | |
| 176 | D A L E K N Q Q W L V Y D Q Q | 1 | |
| 185 | L V Y D Q Q R E V Y V K G L L | 1 | |
| 197 | G L L A K I F E L E K K T E T | 1 | |
| 198 | L L A K I F E L E K K T E T A | 1 | |
| 211 | T A A H S L P Q Q T K K P E S | 1 | |
| 219 | Q T K K P E S E G Y L Q E E K | 1 | |
| 230 | Q E E K Q K C Y N D L L A S A | 1 | |
| 236 | C Y N D L L A S A K K D L E V | 1 | |
| 240 | L L A S A K K D L E V E R Q T | 1 | |
| 261 | E L S E F R R K Y E E T Q K E | 1 | |
| 264 | E F R R K Y E E T Q K E V H N | 1 | |
| 271 | E T Q K E V H N L N Q L L Y S | 1 | |
| 272 | T Q K E V H N L N Q L L Y S Q | 1 | |
| 286 | Q R R A D V Q H L E D D R H K | 1 | |
| 289 | A D V Q H L E D D R H K T E K | 1 | |
| 293 | H L E D D R H K T E K I Q K L | 1 | |
| 294 | L E D D R H K T E K I Q K L R | 1 | |
| 296 | D D R H K T E K I Q K L R E E | 1 | |
| 299 | H K T E K I Q K L R E E N D I | 1 | |
| 306 | K L R E E N D I A R G K L E E | 1 | |
| 308 | R E E N D I A R G K L E E E K | 1 | |
| 313 | I A R G K L E E E K K R S E E | 1 | |
| 318 | L E E E K K R S E E L L S Q V | 1 | |
| 341 | K Q Q E E Q T R V A L L E Q Q | 1 | |
| 344 | E E Q T R V A L L E Q Q M Q A | 1 | |
| 351 | L L E Q Q M Q A C T L D F E N | 1 | |
| 357 | Q A C T L D F E N E K L D R Q | 1 | |
| 361 | L D F E N E K L D R Q H V Q H | 1 | |
| 363 | F E N E K L D R Q H V Q H Q L | 1 | |
| 368 | L D R Q H V Q H Q L H V I L K | 1 | |
| 369 | D R Q H V Q H Q L H V I L K E | 1 | |
| 379 | V I L K E L R K A R N Q I T Q | 1 | |
| 384 | L R K A R N Q I T Q L E S L K | 1 | |
| 387 | A R N Q I T Q L E S L K Q L H | 1 | |
| 395 | E S L K Q L H E F A I T E P L | 1 | |
| 405 | I T E P L V T F Q G E T E N R | 1 | |
| 408 | P L V T F Q G E T E N R E K V | 1 | |
| 410 | V T F Q G E T E N R E K V A A | 1 | |
| 423 | A A S P K S P T A A L N E S L | 1 | |
| 428 | S P T A A L N E S L V E C P K | 1 | |
| 433 | L N E S L V E C P K C N I Q Y | 1 | |
| 447 | Y P A T E H R D L L V H V E Y | 1 | |

| TABLE LI 121P2A3 v.3: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 6 | K G K L T D K E R Q R L L E K | 18 | |
| 15 | Q R L L E K I R V L E A E K E | 18 | |
| 12 | K E R Q R L L E K I R V L E A | 16 | |
| 14 | R Q R L L E K I R V L E A E K | 15 | |
| 4 | S G K G K L T D K E R Q R L L | 10 | |
| 8 | K L T D K E R Q R L L E K I R | 9 | |
| 11 | D K E R Q R L L E K I R V L E | 7 | |
| 3 | T S G K G K L T D K E R Q R L | 6 | |
| 7 | G K L T D K E R Q R L L E K I | 2 | |
| 13 | E R Q R L L E K I R V L E A E | 2 | |
| 2 | I T S G K G K L T D K E R Q R | 1 | |
| 5 | G K G K L T D K E R Q R L L E | 1 | |
| 10 | T D K E R Q R L L E K I R V L | 1 | |

| TABLE LI 121P2A3 v.7: HLA Peptide Scoring Results DRB1*1101 15 - mers SYFPEITHI | | | |
|---|---|---|---|
| Pos | 1 2 3 4 5 6 7 8 9 0 1 2 3 4 5 | score | SEQ. ID NO. |
| 11 | Q H Q L L V I L K E L R K A R | 22 | |
| 15 | L V I L K E L R K A R N Q I T | 22 | |
| 4 | K L D R Q H V Q H Q L L V I L | 15 | |
| 12 | H Q L L V I L K E L R K A R N | 14 | |
| 13 | Q L L V I L K E L R K A R N Q | 14 | |
| 14 | L L V I L K E L R K A R N Q I | 14 | |
| 9 | H V Q H Q L L V I L K E L R K | 9 | |
| 1 | E N E K L D R Q H V Q H Q L L | 8 | |
| 7 | R Q H V Q H Q L L V I L K E L | 7 | |
| 8 | Q H V Q H Q L L V I L K E L R | 7 | |
| 2 | N E K L D R Q H V Q H Q L L V | 6 | |
| 10 | V Q H Q L L V I L K E L R K A | 6 | |
| 3 | E K L D R Q H V Q H Q L L V I | 2 | |
| 5 | L D R Q H V Q H Q L L V I L K | 2 | |
| 6 | D R Q H V Q H Q L L V I L K E | 1 | |

**Table LII. Peptides Used to Generate HLA Tables and Scoring Results and Position Determination Key**

>121P2A3 variants

>121P2A3 v.1 nonamers, decamers, 15-mers

MSSRSTKDLI KSKWGSKPSN SKSETTLEKL KGEIAHLKTS VDEITSGKGK LTDKERHRLL
EKIRVLEAEK EKNAYQLTEK DKEIQRLRDQ LKARYSTTAL LEQLEETTRE GERREQVLKA
LSEEKDVLKQ QLSAATSRIA ELESKTNTLR LSQTVAPNCF NSSINNIHEM EIQLKDALEK
NQQWLVYDQQ REVYVKGLLA KIFELEKKTE TAAHSLPQQT KKPESEGYLQ EEKQKCYNDL
LASAKKDLEV ERQTITQLSF ELSEFRRKYE ETQKEVHNLN QLLYSQRRAD VQHLEDDRHK
TEKIQKLREE NDIARGKLEE EKKRSEELLS QVQFLYTSLL KQQEEQTRVA LLEQQMQACT
LDFENEKLDR QHVQHQLHVI LKELRKARNQ ITQLESLKQL HEFAITEPLV TFQGETENRE
KVAASPKSPT AALNESLVEC PKCNIQYPAT EHRDLLVHVE YCSK

>121P2A3 v.3
nonamers (aa 49-65)
GKLTDKERQRLLEKIRV
decamers (aa 48-66)

(continued)

SGKGICLTDKERQRLLEKIRVL
15-mers (aa 43-71)
EITSGKGKLTDKERQRLLEKIRVLEAEKE

>121P2A3 v.4
nonamers (aa 91-107)
LKARYSTTTLLEQLEET
Decamers (aa 90-108)
QLKARYSTTTLLEQLEETT
15-mers (aa 85-113)
QRLRDQLICARYSTTTLLEQLEETTREGE

>121P2A3 v.6
nonamers (aa 326-342)
EELLSQVQSLYTSLLKQ
Decamers (aa 325-343)
SEELLSQVQSLYTSLLKQQ
15-mers (aa 320-348)
EEKKRSEELLSQVQSLYTSLLKQQEEQTR

>121P2A3 v.7
nonamers (aa 370-386)
RQHVQHQLLVILKELRK
decamers (aa 369-387)
DRQHVQHQLLVILKELRKA
15-mers (aa 364-392)
ENEKLDRQHVQHQLLVILKELRKARNQIT

>121P2A3 v.8
nonamers (aa 427-443)
KSPTAALNGSLVECPKC
Decamers (aa 426-444)
PKSPTAALNGSLVECPKC
15-mers (aa 421-449)
KVAASPKSPTAALNGSLVECPKCNIQYPA
(121P2A3 v.5 and v.9 code for the same sequence as v.1. V2 is shorter than variant 1 but nonetheless shares the same sequence over its length.)

Table LIII. Exon compositions of 121P2A3 v.1

| Exon Number | Start | End |
|---|---|---|
| Exon 1 | 2 | 162 |
| Exon 2 | 163 | 357 |
| Exon 3 | 358 | 633 |
| Exon 4 | 634 | 702 |
| Exon 5 | 703 | B53 |
| Exon 6 | 854 | 1167 |
| Exon 7 | 1168 | 1239 |

(continued)

| Exon Number | Start | End |
|---|---|---|
| Exon 8 | 1240 | 1365 |
| Exon 9 | 1366 | 2473 |

**Table LIV. Nucleotide sequence of transcript variant 121P2A3 v.2**

```
gggaccgcca gggagggcag gtcagtgggc agatcgcgtc cgcgggattc aatctctgcc    60
cgctctgata acagtccttt tccctggcgc tcacttcgtg cctggcaccc ggctgggcgc   120
ctcaagaccg ttgtctcttc gatcgcttct ttggacttgg cgaccatttc agagatgtct   180
tccagaagta ccaaagattt aattaaaaaa aattcgagtc cttgaggctg agaaggagaa   240
gaatgcttat caactcacag agaaggacaa agaaatacag cgactgagag accaactgaa   300
ggccagatat agtactaccg cattgcttga acagctggaa gagacaacga gagaaggaga   360
aaggagggag caggtgttga aagccttatc tgaagagaaa gacgtattga aacaacagtt   420
gtctgctgca acctcacgaa ttgctgaact tgaaagcaaa accaatacac tccgtttatc   480
acagactgtg gctccaaact gcttcaactc atcaataaat aatattcatg aaatggaaat   540
acagctgaaa gatgctctgg agaaaaatca gcagtggctc gtgtatgatc agcagcggga   600
agtctatgta aaaggacttt tagcaaagat ctttgagttg gaaaagaaaa cggaaacagc   660
tgctcattca ctcccacagc agacaaaaaa gcctgaatca gaaggttatc ttcaagaaga   720
gaagcagaaa tgttacaacg atctcttggc aagtgcaaaa aaagatcttg aggttgaacg   780
acaaaccata actcagctga gttttgaact gagtgaattt cgaagaaaat atgaagaaac   840
ccaaaaagaa gttcacaatt taaatcagct gttgtattca caaagaaggg cagatgtgca   900
acatctggaa gatgataggc ataaaacaga gaagatacaa aaactcaggg aagagaatga   960
tattgctagg ggaaaacttg aagaagagaa gaagagatcc gaagagctct tatctcaggt  1020
ccagtttctt tacacatctc tgctaaagca gcaagaagaa caaacaaggg tagctctgtt  1080
ggaacaacag atgcaggcat gtactttaga ctttgaaaat gaaaaactcg accgtcaaca  1140
tgtgcagcat caattgcatg taattcttaa ggagctccga aaagcaagaa atcaaataac  1200
acagttggaa tccttgaaac agcttcatga gtttgccatc acagagccat tagtcacttt  1260
ccaaggagag actgaaaaca gagaaaaagt tgccgcctca ccaaaaagtc ccactgctgc  1320
actcaatgaa agcctggtgg aatgtcccaa gtgcaatata cagtatccag ccactgagca  1380
tcgcgatctg cttgtccatg tggaatactg ttcaaagtag caaaataagt atttgttttg  1440
atattaaaag attcaatact gtattttctg ttagcttgtg ggcattttga attatatatt  1500
tcacatttg cataaaactg cctatctacc tttgacactc cagcatgcta gtgaatcatg  1560
tatcttttag gctgctgtgc atttctcttg gcagtgatac ctccctgaca tggttcatca  1620
tcaggctgca atgacagaat gtggtgagca gcgtctactg agatactaac atttgcact  1680
gtcaaaatac ttggtgagga aaagatagct caggttattg ctaatgggtt aatgcaccag  1740
caagcaaaat attttatgtt ttgggggttt tgaaaaatca aagataatta accaaggatc  1800
ttaactgtgt tcgcattttt tatccaagca cttagaaaac ctacaatcct aattttgatg  1860
tccattgtta agaggtggtg atagatacta ttttttttt catattgtat agcggttatt  1920
agaaaagttg gggattttct tgatctttat tgctgcttac cattgaaact taacccagct  1980
gtgttcccca actctgttct gcgcacgaaa cagtatctgt ttgaggcata atcttaagtg  2040
gccacacaca atgttttctc ttatgttatc tggcagtaac tgtaacttga attacattag  2100
cacattctgc ttagctaaaa ttgttaaaat aaactttaat aaacccatgt agccctctca  2160
tttgattgac agtattttag ttatttttgg cattcttaaa gctgggcaat gtaatgatca  2220
gatctttgtt tgtctgaaca ggtattttta tacatgcttt ttgtaaacca aaaactttta  2280
aatttcttca ggttttctaa catgcttacc actgggctac tgta                    2324
```

**Table LV. Nucleotide sequence alignment of 121P1F1 v.1 and 121P2A3 v.2**

```
121P2A3v.1     GGGACCGCCAGGGAGGGCAGGTCAGTGGGCAGATCGCGTCCGCGGGATTCAATCTCTGCC 60
121P2A3v.2     GGGACCGCCAGGGAGGGCAGGTCAGTGGGCAGATCGCGTCCGCGGGATTCAATCTCTGCC 60
               ************************************************************

121P2A3v.1     CGCTCTGATAACAGTCCTTTTCCCTGGCGCTCACTTCGTGCCTGGCACCCGGCTGGGCGC 120
```

```
121P2A3v.2       CGCTCTGATAACAGTCCTTTTCCCTGGCGCTCACTTCGTGCCTGGCACCCGGCTGGGCGC 120
                 ************************************************************

121P2A3v.1       CTCAAGACCGTTGTCTCTTCGATCGCTTCTTTGGACTTGGCGACCATTTCAGAGATGTCT 180
121P2A3v.2       CTCAAGACCGTTGTCTCTTCGATCGCTTCTTTGGACTTGGCGACCATTTCAGAGATGTCT 180
                 ************************************************************

121P2A3v.1       TCCAGAAGTACCAAAGATTTAATTAAAAGTAAGTGGGGATCGAAGCCTAGTAACTCCAAA 240
121P2A3v.2       TCCAGAAGTACCAAAGATTTAATTAAAA-------------------------------- 208
                 ****************************

121P2A3v.1       TCCGAAACTACATTAGAAAAATTAAAGGGAGAAATTGCACACTTAAAGACATCAGTGGAT 300
121P2A3v.2       ------------------------------------------------------------

121P2A3v.1       GAAATCACAAGTGGGAAAGGAAAGCTGACTGATAAAGAGAGACACAGACTTTTGGAGAAA 360
121P2A3v.2       ---------------------------------------------------------AAA 211
                                                                          ***

121P2A3v.1       ATTCGAGTCCTTGAGGCTGAGAAGGAGAAGAATGCTTATCAACTCACAGAGAAGGACAAA 420
121P2A3v.2       ATTCGAGTCCTTGAGGCTGAGAAGGAGAAGAATGCTTATCAACTCACAGAGAAGGACAAA 271
                 ************************************************************

121P2A3v.1       GAAATACAGCGACTGAGAGACCAACTGAAGGCCAGATATAGTACTACCGCATTGCTTGAA 480
121P2A3v.2       GAAATACAGCGACTGAGAGACCAACTGAAGGCCAGATATAGTACTACCGCATTGCTTGAA 331
                 ************************************************************

121P2A3v.1       CAGCTGGAAGAGACAACGAGAGAAGGAGAAAGGAGGGAGCAGGTGTTGAAAGCCTTATCT 540
121P2A3v.2       CAGCTGGAAGAGACAACGAGAGAAGGAGAAAGGAGGGAGCAGGTGTTGAAAGCCTTATCT 391
                 ************************************************************

121P2A3v.1       GAAGAGAAAGACGTATTGAAACAACAGTTGTCTGCTGCAACCTCACGAATTGCTGAACTT 600
121P2A3v.2       GAAGAGAAAGACGTATTGAAACAACAGTTGTCTGCTGCAACCTCACGAATTGCTGAACTT 451
                 ************************************************************

121P2A3v.1       GAAAGCAAAACCAATACACTCCGTTTATCACAGACTGTGGCTCCAAACTGCTTCAACTCA 660
121P2A3v.2       GAAAGCAAAACCAATACACTCCGTTTATCACAGACTGTGGCTCCAAACTGCTTCAACTCA 511
                 ************************************************************

121P2A3v.1       TCAATAAATAATATTCATGAAATGGAAATACAGCTGAAAGATGCTCTGGAGAAAAATCAG 720
121P2A3v.2       TCAATAAATAATATTCATGAAATGGAAATACAGCTGAAAGATGCTCTGGAGAAAAATCAG 571
                 ************************************************************

121P2A3v.1       CAGTGGCTCGTGTATGATCAGCAGCGGGAAGTCTATGTAAAAGGACTTTTAGCAAAGATC 780
121P2A3v.2       CAGTGGCTCGTGTATGATCAGCAGCGGGAAGTCTATGTAAAAGGACTTTTAGCAAAGATC 631
                 ************************************************************

121P2A3v.1       TTTGAGTTGGAAAAGAAAACGGAAACAGCTGCTCATTCACTCCCACAGCAGACAAAAAAG 840
121P2A3v.2       TTTGAGTTGGAAAAGAAAACGGAAACAGCTGCTCATTCACTCCCACAGCAGACAAAAAAG 691
                 ************************************************************

121P2A3v.1       CCTGAATCAGAAGGTTATCTTCAAGAAGAGAAGCAGAAATGTTACAACGATCTCTTGGCA 900
121P2A3v.2       CCTGAATCAGAAGGTTATCTTCAAGAAGAGAAGCAGAAATGTTACAACGATCTCTTGGCA 751
                 ************************************************************

121P2A3v.1       AGTGCAAAAAAAGATCTTGAGGTTGAACGACAAACCATAACTCAGCTGAGTTTTGAACTG 960
121P2A3v.2       AGTGCAAAAAAAGATCTTGAGGTTGAACGACAAACCATAACTCAGCTGAGTTTTGAACTG 811
                 ************************************************************

121P2A3v.1       AGTGAATTTCGAAGAAAATATGAAGAAACCCAAAAAGAAGTTCACAATTTAAATCAGCTG 1020
121P2A3v.2       AGTGAATTTCGAAGAAAATATGAAGAAACCCAAAAAGAAGTTCACAATTTAAATCAGCTG 871
                 ************************************************************

121P2A3v.1       TTGTATTCACAAAGAAGGGCAGATGTGCAACATCTGGAAGATGATAGGCATAAAACAGAG 1080
121P2A3v.2       TTGTATTCACAAAGAAGGGCAGATGTGCAACATCTGGAAGATGATAGGCATAAAACAGAG 931
                 ************************************************************

121P2A3v.1       AAGATACAAAAACTCAGGGAAGAGAATGATATTGCTAGGGGAAAACTTGAAGAAGAGAAG 1140
121P2A3v.2       AAGATACAAAAACTCAGGGAAGAGAATGATATTGCTAGGGGAAAACTTGAAGAAGAGAAG 991
                 ************************************************************

121P2A3v.1       AAGAGATCCGAAGAGCTCTTATCTCAGGTCCAGTTTCTTTACACATCTCTGCTAAAGCAG 1200
121P2A3v.2       AAGAGATCCGAAGAGCTCTTATCTCAGGTCCAGTTTCTTTACACATCTCTGCTAAAGCAG 1051
                 ************************************************************
```

```
121P2A3v.1      CAAGAAGAACAAACAAGGGTAGCTCTGTTGGAACAACAGATGCAGGCATGTACTTTAGAC 1260
121P2A3v.2      CAAGAAGAACAAACAAGGGTAGCTCTGTTGGAACAACAGATGCAGGCATGTACTTTAGAC 1111
                ************************************************************

121P2A3v.1      TTTGAAAATGAAAAACTCGACCGTCAACATGTGCAGCATCAATTGCATGTAATTCTTAAG 1320
121P2A3v.2      TTTGAAAATGAAAAACTCGACCGTCAACATGTGCAGCATCAATTGCATGTAATTCTTAAG 1171
                ************************************************************

121P2A3v.1      GAGCTCCGAAAAGCAAGAAATCAAATAACACAGTTGGAATCCTTGAAACAGCTTCATGAG 1380
121P2A3v.2      GAGCTCCGAAAAGCAAGAAATCAAATAACACAGTTGGAATCCTTGAAACAGCTTCATGAG 1231
                ************************************************************

121P2A3v.1      TTTGCCATCACAGAGCCATTAGTCACTTTCCAAGGAGAGACTGAAAACAGAGAAAAAGTT 1440
121P2A3v.2      TTTGCCATCACAGAGCCATTAGTCACTTTCCAAGGAGAGACTGAAAACAGAGAAAAAGTT 1291
                ************************************************************

121P2A3v.1      GCCGCCTCACCAAAAAGTCCCACTGCTGCACTCAATGAAAGCCTGGTGGAATGTCCCAAG 1500
121P2A3v.2      GCCGCCTCACCAAAAAGTCCCACTGCTGCACTCAATGAAAGCCTGGTGGAATGTCCCAAG 1351
                ************************************************************

121P2A3v.1      TGCAATATACAGTATCCAGCCACTGAGCATCGCGATCTGCTTGTCCATGTGGAATACTGT 1560
121P2A3v.2      TGCAATATACAGTATCCAGCCACTGAGCATCGCGATCTGCTTGTCCATGTGGAATACTGT 1411
                ************************************************************

121P2A3v.1      TCAAAGTAGCAAAATAAGTATTTGTTTTGATATTAAAAGATTCAATACTGTATTTTCTGT 1620
121P2A3v.2      TCAAAGTAGCAAAATAAGTATTTGTTTTGATATTAAAAGATTCAATACTGTATTTTCTGT 1471
                ************************************************************

121P2A3v.1      TAGCTTGTGGGCATTTTGAATTATATATTTCACATTTTGCATAAAACTGCCTATCTACCT 1680
121P2A3v.2      TAGCTTGTGGGCATTTTGAATTATATATTTCACATTTTGCATAAAACTGCCTATCTACCT 1531
                ************************************************************

121P2A3v.1      TTGACACTCCAGCATGCTAGTGAATCATGTATCTTTTAGGCTGCTGTGCATTTCTCTTGG 1740
121P2A3v.2      TTGACACTCCAGCATGCTAGTGAATCATGTATCTTTTAGGCTGCTGTGCATTTCTCTTGG 1591
                ************************************************************

121P2A3v.1      CAGTGATACCTCCCTGACATGGTTCATCATCAGGCTGCAATGACAGAATGTGGTGAGCAG 1800
121P2A3v.2      CAGTGATACCTCCCTGACATGGTTCATCATCAGGCTGCAATGACAGAATGTGGTGAGCAG 1651
                ************************************************************

121P2A3v.1      CGTCTACTGAGATACTAACATTTTGCACTGTCAAAATACTTGGTGAGGAAAAGATAGCTC 1860
121P2A3v.2      CGTCTACTGAGATACTAACATTTTGCACTGTCAAAATACTTGGTGAGGAAAAGATAGCTC 1711
                ************************************************************

121P2A3v.1      AGGTTATTGCTAATGGGTTAATGCACCAGCAAGCAAAATATTTTATGTTTTGGGGGTTTT 1920
121P2A3v.2      AGGTTATTGCTAATGGGTTAATGCACCAGCAAGCAAAATATTTTATGTTTTGGGGGTTTT 1771
                ************************************************************

121P2A3v.1      GAAAAATCAAAGATAATTAACCAAGGATCTTAACTGTGTTCGCATTTTTTATCCAAGCAC 1980
121P2A3v.2      GAAAAATCAAAGATAATTAACCAAGGATCTTAACTGTGTTCGCATTTTTTATCCAAGCAC 1831
                ************************************************************

121P2A3v.1      TTAGAAAACCTACAATCCTAATTTTGATGTCCATTGTTAAGAGGTGGTGATAGATACTAT 2040
121P2A3v.2      TTAGAAAACCTACAATCCTAATTTTGATGTCCATTGTTAAGAGGTGGTGATAGATACTAT 1891
                ************************************************************

121P2A3v.1      TTTTTTTTTCATATTGTATAGCGGTTATTAGAAAAGTTGGGGATTTTCTTGATCTTTATT 2100
121P2A3v.2      TTTTTTTTTCATATTGTATAGCGGTTATTAGAAAAGTTGGGGATTTTCTTGATCTTTATT 1951
                ************************************************************

121P2A3v.1      GCTGCTTACCATTGAAACTTAACCCAGCTGTGTTCCCCAACTCTGTTCTGCGCACGAAAC 2160
121P2A3v.2      GCTGCTTACCATTGAAACTTAACCCAGCTGTGTTCCCCAACTCTGTTCTGCGCACGAAAC 2011
                ************************************************************

121P2A3v.1      AGTATCTGTTTGAGGCATAATCTTAAGTGGCCACACACAATGTTTTCTCTTATGTTATCT 2220
121P2A3v.2      AGTATCTGTTTGAGGCATAATCTTAAGTGGCCACACACAATGTTTTCTCTTATGTTATCT 2071
                ************************************************************

121P2A3v.1      GGCAGTAACTGTAACTTGAATTACATTAGCACATTCTGCTTAGCTAAAATTGTTAAAATA 2280
121P2A3v.2      GGCAGTAACTGTAACTTGAATTACATTAGCACATTCTGCTTAGCTAAAATTGTTAAAATA 2131
                ************************************************************

121P2A3v.1      AACTTTAATAAACCCATGTAGCCCTCTCATTTGATTGACAGTATTTTAGTTATTTTTGGC 2340
```

```
121P2A3v.2      AACTTTAATAAACCCATGTAGCCCTCTCATTTGATTGACAGTATTTAGTTATTTTTGGC 2191
                ***********************************************************

121P2A3v.1      ATTCTTAAAGCTGGGCAATGTAATGATCAGATCTTTGTTTGTCTGAACAGGTATTTTTAT 2400
121P2A3v.2      ATTCTTAAAGCTGGGCAATGTAATGATCAGATCTTTGTTTGTCTGAACAGGTATTTTTAT 2251
                ************************************************************

121P2A3v.1      ACATGCTTTTTGTAAACCAAAAACTTTTAAATTTCTTCAGGTTTTCTAACATGCTTACCA 2460
121P2A3v.2      ACATGCTTTTTGTAAACCAAAAACTTTTAAATTTCTTCAGGTTTTCTAACATGCTTACCA 2311
                ************************************************************

121P2A3v.1      CTGGGCTACTGTA 2473
121P2A3v.2      CTGGGCTACTGTA 2324
                *************
```

**Table LVI. Peptide sequences of protein coded by 121P2A3 v.2**

```
MEIQLKDALE KNQQWLVYDQ QREVYVKGLL AKIFELEKKT ETAAHSLPQQ TKKPESEGYL    60
QEEKQKCYND LLASAKKDLE VERQTITQLS FELSEFRRKY EETQKEVHNL NQLLYSQRRA   120
DVQHLEDDRH KTEKIQKLRE ENDIARGKLE EEKKRSEELL SQVQFLYTSL LKQQEEQTRV   180
ALLEQQMQAC TLDFENEKLD RQHVQHQLHV ILKELRKARN QITQLESLKQ LHEFAITEPL   240
VTFQGETENR EKVAASPKSP TAALNESLVE CPKCNIQYPA TEHRDLLVHV EYCSK        295
```

**Table LVII. Amino acid sequence alignment of 121P1F1 v.1 and 121P2A3 v.2**

```
121P2A3v.1      MSSRSTKDLIKSKWGSKPSNSKSETTLEKLKGEIAHLKTSVDEITSGKGKLTDKERHRLL 60
121P2A3v.2      ------------------------------------------------------------

121P2A3v.1      EKIRVLEAEKEKNAYQLTEKDKEIQRLRDQLKARYSTTALLEQLEETTREGERREQVLKA 120
121P2A3v.2      ------------------------------------------------------------

121P2A3v.1      LSEEKDVLKQQLSAATSRIAELESKTNTLRLSQTVAPNCFNSSINNIHEMEIQLKDALEK 180
121P2A3v.2      -------------------------------------------------MEIQLKDALEK 11
                                                                 ***********

121P2A3v.1      NQQWLVYDQQREVYVKGLLAKIFELEKKTETAAHSLPQQTKKPESEGYLQEEKQKCYNDL 240
121P2A3v.2      NQQWLVYDQQREVYVKGLLAKIFELEKKTETAAHSLPQQTKKPESEGYLQEEKQKCYNDL 71
                ************************************************************

121P2A3v.1      LASAKKDLEVERQTITQLSFELSEFRRKYEETQKEVHNLNQLLYSQRRADVQHLEDDRHK 300
121P2A3v.2      LASAKKDLEVERQTITQLSFELSEFRRKYEETQKEVHNLNQLLYSQRRADVQHLEDDRHK 131
                ************************************************************

121P2A3v.1      TEKIQKLREENDIARGKLEEEKKRSEELLSQVQFLYTSLLKQQEEQTRVALLEQQMQACT 360
121P2A3v.2      TEKIQKLREENDIARGKLEEEKKRSEELLSQVQFLYTSLLKQQEEQTRVALLEQQMQACT 191
                ************************************************************

121P2A3v.1      LDFENEKLDRQHVQHQLHVILKELRKARNQITQLESLKQLHEFAITEPLVTFQGETENRE 420
121P2A3v.2      LDFENEKLDRQHVQHQLHVILKELRKARNQITQLESLKQLHEFAITEPLVTFQGETENRE 251
                ************************************************************

121P2A3v.1      KVAASPKSPTAALNESLVECPKCNIQYPATEHRDLLVHVEYCSK 464
121P2A3v.2      KVAASPKSPTAALNESLVECPKCNIQYPATEHRDLLVHVEYCSK 295
                ********************************************
```

**Table LVIII Amino acid sequence alignment of 121P1F1 variants (variants 5 and 9 have the same sequence as variant 1)**

```
v.1     MSSRSTKDLIKSKWGSKPSNSKSETTLEKLKGEIAHLKTSVDEITSGKGKLTDKERHRLL
v.2     ------------------------------------------------------------
v.3     MSSRSTKDLIKSKWGSKPSNSKSETTLEKLKGEIAHLKTSVDEITSGKGKLTDKERQRLL
v.4     MSSRSTKDLIKSKWGSKPSNSKSETTLEKLKGEIAHLKTSVDEITSGKGKLTDKERHRLL
v.6     MSSRSTKDLIKSKWGSKPSNSKSETTLEKLKGEIAHLKTSVDEITSGKGKLTDKERHRLL
v.7     MSSRSTKDLIKSKWGSKPSNSKSETTLEKLKGEIAHLKTSVDEITSGKGKLTDKERHRLL
v.8     MSSRSTKDLIKSKWGSKPSNSKSETTLEKLKGEIAHLKTSVDEITSGKGKLTDKERHRLL
```

```
v.1    EKIRVLEAEKEKNAYQLTEKDKEIQRLRDQLKARYSTTALLEQLEETTREGERREQVLKA
v.2    ------------------------------------------------------------
v.3    EKIRVLEAEKEKNAYQLTEKDKEIQRLRDQLKARYSTTALLEQLEETTREGERREQVLKA
v.4    EKIRVLEAEKEKNAYQLTEKDKEIQRLRDQLKARYSTTTLLEQLEETTREGERREQVLKA
v.6    EKIRVLEAEKEKNAYQLTEKDKEIQRLRDQLKARYSTTALLEQLEETTREGERREQVLKA
v.7    EKIRVLEAEKEKNAYQLTEKDKEIQRLRDQLKARYSTTALLEQLEETTREGERREQVLKA
v.8    EKIRVLEAEKEKNAYQLTEKDKEIQRLRDQLKARYSTTALLEQLEETTREGERREQVLKA

v.1    LSEEKDVLKQQLSAATSRIAELESKTNTLRLSQTVAPNCFNSSINNIHEMEIQLKDALEK
v.2    -------------------------------------------------MEIQLKDALEK
v.3    LSEEKDVLKQQLSAATSRIAELESKTNTLRLSQTVAPNCFNSSINNIHEMEIQLKDALEK
v.4    LSEEKDVLKQQLSAATSRIAELESKTNTLRLSQTVAPNCFNSSINNIHEMEIQLKDALEK
v.6    LSEEKDVLKQQLSAATSRIAELESKTNTLRLSQTVAPNCFNSSINNIHEMEIQLKDALEK
v.7    LSEEKDVLKQQLSAATSRIAELESKTNTLRLSQTVAPNCFNSSINNIHEMEIQLKDALEK
v.8    LSEEKDVLKQQLSAATSRIAELESKTNTLRLSQTVAPNCFNSSINNIHEMEIQLKDALEK
                                                        ***********

v.1    NQQWLVYDQQREVYVKGLLAKIFELEKKTETAAHSLPQQTKKPESEGYLQEEKQKCYNDL
v.2    NQQWLVYDQQREVYVKGLLAKIFELEKKTETAAHSLPQQTKKPESEGYLQEEKQKCYNDL
v.3    NQQWLVYDQQREVYVKGLLAKIFELEKKTETAAHSLPQQTKKPESEGYLQEEKQKCYNDL
v.4    NQQWLVYDQQREVYVKGLLAKIFELEKKTETAAHSLPQQTKKPESEGYLQEEKQKCYNDL
v.6    NQQWLVYDQQREVYVKGLLAKIFELEKKTETAAHSLPQQTKKPESEGYLQEEKQKCYNDL
v.7    NQQWLVYDQQREVYVKGLLAKIFELEKKTETAAHSLPQQTKKPESEGYLQEEKQKCYNDL
v.8    NQQWLVYDQQREVYVKGLLAKIFELEKKTETAAHSLPQQTKKPESEGYLQEEKQKCYNDL
       ************************************************************

v.1    LASAKKDLEVERQTITQLSFELSEFRRKYEETQKEVHNLNQLLYSQRRADVQHLEDDRHK
v.2    LASAKKDLEVERQTITQLSFELSEFRRKYEETQKEVHNLNQLLYSQRRADVQHLEDDRHK
v.3    LASAKKDLEVERQTITQLSFELSEFRRKYEETQKEVHNLNQLLYSQRRADVQHLEDDRHK
v.4    LASAKKDLEVERQTITQLSFELSEFRRKYEETQKEVHNLNQLLYSQRRADVQHLEDDRHK
v.6    LASAKKDLEVERQTITQLSFELSEFRRKYEETQKEVHNLNQLLYSQRRADVQHLEDDRHK
v.7    LASAKKDLEVERQTITQLSFELSEFRRKYEETQKEVHNLNQLLYSQRRADVQHLEDDRHK
v.8    LASAKKDLEVERQTITQLSFELSEFRRKYEETQKEVHNLNQLLYSQRRADVQHLEDDRHK
       ************************************************************

v.1    TEKIQKLREENDIARGKLEEEKKRSEELLSQVQFLYTSLLKQQEEQTRVALLEQQMQACT
v.2    TEKIQKLREENDIARGKLEEEKKRSEELLSQVQFLYTSLLKQQEEQTRVALLEQQMQACT
v.3    TEKIQKLREENDIARGKLEEEKKRSEELLSQVQFLYTSLLKQQEEQTRVALLEQQMQACT
v.4    TEKIQKLREENDIARGKLEEEKKRSEELLSQVQFLYTSLLKQQEEQTRVALLEQQMQACT
v.6    TEKIQKLREENDIARGKLEEEKKRSEELLSQVQSLYTSLLKQQEEQTRVALLEQQMQACT
v.7    TEKIQKLREENDIARGKLEEEKKRSEELLSQVQFLYTSLLKQQEEQTRVALLEQQMQACT
v.8    TEKIQKLREENDIARGKLEEEKKRSEELLSQVQFLYTSLLKQQEEQTRVALLEQQMQACT
       ********************************* **************************

v.1    LDFENEKLDRQHVQHQLHVILKELRKARNQITQLESLKQLHEFAITEPLVTFQGETENRE
v.2    LDFENEKLDRQHVQHQLHVILKELRKARNQITQLESLKQLHEFAITEPLVTFQGETENRE
v.3    LDFENEKLDRQHVQHQLHVILKELRKARNQITQLESLKQLHEFAITEPLVTFQGETENRE
v.4    LDFENEKLDRQHVQHQLHVILKELRKARNQITQLESLKQLHEFAITEPLVTFQGETENRE
v.6    LDFENEKLDRQHVQHQLHVILKELRKARNQITQLESLKQLHEFAITEPLVTFQGETENRE
v.7.   LDFENEKLDRQHVQHQLLVILKELRKARNQITQLESLKQLHEFAITEPLVTFQGETENRE
v.8    LDFENEKLDRQHVQHQLHVILKELRKARNQITQLESLKQLHEFAITEPLVTFQGETENRE
       ***************** ******************************************

v.1    KVAASPKSPTAALNESLVECPKCNIQYPATEHRDLLVHVEYCSK
v.2    KVAASPKSPTAALNESLVECPKCNIQYPATEHRDLLVHVEYCSK
v.3    KVAASPKSPTAALNESLVECPKCNIQYPATEHRDLLVHVEYCSK
v.4    KVAASPKSPTAALNESLVECPKCNIQYPATEHRDLLVHVEYCSK
v.6    KVAASPKSPTAALNESLVECPKCNIQYPATEHRDLLVHVEYCSK
v.7    KVAASPKSPTAALNESLVECPKCNIQYPATEHRDLLVHVEYCSK
v.8    KVAASPKSPTAALNGSLVECPKCNIQYPATEHRDLLVHVEYCSK
       ************** *****************************
```

## Claims

**1.** Use of:

(a) a protein having an identical amino acid sequence as that shown in Figure 3A, 3B, 3C, 3D, 3E, 3F or 3G;
(b) a fragment, variant, homolog or analog of a protein of (a); or
(c) a nucleotide sequence that encodes a protein of (a); for the preparation of a vaccine for the prevention and/or treatment of cancers that express a protein having an identical amino acid sequence as that shown in Figure 3A, 3B, 3C, 3D, 3E, 3F or 3G.

**2.** Use according to claim 1, wherein the variant has 90% or more homology to the protein shown in Figure 3A, 3B, 3C, 3D, 3E, 3F or 3G.

**3.** Use according to claim 1 or 2, wherein the fragment comprises 4 or more contiguous amino acids of the protein shown in Figure 3A, 3B, 3C, 3D, 3E, 3F or 3G.

**4.** Use according to any one of the preceding claims, wherein the protein, fragment, variant, homolog or analog comprises a CTL epitope shown in any one of Tables V to XVIII and XXII-LI or a peptide of 8, 9, 10 or 11 amino acids specified by an HLA Class I motif/supermotif and/or a peptide of at least 9 amino acids that comprises an HLA Class II motif/supermotif.

**5.** Use according to any one of the preceding claims, wherein the cancer is prostate cancer or colon cancer.

**6.** A recombinant expression vector comprising a nucleotide sequence that encodes a protein shown in Figure 3A, 3B, 3C, 3D, 3E, 3F or 3G.

**7.** A recombinant expression vector according to claim 6, wherein the recombinant expression vector is a viral vector.

**8.** A recombinant expression vector according to claim 7, wherein the viral vector is selected from the group consisting of vaccinia, fowlpox, canarypox, adenovirus, influenza, poliovirus, adeno-associated virus, lentivirus, and sindbis virus.

**9.** Use of a recombinant expression vector according to any one of claims 6 to 8 for the preparation of a medicament for generating a mammalian immune response directed to a protein of Figure 3A, 3B, 3C, 3D, 3E, 3F or 3G.

**10.** Use according to claim 9, wherein the immune response comprises activation of B-cells which produce antibodies that specifically bind to the protein shown in Figure 3A, 3B, 3C, 3D, 3E, 3F or 3G.

**<u>Figure 1</u> 121P2A3 SSH sequence of 259 nucleotides.**

```
  1 GATCATTACA TTGCCCAGCT TTAAGAATGC CAAAAATAAC TAAAATACTG TCAATCAAAT
 61 GAGAGGGCTA CATGGGTTTA TTAAAGTTTA TTTTAACAAT TTTAGCTAAG CAGAATGTGC
121 TAATGTAATT CAAGTTACAG TTACTGCCAG ATAACATAAG AGAAAACATT GTGTGTGGCC
181 ACTTAAGATT ATGCCTCAAA CAGATACTGT TTCGTGCGCA GAACAGAGTT GGGGAACACA
241 GCTGGGGATT TTCTTGATC
```

<u>Figure 2A.</u> **The cDNA (SEQ ID. NO. :____) and amino acid sequence (SEQ ID. NO. :____) of 121P2A3 v.1 clone 5.** The start methionine is underlined. The open reading frame extends from nucleic acid 175-1569 including the stop codon.

```
  1 gggaccgccagggagggcaggtcagtggcagatcgcgtccgcgggattcaatctctgcc
 61 cgctctgataacagtcctttccctggcgctcacttcgtgcctggcacccggctgggcgc
  1                                                      M  S
121 ctcaagaccgttgtctcttcgatcgcttctttggacttggcgaccatttcagagATGTCT
  3 S  R  S  T  K  D  L  I  K  S  K  W  G  S  K  P  S  N  S  K
181 TCCAGAAGTACCAAAGATTTAATTAAAAGTAAGTGGGGATCGAAGCCTAGTAACTCCAAA
 23 S  E  T  T  L  E  K  L  K  G  E  I  A  H  L  K  T  S  V  D
241 TCCGAAACTACATTAGAAAAATTAAAGGGAGAAATTGCACACTTAAAGACATCAGTGGAT
 43 E  I  T  S  G  K  G  K  L  T  D  K  E  R  H  R  L  L  E  K
301 GAAATCACAAGTGGGAAAGGAAAGCTGACTGATAAAGAGAGACACAGACTTTTGGAGAAA
 63 I  R  V  L  E  A  E  K  E  K  N  A  Y  Q  L  T  E  K  D  K
361 ATTCGAGTCCTTGAGGCTGAGAAGGAGAAGAATGCTTATCAACTCACAGAGAAGGACAAA
 83 E  I  Q  R  L  R  D  Q  L  K  A  R  Y  S  T  T  A  L  L  E
421 GAAATACAGCGACTGAGAGACCAACTGAAGGCCAGATATAGTACTACCGCATTGCTTGAA
103 Q  L  E  E  T  T  R  E  G  E  R  R  E  Q  V  L  K  A  L  S
481 CAGCTGGAAGAGACAACGAGAGAAGGAGAAAGGAGGGAGCAGGTGTTGAAAGCCTTATCT
123 E  E  K  D  V  L  K  Q  Q  L  S  A  A  T  S  R  I  A  E  L
541 GAAGAGAAAGACGTATTGAAACAACAGTTGTCTGCTGCAACCTCACGAATTGCTGAACTT
143 E  S  K  T  N  T  L  R  L  S  Q  T  V  A  P  N  C  F  N  S
601 GAAAGCAAAACCAATACACTCCGTTTATCACAGACTGTGGCTCCAAACTGCTTCAACTCA
163 S  I  N  N  I  H  E  M  E  I  Q  L  K  D  A  L  E  K  N  Q
661 TCAATAAATAATATTCATGAAATGGAAATACAGCTGAAAGATGCTCTGGAGAAAAATCAG
183 Q  W  L  V  Y  D  Q  Q  R  E  V  Y  V  K  G  L  L  A  K  I
721 CAGTGGCTCGTGTATGATCAGCAGCGGGAAGTCTATGTAAAAGGACTTTTAGCAAAGATC
203 F  E  L  E  K  K  T  E  T  A  A  H  S  L  P  Q  Q  T  K  K
781 TTTGAGTTGGAAAAGAAAACGGAAACAGCTGCTCATTCACTCCCACAGCAGACAAAAAAG
```

223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacattttgcactgtcaaaatacttggtgaggaaaagatagctc
1861 aggttattgctaatgggttaatgcaccagcaagcaaaatattttatgttttggggtttt

1921 gaaaaatcaaagataattaaccaaggatcttaactgtgttcgcatttttatccaagcac
1981 ttagaaaacctacaatcctaatttgatgtccattgttaagaggtggtgatagatactat
2041 ttttttttcatattgtatagcggttattagaaaagttggggattttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat
2401 acatgctttttgtaaaccaaaaactttttaaatttcttcaggttttctaacatgcttacca
2461 ctgggctactgta

**Figure 2B.** **The cDNA (SEQ ID. NO. :____) and amino acid sequence (SEQ ID. NO. :____) of 121P2A3 v.2.** The start methionine is underlined. The open reading frame extends from nucleic acid 533-1420 including the stop codon.

1 gggaccgccagggagggcaggtcagtgggcagatcgcgtccgcgggattcaatctctgcc
61 cgctctgataacagtccttttccctggcgctcacttcgtgcctggcacccggctgggcgc
121 ctcaagaccgttgtctcttcgatcgcttctttggacttggcgaccatttcagagatgtct
181 tccagaagtaccaaagatttaattaaaaaaaattcgagtccttgaggctgagaaggagaa
241 gaatgcttatcaactcacagagaaggacaaagaaatacagcgactgagagaccaactgaa
301 ggccagatatagtactaccgcattgcttgaacagctggaagagacaacgagagaaggaga
361 aaggagggagcaggtgttgaaagccttatctgaagagaaagacgtattgaaacaacagtt
421 gtctgctgcaacctcacgaattgctgaacttgaaagcaaaaccaatacactccgtttatc
1 M E I
481 acagactgtggctccaaactgcttcaactcatcaataaataatattcatgaaATGGAAAT
4 Q L K D A L E K N Q Q W L V Y D Q Q R E
541 ACAGCTGAAAGATGCTCTGGAGAAAAATCAGCAGTGGCTCGTGTATGATCAGCAGCGGGA
24 V Y V K G L L A K I F E L E K K T E T A
601 AGTCTATGTAAAAGGACTTTTAGCAAAGATCTTTGAGTTGGAAAAGAAAACGGAAACAGC
44 A H S L P Q Q T K K P E S E G Y L Q E E

661 TGCTCATTCACTCCCACAGCAGACAAAAAAGCCTGAATCAGAAGGTTATCTTCAAGAAGA
64 K Q K C Y N D L L A S A K K D L E V E R
721 GAAGCAGAAATGTTACAACGATCTCTTGGCAAGTGCAAAAAAAGATCTTGAGGTTGAACG
84 Q T I T Q L S F E L S E F R R K Y E E T
781 ACAAACCATAACTCAGCTGAGTTTTGAACTGAGTGAATTTCGAAGAAAATATGAAGAAAC
104 Q K E V H N L N Q L L Y S Q R R A D V Q
841 CCAAAAAGAAGTTCACAATTTAAATCAGCTGTTGTATTCACAAAGAAGGGCAGATGTGCA
124 H L E D D R H K T E K I Q K L R E E N D
901 ACATCTGGAAGATGATAGGCATAAAACAGAGAAGATACAAAAACTCAGGGAAGAGAATGA
144 I A R G K L E E E K K R S E E L L S Q V
961 TATTGCTAGGGGAAAACTTGAAGAAGAGAAGAAGAGATCCGAAGAGCTCTTATCTCAGGT
164 Q F L Y T S L L K Q Q E E Q T R V A L L
1021 CCAGTTTCTTTACACATCTCTGCTAAAGCAGCAAGAAGAACAAACAAGGGTAGCTCTGTT
184 E Q Q M Q A C T L D F E N E K L D R Q H
1081 GGAACAACAGATGCAGGCATGTACTTTAGACTTTGAAAATGAAAAACTCGACCGTCAACA
204 V Q H Q L H V I L K E L R K A R N Q I T
1141 TGTGCAGCATCAATTGCATGTAATTCTTAAGGAGCTCCGAAAAGCAAGAAATCAAATAAC
224 Q L E S L K Q L H E F A I T E P L V T F
1201 ACAGTTGGAATCCTTGAAACAGCTTCATGAGTTTGCCATCACAGAGCCATTAGTCACTTT
244 Q G E T E N R E K V A A S P K S P T A A
1261 CCAAGGAGAGACTGAAAACAGAGAAAAAGTTGCCGCCTCACCAAAAAGTCCCACTGCTGC
264 L N E S L V E C P K C N I Q Y P A T E H
1321 ACTCAATGAAAGCCTGGTGGAATGTCCCAAGTGCAATATACAGTATCCAGCCACTGAGCA
284 R D L L V H V E Y C S K *
1381 TCGCGATCTGCTTGTCCATGTGGAATACTGTTCAAAGTAGcaaaataagtatttgttttg
1441 atattaaaagattcaatactgtattttctgttagcttgtgggcattttgaattatatatt
1501 tcacattttgcataaaactgcctatctacctttgacactccagcatgctagtgaatcatg
1561 tatcttttaggctgctgtgcatttctcttggcagtgatacctccctgacatggttcatca
1621 tcaggctgcaatgacagaatgtggtgagcagcgtctactgagatactaacattttgcact
1681 gtcaaaatacttggtgaggaaaagatagctcaggttattgctaatgggttaatgcaccag
1741 caagcaaaatattttatgttttgggggttttgaaaaatcaaagataattaaccaaggatc

1801 ttaactgtgttcgcatttttatccaagcacttagaaaacctacaatcctaattttgatg
1861 tccattgttaagaggtggtgatagatactattttttttttcatattgtatagcggttatt
1921 agaaaagttggggattttcttgatctttattgctgcttaccattgaaacttaacccagct
1981 gtgttccccaactctgttctgcgcacgaaacagtatctgtttgaggcataatcttaagtg
2041 gccacacacaatgttttctcttatgttatctggcagtaactgtaacttgaattacattag
2101 cacattctgcttagctaaaattgttaaaataaactttaataaacccatgtagccctctca
2161 tttgattgacagtattttagttatttttggcattcttaaagctgggcaatgtaatgatca
2221 gatctttgtttgtctgaacaggtatttttatacatgctttttgtaaaccaaaaactttta
2281 aatttcttcaggttttctaacatgcttaccactgggctactgta

<u>Figure 2C.</u> **The cDNA (SEQ ID. NO. :____) and amino acid sequence (SEQ ID. NO. :____) of 121P2A3 v.3.** The start methionine is underlined. The open reading frame extends from nucleic acid 175-1569 including the stop codon.

```
  1 gggaccgccagggagggcaggtcagtgggcagatcgcgtccgcgggattcaatctctgcc
 61 cgctctgataacagtccttttccctggcgctcacttcgtgcctggcacccggctgggcgc
  1                                                       M  S
121 ctcaagaccgttgtctcttcgatcgcttctttggacttggcgaccatttcagagATGTCT
  3 S  R  S  T  K  D  L  I  K  S  K  W  G  S  K  P  S  N  S  K
181 TCCAGAAGTACCAAAGATTTAATTAAAAGTAAGTGGGGATCGAAGCCTAGTAACTCCAAA
 23 S  E  T  T  L  E  K  L  K  G  E  I  A  H  L  K  T  S  V  D
241 TCCGAAACTACATTAGAAAAATTAAAGGGAGAAATTGCACACTTAAAGACATCAGTGGAT
 43 E  I  T  S  G  K  G  K  L  T  D  K  E  R  Q  R  L  L  E  K
301 GAAATCACAAGTGGGAAAGGAAAGCTGACTGATAAAGAGAGACAGAGACTTTTGGAGAAA
 63 I  R  V  L  E  A  E  K  E  K  N  A  Y  Q  L  T  E  K  D  K
361 ATTCGAGTCCTTGAGGCTGAGAAGGAGAAGAATGCTTATCAACTCACAGAGAAGGACAAA
 83 E  I  Q  R  L  R  D  Q  L  K  A  R  Y  S  T  T  A  L  L  E
421 GAAATACAGCGACTGAGAGACCAACTGAAGGCCAGATATAGTACTACCGCATTGCTTGAA
103 Q  L  E  E  T  T  R  E  G  E  R  R  E  Q  V  L  K  A  L  S
481 CAGCTGGAAGAGACAACGAGAGAAGGAGAAAGGAGGGAGCAGGTGTTGAAAGCCTTATCT
123 E  E  K  D  V  L  K  Q  Q  L  S  A  A  T  S  R  I  A  E  L
541 GAAGAGAAAGACGTATTGAAACAACAGTTGTCTGCTGCAACCTCACGAATTGCTGAACTT
143 E  S  K  T  N  T  L  R  L  S  Q  T  V  A  P  N  C  F  N  S
601 GAAAGCAAAACCAATACACTCCGTTTATCACAGACTGTGGCTCCAAACTGCTTCAACTCA
163 S  I  N  N  I  H  E  M  E  I  Q  L  K  D  A  L  E  K  N  Q
661 TCAATAAATAATATTCATGAAATGGAAATACAGCTGAAAGATGCTCTGGAGAAAAATCAG
183 Q  W  L  V  Y  D  Q  Q  R  E  V  Y  V  K  G  L  L  A  K  I
721 CAGTGGCTCGTGTATGATCAGCAGCGGGAAGTCTATGTAAAAGGACTTTTAGCAAAGATC
203 F  E  L  E  K  K  T  E  T  A  A  H  S  L  P  Q  Q  T  K  K
```

781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacattttgcactgtcaaaatacttggtgaggaaaagatagctc

1861 aggttattgctaatgggttaatgcaccagcaagcaaaatatttatgttttgggggtttt
1921 gaaaaatcaaagataattaaccaaggatcttaactgtgttcgcatttttatccaagcac
1981 ttagaaaacctacaatcctaattttgatgtccattgttaagaggtggtgatagatactat
2041 tttttttttcatattgtatagcggttattagaaaagttggggattttcttgatctttatt
2101 gctgcttaccattgaaacttaacccagctgtgttccccaactctgttctgcgcacgaaac
2161 agtatctgtttgaggcataatcttaagtggccacacacaatgtttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat
2401 acatgcttttgtaaaccaaaaactttaaatttcttcaggttttctaacatgcttacca
2461 ctgggctactgta

**Figure 2D. The cDNA (SEQ ID. NO. :____) and amino acid sequence (SEQ ID. NO. :____) of 121P2A3 v.4.** The start methionine is underlined. The open reading frame extends from nucleic acid 175-1569 including the stop codon.

1 gggaccgccagggagggcaggtcagtgggcagatcgcgtccgcgggattcaatctctgcc
61 cgctctgataacagtccttttccctggcgctcacttcgtgcctggcacccggctgggcgc
1 M S
121 ctcaagaccgttgtctcttcgatcgcttctttggacttggcgaccatttcagag<u>ATG</u>TCT
3 S R S T K D L I K S K W G S K P S N S K
181 TCCAGAAGTACCAAAGATTTAATTAAAAGTAAGTGGGGATCGAAGCCTAGTAACTCCAAA
23 S E T T L E K L K G E I A H L K T S V D
241 TCCGAAACTACATTAGAAAAATTAAAGGGAGAAATTGCACACTTAAAGACATCAGTGGAT
43 E I T S G K G K L T D K E R H R L L E K
301 GAAATCACAAGTGGGAAAGGAAAGCTGACTGATAAAGAGAGACACAGACTTTTGGAGAAA
63 I R V L E A E K E K N A Y Q L T E K D K
361 ATTCGAGTCCTTGAGGCTGAGAAGGAGAAGAATGCTTATCAACTCACAGAGAAGGACAAA
83 E I Q R L R D Q L K A R Y S T T T L L E
421 GAAATACAGCGACTGAGAGACCAACTGAAGGCCAGATATAGTACTACCACATTGCTTGAA

103 Q L E E T T R E G E R R E Q V L K A L S
481 CAGCTGGAAGAGACAACGAGAGAAGGAGAAAGGAGGGAGCAGGTGTTGAAAGCCTTATCT
123 E E K D V L K Q Q L S A A T S R I A E L
541 GAAGAGAAAGACGTATTGAAACAACAGTTGTCTGCTGCAACCTCACGAATTGCTGAACTT
143 E S K T N T L R L S Q T V A P N C F N S
601 GAAAGCAAAACCAATACACTCCGTTTATCACAGACTGTGGCTCCAAACTGCTTCAACTCA
163 S I N N I H E M E I Q L K D A L E K N Q
661 TCAATAAATAATATTCATGAAATGGAAATACAGCTGAAAGATGCTCTGGAGAAAAATCAG
183 Q W L V Y D Q Q R E V Y V K G L L A K I
721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V

1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacatttgcactgtcaaaatacttggtgaggaaaagatagctc
1861 aggttattgctaatgggttaatgcaccagcaagcaaaatattttatgttttgggggtttt
1921 gaaaaatcaaagataattaaccaaggatcttaactgtgttcgcatttttttatccaagcac
1981 ttagaaaacctacaatcctaatttgatgtccattgttaagaggtggtgatagatactat
2041 tttttttttcatattgtatagcggttattagaaaagttggggatttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat
2401 acatgcttttttgtaaaccaaaaacttttaaatttcttcaggtttctaacatgcttacca
2461 ctgggctactgta

**<u>Figure 2E.</u> The cDNA (SEQ ID. NO. :____) and amino acid sequence (SEQ ID. NO. :____) of 121P2A3 v.5.** The start methionine is underlined. The open reading frame extends from nucleic acid 175-1569 including the stop codon.

1 gggaccgccagggagggcaggtcagtgggcagatcgcgtccgcgggattcaatctctgcc
61 cgctctgataacagtccttttccctggcgctcacttcgtgcctggcacccggctgggcgc
1 M S

121 ctcaagaccgttgtctcttcgatcgcttctttggacttggcgaccatttcagagATGTCT
3 S R S T K D L I K S K W G S K P S N S K
181 TCCAGAAGTACCAAAGATTTAATTAAAAGTAAGTGGGGATCGAAGCCTAGTAACTCCAAA
23 S E T T L E K L K G E I A H L K T S V D
241 TCCGAAACTACATTAGAAAAATTAAAGGGAGAAATTGCACACTTAAAGACATCAGTGGAT
43 E I T S G K G K L T D K E R H R L L E K
301 GAAATCACAAGTGGGAAAGGAAAGCTGACTGATAAAGAGAGACACAGACTTTTGGAGAAA
63 I R V L E A E K E K N A Y Q L T E K D K
361 ATTCGAGTCCTTGAGGCTGAGAAGGAGAAGAATGCTTATCAACTCACAGAGAAGGACAAA
83 E I Q R L R D Q L K A R Y S T T A L L E
421 GAAATACAGCGACTGAGAGACCAACTGAAGGCCAGATATAGTACTACCGCATTGCTTGAA
103 Q L E E T T R E G E R R E Q V L K A L S
481 CAGCTGGAAGAGACAACGAGAGAAGGAGAACGGAGGGAGCAGGTGTTGAAAGCCTTATCT
123 E E K D V L K Q Q L S A A T S R I A E L
541 GAAGAGAAAGACGTATTGAAACAACAGTTGTCTGCTGCAACCTCACGAATTGCTGAACTT
143 E S K T N T L R L S Q T V A P N C F N S
601 GAAAGCAAAACCAATACACTCCGTTTATCACAGACTGTGGCTCCAAACTGCTTCAACTCA
163 S I N N I H E M E I Q L K D A L E K N Q
661 TCAATAAATAATATTCATGAAATGGAAATACAGCTGAAAGATGCTCTGGAGAAAAATCAG
183 Q W L V Y D Q Q R E V Y V K G L L A K I
721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG

303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacattttgcactgtcaaaatacttggtgaggaaaagatagctc
1861 aggttattgctaatgggttaatgcaccagcaagcaaaatattttatgtttgggggtttt
1921 gaaaaatcaaagataattaaccaaggatcttaactgtgttcgcatttttttatccaagcac
1981 ttagaaaacctacaatcctaattttgatgtccattgttaagaggtggtgatagatactat
2041 tttttttttcatattgtatagcggttattagaaaagttggggattttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat

2401 acatgcttttgtaaaccaaaactttaaatttcttcaggttttctaacatgcttacca
2461 ctgggctactgta

**Figure 2F.** **The cDNA (SEQ ID. NO. :____) and amino acid sequence (SEQ ID. NO. :____) of 121P2A3 v.6.** The start methionine is underlined. The open reading frame extends from nucleic acid 175-1569 including the stop codon.

```
  1 gggaccgccagggagggcaggtcagtgggcagatcgcgtccgcgggattcaatctctgcc
 61 cgctctgataacagtccttttccctggcgctcacttcgtgcctggcacccggctgggcgc
  1                                                     M  S
121 ctcaagaccgttgtctcttcgatcgcttctttggacttggcgaccatttcagagATGTCT
  3 S  R  S  T  K  D  L  I  K  S  K  W  G  S  K  P  S  N  S  K
181 TCCAGAAGTACCAAAGATTTAATTAAAAGTAAGTGGGGATCGAAGCCTAGTAACTCCAAA
 23 S  E  T  T  L  E  K  L  K  G  E  I  A  H  L  K  T  S  V  D
241 TCCGAAACTACATTAGAAAAATTAAAGGGAGAAATTGCACACTTAAAGACATCAGTGGAT
 43 E  I  T  S  G  K  G  K  L  T  D  K  E  R  H  R  L  L  E  K
301 GAAATCACAAGTGGGAAAGGAAAGCTGACTGATAAAGAGAGACACAGACTTTTGGAGAAA
 63 I  R  V  L  E  A  E  K  E  K  N  A  Y  Q  L  T  E  K  D  K
361 ATTCGAGTCCTTGAGGCTGAGAAGGAGAAGAATGCTTATCAACTCACAGAGAAGGACAAA
 83 E  I  Q  R  L  R  D  Q  L  K  A  R  Y  S  T  T  A  L  L  E
421 GAAATACAGCGACTGAGAGACCAACTGAAGGCCAGATATAGTACTACCGCATTGCTTGAA
103 Q  L  E  E  T  T  R  E  G  E  R  R  E  Q  V  L  K  A  L  S
481 CAGCTGGAAGAGACAACGAGAGAAGGAGAAAGGAGGGAGCAGGTGTTGAAAGCCTTATCT
123 E  E  K  D  V  L  K  Q  Q  L  S  A  A  T  S  R  I  A  E  L
541 GAAGAGAAAGACGTATTGAAACAACAGTTGTCTGCTGCAACCTCACGAATTGCTGAACTT
143 E  S  K  T  N  T  L  R  L  S  Q  T  V  A  P  N  C  F  N  S
601 GAAAGCAAAACCAATACACTCCGTTTATCACAGACTGTGGCTCCAAACTGCTTCAACTCA
163 S  I  N  N  I  H  E  M  E  I  Q  L  K  D  A  L  E  K  N  Q
661 TCAATAAATAATATTCATGAAATGGAAATACAGCTGAAAGATGCTCTGGAGAAAAATCAG
183 Q  W  L  V  Y  D  Q  Q  R  E  V  Y  V  K  G  L  L  A  K  I
```

721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q S L Y T S L L K Q
1141 AAGAGATCCGAAGAGCTCTTATCTCAGGTCCAGTCTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg

1741 cagtgatacctccctgacatggttcatcatcaggctgcaatgacagaatgtggtgagcag
1801 cgtctactgagatactaacattttgcactgtcaaaatacttggtgaggaaaagatagctc
1861 aggttattgctaatgggttaatgcaccagcaagcaaaatattttatgttttggggtttt
1921 gaaaaatcaaagataattaaccaaggatcttaactgtgttcgcatttttatccaagcac
1981 ttagaaaacctacaatcctaattttgatgtccattgttaagaggtggtgatagatactat
2041 tttttttttcatattgtatagcggttattagaaaagttggggatttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtatttagttatttttggc
2341 attcttaaagctgggcaatgtaatgatcagatctttgtttgtctgaacaggtatttttat
2401 acatgcttttgtaaaccaaaaacttttaaatttcttcaggttttctaacatgcttacca
2461 ctgggctactgta

**Figure 2G. The cDNA (SEQ ID. NO. :____) and amino acid sequence (SEQ ID. NO. :____) of 121P2A3 v.7.** The start methionine is underlined. The open reading frame extends from nucleic acid 175-1569 including the stop codon.

1 gggaccgccagggagggcaggtcagtgggcagatcgcgtccgcgggattcaatctctgcc
61 cgctctgataacagtccttttccctggcgctcacttcgtgcctggcacccggctgggcgc
1 M S
121 ctcaagaccgttgtctcttcgatcgcttctttggacttggcgaccatttcagag<u>ATG</u>TCT
3 S R S T K D L I K S K W G S K P S N S K
181 TCCAGAAGTACCAAAGATTTAATTAAAAGTAAGTGGGGATCGAAGCCTAGTAACTCCAAA
23 S E T T L E K L K G E I A H L K T S V D
241 TCCGAAACTACATTAGAAAAATTAAAGGGAGAAATTGCACACTTAAAGACATCAGTGGAT
43 E I T S G K G K L T D K E R H R L L E K
301 GAAATCACAAGTGGGAAAGGAAAGCTGACTGATAAAGAGAGACACAGACTTTTGGAGAAA
63 I R V L E A E K E K N A Y Q L T E K D K
361 ATTCGAGTCCTTGAGGCTGAGAAGGAGAAGAATGCTTATCAACTCACAGAGAAGGACAAA

83 E I Q R L R D Q L K A R Y S T T A L L E
421 GAAATACAGCGACTGAGAGACCAACTGAAGGCCAGATATAGTACTACCGCATTGCTTGAA
103 Q L E E T T R E G E R R E Q V L K A L S
481 CAGCTGGAAGAGACAACGAGAGAAGGAGAAAGGAGGGAGCAGGTGTTGAAAGCCTTATCT
123 E E K D V L K Q Q L S A A T S R I A E L
541 GAAGAGAAAGACGTATTGAAACAACAGTTGTCTGCTGCAACCTCACGAATTGCTGAACTT
143 E S K T N T L R L S Q T V A P N C F N S
601 GAAAGCAAAACCAATACACTCCGTTTATCACAGACTGTGGCTCCAAACTGCTTCAACTCA
163 S I N N I H E M E I Q L K D A L E K N Q
661 TCAATAAATAATATTCATGAAATGGAAATACAGCTGAAAGATGCTCTGGAGAAAAATCAG
183 Q W L V Y D Q Q R E V Y V K G L L A K I
721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L L V I L K
1261 TTTGAAAATGAAAAACTCGACCGTCAACATGTGCAGCATCAATTGCTTGTAATTCTTAAG
383 E L R K A R N Q I T Q L E S L K Q L H E

1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacatttgcactgtcaaaatacttggtgaggaaaagatagctc
1861 aggttattgctaatgggttaatgcaccagcaagcaaaatattttatgttttggggtttt
1921 gaaaaatcaaagataattaaccaaggatcttaactgtgttcgcatttttatccaagcac
1981 ttagaaaacctacaatcctaattttgatgtccattgttaagaggtggtgatagatactat
2041 tttttttttcatattgtatagcggttattagaaaagttggggattttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat
2401 acatgctttttgtaaaccaaaaactttttaaatttcttcaggttttctaacatgcttacca
2461 ctgggctactgta

__Figure 2H.__ **The cDNA (SEQ ID. NO. :____) and amino acid sequence (SEQ ID. NO. :____) of 121P2A3 v.8.** The start methionine is underlined. The open reading frame extends from nucleic acid 175-1569 including the stop codon.

1 gggaccgccagggagggcaggtcagtgggcagatcgcgtccgcgggattcaatctctgcc

61 cgctctgataacagtccttttccctggcgctcacttcgtgcctggcacccggctgggcgc
1 M S
121 ctcaagaccgttgtctcttcgatcgcttctttggacttggcgaccatttcagagATGTCT
3 S R S T K D L I K S K W G S K P S N S K
181 TCCAGAAGTACCAAAGATTTAATTAAAAGTAAGTGGGGATCGAAGCCTAGTAACTCCAAA
23 S E T T L E K L K G E I A H L K T S V D
241 TCCGAAACTACATTAGAAAAATTAAAGGGAGAAATTGCACACTTAAAGACATCAGTGGAT
43 E I T S G K G K L T D K E R H R L L E K
301 GAAATCACAAGTGGGAAAGGAAAGCTGACTGATAAAGAGAGACACAGACTTTTGGAGAAA
63 I R V L E A E K E K N A Y Q L T E K D K
361 ATTCGAGTCCTTGAGGCTGAGAAGGAGAAGAATGCTTATCAACTCACAGAGAAGGACAAA
83 E I Q R L R D Q L K A R Y S T T A L L E
421 GAAATACAGCGACTGAGAGACCAACTGAAGGCCAGATATAGTACTACCGCATTGCTTGAA
103 Q L E E T T R E G E R R E Q V L K A L S
481 CAGCTGGAAGAGACAACGAGAGAAGGAGAAAGGAGGGAGCAGGTGTTGAAAGCCTTATCT
123 E E K D V L K Q Q L S A A T S R I A E L
541 GAAGAGAAAGACGTATTGAAACAACAGTTGTCTGCTGCAACCTCACGAATTGCTGAACTT
143 E S K T N T L R L S Q T V A P N C F N S
601 GAAAGCAAAACCAATACACTCCGTTTATCACAGACTGTGGCTCCAAACTGCTTCAACTCA
163 S I N N I H E M E I Q L K D A L E K N Q
661 TCAATAAATAATATTCATGAAATGGAAATACAGCTGAAAGATGCTCTGGAGAAAAATCAG
183 Q W L V Y D Q Q R E V Y V K G L L A K I
721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG

```
283 L  Y  S  Q  R  R  A  D  V  Q  H  L  E  D  D  R  H  K  T  E
1021 TTGTATTCACAAAGAAGGGCAGATGTGCAACATCTGGAAGATGATAGGCATAAAACAGAG
303 K  I  Q  K  L  R  E  E  N  D  I  A  R  G  K  L  E  E  E  K
1081 AAGATACAAAAACTCAGGGAAGAGAATGATATTGCTAGGGGAAAACTTGAAGAAGAGAAG
323 K  R  S  E  E  L  L  S  Q  V  Q  F  L  Y  T  S  L  L  K  Q
1141 AAGAGATCCGAAGAGCTCTTATCTCAGGTCCAGTTTCTTTACACATCTCTGCTAAAGCAG
343 Q  E  E  Q  T  R  V  A  L  L  E  Q  Q  M  Q  A  C  T  L  D
1201 CAAGAAGAACAAACAAGGGTAGCTCTGTTGGAACAACAGATGCAGGCATGTACTTTAGAC
363 F  E  N  E  K  L  D  R  Q  H  V  Q  H  Q  L  H  V  I  L  K
1261 TTTGAAAATGAAAAACTCGACCGTCAACATGTGCAGCATCAATTGCATGTAATTCTTAAG
383 E  L  R  K  A  R  N  Q  I  T  Q  L  E  S  L  K  Q  L  H  E
1321 GAGCTCCGAAAAGCAAGAAATCAAATAACACAGTTGGAATCCTTGAAACAGCTTCATGAG
403 F  A  I  T  E  P  L  V  T  F  Q  G  E  T  E  N  R  E  K  V
1381 TTTGCCATCACAGAGCCATTAGTCACTTTCCAAGGAGAGACTGAAAACAGAGAAAAAGTT
423 A  A  S  P  K  S  P  T  A  A  L  N  G  S  L  V  E  C  P  K
1441 GCCGCCTCACCAAAAAGTCCCACTGCTGCACTCAATGGAAGCCTGGTGGAATGTCCCAAG
443 C  N  I  Q  Y  P  A  T  E  H  R  D  L  L  V  H  V  E  Y  C
1501 TGCAATATACAGTATCCAGCCACTGAGCATCGCGATCTGCTTGTCCATGTGGAATACTGT
463 S  K  *
1561 TCAAAGTAGcaaaataagtatttgttttgatattaaaagattcaatactgtattttctgt
1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacattttgcactgtcaaaatacttggtgaggaaaagatagctc
1861 aggttattgctaatgggttaatgcaccagcaagcaaaatattttatgttttgggggtttt
1921 gaaaaatcaaagataattaaccaaggatcttaactgtgttcgcatttttatccaagcac
1981 ttagaaaacctacaatcctaattttgatgtccattgttaagaggtggtgatagatactat
2041 tttttttttcatattgtatagcggttattagaaaagttggggatttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
```

2281 aactttaataaacccatgtagccctctcatttgattgacagtattttagttatttttggc
2341 attcttaaagctgggcaatgtaatgatcagatctttgtttgtctgaacaggtatttttat
2401 acatgctttttgtaaaccaaaaacttttaaatttcttcaggttttctaacatgcttacca
2461 ctgggctactgta

**Figure 2I. The cDNA (SEQ ID. NO. :____) and amino acid sequence (SEQ ID. NO. :____) of 121P2A3 v.9.** The start methionine is underlined. The open reading frame extends from nucleic acid 175-1569 including the stop codon.

```
  1 gggaccgccagggagggcaggtcagtgggcagatcgcgtccgcgggattcaatctctgcc
 61 cgctctgataacagtccttttccctggcgctcacttcgtgcctggcacccggctgggcgc
  1                                                       M  S
121 ctcaagaccgttgtctcttcgatcgcttctttggacttggcgaccatttcagagATGTCT
  3 S  R  S  T  K  D  L  I  K  S  K  W  G  S  K  P  S  N  S  K
181 TCCAGAAGTACCAAAGATTTAATTAAAAGTAAGTGGGGATCGAAGCCTAGTAACTCCAAA
 23 S  E  T  T  L  E  K  L  K  G  E  I  A  H  L  K  T  S  V  D
241 TCCGAAACTACATTAGAAAAATTAAAGGGAGAAATTGCACACTTAAAGACATCAGTGGAT
 43 E  I  T  S  G  K  G  K  L  T  D  K  E  R  H  R  L  L  E  K
301 GAAATCACAAGTGGGAAAGGAAAGCTGACTGATAAAGAGAGACACAGACTTTTGGAGAAA
 63 I  R  V  L  E  A  E  K  E  K  N  A  Y  Q  L  T  E  K  D  K
361 ATTCGAGTCCTTGAGGCTGAGAAGGAGAAGAATGCTTATCAACTCACAGAGAAGGACAAA
 83 E  I  Q  R  L  R  D  Q  L  K  A  R  Y  S  T  T  A  L  L  E
421 GAAATACAGCGACTGAGAGACCAACTGAAGGCCAGATATAGTACTACCGCATTGCTTGAA
103 Q  L  E  E  T  T  R  E  G  E  R  R  E  Q  V  L  K  A  L  S
481 CAGCTGGAAGAGACAACGAGAGAAGGAGAAAGGAGGGAGCAGGTGTTGAAAGCCTTATCT
123 E  E  K  D  V  L  K  Q  Q  L  S  A  A  T  S  R  I  A  E  L
541 GAAGAGAAAGACGTATTGAAACAACAGTTGTCTGCTGCAACCTCACGAATTGCTGAACTT
143 E  S  K  T  N  T  L  R  L  S  Q  T  V  A  P  N  C  F  N  S
601 GAAAGCAAAACCAATACACTCCGTTTATCACAGACTGTGGCTCCAAACTGCTTCAACTCA
163 S  I  N  N  I  H  E  M  E  I  Q  L  K  D  A  L  E  K  N  Q
```

661 TCAATAAATAATATTCATGAAATGGAAATACAGCTGAAAGATGCTCTGGAGAAAAATCAG
183 Q W L V Y D Q Q R E V Y V K G L L A K I
721 CAGTGGCTCGTGTATGATCAGCAGCGGGAAGTCTATGTAAAAGGACTTTTAGCAAAGATC
203 F E L E K K T E T A A H S L P Q Q T K K
781 TTTGAGTTGGAAAAGAAAACGGAAACAGCTGCTCATTCACTCCCACAGCAGACAAAAAAG
223 P E S E G Y L Q E E K Q K C Y N D L L A
841 CCTGAATCAGAAGGTTATCTTCAAGAAGAGAAGCAGAAATGTTACAACGATCTCTTGGCA
243 S A K K D L E V E R Q T I T Q L S F E L
901 AGTGCAAAAAAAGATCTTGAGGTTGAACGACAAACCATAACTCAGCTGAGTTTTGAACTG
263 S E F R R K Y E E T Q K E V H N L N Q L
961 AGTGAATTTCGAAGAAAATATGAAGAAACCCAAAAAGAAGTTCACAATTTAAATCAGCTG
283 L Y S Q R R A D V Q H L E D D R H K T E
1021 TTGTATTCACAAAGAAGGGCAGATGTGCAACATCTGGAAGATGATAGGCATAAAACAGAG
303 K I Q K L R E E N D I A R G K L E E E K
1081 AAGATACAAAAACTCAGGGAAGAGAATGATATTGCTAGGGGAAAACTTGAAGAAGAGAAG
323 K R S E E L L S Q V Q F L Y T S L L K Q
1141 AAGAGATCCGAAGAGCTCTTATCTCAGGTCCAGTTTCTTTACACATCTCTGCTAAAGCAG
343 Q E E Q T R V A L L E Q Q M Q A C T L D
1201 CAAGAAGAACAAACAAGGGTAGCTCTGTTGGAACAACAGATGCAGGCATGTACTTTAGAC
363 F E N E K L D R Q H V Q H Q L H V I L K
1261 TTTGAAAATGAAAAACTCGACCGTCAACATGTGCAGCATCAATTGCATGTAATTCTTAAG
383 E L R K A R N Q I T Q L E S L K Q L H E
1321 GAGCTCCGAAAAGCAAGAAATCAAATAACACAGTTGGAATCCTTGAAACAGCTTCATGAG
403 F A I T E P L V T F Q G E T E N R E K V
1381 TTTGCCATCACAGAGCCATTAGTCACTTTCCAAGGAGAGACTGAAAACAGAGAAAAAGTT
423 A A S P K S P T A A L N E S L V E C P K
1441 GCCGCCTCACCAAAAAGTCCCACTGCTGCACTCAATGAAAGCCTGGTGGAATGTCCCAAG
443 C N I Q Y P A T E H R D L L V H V E Y C
1501 TGCAATATACAGTATCCAGCCACTGAGCATCGCGATCTGCTTGTCCATGTGGAATACTGT
463 S K *
1561 TCAAAGTAGcaaaataagtatttgttttgatattaaaagattcaatactgtattttctgt

1621 tagcttgtgggcattttgaattatatatttcacattttgcataaaactgcctatctacct
1681 ttgacactccagcatgctagtgaatcatgtatcttttaggctgctgtgcatttctcttgg
1741 cagtgatacctccctgacatggttcatcatcaggctgcaatgacagaatgtggtgagcag
1801 cgtctactgagatactaacattttgcactgtcaaaatacttggtgaggaaaagatagctc
1861 aggttattgctaatgggttaatgcaccagcaagcaaaatattttatgtttcgggggtttt
1921 gaaaaatcaaagataattaaccaaggatcttaactgtgttcgcatttttttatccaagcac
1981 ttagaaaacctacaatcctaattttgatgtccattgttaagaggtggtgatagatactat
2041 tttttttttcatattgtatagcggttattagaaaagttggggatttcttgatctttatt
2101 gctgcttaccattgaaacttaacccagctgtgttccccaactctgttctgcgcacgaaac
2161 agtatctgtttgaggcataatcttaagtggccacacacaatgttttctcttatgttatct
2221 ggcagtaactgtaacttgaattacattagcacattctgcttagctaaaattgttaaaata
2281 aactttaataaacccatgtagccctctcatttgattgacagtattttagttatttttggc
2341 attcttaaagctgggcaatgtaatgatcagatctttgtttgtctgaacaggtatttttat
2401 acatgcttttgtaaaccaaaaactttaaatttcttcaggttttctaacatgcttacca
2461 ctgggctactgta

**Figure 3A** **Amino acid sequence of 121P2A3 v.1 clone 5 (SEQ ID. NO. :____).** The 121P2A3 v.1 clone 5 protein has 464 amino acids.

```
  1  MSSRSTKDLI KSKWGSKPSN SKSETTLEKL KGEIAHLKTS VDEITSGKGK
 51  LTDKERHRLL EKIRVLEAEK EKNAYQLTEK DKEIQRLRDQ LKARYSTTAL
101  LEQLEETTRE GERREQVLKA LSEEKDVLKQ QLSAATSRIA ELESKTNTLR
151  LSQTVAPNCF NSSINNIHEM EIQLKDALEK NQQWLVYDQQ REVYVKGLLA
201  KIFELEKKTE TAAHSLPQQT KKPESEGYLQ EEKQKCYNDL LASAKKDLEV
251  ERQTITQLSF ELSEFRRKYE ETQKEVHNLN QLLYSQRRAD VQHLEDDRHK
301  TEKIQKLREE NDIARGKLEE EKKRSEELLS QVQFLYTSLL KQQEEQTRVA
351  LLEQQMQACT LDFENEKLDR QHVQHQLHVI LKELRKARNQ ITQLESLKQL
401  HEFAITEPLV TFQGETENRE KVAASPKSPT AALNESLVEC PKCNIQYPAT
451  EHRDLLVHVE YCSK
```

**Figure 3B** **Amino acid sequence of 121P2A3 v.2 (SEQ ID. NO. :____).** The 121P2A3 v.2 protein has 295 amino acids.

```
  1 MEIQLKDALE KNQQWLVYDQ QREVYVKGLL AKIFELEKKT ETAAHSLPQQ TKKPESEGYL
 61 QEEKQKCYND LLASAKKDLE VERQTITQLS FELSEFRRKY EETQKEVHNL NQLLYSQRRA
121 DVQHLEDDRH KTEKIQKLRE ENDIARGKLE EEKKRSEELL SQVQFLYTSL LKQQEEQTRV
181 ALLEQQMQAC TLDFENEKLD RQHVQHQLHV ILKELRKARN QITQLESLKQ LHEFAITEPL
241 VTFQGETENR EKVAASPKSP TAALNESLVE CPKCNIQYPA TEHRDLLVHV EYCSK
```

**Figure 3C** **Amino acid sequence of 121P2A3 v.3 (SEQ ID. NO. :____).** The 121P2A3 v.3 protein has 464 amino acids.

```
  1  MSSRSTKDLI KSKWGSKPSN SKSETTLEKL KGEIAHLKTS VDEITSGKGK
 51  LTDKERQRLL EKIRVLEAEK EKNAYQLTEK DKEIQRLRDQ LKARYSTTAL
101  LEQLEETTRE GERREQVLKA LSEEKDVLKQ QLSAATSRIA ELESKTNTLR
151  LSQTVAPNCF NSSINNIHEM EIQLKDALEK NQQWLVYDQQ REVYVKGLLA
201  KIFELEKKTE TAAHSLPQQT KKPESEGYLQ EEKQKCYNDL LASAKKDLEV
251  ERQTITQLSF ELSEFRRKYE ETQKEVHNLN QLLYSQRRAD VQHLEDDRHK
301  TEKIQKLREE NDIARGKLEE EKKRSEELLS QVQFLYTSLL KQQEEQTRVA
351  LLEQQMQACT LDFENEKLDR QHVQHQLHVI LKELRKARNQ ITQLESLKQL
```

```
401    HEFAITEPLV TFQGETENRE KVAASPKSPT AALNESLVEC PKCNIQYPAT
451    EHRDLLVHVE YCSK
```

**Figure 3D** **Amino acid sequence of 121P2A3 v.4 (SEQ ID. NO. :____).** The 121P2A3 v.4 protein has 464 amino acids.

```
  1    MSSRSTKDLI KSKWGSKPSN SKSETTLEKL KGEIAHLKTS VDEITSGKGK
 51    LTDKERHRLL EKIRVLEAEK EKNAYQLTEK DKEIQRLRDQ LKARYSTTTL
101    LEQLEETTRE GERREQVLKA LSEEKDVLKQ QLSAATSRIA ELESKTNTLR
151    LSQTVAPNCF NSSINNIHEM EIQLKDALEK NQQWLVYDQQ REVYVKGLLA
201    KIFELEKKTE TAAHSLPQQT KKPESEGYLQ EEKQKCYNDL LASAKKDLEV
251    ERQTITQLSF ELSEFRRKYE ETQKEVHNLN QLLYSQRRAD VQHLEDDRHK
301    TEKIQKLREE NDIARGKLEE EKKRSEELLS QVQFLYTSLL KQQEEQTRVA
351    LLEQQMQACT LDFENEKLDR QHVQHQLHVI LKELRKARNQ ITQLESLKQL
401    HEFAITEPLV TFQGETENRE KVAASPKSPT AALNESLVEC PKCNIQYPAT
451    EHRDLLVHVE YCSK
```

**Figure 3E** **Amino acid sequence of 121P2A3 v.6 (SEQ ID. NO. :____).** The 121P2A3 v.6 protein has 464 amino acids.

```
  1    MSSRSTKDLI KSKWGSKPSN SKSETTLEKL KGEIAHLKTS VDEITSGKGK
 51    LTDKERHRLL EKIRVLEAEK EKNAYQLTEK DKEIQRLRDQ LKARYSTTAL
101    LEQLEETTRE GERREQVLKA LSEEKDVLKQ QLSAATSRIA ELESKTNTLR
151    LSQTVAPNCF NSSINNIHEM EIQLKDALEK NQQWLVYDQQ REVYVKGLLA
201    KIFELEKKTE TAAHSLPQQT KKPESEGYLQ EEKQKCYNDL LASAKKDLEV
251    ERQTITQLSF ELSEFRRKYE ETQKEVHNLN QLLYSQRRAD VQHLEDDRHK
301    TEKIQKLREE NDIARGKLEE EKKRSEELLS QVQSLYTSLL KQQEEQTRVA
351    LLEQQMQACT LDFENEKLDR QHVQHQLHVI LKELRKARNQ ITQLESLKQL
401    HEFAITEPLV TFQGETENRE KVAASPKSPT AALNESLVEC PKCNIQYPAT
451    EHRDLLVHVE YCSK
```

**Figure 3F** **Amino acid sequence of 121P2A3 v.7 (SEQ ID. NO. :____).** The 121P2A3 v.7 protein has 464 amino acids.

```
  1  MSSRSTKDLI KSKWGSKPSN SKSETTLEKL KGEIAHLKTS VDEITSGKGK
 51  LTDKERHRLL EKIRVLEAEK EKNAYQLTEK DKEIQRLRDQ LKARYSTTAL
101  LEQLEETTRE GERREQVLKA LSEEKDVLKQ QLSAATSRIA ELESKTNTLR
151  LSQTVAPNCF NSSINNIHEM EIQLKDALEK NQQWLVYDQQ REVYVKGLLA
201  KIFELEKKTE TAAHSLPQQT KKPESEGYLQ EEKQKCYNDL LASAKKDLEV
251  ERQTITQLSF ELSEFRRKYE ETQKEVHNLN QLLYSQRRAD VQHLEDDRHK
301  TEKIQKLREE NDIARGKLEE EKKRSEELLS QVQFLYTSLL KQQEEQTRVA
351  LLEQQMQACT LDFENEKLDR QHVQHQLLVI LKELRKARNQ ITQLESLKQL
401  HEFAITEPLV TFQGETENRE KVAASPKSPT AALNESLVEC PKCNIQYPAT
451  EHRDLLVHVE YCSK
```

**Figure 3G** **Amino acid sequence of 121P2A3 v.8 (SEQ ID. NO. :____).** The 121P2A3 v.8 protein has 464 amino acids.

```
  1  MSSRSTKDLI KSKWGSKPSN SKSETTLEKL KGEIAHLKTS VDEITSGKGK
 51  LTDKERHRLL EKIRVLEAEK EKNAYQLTEK DKEIQRLRDQ LKARYSTTAL
101  LEQLEETTRE GERREQVLKA LSEEKDVLKQ QLSAATSRIA ELESKTNTLR
151  LSQTVAPNCF NSSINNIHEM EIQLKDALEK NQQWLVYDQQ REVYVKGLLA
201  KIFELEKKTE TAAHSLPQQT KKPESEGYLQ EEKQKCYNDL LASAKKDLEV
251  ERQTITQLSF ELSEFRRKYE ETQKEVHNLN QLLYSQRRAD VQHLEDDRHK
301  TEKIQKLREE NDIARGKLEE EKKRSEELLS QVQFLYTSLL KQQEEQTRVA
351  LLEQQMQACT LDFENEKLDR QHVQHQLHVI LKELRKARNQ ITQLESLKQL
401  HEFAITEPLV TFQGETENRE KVAASPKSPT AALNGSLVEC PKCNIQYPAT
451  EHRDLLVHVE YCSK
```

**Figure 4A.** Amino acid alignment of 121P2A3 variants.

```
V.1-MSSRSTKDLIKSKWGSKPSNSKSETTLEKLKGEIAHLKTS-40
V.2----------------------------------------0
V.3-MSSRSTKDLIKSKWGSKPSNSKSETTLEKLKGEIAHLKTS-40
V.4-MSSRSTKDLIKSKWGSKPSNSKSETTLEKLKGEIAHLKTS-40
V.5-MSSRSTKDLIKSKWGSKPSNSKSETTLEKLKGEIAHLKTS-40
V.6-MSSRSTKDLIKSKWGSKPSNSKSETTLEKLKGEIAHLKTS-40
V.7-MSSRSTKDLIKSKWGSKPSNSKSETTLEKLKGEIAHLKTS-40
V.8-MSSRSTKDLIKSKWGSKPSNSKSETTLEKLKGEIAHLKTS-40
V.9-MSSRSTKDLIKSKWGSKPSNSKSETTLEKLKGEIAHLKTS-40
I
V.1-VDEITSGKGKLTDKERHRLLEKIRVLEAEKEKNAYQLTEK-80
V.2----------------------------------------0
V.3-VDEITSGKGKLTDKERQRLLEKIRVLEAEKEKNAYQLTEK-80
V.4-VDEITSGKGKLTDKERHRLLEKIRVLEAEKEKNAYQLTEK-80
V.5-VDEITSGKGKLTDKERHRLLEKIRVLEAEKEKNAYQLTEK-80
V.6-VDEITSGKGKLTDKERHRLLEKIRVLEAEKEKNAYQLTEK-80
V.7-VDEITSGKGKLTDKERHRLLEKIRVLEAEKEKNAYQLTEK-80
V.8-VDEITSGKGKLTDKERHRLLEKIRVLEAEKEKNAYQLTEK-80
V.9-VDEITSGKGKLTDKERHRLLEKIRVLEAEKEKNAYQLTEK-80
I
V.1-DKEIQRLRDQLKARYSTTALLEQLEETTREGERREQVLKA-120
V.2----------------------------------------0
V.3-DKEIQRLRDQLKARYSTTALLEQLEETTREGERREQVLKA-120
V.4-DKEIQRLRDQLKARYSTTILLEQLEETTREGERREQVLKA-120
V.5-DKEIQRLRDQLKARYSTTALLEQLEETTREGERREQVLKA-120
V.6-DKEIQRLRDQLKARYSTTALLEQLEETTREGERREQVLKA-120
V.7-DKEIQRLRDQLKARYSTTALLEQLEETTREGERREQVLKA-120
V.8-DKEIQRLRDQLKARYSTTALLEQLEETTREGERREQVLKA-120
V.9-DKEIQRLRDQLKARYSTTALLEQLEETTREGERREQVLKA-120
I
V.1-LSEEKDVLKQQLSAATSRIAELESKTNTLRLSQTVAPNCF-160
V.2----------------------------------------0
V.3-LSEEKDVLKQQLSAATSRIAELESKTNTLRLSQTVAPNCF-160
V.4-LSEEKDVLKQQLSAATSRIAELESKTNTLRLSQTVAPNCF-160
V.5-LSEEKDVLKQQLSAATSRIAELESKTNTLRLSQTVAPNCF-160
V.6-LSEEKDVLKQQLSAATSRIAELESKTNTLRLSQTVAPNCF-160
V.7-LSEEKDVLKQQLSAATSRIAELESKTNTLRLSQTVAPNCF-160
V.8-LSEEKDVLKQQLSAATSRIAELESKTNTLRLSQTVAPNCF-160
V.9-LSEEKDVLKQQLSAATSRIAELESKTNTLRLSQTVAPNCF-160
I
V.1-NSSINNIHEMEIQLKDALEKNQQWLVYDQQREVYVKGLLA-200
V.2----------MEIQLKDALEKNQQWLVYDQQREVYVKGLLA-31
V.3-NSSINNIHEMEIQLKDALEKNQQWLVYDQQREVYVKGLLA-200
V.4-NSSINNIHEMEIQLKDALEKNQQWLVYDQQREVYVKGLLA-200
V.5-NSSINNIHEMEIQLKDALEKNQQWLVYDQQREVYVKGLLA-200
V.6-NSSINNIHEMEIQLKDALEKNQQWLVYDQQREVYVKGLLA-200
V.7-NSSINNIHEMEIQLKDALEKNQQWLVYDQQREVYVKGLLA-200
V.8-NSSINNIHEMEIQLKDALEKNQQWLVYDQQREVYVKGLLA-200
```

V.9-NSSINNIHEMEIQLKDALEKNQQWLVYDQQREVYVKGLLA-200
I
V.1-KIFELEKKTETAAHSLPQQTKKPESEGYLQEEKQKCYNDL-240
V.2-KIFELEKKTETAAHSLPQQTKKPESEGYLQEEKQKCYNDL-71
V.3-KIFELEKKTETAAHSLPQQTKKPESEGYLQEEKQKCYNDL-240
V.4-KIFELEKKTETAAHSLPQQTKKPESEGYLQEEKQKCYNDL-240
V.5-KIFELEKKTETAAHSLPQQTKKPESEGYLQEEKQKCYNDL-240
V.6-KIFELEKKTETAAHSLPQQTKKPESEGYLQEEKQKCYNDL-240
V.7-KIFELEKKTETAAHSLPQQTKKPESEGYLQEEKQKCYNDL-240
V.8-KIFELEKKTETAAHSLPQQTKKPESEGYLQEEKQKCYNDL-240
V.9-KIFELEKKTETAAHSLPQQTKKPESEGYLQEEKQKCYNDL-240
I
V.1-LASAKKDLEVERQTITQLSFELSEFRRKYEETQKEVHNLN-280
V.2-LASAKKDLEVERQTITQLSFELSEFRRKYEETQKEVHNLN-111
V.3-LASAKKDLEVERQTITQLSFELSEFRRKYEETQKEVHNLN-280
V.4-LASAKKDLEVERQTITQLSFELSEFRRKYEETQKEVHNLN-280
V.5-LASAKKDLEVERQTITQLSFELSEFRRKYEETQKEVHNLN-280
V.6-LASAKKDLEVERQTITQLSFELSEFRRKYEETQKEVHNLN-280
V.7-LASAKKDLEVERQTITQLSFELSEFRRKYEETQKEVHNLN-280
V.8-LASAKKDLEVERQTITQLSFELSEFRRKYEETQKEVHNLN-280
V.9-LASAKKDLEVERQTITQLSFELSEFRRKYEETQKEVHNLN-280
I
V.1-QLLYSQRRADVQHLEDDRHKTEKIQKLREENDIARGKLEE-320
V.2-QLLYSQRRADVQHLEDDRHKTEKIQKLREENDIARGKLEE-151
V.3-QLLYSQRRADVQHLEDDRHKTEKIQKLREENDIARGKLEE-320
V.4-QLLYSQRRADVQHLEDDRHKTEKIQKLREENDIARGKLEE-320
V.5-QLLYSQRRADVQHLEDDRHKTEKIQKLREENDIARGKLEE-320
V.6-QLLYSQRRADVQHLEDDRHKTEKIQKLREENDIARGKLEE-320
V.7-QLLYSQRRADVQHLEDDRHKTEKIQKLREENDIARGKLEE-320
V.8-QLLYSQRRADVQHLEDDRHKTEKIQKLREENDIARGKLEE-320
V.9-QLLYSQRRADVQHLEDDRHKTEKIQKLREENDIARGKLEE-320
I
V.1-EKKRSEELLSQVQFLYTSLLKQQEEQTRVALLEQQMQACT-360
V.2-EKKRSEELLSQVQFLYTSLLKQQEEQTRVALLEQQMQACT-191
V.3-EKKRSEELLSQVQFLYTSLLKQQEEQTRVALLEQQMQACT-360
V.4-EKKRSEELLSQVQFLYTSLLKQQEEQTRVALLEQQMQACT-360
V.5-EKKRSEELLSQVQFLYTSLLKQQEEQTRVALLEQQMQACT-360
V.6-EKKRSEELLSQVQSLYTSLLKQQEEQTRVALLEQQMQACT-360
V.7-EKKRSEELLSQVQFLYTSLLKQQEEQTRVALLEQQMQACT-360
V.8-EKKRSEELLSQVQFLYTSLLKQQEEQTRVALLEQQMQACT-360
V.9-EKKRSEELLSQVQFLYTSLLKQQEEQTRVALLEQQMQACT-360

I
V.1-LDFENEKLDRQHVQHQLHVILKELRKARNQITQLESLKQL-400
V.2-LDFENEKLDRQHVQHQLHVILKELRKARNQITQLESLKQL-231
V.3-LDFENEKLDRQHVQHQLHVILKELRKARNQITQLESLKQL-400
V.4-LDFENEKLDRQHVQHQLHVILKELRKARNQITQLESLKQL-400
V.5-LDFENEKLDRQHVQHQLHVILKELRKARNQITQLESLKQL-400
V.6-LDFENEKLDRQHVQHQLHVILKELRKARNQITQLESLKQL-400
V.7-LDFENEKLDRQHVQHQLLVILKELRKARNQITQLESLKQL-400
V.8-LDFENEKLDRQHVQHQLHVILKELRKARNQITQLESLKQL-400
V.9-LDFENEKLDRQHVQHQLHVILKELRKARNQITQLESLKQL-400
I
V.1-HEFAITEPLVTFQGETENREKVAASPKSPTAALNESLVEC-440
V.2-HEFAITEPLVTFQGETENREKVAASPKSPTAALNESLVEC-271
V.3-HEFAITEPLVTFQGETENREKVAASPKSPTAALNESLVEC-440
V.4-HEFAITEPLVTFQGETENREKVAASPKSPTAALNESLVEC-440
V.5-HEFAITEPLVTFQGETENREKVAASPKSPTAALNESLVEC-440
V.6-HEFAITEPLVTFQGETENREKVAASPKSPTAALNESLVEC-440
V.7-HEFAITEPLVTFQGETENREKVAASPKSPTAALNESLVEC-440
V.8-HEFAITEPLVTFQGETENREKVAASPKSPTAALNGSLVEC-440
V.9-HEFAITEPLVTFQGETENREKVAASPKSPTAALNESLVEC-440
I
V.1-PKCNIQYPATEHRDLLVHVEYCSK -464
V.2-PKCNIQYPATEHRDLLVHVEYCSK -295
V.3-PKCNIQYPATEHRDLLVHVEYCSK -464
V.4-PKCNIQYPATEHRDLLVHVEYCSK -464
V.5-PKCNIQYPATEHRDLLVHVEYCSK -464
V.6-PKCNIQYPATEHRDLLVHVEYCSK -464
V.7-PKCNIQYPATEHRDLLVHVEYCSK -464
V.8-PKCNIQYPATEHRDLLVHVEYCSK -464
V.9-PKCNIQYPATEHRDLLVHVEYCSK -464
I

**Figure 4B. Nucleic Acid sequence alignment of 121P2A3 v.1 with the hypothetical protein FLJ10540.**

```
>gi|8922501|ref|NM_018131.1| Homo sapiens hypothetical protein FLJ10540
   (FLJ10540), mRNA      Length = 2232

  Score = 4298 bits (2168), Expect = 0.0
  Identities = 2196/2203 (99%), Gaps = 3/2203 (0%)
  Strand = Plus / Plus

Query: 271   gaaattgcacacttaaagacatcagtggatgaaatcacaagtgggaaaggaaagctgact 330
             ||||||||||||||||||||||||||||||||||||||||||||||||||||||||||||
Sbjct: 1     gaaattgcacacttaaagacatcagtggatgaaatcacaagtgggaaaggaaagctgact 60


Query: 331   gataaagagagacacagacttttggagaaaattcgagtccttgaggctgagaaggagaag 390
             ||||||||||||| ||||||||||||||||||||||||||||||||||||||||||||||
Sbjct: 61    gataaagagagacagagacttttggagaaaattcgagtccttgaggctgagaaggagaag 120


Query: 391   aatgcttatcaactcacagagaaggacaaagaaatacagcgactgagagaccaactgaag 450
             ||||||||||||||||||||||||||||||||||||||||||||||||||||||||||||
Sbjct: 121   aatgcttatcaactcacagagaaggacaaagaaatacagcgactgagagaccaactgaag 180


Query: 451   gccagatatagtactaccgcattgcttgaacagctggaagagacaacgagagaaggagaa 510
             ||||||||||||||||||||||||||||||||||||||||||||||||||||||||||||
Sbjct: 181   gccagatatagtactaccgcattgcttgaacagctggaagagacaacgagagaaggagaa 240


Query: 511   aggagggagcaggtgttgaaagccttatctgaagagaaagacgtattgaaacaacagttg 570
             ||||||||||||||||||||||||||||||||||||||||||||||||||||||||||||
Sbjct: 241   aggagggagcaggtgttgaaagccttatctgaagagaaagacgtattgaaacaacagttg 300


Query: 571   tctgctgcaacctcacgaattgctgaacttgaaagcaaaaccaatacactccgtttatca 630
             ||||||||||||||||||||||||||||||||||||||||||||||||||||||||||||
Sbjct: 301   tctgctgcaacctcacgaattgctgaacttgaaagcaaaaccaatacactccgtttatca 360


Query: 631   cagactgtggctccaaactgcttcaactcatcaataaataatattcatgaaatggaaata 690
             ||||||||||||||||||||||||||||||||||||||||||||||||||||||||||||
Sbjct: 361   cagactgtggctccaaactgcttcaactcatcaataaataatattcatgaaatggaaata 420


Query: 691   cagctgaaagatgctctggagaaaaatcagcagtggctcgtgtatgatcagcagcgggaa 750
             ||||||||||||||||||||||||||||||||||||||||||||||||||||||||||||
Sbjct: 421   cagctgaaagatgctctggagaaaaatcagcagtggctcgtgtatgatcagcagcgggaa 480


Query: 751   gtctatgtaaaaggacttttagcaaagatctttgagttggaaaagaaaacggaaacagct 810
             ||||||||||||||||||||||||||||||||||||||||||||||||||||||||||||
```

```
Sbjct: 481  gtctatgtaaaaggacttttagcaaagatctttgagttggaaaagaaaacggaaacagct 540

Query: 811  gctcattcactcccacagcagacaaaaaagcctgaatcagaaggttatcttcaagaagag 870
            ||||||||||||||||||||||||||||||||||||||||||||||||||||||||||||
Sbjct: 541  gctcattcactcccacagcagacaaaaaagcctgaatcagaaggttatcttcaagaagag 600

Query: 871  aagcagaaatgttacaacgatctcttggcaagtgcaaaaaaagatcttgaggttgaacga 930
            ||||||||||||||||||||||||||||||||||||||||||||||||||||||||||||
Sbjct: 601  aagcagaaatgttacaacgatctcttggcaagtgcaaaaaaagatcttgaggttgaacga 660

Query: 931  caaaccataactcagctgagttttgaactgagtgaatttcgaagaaaatatgaagaaacc 990
            ||||||||||||||||||||||||||||||||||||||||||||||||||||||||||||
Sbjct: 661  caaaccataactcagctgagttttgaactgagtgaatttcgaagaaaatatgaagaaacc 720

Query: 991  caaaaagaagttcacaatttaaatcagctgttgtattcacaaagaagggcagatgtgcaa 1050
            ||||||||||||||||||||||||||||||||||||||||||||||||||||||||||||
Sbjct: 721  caaaaagaagttcacaatttaaatcagctgttgtattcacaaagaagggcagatgtgcaa 780

Query: 1051 catctggaagatgataggcataaaacagagaagatacaaaaactcagggaagagaatgat 1110
            ||||||||||||||||||||||||||||||||||||||||||||||||||||||||||||
Sbjct: 781  catctggaagatgataggcataaaacagagaagatacaaaaactcagggaagagaatgat 840

Query: 1111 attgctaggggaaaacttgaagaagagaagaagagatccgaagagctcttatctcaggtc 1170
            ||||||||||||||||||||||||||||||||||||||||||||||||||||||||||||
Sbjct: 841  attgctaggggaaaacttgaagaagagaagaagagatccgaagagctcttatctcaggtc 900

Query: 1171 cagtttctttacacatctctgctaaagcagcaagaagaacaaacaagggtagctctgttg 1230
            |||| |||||||||||||||||||||||||||||||||||||||||||||||||||||||
Sbjct: 901  cagtctctttacacatctctgctaaagcagcaagaagaacaaacaagggtagctctgttg 960

Query: 1231 gaacaacagatgcaggcatgtactttagactttgaaaatgaaaaactcgaccgtcaacat 1290
            ||||||||||||||||||||||||||||||||||||||||||||||||||||||||||||
Sbjct: 961  gaacaacagatgcaggcatgtactttagactttgaaaatgaaaaactcgaccgtcaacat 1020

Query: 1291 gtgcagcatcaattgcatgtaattcttaaggagctccgaaaagcaagaaatcaaataaca 1350
            ||||||||||||||||||||||||||||||||||||||||||||||||||  ||||||||
Sbjct: 1021 gtgcagcatcaattgcatgtaattcttaaggagctccgaaaagcaagaaa--aaataaca 1078

Query: 1351 cagttggaatccttgaaacagcttcatgagtttgccatcacagagccattagtcactttc 1410
            ||||||||||||||||||||||||||||||||||||||||||||||||||||||||||||
Sbjct: 1079 cagttggaatccttgaaacagcttcatgagtttgccatcacagagccattagtcactttc 1138
```

```
Query: 1411 caaggagagactgaaaacagagaaaaagttgccgcctcaccaaaaagtcccactgctgca 1470
            ||||||||||||||||||||||||||||||||||||||||||||||||||||||||||||
Sbjct: 1139 caaggagagactgaaaacagagaaaaagttgccgcctcaccaaaaagtcccactgctgca 1198

Query: 1471 ctcaatgaaagcctggtggaatgtcccaagtgcaatatacagtatccagccactgagcat 1530
            ||||||| ||||||||||||||||||||||||||||||||||||||||||||||||||||
Sbjct: 1199 ctcaatggaagcctggtggaatgtcccaagtgcaatatacagtatccagccactgagcat 1258

Query: 1531 cgcgatctgcttgtccatgtggaatactgttcaaagtagcaaaataagtatttgttttga 1590
            ||||||||||||||||||||||||||||||||||||||||||||||||||||||||||||
Sbjct: 1259 cgcgatctgcttgtccatgtggaatactgttcaaagtagcaaaataagtatttgttttga 1318

Query: 1591 tattaaaagattcaatactgtattttctgttagcttgtgggcattttgaattatatattt 1650
            ||||||||||||||||||||||||||||||||||||||||||||||||||||||||||||
Sbjct: 1319 tattaaaagattcaatactgtattttctgttagcttgtgggcattttgaattatatattt 1378

Query: 1651 cacattttgcataaaactgcctatctacctttgacactccagcatgctagtgaatcatgt 1710
            ||||||||||||||||||||||||||||||||||||||||||||||||||||||||||||
Sbjct: 1379 cacattttgcataaaactgcctatctacctttgacactccagcatgctagtgaatcatgt 1438

Query: 1711 atcttttaggctgctgtgcatttctcttggcagtgatacctccctgacatggttcatcat 1770
            ||||||||||||||||||||||||||||||||||||||||||||||||||||||||||||
Sbjct: 1439 atcttttaggctgctgtgcatttctcttggcagtgatacctccctgacatggttcatcat 1498

Query: 1771 caggctgcaatgacagaatgtggtgagcagcgtctactgagatactaacattttgcactg 1830
            ||||||||||||||||||||||||||||||||||||||||||||||||||||||||||||
Sbjct: 1499 caggctgcaatgacagaatgtggtgagcagcgtctactgagatactaacattttgcactg 1558

Query: 1831 tcaaaatacttggtgaggaaaagatagctcaggttattgctaatgggttaatgcaccagc 1890
            ||||||||||||||||||||||||||||||||||||||||||||||||||||||||||||
Sbjct: 1559 tcaaaatacttggtgaggaaaagatagctcaggttattgctaatgggttaatgcaccagc 1618

Query: 1891 aagcaaaatatttatgttttgggggttttgaaaaatcaaagataattaaccaaggatct 1950
            ||||||||||||||||||| ||||||||||||||||||||||||||||||||||||||||
Sbjct: 1619 aagcaaaatatttatgtttcgggggttttgaaaaatcaaagataattaaccaaggatct 1678

Query: 1951 taactgtgttcgcatttttatccaagcacttagaaaacctacaatcctaattttgatgt 2010
            ||||||||||||||||||||||||||||||||||||||||||||||||||||||||||||
Sbjct: 1679 taactgtgttcgcatttttatccaagcacttagaaaacctacaatcctaattttgatgt 1738
```

```
Query: 2011 ccattgttaagaggtggtgatagatactatttttttttcatattgtatagcggttatta 2070
            ||||||||||||||||||||||||||| ||||||||||||||||||||||||||||||||
Sbjct: 1739 ccattgttaagaggtggtgatagatacta-tttttttttcatattgtatagcggttatta 1797

Query: 2071 gaaaagttggggattttcttgatctttattgctgcttaccattgaaacttaacccagctg 2130
            ||||||||||||||||||||||||||||||||||||||||||||||||||||||||||||
Sbjct: 1798 gaaaagttggggattttcttgatctttattgctgcttaccattgaaacttaacccagctg 1857

Query: 2131 tgttccccaactctgttctgcgcacgaaacagtatctgtttgaggcataatcttaagtgg 2190
            ||||||||||||||||||||||||||||||||||||||||||||||||||||||||||||
Sbjct: 1858 tgttccccaactctgttctgcgcacgaaacagtatctgtttgaggcataatcttaagtgg 1917

Query: 2191 ccacacacaatgttttctcttatgttatctggcagtaactgtaacttgaattacattagc 2250
            ||||||||||||||||||||||||||||||||||||||||||||||||||||||||||||
Sbjct: 1918 ccacacacaatgttttctcttatgttatctggcagtaactgtaacttgaattacattagc 1977

Query: 2251 acattctgcttagctaaaattgttaaaataaactttaataaacccatgtagccctctcat 2310
            ||||||||||||||||||||||||||||||||||||||||||||||||||||||||||||
Sbjct: 1978 acattctgcttagctaaaattgttaaaataaactttaataaacccatgtagccctctcat 2037

Query: 2311 ttgattgacagtattttagttattttggcattcttaaagctgggcaatgtaatgatcag 2370
            ||||||||||||||||||||||||||||||||||||||||||||||||||||||||||||
Sbjct: 2038 ttgattgacagtattttagttattttggcattcttaaagctgggcaatgtaatgatcag 2097

Query: 2371 atctttgtttgtctgaacaggtatttttatacatgctttttgtaaaccaaaaacttttaa 2430
            ||||||||||||||||||||||||||||||||||||||||||||||||||||||||||||
Sbjct: 2098 atctttgtttgtctgaacaggtatttttatacatgctttttgtaaaccaaaaacttttaa 2157

Query: 2431 atttcttcaggttttctaacatgcttaccactgggctactgta 2473
            |||||||||||||||||||||||||||||||||||||||||||
Sbjct: 2158 atttcttcaggttttctaacatgcttaccactgggctactgta 2200
```

**Figure 4C. Nucleic Acid sequence alignment of 121P2A3 v.1 with cDNA similar to RIKEN 1200008012 gene.**

```
>gi|14286293|gb|BC008947.1|BC008947 Homo sapiens, Similar to RIKEN cDNA
1200008012 gene, clone MGC:3422   IMAGE:3028566, mRNA, complete cds
          Length = 2644

 Score = 4863 bits (2453), Expect = 0.0
 Identities = 2470/2473 (99%), Gaps = 2/2473 (0%)
 Strand = Plus / Plus
```

```
Query: 2    ggaccgccagggagggcaggtcagtgggcagatcgcgtccgcgggattcaatctctgccc 61
            ||||||||||||||||||||||||||||||||||||||||||||||||||||||||||||
Sbjct: 121  ggaccgccagggagggcaggtcagtgggcagatcgcgtccgcgggattcaatctctgccc 180

Query: 62   gctctgataacagtccttttccctggcgctcacttcgtgcctggcacccggctgggcgcc 121
            ||||||||||||||||||||||||||||||||||||||||||||||||||||||||||||
Sbjct: 181  gctctgataacagtccttttccctggcgctcacttcgtgcctggcacccggctgggcgcc 240

Query: 122  tcaagaccgttgtctcttcgatcgcttctttggacttggcgaccatttcagagatgtctt 181
            ||||||||||||||||||||||||||||||||||||||||||||||||||||||||||||
Sbjct: 241  tcaagaccgttgtctcttcgatcgcttctttggacttggcgaccatttcagagatgtctt 300

Query: 182  ccagaagtaccaaagatttaattaaaagtaagtggggatcgaagcctagtaactccaaat 241
            ||||||||||||||||||||||||||||||||||||||||||||||||||||||||||||
Sbjct: 301  ccagaagtaccaaagatttaattaaaagtaagtggggatcgaagcctagtaactccaaat 360

Query: 242  ccgaaactacattagaaaaattaaagggagaaattgcacacttaaagacatcagtggatg 301
            ||||||||||||||||||||||||||||||||||||||||||||||||||||||||||||
Sbjct: 361  ccgaaactacattagaaaaattaaagggagaaattgcacacttaaagacatcagtggatg 420

Query: 302  aaatcacaagtgggaaaggaaagctgactgataaagagagacacagacttttggagaaaa 361
            ||||||||||||||||||||||||||||||||||||||||||||||||||||||||||||
Sbjct: 421  aaatcacaagtgggaaaggaaagctgactgataaagagagacacagacttttggagaaaa 480

Query: 362  ttcgagtccttgaggctgagaaggagaagaatgcttatcaactcacagagaaggacaaag 421
            ||||||||||||||||||||||||||||||||||||||||||||||||||||||||||||
Sbjct: 481  ttcgagtccttgaggctgagaaggagaagaatgcttatcaactcacagagaaggacaaag 540

Query: 422  aaatacagcgactgagagaccaactgaaggccagatatagtactaccgcattgcttgaac 481
            ||||||||||||||||||||||||||||||||||||||||||||||||||||||||||||
Sbjct: 541  aaatacagcgactgagagaccaactgaaggccagatatagtactaccgcattgcttgaac 600

Query: 482  agctggaagagacaacgagagaaggagaaaggagggagcaggtgttgaaagccttatctg 541
            ||||||||||||||||||||||||||||||||||||||||||||||||||||||||||||
Sbjct: 601  agctggaagagacaacgagagaaggagaaaggagggagcaggtgttgaaagccttatctg 660

Query: 542  aagagaaagacgtattgaaacaacagttgtctgctgcaacctcacgaattgctgaacttg 601
            ||||||||||||||||||||||||||||||||||||||||||||||||||||||||||||
Sbjct: 661  aagagaaagacgtattgaaacaacagttgtctgctgcaacctcacgaattgctgaacttg 720
```

```
Query: 602  aaagcaaaaccaatacactccgtttatcacagactgtggctccaaactgcttcaactcat 661
            ||||||||||||||||||||||||||||||||||||||||||||||||||||||||||||
Sbjct: 721  aaagcaaaaccaatacactccgtttatcacagactgtggctccaaactgcttcaactcat 780

Query: 662  caataaataatattcatgaaatggaaatacagctgaaagatgctctggagaaaaatcagc 721
            ||||||||||||||||||||||||||||||||||||||||||||||||||||||||||||
Sbjct: 781  caataaataatattcatgaaatggaaatacagctgaaagatgctctggagaaaaatcagc 840

Query: 722  agtggctcgtgtatgatcagcagcgggaagtctatgtaaaaggacttttagcaaagatct 781
            ||||||||||||||||||||||||||||||||||||||||||||||||||||||||||||
Sbjct: 841  agtggctcgtgtatgatcagcagcgggaagtctatgtaaaaggacttttagcaaagatct 900

Query: 782  ttgagttggaaaagaaaacggaaacagctgctcattcactcccacagcagacaaaaaagc 841
            ||||||||||||||||||||||||||||||||||||||||||||||||||||||||||||
Sbjct: 901  ttgagttggaaaagaaaacggaaacagctgctcattcactcccacagcagacaaaaaagc 960

Query: 842  ctgaatcagaaggttatcttcaagaagagaagcagaaatgttacaacgatctcttggcaa 901
            ||||||||||||||||||||||||||||||||||||||||||||||||||||||||||||
Sbjct: 961  ctgaatcagaaggttatcttcaagaagagaagcagaaatgttacaacgatctcttggcaa 1020

Query: 902  gtgcaaaaaaagatcttgaggttgaacgacaaaccataactcagctgagttttgaactga 961
            ||||||||||||||||||||||||||||||||||||||||||||||||||||||||||||
Sbjct: 1021 gtgcaaaaaaagatcttgaggttgaacgacaaaccataactcagctgagttttgaactga 1080

Query: 962  gtgaatttcgaagaaaatatgaagaaacccaaaaagaagttcacaatttaaatcagctgt 1021
            ||||||||||||||||||||||||||||||||||||||||||||||||||||||||||||
Sbjct: 1081 gtgaatttcgaagaaaatatgaagaaacccaaaaagaagttcacaatttaaatcagctgt 1140

Query: 1022 tgtattcacaaagaagggcagatgtgcaacatctggaagatgataggcataaaacagaga 1081
            ||||||||||||||||||||||||||||||||||||||||||||||||||||||||||||
Sbjct: 1141 tgtattcacaaagaagggcagatgtgcaacatctggaagatgataggcataaaacagaga 1200

Query: 1082 agatacaaaaactcagggaagagaatgatattgctaggggaaaacttgaagaagagaaga 1141
            ||||||||||||||||||||||||||||||||||||||||||||||||||||||||||||
Sbjct: 1201 agatacaaaaactcagggaagagaatgatattgctaggggaaaacttgaagaagagaaga 1260

Query: 1142 agagatccgaagagctcttatctcaggtccagtttctttacacatctctgctaaagcagc 1201
            ||||||||||||||||||||||||||||||||||||||||||||||||||||||||||||
Sbjct: 1261 agagatccgaagagctcttatctcaggtccagtttctttacacatctctgctaaagcagc 1320

Query: 1202 aagaagaacaaacaagggtagctctgttggaacaacagatgcaggcatgtactttagact 1261
```

```
             ||||||||||||||||||||||||||||||||||||||||||||||||||||||||||||
Sbjct: 1321 aagaagaacaaacaagggtagctctgttggaacaacagatgcaggcatgtactttagact 1380

Query: 1262 ttgaaaatgaaaaactcgaccgtcaacatgtgcagcatcaattgcatgtaattcttaagg 1321
             |||||||||||||||||||||||||||||||||||||||||||||| |||||||||||||
Sbjct: 1381 ttgaaaatgaaaaactcgaccgtcaacatgtgcagcatcaattgcttgtaattcttaagg 1440

Query: 1322 agctccgaaaagcaagaaatcaaataacacagttggaatccttgaaacagcttcatgagt 1381
             ||||||||||||||||||||||||||||||||||||||||||||||||||||||||||||
Sbjct: 1441 agctccgaaaagcaagaaatcaaataacacagttggaatccttgaaacagcttcatgagt 1500

Query: 1382 ttgccatcacagagccattagtcactttccaaggagagactgaaaacagagaaaaagttg 1441
             ||||||||||||||||||||||||||||||||||||||||||||||||||||||||||||
Sbjct: 1501 ttgccatcacagagccattagtcactttccaaggagagactgaaaacagagaaaaagttg 1560

Query: 1442 ccgcctcaccaaaaagtcccactgctgcactcaatgaaagcctggtggaatgtcccaagt 1501
             ||||||||||||||||||||||||||||||||||||||||||||||||||||||||||||
Sbjct: 1561 ccgcctcaccaaaaagtcccactgctgcactcaatgaaagcctggtggaatgtcccaagt 1620

Query: 1502 gcaatatacagtatccagccactgagcatcgcgatctgcttgtccatgtggaatactgtt 1561
             ||||||||||||||||||||||||||||||||||||||||||||||||||||||||||||
Sbjct: 1621 gcaatatacagtatccagccactgagcatcgcgatctgcttgtccatgtggaatactgtt 1680

Query: 1562 caaagtagcaaaataagtatttgttttgatattaaaagattcaatactgtatttctgtt 1621
             ||||||||||||||||||||||||||||||||||||||||||||||||||||||||||||
Sbjct: 1681 caaagtagcaaaataagtatttgttttgatattaaaagattcaatactgtatttctgtt 1740

Query: 1622 agcttgtgggcattttgaattatatatttcacattttgcataaaactgcctatctacctt 1681
             ||||||||||||||||||||||||||||||||||||||||||||||||||||||||||||
Sbjct: 1741 agcttgtgggcattttgaattatatatttcacattttgcataaaactgcctatctacctt 1800

Query: 1682 tgacactccagcatgctagtgaatcatgtatcttttaggctgctgtgcatttctcttggc 1741
             ||||||||||||||||||||||||||||||||||||||||||||||||||||||||||||
Sbjct: 1801 tgacactccagcatgctagtgaatcatgtatcttttaggctgctgtgcatttctcttggc 1860

Query: 1742 agtgatacctccctgacatggttcatcatcaggctgcaatgacagaatgtggtgagcagc 1801
             ||||||||||||||||||||||||||||||||||||||||||||||||||||||||||||
Sbjct: 1861 agtgatacctccctgacatggttcatcatcaggctgcaatgacagaatgtggtgagcagc 1920

Query: 1802 gtctactgaga-tactaacattttgcactgtcaaaatacttggtgaggaaaagatagctc 1860
             ||||||||||| ||||||||||||||||||||||||||||||||||||||||||||||||
```

```
Sbjct: 1921 gtctactgagactactaacattttgcactgtcaaaatacttggtgaggaaaagatagctc 1980

Query: 1861 aggttattgctaatgggttaatgcaccagcaagcaaaatatttatgttttgggggtttt 1920
            ||||||||||||||||||||||||||||||||||||||||||||||||||||||| |||
Sbjct: 1981 aggttattgctaatgggttaatgcaccagcaagcaaaatatttatgttttgggggg-ttt 2039

Query: 1921 gaaaaatcaaagataattaaccaaggatcttaactgtgttcgcatttttatccaagcac 1980
            ||||||||||||||||||||||||||||||||||||||||||||||||||||||||||||
Sbjct: 2040 gaaaaatcaaagataattaaccaaggatcttaactgtgttcgcatttttatccaagcac 2099

Query: 1981 ttagaaaacctacaatcctaattttgatgtccattgttaagaggtggtgatagatactat 2040
            ||||||||||||||||||||||||||||||||||||||||||||||||||||||||||||
Sbjct: 2100 ttagaaaacctacaatcctaattttgatgtccattgttaagaggtggtgatagatactat 2159

Query: 2041 tttttttttcatattgtatagcggttattagaaaagttggggatttcttgatctttatt 2100
            ||||||||||||||||||||||||||||||||||||||||||||||||||||||||||||
Sbjct: 2160 tttttttttcatattgtatagcggttattagaaaagttggggatttcttgatctttatt 2219

Query: 2101 gctgcttaccattgaaacttaacccagctgtgttccccaactctgttctgcgcacgaaac 2160
            ||||||||||||||||||||||||||||||||||||||||||||||||||||||||||||
Sbjct: 2220 gctgcttaccattgaaacttaacccagctgtgttccccaactctgttctgcgcacgaaac 2279

Query: 2161 agtatctgtttgaggcataatcttaagtggccacacacaatgttttctcttatgttatct 2220
            ||||||||||||||||||||||||||||||||||||||||||||||||||||||||||||
Sbjct: 2280 agtatctgtttgaggcataatcttaagtggccacacacaatgttttctcttatgttatct 2339

Query: 2221 ggcagtaactgtaacttgaattacattagcacattctgcttagctaaaattgttaaaata 2280
            ||||||||||||||||||||||||||||||||||||||||||||||||||||||||||||
Sbjct: 2340 ggcagtaactgtaacttgaattacattagcacattctgcttagctaaaattgttaaaata 2399

Query: 2281 aactttaataaacccatgtagccctctcatttgattgacagtatttagttatttttggc 2340
            ||||||||||||||||||||||||||||||||||||||||||||||||||||||||||||
Sbjct: 2400 aactttaataaacccatgtagccctctcatttgattgacagtatttagttatttttggc 2459

Query: 2341 attcttaaagctgggcaatgtaatgatcagatctttgtttgtctgaacaggtatttttat 2400
            ||||||||||||||||||||||||||||||||||||||||||||||||||||||||||||
Sbjct: 2460 attcttaaagctgggcaatgtaatgatcagatctttgtttgtctgaacaggtatttttat 2519

Query: 2401 acatgcttttgtaaaccaaaaacttttaaatttcttcaggttttctaacatgcttacca 2460
            ||||||||||||||||||||||||||||||||||||||||||||||||||||||||||||
Sbjct: 2520 acatgcttttgtaaaccaaaaacttttaaatttcttcaggttttctaacatgcttacca 2579
```

```
Query: 2461 ctgggctactgta 2473
            |||||||||||||
Sbjct: 2580 ctgggctactgta 2592
```

**Figure 4D. Amino acid sequence alignment of 121P2A3 v.1 with the hypothetical protein FLJ10540.**

```
>gi|8922502|ref|NP_060601.1| (NM_018131) hypothetical protein FLJ10540
[Homo sapiens]
          Length = 231

 Score =  296 bits (757), Expect = 4e-79
 Identities = 219/223 (98%), Positives = 219/223 (98%)

Query: 170 MEIQLKDALEKNQQWLVYDQQREVYVKGLLAKIFELEKKTETAAHSLPQQTKKPESEGYL 229
            MEIQLKDALEKNQQWLVYDQQREVYVKGLLAKIFELEKKTETAAHSLPQQTKKPESEGYL
Sbjct: 1   MEIQLKDALEKNQQWLVYDQQREVYVKGLLAKIFELEKKTETAAHSLPQQTKKPESEGYL 60

Query: 230 QEEKQKCYNDLLASAKKDLEVERQTITQLSFELSEFRRKYEETQKEVHNLNQLLYSQRRA 289
            QEEKQKCYNDLLASAKKDLEVERQTITQLSFELSEFRRKYEETQKEVHNLNQLLYSQRRA
Sbjct: 61  QEEKQKCYNDLLASAKKDLEVERQTITQLSFELSEFRRKYEETQKEVHNLNQLLYSQRRA 120

Query: 290 DVQHLEDDRHKTEKIQKLREENDIARGKLEEEKKRSEELLSQVQFLYTSLLKQQEEQTRV 349
            DVQHLEDDRHKTEKIQKLREENDIARGKLEEEKKRSEELLSQVQ LYTSLLKQQEEQTRV
Sbjct: 121 DVQHLEDDRHKTEKIQKLREENDIARGKLEEEKKRSEELLSQVQSLYTSLLKQQEEQTRV 180

Query: 350 ALLEQQMQACTLDFENEKLDRQHVQHQLHVILKELRKARNQIT 392
            ALLEQQMQACTLDFENEKLDRQHVQHQLHVILKELRKAR   T
Sbjct: 181 ALLEQQMQACTLDFENEKLDRQHVQHQLHVILKELRKARKNNT 223
```

**Figure 4E. Amino acid sequence alignment of 121P2A3 v.1 with protein XM_005908 similar to RIKEN cDNA 1200008012.**

```
>gi|14745180|ref|XP_005908.3| (XM_005908) similar to RIKEN cDNA
1200008012 gene [Homo sapiens]
          Length = 464

 Score =  654 bits (1687), Expect = 0.0
 Identities = 463/464 (99%), Positives = 463/464 (99%)

Query: 1   MSSRSTKDLIKSKWGSKPSNSKSETTLEKLKGEIAHLKTSVDEITSGKGKLTDKERHRLL 60
            MSSRSTKDLIKSKWGSKPSNSKSETTLEKLKGEIAHLKTSVDEITSGKGKLTDKERHRLL
Sbjct: 1   MSSRSTKDLIKSKWGSKPSNSKSETTLEKLKGEIAHLKTSVDEITSGKGKLTDKERHRLL 60

Query: 61  EKIRVLEAEKEKNAYQLTEKDKEIQRLRDQLKARYSTTALLEQLEETTREGERREQVLKA 120
            EKIRVLEAEKEKNAYQLTEKDKEIQRLRDQLKARYSTT LLEQLEETTREGERREQVLKA
Sbjct: 61  EKIRVLEAEKEKNAYQLTEKDKEIQRLRDQLKARYSTTTLLEQLEETTREGERREQVLKA 120

Query: 121 LSEEKDVLKQQLSAATSRIAELESKTNTLRLSQTVAPNCFNSSINNIHEMEIQLKDALEK 180
            LSEEKDVLKQQLSAATSRIAELESKTNTLRLSQTVAPNCFNSSINNIHEMEIQLKDALEK
Sbjct: 121 LSEEKDVLKQQLSAATSRIAELESKTNTLRLSQTVAPNCFNSSINNIHEMEIQLKDALEK 180

Query: 181 NQQWLVYDQQREVYVKGLLAKIFELEKKTETAAHSLPQQTKKPESEGYLQEEKQKCYNDL 240
            NQQWLVYDQQREVYVKGLLAKIFELEKKTETAAHSLPQQTKKPESEGYLQEEKQKCYNDL
Sbjct: 181 NQQWLVYDQQREVYVKGLLAKIFELEKKTETAAHSLPQQTKKPESEGYLQEEKQKCYNDL 240
```

```
Query: 241 LASAKKDLEVERQTITQLSFELSEFRRKYEETQKEVHNLNQLLYSQRRADVQHLEDDRHK 300
           LASAKKDLEVERQTITQLSFELSEFRRKYEETQKEVHNLNQLLYSQRRADVQHLEDDRHK
Sbjct: 241 LASAKKDLEVERQTITQLSFELSEFRRKYEETQKEVHNLNQLLYSQRRADVQHLEDDRHK 300

Query: 301 TEKIQKLREENDIARGKLEEEKKRSEELLSQVQFLYTSLLKQQEEQTRVALLEQQMQACT 360
           TEKIQKLREENDIARGKLEEEKKRSEELLSQVQFLYTSLLKQQEEQTRVALLEQQMQACT
Sbjct: 301 TEKIQKLREENDIARGKLEEEKKRSEELLSQVQFLYTSLLKQQEEQTRVALLEQQMQACT 360

Query: 361 LDFENEKLDRQHVQHQLHVILKELRKARNQITQLESLKQLHEFAITEPLVTFQGETENRE 420
           LDFENEKLDRQHVQHQLHVILKELRKARNQITQLESLKQLHEFAITEPLVTFQGETENRE
Sbjct: 361 LDFENEKLDRQHVQHQLHVILKELRKARNQITQLESLKQLHEFAITEPLVTFQGETENRE 420

Query: 421 KVAASPKSPTAALNESLVECPKCNIQYPATEHRDLLVHVEYCSK 464
           KVAASPKSPTAALNESLVECPKCNIQYPATEHRDLLVHVEYCSK
Sbjct: 421 KVAASPKSPTAALNESLVECPKCNIQYPATEHRDLLVHVEYCSK 464
```

**Figure 4F. Amino acid sequence alignment of 121P2A3 v.1 with the mouse putative protein clone NT2RP2001245.**

```
>gi|12835981|dbj|BAB23446.1| (AK004655) data source:SPTR, source
key:Q9NVS7,
          evidence:ISS~homolog to CDNA FLJ10540 FIS, CLONE
          NT2RP2001245~putative [Mus musculus]
         Length = 462

 Score =  479 bits (1233), Expect = e-134
 Identities = 349/464 (75%), Positives = 404/464 (86%), Gaps = 2/464 (0%)

Query: 1   MSSRSTKDLIKSKWGSKPSNSKSETTLEKLKGEIAHLKTSVDEITSGKGKLTDKERHRLL 60
           MSSRS KDLIKSKWGS+PS+SKS+T LEK KGEIA  KTS+DEITSGKGK+ +K R RLL
Sbjct: 1   MSSRSPKDLIKSKWGSRPSSSKSDTALEKFKGEIAAFKTSLDEITSGKGKMAEKGRSRLL 60

Query: 61  EKIRVLEAEKEKNAYQLTEKDKEIQRLRDQLKARYSTTALLEQLEETTREGERREQVLKA 120
           EKI+VLEAE+EKN Y L EKDKEIQRL+D L++RYS+++L EQLEE T+E E+++Q+L++
Sbjct: 61  EKIQVLEAEREKNVYYLLEKDKEIQRLKDHLRSRYSSSSLFEQLEEKTKECEKKQQLLES 120

Query: 121 LSEEKDVLKQQLSAATSRIAELESKTNTLRLSQTVAPNCFNSSINNIHEMEIQLKDALEK 180
           LS+E DVLK QLSA T R++ELESK +TL LSQ++  NCFNSS+N+IHE E+QLKDALEK
Sbjct: 121 LSKETDVLKNQLSATTKRLSELESKASTLHLSQSMPANCFNSSMNSIHEKEMQLKDALEK 180

Query: 181 NQQWLVYDQQREVYVKGLLAKIFELEKKTETAAHSLPQQTKKPESEGYLQEEKQKCYNDL 240
           NQQWLVYDQQRE YVKGLLAKIFELEK+TETAA SL QQ KK ESEGYLQ EKQK Y+ L
Sbjct: 181 NQQWLVYDQQREAYVKGLLAKIFELEKRTETAAASLTQQMKKIESEGYLQVEKQK-YDHL 239

Query: 241 LASAKKDLEVERQTITQLSFELSEFRRKYEETQKEVHNLNQLLYSQRRADVQHLEDDRHK 300
           L +AKKDLEVERQ +TQL  EL EFRRKYEE +KEV +LNQLL SQR+AD+QHLE+D+ K
Sbjct: 240 LENAKKDLEVERQAVTQLRLELDEFRRKYEEARKEVEDLNQLLSSQRKADIQHLEEDKQK 299

Query: 301 TEKIQKLREENDIARGKLEEEKKRSEELLSQVQFLYTSLLKQQEEQTRVALLEQQMQACT 360
           TE+IQKLREE+ I +GKLEEE+KRSEELLSQV+ LY SLLK QEEQ RVALLEQQMQACT
Sbjct: 300 TERIQKLREESSIFKGKLEEERKRSEELLSQVRILYDSLLKHQEEQARVALLEQQMQACT 359
```

```
Query: 361 LDFENEKLDRQHVQHQLHVILKELRKARNQITQLESLKQLHEFAITEPLVTFQGETENRE 420
           LDFENEKLDRQ++QHQL+VILKELRKA++QITQLESLKQLH F ITE     Q E E+R
Sbjct: 360 LDFENEKLDRQNMQHQLYVILKELRKAKSQITQLESLKQLHGFTITEQPFPLQREPESRV 419

Query: 421 KVAASPKSPTAALNESLVECPKCNIQYPATEHRDLLVHVEYCSK 464
           K A SPKSP+AALN+SLVECPKC++QYPATEHRDLLVHVEYC K
Sbjct: 420 K-ATSPKSPSAALNDSLVECPKCSVQYPATEHRDLLVHVEYCMK 462
```

**Figure 4G. Amino acid sequence alignment of 121P2A3 v.1 with human nef-associated factor 1.**

```
>gi|5174609|ref|NP_006049.1| (NM_006058) Nef-associated factor 1 [Homo
sapiens]
 gi|3758821|emb|CAA09856.1| (AJ011896) Naf1 beta protein [Homo sapiens]
          Length = 635

 Score = 45.4 bits (106), Expect = 0.001
 Identities = 79/339 (23%), Positives = 139/339 (40%), Gaps = 55/339
(16%)

Query: 83  EIQRLRDQLKARYSTTALLEQLEETTREG------------ERREQVLKALSEEKDVLKQ 130
           E  RL  ++      T++L+ L E  R+             E+R+Q  + L EE   LK+
Sbjct: 190 EFNRLASKVHKNEQRTSILQTLCEQLRKENEALKAKLDKGLEQRDQAAERLREENLELKK 249

Query: 131 QLSA-----------------ATSRIAELESKTNTLRLSQTVAPNCFNSSINNIHEMEIQ 173
            L +                  T + A    +   ++  +         ++  +  +E Q
Sbjct: 250 LLMSNGNKEGASGRPGSPKMEGTGKKAVAGQQQASVTAGKVPEVVALGAAEKKVKMLEQQ 309

Query: 174 LKDALEKNQQWLVYDQQREVYVKGLLAKIFELEKKTETAAHSLPQQTKKPESEGYLQEEK 233
             + LE N+QW   DQ      +    KI EL +K       L +Q    E+E   +E+K
Sbjct: 310 RSELLEVNKQW---DQHFRSMKQQYEQKITELRQKLA----DLQKQVTDLEAE---REQK 359

Query: 234 QKCYNDLLASAKKDLEVERQTITQLSFELSEFRRKYEETQKEVHNLNQLLYSQRRADVQH 293
           Q+ ++  L   AK +E+E     QL+ E  E R+K +  Q ++  L +    Q + ++Q
Sbjct: 360 QRDFDRKLLLAKSKIEMEETDKEQLTAEAKELRQKVKYLQDQLSPLTRQREYQEK-EIQR 418

Query: 294 LEDDRHKTEKIQKLREENDIARGKLEEEKKRSEELLSQVQFLYTSLLKQQEEQTRVALLE 353
           L     +   IQ        A G  E                  +LL++QE  T+  LL+
Sbjct: 419 LNKALEEALSIQTPPSSPPTAFGSPEGAG---------------ALLRKQELVTQNELLK 463

Query: 354 QQMQACTLDFENEKLDRQHVQHQLHVILKELRKARNQIT 392
           QQ++    DF+ E+ DR+ +  +   + K++ K + Q+T
Sbjct: 464 QQVKIFEEDFQRERSDRERMNEEKEELKKQVEKLQAQVT 502
```

## Figure 4H. Comparison to Mouse FLJ10540

```
 Score =  479 bits (1233), Expect = e-134
 Identities = 349/464 (75%), Positives = 404/464 (86%), Gaps = 2/464 (0%)

Query: 1   MSSRSTKDLIKSKWGSKPSNSKSETTLEKLKGEIAHLKTSVDEITSGKGKLTDKERHRLL 60
            MSSRS KDLIKSKWGS+PS+SKS+T LEK KGEIA  KTS+DEITSGKGK+ +K R RLL
Sbjct: 1   MSSRSPKDLIKSKWGSRPSSSKSDTALEKFKGEIAAFKTSLDEITSGKGKMAEKGRSRLL 60

Query: 61  EKIRVLEAEKEKNAYQLTEKDKEIQRLRDQLKARYSTTALLEQLEETTREGERREQVLKA 120
            EKI+VLEAE+EKN Y L EKDKEIQRL+D L++RYS++L EQLEE T+E E+++Q+L++
Sbjct: 61  EKIQVLEAEREKNVYYLLEKDKEIQRLKDHLRSRYSSSSLFEQLEEKTKECEKKQQLLES 120

Query: 121 LSEEKDVLKQQLSAATSRIAELESKTNTLRLSQTVAPNCFNSSINNIHEMEIQLKDALEK 180
            LS+E DVLK QLSA T R++ELESK +TL LSQ++  NCFNSS+N+IHE E+QLKDALEK
Sbjct: 121 LSKETDVLKNQLSATTKRLSELESKASTLHLSQSMPANCFNSSMNSIHEKEMQLKDALEK 180

Query: 181 NQQWLVYDQQREVYVKGLLAKIFELEKKTETAAHSLPQQTKKPESEGYLQEEKQKCYNDL 240
            NQQWLVYDQQRE YVKGLLAKIFELEK+TETAA SL QQ KK ESEGYLQ EKQK Y+ L
Sbjct: 181 NQQWLVYDQQREAYVKGLLAKIFELEKRTETAAASLTQQMKKIESEGYLQVEKQK-YDHL 239

Query: 241 LASAKKDLEVERQTITQLSFELSEFRRKYEETQKEVHNLNQLLYSQRRADVQHLEDDRHK 300
            L +AKKDLEVERQ +TQL  EL EFRRKYEE +KEV +LNQLL SQR+AD+QHLE+D+ K
Sbjct: 240 LENAKKDLEVERQAVTQLRLELDEFRRKYEEARKEVEDLNQLLSSQRKADIQHLEEDKQK 299

Query: 301 TEKIQKLREENDIARGKLEEEKKRSEELLSQVQFLYTSLLKQQEEQTRVALLEQQMQACT 360
            TE+IQKLREE+ I +GKLEEE+KRSEELLSQV+ LY SLLK QEEQ RVALLEQQMQACT
Sbjct: 300 TERIQKLREESSIFKGKLEEERKRSEELLSQVRILYDSLLKHQEEQARVALLEQQMQACT 359

Query: 361 LDFENEKLDRQHVQHQLHVILKELRKARNQITQLESLKQLHEFAITEPLVTFQGETENRE 420
            LDFENEKLDRQ++QHQL+VILKELRKA++QITQLESLKQLH F ITE     Q E E+R
Sbjct: 360 LDFENEKLDRQNMQHQLYVILKELRKAKSQITQLESLKQLHGFTITEQPFPLQREPESRV 419

Query: 421 KVAASPKSPTAALNESLVECPKCNIQYPATEHRDLLVHVEYCSK 464
            K A SPKSP+AALN+SLVECPKC++QYPATEHRDLLVHVEYC K
Sbjct: 420 K-ATSPKSPSAALNDSLVECPKCSVQYPATEHRDLLVHVEYCMK 462
```

**Figure 4I. Comparison to mouse Rho/rac interacting citron kinase**

```
Score = 47.8 bits (112), Expect = 3e-04
 Identities = 84/405 (20%), Positives = 172/405 (41%), Gaps = 39/405 (9%)

Query: 1   MSSRSTKDLIKSKWGSKPSNSKSETTLEKLKGEIAHLKTSVDEITSGKGKLTDKERHRLL 60
           M ++  +DL+ ++   +    +SE    +L  E    K +  +    K K   K
Sbjct: 566 MMNQLEEDLVSAR--RRSDLYESELRESRLAAEEFKRKANECQHKLMKAKDQGKPEVGEY 623

Query: 61  EKIRVLEAEKEKNAYQLTEKDKEIQRLRDQLKARYSTTALLEQLEETTREGERREQVLKA 120
            K+  + AE++    +L EK      L   +KA    T LL+ + +     ER  + L
Sbjct: 624 SKLEKINAEQQLKIQELQEK------LEKAVKASTEATELLQNIRQAKERAERELEKLHN 677

Query: 121 LSEEKDVLKQQLSAATSRIAELESKTN---TLRLSQTVAPNCFNSSINNIHEMEIQLKDA 177
             +  + +K++L  A  R   LE+K     T+   +    +   +    I +M  ++ +
Sbjct: 678 REDSSEGIKKKLVEAEERRHSLENKVKRLETMERRENRLKDDIQTKSEQIQQMADKILEL 737

Query: 178 LEKNQQWLVYDQQREVYVKGLLAKIFELEKKTETAAHSLPQQTKKPESEGYLQEEKQKCY 237
            EK+++  V  Q  EV++K       + E+  E     L  Q KK  ++    E   + +
Sbjct: 738 EEKHREAQVSAQHLEVHLK-------QKEQHYEEKIKVLDNQIKKDLADKESLENMMQRH 790

Query: 238 NDLLASAKKDLEVERQTITQLSFELSEF-RRKYEETQKEVHNLNQLLYSQRRADVQHLED 296
            +      K L  ++  I  +  ++    +R  E ++      N  L++QR    Q
Sbjct: 791 EEEAHEKGKILSEQKAMINAMDSKIRSLEQRIVELSEANKLAANSSLFTQRNMKAQE--- 847

Query: 297 DRHKTEKIQKLREEN---DIARGKLEEEKKRSEELLSQVQFLYTSLLKQQEEQTRVALLE 353
                E I +LR++    +   GKLE + ++ EE L ++        +   +++R+  LE
Sbjct: 848 -----EMISELRQQKFYLETQAGKLEAQNRKLEEQLEKISH------QDHSDKSRLLELE 896

Query: 354 QQMQACTLDFENEKLDRQHVQHQLHVILKELRKARNQITQLESLK 398
            +++  +L+ E +KL+   ++ QL  +   L++  +Q+T L++ +
Sbjct: 897 TRLREVSLEHEEQKLE---LKRQLTELQLSLQERESQLTALQAAR 938
```

# Figure 5
## 121P2A3 Hydrophilicity profile
(Hopp T.P., Woods K.R., 1981. Proc. Natl. Acad. Sci. U.S.A. 78:3824-3828)

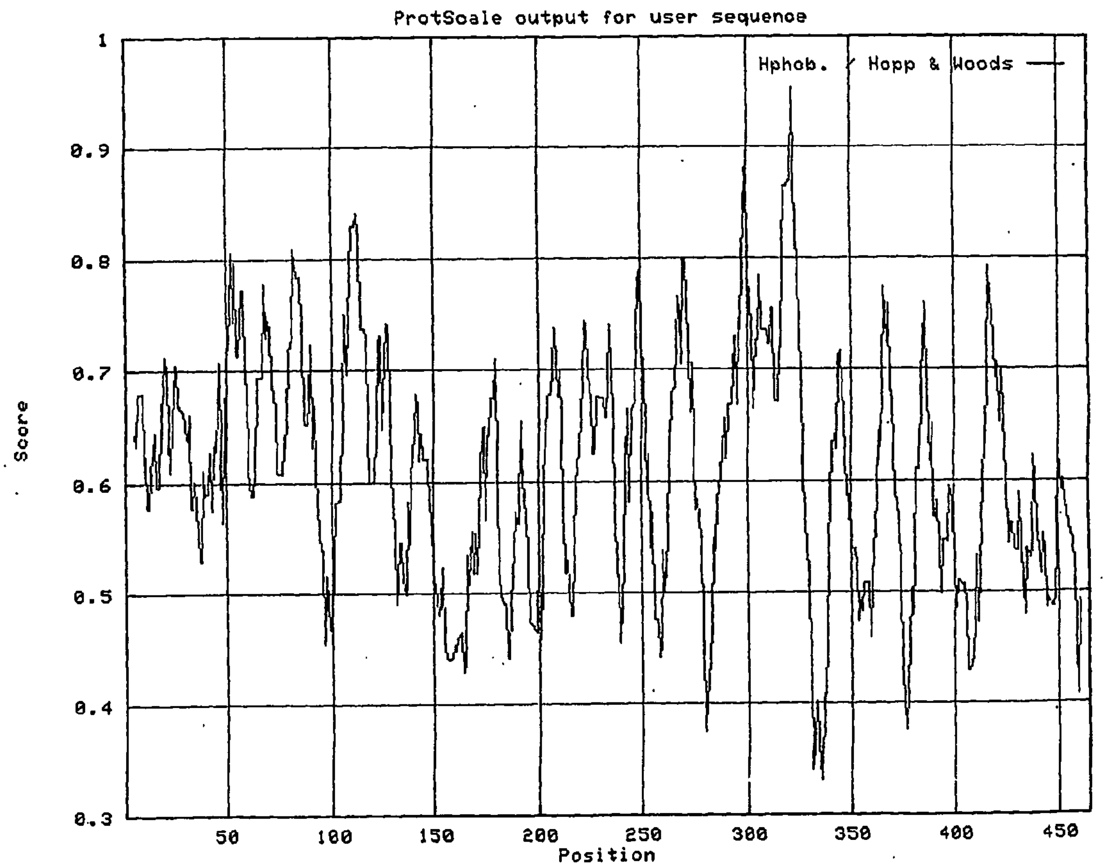

# Figure 6
## 121P2A3 Hydropathicity Profile
(Kyte J., Doolittle R.F., 1982. J. Mol. Biol. 157:105-132).

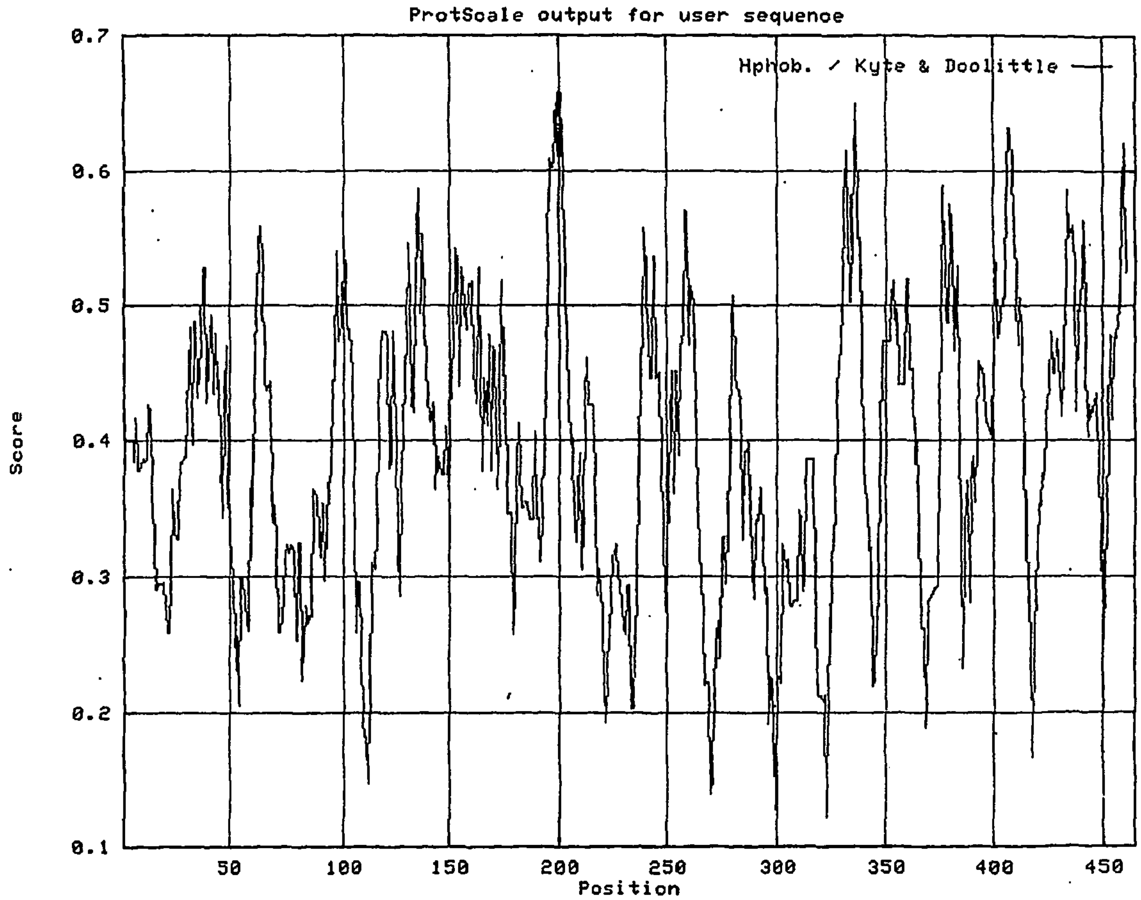

# Figure 7
# 121P2A3 % Accessible Residues Profile
(Janin J., 1979. Nature 277:491-492)

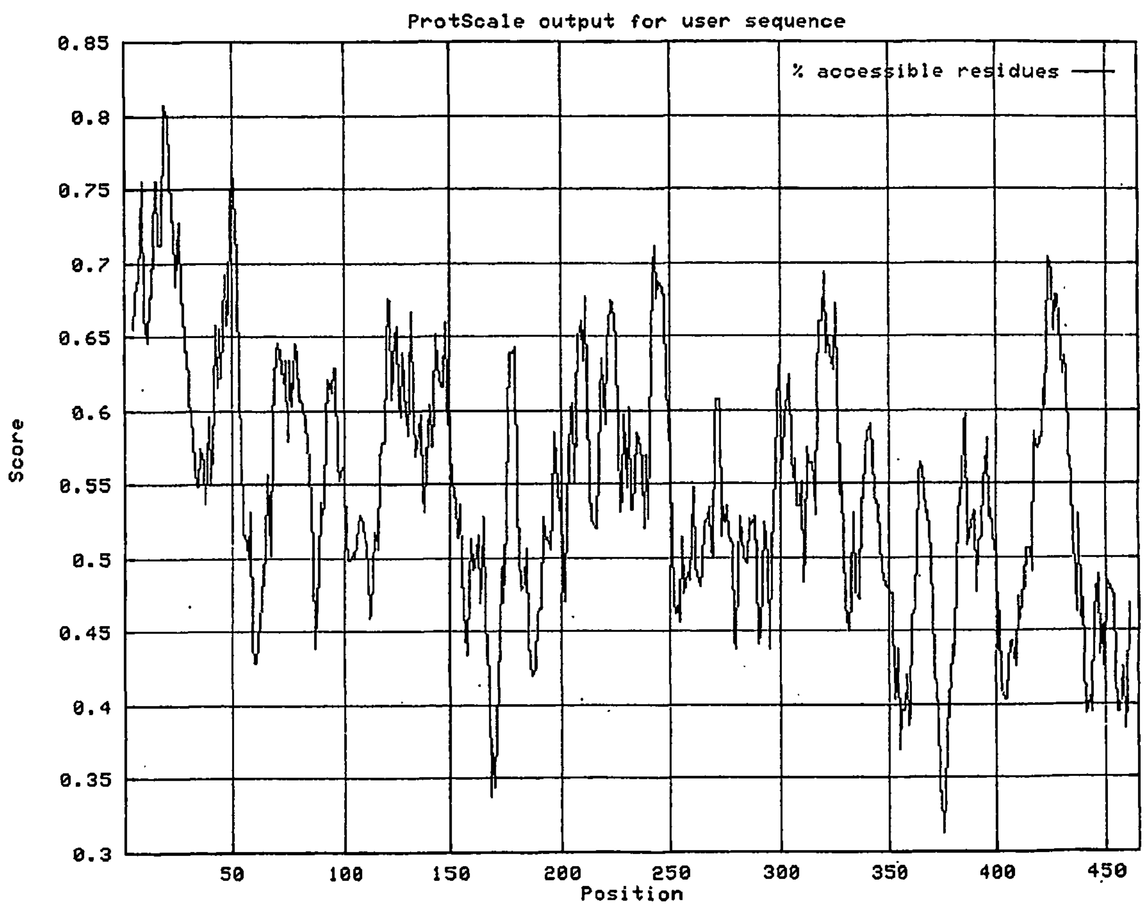

# Figure 8
## 121P2A3 Average Flexibility Profile
(Bhaskaran R., Ponnuswamy P.K., 1988.
Int. J. Pept. Protein Res. 32:242-255)

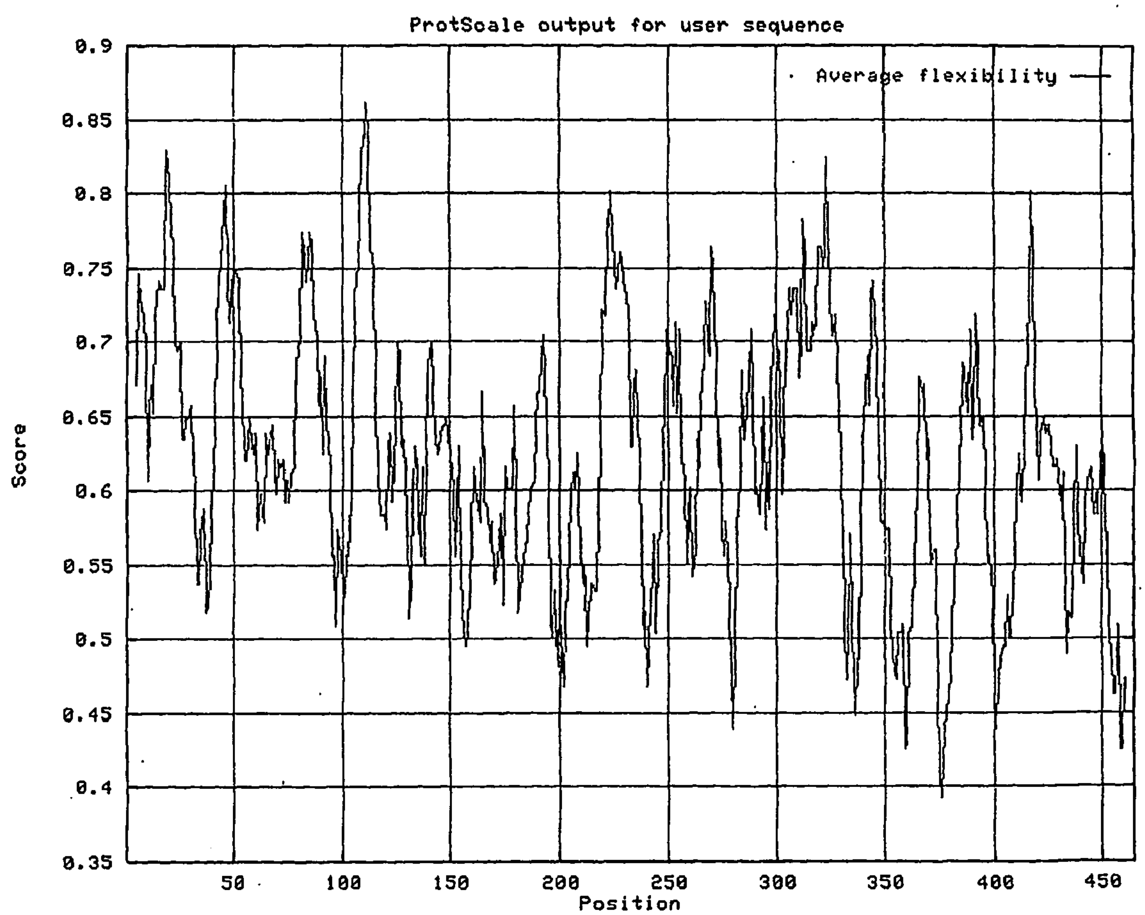

# Figure 9
## 121P2A3 Beta-turn Profile
(Deleage, G., Roux B. 1987. Protein Engineering 1:289-294)

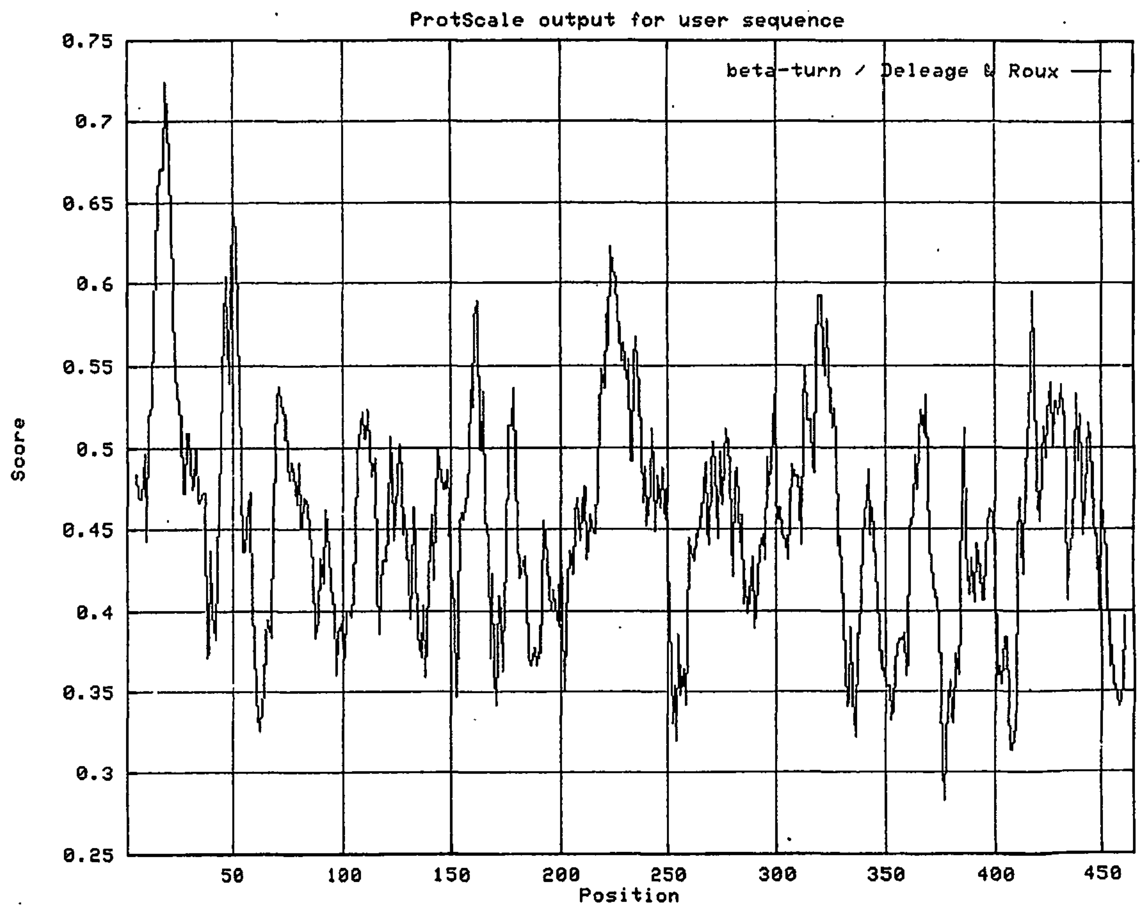

# Figure 10

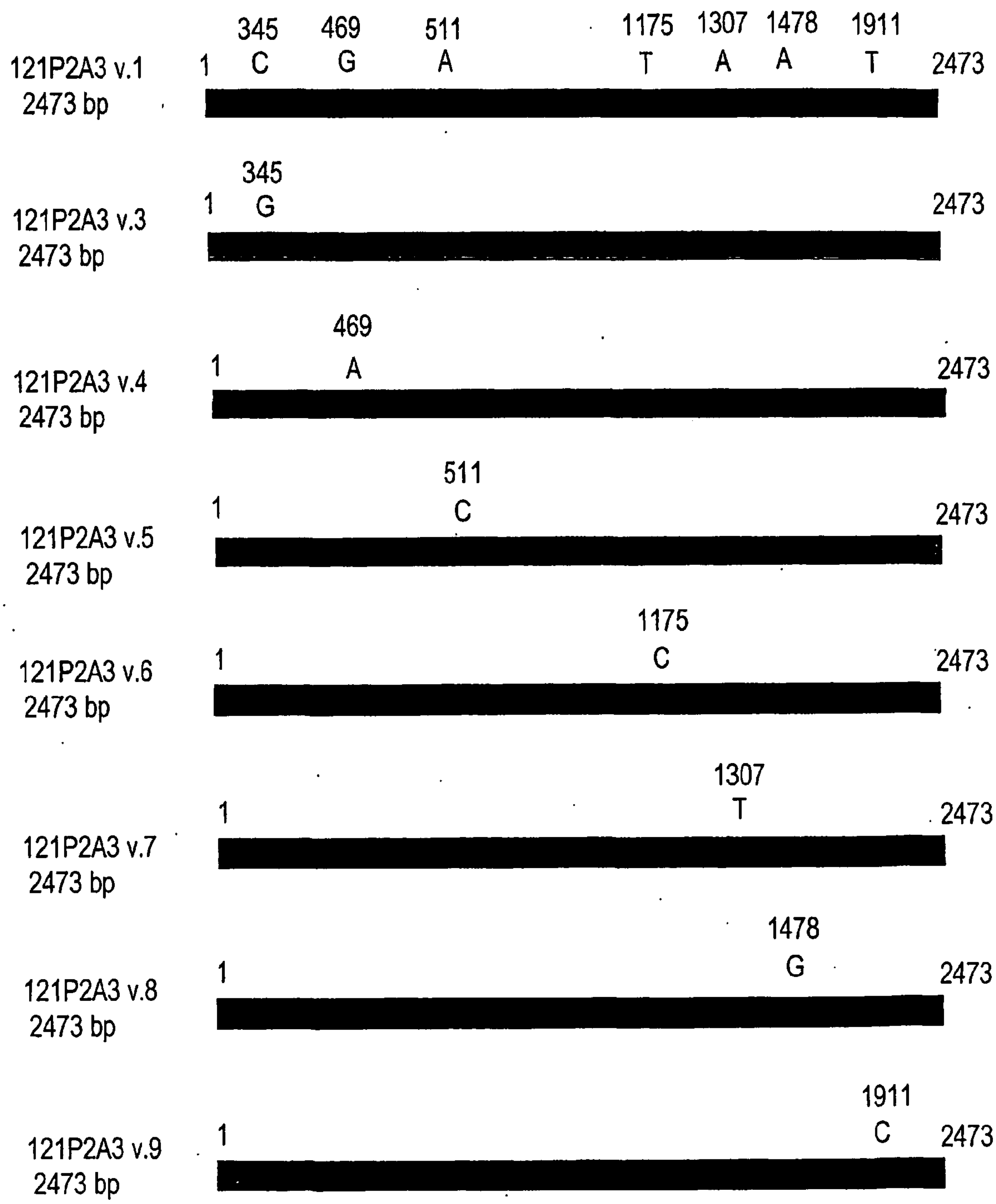
121P2A3 v.1
2473 bp
1
345 C
469 G
511 A
1175 T
1307 A
1478 A
1911 T
2473
121P2A3 v.3
2473 bp
1
345 G
2473
121P2A3 v.4
2473 bp
1
469 A
2473
121P2A3 v.5
2473 bp
1
511 C
2473
121P2A3 v.6
2473 bp
1
1175 C
2473
121P2A3 v.7
2473 bp
1
1307 T
2473
121P2A3 v.8
2473 bp
1
1478 G
2473
121P2A3 v.9
2473 bp
1
1911 C
2473

# Figure 11

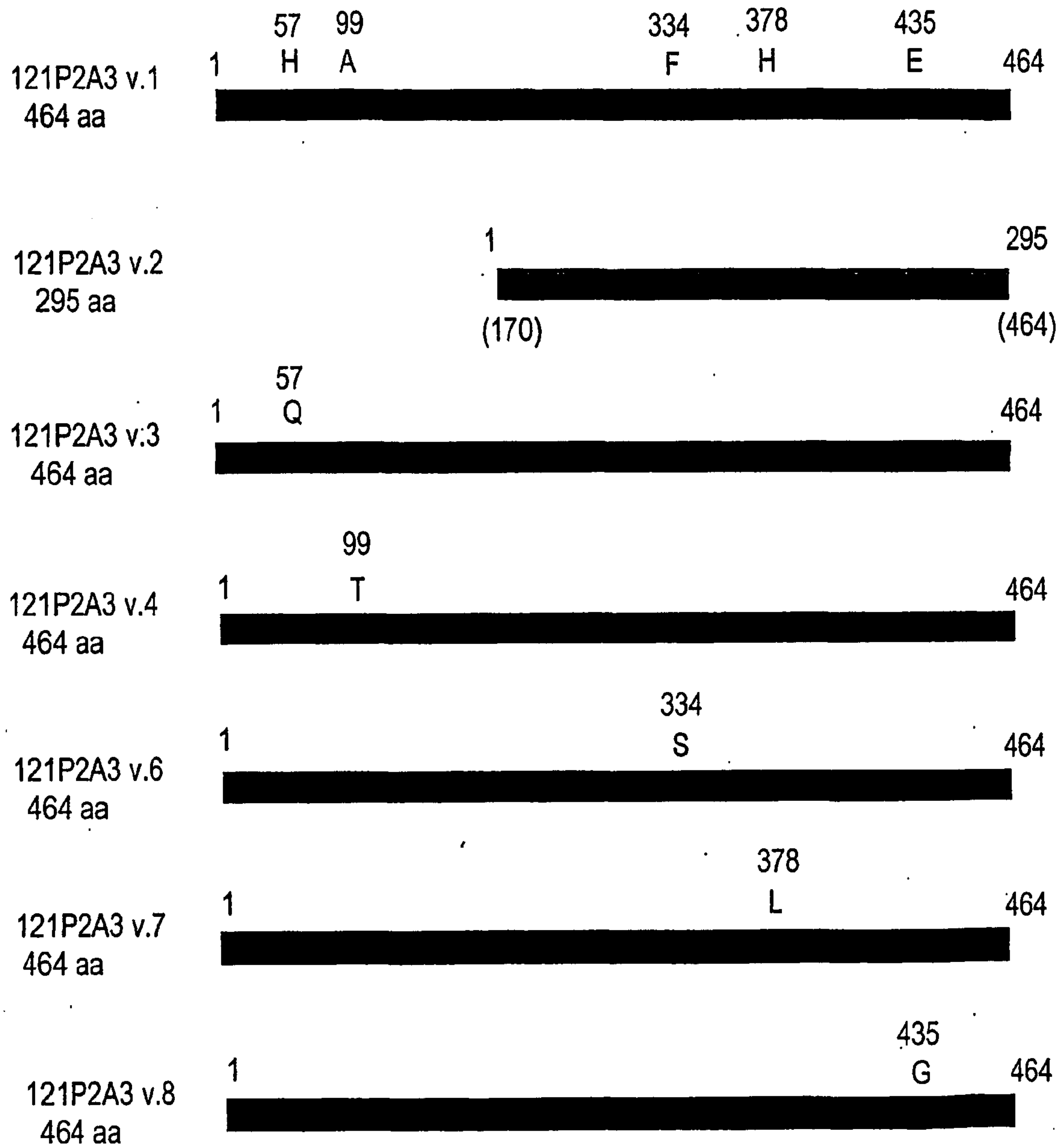
121P2A3 v.1
464 aa
1
57
H
99
A
334
F
378
H
435
E
464
121P2A3 v.2
295 aa
1
295
(170)
(464)
121P2A3 v.3
464 aa
1
57
Q
464
121P2A3 v.4
464 aa
1
99
T
464
121P2A3 v.6
464 aa
1
334
S
464
121P2A3 v.7
464 aa
1
378
L
464
121P2A3 v.8
464 aa
1
435
G
464

# Figure 12

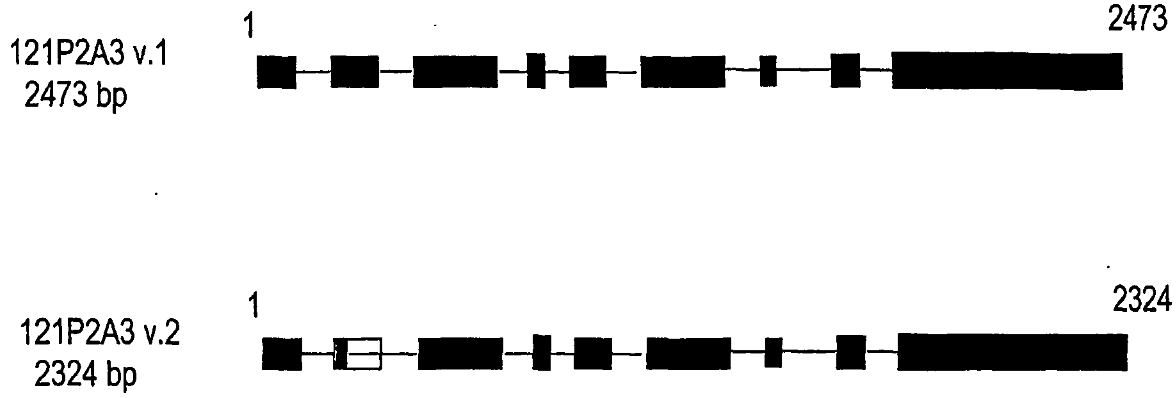

# Figure 13 Secondary structure prediction of 121P2A3

```
         10         20         30         40         50         60         70
          |          |          |          |          |          |          |
MSSRSTKDLIKSKWGSKPSNSKSETTLEKLKGEIAHLKTSVDEITSGKGKLTDKERHRLLEKIRVLEAEK
cccchhhhhhhhhcccccccccchhhhhhccchheeeecchhhhcccccccchhhhhhhhhhhhhhhhhhh
EKNAYQLTEKDKEIQRLRDQLKARYSTTALLEQLEETTREGERREQVLKALSEEKDVLKQQLSAATSRIA
hccccccchchhhhhhhhhhhhcccchhhhhhhhhhhhcccchhhhhhhhhhhhhhhhhhhhhhhhhhhh
ELESKTNTLRLSQTVAPNCFNSSINNIHEMEIQLKDALEKNQQWLVYDQQREVYVKGLLAKIFELEKKTE
hhhccccceeeeecccccchhhhhhhhhhhhhhhhhhhccceeehhhhhhhhhhhhhhhhhhhhhcccc
TAAHSLPQQTKKPESEGYLQEEKQKCYNDLLASAKKDLEVERQTITQLSFELSEFRRKYEETQKEVHNLN
chhccccccccccccccchhhhhhhhhhhhhhhhhhhhhhhhhhhhhhhhhhhhhhhhhhhhhhhhhhhh
QLLYSQRRADVQHLEDDRHKTEKIQKLREENDIARGKLEEEKKRSEELLSQVQFLYTSLLKQQEEQTRVA
hhhhhhcccccccccccccchhhhhhhhhhhhhhhhcchhhcchhhhhhhhhhhhhhhhhchhhhhhhhh
LLEQQMQACTLDFENEKLDRQHVQHQLHVILKELRKARNQITQLESLKQLHEFAITEPLVTFQGETENRE
hhhhhhhhhcccchhhhhhhhhhhhhhhhhhhhhhhhhhhhhchhhhhhhhhheehcccceeecccccccc
KVAASPKSPTAALNESLVECPKCNIQYPATEHRDLLVHVEYCSK
cccccccccchccchchhccccccccccchhhhhhheeeeeccc
```

```
Alpha helix      (Hh) :  296 is  63.79%
Extended strand  (Ee) :   22 is   4.74%
Random coil      (Cc) :  146 is  31.47%
```

# Figure 14. 121P2A3 Expression by RT-PCR

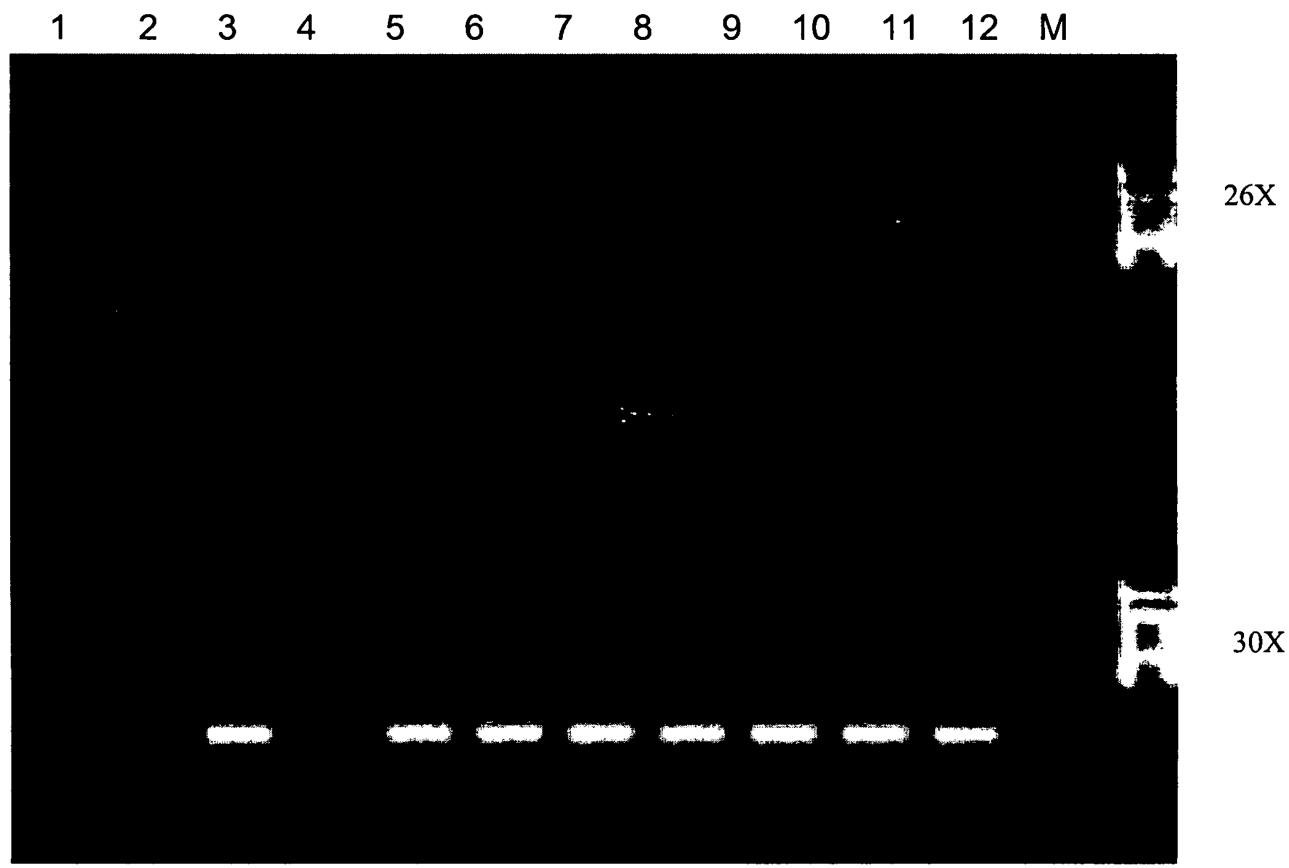


1. Vital Pool 1
2. Vital Pool 2
3. Xenograft cancer pool
4. Prostate cancer pool
5. Bladder Cancer Pool
6. Kidney cancer Pool
7. Colon Cancer Pool
8. Lung Cancer Pool
9. Ovary Cancer Pool
10. Breast Cancer Pool
11. Metastasis Cancer Pool
12. H2O

M = Marker

**<u>Figure 15</u> Expression of 121P2A3 in Normal Tissues and in Prostate Cancer Xenografts**

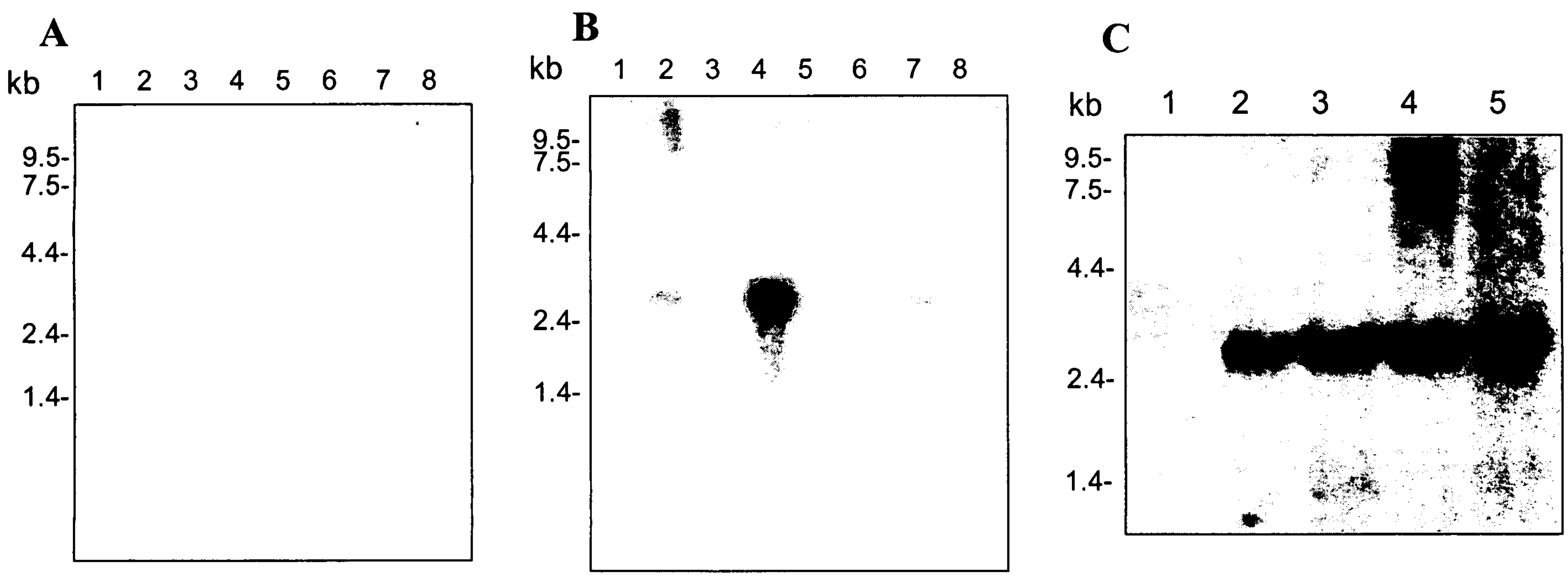


**A**
1. Heart
2. Brain
3. Placenta
4. Lung
5. Liver
6. Skeletal Muscle
7. Kidney
8. Pancreas

**B**
1. Spleen
2. Thymus
3. Prostate
4. Testis
5. Ovary
6. Small Intestine
7. Colon
8. Leukocytes

**C**
1. Normal Prostate
2. LAPC-4 AD
2. LAPC-4 AI
3. LAPC-9 AD
4. LAPC-9 AI

**Figure 16** Expression of 121P2A3 in Human Cancer Cell Lines

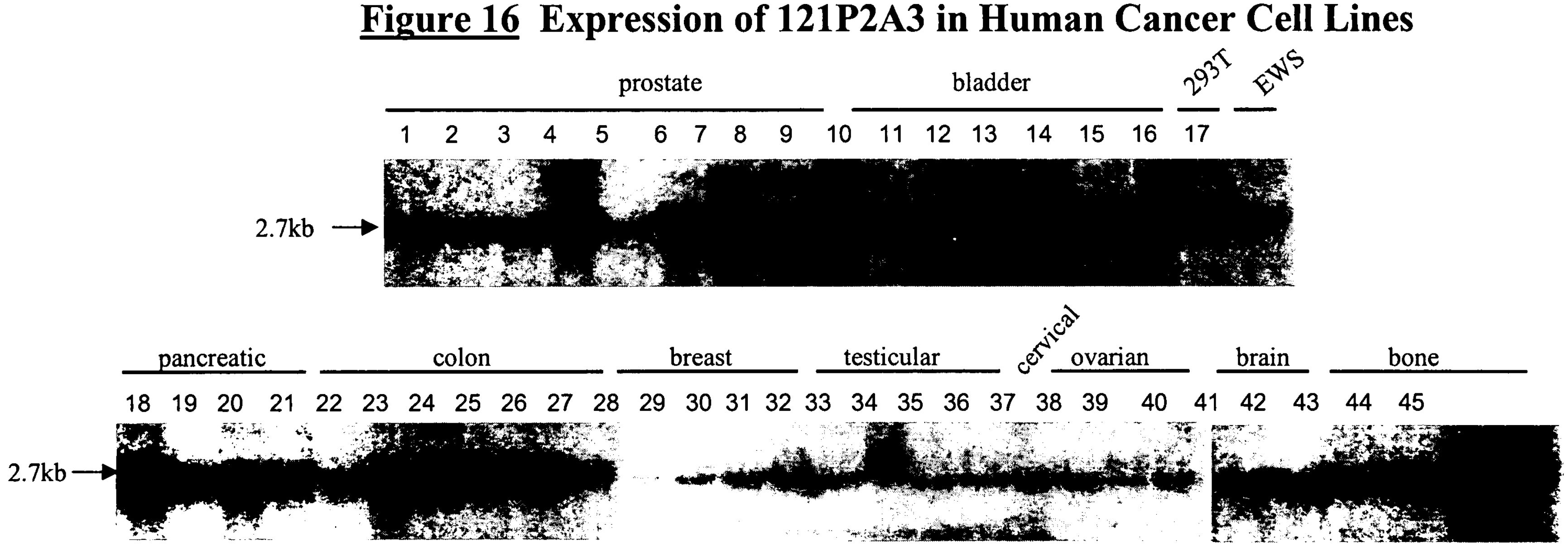


1. LAPC-4 AD
2. LAPC-4 AI
3. LAPC-9 AD
4. LAPC-9 AI
5. LNCaP
6. PC-3
7. DU145
8. TsuPr1
9. LAPC-4 CL
10. HT1197
11. SCaBER
12. UM-UC-3
13. TCCSUP
14. J82
15. 5637
16. 293T
17. RD-ES
18. PANC-1
19. BxPC-3
20. HPAC
21. Capan-1
22. SK-CO-1
23. CaCo-2
24. LoVo
25. T84
26. Colo-205
27. KCL 22
28. CAMA-1
29. DU4475
30. MCF-7
31. MDA-MB-435s
32. NTERRA-2
33. NCCIT
34. TERA-1
35. TERA-2
36. A431
37. OV-1063
38. PA-1
39. SW 626
40. PFSK-1
41. T98G
42. SK-ES-1
43. HOS
44. U-2 OS
45. RD-ES

# Figure 17 Expression of 121P2A3 in Bladder Cancer Patient Specimens

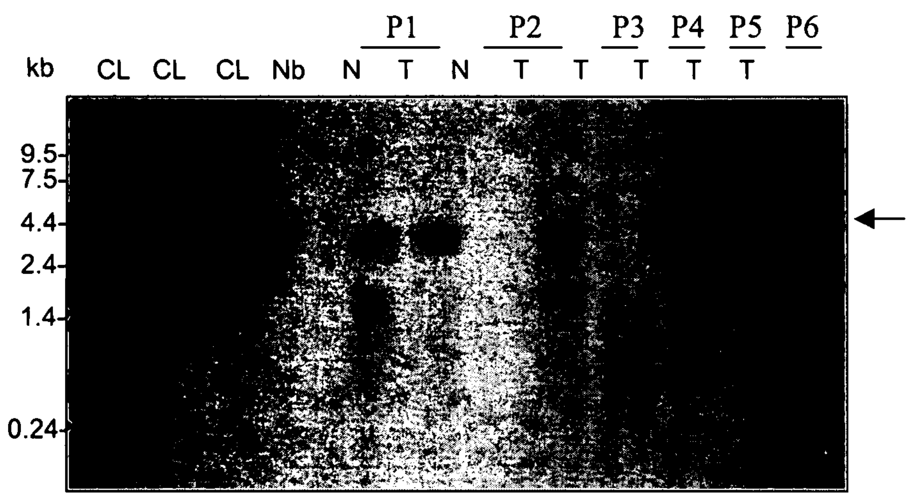


P1 - Transitional, grade 4
P2 - Squamous inv.
P3 - Transitional, grade 3
P4 - Papillary non -inv, grade 1/3
P5 - Papillary, grade 3/3
P6 - Transitional, grade 3/2

*CL = Cell lines (from left to right): UM-UC-3, J82, SCABER*
*P = Patient*
*Nb = Normal Bladder*
*N = Normal adjacent tissue*
*T = Tumor*

# Figure 18 Expression of 121P2A3 in Kidney Patient Cancer Specimens

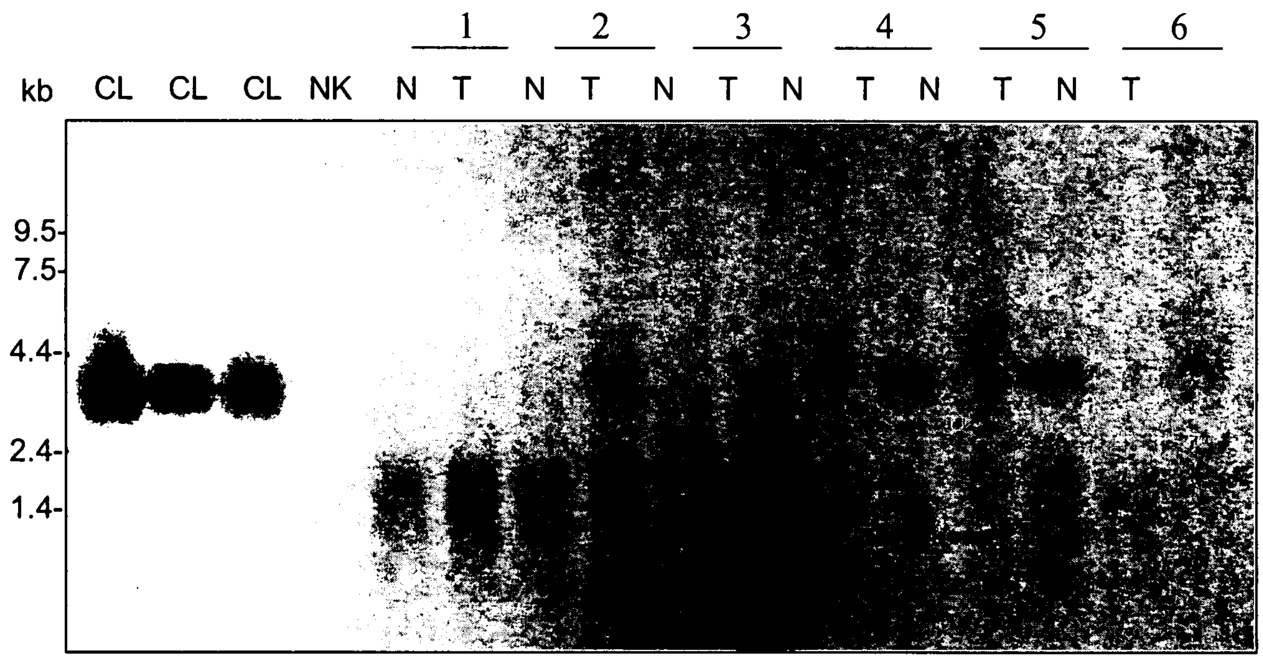


*CL = Cell lines (from left to right):* 769-P, A498, SW839
*NK = Normal kidney*
*N = Normal adjacent tissue*
*T = Tumor*
Patient 1- Papillary Type, Stage I, Grade 2/4
Patient 2- Invasive papillary carcinoma, Grade 2/4
Patient 3- Clear cell type Grade 1/3, focally 2/3
Patient 4- Clear cell type, stage III, Grade 2/4
Patient 5- Clear cell type, stage III, Grade 3/4
Patient 6- Clear cell type, stage III, Grade 3/4

# Figure 19 Expression of 121P2A3 in Stomach and Rectum Patient Cancer Specimens

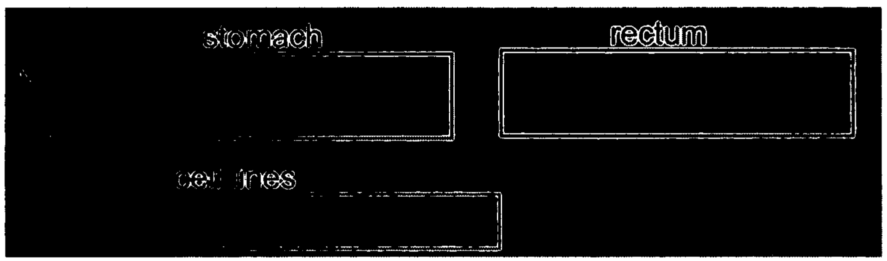


*T = tumor RNA*
*N = normal adjacent tissue RNA*

***Cancer cell lines are:***
*(from left to right)*

HeLa (cervical carcinoma)
Daudi (Burkitt's lymphoma)
K562 (CML)
HL-60 (PML)
G361 (melanoma)
A549 (lung carcinoma)
MOLT-4 (lymphoblastic leuk.)
SW480 (colorectal carcinoma)
Raji (Burkitt's lymphoma)

# Figure 20 Androgen Regulation of 121P2A3

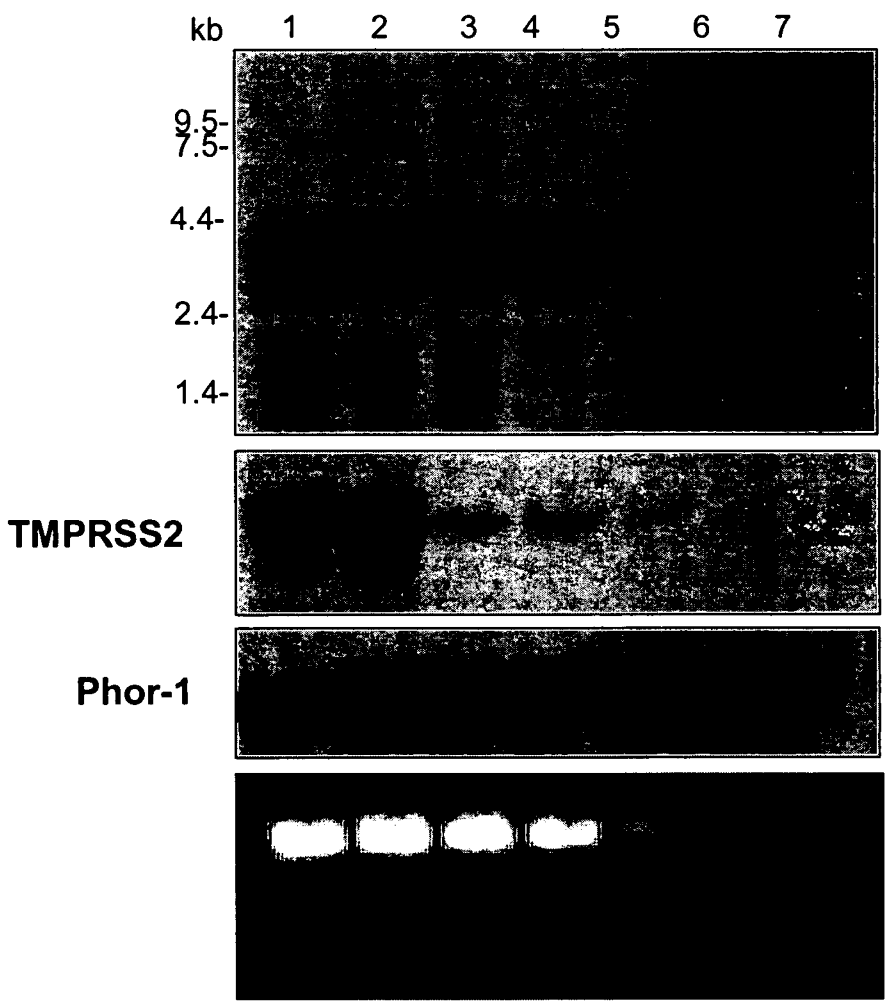


1. LAPC-9AD Day 0
2. LAPC-9AD Day 0
3. LAPC-9AD Day 7
4. LAPC-9AD Day 7
5. LAPC-9AD Day 15
6. LAPC-9AD Day 15
7. LAPC-9AD Day 21

**<u>Figure 21.</u> 121P2A3 Expression in 293T Cells Following Transfection**

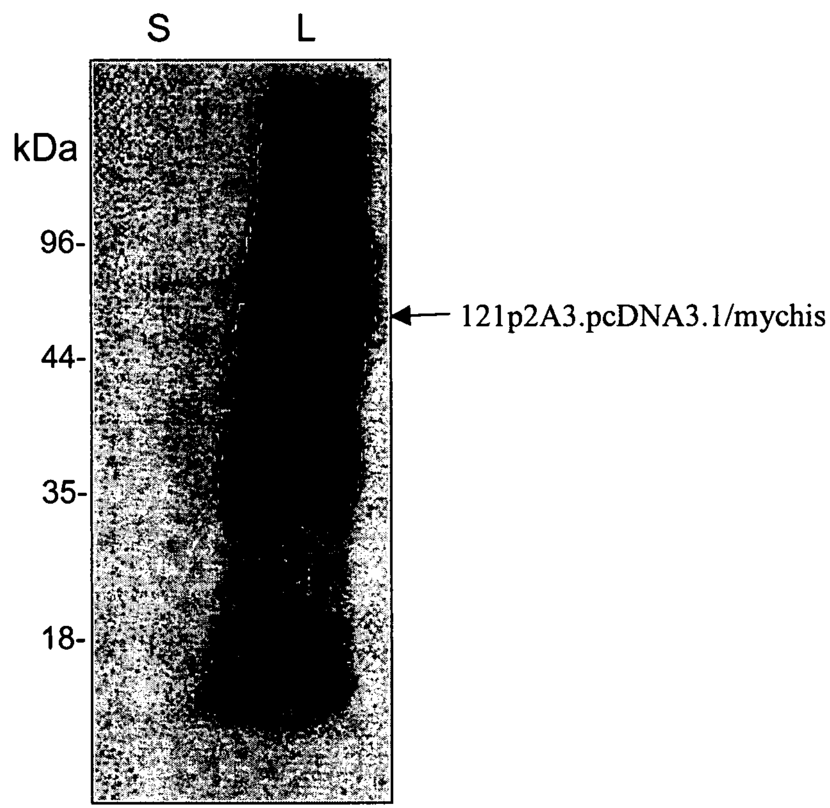


*S = Supernatant*
*L= Lysate*

European Patent Office

# EUROPEAN SEARCH REPORT

Application Number
EP 07 00 1331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/22920 A2 (HUMAN GENOME SCIENCES, INC; RUBEN, STEVEN, M; BARASH, STEVEN, C; BIRSE) 5 April 2001 (2001-04-05) SEQ ID NO: 1977, 6254 ----- | 1-10 | INV. C07K14/47 C12N15/12 C07K16/00 A61K38/00 A61K39/00 |
| E | WO 02/31111 A2 (HYSEQ, INC; TANG, Y. TOM; LIU, CHENGHUA; ZHOU, PING; ASUNDI, VINOD; ZH) 18 April 2002 (2002-04-18) SEQ ID NO: 289, 735 ----- | 1-10 | |
| E | WO 02/072757 A2 (CURAGEN CORPORATION; PADIGARU, MURALIDHARA; SPYTEK, KIMBERLY, A; SHENO) 19 September 2002 (2002-09-19) * page 212 - page 214 * ----- | 1-10 | |
| L | DATABASE EMBL [Online] 17 December 2003 (2003-12-17), "Sequence 11575 from Patent EP1074617." XP002316079 retrieved from EBI accession no. EM_PRO:AX876670 Database accession no. AX876670 * document is cited to provide SEQ ID NO: 11575 disclosed in EP1074617 * * the whole document * | 1-10 | TECHNICAL FIELDS SEARCHED (IPC) C07K C12N |
| X | -& EP 1 074 617 A (RESEARCH ASSOCIATION FORBIOTECHNOLOGY) 7 February 2001 (2001-02-07) SEQ ID NO: 11575, 11576 ----- -/-- | 1-10 | |

The present search report has been drawn up for all claims

| Place of search | Date of completion of the search | Examiner |
|---|---|---|
| The Hague | 18 April 2007 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 00 1331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | DATABASE EPO Proteins [Online] 17 December 2003 (2003-12-17), "Sequence 11576 from Patent EP1074617." XP002316080 retrieved from EBI accession no. EPOP:AX876671 Database accession no. AX876671 * document is cited to provide SEQ ID NO: 11576 disclosed in EP1074617 * * the whole document * | 1-10 | |
| X | & EP 1 074 617 A (RESEARCH ASSOCIATION FORBIOTECHNOLOGY) 7 February 2001 (2001-02-07) SEQ ID NO: 11575, 11576 ----- | 1-10 | |
| A | DATABASE EMBL [Online] 22 February 2000 (2000-02-22), "Homo sapiens cDNA FLJ10540 fis, clone NT2RP2001245." XP002316084 retrieved from EBI accession no. EM_PRO:AK001402 Database accession no. AK001402 * the whole document * ----- | 1-10 | |
| | -/-- | | TECHNICAL FIELDS SEARCHED (IPC) |

The present search report has been drawn up for all claims

| Place of search | Date of completion of the search | Examiner |
|---|---|---|
| The Hague | 18 April 2007 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

5

EPO FORM 1503 03.82 (P04C01)

European Patent Office

# EUROPEAN SEARCH REPORT

Application Number
EP 07 00 1331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE EMBL [Online] 16 May 2000 (2000-05-16), "Human DNA sequence from clone RP11-30E16 on chromosome 10 Contains the C10orf3 gene for chromosome 10 open reading frame 3, the RBP4 gene for retinol binding protein 4 (plasma), the 5' end of the PDE6C gene for phosphodiesterase 6C (cGMP-specific, cone, alpha prime), the GPR120 gene for G protein-co" XP002316085 retrieved from EBI accession no. EM_PRO:AL356214 Database accession no. AL356214 Positions 44100 - 45210 ----- | 1-10 | |
| A | DATABASE EMBL [Online] 8 February 2001 (2001-02-08), "Mus musculus adult male lung cDNA, RIKEN full-length enriched library, clone:1200008012 product:hypothetical protein, full insert sequence." XP002316086 retrieved from EBI accession no. EM_PRO:AK004655 Database accession no. AK004655 * the whole document * ----- | 1-10 | TECHNICAL FIELDS SEARCHED (IPC) |
| A | WO 00/73801 A2 (LUDWIG INSTITUTE FOR CANCER RESEARCH; OBATA, YUICHI) 7 December 2000 (2000-12-07) * the whole document * ----- | 1-10 | |
| | -/-- | | |

The present search report has been drawn up for all claims

| Place of search | Date of completion of the search | Examiner |
|---|---|---|
| The Hague | 18 April 2007 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 00 1331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LANTZ VALERIE A ET AL: "A class VI unconventional myosin is associated with a homologue of a microtubule-binding protein, cytoplasmic linker protein-170, in neurons and at the posterior pole of Drosophila embryos" JOURNAL OF CELL BIOLOGY, vol. 140, no. 4, 23 February 1998 (1998-02-23), pages 897-910, XP002315987 ISSN: 0021-9525 * the whole document * ----- | 1-10 | |
| A,D | FUKUSHI M ET AL: "Identification and cloning of a novel cellular protein Naf1, Nef-associated factor 1, that increases cell surface CD4 expression" FEBS LETTERS, ELSEVIER SCIENCE PUBLISHERS, AMSTERDAM, NL, vol. 442, no. 1, 8 January 1999 (1999-01-08), pages 83-88, XP004259009 ISSN: 0014-5793 * the whole document * ----- | 1-10 | TECHNICAL FIELDS SEARCHED (IPC) |
| A | YAMADA YOICHI ET AL: "Receptor for hyaluronan-mediated motility and CD44 expressions in colon cancer assessed by quantitative analysis using real-time reverse transcriptase-polymerase chain reaction" JAPANESE JOURNAL OF CANCER RESEARCH, vol. 90, no. 9, September 1999 (1999-09), pages 987-992, XP002316136 ISSN: 0910-5050 * the whole document * ----- | 1-10 | |

The present search report has been drawn up for all claims

| Place of search | Date of completion of the search | Examiner |
|---|---|---|
| The Hague | 18 April 2007 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 00 1331

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2007

| Patent document cited in search report | Publication date | Patent family member(s) | Publication date |
|---|---|---|---|
| WO 0122920 A2 | 05-04-2001 | AU 7721500 A | 30-04-2001 |
| | | CA 2384713 A1 | 05-04-2001 |
| | | EP 1265582 A2 | 18-12-2002 |
| WO 0231111 A2 | 18-04-2002 | AU 9623501 A | 22-04-2002 |
| | | CA 2425827 A1 | 18-04-2002 |
| | | EP 1325120 A2 | 09-07-2003 |
| WO 02072757 A2 | 19-09-2002 | NONE | |
| EP 1074617 A | 07-02-2001 | NONE | |
| WO 0073801 A2 | 07-12-2000 | AU 5047800 A | 18-12-2000 |
| | | EP 1259812 A2 | 27-11-2002 |
| | | JP 2003518364 T | 10-06-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 28273901 P
- US 28663001 P
- US 30037301 P
- WO 9733602 A
- US 4640835 A
- US 4496689 A
- US 4301144 A
- US 4670417 A
- US 4791192 A
- US 4179337 A
- US 5428130 A
- WO 9824893 A, Kucherlapati and Jakobovits
- US 6162963 A
- US 6150584 A
- US 6114598 A
- US 4736866 A
- US 4870009 A
- US 5837501 A
- US 5382510 A
- US 5952170 A
- US 5955280 A
- US 5925523 A
- US 5846722 A
- US 6004746 A
- US 5723286 A
- US 5733731 A
- US 5928868 A
- US 6146635 A
- US 5962428 A
- US 5580859 A
- US 5589466 A
- US 5804566 A
- US 5739118 A
- US 5736524 A
- US 5679647 A
- WO 9804720 A
- US 5922687 A
- US 4722848 A
- US 5416064 A
- US 5736142 A
- WO 9324640 A
- US 5279833 A
- WO 9106309 A
- US 5204253 A
- WO 9507707 A
- US 4235871 A
- US 4501728 A
- US 4837028 A
- US 5019369 A
- US 5840501 A
- US 5939533 A
- US 5919652 A
- WO 9816628 A
- US 6107540 A
- WO 9420127 A
- WO 9403205 A
- GB 2211504 A


### Non-patent literature cited in the description


- **KLEIN et al.** *Nat. Med.,* 1997, vol. 3, 402
- **SU et al.** *Proc. Natl. Acad. Sci. USA,* 1996, vol. 93, 7252
- **PINTO et al.** *Cancer Res,* September 1996, vol. 2 (9), 1445-51
- **HUBERT et al.** *Proc Natl Acad Sci U S A.,* 07 December 1999, vol. 96 (25), 14523-8
- **REITER et al.** *Proc. Natl. Acad. Sci. USA,* 1998, vol. 95, 1735
- **HOPP T.P. ; WOODS K.R.** *Proc. Natl. Acad. Sci. U.S.A.,* 1981, vol. 78, 3824-3828
- **KYTE J. ; DOOLITTLE R.F.** *J. Mol. Biol.,* 1982, vol. 157, 105-132
- **JANIN J.** *Nature,* 1979, vol. 277, 491-492
- **BHASKARAN R ; PONNUSWAMY P.K.** *Int. J. Pept. Protein Res.,* 1988, vol. 32, 242-255
- **DELEAGE, G. ; ROUX B.** *Protein Engineering,* 1987, vol. 1, 289-294
- **SAMBROOK et al.** Molecular Cloning: A Laboratory Manual. Cold Spring Harbor Laboratory Press, 1989
- **STITES et al.** IMMUNOLOGY. Lange Publishing, 1994
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **KRAJINOVIC et al.** *Mutat. Res.,* 1998, vol. 382 (3-4), 81-83

- **JOHANSSON et al.** *Blood,* 1995, vol. 86 (10), 3905-3914
- **FINGER et al.** *P.N.A.S.,* 1988, vol. 85 (23), 9158-9162
- **EVANS et al.** *Am. J. Obstet. Gynecol,* 1994, vol. 171 (4), 1055-1057
- **MARROGI et al.** *J. Cutan. Pathol.,* 1999, vol. 26 (8), 369-378
- **JACK COHEN.** Oligodeoxynucleotides, Antisense Inhibitors of Gene Expression. CRC Press, 1989
- *Synthesis,* 1988, vol. 1, 1-5
- **IYER, R. P. et al.** *J. Org. Chem.,* 1990, vol. 55, 4693-4698
- **IYER, R. P. et al.** *J. Am. Chem. Soc.,* 1990, vol. 112, 1253-1254
- **PARTRIDGE et al.** *Antisense & Nucleic Acid Drug Development,* 1996, vol. 6, 169-175
- **L. A. COUTURE ; D. T. STINCHCOMB.** *Trends Genet,* 1996, vol. 12, 510-515
- **SAMBROOK, J. et al.** Molecular Cloning: A Laboratory Manual. Cold Spring Harbor Press, 1989
- Current Protocols in Molecular Biology. Wiley and Sons, 1995
- Current Protocols in Molecular Biology. 1995
- **MULLER et al.** *MCB,* 1991, vol. 11, 1785
- **KOZAK.** *Mol. Cell Biol.,* 1989, vol. 9, 5073-5080
- **KOZAK.** *PNAS,* 1995, vol. 92 (7), 2662-2666
- **KOZAK.** *NAR,* 1987, vol. 15 (20), 8125-8148
- Biochemistry. Stanford University, 13-15
- **HENIKOFF et al.** *PNAS,* 1992, vol. 89, 10915-10919
- **LEI et al.** *J Biol Chem,* 19 May 1995, vol. 270 (20), 11882-6
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CREIGHTON.** The Proteins. W.H. Freeman & Co,
- **CHOTHIA.** *J. Mol. Biol.,* 1976, vol. 150, 1
- **NAIR et al.** *J. Immunol,* 2000, vol. 165 (12), 6949-6955
- **HEBBES et al.** *Mol Immunol,* 1989, vol. 26 (9), 865-73
- **SCHWARTZ et al.** *J Immunol,* 1985, vol. 135 (4), 2598-608
- **CHEN et al.** *Lab Invest.,* 1998, vol. 78 (2), 165-174
- **GAIDDON et al.** *Endocrinology,* 1995, vol. 136 (10), 4331-4338
- **HALL et al.** *Nucleic Acids Research,* 1996, vol. 24 (6), 1119-1126
- **PETERZIEL et al.** *Oncogene,* 1999, vol. 18 (46), 6322-6329
- **O'BRIAN.** *Oncol. Rep.,* 1998, vol. 5 (2), 305-309
- **DENNIS et al.** *Biochem. Biophys. Acta,* 1999, vol. 1473 (1), 21-34
- **RAJU et al.** *Exp. Cell Res.,* 1997, vol. 235 (1), 145-154
- **TRESTON et al.** *J. Natl. Cancer Inst. Monogr.,* 1992, 169-175
- **SETTE.** *Immunogenetics,* 1999, vol. 50 (3-4), 201-212
- **SETTE et al.** *J. Immunol.,* 2001, vol. 166 (2), 1389-1397
- **SIDNEY et al.** *Hum. Immunol.,* 1997, vol. 58 (1), 12-20
- **KONDO et al.** *Immunogenetics,* 1997, vol. 45 (4), 249-258
- **SIDNEY et al.** *J. Immunol.,* 1996, vol. 157 (8), 3480-90
- **FALK et al.** *Nature,* 1991, vol. 351, 290-6
- **HUNT et al.** *Science,* 1992, vol. 255, 1261-3
- **PARKER et al.** *J. Immunol.,* 1992, vol. 149, 3580-7
- **PARKER et al.** *J. Immunol.,* 1994, vol. 152, 163-75
- **KAST et al.** *J. IMMUNOL.,* 1994, vol. 152 (8), 3904-12
- **BORRAS-CUESTA et al.** *Hum. Immunol.,* 2000, vol. 61 (3), 266-278
- **ALEXANDER et al.** *J. Immunol.,* 2000, vol. 164 (3), 1625-1633
- **O'SULLIVAN et al.** *J. Immunol.,* 1991, vol. 147 (8), 2663-2669
- **ALEXANDER et al.** *Immunity,* 1994, vol. 1 (9), 751-761
- **ALEXANDER et al.** *Immunol. Res.,* 1998, vol. 18 (2), 79-92
- **HOPP, T.P. ; WOODS, K.R.** *Proc. Natl. Acad. Sci. U.S.A.,* 1981, vol. 78, 3824-3828
- **KYTE, J. ; DOOLITTLE, R.F.** *J. Mol. Biol.,* 1982, vol. 157, 105-132
- **BHASKARAN R. ; PONNUSWAMY P.K.** *Int. J. Pept. Protein Res.,* 1988, vol. 32, 242-255
- **XUE et al.** *Prostate,* 1997, vol. 30, 73-8
- **PESHWA et al.** *Prostate,* 1998, vol. 36, 129-38
- Antibodies: A Laboratory Manual. CSH Press, 1988
- **HARLOW.** Antibodies. Cold Spring Harbor Press, 1989
- **DONNELLY et al.** *Ann. Rev. Immunol.,* 1997, vol. 15, 617-648
- **JONES et al.** *Nature,* 1986, vol. 321, 522-525

- **RIECHMANN et al.** *Nature,* 1988, vol. 332, 323-327
- **VERHOEYEN et al.** *Science,* 1988, vol. 239, 1534-1536
- **CARTER et al.** *Proc. Natl. Acad. Sci. USA,* 1993, vol. 89, 4285
- **SIMS et al.** *J. Immunol.,* 1993, vol. 151, 2296
- **VAUGHAN et al.** *Nature Biotechnology,* 1998, vol. 16, 535-539
- Building an in vitro immune system human antibodies from phage display libraries. **GRIFFITHS ; HOOGENBOOM.** Protein Engineering of Antibody Molecules for Prophylactic and Therapeutic Applications in Man. Nottingham Academic, 1993, 45-64
- **BURTON ; BARBAS.** *Human Antibodies from combinatorial libraries.,* 65-82
- **JAKOBOVITS.** *Exp. Opin. Invest. Drugs,* 1998, vol. 7 (4), 607-614
- **WOLFF et al.** *Cancer Res.,* vol. 53, 2560-2565
- **BUUS, S. et al.** *Cell,* 1986, vol. 47, 1071
- **BABBITT, B. P. et al.** *Nature,* 1985, vol. 317, 359
- **TOWNSEND, A. ; BODMER, H.** *Annu. Rev. Immunol.,* 1989, vol. 7, 601
- **GERMAIN, R. N.** *Annu. Rev. Immunol.,* 1993, vol. 11, 403
- **SOUTHWOOD et al.** *J. Immunol.,* 1998, vol. 160, 3363
- **RAMMENSEE et al.** *Immunogenetics,* 1995, vol. 41, 178
- **SETTE, A. ; SIDNEY, J.** *Curr. Opin. Immunol.,* 1998, vol. 10, 478
- **ENGELHARD, V. H.** *Curr. Opin. Immunol.,* 1994, vol. 6, 13
- **SETTE, A. ; GREY, H. M.** *Curr. Opin. Immunol.,* 1992, vol. 4, 79
- **SINIGAGLIA, F. ; HAMMER.** *J. Curr. Biol.,* 1994, vol. 6, 52
- **RUPPERT et al.** *Cell,* 1993, vol. 74, 929-937
- **KONDO et al.** *J. Immunol.,* 1995, vol. 155, 4307-4312
- **SIDNEY et al.** *J. Immunol.,* 1996, vol. 157, 3480-3490
- **SIDNEY et al.** *Human Immunol.,* 1996, vol. 45, 79-93
- **SETTE, A. ; SIDNEY, J.** *Immunogenetics,* November 1999, vol. 50 (3-4), 201-12
- **MADDEN, D.R.** *Annu. Rev. Immunol.,* 1995, vol. 13, 587
- **SMITH et al.** *Immunity,* 1996, vol. 4, 203
- **FREMONT et al.** *Immunity,* 1998, vol. 8, 305
- **STEM et al.** *Structure,* 1994, vol. 2, 245
- **JONES, E.Y.** *Curr. Opin. Immunol.,* 1997, vol. 9, 75
- **BROWN, J. H. et al.** *Nature,* 1993, vol. 364, 33
- **GUO, H. C. et al.** *Proc. Natl. Acad. Sci. USA,* 1993, vol. 90, 8053
- **GUO, H. C. et al.** *Nature,* 1992, vol. 360, 364
- **SILVER, M. L. et al.** *Nature,* 1992, vol. 360, 367
- **MATSUMURA, M et al.** *Science,* 1992, vol. 257, 927
- **MADDEN et al.** *Cell,* 1992, vol. 70, 1035
- **FREMONT, D. H et al.** *Science,* 1992, vol. 257, 919
- **SAPER, M. A. ; BJORKMAN, P. J. ; WILEY, D. C.** *J. Mol. Biol.,* 1991, vol. 219, 277
- **WENTWORTH, P. A. et al.** *Mol. Immunol.,* 1995, vol. 32, 603
- **CELIS, E. et al.** *Proc. Natl. Acad. Sci. USA,* 1994, vol. 91, 2105
- **TSAI, V. et al.** *J. Immunol.,* 1997, vol. 158, 1796
- **KAWASHIMA, I. et al.** *Human Immunol.,* 1998, vol. 59, 1
- **WENTWORTH, P. A. et al.** *J. Immunol.,* 1996, vol. 26, 97
- **WENTWORTH, P. A. et al.** *Int. Immunol.,* 1996, vol. 8, 651
- **ALEXANDER, J. et al.** *J. Immunol.,* 1997, vol. 159, 4753
- **REHERMANN, B. et al.** *J. Exp. Med.,* 1995, vol. 181, 1047
- **DOOLAN, D. L. et al.** *Immunity,* 1997, vol. 7, 97
- **BERTONI, R. et al.** *J. Clin. Invest.,* 1997, vol. 100, 503
- **THRELKELD, S. C. et al.** *J. Immunol.,* 1997, vol. 159, 1648
- **DIEPOLDER, H. M. et al.** *J. Virol.,* 1997, vol. 71, 6011
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **ALERS et al.** *Lab Invest.,* 1997, vol. 77 (5), 437-438
- **ISAACS et al.** *Cancer Surv.,* 1995, vol. 23, 19-32
- **GREVER et al.** *J. Comp. Neurol.,* 09 December 1996, vol. 376 (2), 306-14
- **MURPHY et al.** *Prostate,* 2000, vol. 42 (4), 315-317
- **SU et al.** *Semin. Surg. Oncol.,* 2000, vol. 18 (1), 17-28
- **FREEMAN et al.** *J Urol,* 15 August 1995, vol. 4 (2 Pt 1), 474-8
- **DE MARZO et al.** *Am. J. Pathol.,* 1999, vol. 155 (6), 1985-1992
- **BROOKS et al.** *Cancer Epidemiol. Biomarkers Prev.,* 1998, vol. 7, 531-536
- **LETHE et al.** *Int. J. Cancer,* 1998, vol. 76 (6), 903-908

- Current Protocols In Molecular Biology. 1995
- **THOMAS.** *Proc. Natl. Acad. Sci. USA,* 1980, vol. 77, 5201-5205
- **VERKAIK et al.** *Urol. Res.,* 1997, vol. 25, 373-384
- **GHOSSEIN et al.** *J. Clin. Oncol.,* 1995, vol. 13, 1195-2000
- **HESTON et al.** *Clin. Chem.,* 1995, vol. 41, 1687-1688
- **BOCKING et al.** *Anal. Quant. Cytol.,* 1984, vol. 6 (2), 74-88
- **EPSTEIN.** *Hum. Pathol.,* 1995, vol. 26 (2), 223-9
- **THORSON et al.** *Mod. Pathol.,* 1998, vol. 11 (6), 543-51
- **BAISDEN et al.** *Am. J. Surg. Pathol.,* 1999, vol. 23 (8), 918-24
- **MARCOTTE et al.** *Nature,* 04 November 1999, vol. 402, 83-86
- **HAMILTON B.J. et al.** *Biochem Biophys. Res. Commun.,* 1999, vol. 261, 646-51
- **HODGE et al.** *Int. J. Cancer,* 1995, vol. 63, 231-237
- **FONG et al.** *J. Immunol.,* 1997, vol. 159, 3113-3117
- **HERYLN et al.** *Ann Med,* February 1999, vol. 31 (1), 66-78
- **MARUYAMA et al.** *Cancer Immunol Immunother,* 04 June 2000, vol. 9 (3), 123-32
- **VITIELLO, A. et al.** *J. Clin. Invest.,* 1995, vol. 95, 341
- **ELDRIDGE et al.** *Molec. Immunol.,* 1991, vol. 28, 287-294
- **ALONSO et al.** *Vaccine,* 1994, vol. 12, 299-306
- **JONES et al.** *Vaccine,* 1995, vol. 13, 675-681
- **TAKAHASHI et al.** *Nature,* 1990, vol. 344, 873-875
- **HU et al.** *Clin Exp Immunol.,* 1998, vol. 113, 235-243
- **TAM, J. P.** *Proc. Natl. Acad. Sci. U.S.A.,* 1988, vol. 85, 5409-5413
- **TAM, J.P.** *J. Immunol. Methods,* 1996, vol. 196, 17-32
- **PERKUS, M. E. et al.** Concepts in vaccine development. 1996, 379
- **CHAKRABARTI, S. et al.** *Nature,* 1986, vol. 320, 535
- **HU, S. L. et al.** *Nature,* 1986, vol. 320, 537
- **KIENY, M.-P. et al.** *AIDS Bio/Technology,* 1986, vol. 4, 790
- **TOP, F. H. et al.** *J. Infect. Dis.,* 1971, vol. 124, 148
- **CHANDA, P. K. et al.** *Virology,* 1990, vol. 175, 535
- **KOFLER, N. et al.** *J. Immunol. Methods.,* 1996, vol. 192, 25
- **ELDRIDGE, J. H. et al.** *Sem. Hematol.,* 1993, vol. 30, 16
- **FALO, L. D., JR. et al.** *Nature Med.,* 1995, vol. 7, 649
- **WARREN, H. S. ; VOGEL, F. R. ; CHEDID, L. A.** *Annu. Rev. Immunol.,* 1986, vol. 4, 369
- **GUPTA, R. K. et al.** *Vaccine,* 1993, vol. 11, 293
- **REDDY, R. et al.** *J. Immunol.,* 1992, vol. 148, 1585
- **ROCK, K. L.** *Immunol. Today,* 1996, vol. 17, 131
- **ULMER, J. B. et al.** *Science,* 1993, vol. 259, 1745
- **ROBINSON, H. L. ; HUNT, L. A. ; WEBSTER, R. G.** *Vaccine,* 1993, vol. 11, 957
- **SHIVER, J. W. et al.** Concepts in vaccine development. 1996, 423
- **CEASE, K. B. ; BERZOFSKY, J. A.** *Annu. Rev. Immunol.,* 1994, vol. 12, 923
- **WOLFF.** *Science,* 1990, vol. 247, 1465
- **RESTIFO.** *Curr. Opin. Immunol.,* 1996, vol. 8, 658-663
- **TSANG et al.** *J. Natl. Cancer Inst,* 1995, vol. 87, 982-990
- **STOVER et al.** *Nature,* 1991, vol. 351, 456-460
- **TJOA et al.** *Prostate,* 1996, vol. 28, 65-69
- **MURPHY et al.** *Prostate,* 1996, vol. 29, 371-380
- **ARTHUR et al.** *Cancer Gene Ther.,* 1997, vol. 4, 17-25
- **HENDERSON et al.** *Cancer Res.,* 1996, vol. 56, 3763-3770
- **RIBAS et al.** *Cancer Res.,* 1997, vol. 57, 2865-2869
- **ASHLEY et al.** *J. Exp. Med.,* 1997, vol. 186, 1177-1182
- **SLEVERS et al.** *Blood,* 01 June 1999, vol. 93 (11), 3678-3684
- **ARLEN et al.** *Crit. Rev. Immunol.,* 1998, vol. 18, 133-138
- **OZAKI et al.** *Blood,* 1997, vol. 90, 3179-3186
- **TSUNENARI et al.** *Blood,* 1997, vol. 90, 2437-2444
- **KASPRZYK et al.** *Cancer Res.,* 1992, vol. 52, 2771-2776
- **FUNAKOSHI et al.** *J. Immunother. Emphasis Tumor Immunol.,* 1996, vol. 19, 93-101
- **ZHONG et al.** *Leuk. Res.,* 1996, vol. 20, 581-589
- **MOUN et al.** *Cancer Res.,* 1994, vol. 54, 6160-6166
- **VELDERS et al.** *Cancer Res.,* 1995, vol. 55, 4398-4403
- **SHEPARD et al.** *J. Clin. Immunol.,* 1991, vol. 11, 117-127

- **FAN et al.** *Cancer Res.,* 1993, vol. 53, 4637-4642
- **PREWETT et al.** *International J. of Onco.,* 1996, vol. 9, 217-224
- **HANCOCK et al.** *Cancer Res.,* 1991, vol. 51, 4575-4580
- **WAGNER et al.** *Hybridoma,* 1997, vol. 16, 33-40
- **FOON et al.** *J. Clin. Invest.,* 1995, vol. 96, 334-342
- **HERLYN et al.** *Cancer Immunol. Immunother.,* 1996, vol. 43, 65-76
- **DAVILA ; CELIS.** *J. Immunol.,* 2000, vol. 165, 539-547
- **ROSENBERG et al.** *Science,* vol. 278, 1447-1450
- **ISHIOKA et al.** *J. Immunol.,* 1999, vol. 162, 3915-3925
- **AN, L. ; WHITTON, J. L.** *J. Virol.,* 1997, vol. 71, 2292
- **THOMSON, S. A. et al.** *J. Immunol.,* 1996, vol. 157, 822
- **WHITTON, J. L. et al.** *J. Virol.,* 1993, vol. 67, 348
- **HANKE, R. et al.** *Vaccine,* 1998, vol. 16, 426
- **MANNINO ; GOULD-FOGERITE.** *BioTechniques,* 1988, vol. 6 (7), 682
- **FELGNER et al.** *Proc. Nat'l Acad. Sci. USA,* 1987, vol. 84, 7413
- **DERES et al.** *Nature,* 1989, vol. 342, 561
- Remington's Pharmaceutical Sciences. Mack Publishing Co, 1985
- **SZOKA et al.** *Ann. Rev. Biophys. Bioeng.,* 1980, vol. 9, 467
- **MERRILL et al.** *J. Urol.,* 2000, vol. 163 (2), 503-5120
- **POLASCIK et al.** *J. Urol.,* August 1999, vol. 162 (2), 293-306
- **FORTIER et al.** *J. Nat. Cancer Inst.,* 1999, vol. 91 (19), 1635-1640
- **TULCHINSKY et al.** *Int J Mol Med,* July 1999, vol. 4 (1), 99-102
- **MINIMOTO et al.** *Cancer Detect Prev,* 2000, vol. 24 (1), 1-12
- **SHARIEF et al.** *Biochem. Mol. Biol. Int.,* 1994, vol. 33 (3), 567-74
- **OKEGAWA et al.** *J. Urol.,* 2000, vol. 163 (4), 1189-1190
- **STEPHAN et al.** *Urology,* 2000, vol. 55 (4), 560-3
- **ALANEN et al.** *Pathol. Res. Pract.,* 1996, vol. 192 (3), 233-7
- **ALANEN et al.** *Pathol. Res. Pract.,* 1996, vol. 192 (3), 233-237
- **CAETANO-ANOLLES, G.** *Biotechniques,* 1998, vol. 25 (3), 472-476478-480
- **ROBERTSON et al.** *Methods Mol. Biol.,* 1998, vol. 98, 121-154
- **SAWAI et al.** *Fetal Diagn. Ther.,* November 1996, vol. 11 (6), 407-13
- Current Protocols In Molecular Biology. 1995, vol. 2
- **TAKAHAMA K.** *Forensic Sci Int,* 28 June 1996, vol. 80 (1-2), 63-9
- **RICHARDSON ; MARASCO.** *TIBTECH,* 1995, vol. 13
- **RICHARDSON et al.** *Proc. Natl. Acad. Sci. USA,* 1995, vol. 92, 3137-3141
- **BEERLI et al.** *J. Biol. Chem.,* 1994, vol. 289, 23931-23936
- **DESHANE et al.** *Gene Ther.,* 1994, vol. 1, 332-337
- **KLEIN et al.** *Nature Medicine,* 1997, vol. 3, 402-408
- Remington's Pharmaceutical Sciences. 1980
- **KLEIN et al.** *Nature Med.,* 1997, vol. 3, 402-408
- **CRAFT et al.** *Cancer Res.,* 1999, vol. 59, 5030-5036
- **A. SALAMOV ; V. SOLOVYEV.** Ab initio gene finding in Drosophila genomic DNA. *Genome Research.,* April 2000, vol. 10 (4), 516-22
- **SOUTHAN, C.** A genomic perspective on human proteases. *FEBS Lett.,* 08 June 2001, vol. 498 (2-3), 214-8
- **DE SOUZA, S.J. et al.** Identification of human chromosome 22 transcribed sequences with ORF expressed sequence tags. *Proc. Natl Acad Sci U S A.,* 07 November 2000, vol. 97 (23), 12690-3
- **BRENNAN, S.O. et al.** Albumin banks peninsula: a new termination variant characterized by electrospray mass spectrometry. *Biochem Biophys Acta.,* 17 August 1999, vol. 1433 (1-2), 321-6
- **FERRANTI P et al.** Differential splicing of pre-messenger RNA produces multiple forms of mature caprine alpha(sl)-casein. *Eur J Biochem.,* 01 October 1997, vol. 249 (1), 1-7
- **WELLMANN S et al.** Specific reverse transcription-PCR quantification of vascular endothelial growth factor (VEGF) splice variants by LightCycler technology. *Clin Chem.,* April 2001, vol. 47 (4), 654-60
- **JIA, H.P. et al.** Discovery of new human beta-defensins using a genomics-based approach. *Gene,* 24 January 2001, vol. 263 (1-2), 211-8
- **BRIGLE, K.E. et al.** Organization of the murine reduced folate carrier gene and identification of variant splice forms. *Biochem Biophys Acta.,* 07 August 1997, vol. 1353 (2), 191-8
- **P. NOWOTNY ; J. M. KWON ; A. M. GOATE.** SNP analysis to dissect human traits. *Curr. Opin. Neurobiol.,* October 2001, vol. 11 (5), 637-641
- **M. PIRMOHAMED ; B. K. PARK.** Genetic susceptibility to adverse drug reactions. *Trends Pharmacol. Sci.,* June 2001, vol. 22 (6), 298-305

- **J. H. RILEY ; C. J. ALLAN ; E. LAI ; A. ROSES.** The use of single nucleotide polymorphisms in the isolation of common disease genes. *Pharmacogenomics,* February 2000, vol. 1 (1), 39-47
- **R. JUDSON ; J. C. STEPHENS ; A. WINDEMUTH.** The predictive power of haplotypes in clinical response. *Pharmacogenomics,* February 2000, vol. 1 (1), 15-26
- **P. BEAN.** The promising voyage of SNP target discovery. *Am. Clin. Lab.,* October 2001, vol. 20 (9), 18-20
- **K. M. WEISS.** In search of human variation. *Genome Res.,* July 1998, vol. 8 (7), 691-697
- **M. M. SHE.** Enabling large-scale pharmacogenetic studies by high-throughput mutation detection and genotyping technologies. *Clin. Chem.,* February 2001, vol. 47 (2), 164-172
- **Z. GU ; L. HILLIER ; P. Y. KWOK.** Single nucleotide polymorphism hunting in cyberspace. *Hum. Mutat.,* 1998, vol. 12 (4), 221-225
- **P. Y. KWOK.** Methods for genotyping single nucleotide polymorphisms. *Annu. Rev. Genomics Hum. Genet.,* 2001, vol. 2, 235-258
- **M. KOKORIS ; K. DIX ; K. MOYNIHAN ; J. MATHIS ; B. ERWIN ; P. GRASS ; B. HINES ; A. DUESTERHOEFT.** High-throughput SNP genotyping with the Masscode system. *Mol. Diagn.,* December 2000, vol. 5 (4), 329-340
- Production of 121P2A3 in Prokaryotic Systems. Current Protocols In Molecular Biology. 1995, vol. 2
- **LINSLEY, P.S. ; BRADY, W. ; URNES, M. ; GROSMAIRE, L. ; DAMLE, N. ; LEDBETTER, L.** *J.Exp. Med.,* 1991, vol. 174, 561-566
- **WELFORD K.** *Opt. Quant. Elect,* 1991, vol. 23, 1
- **MORTON ; MYSZKA.** *Methods in Enzymology,* 1998, vol. 295, 268
- **SIDNEY et al.** *Current Protocols in Immunology,* 1998, vol. 18, 3.1
- **SIDNEY et al.** *J. Immunol.,* 1995, vol. 154, 247
- **SETTE et al.** *Mol. Immunol.,* 1994, vol. 31, 813
- **GULUKOTA et al.** *J. Mol. Biol.,* 1997, vol. 267, 1258-126
- **SOUTHWOOD et al.** *J. Immunol.,* 1998, vol. 160, 3363-3373
- **TSAI et al.** *Critical Reviews in Immunology,* 1998, vol. 18 (1-2), 65-75
- **PARKHURST et al.** *J. Immunol.,* 1996, vol. 157, 2539
- **POGUE et al.** *Proc. Natl. Acad. Sci. USA,* 1995, vol. 92, 8166
- **SETTE et al.** Persistent Viral Infections. John Wiley & Sons, 1999
- **GELUK et al.** *J. Immunol.,* 1994, vol. 152, 5742-5748
- **BERTONI et al.** *J. Clin. Invest.,* 1997, vol. 100, 503
- **DOOLAN et al.** *Immunity,* 1997, vol. 7, 97
- **THRELKELD et al.** *J. Immunol.,* 1997, vol. 159, 1648
- **OSBORNE, M.J. ; RUBINSTEIN, A.** A course in game theory. MIT Press, 1994
- **ALEXANDER et al.** *J. Immunol.,* 1997, vol. 159, 4753-4761
- **VITIELLO et al.** *J. Exp. Med.,* 1991, vol. 173, 1007
- **SIJTS et al.** *J. Immunol.,* 1996, vol. 156, 683-692
- **DEMOTZ et al.** *Nature,* 1989, vol. 342, 682-684
- **KAGEYAMA et al.** *J. Immunol.,* 1995, vol. 154, 567-576
- **ALEXANDER et al.** *Immunity,* 1994, vol. 1, 751-761
- **BARNETT et al.** *Aids Res. and Human Retroviruses,* 1998, vol. 14 (3), S299-S309
- **HANKE et al.** *Vaccine,* 1998, vol. 16, 439-445
- **SEDEGAH et al.** *Proc. Natl. Acad. Sci USA,* 1998, vol. 95, 7648-53
- **HANKE ; MCMICHAEL.** *Immunol. Letters,* 1999, vol. 66, 177-181
- **ROBINSON et al.** *Nature Med.,* 1999, vol. 5, 526-34
- **OGG et al.** *Science,* 1998, vol. 279, 2103-2106
- **MUSEY et al.** *N. Engl. J. Med.,* 1997, vol. 337, 1267
- **REHERMANN et al.** *Nature Med.,* 1996, vol. 2, 1104, 1108
- **REHERMANN et al.** *J. Clin. Invest.,* 1996, vol. 97, 1655-1665
- **REHERMANN et al.** *J. Clin. Invest.,* 1996, vol. 98, 1432-1440
- **GUILHOT et al.** *J. Virol.,* 1992, vol. 66, 2670-2678
- *Nature Med.,* 1998, vol. 4, 328
- *Nature Med.,* 1996, vol. 2, 52
- *Prostate,* 1997, vol. 32, 272
- **KUBO et al.** *J. Immunol.,* 1994, vol. 152, 3913
- **BOLTON et al.** *Biochem. J.,* 1973, vol. 133, 529
- **MILD T et al.** *Oncol Res.,* 2001, vol. 12, 209
- **FU X et al.** *Int J Cancer.,* 1991, vol. 49, 938
- **KIGUCHI KET.** *Clin Exp Metastasis.,* 1998, vol. 16, 751
- **KAIGHN, M.E. et al.** *Invest Urol,* 1979, vol. 17 (1), 16-23
- **SAFFRAN, D. et al.** *PNAS,* vol. 10, 1073-1078, www.pnas.org/cgi/doi/10.1073/Rnas.051624698
- **HUBERT, R.S. et al.** *Proc Natl Acad Sci U S A,* 1999, vol. 96 (25), 14523

- **YOSHIDA Y et al.** *Anticancer Res.,* 1998, vol. 18, 327
- **AHN et al.** *Tumour Biol.,* 2001, vol. 22, 146
- **FU, X. et al.** *Int J Cancer,* 1992, vol. 52 (6), 987-90
- **KUBOTA, T.** *J Cell Biochem,* 1994, vol. 56 (1), 4-8
- **LIN S et al.** *Cancer Detect Prev.,* 2001, vol. 25, 202
- **MCCLUGGAGE W et al.** *Histopathol,* 2001, vol. 38, 542
- **POTAMIANOS S.** *Anticancer Res,* 2000, vol. 20 (2A), 925-948
- **MEERSON, N. R.** *Hepatology,* 1998, vol. 27, 563-568
- **DIVGI et al.** *J. Natl. Cancer Inst.,* 1991, vol. 83, 97-104
- **FUKUSHI M et al.** *Febs,* 1999, vol. 442, 83
- **GUPTA, K. et al.** *J. Virol,* 2000, vol. 74, 11811
- **BRIGGS SD et al.** *J Biol Chem.,* 1997, vol. 272, 17899
- **BRIGGS SD et al.** *J Biol Chem.,* 2001, vol. 276, 13847
- **BAUR AS et al.** *Immunity,* 1997, vol. 6, 283
- **XU XN ; SCREATON G.** *Nat Immunol.,* 2001, vol. 2, 384
- **KRAMER-HAMMERLE S et al.** *AIDS Res Hum Retroviruses.,* 2001, vol. 17, 597
- **DI CUNTO F et al.** *J Biol Chem,* 1998, vol. 273, 29706
- **MERMOD N et al.** *Cell,* 1989, vol. 58, 741
- *J Neurochem.,* 2001, vol. 76, 217-223
- **HEYNINCK, K. et al.** *J. Cell. Biol.,* 1999, vol. 145, 1471
- *Cell Growth Differ.,* 2000, vol. 11, 279
- *J Biol Chem.,* 1999, vol. 274, 801
- *Oncogene,* 2000, vol. 19, 3003
- *J. Cell Biol.,* 1997, vol. 138, 913
- **FRASER SP ; GRIMES JA ; DJAMGOZ MB.** *Prostate,* 2000, vol. 44, 61
- **JOHNSON DE ; OCHIENG J ; EVANS SL.** *Anticancer Drugs.,* 1996, vol. 7, 288
- **SONG Z. et al.** *Cancer Res.,* 2000, vol. 60, 6730
- *Cancer Res.,* 1999, vol. 59, 6010
- **ABDEL-MALEK ZA.** *J Cell Physiol.,* 1988, vol. 136, 247
- **HANAHAN D ; FOLKMAN J.** *Cell,* 1996, vol. 86, 353
- **FOLKMAN J.** *Endocrinology,* 1998, vol. 139, 441
- **SMID-KOOPMAN E et al.** *Br J Cancer.,* 2000, vol. 83, 246
- **CHEN K et al.** *Thyroid,* 2001, vol. 11, 41
- **HAIER et al.** *Br. J. Cancer.,* 1999, vol. 80, 1867
- **LEHR ; PIENTA.** *J. Natl. Cancer Inst.,* 1998, vol. 90, 118
- **RENKEMA GH et al.** *Curr Biol.,* 1999, vol. 9, 1407
- **KARAKESISOGLOU I ; YANG Y ; FUCHS E.** *J Cell Biol.,* 2000, vol. 149, 195
- *Curr Opin Chem Biol.,* 1999, vol. 3, 64